# EUROPEAN PATENT APPLICATION

(11) **EP 3 825 303 A1**
(43) Date of publication of application: **26.05.2021**
(21) Application number: 19838574.2
(22) Date of filing: 17.07.2019
(51) Int. Cl.: C07D 205/04

(54) **T-TYPE CALCIUM CHANNEL BLOCKER**

(30) Priority: 17.07.2018 JP 2018134402
(71) Applicant: Nippon Chemiphar Co., Ltd., Tokyo 101-0032 (JP); Kinki University, Higashi-Osaka-shi Osaka 577-8502 (JP)
(72) Inventor: TANAKA, Hiroto, Misato-shi, Saitama 341-0005 (JP); OOI, Isao, Misato-shi, Saitama 341-0005 (JP); SAITO, Daisuke, Misato-shi, Saitama 341-0005 (JP); HAYASHIDA, Kohei, Misato-shi, Saitama 341-0005 (JP); YAMAMOTO, Kohei, Misato-shi, Saitama 341-0005 (JP); KAWABATA, Atsufumi, Higashiosaka-shi, Osaka 577-8502 (JP)
(74) Representative: V.O.
(86) International application number: PCT/JP2019/028168
(87) International publication number: WO 2020/017569

(57) **Abstract**

To provide a novel T-type calcium channel blocker.

A compound represented by the following General Formula (I), a tautomer or a stereoisomer of the compound, a pharmaceutically acceptable salt of the compound, or a solvate of the compound, the tautomer, the stereoisomer, or the salt is used as a T-type calcium channel blocker. 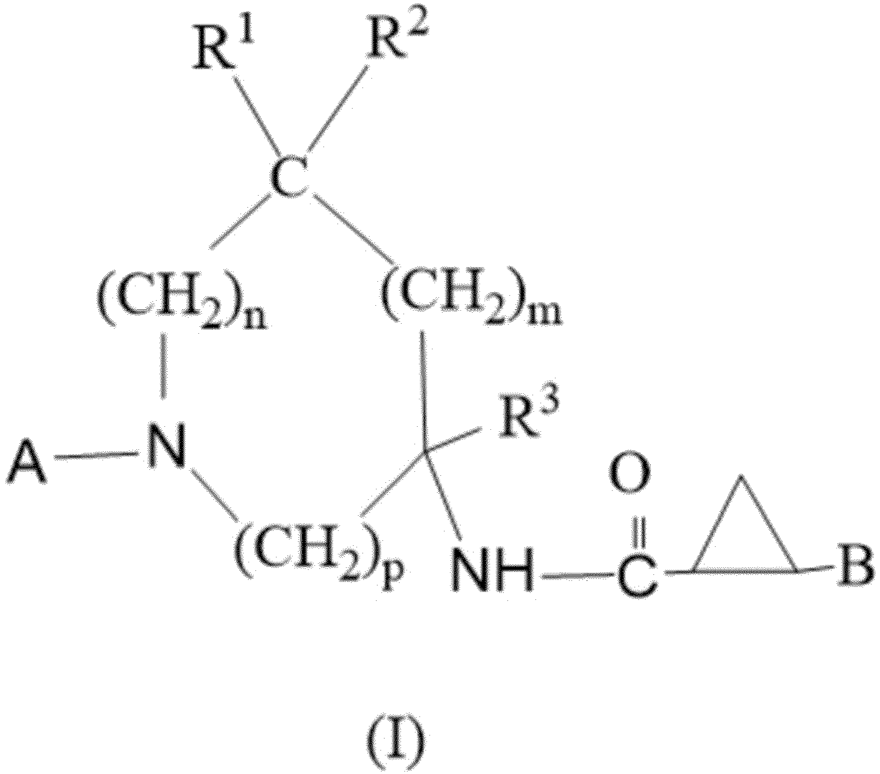 wherein A represents a phenyl which may have a substituent, a 4-membered to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent cyclic amino by means of a carbon atom constituting these rings;
B represents a phenyl which may have a substituent, a 5-membered or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to the adjacent cyclopropyl ring by means of a carbon atom constituting these rings;
R¹ and R², which may be identical or different, each represent a hydrogen atom, a halogen atom, or the like;
R³ represents a hydrogen atom, a halogen atom, or the like;
n and m, which may be identical or different, each represent 0 or 1; and p represents 1 or 2.

## Description

### Technical Field

The present invention relates to cyclic amine compounds having amide bonds, and to a T-type calcium channel blocker containing these as active ingredients.

### Background Art

T-type calcium channels are expressed at the dorsal root ganglia and the spinal cord dorsal horns of primary afferent nerve fibers (Non Patent Literature 1). It has been reported that in knockout mice deficient in T-type calcium channels (Cav3.2 channels), pain-related behaviors in acute pain, inflammatory pain, and visceral pain are reduced (Non Patent Literature 2), and therefore, Cav3.2 channels are closely involved in the transmission of pain. Furthermore, it has been reported that at the dorsal root ganglia of pathological animal models associated with pain such as neuropathic pain (Non Patent Literature 3), painful diabetic neuropathy (Non Patent Literature 4 and 5), and irritable bowel syndrome (Non Patent Literature 6), the current density of T-type calcium channel increases, and T-type calcium channels become overactive. Thus, T-type calcium channels can serve as a target for a pain treatment drug of a new mechanism. Since T-type calcium channel blockers have an action mechanism different from that of opioid receptor agonists and α2δ ligands, it is expected that T-type calcium channel blockers may also be effective against chronic pain that is resistant to existing drugs.

Incidentally, regarding the cyclic amine compounds having amide bonds,
a compound represented by the following formula is known in Patent Literature 1: 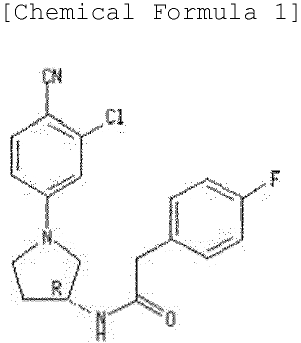 a compound represented by the following formula is known in Patent Literature 2: 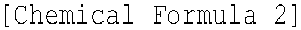 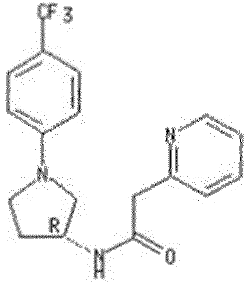 a compound represented by the following formula is known in Patent Literature 3: 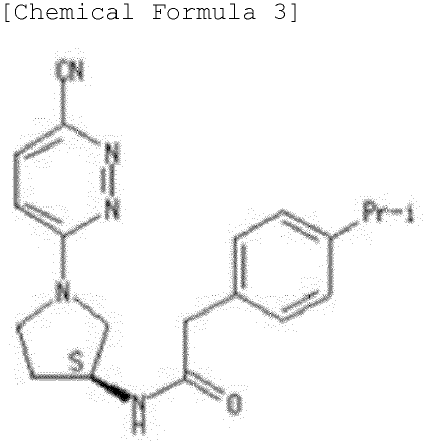 and a compound represented by the following formula is known in Patent Literature 4:

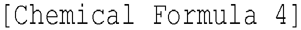

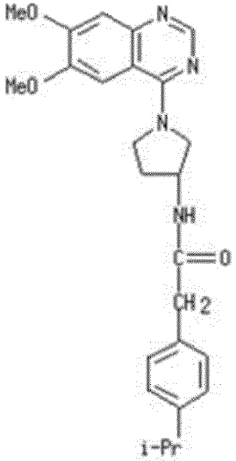

However, in the above-described Patent Literature 1 to 4, there is no description to the effect that these compounds have T-type calcium channel blocking action.

On the other hand, regarding compounds having voltage-dependent T-type calcium channel blocking action,
a compound represented by the following general formula is described in Patent Literature 5: 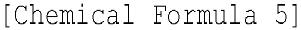 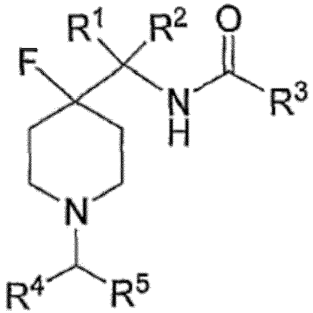 and a compound represented by the following general formula is described in Patent Literature 6:

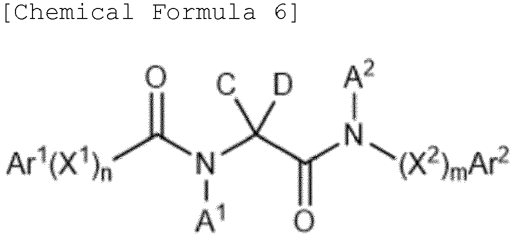

Furthermore, a compound (RQ-00311651) represented by the following formula is described in Patent Literature 7: 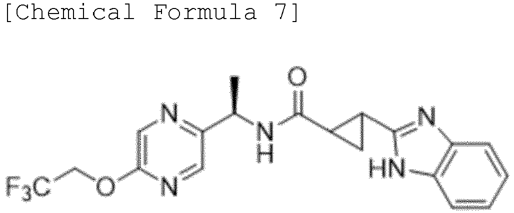 a compound represented by the following formula is described in Patent Literature 8: 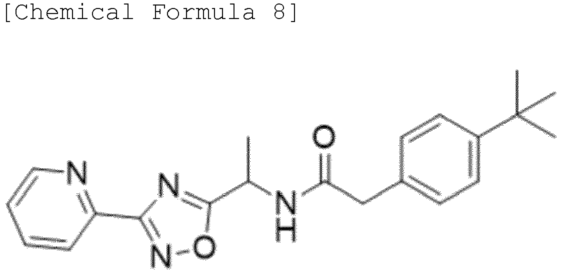 and a compound represented by the following general formula is described in Patent Literature 9:

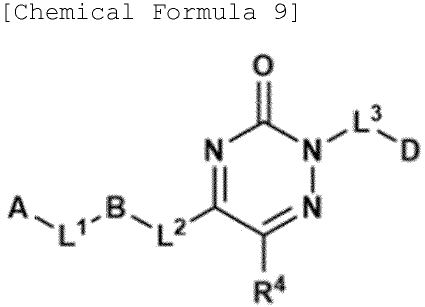

Also, the inventors of the present invention have filed a patent application (Patent Literature 10) related to a compound represented by the following general formula:

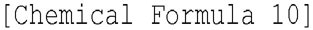

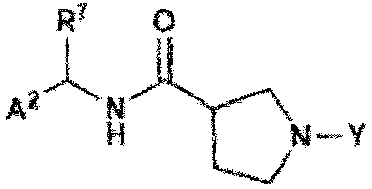

Meanwhile, in the above-described Patent Literature 9, pain is mentioned as a target disease for the compound having T-type calcium channel blocking action, chronic pain is mentioned as a limited target disease, and neuropathic pain is mentioned as a further limited target disease.

Then, in this patent literature, it is described that "pain is classified into chronic and acute pains including neuropathic, inflammatory, cancerous, and visceral pains, and causative diseases thereof include diabetic neuropathy, traumatic neuropathy, nerve compression, strangulation, spinal cord injury, stroke, fibromyalgia, carpal tunnel syndrome, arthrosis deformans, rheumatoid arthritis and multiple sclerosis, herpes zoster, herpes simplex, syphilis, cancer chemotherapy, neuropathy induced by HIV and HIV treatment, chronic arthralgia, postherpetic neuralgia, neuroma pain, trigeminal neuralgia, phantom limb pain, postoperative pain, stump pain, toothache, nerve plexus neuropathy, glossopharyngeal neuralgia, laryngeal neuralgia, migraine, cancerous neuropathy, polyneuropathy, causalgia, low back pain, complex regional pain syndrome (CRPS), and thalamic pain". Furthermore, it is also described to the effect that pains originating from causes other than the causes listed above are included in the target diseases of the active ingredient described in the above-described patent literature. Also, it is described to the effect that "the diseases other than pain include diseases associated with disorders in the central nervous system (CNS), diseases associated with disorders in the bladder function, stroke, pruritus, atopic dermatitis, hypertension, hyperaldosteronemia, edema, ischemic heart disease, age-related macular degeneration, cancer, diabetes mellitus, infertility and sexual dysfunction, arrhythmia, and kidney diseases; the diseases associated with disorders in the central nervous system (CNS) include epilepsy, essential tremor, schizophrenia, Parkinson's disease, manic depression, bipolar disorder, depression, anxiety, cognitive impairment, drug dependence, Huntington's disease, and sleep disorders; and the diseases associated with disorders in the bladder function include overactive bladder, and the like".

### Citation List

### Patent Literature

Patent Literature 1: WO 2009/131196
Patent Literature 2: WO 2005/095327
Patent Literature 3: WO 2017/223474
Patent Literature 4: WO 2006/135649
Patent Literature 5: JP 2009-500340 A
Patent Literature 6: WO 2008/110008
Patent Literature 7: WO 2010/137351
Patent Literature 8: WO 2011/053542
Patent Literature 9: WO 2015/093534
Patent Literature 10: WO 2017/122754

### Non Patent Literature

Non Patent Literature 1: Jacus MO, Uebele VN, Renger JJ, Todorovic SM.; Presynaptic Cav3.2 channels regulate excitatory neurotransmission in nociceptive dorsal horn neurons. J Neurosci. 2012, 32, 9374-82.
Non Patent Literature 2: Choi S, Na HS, Kim J, Lee J, Lee S, Kim D, Park J, Chen CC, Campbell KP, Shin HS.; Attenuated pain responses in mice lacking CaV3.2 T-type channels. Genes Brain Behav. 2007, 6, 425-331.
Non Patent Literature 3: Jagodic MM, Pathirathna S, Joksovic PM, Lee W, Nelson MT, Naik AK, Su P, Jevtovic-Todorovic V, Todorovic SM.; Upregulation of the T-type calcium current in small rat sensory neurons after chronic constrictive injury of the sciatic nerve. J Neurophysiol. 2008, 99, 3151-6.
Non Patent Literature 4: Messinger RB, Naik AK, Jagodic MM, Nelson MT, Lee WY, Choe WJ, Orestes P, Latham JR, Todorovic SM, Jevtovic-Todorovic V.; In vivo silencing of the Ca(V)3.2 T-type calcium channels in sensory neurons alleviates hyperalgesia in rats with streptozocin-induced diabetic neuropathy. Pain. 2009, 145, 184-95.
Non Patent Literature 5: Jagodic MM, Pathirathna S, Nelson MT, Mancuso S, Joksovic PM, Rosenberg ER, Bayliss DA, Jevtovic-Todorovic V, Todorovic SM.; Cell-specific alterations of T-type calcium current in painful diabetic neuropathy enhance excitability of sensory neurons. J Neurosci. 2007, 27, 3305-16.
Non Patent Literature 6: Marger F, Gelot A, Alloui A, Matricon J, Ferrer JF, Barrere C, Pizzoccaro A, Muller E, Nargeot J, Snutch TP, Eschalier A, Bourinet E, Ardid D.; T-type calcium channels contribute to colonic hypersensitivity in a rat model of irritable bowel syndrome. Proc Natl Acad Sci USA. 2011, 5. 11268-73.

### Summary of Invention

### Technical Problem

It is an object of the present invention to provide cyclic amine compounds having amide bonds, the cyclic amine compounds having T-type calcium channel blocking action, and to provide a T-type calcium channel blocker containing these as active ingredients.

### Solution to Problem

The present inventors conducted an investigation on T-type calcium channel blockers, and as a result, the inventors found that cyclic amine compounds having amide bonds and having structures different from the structures of the compounds described in the above-described Patent Literature 1 to 10, have excellent T-type calcium channel blocking action, thus completing the present invention.

That is, the present invention relates to a compound represented by the following General Formula (I), a tautomer or a stereoisomer of the compound, a pharmaceutically acceptable salt of the compound, or a solvate of the compound, the tautomer, the stereoisomer, or the salt. 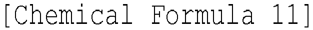 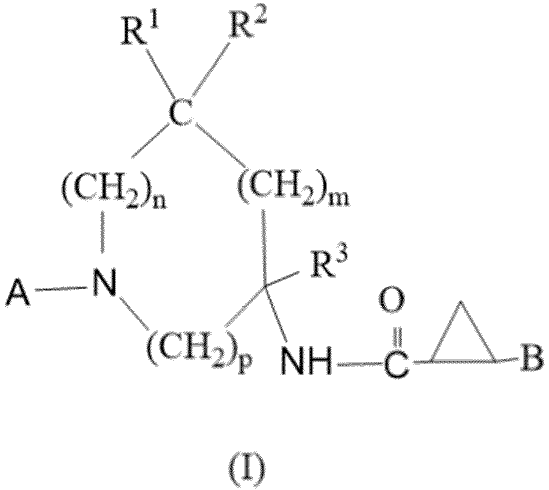
wherein A represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, while herein, the heteroaryl ring or the heterocondensed ring may have a substituent and is bonded to a nitrogen atom of the adjacent cyclic amine by means of a carbon atom constituting these rings;
B represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to the adjacent cyclopropane ring by means of a carbon atom constituting these rings;
R¹ and R², which may be identical or different, each represent a hydrogen atom, a halogen atom, a hydroxy group, a C₁₋₈ alkyl group, or a C₁₋₈ alkyl group substituted with one to three halogen atoms;
or R¹, R², and the carbon atom to which R¹ and R² are bonded may be joined together and form a 3- to 5-membered cycloalkyl group;
R³ represents a hydrogen atom, a halogen atom, a carboxyl group, a cyano group, a carbamoyl group, a C₁₋₈ alkyl group, a C₁₋₈ alkoxycarbonyl group, a C₁₋₈ alkyl group substituted with a C₁₋₈ alkoxy group, a C₁₋₈ alkyl group substituted with a hydroxy group, a C₁₋₈ alkyl group substituted with one to three halogen atoms, or a C₁₋₈ alkyl group substituted with an acyloxy group;
or R² and R³ may be joined together and form methylene or ethylene;
n and m, which may be identical or different, each represent 0 or 1; and p represents 1 or 2;
further, the substituent that may be carried by the phenyl of A, the heteroaryl ring of A, and the heterocondensed ring of A is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B, the heteroaryl ring of B, and the heterocondensed ring of B is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B, the heteroaryl ring of B, and the heterocondensed ring of B is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

Furthermore, the present invention relates to a compound represented by the following General Formula (II), a tautomer or a stereoisomer of the compound, a pharmaceutically acceptable salt of the compound, or a solvate of the compound, the tautomer, the stereoisomer, or the salt. 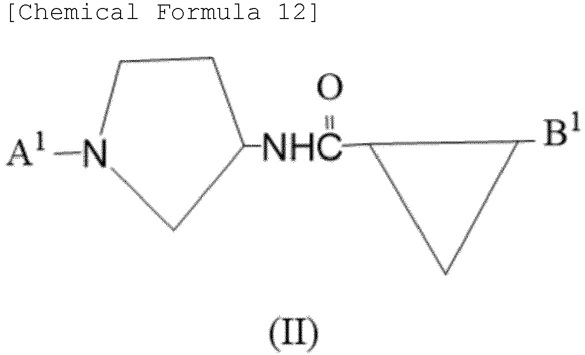
wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent pyrrolidine by means of a carbon atom constituting these rings; and
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to the adjacent cyclopropane ring by means of a carbon atom constituting these rings;
further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

Furthermore, the present invention relates to a compound represented by the following General Formula (III), a tautomer or a stereoisomer of the compound, a pharmaceutically acceptable salt of the compound, or a solvate of the compound, the tautomer, the stereoisomer, or the salt. 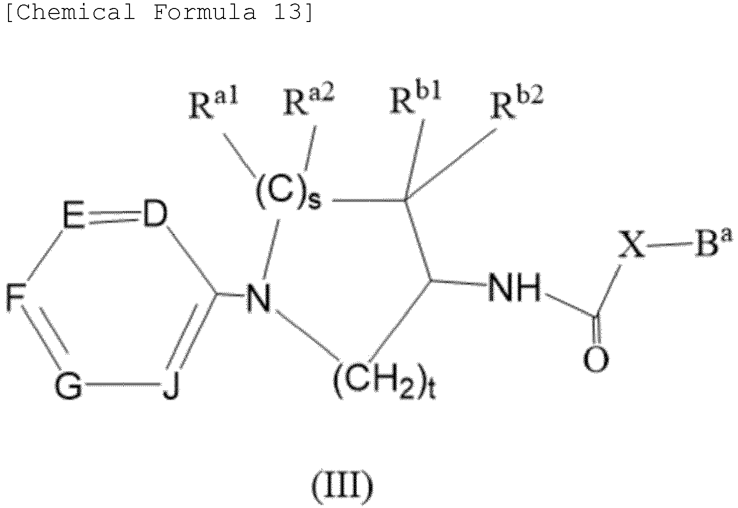
wherein D, E, F, G, and J are such that any two of them each represent N while the others represent CRs which may be identical or different, or any one of them represents N while the others represent CRs which may be identical or different, or all of them are CRs which may be identical or different;
wherein R represents a hydrogen atom, a C₁₋₈ alkyl group, a C₁₋₈ alkoxy group, a C₁₋₈ alkyl group substituted with one to three halogen atoms, a C₁₋₈ alkoxy group which may be substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, or a C₁₋₈ alkoxy group substituted with a C₁₋₈ alkoxycarbonyl group;
R^{a1}, R^{a2}, R^{b1}, and R^{b2}, which may be identical or different, each represent a hydrogen atom, a halogen atom, a hydroxy group, a C₁₋₈ alkyl group, or a C₁₋₈ alkyl group substituted with one to three halogen atoms;
or R^{a1}, R^{a2}, and the carbon atom to which R^{a1} and R^{a2} are bonded may be joined together and form a 3- to 5-membered cycloalkyl group, or R^{b1}, R^{b2}, and the carbon atom to which R^{b1} and R^{b2} are bonded may be joined together and form a 3- to 5-membered cycloalkyl group; s represents 0, 1, or 2; t represents 1 or 2;
B^{a} represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to adjacent X by means of a carbon atom constituting these rings;
X represents: 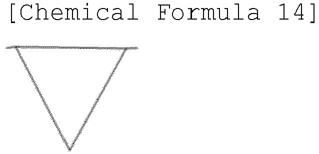
or 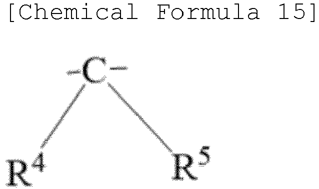
wherein - represents a linking bond;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group;
or R⁴, R⁵, and the carbon atom to which R⁴ and R⁵ are bonded may be joined together and form a 3- to 5-membered cycloalkyl group;
further, the substituent that may be carried by the phenyl of B^{a}, the heteroaryl ring of B^{a}, and the heterocondensed ring of B^{a} is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B^{a}, the heteroaryl ring of B^{a}, and the heterocondensed ring of B^{a} is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that, when a 6-membered ring composed of D to J may be a phenyl which may have a substituent, or a pyridazine which may have a substituent, and X represents the following: 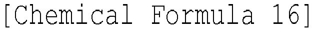 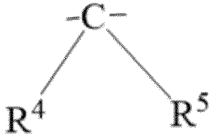
B^{a} represents a heterocondensed ring which may have the above-mentioned substituent.

Furthermore, the present invention relates to a compound represented by the following General Formula (IV), a tautomer or a stereoisomer of the compound, a pharmaceutically acceptable salt of the compound, or a solvate of the compound, the tautomer, the stereoisomer, or the salt. 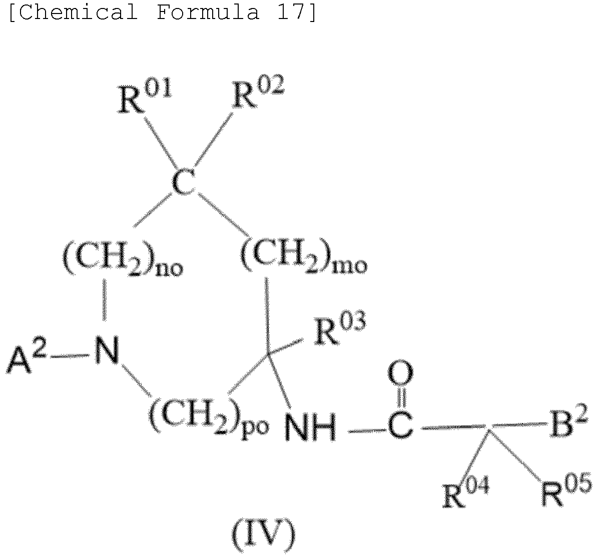
wherein A² represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent cyclic amine by means of a carbon atom constituting these rings;
B² represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to adjacent C(R⁰⁴) (R⁰⁵) by means of a carbon atom constituting these rings;
R⁰¹ and R⁰², which may be identical or different, each represent a hydrogen atom, a halogen atom, a hydroxy group, a C₁₋₈ alkyl group, or a C₁₋₈ alkyl group substituted with one to three halogen atoms;
or R⁰¹, R⁰², and the carbon atom to which R⁰¹ and R⁰² are bonded may be joined together and form a 3- to 5-membered cycloalkyl group;
R⁰³ represents a hydrogen atom, a halogen atom, a carboxyl group, a cyano group, a carbamoyl group, a C₁₋₈ alkyl group, a C₁₋₈ alkoxycarbonyl group, a C₁₋₈ alkyl group substituted with a C₁₋₈ alkoxy group, a C₁₋₈ alkyl group substituted with a hydroxy group, a C₁₋₈ alkyl group substituted with one to three halogen atoms, or a C₁₋₈ alkyl group substituted with an acyloxy group;
or R⁰² and R⁰³ may be joined together and form methylene or ethylene;
R⁰⁴ and R⁰⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group;
or R⁰⁴, R⁰⁵, and the carbon atom to which R⁰⁴ and R⁰⁵ are bonded may be joined together and form a 3- to 5-membered cycloalkyl group;
no and mo, which may be identical or different, each represent 0 or 1; and po represents 1 or 2;
further, the substituent that may be carried by the phenyl of A², the heteroaryl ring of A², and the heterocondensed ring of A² is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B², the heteroaryl ring of B², and the heterocondensed ring of B² is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B², the heteroaryl ring of B², and the heterocondensed ring of B² is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that, when A² represents a phenyl which may have a substituent, a pyridazine which may have a substituent, and a quinazoline which may have a substituent, B² represents a heterocondensed ring which may have the above-mentioned substituent.

Furthermore, the present invention relates to a compound represented by the following General Formula (V), a tautomer or a stereoisomer of the compound, a pharmaceutically acceptable salt of the compound, or a solvate of the compound, the tautomer, the stereoisomer, or the salt. 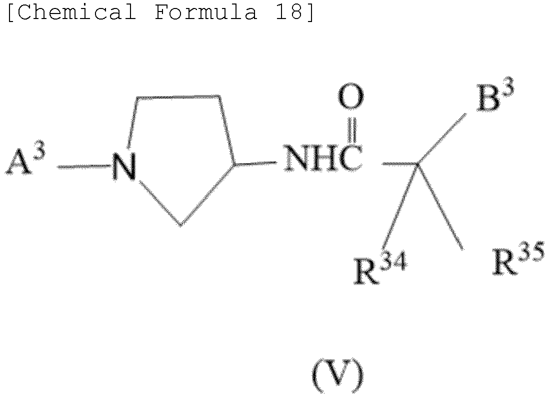
wherein A³ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein, the heteroaryl ring or the heterocondensed ring may have a substituent and is bonded to a nitrogen atom of the adjacent pyrrolidine by means of a carbon atom constituting these rings; and
B³ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to adjacent C(R³⁴)(R³⁵) by means of a carbon atom constituting these rings;
R³⁴ and R³⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group;
or R³⁴, R³⁵, and the carbon atom to which R³⁴ and R³⁵ are bonded may be joined together and form a 3- to 5-membered cycloalkyl group;
further, the substituent that may be carried by the phenyl of A³, the heteroaryl ring of A³, and the heterocondensed ring of A³ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B³, the heteroaryl ring of B³, and the heterocondensed ring of B³ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B³, the heteroaryl ring of B³, and the heterocondensed ring of B³ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that, when A³ represents a phenyl which may have a substituent, a pyridazine which may have a substituent, and a quinazoline which may have a substituent, B³ represents a heterocondensed ring which may have the above-mentioned substituent.

Furthermore, the present invention relates to a compound represented by the following General Formula (VI), a tautomer or a stereoisomer of the compound, a pharmaceutically acceptable salt of the compound, or a solvate of the compound, the tautomer, the stereoisomer, or the salt. 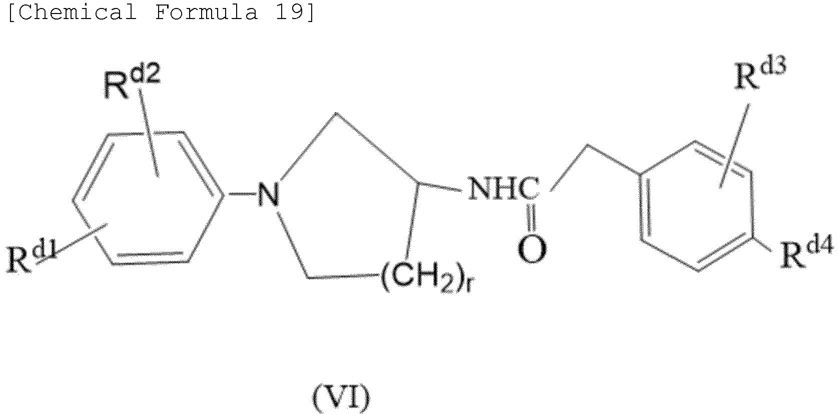
wherein R^{d1}, R^{d2}, and R^{d3}, which are identical or different, each represent a hydrogen atom, a halogen atom, a C₁₋₈ alkyl group, a C₁₋₈ alkyl group substituted with one to three halogen atoms, a C₁₋₈ alkoxy group, a C₁₋₈ alkoxy group substituted with one to three halogen atoms, a hydroxy group, or a C₁₋₈ alkoxy group substituted with a C₁₋₈ alkoxycarbonyl group;
R^{d4} represents a C₁₋₈ alkyl substituted with one to three halogen atoms, a tert-butyl, or a cyclopropyl;
and r represents 0, 1, or 2.

Furthermore, the present invention relates to a pharmaceutical composition containing a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, as an active ingredient.

Furthermore, the present invention relates to a T-type calcium channel blocker containing a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, as an active ingredient.

Furthermore, the present invention relates to a therapeutic or prophylactic agent for stroke, atopic dermatitis, hypertension, hyperaldosteronemia, edema, ischemic heart disease, age-related macular degeneration, cancer, diabetes mellitus, infertility, sexual dysfunction, arrhythmia, kidney diseases, epilepsy, essential tremor, schizophrenia, Parkinson's disease, manic depression, bipolar disorder, depression, anxiety, cognitive impairment, drug dependence, Huntington's disease, sleep disorders, or overactive bladder, the therapeutic or prophylactic agent containing a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, as an active ingredient.

Furthermore, the present invention relates to a therapeutic or prophylactic agent for a disease related to T-type calcium channels, the therapeutic or prophylactic agent containing a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, as an active ingredient.

Furthermore, the present invention relates to a prophylactic or therapeutic agent for acute pain, chronic pain, or neuropathic pain, the therapeutic or prophylactic agent containing a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, as an active ingredient.

Furthermore, the present invention relates to the use of a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, for the prevention or treatment of stroke, atopic dermatitis, hypertension, hyperaldosteronemia, edema, ischemic heart disease, age-related macular degeneration, cancer, diabetes mellitus, infertility, sexual dysfunction, arrhythmia, kidney diseases, epilepsy, essential tremor, schizophrenia, Parkinson's disease, manic depression, bipolar disorder, depression, anxiety, cognitive impairment, drug dependence, Huntington's disease, sleep disorders, or overactive bladder.

Furthermore, the present invention relates to the use of a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, for the prevention or treatment of acute pain, chronic pain, or neuropathic pain.

Moreover, the present invention relates to a method for treating acute pain, chronic pain, or neuropathic pain in a human being, the method including a step of administering an effective amount of a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, to the human being.

Furthermore, the present invention relates to the use of a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, for the production of a T-type calcium channel blocker.

Furthermore, the present invention relates to the use of a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, for the production of a prophylactic agent or a therapeutic agent for stroke, atopic dermatitis, hypertension, hyperaldosteronemia, edema, ischemic heart disease, age-related macular degeneration, cancer, diabetes mellitus, infertility, sexual dysfunction, arrhythmia, kidney diseases, epilepsy, essential tremor, schizophrenia, Parkinson's disease, manic depression, bipolar disorder, depression, anxiety, cognitive impairment, drug dependence, Huntington's disease, sleep disorders, or overactive bladder.

Furthermore, the present invention relates to the use of a compound represented by any of the above-described General Formulae (I) to (VI), a tautomer or a stereoisomer of the compound, a pharmaceutically acceptable salt of the compound, or a solvate of the compound, the tautomer, the stereoisomer, or the salt, for the production of a prophylactic agent or a therapeutic agent for acute pain, chronic pain, or neuropathic pain.

### Advantageous Effects of Invention

The compound of the present invention has excellent T-type calcium channel blocking action.

### Brief Description of Drawings

Fig. 1 is a diagram showing the test results for allodynia in a PSNL model using the compound described in Example 4.
Fig. 2 is a diagram showing the test results for allodynia in a PSNL model using the compound described in Example 144.

### Description of Embodiments

Next, the present invention will be described in more detail.

According to the present specification, the C₁₋₈ alkyl group may be a linear, branched, or cyclic alkyl group such as a methyl group, an ethyl group, a propyl group, an isopropyl group, a butyl group, a cyclopropyl group, a cyclopentyl group, a cyclohexyl group, an isobutyl group, a tert-butyl group, a pentyl group, or a hexyl group.

The C₁₋₈ alkyl group substituted with one to three halogen atoms may be a methyl group, an ethyl group, a propyl group, an isopropyl group, a butyl group, a tert-butyl group, or the like, each group being substituted with one to three of a halogen atom such as a fluorine atom, a chlorine atom, or a bromine atom, and preferred examples include a trifluoromethyl group, a chloromethyl group, a 2-chloroethyl group, a 2-bromoethyl group, a 2-fluoroethyl group, and the like.

The C₁₋₈ alkoxy group may be a methoxy group, an ethoxy group, a propoxy group, an isopropoxy group, a butoxy group, an isobutoxy group, a tert-butoxy group, a pentyloxy group, a hexyloxy group, or the like.

The C₁₋₈ alkoxy group substituted with one to three halogen atoms may be a methoxy group, an ethoxy group, a propoxy group, an isopropoxy group, a butoxy group, a tert-butoxy group, or the like, each group being substituted with one to three of a halogen atom such as a fluorine atom, a chlorine atom, or a bromine atom, and preferred examples include a trifluoromethoxy group, a chloromethoxy group, a 2-chloroethoxy group, a 2-bromoethoxy group, a 2-fluoroethoxy group, a 2,2,2-trifluoroethoxy group, and the like.

The halogen atom may be a fluorine atom, a chlorine atom, a bromine atom, or the like.

The C₁₋₈ alkoxycarbonyl group may be a methoxycarbonyl group, an ethoxycarbonyl group, a butoxycarbonyl group, a tert-butoxycarbonyl group, or the like.

The acyl group may be preferably an acyl group having 1 to 7 carbon atoms, and more preferred examples include an acetyl group, a propionyl group, a benzoyl group, and the like.

The C₁₋₈ alkyl group substituted with a hydroxy group may be a hydroxymethyl group, a 2-hydroxypropan-2-yl, or the like.

The C₁₋₈ alkyl group substituted with an acyloxy group may be an acetyloxymethyl group, or the like.

The C₁₋₈ alkoxy group substituted with a hydroxy group may be a 2-hydroxyethoxy group, or the like.

The C₁₋₈ alkylsulfonyl group may be a methanesulfonyl group or the like.

The (C₁₋₈ alkoxy-substituted C₁₋₈ alkyl) amino group may be a (2-methoxyethyl)amino group, or the like.

The 3-membered to 5-membered cycloalkyl group may be a cyclopropyl group, a cyclobutyl group, or the like.

The C₁₋₈ alkylamino group may be an ethylamino group, or the like.

The C₂₋₁₂ dialkylamino group may be a dimethylamino group, a diethylamino group, or the like.

The (C₁₋₈ alkyl) (C₁₋₈ alkoxy-substituted C₁₋₈ alkyl)amino group may be an N-ethyl-N-(2-methoxyethyl)amino group, or the like.

The tri(Cₗ-₈ alkyl) silyl group may be a trimethylsilyl group, a triethylsilyl group, or the like.

The acylamino group may be an acetylamino group, or the like.

The (N-acyl) (N-C₁₋₈ alkyl) amino group may be an (N-acetyl)(N-ethyl)amino group, or the like.

The 3-membered to 6-membered cyclic ether may be THF, oxetane, or the like.

The C₁₋₈ alkyl group substituted with a C₁₋₈ alkoxy group may be a linear, branched, or cyclic alkyl group such as a methyl group, an ethyl group, a propyl group, an isopropyl group, a butyl group, a cyclopropyl group, a cyclopentyl group, a cyclohexyl group, an isobutyl group, a tert-butyl group, a pentyl group, or a hexyl group, each group being substituted with an alkoxy group having 1 to 6 carbon atoms, such as a methoxy group, an ethoxy group, a propoxy group, an isopropoxy group, a butoxy group, an isobutoxy group, a tert-butoxy group, a pentyloxy group, or a hexyloxy group.

The C₁₋₈ alkoxy group substituted with a C₁₋₈ alkoxycarbonyl group may be an isopropyloxy group substituted with ethoxycarbonyl, or the like.

The 4-membered to 6-membered heteroaryl ring composed of one to three identical or different heteroatoms selected from an oxygen atom, a sulfur atom, and a nitrogen atom and carbon atoms as ring-constituting atoms for A of General Formula (I), A¹ of General Formula (II), A² of General Formula (IV), and A³ of General Formula (V), may be pyridine, pyrazine, pyrimidine, pyridazine, oxazole, thiazole, pyrimidine, furan, thiophene, or the like.

The heterocondensed ring composed of a 4-membered to 6-membered heteroaryl ring composed of one to three identical or different heteroatoms selected from an oxygen atom, a sulfur atom, and a nitrogen atom and carbon atoms as ring-constituting atoms and a benzene ring or a 6-membered heteroaryl ring composed of one to two nitrogen atoms and carbon atoms, for A of General Formula (I), A¹ of General Formula (II), A² of General Formula (IV), and A³ of General Formula (V), may be quinoline, isoquinoline, benzimidazole, benzothiazole, pyridopyrazine, purine, imidazo[1,2-a]pyridine, or the like.

The 5-membered or 6-membered heteroaryl ring composed of one to three identical or different heteroatoms selected from an oxygen atom, a sulfur atom, and a nitrogen atom and carbon atoms as ring-constituting atoms for B of General Formula (I), B¹ of General Formula (II), B^{a} of General Formula (III), B² of General Formula (IV), and B³ of General Formula (V), may be pyridine, pyrazole, pyrrole, oxazole, or the like.

The heterocondensed ring composed of a 5-membered or 6-membered heteroaryl ring composed of one to three identical or different heteroatoms selected from an oxygen atom, a sulfur atom, and a nitrogen atom and carbon atoms as ring-constituting atoms and either a benzene ring or a 6-membered heteroaryl ring composed of one to two nitrogen atoms and carbon atoms, for B of General Formula (I), B¹ of General Formula (II), B^{a} of General Formula (III), B² of General Formula (IV), and B³ of General Formula (V), may be benzimidazole, benzoxazole, indazole, indole, pyridopyrazine, quinoline, quinazoline, benzothiazole, benzo[1,2,3]triazole, or the like.

The 4-membered to 6-membered heteroaryl ring composed of one to three identical or different heteroatoms selected from an oxygen atom, a sulfur atom, and a nitrogen atom and carbon atoms as ring-constituting atoms, which may have a substituent, the heteroaryl ring being a substituent that may be carried by the phenyl of B, the heteroaryl ring of B, and the heterocondensed ring of B in General Formula (I), may be oxazole, pyrazole, pyrazine, or the like.

Examples of the 4-membered to 6-membered heteroaryl ring composed of one to three identical or different heteroatoms selected from an oxygen atom, a sulfur atom, and a nitrogen atom and carbon atoms as ring-constituting atoms, which may have a substituent, in B¹ of General Formula (II), B^{a} of General Formula (III), B² of General Formula (IV), and B³ of General Formula (V), also include similar ones.

Furthermore, the number of substituents that may be carried by the phenyl, the 4-membered to 6-membered heteroaryl ring composed of one to three identical or different heteroatoms selected from an oxygen atom, a sulfur atom, and a nitrogen atom and carbon atoms as ring-constituting atoms, or the heterocondensed ring composed of the heteroaryl ring and either a benzene ring or a 6-membered heteroaryl ring composed of one to two nitrogen atoms and carbon atoms for A in General Formula (I), is preferably 1 to 5. Similarly, the rings for A¹ of General Formula (II), A² of General Formula (IV), or A³ of General Formula (V) may also have a similar number of substituents.

Furthermore, the number of substituents that may be carried by the phenyl, the 5-membered or 6-membered heteroaryl ring composed of one to three identical or different heteroatoms selected from an oxygen atom, a sulfur atom, and a nitrogen atom and carbon atoms as ring-constituting atoms, or the heterocondensed ring composed of the heteroaryl ring and either a benzene ring or a 6-membered heteroaryl ring composed of one to two nitrogen atoms and carbon atoms for B in General Formula (I), is preferably 1 to 5. Similarly, the rings for B¹ of General Formula (II), B^{a} of General Formula (III), B² of General Formula (IV), or B³ of General Formula (V) may also have a similar number of substituents.
(1) Among the above-described compound represented by General Formula (I), a tautomer or a stereoisomer of the compound, a pharmaceutically acceptable salt of the compound, or a solvate of the compound, the tautomer, the stereoisomer, or the salt, preferred examples include the following compounds.
(2) The compound represented by General Formula (I), a tautomer or a stereoisomer of the compound, a pharmaceutically acceptable salt of the compound, or a solvate of the compound, the tautomer, the stereoisomer, or the salt, wherein A represents a phenyl which may have a substituent.
(3) The compound represented by General Formula (I), a tautomer or a stereoisomer of the compound, a pharmaceutically acceptable salt of the compound, or a solvate of the compound, the tautomer, the stereoisomer, or the salt, wherein A represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent.
(4) The compound described in the above item (3), a tautomer or a stereoisomer of the compound, a pharmaceutically acceptable salt of the compound, or a solvate of the compound, the tautomer, the stereoisomer, or the salt, wherein the heteroaryl ring of A is thiophene, oxazole, thiazole, pyridine, pyrazine, pyrimidine, or pyridazine.
(5) The compound represented by General Formula (I), a tautomer or a stereoisomer of the compound, a pharmaceutically acceptable salt of the compound, or a solvate of the compound, the tautomer, the stereoisomer, or the salt, wherein A represents an optionally substituted heterocondensed ring of a 4-membered to 6-membered heteroaryl ring composed of one to three identical or different heteroatoms selected from an oxygen atom, a sulfur atom, and a nitrogen atom and carbon atoms as ring-constituting atoms, and a benzene ring.
(6) The compound described in the above item (5), a tautomer or a stereoisomer of the compound, a pharmaceutically acceptable salt of the compound, or a solvate of the compound, the tautomer, the stereoisomer, or the salt, wherein the heterocondensed ring of A is quinoline or benzo[d]thiazole.
(7) The compound represented by General Formula (I) or the compound described in any one of the above items (2) to (6), a tautomer or a stereoisomer of the compound, a pharmaceutically acceptable salt of the compound, or a solvate of the compound, the tautomer, the stereoisomer, or the salt, wherein B represents a phenyl which may have a substituent.
(8) The compound represented by General Formula (I) or the compound described in any one of the above items (2) to (6), a tautomer or a stereoisomer of the compound, a pharmaceutically acceptable salt of the compound, or a solvate of the compound, the tautomer, the stereoisomer, or the salt, wherein B represents a 5-membered or 6-membered heteroaryl ring composed of one to three identical or different heteroatoms selected from an oxygen atom, a sulfur atom, and a nitrogen atom and carbon atoms as ring-constituting atoms, which may have a substituent.
(9) The compound described in the above item (8), a tautomer or a stereoisomer of the compound, a pharmaceutically acceptable salt of the compound, or a solvate of the compound, the tautomer, the stereoisomer, or the salt, wherein the heteroaryl ring of B is pyridine.
(10) The compound represented by General Formula (I) or the compound described in any one of the above items (2) to (6), a tautomer or a stereoisomer of the compound, a pharmaceutically acceptable salt of the compound, or a solvate of the compound, the tautomer, the stereoisomer, or the salt, wherein B represents an optionally substituted heterocondensed ring of a 5-membered or 6-membered heteroaryl ring composed of one to three identical or different heteroatoms selected from an oxygen atom, a sulfur atom, and a nitrogen atom and carbon atoms as ring-constituting atoms, and a benzene ring.
(11) The compound described in the above item (10), a tautomer or a stereoisomer of the compound, a pharmaceutically acceptable salt of the compound, or a solvate of the compound, the tautomer, the stereoisomer, or the salt, wherein the heterocondensed ring of B is indole, benzimidazole, indazole, benzoxazole, benzothiazole, benzo[d][1,2,3]triazole, or quinoline.
(12) The compound represented by General Formula (I) or the compound described in any one of the above items (2) to (11), a tautomer or a stereoisomer of the compound, a pharmaceutically acceptable salt of the compound, or a solvate of the compound, the tautomer, the stereoisomer, or the salt, wherein n is 1, and m is 0.
(13) The compound represented by General Formula (I) or the compound described in any one of the above items (2) to (12), a tautomer or a stereoisomer of the compound, a pharmaceutically acceptable salt of the compound, or a solvate of the compound, the tautomer, the stereoisomer, or the salt, wherein p is 1.
(14) The compound represented by General Formula (I) or the compound described in any one of the above items (2) to (13), a tautomer or a stereoisomer of the compound, a pharmaceutically acceptable salt of the compound, or a solvate of the compound, the tautomer, the stereoisomer, or the salt, wherein R¹ and R² each represent a hydrogen atom.
(15) The compound represented by General Formula (I) or the compound described in any one of the above items (2) to (14), a tautomer or a stereoisomer of the compound, a pharmaceutically acceptable salt of the compound, or a solvate of the compound, the tautomer, the stereoisomer, or the salt, wherein R³ represents a hydrogen atom.
(16) Among the above-described compound represented by General Formula (II), a tautomer or a stereoisomer of the compound, a pharmaceutically acceptable salt of the compound, or a solvate of the compound, the tautomer, the stereoisomer, or the salt, preferred examples include the following compounds.
(17) The compound represented by General Formula (II), a tautomer or a stereoisomer of the compound, a pharmaceutically acceptable salt of the compound, or a solvate of the compound, the tautomer, the stereoisomer, or the salt, wherein A¹ represents a phenyl which may have a substituent.
(18) The compound represented by General Formula (II), a tautomer or a stereoisomer of the compound, a pharmaceutically acceptable salt of the compound, or a solvate of the compound, the tautomer, the stereoisomer, or the salt, wherein A¹ represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent.
(19) The compound described in the above item (18), a tautomer or a stereoisomer of the compound, a pharmaceutically acceptable salt of the compound, or a solvate of the compound, the tautomer, the stereoisomer, or the salt, wherein the heteroaryl ring of A¹ is thiophene, oxazole, thiazole, pyridine, pyrazine, pyrimidine, or pyridazine.
(20) The compound described in the above item (18), a tautomer or a stereoisomer of the compound, a pharmaceutically acceptable salt of the compound, or a solvate of the compound, the tautomer, the stereoisomer, or the salt, wherein the heteroaryl ring of A¹ is pyridine.
(21) The compound represented by General Formula (II), a tautomer or a stereoisomer of the compound, a pharmaceutically acceptable salt of the compound, or a solvate of the compound, the tautomer, the stereoisomer, or the salt, wherein A¹ represents an optionally substituted heterocondensed ring of a 4-membered to 6-membered heteroaryl ring composed of one to three identical or different heteroatoms selected from an oxygen atom, a sulfur atom, and a nitrogen atom and carbon atoms as ring-constituting atoms, and a benzene ring.
(22) The compound described in the above item (21), a tautomer or a stereoisomer of the compound, a pharmaceutically acceptable salt of the compound, or a solvate of the compound, the tautomer, the stereoisomer, or the salt, wherein the heterocondensed ring of A¹ is quinoline or benzo[d]thiazole.
(23) The compound represented by General Formula (II) or the compound described in any one of the above items (17) to (22), a tautomer or a stereoisomer of the compound, a pharmaceutically acceptable salt of the compound, or a solvate of the compound, the tautomer, the stereoisomer, or the salt, wherein B¹ represents a phenyl which may have a substituent.
(24) The compound represented by General Formula (II) or the compound described in any one of the above items (17) to (22), a tautomer or a stereoisomer of the compound, a pharmaceutically acceptable salt of the compound, or a solvate of the compound, the tautomer, the stereoisomer, or the salt, wherein B¹ represents a 5-membered or 6-membered heteroaryl ring composed of one to three identical or different heteroatoms selected from an oxygen atom, a sulfur atom, and a nitrogen atom and carbon atoms as ring-constituting atoms, which may have a substituent.
(25) The compound described in the above item (24), a tautomer or a stereoisomer of the compound, a pharmaceutically acceptable salt of the compound, or a solvate of the compound, the tautomer, the stereoisomer, or the salt, wherein the heteroaryl ring of B¹ is pyridine.
(26) The compound represented by General Formula (II) or the compound described in any one of the above items (17) to (22), a tautomer or a stereoisomer of the compound, a pharmaceutically acceptable salt of the compound, or a solvate of the compound, the tautomer, the stereoisomer, or the salt, wherein B¹ represents an optionally substituted heterocondensed ring of a 5-membered or 6-membered heteroaryl ring composed of one to three identical or different heteroatoms selected from an oxygen atom, a sulfur atom, and a nitrogen atom and carbon atoms as ring-constituting atoms, and a benzene ring.
(27) The compound described in the above item (26), a tautomer or a stereoisomer of the compound, a pharmaceutically acceptable salt of the compound, or a solvate of the compound, the tautomer, the stereoisomer, or the salt, wherein the heterocondensed ring of B¹ is indole, benzimidazole, indazole, benzoxazole, benzothiazole, benzo[d][1,2,3]triazole, or quinoline.
(28) The compound described in the above item (26), a tautomer or a stereoisomer of the compound, a pharmaceutically acceptable salt of the compound, or a solvate of the compound, the tautomer, the stereoisomer, or the salt, wherein the heterocondensed ring of B¹ is benzo[d]oxazole.
(29) Among the above-described compound represented by General Formula (III), a tautomer or a stereoisomer of the compound, a pharmaceutically acceptable salt of the compound, or a solvate of the compound, the tautomer, the stereoisomer, or the salt, preferred examples include the following compounds.
(30) The compound represented by General Formula (III), a tautomer or a stereoisomer of the compound, a pharmaceutically acceptable salt of the compound, or a solvate of the compound, the tautomer, the stereoisomer, or the salt, wherein D and J both represent CH.
(31) The compound represented by General Formula (III), a tautomer or a stereoisomer of the compound, a pharmaceutically acceptable salt of the compound, or a solvate of the compound, the tautomer, the stereoisomer, or the salt, wherein any one of D, E, F, G, and J represents N, and the others each represent CR.
(32) The compound represented by General Formula (III), a tautomer or a stereoisomer of the compound, a pharmaceutically acceptable salt of the compound, or a solvate of the compound, the tautomer, the stereoisomer, or the salt, wherein D, E, F, and J, which may be identical or different, each represent CR, and G represents N.
(33) The compound represented by General Formula (III) or the compound described in any one of the above items (30) to (32), a tautomer or a stereoisomer of the compound, a pharmaceutically acceptable salt of the compound, or a solvate of the compound, the tautomer, the stereoisomer, or the salt, wherein B^{a} represents a phenyl which may have a substituent.
(34) The compound represented by General Formula (III) or the compound described in any one of the above items (30) to (32), a tautomer or a stereoisomer of the compound, a pharmaceutically acceptable salt of the compound, or a solvate of the compound, the tautomer, the stereoisomer, or the salt, wherein B^{a} represents a 5-membered or 6-membered heteroaryl ring composed of one to three identical or different heteroatoms selected from an oxygen atom, a sulfur atom, and a nitrogen atom and carbon atoms as ring-constituting atoms, which may have a substituent.
(35) The compound described in the above item (34), a tautomer or a stereoisomer of the compound, a pharmaceutically acceptable salt of the compound, or a solvate of the compound, the tautomer, the stereoisomer, or the salt, wherein the heteroaryl ring of B^{a} is pyridine.
(36) The compound represented by General Formula (III) or the compound described in any one of the above items (30) to (32), a tautomer or a stereoisomer of the compound, a pharmaceutically acceptable salt of the compound, or a solvate of the compound, the tautomer, the stereoisomer, or the salt, wherein B^{a} represents an optionally substituted heterocondensed ring of a 5-membered or 6-membered heteroaryl ring composed of one to three identical or different heteroatoms selected from an oxygen atom, a sulfur atom, and a nitrogen atom and carbon atoms as ring-constituting atoms, and a benzene ring.
(37) The compound described in the above item (36), a tautomer or a stereoisomer of the compound, a pharmaceutically acceptable salt of the compound, or a solvate of the compound, the tautomer, the stereoisomer, or the salt, wherein the heterocondensed ring of B^{a} is indole, benzimidazole, indazole, benzoxazole, benzothiazole, benzo[d][1,2,3]triazole, or quinoline.
(38) The compound described in the above item (36), a tautomer or a stereoisomer of the compound, a pharmaceutically acceptable salt of the compound, or a solvate of the compound, the tautomer, the stereoisomer, or the salt, wherein the heterocondensed ring of B^{a} is benzoxazole.
(39) The compound represented by General Formula (III) or the compound described in any one of the above items (30) to (38), a tautomer or a stereoisomer of the compound, a pharmaceutically acceptable salt of the compound, or a solvate of the compound, the tautomer, the stereoisomer, or the salt, wherein s is 1.
(40) The compound represented by General Formula (III) or the compound described in any one of the above items (30) to (39), a tautomer or a stereoisomer of the compound, a pharmaceutically acceptable salt of the compound, or a solvate of the compound, the tautomer, the stereoisomer, or the salt, wherein t is 1.
(41) The compound represented by General Formula (III) or the compound described in any one of the above items (30) to (40), a tautomer or a stereoisomer of the compound, a pharmaceutically acceptable salt of the compound, or a solvate of the compound, the tautomer, the stereoisomer, or the salt, wherein R^{a1}, R^{a2}, R^{b1}, and R^{b2} each represent a hydrogen atom.
(42) The compound represented by General Formula (III) or the compound described in any one of the above items (30) to (41), a tautomer or a stereoisomer of the compound, a pharmaceutically acceptable salt of the compound, or a solvate of the compound, the tautomer, the stereoisomer, or the salt, wherein X represents the following: 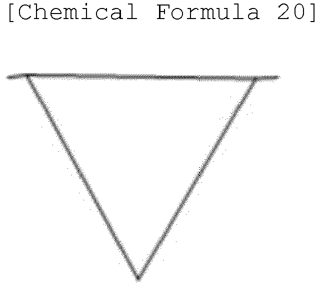
(43) The compound represented by General Formula (III) or the compound described in any one of the above items (30) to (41), a tautomer or a stereoisomer of the compound, a pharmaceutically acceptable salt of the compound, or a solvate of the compound, the tautomer, the stereoisomer, or the salt, wherein X represents the following: 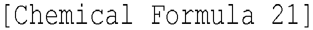 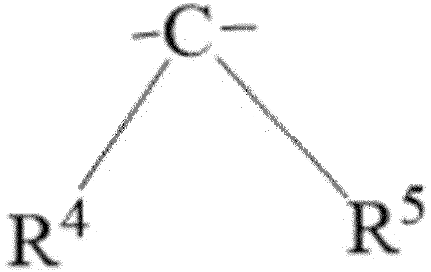
(44) The compound described in the above item (43), a tautomer or a stereoisomer of the compound, a pharmaceutically acceptable salt of the compound, or a solvate of the compound, the tautomer, the stereoisomer, or the salt, wherein the R⁴ and R⁵ each represent a hydrogen atom.
(45) Among the above-described compound represented by General Formula (IV), a tautomer or a stereoisomer of the compound, a pharmaceutically acceptable salt of the compound, or a solvate of the compound, the tautomer, the stereoisomer, or the salt, preferred examples include the following compounds.
(46) The compound represented by General Formula (IV), a tautomer or a stereoisomer of the compound, a pharmaceutically acceptable salt of the compound, or a solvate of the compound, the tautomer, the stereoisomer, or the salt, wherein A² represents a phenyl which may have a substituent.
(47) The compound represented by General Formula (IV), a tautomer or a stereoisomer of the compound, a pharmaceutically acceptable salt of the compound, or a solvate of the compound, the tautomer, the stereoisomer, or the salt, wherein A² represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent.
(48) The compound described in the above item (47), a tautomer or a stereoisomer of the compound, a pharmaceutically acceptable salt of the compound, or a solvate of the compound, the tautomer, the stereoisomer, or the salt, wherein the heteroaryl ring of A² is thiophene, oxazole, thiazole, pyridine, pyrazine, pyrimidine, or pyridazine.
(49) The compound represented by General Formula (IV), a tautomer or a stereoisomer of the compound, a pharmaceutically acceptable salt of the compound, or a solvate of the compound, the tautomer, the stereoisomer, or the salt, wherein A² represents an optionally substituted heterocondensed ring of a 4-membered to 6-membered heteroaryl ring composed of one to three identical or different heteroatoms selected from an oxygen atom, a sulfur atom, and a nitrogen atom and carbon atoms as ring-constituting atoms, and a benzene ring.
(50) The compound described in the above item (49), a tautomer or a stereoisomer of the compound, a pharmaceutically acceptable salt of the compound, or a solvate of the compound, the tautomer, the stereoisomer, or the salt, wherein the heterocondensed ring of A² is quinoline or benzo[d]thiazole.
(51) The compound represented by General Formula (IV) or the compound described in any one of the above items (46) to (50), a tautomer or a stereoisomer of the compound, a pharmaceutically acceptable salt of the compound, or a solvate of the compound, the tautomer, the stereoisomer, or the salt, wherein B² represents a phenyl which may have a substituent.
(52) The compound represented by General Formula (IV) or the compound described in any one of the above items (46) to (50), a tautomer or a stereoisomer of the compound, a pharmaceutically acceptable salt of the compound, or a solvate of the compound, the tautomer, the stereoisomer, or the salt, wherein B² represents a 5-membered or 6-membered heteroaryl ring composed of one to three identical or different heteroatoms selected from an oxygen atom, a sulfur atom, and a nitrogen atom and carbon atoms as ring-constituting atoms, which may have a substituent.
(53) The compound described in the above item (52), a tautomer or a stereoisomer of the compound, a pharmaceutically acceptable salt of the compound, or a solvate of the compound, the tautomer, the stereoisomer, or the salt, wherein the heteroaryl ring of B² is pyridine.
(54) The compound represented by General Formula (IV) or the compound described in any one of the above items (46) to (50), a tautomer or a stereoisomer of the compound, a pharmaceutically acceptable salt of the compound, or a solvate of the compound, the tautomer, the stereoisomer, or the salt, wherein B² represents an optionally substituted heterocondensed ring of a 5-membered or 6-membered heteroaryl ring composed of one to three identical or different heteroatoms selected from an oxygen atom, a sulfur atom, and a nitrogen atom and carbon atoms as ring-constituting atoms, and a benzene ring.
(55) The compound described in the above item (54), a tautomer or a stereoisomer of the compound, a pharmaceutically acceptable salt of the compound, or a solvate of the compound, the tautomer, the stereoisomer, or the salt, wherein the heterocondensed ring of B² is indole, benzimidazole, indazole, benzoxazole, benzothiazole, benzo[d][1,2,3]triazole, or quinoline.
(56) The compound represented by General Formula (IV) or the compound described in any one of the above items (46) to (55), a tautomer or a stereoisomer of the compound, a pharmaceutically acceptable salt of the compound, or a solvate of the compound, the tautomer, the stereoisomer, or the salt, wherein no is 1, and mo is 0.
(57) The compound represented by General Formula (IV) or the compound described in any one of the above items (46) to (56), a tautomer or a stereoisomer of the compound, a pharmaceutically acceptable salt of the compound, or a solvate of the compound, the tautomer, the stereoisomer, or the salt, wherein po is 1.
(58) The compound represented by General Formula (IV) or the compound described in any one of the above items (46) to (57), a tautomer or a stereoisomer of the compound, a pharmaceutically acceptable salt of the compound, or a solvate of the compound, the tautomer, the stereoisomer, or the salt, wherein R⁰¹ and R⁰² each represent a hydrogen atom.
(59) The compound represented by General Formula (IV) or the compound described in any one of the above items (46) to (58), a tautomer or a stereoisomer of the compound, a pharmaceutically acceptable salt of the compound, or a solvate of the compound, the tautomer, the stereoisomer, or the salt, wherein R⁰³ represents a hydrogen atom.
(60) The compound represented by General Formula (IV) or the compound described in any one of the above items (46) to (59), a tautomer or a stereoisomer of the compound, a pharmaceutically acceptable salt of the compound, or a solvate of the compound, the tautomer, the stereoisomer, or the salt, wherein R⁰⁴ and R⁰⁵ each represent a hydrogen atom.
(61) Among the above-described compound represented by General Formula (V), a tautomer or a stereoisomer of the compound, a pharmaceutically acceptable salt of the compound, or a solvate of the compound, the tautomer, the stereoisomer, or the salt, preferred examples include the following compounds.
(62) The compound represented by General Formula (V), a tautomer or a stereoisomer of the compound, a pharmaceutically acceptable salt of the compound, or a solvate of the compound, the tautomer, the stereoisomer, or the salt, wherein A³ represents a phenyl which may have a substituent.
(63) The compound represented by General Formula (V), a tautomer or a stereoisomer of the compound, a pharmaceutically acceptable salt of the compound, or a solvate of the compound, the tautomer, the stereoisomer, or the salt, wherein A³ represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent.
(64) The compound described in the above item (63), a tautomer or a stereoisomer of the compound, a pharmaceutically acceptable salt of the compound, or a solvate of the compound, the tautomer, the stereoisomer, or the salt, wherein the heteroaryl ring of A³ is thiophene, oxazole, thiazole, pyridine, pyrazine, pyrimidine, or pyridazine.
(65) The compound described in the above item (63), a tautomer or a stereoisomer of the compound, a pharmaceutically acceptable salt of the compound, or a solvate of the compound, the tautomer, the stereoisomer, or the salt, wherein the heteroaryl ring of A³ is pyridine.
(66) The compound represented by General Formula (V), a tautomer or a stereoisomer of the compound, a pharmaceutically acceptable salt of the compound, or a solvate of the compound, the tautomer, the stereoisomer, or the salt, wherein A³ represents an optionally substituted heterocondensed ring of a 4-membered to 6-membered heteroaryl ring composed of one to three identical or different heteroatoms selected from an oxygen atom, a sulfur atom, and a nitrogen atom and carbon atoms as ring-constituting atoms, and a benzene ring.
(67) The compound described in the above item (66), a tautomer or a stereoisomer of the compound, a pharmaceutically acceptable salt of the compound, or a solvate of the compound, the tautomer, the stereoisomer, or the salt, wherein the heterocondensed ring of A³ is quinoline or benzo[d]thiazole.
(68) The compound represented by General Formula (V) or the compound described in any one of the above items (62) to (67), a tautomer or a stereoisomer of the compound, a pharmaceutically acceptable salt of the compound, or a solvate of the compound, the tautomer, the stereoisomer, or the salt, wherein B³ represents a phenyl which may have a substituent.
(69) The compound represented by General Formula (V) or the compound described in any one of the above items (62) to (67), a tautomer or a stereoisomer of the compound, a pharmaceutically acceptable salt of the compound, or a solvate of the compound, the tautomer, the stereoisomer, or the salt, wherein B³ represents a 5-membered or 6-membered heteroaryl ring composed of one to three identical or different heteroatoms selected from an oxygen atom, a sulfur atom, and a nitrogen atom and carbon atoms as ring-constituting atoms, which may have a substituent.
(70) The compound described in the above item (69), a tautomer or a stereoisomer of the compound, a pharmaceutically acceptable salt of the compound, or a solvate of the compound, the tautomer, the stereoisomer, or the salt, wherein the heteroaryl ring of B³ is pyridine.
(71) The compound represented by General Formula (V) or the compound described in any one of the above items (62) to (67), a tautomer or a stereoisomer of the compound, a pharmaceutically acceptable salt of the compound, or a solvate of the compound, the tautomer, the stereoisomer, or the salt, wherein B³ represents an optionally substituted heterocondensed ring of a 5-membered or 6-membered heteroaryl ring composed of one to three identical or different heteroatoms selected from an oxygen atom, a sulfur atom, and a nitrogen atom and carbon atoms as ring-constituting atoms, and a benzene ring.
(72) The compound described in the above item (71), a tautomer or a stereoisomer of the compound, a pharmaceutically acceptable salt of the compound, or a solvate of the compound, the tautomer, the stereoisomer, or the salt, wherein the heterocondensed ring of B³ is indole, benzimidazole, indazole, benzoxazole, benzothiazole, benzo[d][1,2,3]triazole, or quinoline.
(73) The compound represented by General Formula (V) or the compound described in any one of the above items (62) to (72), a tautomer or a stereoisomer of the compound, a pharmaceutically acceptable salt of the compound, or a solvate of the compound, the tautomer, the stereoisomer, or the salt, wherein R³⁴ and R³⁵ both represent a hydrogen atom.
(74) Among the above-described compound represented by General Formula (VI), a tautomer or a stereoisomer of the compound, a pharmaceutically acceptable salt of the compound, or a solvate of the compound, the tautomer, the stereoisomer, or the salt, preferred examples include the following compounds.
(75) The compound represented by General Formula (VI), a tautomer or a stereoisomer of the compound, a pharmaceutically acceptable salt of the compound, or a solvate of the compound, the tautomer, the stereoisomer, or the salt, wherein R^{d1}, R^{d2}, and R^{d3} each differently represent a hydrogen atom, a halogen atom, a C₁₋₈ alkyl group, a C₁₋₈ alkyl group substituted with one to three halogen atoms, or a C₁₋₈ alkoxy group.
(76) The compound represented by General Formula (VI) or the compound described in the above item (75), a tautomer or a stereoisomer of the compound, a pharmaceutically acceptable salt of the compound, or a solvate of the compound, the tautomer, the stereoisomer, or the salt, wherein R^{d4} represents trifluoromethyl or cyclopropyl.
(77) The compound represented by General Formula (VI) or the compound described in the above item (75) or (76), a tautomer or a stereoisomer of the compound, a pharmaceutically acceptable salt of the compound, or a solvate of the compound, the tautomer, the stereoisomer, or the salt, wherein r is 1.
(78) Moreover, the compound of the present invention is preferably any of the following compounds, a tautomer or a stereoisomer of the compound, a pharmaceutically acceptable salt of the compound, or a solvate of the compound, the tautomer, the stereoisomer, or the salt.

A compound selected from the following:
(R)-2-(4-cyclopropylphenyl)-N-(1-(5-(trifluoromethyl)pyridin-3-yl)pyrrolidin-3-yl)acetamide,
(R)-2-(5-(trifluoromethyl)pyridin-2-yl)-N-(1-(5-(trifluoromethyl)pyridin-3-yl)pyrrolidin-3-yl)acetamide,
(R)-2-(4-isopropylphenyl)-N-(1-(2-(trifluoromethyl)pyrimidin-5-yl)pyrrolidin-3-yl)acetamide,
(R)-2-(4-isopropylphenyl)-N-(1-(5-(trifluoromethyl)pyridin-3-yl)pyrrolidin-3-yl)acetamide,
2-(4-cyclopropylphenyl)-N-((3S,4S)-4-hydroxy-1-(5-(trifluoromethyl)pyridin-3-yl)pyrrolidin-3-yl)acetamide,
(R)-2-(4-cyclopropylphenyl)-N-(5-(5-(trifluoromethyl)pyridin-3-yl)-5-azaspiro[2.4]heptan-7-yl)acetamide,
(R)-2-(4-cyclopropylphenyl)-N-(5-(6-(trifluoromethyl)pyridin-3-yl)-5-azaspiro[2.4]heptan-7-yl)acetamide,
(R)-2-(4-cyclopropylphenyl)-N-(5-(4-(trifluoromethyl)thiazol-2-yl)-5-azaspiro[2.4]heptan-7-yl)acetamide,
2-(4-cyclopropylphenyl)-N-((3R,5S)-5-methyl-1-(5-(trifluoromethyl)pyridin-3-yl)pyrrolidin-3-yl)acetamide,
2-(4-cyclopropylphenyl)-N-((1S,5R)-3-(5-(trifluoromethyl)pyridin-3-yl)-3-azabicyclo[3.1.0]hexan-1-yl)acetamide,
2-(4-cyclopropylphenyl)-N-((1R,5S)-3-(5-(trifluoromethyl)pyridin-3-yl)-3-azabicyclo[3.1.0]hexan-1-yl)acetamide,
(R)-2-(4-(trifluoromethyl)phenyl)-N-(1-(6-(trifluoromethyl)pyrazin-2-yl)pyrrolidin-3-yl)acetamide,
(R)-N-(1-(6-cyano-5-(trifluoromethyl)pyridin-3-yl)pyrrolidin-3-yl)-2-(4-cyclopropylphenyl)acetamide,
methyl (R)-3-(2-(4-cyclopropylphenyl)acetamido)-1-(5-(trifluoromethyl)pyridin-3-yl)pyrrolidine-3-carboxylate,
methyl (S)-3-(2-(4-cyclopropylphenyl)acetamido)-1-(5-(trifluoromethyl)pyridin-3-yl)pyrrolidine-3-carboxylate,
(R)-3-(2-(4-cyclopropylphenyl)acetamido)-1-(5-(trifluoromethyl)pyridin-3-yl)pyrrolidine-3-carboxylic acid,
(S)-3-(2-(4-cyclopropylphenyl)acetamido)-1-(5-(trifluoromethyl)pyridin-3-yl)pyrrolidine-3-carboxylic acid,
(R)-2-(1H-indazol-6-yl)-N-(1-(5-(trifluoromethyl)pyridin-3-yl)pyrrolidin-3-yl)acetamide,
(R)-2-(4-cyclopropylphenyl)-N-(1-(4-(trifluoromethyl)thiazol-2-yl)pyrrolidin-3-yl)acetamide,
(R)-2-(4-isobutylphenyl)-N-((R)-1-(5-(trifluoromethyl)pyridin-3-yl)pyrrolidin-3-yl)propanamide,
(S)-2-(4-isobutylphenyl)-N-((R)-1-(5-(trifluoromethyl)pyridin-3-yl)pyrrolidin-3-yl)propanamide,
(R)-2-(4-cyclopropylphenyl)-N-(1-(6-methyl-5-(trifluoromethyl)pyridin-3-yl)pyrrolidin-3-yl)acetamide,
(R)-2-(4-cyclopropylphenyl)-N-(1-(5-(2-hydroxypropan-2-yl)pyridin-3-yl)pyrrolidin-3-yl)acetamide,
(R)-2-(4-cyclopropylphenyl)-N-(1-(8-(trifluoromethyl)imidazo[1,2-a]pyridin-6-yl)pyrrolidin-3-yl)acetamide,
(R)-3-(2-(4-cyclopropylphenyl)acetamido)-1-(5-(trifluoromethyl)pyridin-3-yl)pyrrolidine-3-carboxyamide,
(S)-3-(2-(4-cyclopropylphenyl)acetamido)-1-(5-(trifluoromethyl)pyridin-3-yl) pyrrolidine-3-carboxyamide,
(R)-2-(4-hydroxyphenyl)-N-(1-(5-(trifluoromethyl)pyridin-3-yl)pyrrolidin-3-yl)acetamide,
(R)-2-(4-(1,1-dioxide thiomorpholino)phenyl)-N-(1-(5-(trifluoromethyl)pyridin-3-yl)pyrrolidin-3-yl)acetamide,
(R)-1-(4-chlorophenyl)-N-(1-(5-(trifluoromethyl)pyridin-3-yl)pyrrolidin-3-yl)cyclopropane-1-carboxyamide,
(R)-2-(4-bromophenyl)-2-hydroxy-N-((R)-1-(5-(trifluoromethyl)pyridin-3-yl)pyrrolidin-3-yl)acetamide,
(S)-2-(4-bromophenyl)-2-hydroxy-N-((R)-1-(5-(trifluoromethyl)pyridin-3-yl)pyrrolidin-3-yl)acetamide,
(R)-N-(1-(5-(trifluoromethyl)pyridin-3-yl)pyrrolidin-3-yl)-2-(4-(trimethylsilyl)phenyl)acetamide,
(R)-2-(4-(2-hydroxy-2-methylpropoxy)phenyl)-N-(1-(5-(trifluoromethyl)pyridin-3-yl)pyrrolidin-3-yl)acetamide,
2-(4-cyclopropylphenyl)-N-((3S,4S)-4-fluoro-1-(5-(trifluoromethyl)pyridin-3-yl)pyrrolidin-3-yl)acetamide,
(R)-2-(4-(dimethylamino)phenyl)-N-(1-(5-(trifluoromethyl)pyridin-3-yl)pyrrolidin-3-yl)acetamide,
(R)-2-(4-nitrophenyl)-N-(1-(5-(trifluoromethyl)pyridin-3-yl)pyrrolidin-3-yl)acetamide,
(S)-2-(4-isobutylphenyl)-N-((R)-1-(5-(trifluoromethyl)pyridin-3-yl)pyrrolidin-3-yl)propanamide,
(R)-2-(3-fluoro-4-(trifluoromethyl)phenyl)-N-(1-(5-(trifluoromethyl)pyridin-3-yl)pyrrolidin-3-yl)acetamide,
(R)-2-(4-aminophenyl)-N-(1-(5-(trifluoromethyl)pyridin-3-yl)pyrrolidin-3-yl)acetamide,
(R)-1-(4-(trifluoromethyl)phenyl)-N-(1-(5-(trifluoromethyl)pyridin-3-yl)pyrrolidin-3-yl)cyclopropane-1-carboxyamide,
(R)-2-(4-acetamidophenyl)-N-(1-(5-(trifluoromethyl)pyridin-3-yl)pyrrolidin-3-yl)acetamide,
(R)-N-(3-cyano-1-(5-(trifluoromethyl)pyridin-3-yl)pyrrolidin-3-yl)-2-(4-cyclopropylphenyl)acetamide,
(S)-N-(3-cyano-1-(5-(trifluoromethyl)pyridin-3-yl)pyrrolidin-3-yl)-2-(4-cyclopropylphenyl)acetamide,
methyl (R)-3-(2-(1H-indol-6-yl)acetamido)-1-(5-(trifluoromethyl)pyridin-3-yl) pyrrolidine-3-carboxylate,
methyl (S)-3-(2-(1H-indol-6-yl)acetamido)-1-(5-(trifluoromethyl)pyridin-3-yl) pyrrolidine-3-carboxylate,
methyl (R)-3-(2-(4-(trifluoromethyl)phenyl)acetamido)-1-(5-(trifluoromethyl)pyridin-3-yl) pyrrolidine-3-carboxylate,
methyl (S)-3-(2-(4-(trifluoromethyl)phenyl)acetamido)-1-(5-(trifluoromethyl)pyridin-3-yl) pyrrolidine-3-carboxylate,
(1S,2S)-2-(benzo[d]oxazol-2-yl)-N-((R)-1-(5-(trifluoromethyl)pyridin-3-yl)pyrrolidin-3-yl)cyclopropane-1-carboxyamide,
(R)-2-(4-morpholinophenyl)-N-(1-(4-(trifluoromethyl)thiazol-2-yl)pyrrolidin-3-yl) acetamide,
methyl 3-(2-(4-morpholinophenyl)acetamido)-1-(5-(trifluoromethyl)pyridin-3-yl) pyrrolidine-3-carboxylate,
(R)-2-(4-morpholinophenyl)-N-(1-(6-(trifluoromethyl)pyridin-3-yl)pyrrolidin-3-yl)acetamide,
(R)-2-(4-cyclopropylphenyl)-N-(3-ethyl-1-(5-(trifluoromethyl)pyridin-3-yl)pyrrolidin-3-yl)acetamide,
(S)-2-(4-cyclopropylphenyl)-N-(3-ethyl-1-(5-(trifluoromethyl)pyridin-3-yl)pyrrolidin-3-yl)acetamide,
(1S,2S)-2-(6-fluorobenzo[d]oxazol-2-yl)-N-((R)-1-(5-(trifluoromethyl)pyridin-3-yl)pyrrolidin-3-yl)cyclopropane-1-carboxyamide,
(1S,2S)-2-(5,6-difluorobenzo[d]oxazol-2-yl)-N-((R)-1-(5-(trifluoromethyl)pyridin-3-yl)pyrrolidin-3-yl)cyclopropane-1-carboxyamide,
(R)-2-(4-(2-oxa-6-azaspiro[3.3]heptan-6-yl)phenyl)-N-(1-(5-(trifluoromethyl)pyridin-3-yl)pyrrolidin-3-yl)acetamide,
(1S,2S)-2-(benzo[d]oxazol-2-yl)-N-((R)-1-(4-(trifluoromethyl)thiazol-2-yl)pyrrolidin-3-yl) cyclopropane-1-carboxyamide,
(1S,2S)-2-(6-fluorobenzo[d]oxazol-2-yl)-N-((R)-1-(6-(trifluoromethyl)pyridin-3-yl)pyrrolidin-3-yl)cyclopropane-1-carboxyamide,
(1S,2S)-2-(5-fluorobenzo[d]oxazol-2-yl)-N-((R)-1-(5-(trifluoromethyl)pyridin-3-yl)pyrrolidin-3-yl)cyclopropane-1-carboxyamide,
(1R,2R)-2-(benzo[d]oxazol-2-yl)-N-((R)-1-(5-(trifluoromethyl)pyridin-3-yl)pyrrolidin-3-yl)cyclopropane-1-carboxyamide,
(1S,2S)-2-(benzo[d]oxazol-2-yl)-N-((R)-1-(5-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(4-(trifluoromethyl)phenyl)pyrrolidin-3-yl)cyclopropane-1-carboxyamide,
(1R,2R)-2-(1H-benzo[d] imidazol-2-yl)-N-((R)-1-(4-(trifluoromethyl)phenyl)pyrrolidin-3-yl)cyclopropane-1-carboxyamide,
(1S,2S)-2-(1H-benzo[d]imidazol-2-yl)-N-((S)-1-(4-(trifluoromethyl)phenyl)pyrrolidin-3-yl)cyclopropane-1-carboxyamide,
(1R,2R)-2-(1H-benzo[d]imidazol-2-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((S)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(5-(trifluoromethyl)pyrimidin-2-yl)pyrrolidin-3-yl)cyclopropane-1-carboxyamide,
(1S,2S)-2-(1H-benzo[d]imidazol-2-yl)-N-((R)-1-(4-fluoro-3-(trifluoromethyl)phenyl)pyrrolidin-3-yl)cyclopropane-1-carboxyamide,
(1S,2S)-N-((R)-1-(4-fluoro-3-(trifluoromethyl)phenyl)pyrrolidin-3-yl)-2-(quinazolin-2-yl)cyclopropane-1-carboxyamide,
(1S,2S)-2-(1H-benzo[d]imidazol-2-yl)-N-((R)-1-(3-(methylsulfonyl)phenyl)pyrrolidin-3-yl)cyclopropane-1-carboxyamide,
(1S,2S)-2-(1H-benzo[d] imidazol-2-yl)-N-((R)-1-(3-cyanophenyl)pyrrolidin-3-yl)cyclopropane-1-carboxyamide,
(1S,2S)-2-(5-cyano-1H-benzo[d]imidazol-2-yl)-N-((R)-1-(6-(trifluoromethyl)pyridin-3-yl)pyrrolidin-3-yl)cyclopropane-1-carboxyamide,
(1R,2R)-2-(5-cyano-1H-benzo[d]imidazol-2-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(2-(trifluoromethyl)pyridin-4-yl)pyrrolidin-3-yl)cyclopropane-1-carboxyamide,
(1S,2S)-2-(1H-benzo[d]imidazol-2-yl)-N-((R)-1-(6-(trifluoromethyl)pyridin-3-yl)piperidin-3-yl)cyclopropane-1-carboxyamide,
(1R,2R)-2-(1H-benzo[d]imidazol-2-yl)-N-((R)-1-(6-(trifluoromethyl)pyridin-3-yl)piperidin-3-yl)cyclopropane-1-carboxyamide,
(1S,2S)-2-(1H-benzo[d]imidazol-2-yl)-N-((R)-1-(4-(trifluoromethyl)thiazol-2-yl)pyrrolidin-3-yl)cyclopropane-1-carboxyamide,
(1S,2S)-2-(1H-benzo[d]imidazol-2-yl)-N-((R)-1-(4-(trifluoromethyl)phenyl)piperidin-3-yl)cyclopropane-1-carboxyamide,
(1S,2S)-2-(1H-indol-3-yl)-N-((R)-1-(6-(trifluoromethyl)pyridin-3-yl)pyrrolidin-3-yl)cyclopropane-1-carboxyamide,
(1R,2R)-2-(1H-indol-3-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(6-methoxy-5-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(4-(tert-butyl)thiazol-2-yl)pyrrolidin-3-yl)cyclopropane-1-carboxyamide,
(1S,2S)-2-(1H-benzo[d]imidazol-2-yl)-N-((R)-1-(6-(trifluoromethyl)pyridazin-3-yl)pyrrolidin-3-yl)cyclopropane-1-carboxyamide,
(1S,2S)-2-(1H-benzo[d]imidazol-2-yl)-N-((R)-1-(2-(trifluoromethyl)pyrimidin-5-yl)pyrrolidin-3-yl)cyclopropane-1-carboxyamide,
(1S,2S)-2-(1H-benzo[d]imidazol-2-yl)-N-((R)-1-(5-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(6-fluorobenzo[d]thiazol-2-yl)pyrrolidin-3-yl)cyclopropane-1-carboxyamide,
(1S,2S)-2-(1-cyclopropyl-1H-benzo[d]imidazol-2-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(6-fluoroquinolin-2-yl)pyrrolidin-3-yl)cyclopropane-1-carboxyamide,
(1S,2S)-2-(1-cyclopropyl-1H-benzo[d]imidazol-2-yl)-N-((R)-1-(3-(trifluoromethyl)phenyl)pyrrolidin-3-yl)cyclopropane-1-carboxyamide,
(1S,2S)-2-(1H-benzo[d]imidazol-2-yl)-N-((R)-1-(5-bromopyridin-3-yl)pyrrolidin-3-yl)cyclopropane-1-carboxyamide,
(R)-2-(quinolin-6-yl)-N-(1-(5-(trifluoromethyl)pyridin-3-yl)pyrrolidin-3-yl)acetamide,
(R)-2-(1H-indol-3-yl)-N-(1-(5-(trifluoromethyl)pyridin-3-yl)pyrrolidin-3-yl)acetamide,
(R)-2-(quinolin-7-yl)-N-(1-(5-(trifluoromethyl)pyridin-3-yl)pyrrolidin-3-yl)acetamide,
(R)-2-(4-cyclopropylphenyl)-N-(1-(4-(trifluoromethyl)oxazol-2-yl)pyrrolidin-3-yl) acetamide,
(R)-2-(1-methyl-5-(trifluoromethyl)-1H-pyrazol-3-yl)-N-(1-(5-(trifluoromethyl)pyridin-3-yl)pyrrolidin-3-yl)acetamide,
(R)-2-(6-(trifluoromethyl)pyridin-3-yl)-N-(1-(5-(trifluoromethyl)pyridin-3-yl)pyrrolidin-3-yl)acetamide,
(R)-2-(4-(trifluoromethyl)phenyl)-N-(1-(5-(trifluoromethyl)pyridin-3-yl)pyrrolidin-3-yl)acetamide-2, 2-d₂,
(R)-2-(4-(3,3-difluoropyrrolidin-1-yl)phenyl)-N-(1-(6-(trifluoromethyl)pyridin-3-yl)pyrrolidin-3-yl)acetamide,
(R)-2-(1H-indol-6-yl)-N-(1-(6-(trifluoromethyl)pyridin-3-yl)pyrrolidin-3-yl)acetamide,
2-(4-((2R,6S)-2,6-dimethylmorpholino)phenyl)-N-((R)-1-(5-(trifluoromethyl)pyridin-3-yl)pyrrolidin-3-yl)acetamide,
(R)-2-(4-(pyrrolidin-1-yl)phenyl)-N-(1-(5-(trifluoromethyl)pyridin-3-yl)pyrrolidin-3-yl)acetamide,
(R)-2-(4-((2-methoxyethyl)(methyl)amino)phenyl)-N-(1-(5-(trifluoromethyl)pyridin-3-yl)pyrrolidin-3-yl)acetamide,
2-(4-(trifluoromethyl)phenyl)-N-(1-(5-(trifluoromethyl)pyridin-3-yl)azetidin-3-yl)acetamide,
2-(1H-indol-6-yl)-N-(1-(5-(trifluoromethyl)pyridin-3-yl)azetidin-3-yl)acetamide,
(R)-2-(1-methyl-1H-indol-6-yl)-N-(1-(5-(trifluoromethyl)pyridin-3-yl)pyrrolidin-3-yl)acetamide,
(R)-2-(1-methyl-1H-indol-6-yl)-N-(1-(6-(trifluoromethyl)pyridin-3-yl)pyrrolidin-3-yl)acetamide,
(R)-2-(4-(4-methylpiperazin-1-yl)phenyl)-N-(1-(5-(trifluoromethyl)pyridin-3-yl)pyrrolidin-3-yl)acetamide,
(R)-2-(4-(piperazin-1-yl)phenyl)-N-(1-(5-(trifluoromethyl)pyridin-3-yl)pyrrolidin-3-yl)acetamide,
(R)-2-(4-(4-acetylpiperazin-1-yl)phenyl)-N-(1-(5-(trifluoromethyl)pyridin-3-yl)pyrrolidin-3-yl)acetamide,
(R)-2-(4-(2-oxopiperidin-1-yl)phenyl)-N-(1-(5-(trifluoromethyl)pyridin-3-yl)pyrrolidin-3-yl)acetamide,
(R)-2-(6-chlorobenzo[d]oxazol-2-yl)-N-(1-(5-(trifluoromethyl)pyridin-3-yl)pyrrolidin-3-yl)acetamide,
(R)-2-(benzo[d]oxazol-2-yl)-N-(1-(5-(trifluoromethyl)pyridin-3-yl)pyrrolidin-3-yl)acetamide,
(1S,2S)-2-(3,5-dichlorophenyl)-N-((R)-1-(5-(trifluoromethyl)pyridin-3-yl)pyrrolidin-3-yl)cyclopropane-1-carboxyamide,
(1R,2R)-2-(3,5-dichlorophenyl)-N-((R)-1-(5-(trifluoromethyl)pyridin-3-yl)pyrrolidin-3-yl)cyclopropane-1-carboxyamide,
(1S,2S)-2-(4-bromophenyl)-N-((R)-1-(5-(trifluoromethyl)pyridin-3-yl)pyrrolidin-3-yl)cyclopropane-1-carboxyamide,
(1R,2R)-2-(4-bromophenyl)-N-((R)-1-(5-(trifluoromethyl)pyridin-3-yl)pyrrolidin-3-yl)cyclopropane-1-carboxyamide,
(R)-2-(4-(1,4-dioxa-8-azaspiro[4.5]decan-8-yl)phenyl)-N-(1-(5-(trifluoromethyl)pyridin-3-yl)pyrrolidin-3-yl) acetamide,
(R)-2-(4-(4-oxopiperidin-1-yl)phenyl)-N-(1-(5-(trifluoromethyl)pyridin-3-yl)pyrrolidin-3-yl)acetamide,
(R)-2-(4-cyclopropylphenyl)-N-(3-(hydroxymethyl)-1-(5-(trifluoromethyl)pyridin-3-yl)pyrrolidin-3-yl)acetamide,
(S)-2-(4-cyclopropylphenyl)-N-(3-(hydroxymethyl)-1-(5-(trifluoromethyl)pyridin-3-yl)pyrrolidin-3-yl)acetamide,
(3-(2-(4-cyclopropylphenyl)acetamido)-1-(5-(trifluoromethyl)pyridin-3-yl)pyrrolidin-3-yl)methyl (R)-acetate,
(3-(2-(4-cyclopropylphenyl)acetamido)-1-(5-(trifluoromethyl)pyridin-3-yl)pyrrolidin-3-yl)methyl (S)-acetate,
(R)-2-(4-cyclopropylphenyl)-N-(3-(methoxymethyl)-1-(5-(trifluoromethyl)pyridin-3-yl)pyrrolidin-3-yl)acetamide,
(S)-2-(4-cyclopropylphenyl)-N-(3-(methoxymethyl)-1-(5-(trifluoromethyl)pyridin-3-yl)pyrrolidin-3-yl)acetamide,
(1S,2S)-2-(4-cyclopropylphenyl)-N-((R)-1-(5-(trifluoromethyl)pyridin-3-yl)pyrrolidin-3-yl)cyclopropane-1-carboxyamide,
(1R,2R)-2-(4-cyclopropylphenyl)-N-((R)-1-(5-(trifluoromethyl)pyridin-3-yl)pyrrolidin-3-yl)cyclopropane-1-carboxyamide,
(1S,2S)-2-(3,4-difluorophenyl)-N-((R)-1-(5-(trifluoromethyl)pyridin-3-yl)pyrrolidin-3-yl)cyclopropane-1-carboxyamide,
(1R,2R)-2-(3,4-difluorophenyl)-N-((R)-1-(5-(trifluoromethyl)pyridin-3-yl)pyrrolidin-3-yl)cyclopropane-1-carboxyamide,
(1S,2S)-2-(4-chlorophenyl)-N-((R)-1-(5-(trifluoromethyl)pyridin-3-yl)pyrrolidin-3-yl)cyclopropane-1-carboxyamide,
(1R,2R)-2-(4-chlorophenyl)-N-((R)-1-(5-(trifluoromethyl)pyridin-3-yl)pyrrolidin-3-yl)cyclopropane-1-carboxyamide,
2-(4-isopropylphenyl)-N-(1-(5-(trifluoromethyl)pyridin-3-yl)piperidin-4-yl)acetamide,
(R)-2-(4-isopropylphenyl)-N-(1-(6-methoxy-5-(trifluoromethyl)pyridin-3-yl)pyrrolidin-3-yl)acetamide,
(R)-2-(4-isopropylphenyl)-N-(1-(5-(trifluoromethyl)pyrimidin-2-yl)pyrrolidin-3-yl)acetamide,
(R)-2-(4-isopropylphenyl)-N-(1-(4-(trifluoromethyl)thiazol-2-yl)pyrrolidin-3-yl)acetamide,
(R)-2-(4-isopropylphenyl)-N-(1-(6-(trifluoromethyl)pyridin-3-yl)pyrrolidin-3-yl)acetamide,
(S)-2-(4-isopropylphenyl)-N-(1-(5-(trifluoromethyl)pyridin-3-yl)pyrrolidin-3-yl)acetamide,
(R)-2-(4-(trifluoromethoxy)phenyl)-N-(1-(5-(trifluoromethyl)pyridin-3-yl)pyrrolidin-3-yl)acetamide,
(R)-2-(4-(2,2,2-trifluoroethoxy)phenyl)-N-(1-(5-(trifluoromethyl)pyridin-3-yl)pyrrolidin-3-yl)acetamide,
2-(4-isopropylphenyl)-N-(1-(5-(trifluoromethyl)pyridin-3-yl)azetidin-3-yl)acetamide,
2-(4-isopropylphenyl)-N-(1-(6-(trifluoromethyl)pyridin-3-yl)azetidin-3-yl)acetamide,
(R)-2-(3,5-dichlorophenyl)-N-(1-(5-(trifluoromethyl)pyridin-3-yl)pyrrolidin-3-yl)acetamide,
(R)-2-(3-chloro-5-fluorophenyl)-N-(1-(5-(trifluoromethyl)pyridin-3-yl)pyrrolidin-3-yl)acetamide,
(R)-2-(4-cyclopropylphenyl)-N-(1-(6-(trifluoromethyl)pyridin-3-yl)pyrrolidin-3-yl)acetamide,
(R)-2-(4-isopropylphenyl)-N-(1-(5-(trifluoromethyl)thiophen-2-yl)pyrrolidin-3-yl)acetamide,
(R)-2-(4-(trifluoromethyl)phenyl)-N-(1-(5-(trifluoromethyl)pyridin-3-yl)pyrrolidin-3-yl)acetamide,
(R)-2-(1H-indol-6-yl)-N-(1-(5-(trifluoromethyl)pyridin-3-yl)pyrrolidin-3-yl)acetamide,
(R)-2-(4-cyclopropylphenyl)-N-(1-(6-(2,2,2-trifluoroethoxy)pyridin-3-yl)pyrrolidin-3-yl) acetamide,
(R)-2-(4-cyclopropylphenyl)-N-(1-(5-(2,2,2-trifluoroethoxy)pyridin-3-yl)pyrrolidin-3-yl) acetamide,
(R)-2-(4-cyclopropylphenyl)-N-(1-(4-(2,2,2-trifluoroethoxy)pyridin-2-yl)pyrrolidin-3-yl) acetamide,
(R)-2-(4-cyclopropylphenyl)-N-(1-(2-methoxy-5-(trifluoromethyl)pyridin-3-yl)pyrrolidin-3-yl)acetamide,
(R)-2-(4-cyclopropylphenyl)-N-(1-(5-(trifluoromethyl)pyridin-3-yl)piperidin-3-yl) acetamide,
(R)-2-(4-cyclopropylphenyl)-N-(1-(6-(trifluoromethyl)pyridin-3-yl)piperidin-3-yl) acetamide,
(R)-2-(4-(2-hydroxypropan-2-yl)phenyl)-N-(1-(5-(trifluoromethyl)pyridin-3-yl)pyrrolidin-3-yl)acetamide,
(R)-2-(4-(3-methylpyrazin-2-yl)phenyl)-N-(1-(5-(trifluoromethyl)pyridin-3-yl)pyrrolidin-3-yl)acetamide,
(R)-2-(4-(4-methyloxazol-5-yl)phenyl)-N-(1-(5-(trifluoromethyl)pyridin-3-yl)pyrrolidin-3-yl)acetamide,
(R)-3-(3-(2-(4-cyclopropylphenyl)acetamido)pyrrolidin-1-yl)-5-(trifluoromethyl)pyridine 1-oxide,
(3R)-3-(2-(4-cyclopropylphenyl)acetamido)-1-(5-(trifluoromethyl)pyridin-3-yl)pyrrolidine 1-oxide,
(R)-2-(4-cyclopropylphenyl)-2-methyl-N-(1-(5-(trifluoromethyl)pyridin-3-yl)pyrrolidin-3-yl)propanamide,
(R)-2-(4-cyclopropylphenyl)-N-(1-(3-fluoro-5-(trifluoromethyl)phenyl)pyrrolidin-3-yl)acetamide,
(R)-2-amino-2-(4-cyclopropylphenyl)-N-((R)-1-(5-(trifluoromethyl)pyridin-3-yl)pyrrolidin-3-yl)acetamide,
(S)-2-amino-2-(4-cyclopropylphenyl)-N-((R)-1-(5-(trifluoromethyl)pyridin-3-yl)pyrrolidin-3-yl)acetamide,
(R)-2-(4-cyclopropylphenyl)-2-(dimethylamino)-N-((R)-1-(5-(trifluoromethyl)pyridin-3-yl)pyrrolidin-3-yl)acetamide,
(S)-2-(4-cyclopropylphenyl)-2-(dimethylamino)-N-((R)-1-(5-(trifluoromethyl)pyridin-3-yl)pyrrolidin-3-yl)acetamide,
(R)-2-(1-methyl-1H-indol-5-yl)-N-(1-(5-(trifluoromethyl)pyridin-3-yl)pyrrolidin-3-yl)acetamide,
(R)-2-(2,4-bis(trifluoromethyl)phenyl)-N-(1-(5-(trifluoromethyl)pyridin-3-yl)pyrrolidin-3-yl)acetamide,
(R)-2-(2-fluoro-4-(trifluoromethyl)phenyl)-N-(1-(5-(trifluoromethyl)pyridin-3-yl)pyrrolidin-3-yl)acetamide,
(R)-2-(1-methyl-1H-indazol-5-yl)-N-(1-(5-(trifluoromethyl)pyridin-3-yl)pyrrolidin-3-yl)acetamide,
(R)-2-(1-methyl-1H-indazol-5-yl)-N-(1-(4-(trifluoromethyl)thiazol-2-yl)pyrrolidin-3-yl)acetamide,
(R)-2-(1-methyl-1H-indazol-5-yl)-N-(1-(6-(trifluoromethyl)pyridin-3-yl)pyrrolidin-3-yl)acetamide,
(R)-2-(4-(2,2-dimethylmorpholino)phenyl)-N-(1-(5-(trifluoromethyl)pyridin-3-yl)pyrrolidin-3-yl)acetamide,
(R)-2-(4-(1H-pyrazol-1-yl)phenyl)-N-(1-(5-(trifluoromethyl)pyridin-3-yl)pyrrolidin-3-yl)acetamide,
(R)-N-(3-methyl-l-(5-(trifluoromethyl)pyridin-3-yl)pyrrolidin-3-yl)-2-(4-morpholinophenyl)acetamide,
(R)-2-(4-cyclopropylphenyl)-N-(3-methyl-1-(5-(trifluoromethyl)pyridin-3-yl)pyrrolidin-3-yl)acetamide,
2-(4-(3-oxa-8-azabicyclo[3.2.1]octan-8-yl)phenyl)-N-((R)-1-(5-(trifluoromethyl)pyridin-3-yl)pyrrolidin-3-yl)acetamide,
2-(4-(3-oxa-8-azabicyclo[3.2.1]octan-8-yl)phenyl)-N-((R)-1-(6-(trifluoromethyl)pyridin-3-yl)pyrrolidin-3-yl)acetamide,
(R)-2-(benzo[d]oxazol-5-yl)-N-(1-(6-(trifluoromethyl)pyridin-3-yl)pyrrolidin-3-yl)acetamide,
(R)-2-(benzo[d]oxazol-5-yl)-N-(1-(5-(trifluoromethyl)pyridin-3-yl)pyrrolidin-3-yl)acetamide,
(R)-2-(2-methylbenzo[d]oxazol-5-yl)-N-(1-(5-(trifluoromethyl)pyridin-3-yl)pyrrolidin-3-yl)acetamide,
(R)-2-(2-methylbenzo[d]oxazol-5-yl)-N-(1-(6-(trifluoromethyl)pyridin-3-yl)pyrrolidin-3-yl)acetamide,
2-(4-((1S,4S)-2-oxa-5-azabicyclo[2.2.2]octan-5-yl)phenyl)-N-((R)-1-(5-(trifluoromethyl)pyridin-3-yl)pyrrolidin-3-yl)acetamide,
2-(4-((1S,4S)-2-oxa-5-azabicyclo[2.2.2]octan-5-yl)phenyl)-N-((R)-1-(6-(trifluoromethyl)pyridin-3-yl)pyrrolidin-3-yl)acetamide,
(R)-2-(benzo[d]thiazol-6-yl)-N-(1-(5-(trifluoromethyl)pyridin-3-yl)pyrrolidin-3-yl)acetamide,
(R)-2-(benzo[d]thiazol-6-yl)-N-(1-(6-(trifluoromethyl)pyridin-3-yl)pyrrolidin-3-yl)acetamide,
(R)-2-(1H-indazol-5-yl)-N-(1-(5-(trifluoromethyl)pyridin-3-yl)pyrrolidin-3-yl)acetamide,
(R)-2-(1H-indazol-5-yl)-N-(1-(6-(trifluoromethyl)pyridin-3-yl)pyrrolidin-3-yl)acetamide,
2-(4-cyclopropylphenyl)-N-(3-(trifluoromethyl)-1-(5-(trifluoromethyl)pyridin-3-yl)pyrrolidin-3-yl)acetamide,
(R)-N-(3-(trifluoromethyl)-1-(5-(trifluoromethyl)pyridin-3-yl)pyrrolidin-3-yl)-2-(4-(trifluoromethyl)phenyl)acetamide,
(S)-N-(3-(trifluoromethyl)-1-(5-(trifluoromethyl)pyridin-3-yl)pyrrolidin-3-yl)-2-(4-(trifluoromethyl)phenyl)acetamide,
(R)-2-(1H-benzo[d][1,2,3]triazol-5-yl)-N-(1-(5-(trifluoromethyl)pyridin-3-yl)pyrrolidin-3-yl)acetamide,
(R)-2-(1H-benzo[d][1,2,3]triazol-5-yl)-N-(1-(6-(trifluoromethyl)pyridin-3-yl)pyrrolidin-3-yl)acetamide,
2-(4-((1R,4R)-2-oxa-5-azabicyclo[2.2.1]heptan-5-yl)phenyl)-N-((R)-1-(5-(trifluoromethyl)pyridin-3-yl)pyrrolidin-3-yl)acetamide,
2-(4-((1S,4S)-2-oxa-5-azabicyclo[2.2.1]heptan-5-yl)phenyl)-N-((R)-1-(5-(trifluoromethyl)pyridin-3-yl)pyrrolidin-3-yl)acetamide,
2-(4-((1R,4R)-2-oxa-5-azabicyclo[2.2.1]heptan-5-yl)phenyl)-N-((R)-1-(6-(trifluoromethyl)pyridin-3-yl)pyrrolidin-3-yl)acetamide,
2-(4-((1S,4S)-2-oxa-5-azabicyclo[2.2.1]heptan-5-yl)phenyl)-N-((R)-1-(6-(trifluoromethyl)pyridin-3-yl)pyrrolidin-3-yl)acetamide,
(R)-2-(1-methyl-1H-benzo[d][1,2,3]triazol-5-yl)-N-(1-(5-(trifluoromethyl)pyridin-3-yl)pyrrolidin-3-yl)acetamide,
(R)-2-(1-methyl-1H-benzo[d][1,2,3]triazol-5-yl)-N-(1-(6-(trifluoromethyl)pyridin-3-yl)pyrrolidin-3-yl)acetamide,
2-(4-cyclopropylphenyl)-N-(1-(4-(trifluoromethyl)phenyl)azetidin-3-yl)acetamide,
2-(4-cyclopropylphenyl)-N-(1-(4-fluoro-3-(trifluoromethyl)phenyl)azetidin-3-yl)acetamide,
(R)-2-(4-bromophenyl)-N-(1-(5-(trifluoromethyl)pyridin-3-yl)pyrrolidin-3-yl)acetamide,
(R)-2-(4-cyclopropylphenyl)-N-(1-(6-fluorobenzo[d]thiazol-2-yl)pyrrolidin-3-yl)acetamide,
(R)-N-(1-(5-bromothiazol-2-yl)pyrrolidin-3-yl)-2-(4-cyclopropylphenyl)acetamide,
(R)-2-(4-cyclopropylphenyl)-N-(1-(4-fluoro-3-(trifluoromethyl)phenyl)pyrrolidin-3-yl) acetamide,
(R)-2-(4-cyclopropylphenyl)-N-(1-(3-methoxyphenyl)pyrrolidin-3-yl) acetamide,
(R)-2-(4-(3,3-difluoropyrrolidin-1-yl)phenyl)-N-(1-(5-(trifluoromethyl)pyridin-3-yl)pyrrolidin-3-yl)acetamide,
ethyl (R)-2-(3-(3-(2-(4-cyclopropylphenyl)acetamido)pyrrolidin-1-yl)phenoxy)-2-methylpropanoate,
(R)-2-(4-cyclopropylphenyl)-N-(1-(3-hydroxyphenyl)pyrrolidin-3-yl) acetamide,
(R)-2-(4-cyclopropylphenyl)-N-(1-(6-(trifluoromethyl)pyridin-2-yl)pyrrolidin-3-yl)acetamide,
(R)-2-(4-cyclopropylphenyl)-N-(1-(4, 6-dimethoxypyrimidin-2-yl)pyrrolidin-3-yl) acetamide,
(R)-2-(4-morpholinophenyl)-N-(1-(5-(trifluoromethyl)pyridin-3-yl)pyrrolidin-3-yl)acetamide,
(R)-2-(4-cyclopropylphenyl)-N-(1-(4-(trifluoromethyl)pyridin-2-yl)pyrrolidin-3-yl)acetamide,
(R)-2-(4-(trifluoromethyl)phenyl)-N-(1-(6-(trifluoromethyl)pyridin-3-yl)pyrrolidin-3-yl)acetamide,
(R)-N-(1-(4-fluoro-3-(trifluoromethyl)phenyl)pyrrolidin-3-yl)-2-(4-(trifluoromethyl)phenyl)acetamide,
(R)-2-(4-(trifluoromethyl)phenyl)-N-((R)-1-(6-(trifluoromethyl)pyridin-3-yl)pyrrolidin-3-yl)propanamide,
(S)-2-(4-(trifluoromethyl)phenyl)-N-((R)-1-(6-(trifluoromethyl)pyridin-3-yl)pyrrolidin-3-yl)propanamide,
(1S,2S)-2-(1H-benzo[d]imidazol-2-yl)-N-((R)-1-(5-(trifluoromethyl)pyridin-3-yl)piperidin-3-yl)cyclopropane-1-carboxyamide,
(R)-2-(1H-indol-6-yl)-N-(1-(4-(trifluoromethyl)thiazol-2-yl)pyrrolidin-3-yl) acetamide, and
(R)-2-(1-methyl-1H-indol-5-yl)-N-(1-(6-(trifluoromethyl)pyridin-3-yl)pyrrolidin-3-yl)acetamide,
a tautomer or a stereoisomer of the compound, a pharmaceutically acceptable salt of the compound, or a solvate of the compound, the tautomer, the stereoisomer, or the salt.

Examples of pharmacologically acceptable salts of the compounds represented by the above-described General Formulae (I) to (VI) include acid addition salts with mineral acids such as hydrochloric acid, sulfuric acid, and phosphoric acid; acid addition salts with organic acids such as formic acid, acetic acid, citric acid, tartaric acid, and methanesulfonic acid; salts with inorganic bases, such as sodium salts, potassium salts, lithium salts, and calcium salts; and addition salts with organic bases such as arginine and piperazine.

Furthermore, the compounds of the present invention may have cis/trans isomers, optically active substances, and stereoisomers such as racemates; however, all of them are included in the present invention, and mixtures of enantiomers or diastereomers are also included in the present invention.

Furthermore, the compounds of the present invention also include stable isotopes.

Furthermore, the compounds of the present invention also include a compound in which the pyridine in Example 134 that will be described below is pyridine 1-oxide, a compound in which the pyridine is pyrrolidine 1-oxide or piperidine 1-oxide, and the like.

Moreover, the compounds of the present invention may also be tautomers, hydrates, solvates with an organic solvent such as an alcohol, derivatives substituted with a stable isotope such as deuterium, or prodrugs.

### General synthesis method

The compounds of the present invention can be each produced using commercially available compounds as raw materials and using any known method or the method described below. Examples of the known method include the methods described in Lectures on Experimental Chemistry, 5th Edition (Maruzen Publishing Co., Ltd.), New Edition Heterocyclic Compounds (KODANSHA LTD.), Protective Groups in Organic Synthesis (Wiley), and the like.

Depending on the compounds produced using the present production method, protection or deprotection and conversion or introduction of functional groups may be effective in the various stages of production. In such a case, the operation or procedure is not limited to the operation or procedure of the described production method, and any appropriate operation or procedure can be applied using known methods.

A prodrug of a compound of the present invention can be produced by applying any known method such as amidation, esterification, or alkylation in the various stages of production.

Depending on the compound produced using the present production method, various salts, hydrates, and crystal polymorphisms may be included. Furthermore, in a case in which an optical isomer, a geometric isomer, or a tautomer may exist, unless particularly limited, a mixture at any ratio may be included. A mixture of these isomers can be separated by any known method.

A method for producing the compounds of the present invention will be described below; however, the method for producing the compound of the present invention is not limited to the following method.

In the present specification, the following abbreviations may be used.

M: molar concentration, N: normal, MS: mass spectrum, [M+H]⁺: protonated molecular ion peak, [M+Na]⁺: sodium adduct molecular ion peak, [M-H]⁻: deprotonated molecular ion peak, CDCl₃: deuterated chloroform, DMSO-d₆: deuterated dimethyl sulfoxide, CD₃OD: deuterated methanol, ¹H NMR: proton nuclear magnetic resonance, br s: broad singlet, Me: methyl group, Et: ethyl group, t-Bu: tert-butyl group, CN: cyano group, CF₃: trifluoromethyl group, Ts: p-toluenesulfonyl group, Boc: tert-butoxycarbonyl group, DMF: N,N-dimethylformamide, THF: tetrahydrofuran, DME: 1,2-dimethoxyethane, HATU: O-(7-azabenzotriazol-1-yl)-N,N,N',N'-tetramethyluronium hexafluorophosphate, HOBT: 1-hydroxybenzotriazole, WSC: 1-ethyl-3-(dimethylaminopropyl)carbodiimide, DMT-MM: 4-(4,6-dimethoxy-1,3,5-triazin-2-yl)-4-methylmorpholinium chloride, DMAP: N,N-dimethyl-4-aminopyridine, DIBAL-H: diisobutylaluminum hydride, L-selectride: tri(sec-butyl)boron lithium hydride, DIPEA: N,N-diisopropylethylamine, BINAP: 2,2'-bis(diphenylphosphino)-1,1'-binaphthyl, DMSO: dimethyl sulfoxide, FBS: fetal bovine serum, DMEM: Dulbecco's modified Eagle medium, CO₂: carbon dioxide, NaCl: sodium chloride, KCl: potassium chloride, MgCl₂: magnesium chloride, CaCl₂: calcium chloride, CsCl: cesium chloride, CsF: cesium fluoride, HEPES: 4-(2-hydroxyethyl)-1-piperazine ethanesulfonic acid, EGTA: glycol ether diamine 4 tetraacetic acid, CYP: cytochrome P450, NADP or NADP⁺: nicotinamide adenine dinucleotide phosphate, G-6-P: glucose-6-phosphate, G-6-P DH(Y): glucose-6-phosphate dehydrogenase (yeast-derived), PSNL: partial sciatic nerve ligation
A production method for General Formula (Z) will be described below.

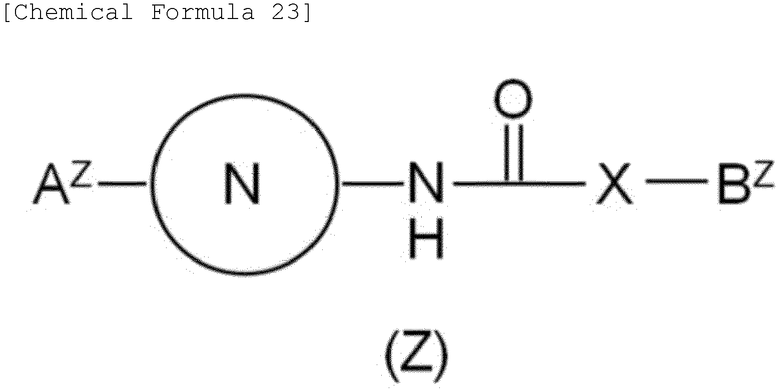

In the general formula, A^{z} represents A in the compound represented by General Formula (I) and a 6-membered ring including D to J in the compound represented by General Formula (III), 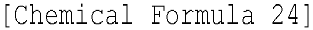 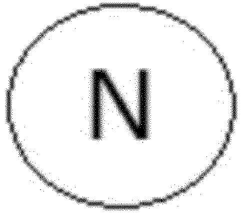 represents the following in the compound represented by General Formula (I): 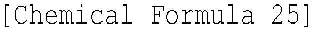 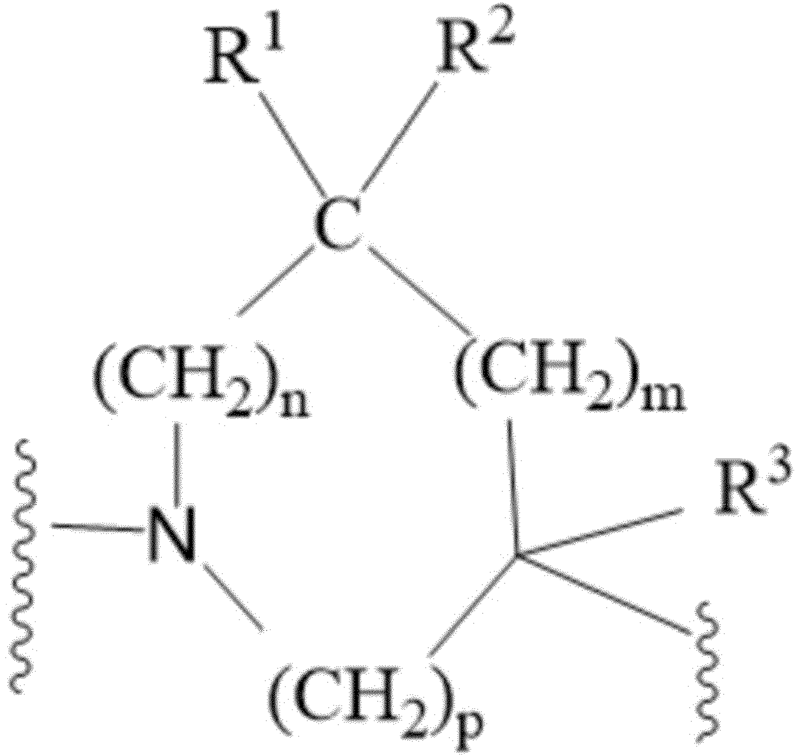 or the following in the compound represented by the above-described General Formula (III):

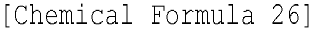

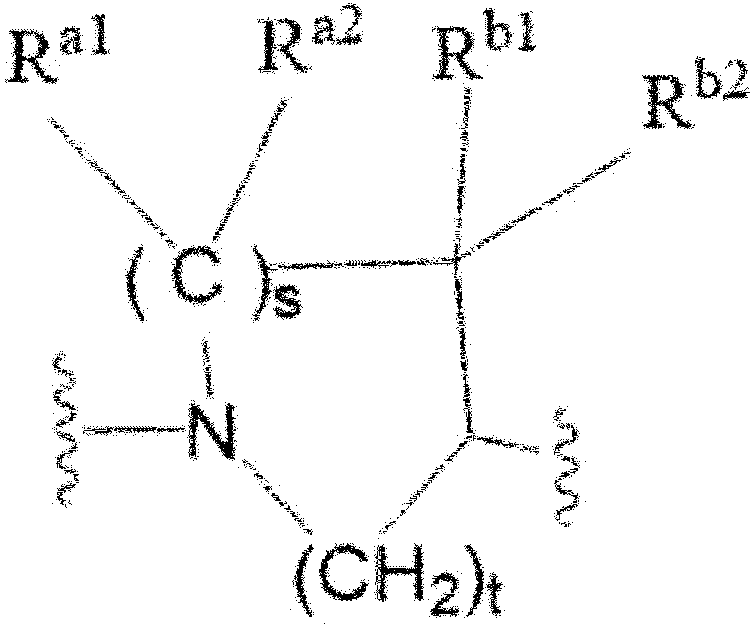

X represents the following in the compound represented by General Formula (I) or the compound represented by General Formula (III): 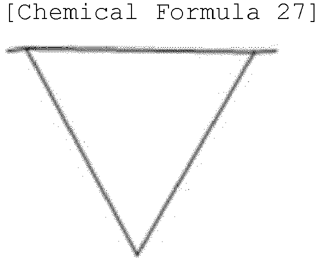 or the following in the compound represented by General Formula (III): 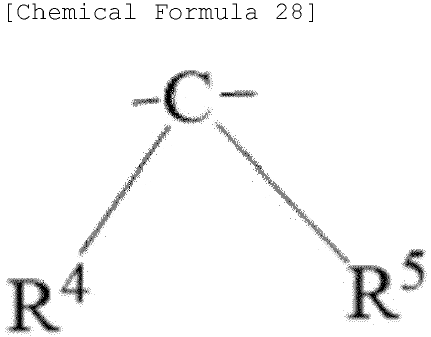 and B^{z} represents B in the compound represented by General Formula (I) or B^{a} in the compound represented by General Formula (III):
Compounds represented by General Formula (II), General Formula (IV), General Formula (V), and General Formula (VI) can also be similarly produced.

### Method for producing compound (Z)

The above-described compound (Z) can be produced by, for example, the method shown below.

### (Production method 1)

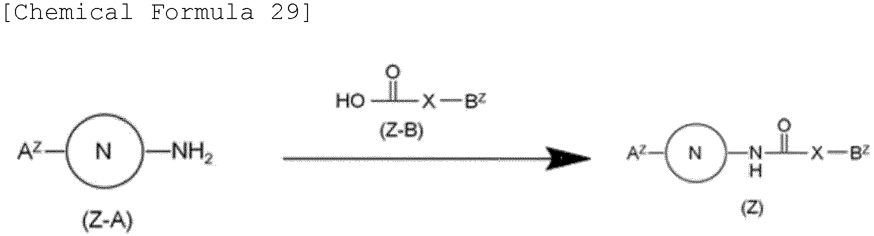

wherein the reference symbols have the same meanings as described above.

Compound (Z) can be produced by causing compound (Z-A) to react with compound (Z-B) in an appropriate solvent such as DMF in the presence of a condensing agent such as HATU or WSC, and if necessary, in the presence of an additive such as HOBT or DMAP and if necessary, a base such as triethylamine or DIPEA, at 0°C to 100°C.

Furthermore, the compound can be produced by causing compound (Z-A) to react with a carboxylic acid chloride or carboxylic acid anhydride corresponding to compound (Z-B) in an appropriate solvent such as tetrahydrofuran, and if necessary, in the presence of a base such as triethylamine, DIPEA, or pyridine, at 0°C to 100°C.

In addition to that, the compound can be produced from compound (Z-A) and compound (Z-B), or compounds respectively equivalent thereto, using the condensation reaction described in Christian A.G.N. Montalbetti, et al, Tetrahedron, 61(46), 2005, 10827-10852, or a condensation reaction equivalent thereto.

Compound (Z-A) can be produced by, for example, the method described below. 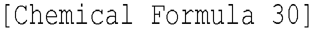 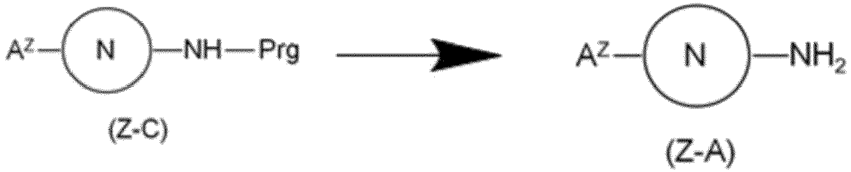 wherein Prg means a protective group and represents an arbitrary functional group that can be converted to hydrogen by a deprotection reaction;
and the other reference symbols have the same meanings as described above.

Compound (Z-A) can be produced by causing compound (Z-C) to react with an acid such as hydrogen chloride or trifluoroacetic acid at -10°C to 100°C in an appropriate solvent such as methanol, or without solvent.

Furthermore, compound (Z-A) can be produced by subjecting compound (Z-C) to a hydrogenation reaction using a catalyst such as palladium-carbon or palladium hydroxide at 0°C to 100°C in an appropriate solvent such as methanol.

In addition to that, compound (Z-A) can be produced from compound (Z-C) or a compound equivalent thereto using a deprotection reaction for a protective group, which is described in Protective Groups in Organic Synthesis (Wiley) or the like, or a deprotection reaction equivalent thereto.

Compound (Z-C) can be produced by, for example, the method described below. 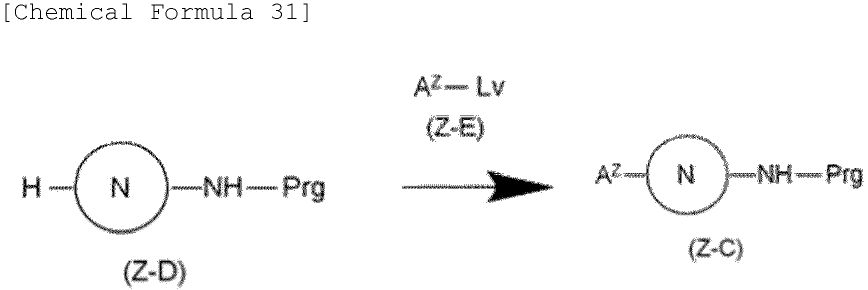 wherein Prg means a protective group and
represents any arbitrary functional group that can be converted to hydrogen by a deprotection reaction; Lv represents a leaving group; and the other reference symbols have the same meanings as described above.

Compound (Z-C) can be produced by causing compound (Z-D) to react with compound (Z-E) in an appropriate solvent such as DMF in the presence of a metal catalyst such as tris(dibenzylidene acetone)dipalladium or copper iodide, and if necessary, in the presence of a ligand such as BINAP or ethylenediamine, and if necessary, a base such as triethylamine or DIPEA, at 0°C to 200°C. Regarding the leaving group, an appropriate functional group such as a halogen or a tosyl group is used.

In addition to that, compound (Z-C) can be produced by causing compound (Z-D) to react with compound (Z-E) in an appropriate solvent such as DMF, in the presence of a base such as sodium hydride or cesium carbonate, at 0°C to 200°C.

Furthermore, the compound can be produced from compound (Z-D) and compound (Z-E), or compounds respectively equivalent thereto, using reactions equivalent to these reactions.

Compound (Z-D) can be a commercially available product or can be produced from a commercially available product according to a known method. For example, the compound can be produced from appropriate starting raw materials by combining known methods according to the synthesis methods described in known patent literatures such as WO 2014/159969, WO 2012/154274, WO 2017/190609, and WO 2016/027195, or according to synthesis methods equivalent to those.

Compound (Z-E) can be a commercially available product or can be produced from a commercially available product according to a known method. For example, the compound can be produced from appropriate starting raw materials by combining known methods according to the synthesis methods described in a known academic literature, Cottet Fabrice, et al, Eur. J. Org. Chem. (2), 2002, 327-330, or known patent literatures such as WO 2013/088404 and WO 2010/064707, or according to synthesis methods equivalent to those.

Compound (Z-B) can be produced by, for example, the method described below. 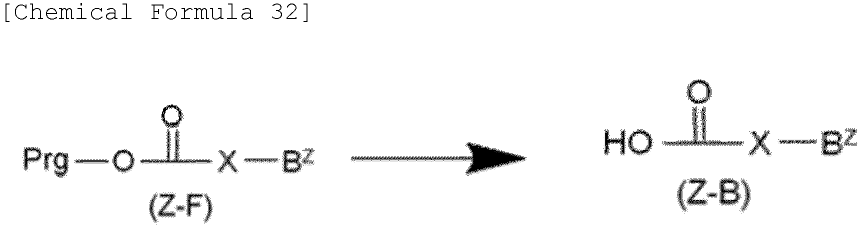 wherein Prg means a protective group and
represents any arbitrary functional group that can be converted to hydrogen by a deprotection reaction; the other reference symbols have the same meanings as described above.

Compound (Z-B) can be produced from compound (Z-F) by causing the compound to react with a base such as lithium hydroxide or sodium hydroxide in an appropriate solvent such as a mixed solvent of water and methanol or tetrahydrofuran, at 0°C to 100°C.

In addition to that, the compound can be produced from compound (Z-F) or a compound equivalent thereto using a deprotection reaction for a protective group, which is described in Protective Groups in Organic Synthesis (Wiley) or the like, or a deprotection reaction equivalent thereto.

Compound (Z-F) can be a commercially available product or can be produced from a commercially available product according to a known method. For example, the compound can be produced from appropriate starting raw materials by combining known methods according to the synthesis methods described in known patent literatures such as WO 2017/122754, WO 2013/026914, and WO 2015/073528 and academic literatures such as Radoslaw Laufer, Bioorg. Med. Chem, 22(17), 2014, 4968-4997, or according to synthesis methods equivalent to those.

Furthermore, compound (Z-B) can be a commercially available product or can be produced from a commercially available product according to a known method. For example, the compound can be produced from appropriate starting raw materials by combining known methods according to the synthesis methods described in known patent literatures such as WO 2013/144295, WO 2014/010748, and WO 2008/125337, or according to synthesis methods equivalent to those.

### (Production method 2)

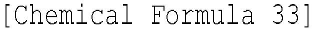

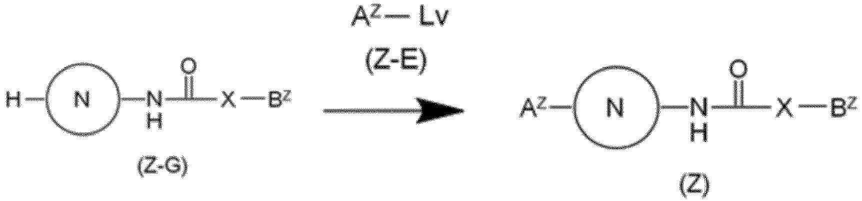

wherein Lv represents a leaving group; and the other reference symbols have the same meanings as described above.

Compound (Z) of the present invention can be produced by causing compound (Z-E) to react with compound (Z-G) in an appropriate solvent such as DMF in the presence of a metal catalyst such as tris(dibenzylidene acetone)dipalladium or copper iodide, and if necessary, in the presence of a ligand such as BINAP or ethylenediamine, and if necessary, a base such as triethylamine or DIPEA, at 0°C to 200°C. Regarding the leaving group, an appropriate functional group such as a halogen or a tosyl group is used.

In addition to that, compound (Z) can be produced by causing compound (Z-E) to react with compound (Z-G) in an appropriate solvent such as DMF in the presence of a base such as sodium hydride or cesium carbonate at 0°C to 200°C.

Furthermore, the compound can be produced from compound (Z-E) and compound (G -G), or compounds respectively equivalent thereto, using reactions equivalent to these reactions.

Compound (Z-G) can be produced by, for example, the method described below. 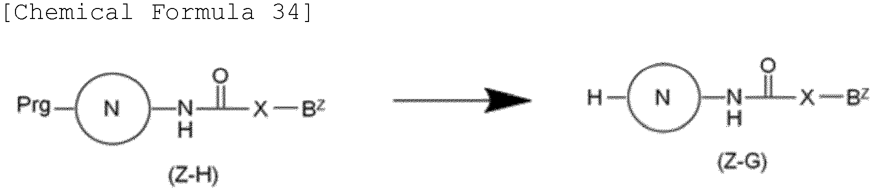 wherein Prg means a protective group and
represents any arbitrary functional group that can be converted to hydrogen by a deprotection reaction; the other reference symbols have the same meanings as described above.

Compound (Z-G) can be produced by causing compound (Z-H) to react with an acid such as hydrochloric acid or trifluoroacetic acid at -10°C to 100°C in an appropriate solvent such as methanol or chloroform, or without solvent.

Furthermore, compound (Z-G) can be produced by subjecting compound (Z-H) to a hydrogenation reaction using a catalyst such as palladium-carbon or palladium hydroxide at 0°C to 100°C in an appropriate solvent such as methanol or THF.

In addition to that, the compound can be produced from compound (Z-H) or a compound equivalent thereto using a deprotection reaction for a protective group, which is described in Protective Groups in Organic Synthesis (Wiley) or the like, or a deprotection reaction equivalent thereto.

Compound (Z-H) can be produced by, for example, the method described below. 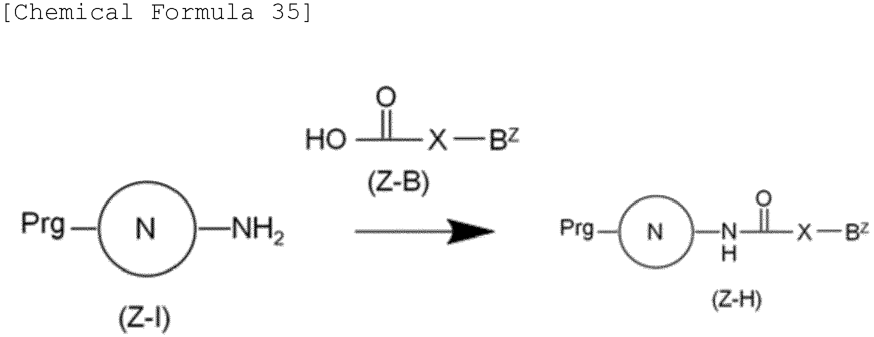 wherein Prg means a protective group and
represents any arbitrary functional group that can be converted to hydrogen by a deprotection reaction; the other reference symbols have the same meanings as described above.

Compound (Z-H) can be produced by causing compound (Z-B) to react with compound (Z-I) in an appropriate solvent such as DMF in the presence of a condensing agent such as HATU or WSC, and if necessary, in the presence of an additive such as HOBT or DMAP and if necessary, a base such as triethylamine or DIPEA, at 0°C to 100°C.

Furthermore, the compound can be produced by causing compound (Z-I) to react with a carboxylic acid chloride or carboxylic acid anhydride corresponding to compound (Z-B) in an appropriate solvent such as tetrahydrofuran, and if necessary, in the presence of a base such as triethylamine, DIPEA, or pyridine, at 0°C to 100°C.

In addition to that, the compound can be produced from compound (Z-B) and compound (Z-I), or compounds respectively equivalent thereto, using the condensation reaction described in Christian A.G.N. Montalbetti, et al, Tetrahedron, 61(46), 2005, 10827-10852, or a condensation reaction equivalent thereto.

Compound (Z-I) can be a commercially available product or can be produced from a commercially available product according to a known method. For example, the compound can be produced from appropriate starting raw materials by combining known methods according to the synthesis methods described in known patent literatures such as WO 2016/100154, WO 2012/125893, and WO 2008/013130 and academic literatures such as Kyoji Tomita, J. Med. Chem, 45(25), 2002, 5564-5575, or according to synthesis methods equivalent to those.

Next, the pharmacological action of the compounds of the present invention will be described.

In Example 188 that will be described below, the results for a pharmacological experiment on the T-type calcium blocking action of the compounds of the present invention obtained by using cells that steadily express human Cav3.2 channels and measuring the intracellular calcium concentration are described.

As is obvious from Tables 1 to 4, it has been made clear that the compounds of the present invention have excellent T-type calcium blocking action.

Meanwhile, it is known that calcium channels have a property referred to as state-dependence.

That is, calcium channels adopt three states, namely, a stationary (closed) state, an activated (activated, open) state, and an inactivated (inactivated) state, depending on the difference in the membrane potential.

The compounds of the present invention also include compounds having superior voltage-dependent T-type calcium channel blocking action in an inactivated state compared to a stationary state or a closed state, and these are expected to have selective voltage-dependent T-type calcium channel blocking action.

Furthermore, the occasion of neuropathic pain is in a state in which depolarization is repeated; however, the compounds of the present invention also include compounds having a property in which antagonistic action is enhanced in such a state (frequency-dependence).

Furthermore, in Example 189 that will be described below, a formalin pain test using SD rats was performed, and as a result, the compounds of the present invention exhibited excellent pain behavioral suppression.

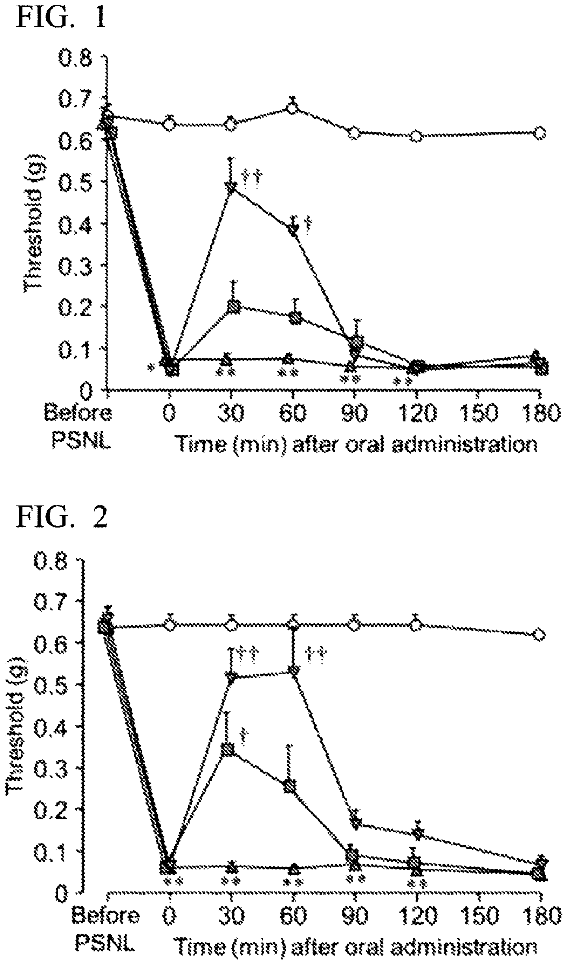

Moreover, in Example 190 that will be described below, in a pharmacological experiment for mechanical allodynia in a mouse partial sciatic nerve ligation (PSNL) model, as is obvious from Figs. 1 and 2, mechanical allodynia could be suppressed statistically significantly compared to a vehicle group, by perorally administering the compound described in Example 4 and the compound described in Example 144 in an amount of 30 mg/kg.

Therefore, the compounds of the present invention have excellent T-type calcium channel blocking action, and the compounds are highly safe T-type calcium channel blockers without causing serious side effects such as cardiotoxicity (hERG) and can be used for diseases such as neuropathic pain.

That is, the target diseases of the compounds of the present invention include pain as described in Patent Literature 9, limited target diseases include chronic pain, and more limited target diseases include neuropathic pain.

Furthermore, as described in the above-mentioned patent literatures, "pain is classified into chronic and acute pains including neuropathic, inflammatory, cancerous, and visceral pains, and causative diseases thereof include diabetic neuropathy, traumatic neuropathy, nerve compression, strangulation, spinal cord injury, stroke, fibromyalgia, carpal tunnel syndrome, arthrosis deformans, rheumatoid arthritis and multiple sclerosis, herpes zoster, herpes simplex, syphilis, cancer chemotherapy, neuropathy induced by HIV and HIV treatment, chronic arthralgia, postherpetic neuralgia, neuroma pain, trigeminal neuralgia, phantom limb pain, postoperative pain, stump pain, toothache, nerve plexus neuropathy, glossopharyngeal neuralgia, laryngeal neuralgia, migraine, cancerous neuropathy, polyneuropathy, causalgia, low back pain, complex regional pain syndrome (CRPS), and thalamic pain." Furthermore, pains originating from causes other than the causes listed above are also included in the target diseases of the present invention. Further, as described in the literatures, examples of the target diseases other than pain include "diseases associated with disorders in the central nervous system (CNS), diseases associated with disorders in the bladder function, stroke, pruritus, atopic dermatitis, hypertension, hyperaldosteronemia, edema, ischemic heart disease, age-related macular degeneration, cancer, diabetes mellitus, infertility and sexual dysfunction, arrhythmia, and kidney diseases; the diseases associated with disorders in the central nervous system (CNS) include epilepsy, essential tremor, schizophrenia, Parkinson's disease, manic depression, bipolar disorder, depression, anxiety, cognitive impairment, drug dependence, Huntington's disease, and sleep disorders; and the diseases associated with disorders in the bladder function include overactive bladder," and the like.

Furthermore, the compounds of the present invention can also be used in combination with known therapeutic agents for pain.

The compounds of the present invention can be administered by any suitable administration method such as oral administration or parenteral administration; and oral administration is preferred.

In order to formulate the compounds, formulations such as a tablet, granules, a powder, a capsule, a suspension, an injectable preparation, and a suppository can be produced by conventional methods in the technical field of preparation.

For the preparation of these, for example, in the case of a tablet, conventional excipients, disintegrants, binders, lubricating agents, colorants, and the like are used. Here, examples of the excipients include lactose, D-mannitol, crystalline cellulose, glucose, and the like; examples of the disintegrants include starch, carboxymethyl cellulose calcium (CMC-Ca), and the like; examples of the lubricating agents include magnesium stearate, talc, and the like; and examples of the binders include hydroxypropyl cellulose (HPC), gelatin, polyvinylpyrrolidone (PVP), and the like. For the preparation of an injectable preparation, a solvent, a stabilizer, a dissolution aid, a suspending agent, an emulsifier, a soothing agent, a buffering agent, a preservative, and the like are used.

The amount of administration is such that usually for an adult, each of the compounds of the present invention as an active ingredient for an injectable preparation is administered in an amount of about 0.01 mg to 100 mg per day, and in an amount of 1 mg to 2,000 mg per day by oral administration; however, the amount of administration can be increased or decreased according to the age, symptoms, and the like.

Next, the present invention will be described in more detail by way of Reference Examples and Examples; however, the present invention is not intended to be limited to these.

Regarding the column, PLC, and TLC used in Reference Examples and Examples, unless particularly stated otherwise, either silica gel or NH silica gel, or both of them were used. For an analysis of a synthesized compound, ¹H-NMR (400 MHz), atmospheric pressure ionization high-resolution time-of-flight mass spectrometry (ESI), and other appropriate analysis methods were used.

Regarding the compound names of Reference Examples and Examples, a compound was denominated based on the name obtained by converting the structural formula drawn using any one of ChemDraw ver. 13, 14, or 15 manufactured by CambridgeSoft Corporation, by means of the denomination algorithm installed in the same software program.

### Reference Example 1-1

### Tert-butyl (R)-(1-(5-(trifluoromethyl)pyridin-3-yl)pyrrolidin-3-yl) carbamate

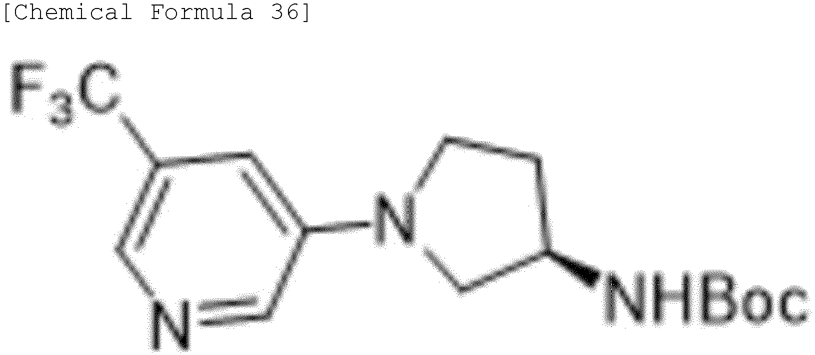

3-Bromo-5-(trifluoromethyl)pyridine (230 mg, 1.02 mmol), tert-butyl (R)-pyrrolidin-3-ylcarbamate (190 mg, 1.02 mmol), tris(dibenzylidene acetone)dipalladium(0) (93 mg, 0.10 mmol), BINAP (67 mg, 0.22 mmol), and sodium tert-butoxide (196 mg, 2.04 mmol) were suspended in toluene (4.0 mL), and the suspension was stirred for 3 hours at 85°C under a nitrogen gas stream. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and thus the title compound (white amorphous, 116 mg, 34%) was obtained.
¹H NMR (CDCl₃, 400MHz) : δ=1. 46 (s, 9H) , 1.9-2.1 (m, 1H) , 2.2-2.4(m,1H),3.23(dd,1H,J=4,10Hz),3.3-3.6(m,2H), 3.65(dd,1H,J=6,10Hz),4.40(br s,1H),4.69(br s,1H), 6.9-7.0(m,1H),8.12(d,1H,J=3Hz),8.21(s,1H).

### Reference Example 1-2

### (R)-1-(5-(trifluoromethyl)pyridin-3-yl)pyrrolidin-3-amine

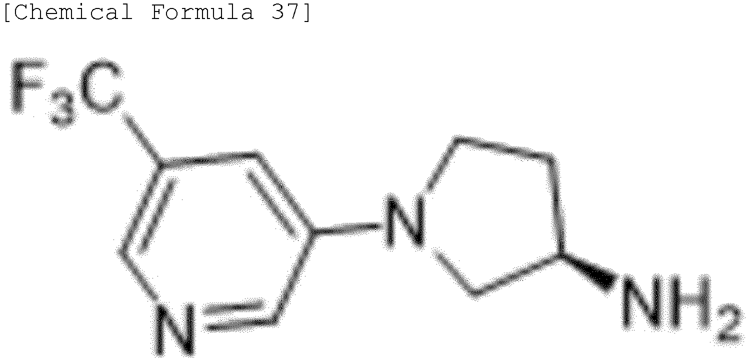

To tert-butyl (R)-(1-(5-(trifluoromethyl)pyridin-3-yl)pyrrolidin-3-yl)carbamate (116 mg, 0.35 mmol) synthesized in Reference Example 1-1, trifluoroacetic acid (2.0 mL) was added under ice cooling, and the mixture was stirred for 1 hour at room temperature. Under ice cooling, a saturated aqueous solution of sodium hydrogen carbonate and ethyl acetate were added to the reaction liquid, the mixture was stirred for a while, subsequently the organic layer was dried over anhydrous sodium sulfate, insoluble materials were filtered, subsequently the solvent was distilled off under reduced pressure, and thus the title compound (brown oily material, 60 mg, 74%) was obtained.
¹H NMR (CDCl3, 400MHz):δ=1.8-1.9(m,1H),2.2-2.3(m,1H), 3.08(dd,1H,J=4,10Hz),3.3-3.5(m,1H),3.5-3.6(m,2H),3.7-3.9(m,1H),6.92(dd,1H,J=2,2Hz),8.11(d,1H,J=3Hz),8.17(s,1H).

The 2H content is not observable.

### Reference Example 2

### Tert-butyl (R)-(1-(2-(trifluoromethyl)pyrimidin-5-yl)pyrrolidin-3-yl) carbamate

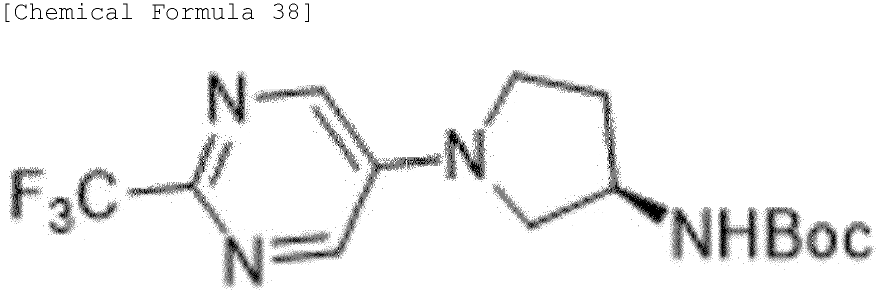

According to a technique similar to Reference Example 1-1, the title compound (white powder, 41 mg, 3.0%) was obtained using 5-bromo-2-(trifluoromethyl)pyrimidine (940 mg, 4.14 mmol) and tert-butyl (R)-pyrrolidin-3-ylcarbamate (950 mg, 5.10 mmol).
¹H NMR (CDCl₃, 400MHz) : δ=1.46 (s, 9H) , 2 .0-2. 1 (m, 1H) , 2.3-2.5(m,1H),3.28(dd,1H,J=4,10Hz),3.4-3.6(m,2H), 3.70(dd,1H,J=6,10Hz),4.3-4.5(m,1H),4.68(br s,1H), 8.10(s,2H).

### Reference Example 3

### Tert-butyl ((3S,4S)-4-hydroxy-1-(5-(trifluoromethyl)pyridin-3-yl)pyrrolidin-3-yl)carbamate

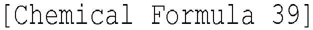

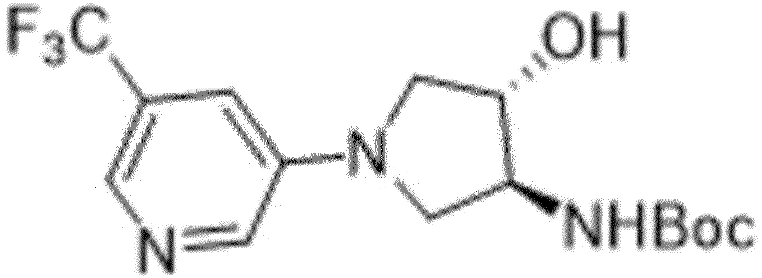

3-Bromo-5-(trifluoromethyl)pyridine (134 mg, 0.59 mmol), tert-butyl ((3S,4S)-4-hydroxypyrrolidin-3-yl)carbamate (100 mg, 0.49 mmol), tris(dibenzylidene acetone)dipalladium(0) (45 mg, 0.05 mmol), XantPhos (57 mg, 0.10 mmol), and potassium carbonate (137 mg, 0.99 mmol) were suspended in toluene (5.0 mL), and the suspension was stirred for 16 hours at 85°C under a nitrogen gas stream. To the reaction liquid that had been left to cool to room temperature, ethyl acetate was added, insoluble materials were filtered through Celite, and the solvent was distilled off under reduced pressure. A residue thus obtained was purified by silica gel column chromatography, and the title compound (white crystals, 106 mg, 62%) was obtained.
¹H NMR (CDCl₃, 400MHz) : δ=1 . 47 (s, 9H) , 3.24(dd,1H, J=5,10Hz), 3.3-3.4(m,2H) , 3.7-3.9(m,2H), 4.1-4.2(m,1H),4.4-4.5(m,1H),4.76(br s,1H), 6.94(dd,1H,J=2,2Hz),8.12(d,1H,J=3Hz),8.24(s,1H).

### Reference Example 4

### Tert-butyl (R)-(5-(5-(trifluoromethyl)pyridin-3-yl)-5-azaspiro[2.4]heptan-7-yl) carbamate

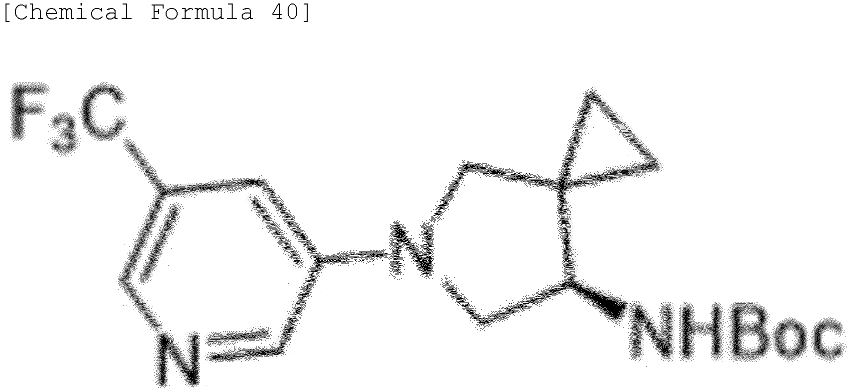

3-Bromo-5-(trifluoromethyl)pyridine (383 mg, 1.70 mmol), tert-butyl (R)-(5-azaspiro[2.4]heptan-7-yl)carbamate (300 mg, 1.41 mmol), tris(dibenzylidene acetone)dipalladium(0) (93 mg, 0.10 mmol), XantPhos (164 mg, 0.28 mmol), and sodium tert-butoxide (272 mg, 2.83 mmol) were suspended in toluene (14 mL), and the suspension was stirred for 16 hours at 85°C under a nitrogen gas stream. To the reaction liquid that had been left to cool to room temperature, ethyl acetate was added, insoluble materials were filtered through Celite, and the solvent was distilled off under reduced pressure. A residue thus obtained was purified by silica gel column chromatography (heptane : ethyl acetate) (concentration gradient: 0 to 100%), and the title compound (yellow crystals, 336 mg, 67%) was obtained.
¹H NMR (CDCl₃, 400MHz) : δ=0.7-0.8 (m, 2H) ,0.8-1.0 (m, 2H) , 1.44 (s, 9H), 3.10 (d, 1H, J=10Hz), 3.5-3.6 (m, 1H), 3.63 (d, 1H, J=9Hz), 3.7-3.8 (m, 1H), 3.8-3.9 (m, 1H), 4.78(br s, 1H), 6.9-7.0 (m, 1H), 8.09 (d, 1H, J=3Hz), 8.21 (s, 1H).

### Reference Example 5

### Tert-butyl (R)-(5-(6-(trifluoromethyl)pyridin-3-yl)-5-azaspiro[2.4]heptan-7-yl)carbamate

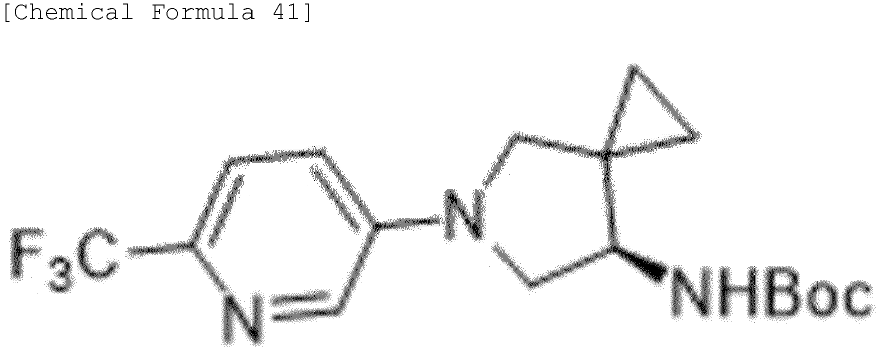

According to a technique similar to Reference Example 4, the title compound (yellow crystals, 271 mg, 80%) was obtained using tert-butyl (R)-(5-azaspiro[2.4]heptan-7-yl)carbamate (200 mg, 0.94 mmol) and 5-bromo-2-(trifluoromethyl)pyridine (255 mg, 1.13 mmol).
¹H NMR (CDCl₃, 400MHz) : δ=0.7-0 . 8 (m, 2H) ,0.8-1.0 (m, 2H) , 1.44 (s, 9H) ,3.11 (d, 1H, J=10Hz) ,3.5-3.6 (m, 1H) , 3.63 (d, 1H, J=9Hz), 3.7-3.8 (m, 1H), 3.8-3.9 (m, 1H), 4.76(br s, 1H), 6.80 (dd, 1H, J=3, 9Hz), 7.49 (d, 1H, J=9Hz), 7.98 (d, 1H,J=3Hz) .

### Reference Example 6

### Tert-butyl (R)-(5-(4-(trifluoromethyl)thiazol-2-yl)-5-azaspiro[2.4]heptan-7-yl) carbamate

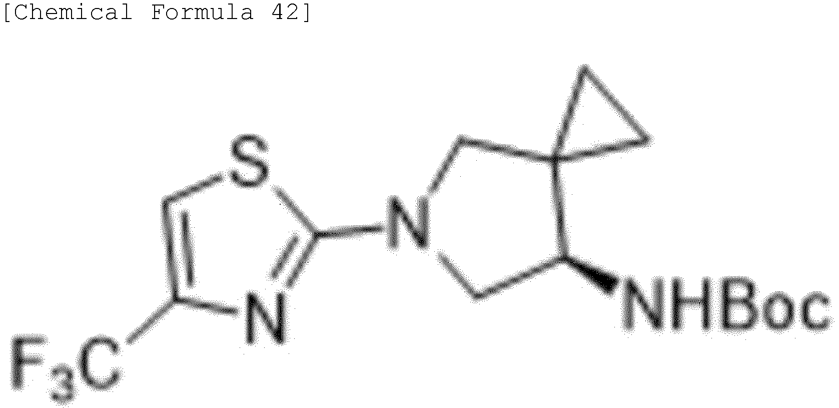

According to a technique similar to Reference Example 4, the title compound (yellow crystals, 192 mg, 56%) was obtained using tert-butyl (R)-(5-azaspiro[2.4]heptan-7-yl)carbamate (200 mg, 0.94 mmol) and 2-bromo-4-(trifluoromethyl)thiazole (255 mg, 1.10 mmol).
¹H NMR (CDCl₃, 400MHz) : δ=0.6-0.8 (m, 2H) ,0.8-1.0 (m, 2H) , 1.44 (s, 9H), 3.32 (d, 1H, J=10Hz), 3.57 (d, 1H, J=8Hz), 3.74 (d, 1H, J=11Hz), 3.8-3.9 (m, 2H), 4.77 (br s,1H), 6.93 (d, 1H, J=0.8Hz).

### Reference Example 7-1

### Tert-butyl (2S,4R)-4-(2-(4-cyclopropylphenyl)acetamido)-2-methylpyrrolidine-1-carboxylate

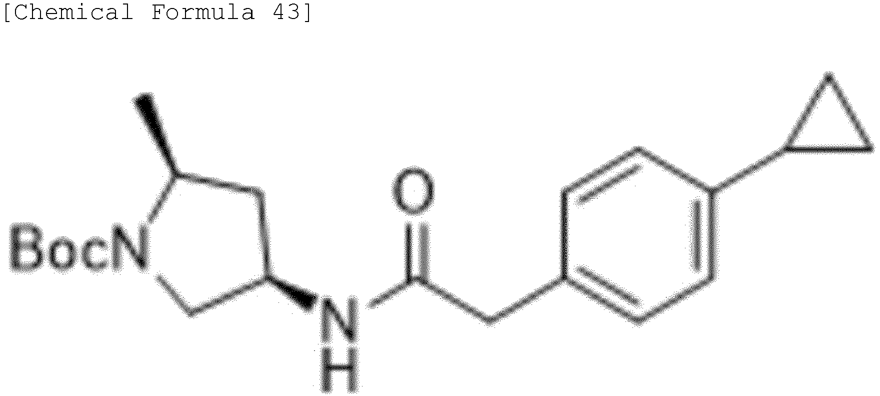

Tert-butyl (2S, 4R)-4-amino-2-methylpyrrolidine-1-carboxylate (250 mg, 1.25 mmol) and 2-(4-cyclopropylphenyl)acetic acid (264 mg, 1.50 mmol) synthesized in Reference Example 33-2 were dissolved in DMF (13 mL), subsequently DIPEA (691 µL, 4.01 mmol) and HATU (570 mg, 1.50 mmol) were added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (colorless oily material, 365 mg, 82%) was obtained.
¹H NMR (CDCl₃, 400MHz) : δ=0.6-0.7 (m, 2H) , 0.9-1.0 (m, 2H) , 1.1-1.2 (m, 3H), 1.43 (s, 9H), 1.7-1.9 (m, 3H), 3.0-3.1 (m, 1H), 3.51 (s, 2H), 3.5-3.7 (m, 1H), 3.7-4.0 (m, 1H), 4.4-4.6 (m, 1H), 5.31 (d,1H, J=7Hz) , 7.04(d,2H, J=8Hz) , 7.10(d,2H, J=8Hz) .

### Reference Example 7-2

### 2-(4-Cyclopropylphenyl)-N-((3R,5S)-5-methylpyrrolidin-3-yl)acetamide

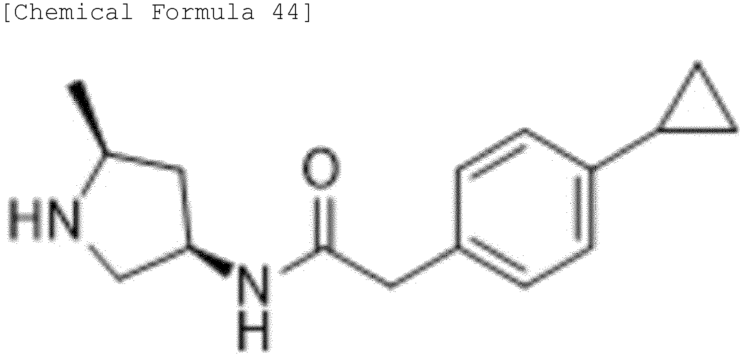

A 2 N hydrochloric acid-methanol solution (15 mL) was added to tert-butyl (2S,4R)-4-(2-(4-cyclopropylphenyl)acetamido)-2-methylpyrrolidine-1-carboxylate (365 mg, 1.02 mmol) synthesized in Reference Example 7-1 under ice cooling, the mixture was stirred for 2 hours at room temperature, and then the solvent was distilled off under reduced pressure. A residue thus obtained was purified by silica gel column chromatography (ethyl acetate : methanol) (concentration gradient: 0 to 40%), and the title compound (white crystals, 270 mg, 103%) was obtained.
¹H NMR (CDCl₃, 400MHz) : δ =0.6-0.7 (m, 2H) , 0. 9-1. 0 (m, 2H) , 1.13 (d, 3H, J=6Hz), 1.5-1.7 (m, 3H), 1.8-1.9 (m, 1H), 2.51 (dd, 1H, J=5, 12Hz), 3.1-3.2 (m, 1H), 3.38 (dd, 1H, J=7, 12Hz) , 3.50 (s, 2H) , 4.3-4.5 (m, 1H), 5.50(br s, 1H), 7.05 (d, 2H, J=8Hz), 7.12 (d, 2H, J=8Hz).

### Reference Example 8

### Tert-butyl (3-(5-(trifluoromethyl)pyridin-3-yl)-3-azabicyclo[3.1.0]hexan-1-yl)carbamate

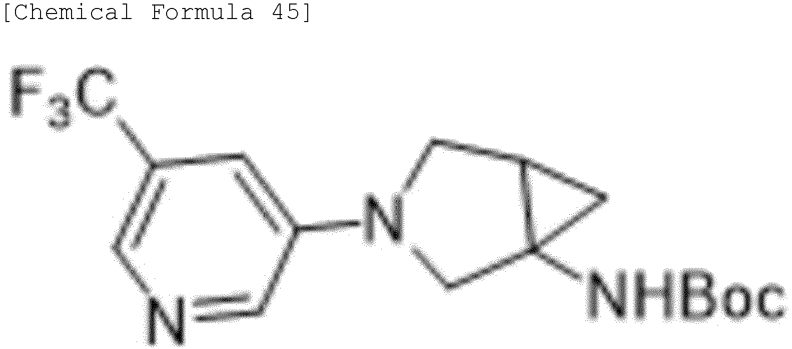

According to a technique similar to Reference Example 4, the title compound (colorless oily material, 103 mg, 23%) was obtained using tert-butyl (3-azabicyclo[3.1.0]hexan-1-yl)carbamate (250 mg, 1.26 mmol) and 3-bromo-5-(trifluoromethyl)pyridine (342 mg, 1.51 mmol) .
¹H NMR(CDCl₃, 400MHz) : δ=1.1-1.2 (m, 1H) , 1.2-1.3 (m, 1H) , 1.46 (s, 9H), 1.8-2.0 (m, 1H), 3.4-3.7 (m, 3H), 3.75 (d, 1H, J=8Hz), 5.13 (br s, 1H), 6.93 (dd, 1H, J=2,2Hz), 8.09 (d, 1H, J=2Hz), 8.20 (s, 1H) .

### Reference Example 9

### Tert-butyl (R)-(1-(6-(trifluoromethyl)pyridin-2-yl)pyrrolidin-3-yl) carbamate

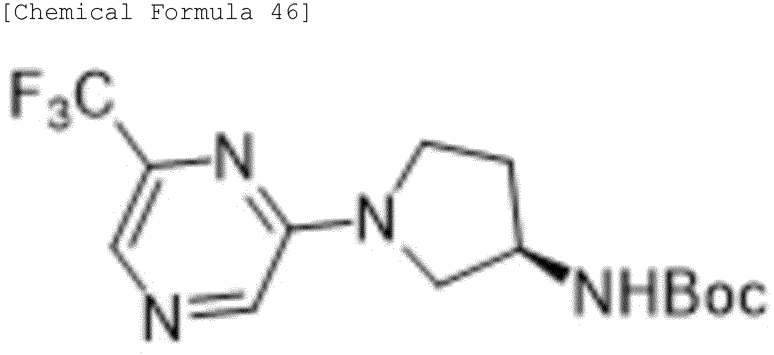

Tert-butyl (R)-pyrrolidin-3-ylcarbamate (250 mg, 1.34 mmol) was dissolved in DMF (13 mL), subsequently 2-chloro-6-(trifluoromethyl)pyrazine (294 mg, 1.61 mmol) and potassium carbonate (371 mg, 2.68 mmol) were added thereto, and the mixture was stirred for 20 hours at 120°C. To the reaction liquid that had been left to cool to room temperature, water was added, the mixture was stirred for a while, and then the mixture was extracted with a mixed liquid of chloroform and methanol. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (white crystals, 292 mg, 65%) was obtained.
¹H NMR (CDCl₃, 400MHz) : δ=1. 46 (s, 9H) , 1.9-2.1 (m, 1H) , 2.2-2.4 (m, 1H), 3.43 (dd, 1H, J=4,11Hz), 3.5-3.7 (m, 2H), 3.82 (dd, 1H, J=6, 11Hz), 4.39 (br s, 1H), 4.74 (d, 1H, J=7Hz), 8.03 (s, 1H), 8.14 (s, 1H).

### Reference Example 10

### Tert-butyl (R)-(1-(6-cyano-5-(trifluoromethyl)pyridin-3-yl)pyrrolidin-3-yl)carbamate

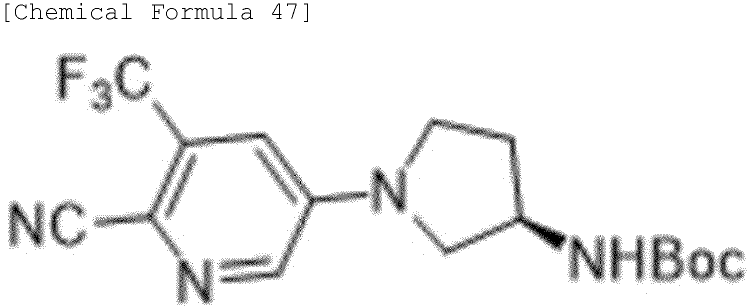

According to a technique similar to Reference Example 4, the title compound (yellow oily material, 139 mg, 30%) was obtained using tert-butyl (R)-pyrrolidin-3-ylcarbamate (200 mg, 1.07 mmol) and 5-bromo-3-(trifluoromethyl)picolinonitrile (323 mg, 1.29 mmol).
¹H NMR (CDCl₃, 400MHz) : δ=1.46 (s, 9H) , 2 .0-2.2 (m, 1H) , 2.3-2.5 (m, 1H), 3.33 (dd, 1H, J=5,10Hz), 3.4-3.6 (m, 2H), 3.7-3.8 (m, 1H), 4.3-4.5 (m, 1H), 4.84(br s,1H), 6.95 (d, 1H, J=3Hz), 8.07 (d, 1H, J=3Hz).

### Reference Example 11-1

### Methyl 1-benzyl-3-(2-(4-cyclopropylphenyl)acetamido)pyrrolidine-3-carboxylate

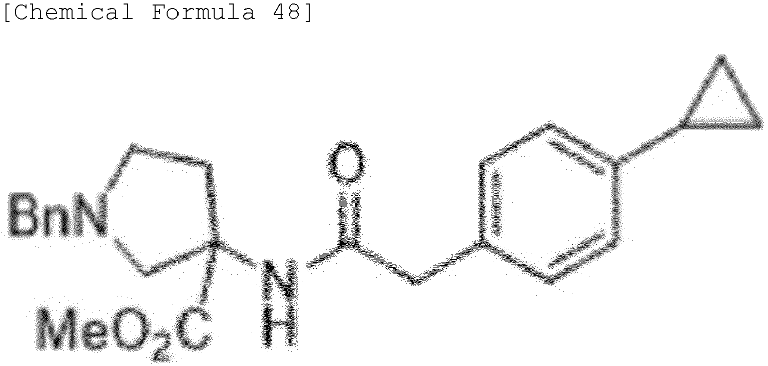

According to a technique similar to Reference Example 7-1, the title compound (white crystals, 362 mg, 86%) was obtained using methyl 3-(azanyl)-1-benzylpyrrolidine-3-carboxylate (250 mg, 1.07 mmol) and 2-(4-cyclopropylphenyl)acetic acid (226 mg, 1.28 mmol) synthesized in Reference Example 33-2.
¹H NMR (CDCl₃, 400MHz) : δ=0.6-0.8 (m, 2H) , 0.9-1.0 (m, 2H) , 1.8-2.0 (m, 2H), 2.4-2.5 (m, 1H), 2.5-2.7 (m, 1H), 2.75 (d, 1H, J=10Hz), 2.79 (d, 1H, J=10Hz), 2.8-2.9 (m, 1H), 3.50 (s, 2H), 3.54 (d, 1H, J=13Hz), 3.62(d, 1H, J=13Hz), 3.70 (s, 3H), 6.03 (br s, 1H), 7.0-7.1 (m, 2H), 7.1-7.2 (m, 2H), 7.2-7.3 (m, 5H).

### Reference Example 11-2

### Methyl 3-(2-(4-cyclopropylphenyl)acetamido)pyrrolidine-3-carboxylate

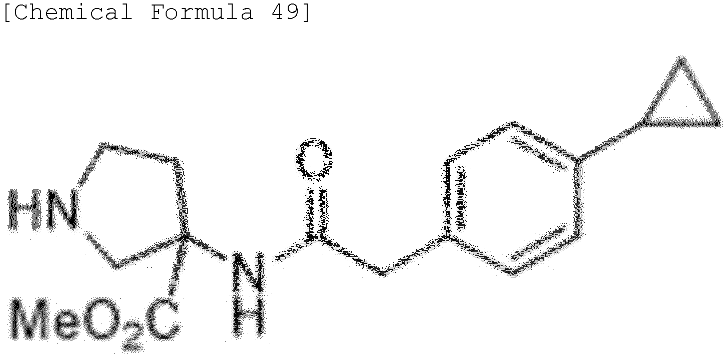

Methyl 1-benzyl-3-(2-(4-cyclopropylphenyl)acetamido)pyrrolidine-3-carboxylate (362 mg, 0.92 mmol) synthesized in Reference Example 11-1 was dissolved in methanol (20 mL), palladium-carbon (36 mg) was added thereto, and the mixture was stirred for 4 hours at room temperature under a hydrogen gas stream. Insoluble materials were filtered, subsequently the solvent was distilled off under reduced pressure, a residue thus obtained was purified by silica gel column chromatography (ethyl acetate : methanol) (concentration gradient: 0 to 40%), and the title compound (94 mg, 34%) was obtained.
¹H NMR (CDCl₃, 400MHz) : δ=0.6-0.7 (m, 2H) , 0.9-1.0 (m, 2H) , 1.8-1.9 (m, 1H), 2.0-2.2 (m, 2H), 2.2-2.3 (m, 1H), 2.9-3.0 (m, 1H), 3.10 (d, 1H, J=12Hz), 3.1-3.2 (m, 1H), 3.33 (d, 1H, J=12Hz), 3.51(s,2H),3.73(s,3H),6.35(br s, 1H), 7.0-7.1 (m, 2H), 7.1-7.2 (m, 2H).

### Reference Example 12-1

### Tert-butyl (R)-(1-(4-(trifluoromethyl)thiazol-2-yl)pyrrolidin-3-yl) carbamate

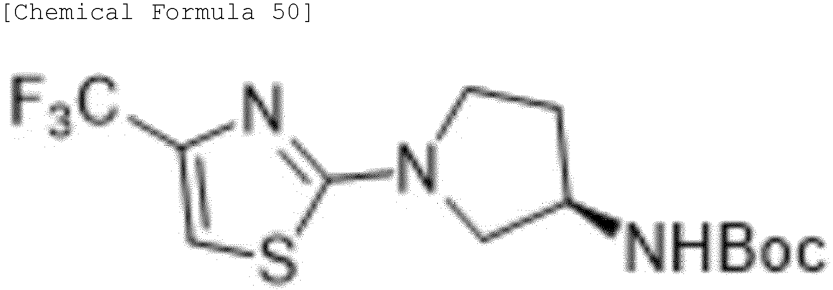

According to a technique similar to Reference Example 9, the title compound (white crystals, 706 mg, 90%) was obtained using 2-bromo-4-(trifluoromethyl)thiazole (540 mg, 2.33 mmol) and tert-butyl (R)-pyrrolidin-3-ylcarbamate (520 mg, 2.79 mmol) .
¹H NMR (CDCl₃, 400MHz) : δ=1. 45 (s, 9H) , 1.9-2. 1 (m, 1H) , 2.2-2.4 (m, 1H), 3.35(dd, 1H, J=4, 10Hz), 3.5-3.7 (m, 2H), 3.75 (dd, 1H, J=6, 10Hz), 4.38(br s, 1H), 4.68 (br s,1H), 6.92 (d, 1H, J=0.7Hz).

### Reference Example 12-2

### (R)-1-(4-(trifluoromethyl)thiazol-2-yl)pyrrolidin-3-amine

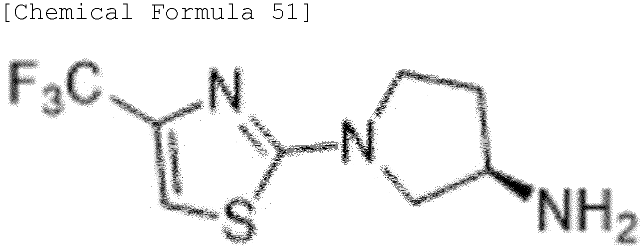

According to a technique similar to Reference Example 7-2, the title compound (white crystals, 480 mg, 97%) was obtained using tert-butyl (R)-(1-(4-(trifluoromethyl)thiazol-2-yl)pyrrolidin-3-yl)carbamat e (706 mg, 2.09 mmol) synthesized in Reference Example 12-1.
¹H NMR (CDCl₃, 400MHz) : δ=1.34 (br s, 2H) , 1.8-1.9 (m, 1H), 2.2-2.3 (m, 1H) , 3.23 (dd, 1H, J=4, 10Hz) , 3.5-3.6 (m, 1H), 3.6-3.7 (m, 2H), 3.7-3.9 (m, 1H), 6.90 (br s, 1H).

### Reference Example 13

### Tert-butyl (R)-(1-(6-methyl-5-(trifluoromethyl)pyridin-3-yl)pyrrolidin-3-yl)carbamate

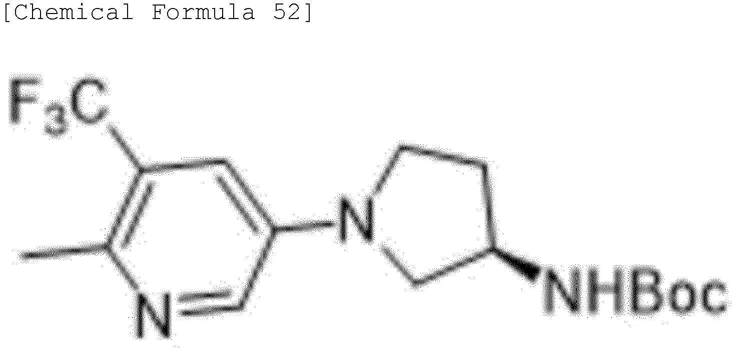

According to a technique similar to Reference Example 4, the title compound (white crystals, 22 mg, 4.9%) was obtained using 5-bromo-2-methyl-3-(trifluoromethyl)pyridine (310 mg, 1.29 mmol) and tert-butyl (R)-pyrrolidin-3-ylcarbamate (289 mg, 1.55 mmol).
¹H NMR (CDCl₃, 400MHz) : δ=1. 46 (s, 9H) , 1.9-2.1 (m, 1H) , 2.2-2.4 (m, 1H) , 2.5-2.6 (m, 3H) , 3.20 (dd, 1H, J=4, 10Hz), 3.3-3.4 (m, 1H), 3.4-3.5 (m, 1H), 3.61 (dd, 1H, J=6,10Hz), 4.40(br s, 1H), 4.70 (br s, 1H), 7.00 (d, 1H, J=3Hz), 7.98 (d, 1H,J=3Hz) .

### Reference Example 14-1

### Tert-butyl (R)-(1-(5-(2-hydroxypropan-2-yl)pyridin-3-yl)pyrrolidin-3-yl)-carbamate

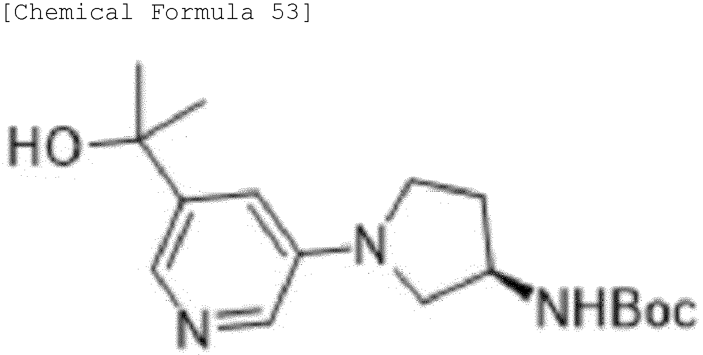

According to a technique similar to Reference Example 3, the title compound (brown amorphous, 187 mg, 60%) was obtained using 2-(5-bromopyridin-3-yl)propan-2-ol (210 mg, 0.97 mmol) and tert-butyl (R)-pyrrolidin-3-ylcarbamate (217 mg, 1.17 mmol).
¹H NMR (CDCl₃, 400MHz) : δ=1. 46 (s, 9H) , 1 .58 (s, 6H) , 1.9-2.0 (m, 1H), 2.2-2.4 (m, 1H), 2.80 (br s,1H), 3.17 (dd, 1H, J=4, 10Hz), 3.3-3.4 (m, 1H), 3.4-3.5 (m, 1H), 3.56 (dd, 1H, J=6, 10Hz), 4.36(br s, 1H), 4.96 (br s,1H), 6.99 (dd, 1H, J=2,2Hz), 7.78 (d, 1H, J=3Hz), 8.01 (d, 1H, J=2Hz).

### Reference Example 14-2

### (R)-2-(5-(3-aminopyrrolidin-1-yl)pyridin-3-yl)propan-2-ol

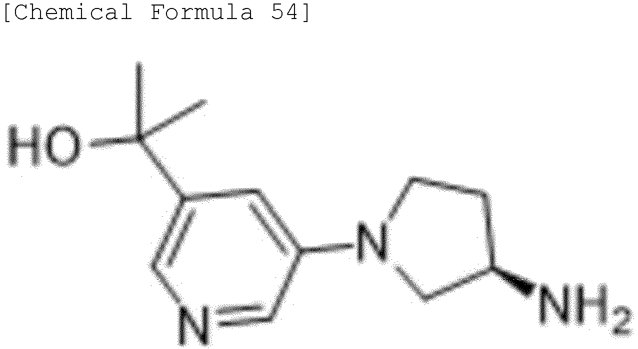

According to a technique similar to Reference Example 7-2, the title compound (brown oily material, 114 mg, 88%) was obtained using tert-butyl (R)-(1-(5-(2-hydroxypropan-2-yl)pyridin-3-yl)pyrrolidin-3-yl)-carbamate (187 mg, 0.58 mmol) synthesized in Reference Example 14-1.
¹H NMR (CDCl₃, 400MHz) : δ=1. 59 (s, 6H) , 1.7-1.9 (m, 1H) , 2.1-2.3 (m, 1H) , 3.03 (dd, 1H, J=4, 10Hz) , 3.3-3.4 (m, 1H), 3.4-3.6 (m, 4H) , 3.7-3.8 (m, 1H) , 6.99 (dd, 1H, J=2, 2Hz), 7.80 (d, 1H,J=3Hz) , 8.00 (d, 1H,J=2Hz) . The 1H content is not observable.

### Reference Example 15

### Tert-butyl (R)-(1-(8-(trifluoromethyl)imidazo[1,2-a]pyridin-6-yl)pyrrolidin-3-yl)-carbamate

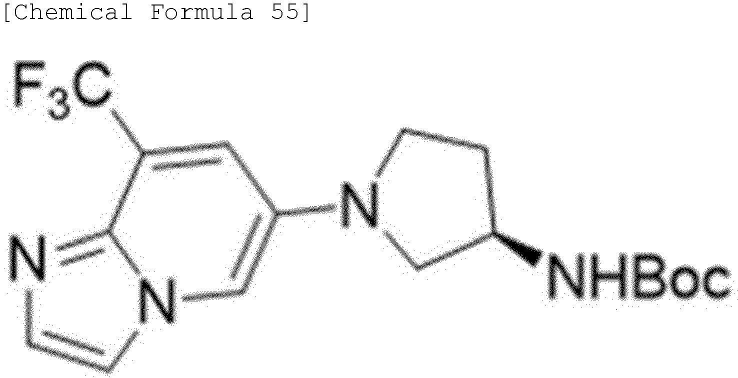

According to a technique similar to Reference Example 4, the title compound (brown oily material, 90 mg, 26%) was obtained using 6-bromo-8-(trifluoromethyl)-imidazo[1,2-a]pyridine (250 mg, 0.94 mmol) and tert-butyl (R)-pyrrolidin-3-ylcarbamate (211 mg, 1.13 mmol).
¹H NMR (CDCl₃, 400MHz) : δ=1. 46 (s, 9H) , 1.9-2.1 (m, 1H) , 2.3-2.4 (m, 1H), 3.17 (dd, 1H, J=4, 9Hz), 3.2-3.4 (m, 1H), 3.4-3.5 (m, 1H), 3.5-3.6 (m, 1H), 4.40 (br s,1H), 4.79(br s, 1H), 7.19 (d, 1H, J=0.8Hz), 7.40 (d, 1H, J=1Hz), 7.56 (d, 1H, J=0. 8Hz) ,7.64 (d, 1H, J=1Hz).

### Reference Example 16

### Ethyl 2-(4-(1,1-dioxidethiomorpholino)-phenyl)acetate

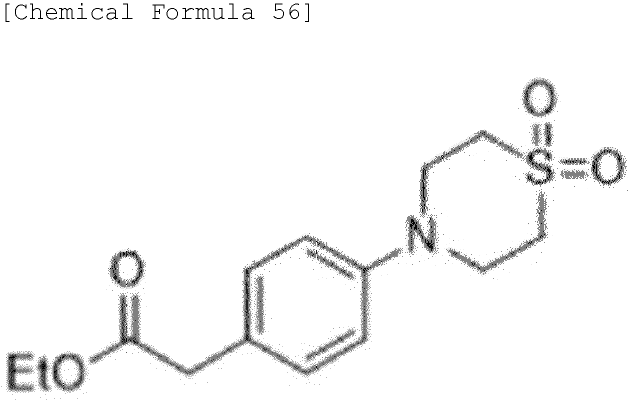

According to a technique similar to Reference Example 3, the title compound (pale yellow oily material, 30 mg, 8.2%) was obtained using ethyl 2-(4-bromophenyl)-acetate (300 mg, 1.23 mmol) and 1,1-dioxide thiomorpholine (334 mg, 2.47 mmol).
¹H NMR (CDCl₃, 400MHz) : δ=1 .26 (t, 3H, J=7Hz) , 3.0-3.1 (m,4H), 3.54 (s, 2H), 3.8-3.9(m, 4H), 4.12 (q, 2H, J=7Hz), 6.8-6.9 (m, 2H), 7.2-7.3 (m, 2H).

### Reference Example 17

### Ethyl 2-(4-(trimethylsilyl)phenyl)acetate

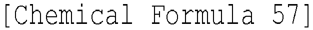

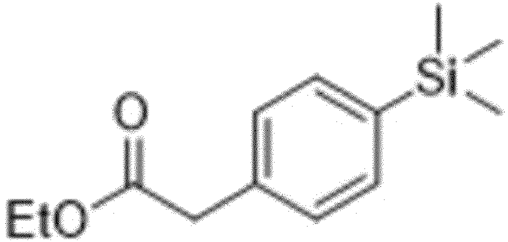

In a nitrogen atmosphere, a small amount of iodine was introduced into a round-bottom flask containing magnesium (168 mg, 6.98 mmol) and THF (1.0 mL), subsequently ethyl 2-(4-bromophenyl)acetate (500 mg, 2.06 mmol) dissolved in THF (3.4 mL) was added thereto, and the mixture was heated to reflux for 30 minutes. The reaction liquid was left to cool to room temperature, chlorotrimethylsilane (520 µL, 4.11 mmol) was added thereto, and the mixture was heated to reflux for 2 hours. To the reaction liquid that had been left to cool to room temperature, a saturated aqueous solution of ammonium chloride was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (colorless oily material, 30 mg, 6.2%) was obtained.
¹H
NMR (CD₃OD, 400MHz) : δ=0.25 (s, 9H), 1 .24 (t, 3H, J=7Hz), 3.59 (s, 2H) , 4.10(q,2H,J=7Hz),7.2-7.3(m,2H),7.4-7.5(m,2H).

### Reference Example 18

### Tert-butyl (3S,4S)-3-(2-(4-cyclopropylphenyl)-acetamido)-4-fluoropyrrolidine-1-carboxylate

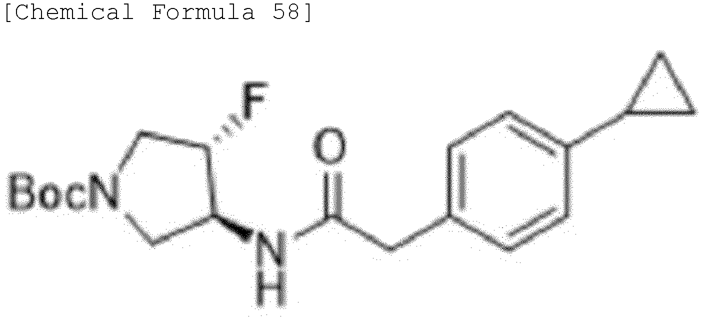

According to a technique similar to Reference Example 7-1, the title compound (yellow powder, 369 mg, 83%) was obtained using tert-butyl (3S,4S)-3-amino-4-fluoropyrrolidine-1-carboxylate (250 mg, 1.22 mmol) and 2-(4-cyclopropylphenyl)acetic acid (259 mg, 1.47 mmol) synthesized in Reference Example 33-2.
¹H NMR (CDCl₃, 400MHz) : δ=0.6-0.7 (m, 2H) , 0.9-1.0 (m, 2H) , 1.45 (s, 9H), 1.8-1.9 (m, 1H), 3.1-3.7 (m, 6H), 4.43 (br s,1H), 4.8-5.1 (m, 1H), 5.32 (br s, 1H), 7.0-7.1 (m, 2H), 7.1-7.2 (m, 2H).

### Reference Example 19

### Tert-butyl 3-cyano-3-(2-(4-cyclopropylphenyl)-acetamido)pyrrolidine-1-carboxylate

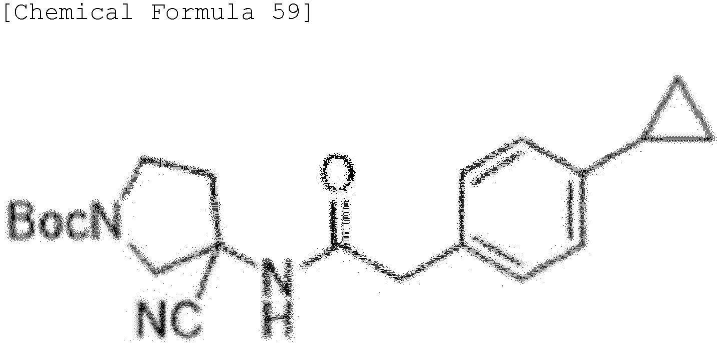

According to a technique similar to Reference Example 7-1, the title compound (yellow oily material, 454 mg, 86%) was obtained using tert-butyl 3-amino-3-cyanopyrrolidine-1-carboxylate (300 mg, 1.42 mmol) and 2-(4-cyclopropylphenyl)acetic acid (300 mg, 1.70 mmol) synthesized in Reference Example 33-2.
¹H NMR (CDCl₃, 400MHz) : δ=0.6-0.7 (m, 2H) , 0.9-1.0 (m, 2H) , 1.44 (s,9H), 1.8-1.9 (m, 1H), 2.2-2.6 (m, 2H), 3.3-3.4 (m, 1H), 3.4-3.6 (m, 4H), 3.9-4.0 (m, 1H), 6.3-6.4 (m, 1H), 7.04 (d, 2H, J=8Hz), 7. 10 (d, 2H, J=8Hz) .

### Reference Example 20-1

### Methyl 1-benzyl-3-((tert-butoxycarbonyl)-amino)pyrrolidine-3-carboxylate

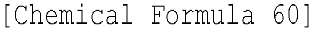

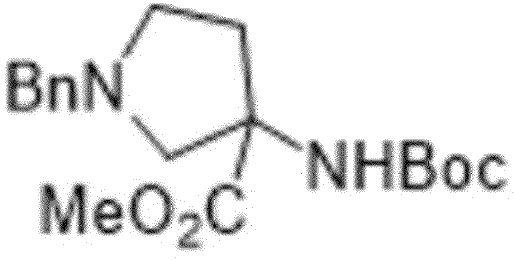

Methyl 3-amino-1-benzylpyrrolidine-3-carboxylate (500 mg, 2.13 mmol) , DIPEA (788 µL, 4.57 mmol), and Boc anhydride (931 mg, 4.27 mmol) were dissolved in chloroform (21 mL), and the solution was stirred for 16 hours at room temperature. Water was added to the reaction liquid, the mixture was stirred for a while, and then the mixture was extracted with chloroform. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (colorless oily material, 522 mg, 73%) was obtained.
¹H NMR(CDCl₃, 400MHz) : δ=1. 42 (s, 9H) , 1.9-2.0 (m, 1H), 2.5-2.7 (m, 2H) , 2.7-3.0 (m, 3H) , 3.61 (d, 1H, J=13Hz), 3.68 (d, 1H, J=13Hz), 3.74 (s, 3H), 5.12 (br s, 1H), 7.2-7.4 (m, 5H).

### Reference Example 20-2

### Methyl 3-((tert-butoxycarbonyl)amino)-1-(5-(trifluoromethyl)pyridin-3-yl) pyrrolidine-3-carboxylat e

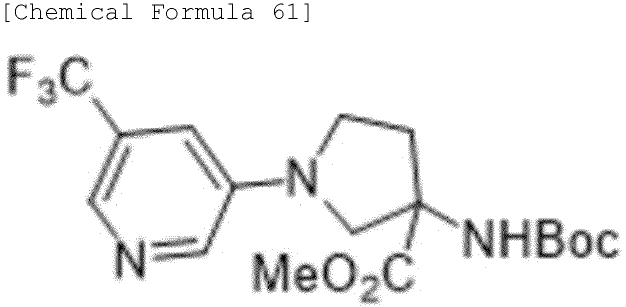

According to a technique similar to Reference Example 11-2, a crude form of methyl 3-((tert-butoxycarbonyl)amino)pyrrolidine-3-carboxylate (colorless oily material, 402 mg) was obtained using methyl 1-benzyl-3-((tert-butoxycarbonyl)amino)pyrrolidine-3-carboxylate (522 mg, 1.56 mmol) synthesized in Example 20-1. According to a technique similar to Reference Example 4, the title compound (34 mg, 5.8%) was obtained using the crude form of methyl 3-((tert-butoxycarbonyl)amino)pyrrolidine-3-carboxylate (402 mg) thus obtained and (3-bromo-5-(trifluoromethyl)pyridine (423 mg, 1.87 mmol).
¹H NMR (CDCl₃, 400MHz) : δ=1. 45 (s, 9H) , 2 .3-2. 5 (m, 1H) , 2.5- 2.6 (m, 1H), 3.5-3.6 (m, 2H), 3.6-3.7 (m, 1H) , 3.79 (s, 3H) , 4.01 (d, 1H, J=10Hz), 5.17 (br s, 1H), 6.9-7.0 (m, 1H), 8.11 (d, 1H, J=3Hz), 8.22 (s, 1H).

### Reference Example 21-1

### Tert-butyl 3-cyano-3-(2-(4-(trifluoromethyl)phenyl)acetamido)pyrrolidine-1-carboxylate

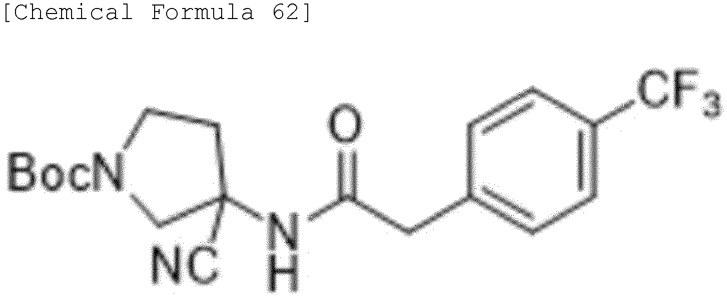

According to a technique similar to Reference Example 7-1, the title compound (white powder, 352 mg, 62%) was obtained using tert-butyl 3-amino-3-cyanopyrrolidine-1-carboxylate (300 mg, 1.42 mmol) and 2-(4-(trifluoromethyl)-phenyl)acetic acid (348 mg, 1.70 mmol).
¹H NMR (CDCl₃, 400MHz) : δ=1. 45 (s, 9H) , 2 .2-2. 6 (m, 2H) , 3.3-3.7 (m, 3H), 3.68 (s, 2H), 3.9-4.1 (m, 1H) , 5.7-5.8 (m, 1H) , 7.40 (d, 2H, J=8Hz) ,7.64 (d, 2H, J=8Hz) .

### Reference Example 21-2

### Methyl 3-(2-(4-(trifluoromethyl)phenyl)-acetamido)pyrrolidine-3-carboxylate

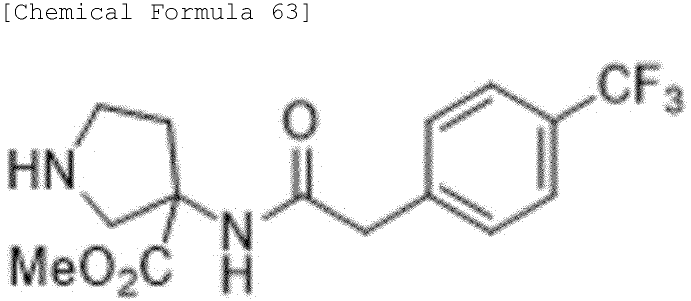

According to a technique similar to Reference Example 7-2, the title compound (white powder, 213 mg, 73%) was obtained using tert-butyl 3-cyano-3-(2-(4-(trifluoromethyl)phenyl)acetamido)pyrrolidine-1-carboxylate (352 mg, 0.89 mmol) synthesized in Reference Example 21-1.
¹H NMR (CDCl₃, 400MHz) : δ=2.0-2.1 (m, 1H) , 2.2-2.4 (m, 2H) , 3.0-3.1 (m, 1H), 3.14 (d, 1H, J=12Hz), 3.1-3.2 (m, 1H), 3.35 (d, 1H, J=12Hz) , 3.61 (s, 2H) , 3.75 (s, 3H) , 6.52 (br s, 1H) , 7.40 (d, 2H, J=8Hz), 7.61 (d, 2H, J=8Hz).

### Reference Example 22-1

### Dimethyl (1S,2S)-cyclopropane-1,2-dicarboxylate

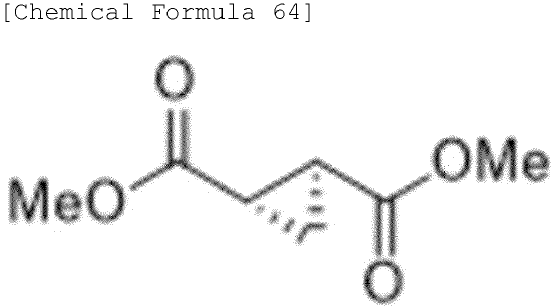

(1S,2S)-cyclopropane-1,2-dicarboxylic acid (2.0 g, 15.4 mmol) was dissolved in methanol (30 mL), thionyl chloride (2.79 mL, 38.4 mmol) and a small amount of DMF were added thereto, the mixture was stirred for 4 hours at room temperature, and then the solvent was distilled off under reduced pressure. Water and ethyl acetate were added to a residue thus obtained, the mixture was stirred for a while, subsequently an organic layer thus separated was washed with a saturated aqueous solution of sodium hydrogen carbonate, and the organic layer was dried over anhydrous sodium sulfate. Insoluble materials were filtered, subsequently the solvent was distilled off under reduced pressure, and the title compound (brown oily material, 2.09 g, 86%) was obtained.
¹H NMR (CDCl₃, 400MHz) : δ=1. 4-1. 5 (m, 2H) , 2. 1-2.2 (m, 2H) , 3.71 (s, 6H).

### Reference Example 22-2

### (1S, 2S)-2-(methoxycarbonyl)cyclopropane-1-carboxylic acid

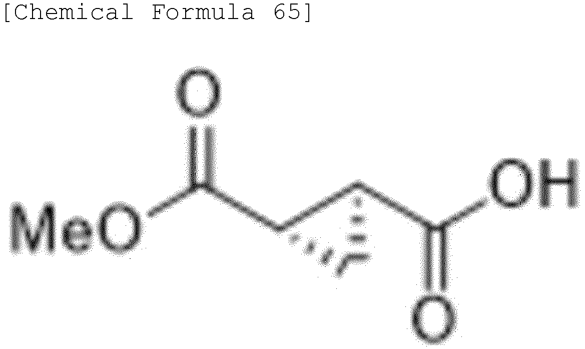

Dimethyl (1S,2S)-cyclopropane-1,2-dicarboxylate (2.09 g, 13.2 mmol) synthesized in Reference Example 22-1 was dissolved in methanol (13 mL) and water (2.0 mL), a 2 N aqueous solution of sodium hydroxide (6.28 mL) was added thereto, the mixture was stirred for 16 hours at room temperature, and then the solvent was distilled off under reduced pressure. Water was added to a residue thus obtained, the mixture was washed with chloroform, a 2 N aqueous solution of hydrochloric acid was added thereto to adjust the pH of the aqueous layer to 2 to 3, and then the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby the title compound (brown oily material, 1.45 g, 76%) was obtained.
¹H NMR(CDCl₃, 400MHz) : δ=1.4-1.6 (m, 2H), 2.1-2.2 (m,1H), 2.2-2.3 (m, 1H), 3.72 (s, 3H). The 1H content is not observable.

### Reference Example 22-3

### Methyl (1S,2S)-2-((2-bromo-4-fluorophenyl)carbamoyl)cyclopropane-1-carboxylate

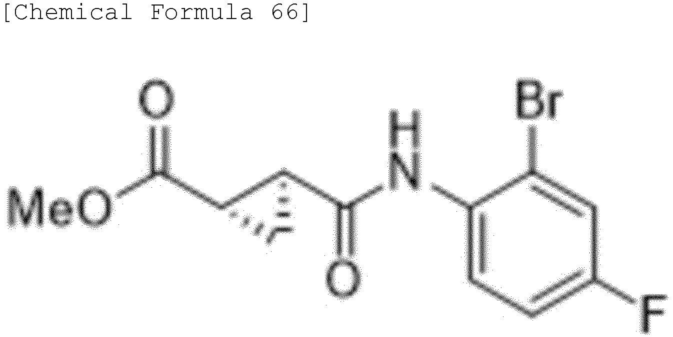

According to a technique similar to Reference Example 7-1, the title compound (515 mg, 65%) was obtained using (1S, 2S)-2-(methoxycarbonyl)cyclopropane-1-carboxylic acid (363 mg, 2.52 mmol) synthesized in Reference Example 22-2 and 2-bromo-4-fluoroaniline (574 mg, 3.02 mmol).
¹H NMR (CDCl₃, 400MHz) : δ=1. 4-1. 5 (m, 1H) , 1.5-1.6 (m, 1H), 2.1-2.2 (m, 1H), 2.2-2.3 (m, 1H), 3.75 (s, 3H), 7.05 (ddd, 1H, J=6,8,9 Hz), 7.31 (dd, 1H, J=3,8Hz), 7.76 (br s, 1H), 8.28 (dd, 1H, J=6,9Hz).

### Reference Example 22-4

### Methyl (1S,2S)-2-(6-fluorobenzo[d]oxazol-2-yl)cyclopropane-1-carboxylate

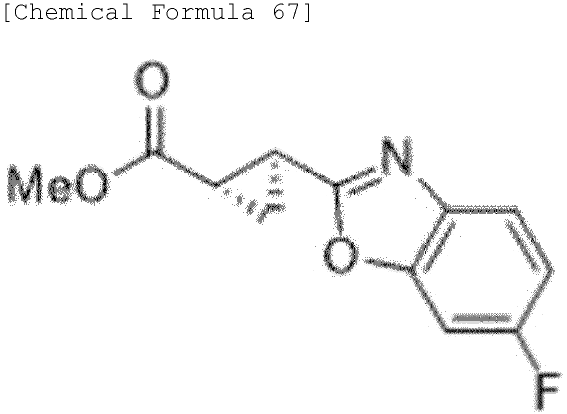

Methyl (1S,2S)-2-((2-bromo-4-fluorophenyl)carbamoyl)cyclopropane-1-carboxylate (515 mg, 1.63 mmol) synthesized in Reference Example 22-3, copper(I) iodide (31 mg, 0.16 mmol), 1,10-phenanthroline (59 mg, 0.33 mmol), and cesium carbonate (796 mg, 2.44 mmol) were suspended in DME (5.0 mL), and the suspension was heated to reflux for 16 hours under a nitrogen gas stream. The reaction liquid that had been left to cool to room temperature was filtered through Celite, and the solvent was distilled off under reduced pressure. A residue thus obtained was purified by silica gel column chromatography (heptane : ethyl acetate) (concentration gradient: 0 to 100%), and the title compound (pale yellow crystals, 226 mg, 59%) was obtained.
¹H NMR(CDCl₃, 400MHz) : δ=1.7-1.8 (m, 2H) , 2.4-2.5 (m, 1H) , 2.7-2.8 (m, 1H), 3.75 (s, 3H), 7.0-7.1 (m, 1H), 7.19 (dd, 1H, J=2, 8Hz), 7.55 (dd, 1H, J=5, 9Hz).

### Reference Example 23-1

### Ethyl 2-(4-morpholinophenyl)acetate

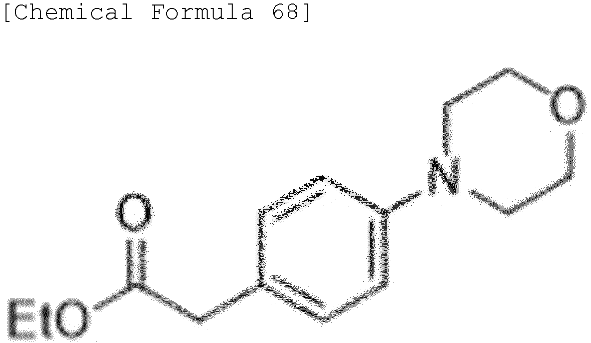

Ethyl 2-(4-bromophenyl)acetate (1.28 g, 5.27 mmol), morpholine (917 µL, 10.5 mmol), palladium acetate (118 mg, 0.53 mmol), tert-butyl XPhos (447 mg, 1.05 mmol), and cesium carbonate (3.43 g, 10.5 mmol) were suspended in toluene (25 mL), and the suspension was stirred for 16 hours at 110°C under a nitrogen gas stream. The reaction liquid that had been left to cool to room temperature was filtered through Celite, and the solvent was distilled off under reduced pressure. A residue thus obtained was purified by silica gel column chromatography (heptane : ethyl acetate) (concentration gradient: 0 to 100%), and the title compound (yellow crystals, 760 mg, 58%) was obtained.
¹H NMR (CDCl₃, 400MHz) : δ=1 .25 (t, 3H, J=7Hz) , 3.1-3.2 (m, 4H), 3.53 (s, 2H), 3.8-3.9 (m, 4H), 4.14 (q, 2H, J=7Hz), 6.8-6.9 (m, 2H), 7.1-7.2 (m, 2H).

### Reference Example 23-2

### 2-(4-Morpholinophenyl)acetic acid

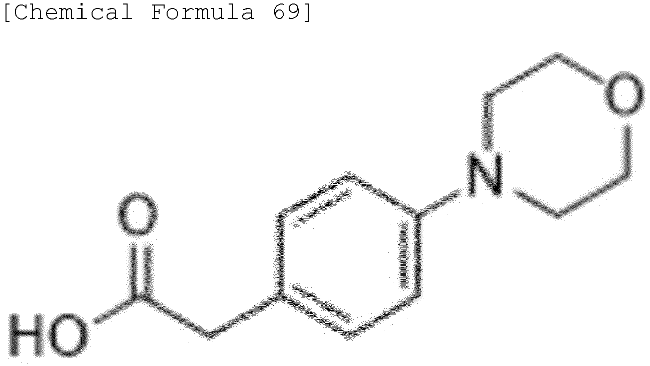

Ethyl 2-(4-morpholinophenyl)acetate (760 mg, 3.05 mmol) synthesized in Reference Example 23-1 was dissolved in methanol (5.0 mL), a 2 N aqueous solution of sodium hydroxide (4.6 mL) was added thereto, the mixture was stirred for 16 hours at room temperature, and the solvent was distilled off under reduced pressure. Water was added to a residue thus obtained, the mixture was washed with chloroform, a 2 N aqueous solution of hydrochloric acid was added thereto to neutralize the mixture, and then the solvent was distilled off under reduced pressure. A mixed liquid of chloroform and methanol was added to a residue thus obtained, the mixture was stirred for a while, insoluble materials were filtered, subsequently the solvent was distilled off under reduced pressure, and a crude form of the title compound (white powder, 733 mg) was obtained.
¹H NMR (CD₃OD, 400MHz) : δ=3.0-3.1 (m, 4H) , 3.38 (s, 2H) , 3.8-3.9 (m,4H), 6.8-6.9 (m, 2H), 7.2-7.3 (m, 2H). The 1H content is not observable.

### Reference Example 24

### Tert-butyl 3-(2-(4-cyclopropylphenyl)acetamido)-3-ethylpyrrolidine-1-carboxylate

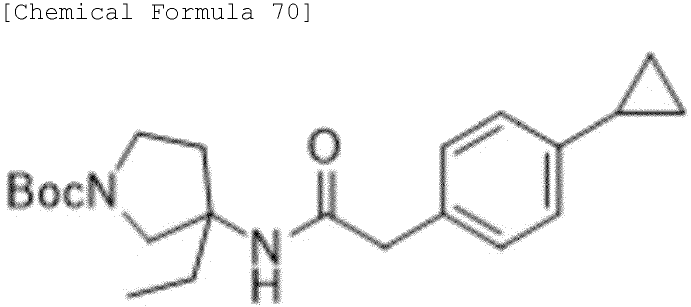

According to a technique similar to Reference Example 7-1, the title compound (white crystals, 782 mg, 90%) was obtained using tert-butyl 3-amino-3-ethylpyrrolidine-1-carboxylate (500 mg, 2.33 mmol) and 2-(4-cyclopropylphenyl)acetic acid (493 mg, 2.80 mmol) synthesized in Reference Example 33-2.
¹H NMR (CDCl₃, 400MHz) : δ=0.6-0.7 (m, 2H) , 0.7-0.8 (m, 3H) , 0.9-1.0(m,2H),1.44(s,9H),1.5-2.5(m,5H),3.1-3.6(m,6H), 5.14 (d, 1H, J=14Hz), 7.0-7.1 (m, 2H), 7.1-7.2 (m, 2H).

### Reference Example 25-1

### Methyl (1S,2S)-2-((2-bromophenyl)carbamoyl)cyclopropane-1-carboxylate

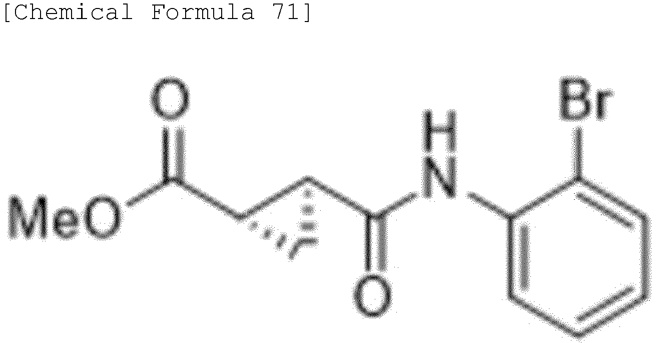

According to a technique similar to Reference Example 22-1, a crude form of dimethyl (1S,2S)-cyclopropane-1,2-dicarboxylate (brown oily material, 695 mg) was obtained using (1S,2S)-cyclopropane-1,2-dicarboxylic acid (500 mg, 3.84 mmol). According to a technique similar to Reference Example 22-2, (1S,2S)-2-(methoxycarbonyl)cyclopropane-1-carboxylic acid (427 mg, 77%) was obtained using the crude form of dimethyl (1S,2S)-cyclopropane-1,2-dicarboxylate (695 mg). According to a technique similar to Reference Example 7-1, the title compound (yellow powder, 397 mg, 45%) was obtained using (1S, 2S)-2-(methoxycarbonyl)cyclopropane-1-carboxylic acid (427 mg, 2.96 mmol) thus obtained and 2-bromoaniline (612 mg, 3.56 mmol).
¹H NMR (CDCl₃, 400MHz) : δ=1. 4-1. 5 (m, 1H) , 1.5-1.6 (m, 1H) , 2.1-2.2 (m, 1H), 2.2-2.3 (m, 1H), 3.74 (s, 3H), 6.9-7.0 (m, 1H), 7.2-7.3 (m, 1H), 7.54 (dd, 1H, J=1, 8Hz), 7.93 (br s,1H), 8.30(d, 1H, J=8Hz) .

### Reference Example 25-2

### Methyl (1S,2S)-2-(benzo[d]oxazol-2-yl)cyclopropane-1-carboxylate

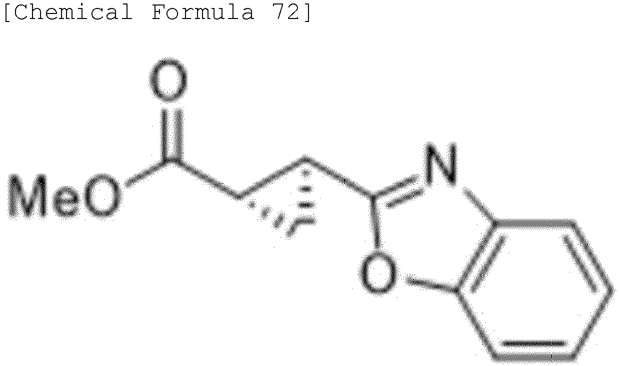

According to a technique similar to Reference Example 22-4, the title compound (95 mg, 33%) was obtained using methyl (1S,2S)-2-((2-bromophenyl)carbamoyl)cyclopropane-1-carboxylate (397 mg, 1.33 mmol) synthesized in Reference Example 25-1.
¹H NMR (CDCl₃, 400MHz) : δ=1.7-1.8 (m, 2H) , 2.4-2.5 (m, 1H) , 2.7-2.8 (m, 1H), 3.75 (s, 3H), 7.2-7.3 (m, 2H), 7.4-7.5 (m, 1H), 7.6-7.7 (m, 1H) .

### Reference Example 26-1

### Methyl (1S,2S)-2-((2-bromo-4,5-difluorophenyl)-carbamoyl)cyclopropane-1-carboxylate

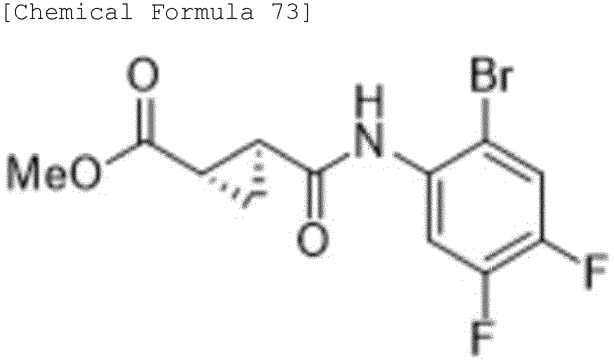

According to a technique similar to Reference Example 7-1, the title compound (313 mg, 39%) was obtained using (1S, 2S)-2-(methoxycarbonyl)cyclopropane-1-carboxylic acid (344 mg, 2.39 mmol) synthesized in Reference Example 22-2 and 2-bromo-4,5-difluoroaniline (596 mg, 2.87 mmol).
¹H NMR(CDCl₃, 400MHz) : δ=1.4-1.6 (m, 2H) , 2.1-2.2 (m, 1H) , 2.3-2.4 (m, 1H), 3.75 (s, 3H), 7.3-7.4 (m, 1H), 7.79 (br s,1H), 8.3-8.4 (m, 1H) .

### Reference Example 26-2

### Methyl (1S,2S)-2-(5,6-difluorobenzo[d]oxazol-2-yl)cyclopropane-1-carboxylate

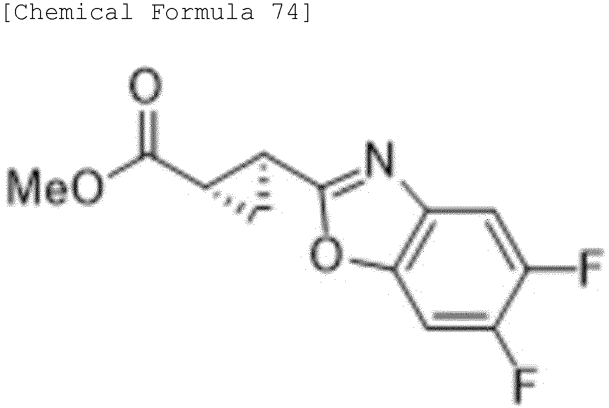

According to a technique similar to Reference Example 22-4, the title compound (pale yellow oily material, 68 mg, 29%) was obtained using methyl (1S,2S)-2-((2-bromo-4,5-difluorophenyl) carbamoyl)cyclopropane-1-carboxylate (313 mg, 0.94 mmol) synthesized in Reference Example 26-1.
¹H NMR(CDCl₃, 400MHz) : δ=1.7-1.8 (m, 2H) , 2.4-2.5 (m, 1H) , 2.7-2.8 (m, 1H), 3.75 (s, 3H), 7.3-7.4 (m, 1H), 7.4-7.5 (m, 1H).

### Reference Example 27

### Ethyl 2-(4-(2-oxa-6-azaspiro[3.3]heptan-6-yl)phenyl)acetate

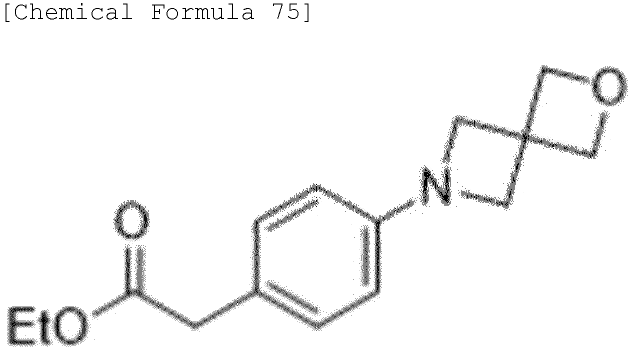

Ethyl 2-(4-bromophenyl)acetate (1.0 g, 4.11 mmol), 2-oxa-6-azaspiro[3.3]heptane (816 mg, 8.23 mmol), palladium acetate (92 mg, 0.41 mmol), XPhos (270 mg, 0.57 mmol), and cesium carbonate (2.68 g, 8.23 mmol) were suspended in toluene (30 mL), and the suspension was stirred for 16 hours at 110°C under a nitrogen gas stream. The suspension was left to cool to room temperature, insoluble materials were filtered through Celite, and the solvent was distilled off under reduced pressure. A residue thus obtained was purified by silica gel column chromatography (heptane : ethyl acetate) (concentration gradient: 0 to 100%), and the title compound (colorless oily material, 1.26 g, 117%) was obtained.
¹H NMR(CDCl₃, 400MHz) : δ=1.24 (t, 3H, J=7Hz) , 3.50 (s, 2H), 4.01(br s, 4H), 4.13 (q, 2H, J=7Hz), 4.83 (br s,4H), 6.4-6.5 (m, 2H), 7.1-7.2 (m, 2H).

### Reference Example 28-1

### Methyl (1S,2S)-2-((2-bromo-5-fluorophenyl)-carbamoyl)cyclopropane-1-carboxylate

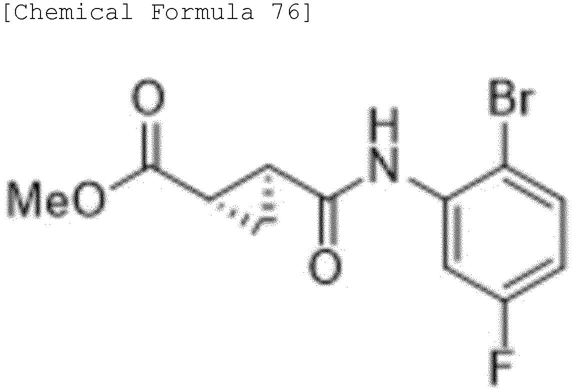

(1S,2S)-2-(methoxycarbonyl)cyclopropane-1-carboxylic acid (294 mg, 2.04 mmol) synthesized in Reference Example 22-2 and 2-bromo-5-fluoroaniline (465 mg, 2.45 mmol) were dissolved in DMF (15 mL), subsequently DIPEA (1.13 mL, 6.56 mmol) and HATU (931 mg, 2.45 mmol) were added thereto, and the mixture was stirred for 16 hours at 50°C. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (ivory-colored powder, 210 mg, 33%) was obtained.
¹H NMR (CDCl₃, 400MHz) : δ=1.4-1.5 (m, 1H), 1.5-1.6 (m, 1H), 2.1-2.2 (m, 1H), 2.3-2.4 (m, 1H), 3.74 (s, 3H), 6.73 (ddd, 1H, J=3,8,9Hz), 7.49 (dd, 1H, J=6, 9Hz), 7.95 (br s, 1H), 8.21 (dd, 1H, J=2, 11Hz).

### Reference Example 28-2

### Methyl (1S,2S)-2-(5-fluorobenzo[d]oxazol-2-yl)cyclopropane-1-carboxylate

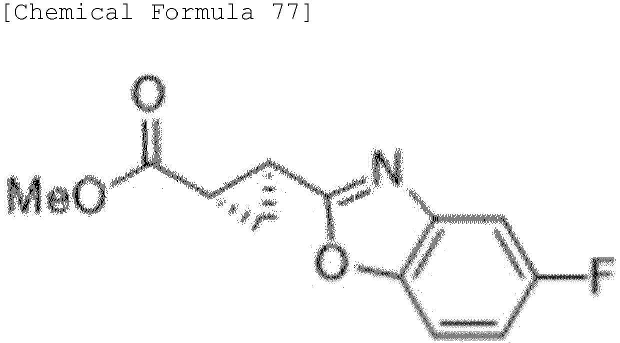

According to a technique similar to Reference Example 22-4, the title compound (ivory-colored powder, 47 mg, 30%) was obtained using methyl (1S,2S)-2-((2-bromo-5-fluorophenyl)carbamoyl)cyclopropane-1-carboxylate (210 mg, 0.66 mmol) synthesized in Reference Example 28-1.
¹H NMR(CDCl₃, 400MHz) : δ=1.7-1.8 (m, 2H), 2.4-2.5 (m, 1H), 2.7-2.8 (m, 1H), 3.75 (s, 3H), 7.0-7.1 (m, 1H), 7.31 (dd, 1H, J=2, 8Hz), 7.38 (dd, 1H, J=4, 9Hz).

### Reference Example 29-1

### Trans-2-(ethoxycarbonyl)cyclopropane-1-carboxylic acid

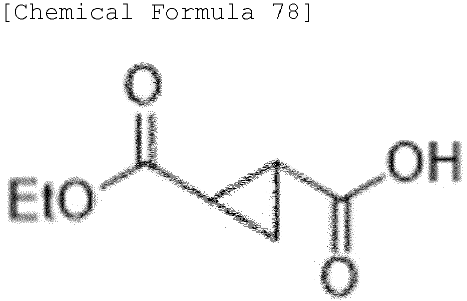

According to a technique similar to Reference Example 22-2, the title compound (white crystals, 5.91 g, 95%) was obtained using diethyl trans-cyclopropane-1,2-dicarboxylate (7.3 g, 39.2 mmol).
¹H NMR (CDCl₃, 400MHz) : δ=1.28 (t, 3H, J=7Hz),1.4-1.6(m,2H),2.1-2.3(m,2H), 4.16 (q, 2H, J=7Hz). The 1H content is not observable.

### Reference Example 29-2

### Ethyl trans-2-((2-bromophenyl)carbamoyl)-cyclopropane-1-carboxylate

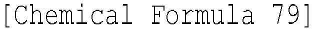

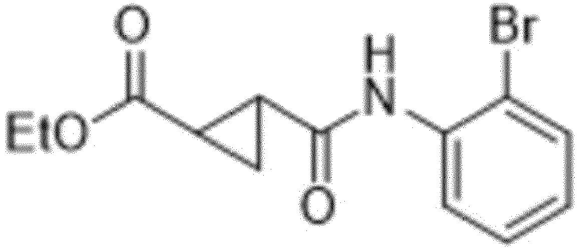

According to a technique similar to Reference Example 28-1, the title compound (white powder, 3.42 g, 87%) was obtained using trans-2-(ethoxycarbonyl)cyclopropane-1-carboxylic acid (2.0 g, 12.6 mmol) synthesized in Reference Example 29-1 and 2-bromoaniline (2.61 g, 15.2 mmol).
¹H NMR (CDCl₃, 400MHz) : δ=1. 30 (t, 3H, J=7Hz), 1.4-1.5 (m, 1H), 1.5-1.6 (m, 1H), 2.1-2.2 (m, 1H), 2.2-2.3 (m, 1H), 4.19 (q, 2H, J=7Hz), 6.9-7.0 (m, 1H), 7.2-7.3 (m, 1H), 7.55 (dd, 1H, J=1, 8Hz), 7.90 (br s, 1H), 8.33 (d, 1H, J=8Hz).

### Reference Example 29-3

### Ethyl trans-2-(benzo[d]oxazol-2-yl)cyclopropane-1-carboxylate

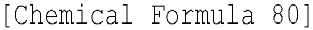

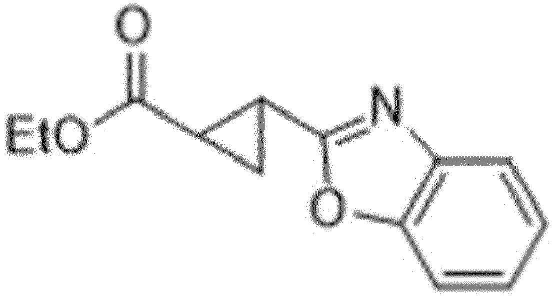

According to a technique similar to Reference Example 22-4, the title compound (pale yellow oily material, 1.22 g, 48%) was obtained using ethyl trans-2-((2-bromophenyl)carbamoyl)cyclopropane-1-carboxylate (3.42 g, 11.0 mmol) synthesized in Reference Example 29-2.
¹H NMR (CDCl₃, 400MHz) : δ=1.29 (t, 3H, J=7Hz), 1.7-1.8 (m, 2H), 2.4-2.5 (m, 1H), 2.7-2.8 (m, 1H), 4.20 (q, 2H, J=7Hz), 7.2-7.3 (m, 2H), 7.4-7.5 (m, 1H), 7.6-7.7 (m, 1H).

### Reference Example 30-1

### (1S,2S)-2-(ethoxycarbonyl)cyclopropane-1-carboxylic acid

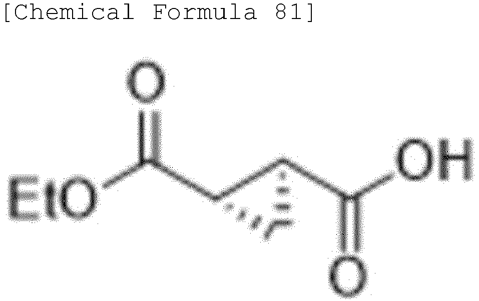

(1S,2S)-cyclopropane-1,2-dicarboxylic acid (2.5 g, 19.2 mmol) was dissolved in ethanol (38 mL), thionyl chloride (3.48 mL, 48.0 mmol) and a small amount of DMF were added thereto, the mixture was stirred for 4 hours at room temperature, and then the solvent was distilled off under reduced pressure. Water and ethyl acetate were added to a residue thus obtained, the mixture was stirred for a while, subsequently an organic layer thus separated was washed with a saturated aqueous solution of sodium hydrogen carbonate, and the organic layer was dried over anhydrous sodium sulfate. Insoluble materials were filtered, subsequently the solvent was distilled off under reduced pressure, and thereby a crude form of (1S,2S)-2-(ethoxycarbonyl)cyclopropane-1-carboxylic acid (pale ivory-colored oily material, 3.66 g) was obtained.

According to a technique similar to Reference Example 22-2, the title compound (yellow oily material, 2.31 g, 76%) was obtained using the crude form of (1S,2S)-2-(ethoxycarbonyl)cyclopropane-1-carboxylic acid (3.66 g) thus obtained.
¹H NMR (CDCl₃, 400MHz) : δ=1.28 (t, 3H, J=7Hz), 1.4-1.6(m,2H),2.1-2.3(m,2H),4.16(q,2H,J=7Hz). The 1H content is not observable.

### Reference Example 30-2

### Ethyl (1S,2S)-2-((2-bromo-5-fluorophenyl)carbamoyl)-cyclopropane-1-carboxylate

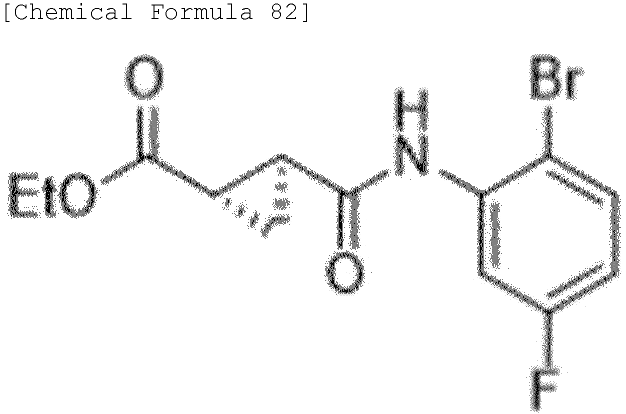

According to a technique similar to Reference Example 28-1, the title compound (ivory-colored powder, 530 g, 51%) was obtained using (1S,2S)-2-(ethoxycarbonyl)cyclopropane-1-carboxylic acid (500 mg, 3.16 mmol) synthesized in Reference Example 30-1 and 2-bromoaniline (721 mg, 3.79 mmol).
¹H NMR (CDCl₃, 400MHz) : δ=1.2-1.3 (m, 3H), 1.4-1.5 (m, 1H), 1.5-1.6 (m, 1H), 2.1-2.2 (m, 1H), 2.2-2.3 (m, 1H), 4.19 (q, 2H, J=7Hz), 6.7-6.8 (m, 1H), 7.49 (dd, 1H, J=6, 8Hz), 7.93 (br s, 1H), 8.23 (dd, 1H, J=3, 11Hz).

### Reference Example 30-3

### Ethyl (1S,2S)-2-(5-fluorobenzo[d]oxazol-2-yl)cyclopropane-1-carboxylate

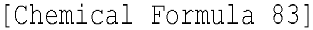

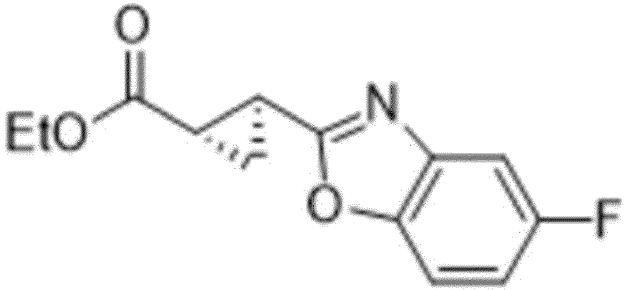

According to a technique similar to Reference Example 22-4, the title compound (brown oily material, 141 mg, 35%) was obtained using ethyl (1S,2S)-2-((2-bromo-5-fluorophenyl)carbamoyl)cyclopropane-1-carboxylate (530 mg, 1.61 mmol) synthesized in Reference Example 30-2.
¹H NMR (CDCl₃, 400MHz) : δ=1.29 (t, 3H, J=7Hz), 1.7-1.8 (m, 2H), 2.4-2.5 (m, 1H), 2.7-2.8 (m, 1H), 4.20 (q, 2H, J=7Hz), 7.02 (ddd, 1H, J=2, 9, 9Hz), 7.31 (dd, 1H, J=3, 8Hz), 7.38 (dd, 1H, J=4, 9Hz).

### Reference Example 31-1

### Dimethyl 2-(5-(trifluoromethyl)pyridin-2-yl)malonate

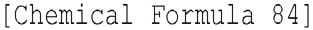

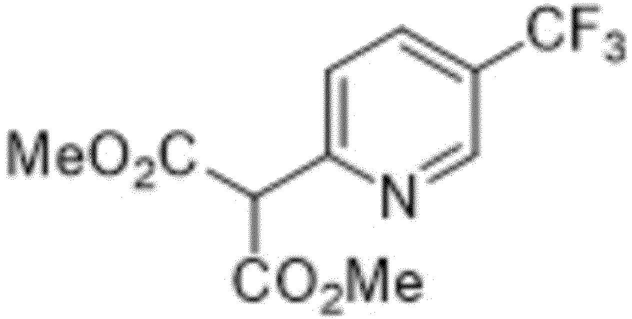

2-Chloro-5-(trifluoromethyl)pyridine (5.00 g, 27.5 mmol), dimethyl malonate (5.46 g, 41.3 mmol), and cesium carbonate (18.0 g, 55.1 mmol) were dissolved in dimethyl sulfoxide (10.0 mL), and the solution was stirred overnight at 110°C under a nitrogen gas stream. To the reaction liquid that had been left to cool to room temperature, water was added, the mixture was stirred for a while, and then the mixture was extracted with ethyl acetate. An organic layer thus separated was washed with saturated brine and dried over anhydrous sodium sulfate, insoluble materials were filtered, and then the solvent was distilled off under reduced pressure. A residue thus obtained was purified by silica gel column chromatography (heptane : ethyl acetate) (concentration gradient: 25 to 75%), and the title compound (yellow oily material, 2.46 g, 32%) was obtained.
¹H NMR (CDCl₃, 400MHz) : δ=3.81 (s, 6H), 5.05 (s, 1H), 7.67 (d, 1H, J=8Hz), 7.97 (d, 1H, J=8Hz), 8.84 (s, 1H).

### Reference Example 31-2

### 2-(5-(Trifluoromethyl)pyridin-2-yl)acetic acid

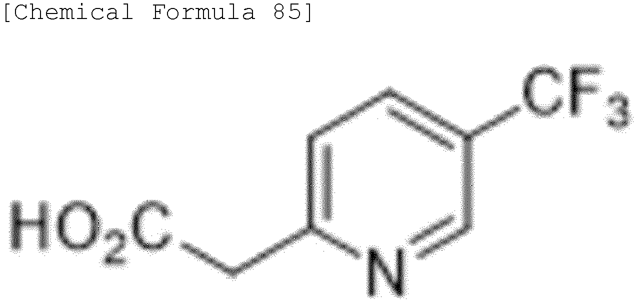

Dimethyl 2-(5-(trifluoromethyl)pyridin-2-yl)malonate (790 mg, 2.85 mmol) synthesized in Reference Example 31-1 was dissolved in methanol (18 mL), a 2 N aqueous solution of sodium hydroxide (3.6 mL) was added thereto, and then the mixture was stirred overnight at 50°C. To the reaction liquid that had been left to cool to room temperature, a 2 N aqueous solution of hydrochloric acid (3.5 mL) was added to adjust the pH of the aqueous layer to 6 to 7, subsequently the solvent was distilled off under reduced pressure, and thereby the title compound (pale yellow crystals, 864 mg) including sodium chloride was obtained.
¹H NMR (DMSO-d₆, 400MHz) : δ=3.85 (s, 2H), 7.60 (d, 1H, J=8Hz), 8.15 (dd, 1H, J=2, 8Hz), 8.86 (s, 1H). The 1H content is not observable.

### Reference Example 32-1

### Tert-butyl (R)-(1-(6-(trifluoromethyl)pyridin-3-yl)pyrrolidin-3-yl) carbamate

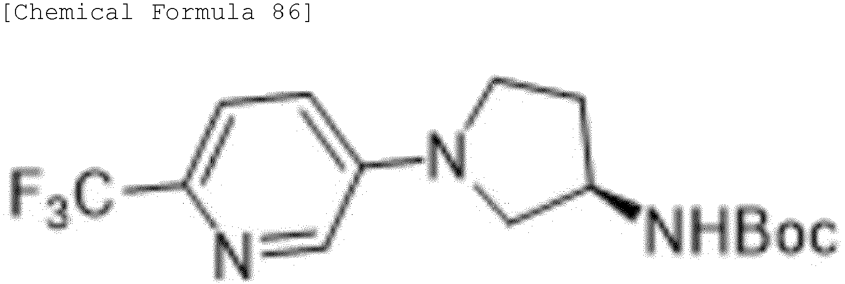

According to a technique similar to Reference Example 4, the title compound (pale yellow amorphous, 2.20 g, 89%) was obtained using 5-bromo-2-(trifluoromethyl)pyridine (2.55 g, 11.3 mmol) and tert-butyl (R)-pyrrolidin-3-ylcarbamate (1.50 g, 7.49 mmol).
¹H NMR (CDCl₃, 400MHz) : δ=1.46 (s, 9H), 1.9-2.0 (m, 1H), 2.2-2.4 (m, 1H), 3.2-3.3 (m, 1H), 3.3-3.6 (m, 2H), 3.6-3.7 (m, 1H), 4.3-4.5 (m, 1H), 4.6-4.8 (m,1H), 6.82 (dd, 1H, J=3, 9Hz), 7.48 (d, 1H, J=9Hz), 8.01 (d, 1H, J=3Hz).

### Reference Example 32-2

### (R)-1-(6-(trifluoromethyl)pyridin-3-yl)pyrrolidin-3-amine

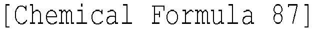

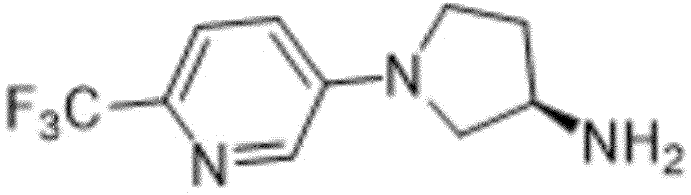

According to a technique similar to Reference Example 1-2, the title compound (pale yellow crystals, 1.50 g, 97%) was obtained using tert-butyl (R)-(1-(6-(trifluoromethyl)pyridin-3-yl)pyrrolidin-3-yl)carbamate (2.20 g, 6.64 mmol) synthesized in Reference Example 32-1.
¹H NMR (CDCl₃, 400MHz) : δ=1.40 (br s, 2H), 1.8-2.0 (m, 1H), 2.2-2.3 (m, 1H), 3.0-3.1 (m, 1H), 3.3-3.5 (m, 1H), 3.5-3.6 (m, 2H), 3.7-3.9 (m, 1H), 6.80 (dd, 1H, J=3, 9Hz), 7.47 (d, 1H, J=9Hz), 8.00 (d, 1H, J=3Hz).

### Reference Example 33-1

### Ethyl 2-(4-cyclopropylphenyl)acetate

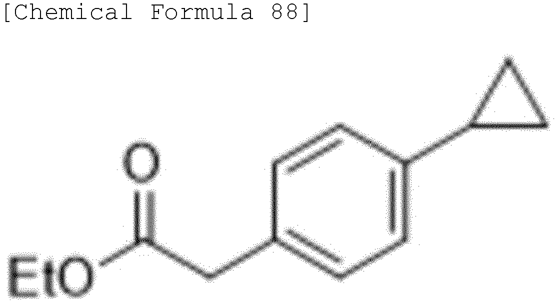

Ethyl 4-bromophenylacetate (6.0 g, 24.7 mmol), cyclopropylboronic acid (2.76 g, 32.1 mmol), palladium acetate (276 mg, 1.23 mmol), tricyclohexylphosphine (0.6 M toluene solution, 4.2 mL, 2.46 mmol), and potassium phosphate monohydrate (19.9 g, 86.4 mmol) were suspended in toluene (60.0 mL) and water (3.0 mL), and the suspension was stirred for 16 hours at 100°C under a nitrogen gas stream. The reaction liquid that had been left to cool to room temperature was filtered through a Celite pad, and then the solvent of the filtrate was distilled off under reduced pressure. A residue thus obtained was diluted with ethyl acetate, an organic layer was washed with an aqueous solution of sodium hydrogen carbonate, water, and saturated brine and then was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 30%), and the title compound (yellow oily material, 4.90 g, 97%) was obtained.
¹H NMR (CDCl₃, 400MHz) : δ=0.6-0.7 (m, 2H), 0.9-1.0 (m, 2H), 1.24 (t, 3H, J=8Hz), 1.8-1.9 (m, 1H), 3.52 (s, 2H), 4.12 (q, 2H, J=8Hz), 7.01 (d, 2H, J=8Hz), 7.15 (d, 2H, J=8Hz).

### Reference Example 33-2

### 2-(4-Cyclopropylphenyl)acetic acid

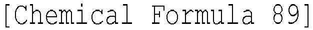

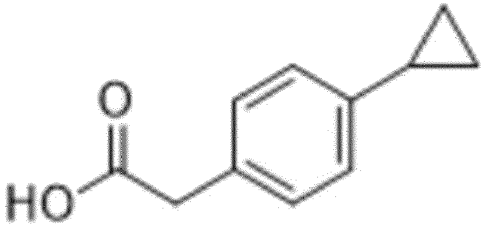

According to a technique similar to Reference Example 22-2, the title compound (white crystals, 3.80 g, 90%) was obtained using ethyl 2-(4-cyclopropylphenyl)-acetate (4.90 g, 24.0 mmol) synthesized in Reference Example 33-1.
¹H NMR (CDCl₃, 400MHz) : δ=0.6-0.7 (m, 2H), 0.9-1.0 (m, 2H), 1.8-2.0 (m, 1H), 3.60 (s, 2H), 7.03 (d, 2H, J=8Hz), 7.16(d,2H,J=8Hz). The 1H content is not observable.

### Reference Example 34

### Tert-butyl (R)-(1-(4-(trifluoromethyl)-phenyl)pyrrolidin-3-yl)carbamate

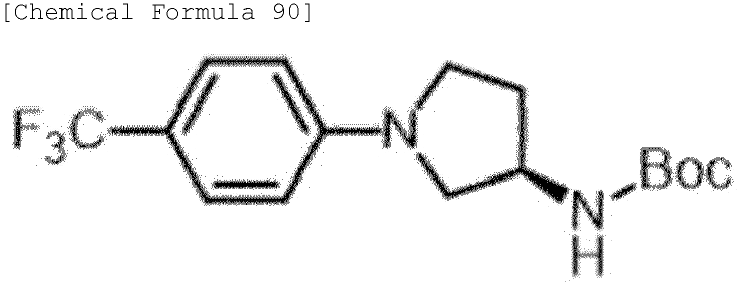

According to a technique similar to Reference Example 1-1, the title compound (pale yellow syrup, 273 mg, 77%) was obtained using 1-bromo-4-(trifluoromethyl)benzene (260 mg, 1.61 mmol) and tert-butyl (R)-pyrrolidin-3-ylcarbamate (200 mg, 1.07 mmol).
¹H NMR (CDCl₃, 400MHz) : δ=1.45 (s, 9H), 1.9-2.1 (m, 1H), 2.2-2.4 (m, 1H), 3.20 (dd, 1H, J=3, 10Hz), 3.3-3.5 (m, 2H), 3.62 (dd, 1H, J=6, 10Hz), 4.38 (br s, 1H), 4.69 (br s, 1H), 6.55 (d, 2H, J=9Hz), 7.45 (d, 2H, J=9Hz).

### Reference Example 35

### Tert-butyl (S)-(1-(4-(trifluoromethyl)-phenyl)pyrrolidin-3-yl)carbamate

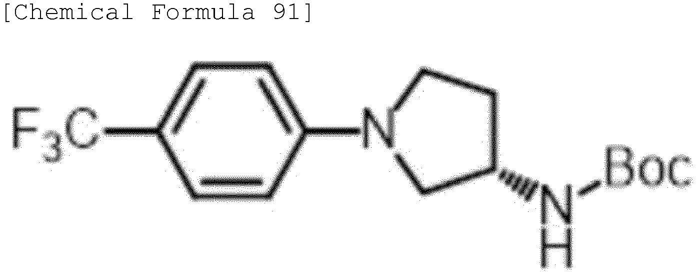

According to a technique similar to Reference Example 1-1, the title compound (pale yellow solid, 236 mg, 67%) was obtained using 1-bromo-4-(trifluoromethyl)benzene (260 mg, 1.61 mmol) and tert-butyl (S)-pyrrolidin-3-ylcarbamate (200 mg, 1.07 mmol).
¹H NMR (CDCl₃, 400MHz) : δ=1.46 (s, 9H), 1.9-2.1 (m, 1H), 2.2-2.4 (m, 1H), 3.20 (dd, 1H, J=3, 10Hz), 3.3-3.5 (m, 2H), 3.62 (dd, 1H, J=6, 10Hz), 4.37 (br s, 1H) , 4.69 (br s, 1H), 6.55 (d, 2H, J=9Hz), 7.45 (d, 2H, J=9Hz).

### Reference Example 36

### Tert-butyl (R)-(1-(6-(trifluoromethyl)pyridin-3-yl)pyrrolidin-3-yl) carbamate

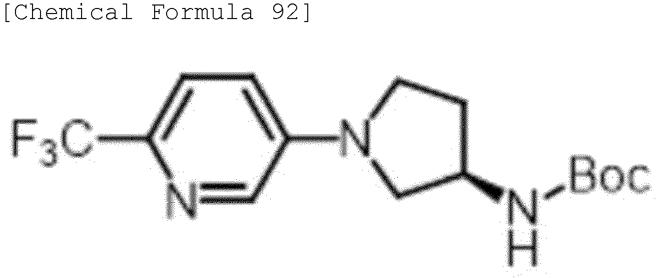

According to a technique similar to Reference Example 1-1, the title compound (pale yellow solid, 53 mg, 59%) was obtained using 5-bromo-2-(trifluoromethyl)pyridine (67 mg, 0.29 mmol) and tert-butyl (R)-pyrrolidin-3-ylcarbamate (50 mg, 0.27 mmol).
¹H NMR (CDCl₃, 400MHz) : δ=1.45 (s, 9H), 1.9-2.1 (m, 1H), 2.2-2.4 (m, 1H), 3.23 (dd, 1H, J=4, 10Hz), 3.3-3.6 (m, 2H), 3.65 (dd, 1H, J=6, 10Hz), 4.40 (br s, 1H) , 4.81 (br s, 1H), 6.82 (dd, 1H, J=3, 9Hz), 7.47 (d, 1H, J=9Hz), 7.99 (d, 1H, J=3Hz).

### Reference Example 37

### Tert-butyl (S)-(1-(6-(trifluoromethyl)pyridin-3-yl)pyrrolidin-3-yl)carbamate

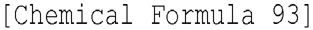

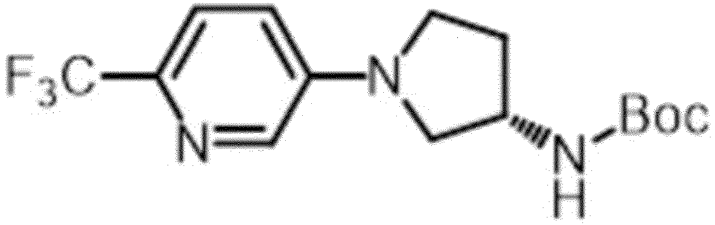

According to a technique similar to Reference Example 1-1, the title compound (pale yellow solid, 59 mg, 66%) was obtained using 5-bromo-2-(trifluoromethyl)pyridine (67 mg, 0.29 mmol) and tert-butyl (S)-pyrrolidin-3-ylcarbamate (50 mg, 0.27 mmol).
¹H NMR (CDCl₃, 400MHz) : δ=1.45 (s, 9H), 1.9-2.1 (m, 1H), 2.2-2.4 (m, 1H), 3.24 (dd, 1H, J=4, 10Hz), 3.3-3.6 (m, 2H), 3.65 (dd, 1H, J=6, 10Hz), 4.40 (br s, 1H), 4.89 (br s, 1H), 6.82 (dd, 1H, J=3, 9Hz), 7.47 (d, 1H, J=9Hz), 7.99 (d, 1H, J=3Hz).

### Reference Example 38-1

### Tert-butyl (R)-(1-(5-(trifluoromethyl)pyrimidin-2-yl)pyrrolidin-3-yl)carbamate

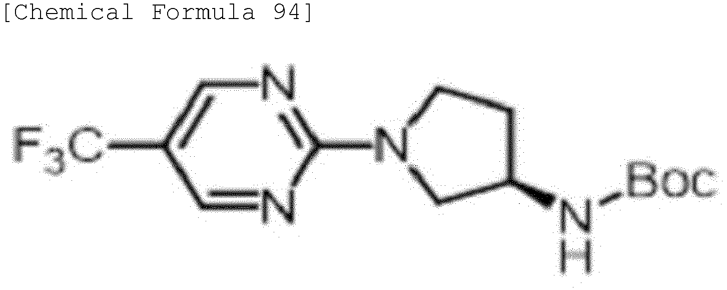

According to a technique similar to Reference Example 9, the title compound (white solid, 86 mg, 97%) was obtained using 2-chloro-5-(trifluoromethyl)pyrimidine (49 mg, 0.27 mmol) and tert-butyl (R)-pyrrolidin-3-ylcarbamate (50 mg, 0.27 mmol).
¹H NMR (CDCl₃, 400MHz) : δ=1.45 (s, 9H), 1.9-2.1 (m, 1H), 2.2-2.4 (m, 1H), 3.50 (dd, 1H, J=5, 12Hz), 3.6-3.8 (m, 2H), 3.88 (dd, 1H, J=6, 12Hz), 4.36 (br s, 1H), 4.73 (d, 1H, J=7Hz), 8.50 (s, 2H).

### Reference Example 38-2

### (R)-1-(5-(trifluoromethyl)pyrimidin-2-yl)pyrrolidin-3-amine

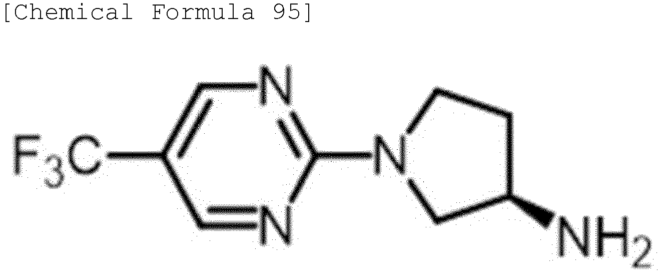

According to a technique similar to Reference Example 1-2, the title compound (white solid, 45 mg, 75%) was obtained using tert-butyl (R)-(1-(5-(trifluoromethyl)pyrimidin-2-yl)pyrrolidin-3-yl)carbamate (86 mg, 0.26 mmol) synthesized in Reference Example 38-1.
¹H NMR (CDCl₃, 400MHz) : δ=1.8-1.9 (m, 1H), 2.1-2.3 (m, 1H), 3.3-3.4 (m, 1H), 3.6-3.9 (m, 4H), 8.50 (s, 2H). The 2H content is not observable.

### Reference Example 39

### Tert-butyl (R)-(1-(4-fluoro-3-(trifluoromethyl)-phenyl)pyrrolidin-3-yl) carbamate

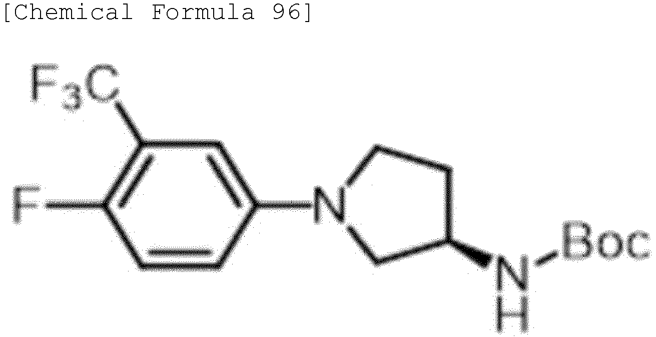

According to a technique similar to Reference Example 1-1, the title compound (pale yellow solid, 112 mg, 60%) was obtained using 4-bromo-1-fluoro-2-(trifluoromethyl)benzene (144 mg, 0.59 mmol) and tert-butyl (R)-pyrrolidin-3-ylcarbamate (100 mg, 0.54 mmol).
¹H NMR (CDCl₃, 400MHz) : δ=1.45 (s, 9H), 1.9-2.1 (m, 1H), 2.2-2.4 (m, 1H), 3.15 (dd, 1H, J=4, 9Hz), 3.2-3.5 (m, 2H), 3.55 (dd, 1H, J=6, 10Hz), 4.37 (br s, 1H), 4.79 (d, 1H, J=7Hz), 6.5-6.6 (m, 2H), 7.04 (t, 1H, J=10Hz).

### Reference Example 40-1

### (1R,2S,5R)-2-isopropyl-5-methylcyclohexyl (1S,2S)-2-((2-formylphenyl)carbamoyl)cyclopropane-1-carboxylate

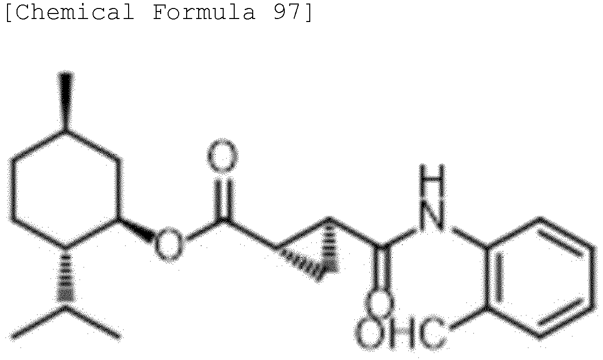

According to a technique similar to Reference Example 28-1, a crude form of the title compound was obtained using (1S,2S)-2-((((1R,2S,5R)-2-isopropyl-5-methylcyclohexyl)oxy) carbonyl)cyclopropane-1-carboxylic acid (1.17 g, 4.35 mmol) and 2-aminobenzaldehyde (988 mg, 8.12 mmol).

### Reference Example 40-2

### (1R,2S,5R)-2-isopropyl-5-methylcyclohexyl (1S,2S)-2-(quinazolin-2-yl)cyclopropane-1-carboxylate

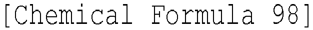

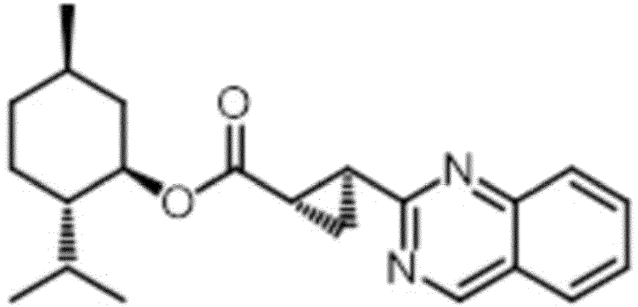

The crude form of (1R,2S,5R)-2-isopropyl-5-methylcyclohexyl (1S,2S)-2-((2-formylphenyl)-carbamoyl)cyclopropane-1-carboxylate synthesized in Reference Example 40-1 and ammonium acetate were heated to reflux for 10 hours at 120°C in toluene. The reaction liquid was left to cool to room temperature, sodium hydrogen carbonate was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 10 to 50%), and the title compound (277 mg, 18%) was obtained.
¹H NMR (CDCl₃, 400MHz) : δ=0.76 (d, 3H, J=7Hz), 0.80(d,3H,J=7Hz),0.8-2.1(m,12H),2.1-2.3(m,2H), 2.4-2.5 (m, 1H), 2.9-3.0 (m, 1H), 4.6-4.8 (m, 1H), 7.58 (ddd, 1H, J=1, 7, 7Hz), 7. 8-8. 0 (m, 3H), 9.27 (s, 1H).

### Reference Example 40-3

### (1S,2S)-2-(quinazolin-2-yl)cyclopropane-1-carboxylic acid

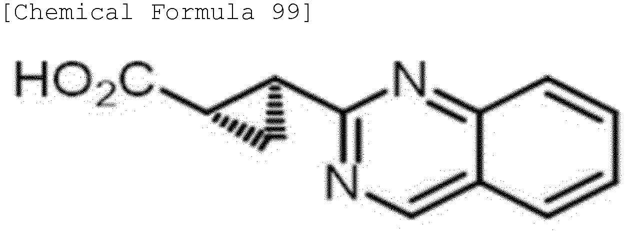

(1R,2S,5R)-2-isopropyl-5-methylcyclohexyl (1S,2S)-2-(quinazolin-2-yl)cyclopropane-1-carboxylate (277 mg, 0.79 mmol) synthesized in Reference Example 40-2 was dissolved in isopropanol (2.1 mL) and water (170 µL), sodium hydroxide (63 mg, 1.57 mmol) was added thereto, the mixture was stirred for 25 hours at 80°C, and then the solvent was distilled off under reduced pressure. 3 N hydrochloric acid and water were added to a residue thus obtained, and the mixture was extracted with ethyl acetate. An organic layer thus separated was dried over anhydrous sodium sulfate, a solid precipitated using ethyl acetate and hexane was collected by filtration, and the title compound (pale yellow solid, 73 mg, 43%) was obtained.
¹H NMR (CDCl₃, 400MHz) : δ=1.6-1.7 (m, 1H), 1.7-1.9 (m, 1H), 2.3-2.4 (m, 1H), 2.8-2.9 (m, 1H), 7.67 (ddd, 1H, J=1, 7, 7Hz), 7.91 (d, 1H, J=7Hz), 7.97 (ddd, 1H, J=1, 7, 7Hz), 8.03 (dd, 1H, J=1, 7Hz) , 9.39 (s, 1H). The 1H content is not observable.

### Reference Example 41-1

### Tert-butyl (R)-(1-(3-(methylsulfonyl)-phenyl)-pyrrolidin-3-yl) carbamate

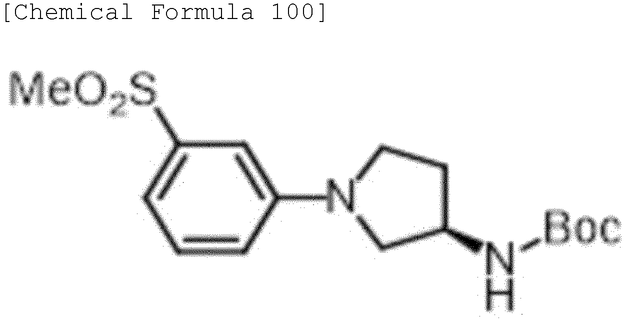

According to a technique similar to Reference Example 1-1, the title compound (pale yellow oil, 57 mg, 62%) was obtained using 1-bromo-3-(methylsulfonyl)benzene (63 mg, 0.27 mmol) and tert-butyl (R)-pyrrolidin-3-ylcarbamate (50 mg, 0.27 mmol).
¹H NMR (CDCl₃, 400MHz) : δ=1.46 (s, 9H), 1.9-2.1 (m, 1H), 2.2-2.4 (m, 1H), 3.04 (s, 3H), 3.21 (dd, 1H, J=4, 10Hz), 3.3-3.5 (m, 2H), 3.63 (dd, 1H, J=6, 10Hz), 4.34 (br s, 1H), 4.78 (br s, 1H), 6.75 (dd, 1H, J=2, 8Hz), 7.02 (t, 1H, J=2Hz), 7.20 (d, 1H, J=8Hz), 7.38 (t, 1H, J=8Hz).

### Reference Example 41-2

### (R)-1-(3-(methylsulfonyl)phenyl)pyrrolidin-3-amine

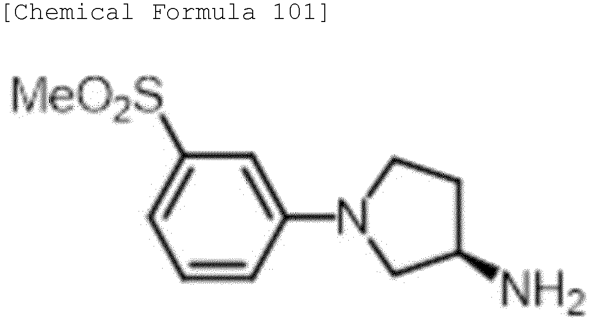

According to a technique similar to Reference Example 1-2, the title compound (pale yellow oil, 12 mg, 30%) was obtained using tert-butyl (R)-(1-(3-(methylsulfonyl)phenyl)pyrrolidin-3-yl)carbamate (57 mg, 0.17 mmol) synthesized in Reference Example 41-1.
¹H NMR (CDCl₃, 400MHz) : δ=1.8-1.9 (m, 1H), 2.2-2.3 (m, 1H), 3.04 (s, 3H), 3.78 (dd, 1H, J=6,10Hz), 3.3-3.6 (m, 3H), 3.77 (quin, 1H, J=6Hz), 6.74 (dd, 1H, J=2, 8Hz), 7.02 (t, 1H, J=2Hz), 7.17 (d, 1H, J=8Hz), 7.37 (t, 1H, J=8Hz) . The 2H content is not observable.

### Reference Example 42-1

### Tert-butyl (R)-(1-(3-cyanophenyl)pyrrolidin-3-yl)carbamate

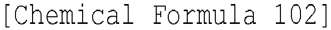

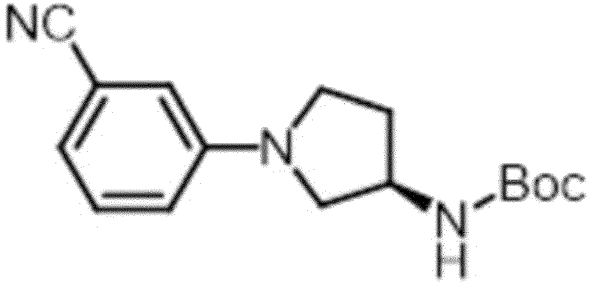

According to a technique similar to Reference Example 1-1, the title compound (pale yellow amorphous, 57 mg, 74%) was obtained using 3-bromobenzonitrile (54 mg, 0.30 mmol) and tert-butyl (R)-pyrrolidin-3-ylcarbamate (50 mg, 0.27 mmol).
¹H NMR (CDCl₃, 400MHz) : δ=1.46 (s, 9H), 1.9-2.1 (m, 1H), 2.2-2.4 (m, 1H), 3.16 (dd, 1H, J=4, 10Hz), 3.3-3.5 (m, 2H), 3.57 (dd, 1H, J=6, 10Hz), 4.37 (br s, 1H), 4.76 (d, 1H, J=6Hz), 6.7-6.8 (m, 2H), 6.94 (d, 1H, J=7Hz), 7.2-7.3 (m, 1H).

### Reference Example 42-2

### (R)-3-(3-aminopyrrolidin-1-yl)benzonitrile

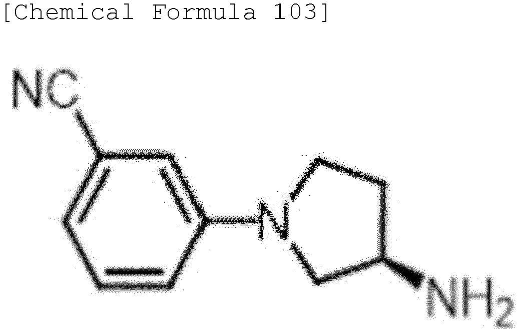

According to a technique similar to Reference Example 1-2, the title compound (pale yellow oil, 32 mg, 85%) was obtained using tert-butyl (R)-(1-(3-cyanophenyl)-pyrrolidin-3-yl)carbamate (57 mg, 0.20 mmol) synthesized in Reference Example 42-1.
¹H NMR (CDCl₃, 400MHz) : δ=1.8-1.9 (m, 1H), 2.2-2.3 (m, 1H), 3.11 (dd, 1H, J=5, 9Hz), 3.2-3.6 (m, 3H), 3.76 (quin, 1H, J=6Hz), 6.6-6.8 (m, 2H), 6.91 (d, 1H, J=7Hz), 7.2-7.3 (m, 1H). The 2H content is not observable.

### Reference Example 43-1

### Ethyl trans-2-((2-amino-5-cyanophenyl)-carbamoyl)cyclopropane-1-carboxylate

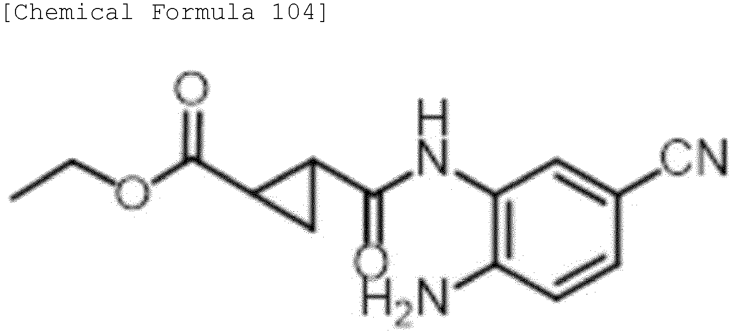

According to a technique similar to Reference Example 7-1, a crude form of the title compound was obtained using trans-2-(ethoxycarbonyl)cyclopropane-1-carboxylic acid (100 mg, 0.63 mmol) and 3,4-diaminobenzonitrile (101 mg, 0.76 mmol).
¹H NMR (CDCl₃, 400MHz) : δ=1. 27 (t, 3H, J=7Hz), 1.3-1.6 (m, 2H), 2.1-2.4 (m, 2H) ,4.15(q,2H,J=7Hz), 4.75 (br s, 2H), 6.72 (d, 1H, J=8Hz), 6.72 (d, 1H, J=8Hz), 7.22 (dd, 1H, J=2, 8Hz), 7.57 (d, 1H, J=2Hz).

### Reference Example 43-2

### Ethyl trans-2-(5-cyano-1H-benzo[d]imidazol-2-yl)cyclopropane-1-carboxylate

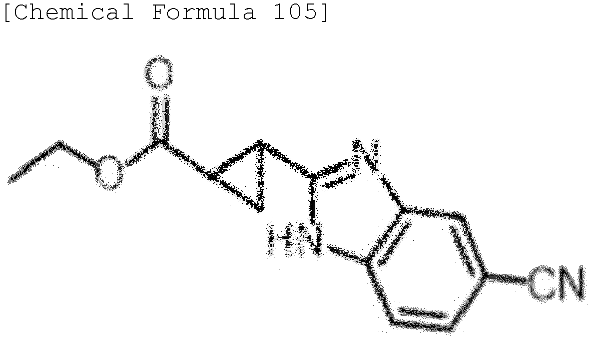

The crude form of ethyl trans-2-((2-amino-5-cyanophenyl)carbamoyl)cyclopropane-1-carboxylate synthesized in Reference Example 43-1 was heated for 5 hours at 85°C in acetic acid and then was left to cool to room temperature, and the solvent was distilled off under reduced pressure. An aqueous solution of sodium hydrogen carbonate was added thereto, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (hexane : ethyl acetate) (concentration gradient: 40 to 80%), and thus the title compound (light brown amorphous, 142 mg, 88%) was obtained.
¹H NMR (CDCl₃, 400MHz) : δ=1.27 (t, 3H, J=7Hz), 1.7-1.9 (m, 2H), 2.4-2.6 (m, 1H), 2.7-2.8 (m, 1H), 4.15 (q, 2H, J=7Hz), 7.4-8.0 (m, 2H), 7.49 (d, 1H, J=7Hz), 11.58 (br s, 1H).

### Reference Example 43-3

### Trans-2-(5-cyano-1H-benzo[d]imidazol-2-yl)cyclopropane-1-carboxylic acid

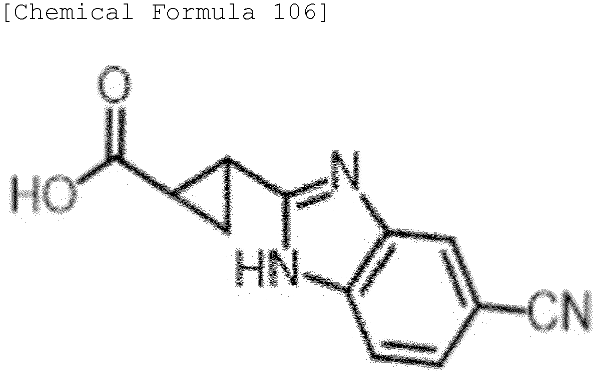

Ethyl trans-2-(5-cyano-1H-benzo[d]imidazol-2-yl)cyclopropane-1-carboxylate (142 mg, 0.56 mmol) synthesized in Reference Example 43-2 was dissolved in methanol (6 mL) and water (1 mL), lithium hydroxide monohydrate (47 mg, 1.11 mmol) was added thereto, the mixture was stirred at room temperature, and then the solvent was distilled off under reduced pressure. 3 N hydrochloric acid was added to a residue thus obtained, and the solvent was distilled off under reduced pressure. A solid precipitated using ethyl acetate and hexane was collected by filtration, and the title compound (85 mg, 67%) was obtained.
¹H NMR (DMSO-d₆, 400MHz) : δ=1.6-1.8 (m, 2H), 2.2-2.4 (m, 1H), 2.6-2.8 (m, 1H), 7.52 (dd, 1H, J=1, 8Hz), 7.61 (d, 1H, J=8Hz), 7.87 (s, 1H). The 2H content is not observable.

### Reference Example 44

### Tert-butyl (R)-(1-(2-(trifluoromethyl)pyridin-4-yl)pyrrolidin-3-yl) carbamate

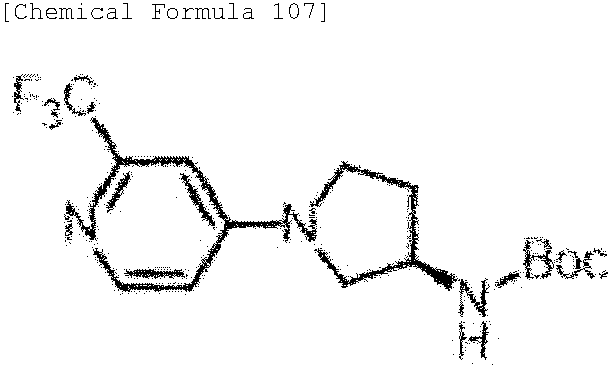

According to a technique similar to Reference Example 9, the title compound (white amorphous) was obtained using 4-chloro-2-(trifluoromethyl)pyridine (49 mg, 0.27 mmol) and tert-butyl (R)-pyrrolidin-3-ylcarbamate (50 mg, 0.27 mmol).
¹H NMR (CDCl₃, 400MHz) : δ=1.46 (s, 9H), 1.9-2.1 (m, 1H), 2.2-2.4 (m, 1H), 3.24 (dd, 1H, J=4, 10Hz), 3.4-3.6 (m, 2H), 3.66 (dd, 1H, J=6, 10Hz), 4.38 (br s, 1H), 4.8-5.1 (m, 1H), 6.46 (dd, 1H, J=2, 6Hz), 6.72 (d, 1H, J=2Hz), 8.28 (d, 1H, J=6Hz).

### Reference Example 45-1

### Tert-butyl (R)-(1-(6-(trifluoromethyl)pyridin-3-yl)piperidin-3-yl)carbamate

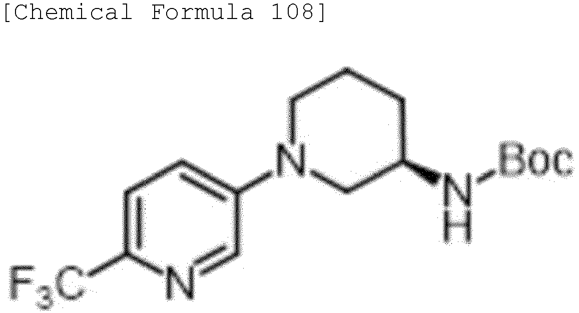

According to a technique similar to Reference Example 1-1, the title compound (light brown solid, 72 mg, 84%) was obtained using 5-bromo-2-(trifluoromethyl)pyridine (62 mg, 0.27 mmol) and tert-butyl (R)-piperidin-3-ylcarbamate (50 mg, 0.25 mmol).
¹H NMR (CDCl₃, 400MHz) : δ=1.4-2.0 (m, 4H), 1.46 (s, 9H), 2.9-4.0 (m, 5H), 4.5-4.9 (m, 1H), 7.33 (s, 1H), 8.31 (s, 1H), 8.46 (d, 1H, J=3Hz).

### Reference Example 45-2

### (R)-1-(6-(trifluoromethyl)pyridin-3-yl)piperidin-3-amine

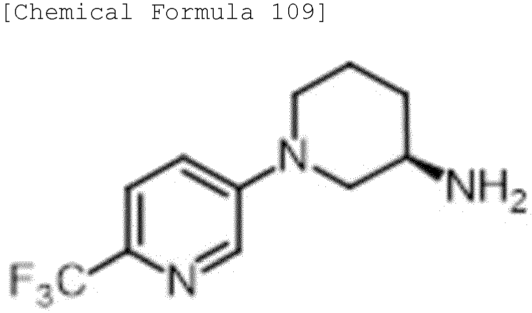

According to a technique similar to Reference Example 1-2, the title compound (light brown syrup, 50 mg, 97%) was obtained using tert-butyl (R)-(1-(6-(trifluoromethyl)pyridin-3-yl)piperidin-3-yl)carbamate (72 mg, 0.21 mmol) synthesized in Reference Example 45-1.
¹H NMR (CDCl₃, 400MHz) : δ=1.2-1.4 (m, 1H), 1.6-1.8 (m, 1H), 1.8-2.1 (m, 2H), 2.68 (dd, 1H, J=9, 12Hz), 2.8-3.1 (m, 2H), 3.5-3.6 (m, 1H), 3.6-3.7 (m, 1H), 7.32 (s, 1H), 8.28 (d, 1H, J=1Hz), 8.45 (d, 1H, J=3Hz). The 2H content is not observable.

### Reference Example 46-1

### 2-Chloro-4-(trifluoromethyl)thiazole

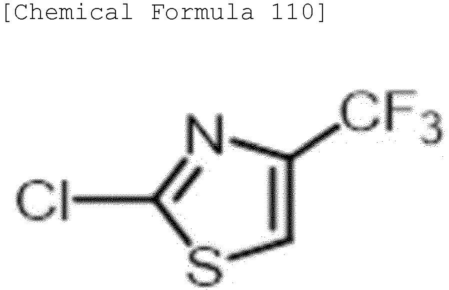

4-(Trifluoromethyl)thiazol-2-amine (100 mg, 0.60 mmol) and copper(II) chloride (96 mg, 0.71 mmol) were suspended in acetonitrile (8 mL), tert-butyl nitrite (92 mg, 0.89 mmol) was added thereto, and the mixture was stirred for one hour at room temperature. The reaction liquid was added to 1 N hydrochloric acid that had been ice-cooled, and the reaction liquid was extracted with diethyl ether. An organic layer thus separated was dried over anhydrous sodium sulfate, insoluble materials were filtered, subsequently the solvent was distilled off at 30°C under reduced pressure (700 mbar), and thereby a crude form of the title compound was obtained.
¹H NMR (CDCl₃, 400MHz) : δ=7.69 (s, 1H) .

### Reference Example 46-2

### Tert-butyl (R)-(1-(4-(trifluoromethyl)thiazol-2-yl)pyrrolidin-3-yl)carbamate

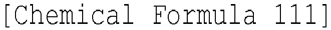

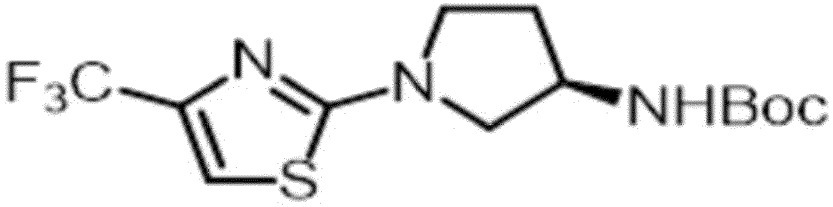

Tert-butyl (R)-pyrrolidin-3-ylcarbamate (111 mg, 0.60 mmol), the crude form of 2-chloro-4-(trifluoromethyl)-thiazole synthesized in Reference Example 46-1, and potassium carbonate (99 mg, 0.72 mmol) were dissolved in acetonitrile (8 mL), and then the mixture was stirred for 17 hours at 60°C. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (hexane : ethyl acetate) (concentration gradient: 10 to 60%), and the title compound (pale yellow solid, 147 mg, 73%) was obtained.
¹H NMR (CDCl₃, 400MHz) : δ=1.45 (s, 9H), 1.9-2.1 (m, 1H), 2.2-2.4 (m, 1H), 3.35 (dd, 1H, J=4, 10Hz), 3.5-3.7 (m, 2H), 3.75 (dd, 1H, J=6, 10Hz), 4.38 (br s, 1H), 4.82 (br s, 1H), 6.92 (s, 1H).

### Reference Example 46-3

### (R)-1-(4-(trifluoromethyl)thiazol-2-yl)pyrrolidin-3-amine

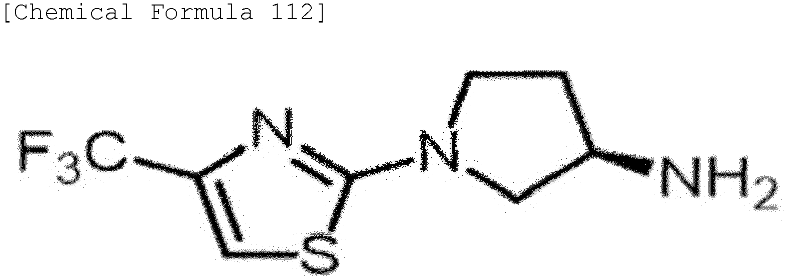

According to a technique similar to Reference Example 1-2, the title compound (pale yellow syrup, 94 mg, 91%) was obtained using tert-butyl (R)-(1-(4-(trifluoromethyl)thiazol-2-yl)pyrrolidin-3-yl)carbamate (147 mg, 0.44 mmol) synthesized in Reference Example 46-2.
¹H NMR (CDCl₃, 400MHz) : δ=1.8-2.0 (m, 1H), 2.2-2.3 (m, 1H), 3.23 (dd, 1H, J=4, 10Hz), 3.4-3.6 (m, 1H), 3.6-3.7 (m, 2H), 3.7-3.9 (m, 1H), 6.90 (d, 1H, J=1Hz). The 2H content is not observable.

### Reference Example 47-1

### Tert-butyl (R)-(1-(4-(trifluoromethyl)-phenyl)piperidin-3-yl)carbamate

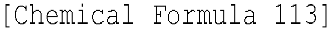

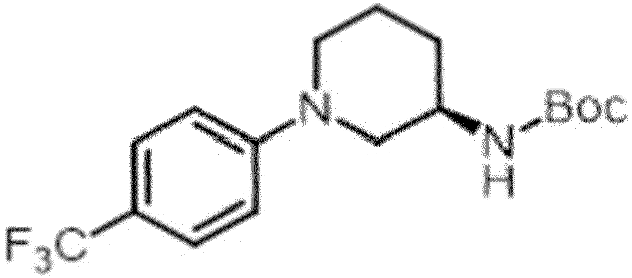

According to a technique similar to Reference Example 1-1, the title compound (pale yellow solid, 59 mg, 68%) was obtained using 1-bromo-4-(trifluoromethyl)benzene (62 mg, 0.27 mmol) and tert-butyl (R)-piperidin-3-ylcarbamate (50 mg, 0.25 mmol).
¹H NMR(CDCl₃, 400MHz): δ=1.4-1.9 (m, 13H), 2.8-3.9 (m, 5H), 4.4-4.9 (m, 1H), 6.94 (d, 2H, J=9Hz), 7.46 (d, 2H, J=9Hz).

### Reference Example 47-2

### (R)-1-(4-(trifluoromethyl)phenyl)piperidin-3-amine

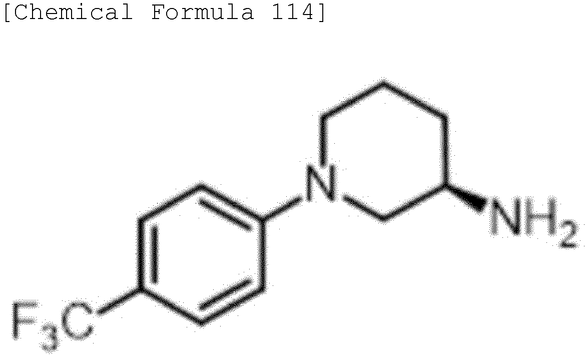

According to a technique similar to Reference Example 1-2, the title compound (light brown syrup, 42 mg, 100%) was obtained using tert-butyl (R)-(1-(4-(trifluoromethyl)phenyl)piperidin-3-yl)carbamate (59 mg, 0.17 mmol) synthesized in Reference Example 47-1.
¹H NMR (CDCl₃, 400MHz) :δ=1.2-1.4 (m, 1H), 1. 6-1. 9 (m, 2H), 1.9-2.1 (m, 1H), 2.67 (dd, 1H, J=9, 12Hz), 2.8-3.0 (m, 2H), 3.54 (dt, 1H, J=4, 12Hz), 3.6-3.7 (m, 1H), 6.92 (d, 2H, J=9Hz), 7.45(d,2H,J=9Hz). The 2H content is not observable.

### Reference Example 48-1

### Tert-butyl (R)-(1-(6-methoxy-5-(trifluoromethyl)-pyridin-3-yl)pyrrolidin-3-yl)carbamate

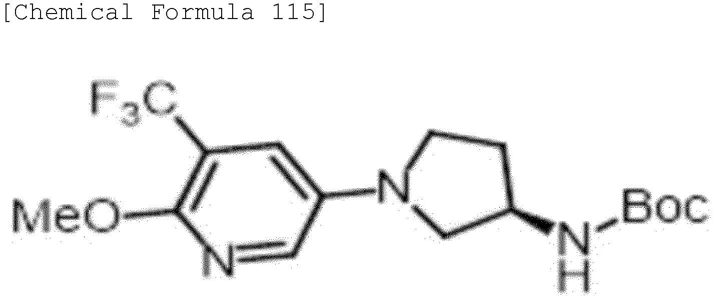

According to a technique similar to Reference Example 1-1, the title compound (pale yellow solid, 26 mg, 13%) was obtained using 5-bromo-2-methoxy-3-(trifluoromethyl)pyridine (151 mg, 0.59 mmol) and tert-butyl (R)-pyrrolidin-3-ylcarbamate (100 mg, 0.54 mmol).
¹H NMR (CDCl₃, 400MHz) : δ=1. 46 (s, 9H), 1.9-2. 0 (m, 1H), 2.2-2.4 (m, 1H), 3.15 (dd, 1H, J=4, 9Hz), 3.29 (dt, 1H, J=6, 9Hz), 3.3-3.5 (m, 1H), 3.53 (dd, 1H, J=6, 10Hz), 3.96 (s, 3H), 4.38 (br s, 1H), 4.75 (d, 1H, J=7Hz), 7.12 (d, 1H, J=3Hz), 7.62 (d, 1H, J=3Hz).

### Reference Example 48-2

### (R)-1-(6-methoxy-5-(trifluoromethyl)pyridin-3-yl)pyrrolidin-3-amine

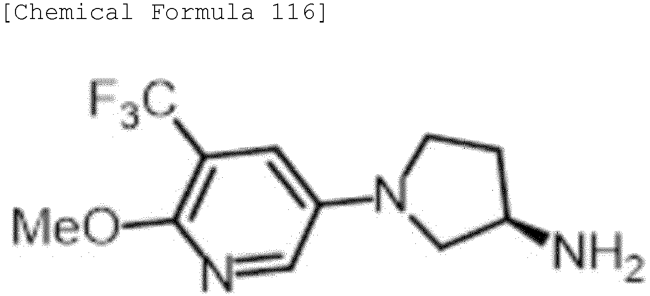

According to a technique similar to Reference Example 1-2, the title compound (pale yellow syrup, 18 mg, 100%) was obtained using tert-butyl (R)-(1-(6-methoxy-5-(trifluoromethyl)pyridin-3-yl)pyrrolidin-3-yl)carbamate (26 mg, 0.07 mmol) synthesized in Reference Example 48-1.
¹H NMR (CDCl₃, 400MHz) : δ=1.7-1.9 (m, 1H) 2.2-2.3 (m, 1H), 3.01 (dd, 1H, J=5, 9Hz), 3.30 (dt, 1H, J=6,8Hz), 3.4-3.5 (m, 2H), 3.76 (quin, 1H, J=6Hz), 3.96 (s, 3H), 7.11 (d, 1H, J=3Hz), 7.62 (d, 1H, J=3Hz). The 2H content is not observable.

### Reference Example 49-1

### Tert-butyl (R)-(1-(4-(tert-butyl)thiazol-2-yl)pyrrolidin-3-yl) carbamate

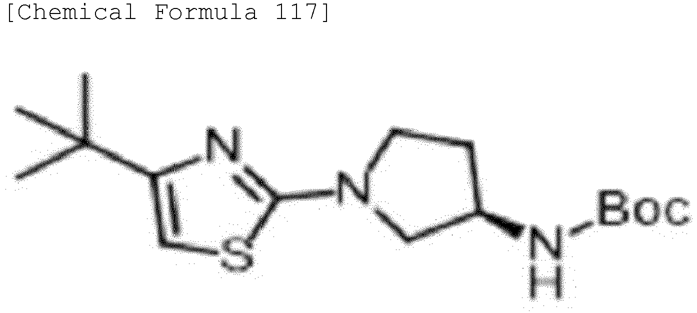

According to a technique similar to Reference Example 9, the title compound (colorless oil, 103 mg, 70%) was obtained using 2-bromo-4-(tert-butyl)thiazole (100 mg, 0.45 mmol) and tert-butyl (R)-pyrrolidin-3-ylcarbamate (127 mg, 0.68 mmol).
¹H NMR (CDCl₃, 400MHz) : δ=1.27 (s, 9H), 1.45 (s, 9H), 1.9-2.0 (m, 1H), 2.2-2.4 (m, 1H), 3.31 (dd, 1H, J=4, 10Hz), 3.4-3.6 (m, 2H)), 3.71 (dd, 1H, J=6, 11Hz), 4.35 (br s, 1H), 4.74(br s, 1H), 6.06 (s, 1H).

### Reference Example 49-2

### (R)-1-(4-(tert-butyl)thiazol-2-yl)pyrrolidin-3-amine

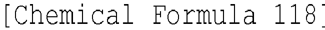

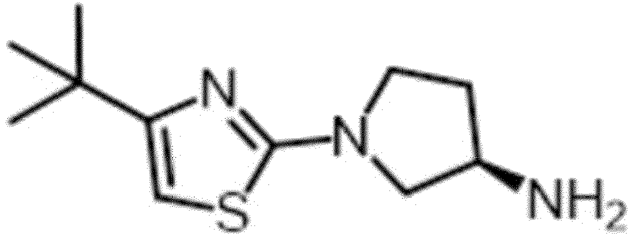

According to a technique similar to Reference Example 1-2, the title compound (colorless oil, 65 mg, 91%) was obtained using tert-butyl (R)-(1-(4-(tert-butyl)thiazol-2-yl)pyrrolidin-3-yl)carbamate (103 mg, 0.32 mmol) synthesized in Reference Example 49-1.
¹H NMR (CDCl₃, 400MHz) : δ=1. 27 (s, 9H), 1.7-1.9 (m, 1H), 2.1-2.3 (m, 1H), 3.17 (dd, 1H, J=4, 10Hz), 3.4-3.8 (m, 4H), 6.04 (s, 1H). The 2H content is not observable.

### Reference Example 50

### Tert-butyl (R)-(1-(6-(trifluoromethyl)pyridazin-3-yl)pyrrolidin-3-yl)carbamate

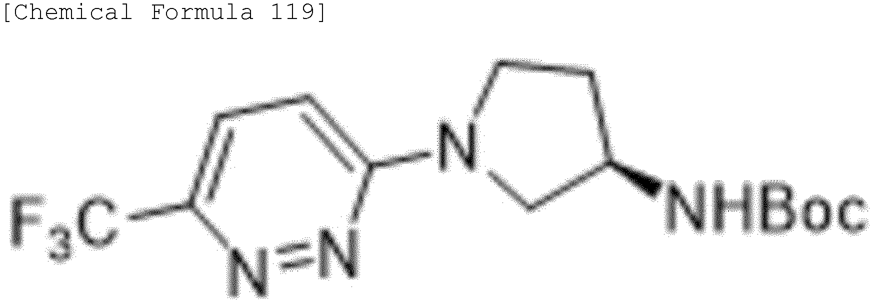

According to a technique similar to Reference Example 9, the title compound (white powder, 90 mg, 84%) was obtained using 3-chloro-6-(trifluoromethyl)pyridazine (60 mg, 0.322 mmol) and tert-butyl (R)-pyrrolidin-3-ylcarbamate (59 mg, 0.322 mmol).
¹H NMR (CDCl₃, 400MHz) : δ=1.46 (s, 9H), 2.0-2.1 (m, 1H), 2.3-2.4 (m, 1H), 3.4-4.0 (m, 4H), 4.41 (br s, 1H), 4.71 (br s, 1H), 6. 67 (d, 1H, J=10Hz), 7.48 (d, 1H, J=10Hz).

### Reference Example 51

### Tert-butyl (R)-(1-(2-(trifluoromethyl)pyrimidin-5-yl)pyrrolidin-3-yl)carbamate

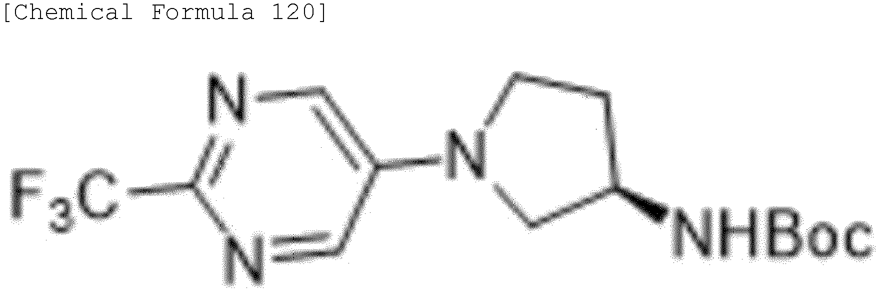

According to a technique similar to Reference Example 1-1, the title compound (yellow powder, 129 mg, 72%) was obtained using 5-bromo-2-(trifluoromethyl)-pyrimidine (134 mg, 0.591 mmol) and tert-butyl (R)-pyrrolidin-3-ylcarbamate (100 mg, 0.537 mmol).
¹H NMR (CDCl₃, 400MHz) : δ=1.46 (s, 9H), 2.0-2.1 (m, 1H), 2.3-2.5 (m, 1H), 3.28 (dd, 1H, J=4, 10Hz), 3.4-3.6 (m, 2H), 3.70 (dd, 1H, J=6, 10Hz), 4.42 (br s, 1H), 4.69 (br s, 1H), 8.10(s,2H).

### Reference Example 52

### Tert-butyl (R)-(1-(6-fluorobenzo[d]thiazol-2-yl)pyrrolidin-3-yl) carbamate

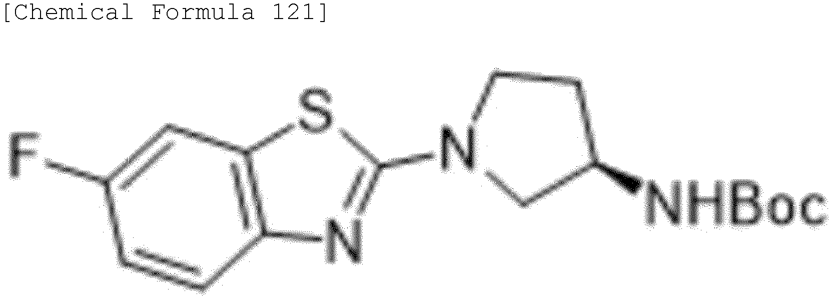

Tert-butyl (R)-pyrrolidin-3-ylcarbamate (50 mg, 0.268 mmol) and 2-chloro-6-fluorobenzothiazole (58 mg, 0.309 mmol) were dissolved in DMF (1 mL), subsequently cesium carbonate (101 mg, 0.309 mmol) was added thereto, and the mixture was stirred for 2 hours at 100°C. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 25 to 40%), and the title compound (pale yellow powder, 85 mg, 94%) was obtained.
¹H NMR (CDCl₃, 400MHz) : δ=1.46 (s, 9H), 2.0-2.1 (m, 1H), 2.3-2.4 (m, 1H), 3.44 (dd, 1H, J=4, 10Hz), 3.6-3.8 (m, 2H), 3.83 (dd, 1H, J=6, 10Hz), 4.41 (br s, 1H), 4.70 (br s, 1H), 7.02 (dd, 1H, J=3, 9Hz), 7.32 (dd, 1H, J=3, 8Hz), 7.50 (dd, 1H, J=5, 9Hz)

### Reference Example 53

### Tert-butyl (R)-(1-(6-fluoroquinolin-2-yl)pyrrolidin-3-yl)carbamate

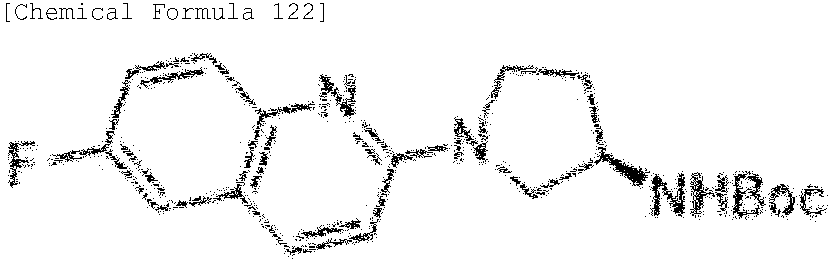

Tert-butyl (R)-pyrrolidin-3-ylcarbamate (50 mg, 0.268 mmol) and 2-chloro-6-fluoroquinoline (54 mg, 0.295 mmol) were dissolved in toluene (1.5 mL) and water (150 µL), subsequently potassium carbonate (44 mg, 0.322 mmol) were added thereto, and the mixture was heated to reflux overnight in a nitrogen atmosphere. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 20 to 32%), and the title compound (white powder, 39 mg, 44%) was obtained.
¹H NMR (CDCl₃, 400MHz) : δ=1.46 (s, 9H), 1.9-2.1 (m, 1H), 2.2-2.4 (m, 1H), 3.51 (dd, 1H, J=4, 10Hz), 3.6-3.8 (m, 2H), 3.87 (dd, 1H, J=6, 10Hz), 4.39 (br s, 1H), 4.73 (br s, 1H), 6.75 (d, 1H, J=9Hz), 7.2-7.4 (m, 2H), 7.67 (dd, 1H, J=6, 9Hz), 7.82 (d, 1H, J=9Hz) .

### Reference Example 54-1

### (1R,2S,5R)-2-isopropyl-5-methylcyclohexyl(1S,2S)-2-(1-cyclopropyl-1H-benzo[d]imidazol-2-yl)cyclopropane-1-carboxylate

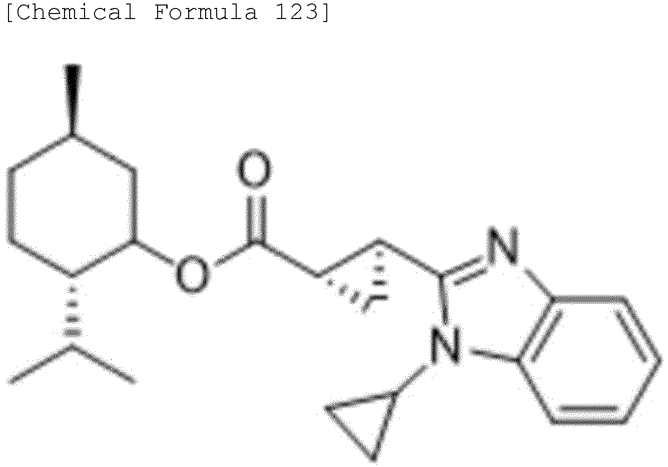

(1R,2S,5R)-2-isopropyl-5-methylcyclohexyl (1S,2S)-2-(1H-benzo[d]imidazol-2-yl)cyclopropane-1-carboxylate (408 mg, 1.1 mmol), cyclopropylboronic acid (184 mg, 2.1 mmol), and sodium carbonate (227 mg, 2.1 mmol) were dissolved in toluene (4 mL), subsequently copper(II) acetate monohydrate (214 mg, 1.1 mmol) and 2,2'-bipyridyl (167 mg, 1.1 mmol) suspended in toluene (7 mL) were added thereto, and the mixture was stirred overnight at 70°C. A saturated aqueous solution of ammonium chloride and water were added to the reaction liquid, and the mixture was extracted with ethyl acetate. An organic layer thus separated was washed with saturated brine and dried over anhydrous sodium sulfate, insoluble materials were filtered, and then the solvent was distilled off under reduced pressure. A residue thus obtained was purified by silica gel column chromatography (hexane : ethyl acetate) (concentration gradient: 10% to 25%), and the title compound (colorless oily material, 270 mg, 66%) was obtained.
¹H NMR (CDCl₃, 400MHz) : δ=0.80 (d, 3H, J=7Hz), 0.9-1.5 (m, 15H), 1.6-2.1 (m, 6H), 2.32 (ddd, 1H, J=4, 6, 9Hz), 2.87 (ddd, 1H, J=4, 6, 9Hz), 3.30 (ddd, 1H, J=4, 7, 11Hz), 4.73 (td, 1H, J=4, 11Hz), 7.2-7.3 (m, 2H), 7.4-7.7 (m, 2H).

### Reference Example 54-2

### (1S,2S)-2-(1-cyclopropyl-1H-benzo[d]imidazol-2-yl)cyclopropane-1-carboxylic acid

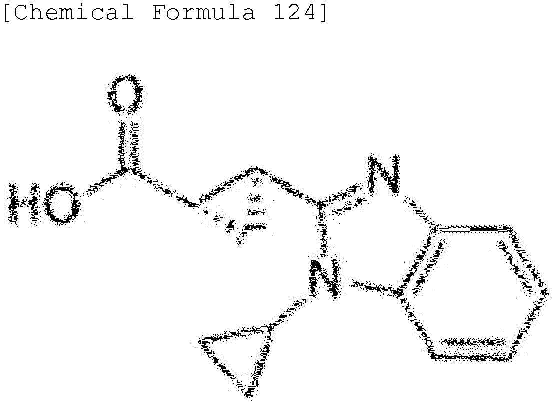

(1R,2S,5R)-2-isopropyl-5-methylcyclohexyl (1S,2S)-2-(1-cyclopropyl-1H-benzo[d]imidazol-2-yl) cyclopropane-1-carboxylate (270 mg, 0.709 mmol) synthesized in Reference Example 54-1 was suspended in isopropanol (2 mL) and water (150 µL), subsequently sodium hydroxide (57 mg, 1.42 mmol) was added thereto, and the mixture was stirred for 5 hours at 80°C. The solvent was distilled off under reduced pressure, subsequently a 0.2 Normal aqueous solution of sodium hydroxide (20 mL) was added to a residue thus obtained, and the mixture was washed twice with diethyl ether. 1 Normal hydrochloric acid (15 mL) was added to the aqueous layer to made the aqueous layer acidic, subsequently to a residue obtained by distilling off the solvent, toluene and water were added, and the mixture was suspended. A solid thus obtained was collected by filtration and dried under reduced pressure at room temperature, and the title compound (white powder, 48 mg, 28%) was obtained.
¹H NMR (DMSO-d₆, 400MHz) : δ=1. 1-1.4 (m, 4H), 1.71 (quin, 1H, J=5Hz), 1.8-1.9 (m, 1H), 2.3-2.5 (m, 1H), 2.93 (ddd, 1H, J=4, 6, 9Hz), 3.5-3. 7(m, 1H), 7.44(quin, 2H, J=8Hz), 7.67 (d, 1H, J=8Hz), 7.77 (d, 1H, J=8Hz) . The 1H content is not observable.

### Reference Example 55

### Tert-butyl (R)-(1-(5-bromopyridin-3-yl)pyrrolidin-3-yl)carbamate

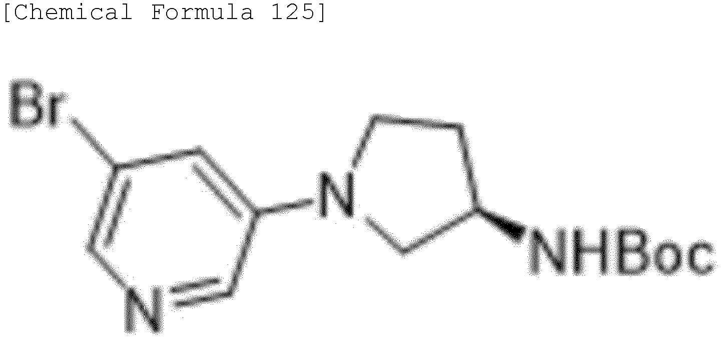

According to a technique similar to Reference Example 1-1, the title compound (white powder, 71 mg, 83%) was obtained using 3,5-dibromopyridine (178 mg, 0.751 mmol) and tert-butyl (R)-pyrrolidin-3-ylcarbamate (47 mg, 0.252 mmol) .
¹H NMR (CDCl₃, 400MHz) : δ=1.46 (s, 9H), 1.9-2.1 (m, 1H), 2.2-2.4 (m, 1H), 3.17 (dd, 1H, J=4, 10Hz), 3.3-3.5 (m, 2H), 3.59 (dd, 1H, J=6, 10Hz), 4.38 (br s, 1H), 4.68 (br s, 1H), 6.95 (t, 1H, J=2Hz), 7.78 (d, 1H, J=3Hz), 8.80 (d, 1H, J=2Hz).

### Reference Example 56

### 2-(4-(Trifluoromethyl)phenyl)acetic acid-2,2-d₂

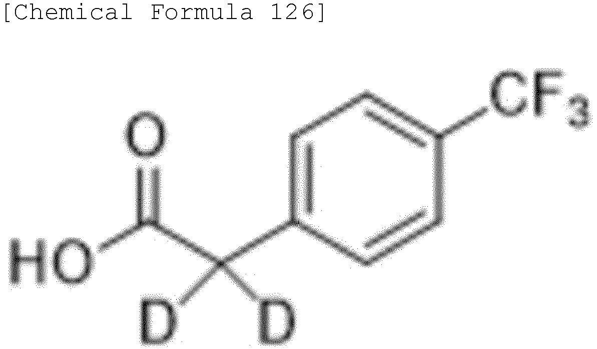

4-(Trifluoromethyl)phenylacetic acid (50 mg, 0.245 mmol) was dissolved in deuterated water (1 mL), subsequently a 40% solution of sodium deuteroxide (0.5 mL, 7.00 mmol) was added thereto, and the mixture was heated to reflux overnight in a nitrogen atmosphere. Deuterated water was added to the reaction liquid that had been left to cool to room temperature, the mixture was washed with diethyl ether, subsequently 3 N hydrochloric acid was added to the aqueous layer to make the aqueous layer acidic, and the aqueous layer was extracted with diethyl ether. An organic layer thus separated was dried over anhydrous sodium sulfate, insoluble materials were filtered, and then the solvent was distilled off under reduced pressure. A residue thus obtained was suspended in chloroform, insoluble materials were filtered, subsequently the solvent was distilled off under reduced pressure, and the title compound (white powder, 30 mg, 59%) was obtained.
¹H NMR (DMSO-d₆, 400MHz) : δ=7.43 (d, 2H, J=8Hz), 7.59(d,2H,J=8Hz). The 1H content is not observable.

### Reference Example 57

### (R)-2-(4-bromophenyl)-N-(1-(6-(trifluoromethyl)pyridin-3-yl)pyrrolidin-3-yl)acetamide

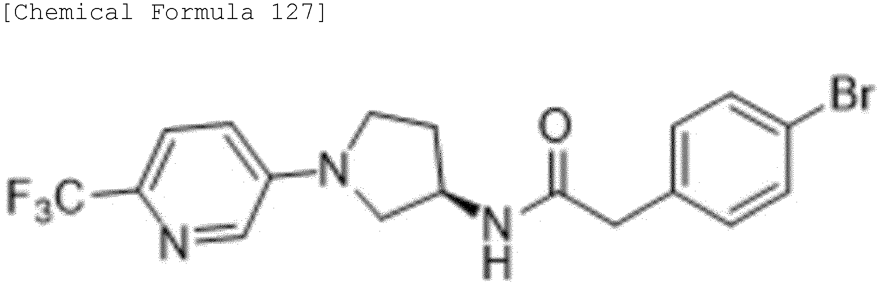

According to a technique similar to Reference Example 7-1, the title compound (pale yellow powder, 88 mg, 95%) was obtained using (R)-1-(6-(trifluoromethyl)pyridin-3-yl)pyrrolidin-3-amine (50 mg, 0.216 mmol) synthesized in Reference Example 32-2 and 4-bromophenylacetic acid (56 mg, 0.259 mmol).
¹H NMR (CDCl₃, 400MHz) : δ=1.9-2.0 (m, 1H), 2.3-2.4 (m, 1H), 3.16 (dd, 1H, J=4, 10Hz), 3.3-3.5 (m, 2H), 3.53 (s, 2H), 3.68 (dd, 1H, J=6, 10Hz), 4.5-4.7 (m, 1H), 5.52 (br s, 1H), 6.80 (dd, 1H, J=3, 9Hz), 7.14 (d, 2H, J=9Hz), 7.4-7.5 (m, 3H), 7.98 (d, 1H, J=3Hz) .

### Reference Example 58-1

### Tert-butyl (1-(5-(trifluoromethyl)pyridin-3-yl)azetidin-3-yl)carbamate

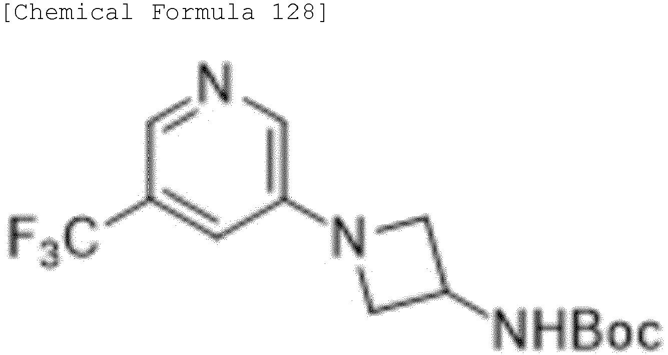

According to a technique similar to Reference Example 4, the title compound (pale yellow powder, 112 mg, 25%) was obtained using 3-bromo-5-(trifluoromethyl)pyridine (390 mg, 1.73 mmol) and tert-butyl azetidin-3-ylcarbamate hydrochloride (300 mg, 1.44 mmol).
¹H NMR (CDCl₃, 400MHz) : δ=1.46 (s, 9H), 3.76 (dd, 2H, J=6, 8Hz), 4.32 (t, 2H, J=7Hz), 4.69 (br s, 1H), 4.99 (br s, 1H), 6.86 (s, 1H), 7.99 (d, 1H, J=3Hz), 8.27 (s, 1H).

### Reference Example 58-2

### 1-(5-(Trifluoromethyl)pyridin-3-yl)azetidin-3-amine

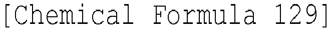

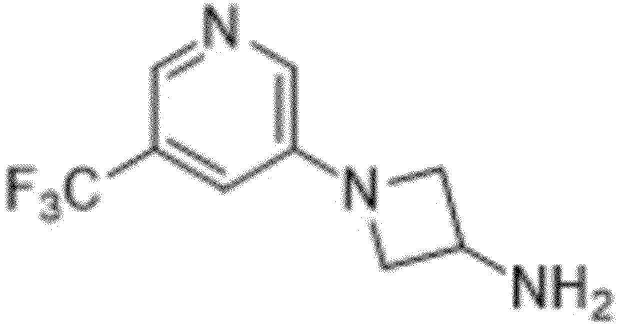

According to a technique similar to Reference Example 1-2, the title compound (white powder, 60 mg, 79%) was obtained using tert-butyl (1-(5-(trifluoromethyl)pyridin-3-yl)azetidin-3-yl)carbamate (112 mg, 0.353 mmol) synthesized in Reference Example 58-1.
¹H NMR (CDCl₃, 400MHz) : δ=3.59 (dd, 2H, J=6, 8Hz), 4.0-4.1 (m, 1H), 4.27 (t, 2H, J=7Hz), 6.85 (t, 1H, J=2Hz), 7.99 (d, 1H, J=3Hz), 8.24 (s, 1H). The 2H content is not observable.

### Reference Example 59

### Benzyl (R)-4-(4-(2-oxo-2-((1-(5-(trifluoromethyl)-pyridin-3-yl)pyrrolidin-3-yl)amino)ethyl)phenyl)piperazine-1-carboxylate

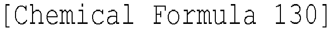

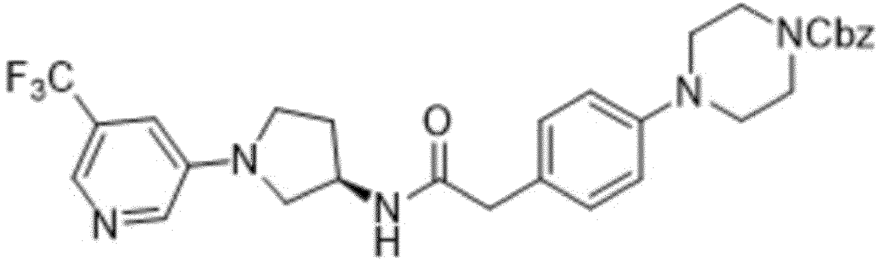

According to a technique similar to Reference Example 7-1, the title compound (yellow powder, 176 mg, 90%) was obtained using (R)-1-(5-(trifluoromethyl)pyridin-3-yl)pyrrolidin-3-amine (80 mg, 0.346 mmol) synthesized in Reference Example 1-2 and 2-(4-(4-((benzyloxy)-carbonyl)piperazin-1-yl)phenyl)acetic acid (135 mg, 0.381 mmol) .
¹H NMR (CDCl₃, 400MHz) : δ=1.8-2.0 (m, 1H), 2.2-2.4 (m, 1H), 3.0-3.2(m,5H),3.37(t,2H,J=7Hz),3.51(s,2H),3.6-3.7(m,5H), 4.5-4.7 (m, 1H), 5.16 (s, 2H), 5.53 (d, 1H, J=7Hz), 6.8-6.9 (m, 3H), 7.14 (d, 2H, J=9Hz), 7.3-7.4 (m, 5H), 8.07 (d, 1H, J=3Hz), 8.20 (s, 1H).

### Reference Example 60

### Ethyl 2-(4-(1,4-dioxa-8-azaspiro[4.5]decan-8-yl)phenyl)acetate

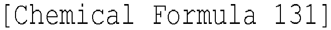

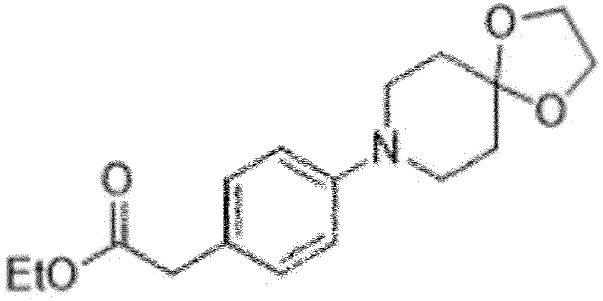

According to a technique similar to Reference Example 23-1, the title compound (yellow oily material, 16 mg, 4%) was obtained using ethyl 2-(4-bromophenyl)acetate (300 mg, 1.23 mmol) and 4-piperidone ethylene ketal (317 µL, 2.46 mmol).
¹H NMR (CDCl₃, 400MHz) :δ=1.25(t,3H,J=7Hz), 1.84(t,4H,J=6Hz),3.31(t,4H,J=6Hz),3.52(s,2H),3.99(s,4H), 4.14(q,2H,J=7Hz),6.90(d,2H,J=9Hz),7.16(d,2H,J=9Hz).

### Reference Example 61-1

### 7-(5-(Trifluoromethyl)pyridin-3-yl)-3-oxa-1,7-diazaspiro[4.4]nonan-2-one

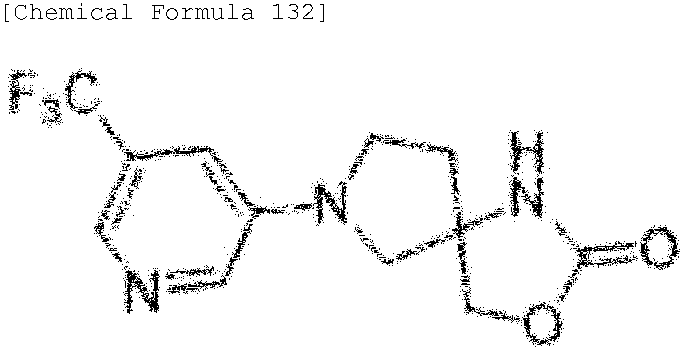

According to a technique similar to Reference Example 1-1, the title compound (white powder, 106 mg, 51%) was obtained using 3-bromo-5-(trifluoromethyl)pyridine (162 mg, 0.717 mmol) and benzyl N-(3-(hydroxymethyl)pyrrolidin-3-yl)carbamate (189 mg, 0.753 mmol).
¹H NMR(CDCl₃,400MHz):5=2.2-2.5(m,2H),3.4-3.6(m,4H), 4.41(d,1H,J=9Hz),4.45(d,1H,J=9Hz),5.57(s,1H), 6.97(t,1H,J=2Hz),8.14(d,1H,J=3Hz),8.29(s,1H).

### Reference Example 61-2

### (3-Amino-1-(5-(trifluoromethyl)pyridin-3-yl)pyrrolidin-3-yl)methanol

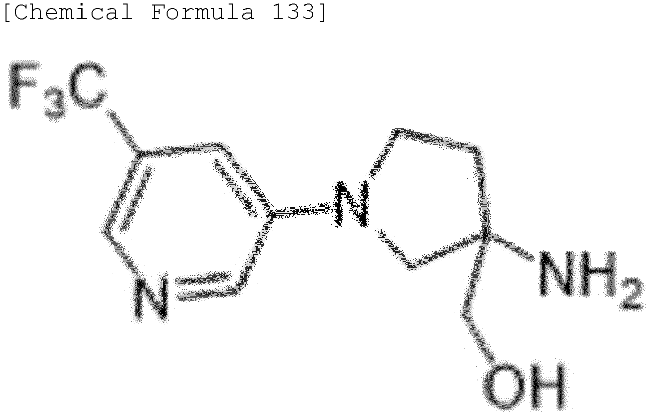

7-(5-(Trifluoromethyl)pyridin-3-yl)-3-oxa-1,7-diazaspiro[4.4]nonan-2-one (95 mg, 0.331 mmol) synthesized in Reference Example 61-1 was dissolved in ethanol (4 mL) and water (1 mL), lithium hydroxide monohydrate (278 mg, 6.61 mmol) was added thereto, and the mixture was heated to reflux overnight in a nitrogen atmosphere. To the reaction liquid that had been left to cool to room temperature, water was added, the mixture was stirred for a while, and then the mixture was extracted with ethyl acetate. An organic layer thus separated was washed with saturated brine and dried over anhydrous sodium sulfate, insoluble materials were filtered, subsequently the solvent was distilled off under reduced pressure, and the title compound (yellow powder, 86 mg, 100%) was obtained.
¹H NMR(CDCl₃,400MHz):δ=1.8-2.0(m,1H),2.1-2.2(m,1H), 3.19(d,1H,J=10Hz),3.4-3.7(m,5H),6.93(t,1H,J=2Hz), 8.11(d,1H,J=3Hz),8.20(s,1H). The 3H content is not observable.

### Reference Example 62

### 2-(4-Isopropylphenyl)-N-(piperidin-4-yl)acetamide

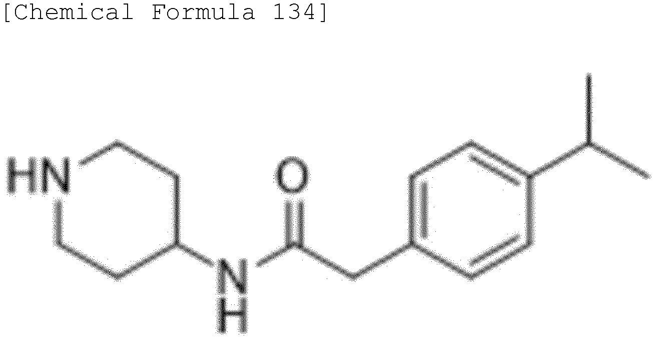

4-Amino-1-tert-butoxycarbonylpiperidine (1.00 g, 4.54 mmol) and 2-(4-isopropylphenyl)acetic acid (0.971 g, 5.45 mmol) were dissolved in DMF (13 mL), subsequently DIPEA (2.3 mL, 13.6 mmol) and HATU (2.42 g, 6.36 mmol) were added thereto, and the mixture was stirred overnight at room temperature. Water was added to the reaction liquid, the mixture was stirred for a while, and then the mixture was extracted with ethyl acetate. An organic layer thus separated was washed with saturated brine and dried over anhydrous sodium sulfate, insoluble materials were filtered, subsequently the solvent was distilled off under reduced pressure, and a crude form of tert-butyl 4-(2-(4-isopropylphenyl)acetamido)piperidine-1-carboxylate (dark brown oily material, 2.45 g) was obtained. According to a technique similar to Reference Example 1-2, the title compound (ocher-colored crystals, 0.911 g, 77%) was obtained using the crude form of tert-butyl 4-(2-(4-isopropylphenyl)acetamido)piperidine-1-carboxylate (2.45 g) thus obtained.
¹H NMR(CDCl₃,400MHz):δ=1.1-1.3(m,9H),1.8-1.9(m,2H), 2.65(td,2H,J=3,12Hz),2.8-3.0(m,3H),3.52(s,2H), 3.8-4.0(m,1H),5.24(d,1H,J=6Hz),7.1-7.3(m,4H).

### Reference Example 63

### (R)-2-(4-isopropylphenyl)-N-(pyrrolidin-3-yl)acetamide

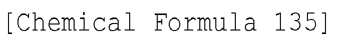

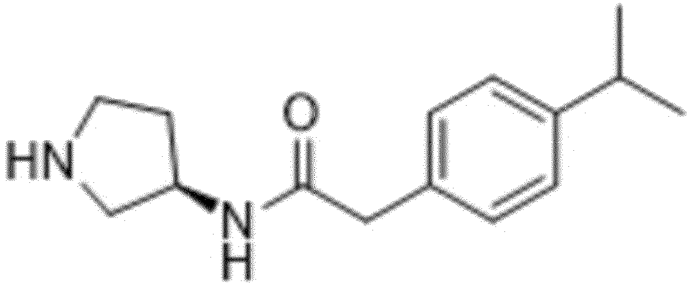

According to a technique similar to Reference Example 62, a crude form of tert-butyl (R)-3-(2-(4-isopropylphenyl)acetamido)pyrrolidine-1-carboxylate (4.25 g) was synthesized using (3R)-(+)-1-(tert-butoxycarbonyl)-3-aminopyrrolidine (2.00 g, 10.7 mmol) and 2-(4-isopropylphenyl)acetic acid (2.30 g, 12.9 mmol), and then the title compound (pale peach-colored crystals, 2.22 g, 84%) was obtained.
¹H NMR (CDCl₃, 400MHz) :δ=1.25(d,6H,J=7Hz), 1.4-1.5(m,1H),2.0-2.2(m,1H),2.65(dd,1H,J=4,11Hz), 2.8-3.0(m,3H),3.11(dd,1H,J=7,11Hz),3.51(s,2H), 4.3-4.4(m,1H),5.55(brs,1H),7.16(d,2H,J=8Hz), 7.21(d,2H,J=8Hz). The 1H content is not observable.

### Reference Example 64-1

### Tert-butyl (S)-(1-(5-(trifluoromethyl)pyridin-3-yl)pyrrolidin-3-yl)carbamate

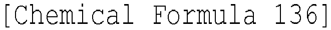

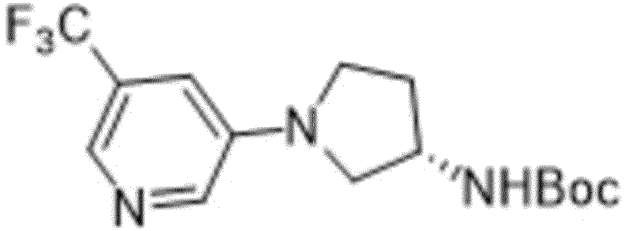

According to a technique similar to Reference Example 4, the title compound (pale yellow crystals, 185 mg, 20%) was obtained using tert-butyl (S)-pyrrolidin-3-ylcarbamate (500 mg, 2.68 mmol) and 5-bromo-2-(trifluoromethyl)pyridine (728 mg, 3.22 mmol).
¹H NMR (CDCl₃, 400MHz) :δ=1.46(s,9H),1.9-2.1(m,1H), 2.3-2.4(m,1H),3.23(dd,1H,J=4,10Hz),3.3-3.6(m,2H), 3.65(dd,1H,J=6,10Hz),4.40(brs,1H),4.71(brs,1H), 6.94(s,1H),8.12(d,1H,J=3Hz),8.21(s,1H).

### Reference Example 64-2

### (S)-1-(5-(trifluoromethyl)pyridin-3-yl)pyrrolidin-3-amine

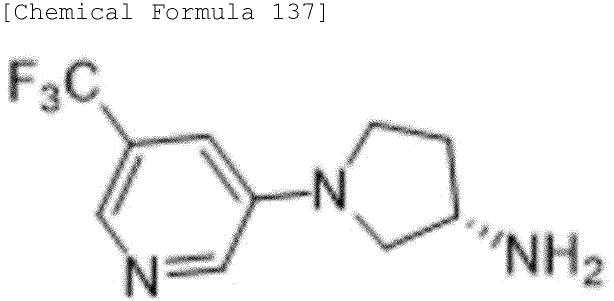

According to a technique similar to Reference Example 1-2, the title compound (pale yellow crystals, 100 mg, 77%) was obtained using tert-butyl (S)-(1-(5-(trifluoromethyl)pyridin-3-yl)pyrrolidin-3-yl)carbamate (184 mg, 0.555 mmol) synthesized in Reference Example 64-1.
¹H NMR (CDCl₃, 400MHz) :δ=1.8-2.0 (m,1H),2.2-2.4 (m,1H), 3.08(dd,1H,J=5,10Hz),3.3-3.6(m,3H), 3.81(quin,1H,J=6Hz),6.92(s,1H),8.11(d,1H,J=3Hz),8.17(s,1H). The 2H content is not observable.

### Reference Example 65

### Tert-butyl 3-(2-(4-isopropylphenyl)-acetamido)azetidine-1-carboxylate

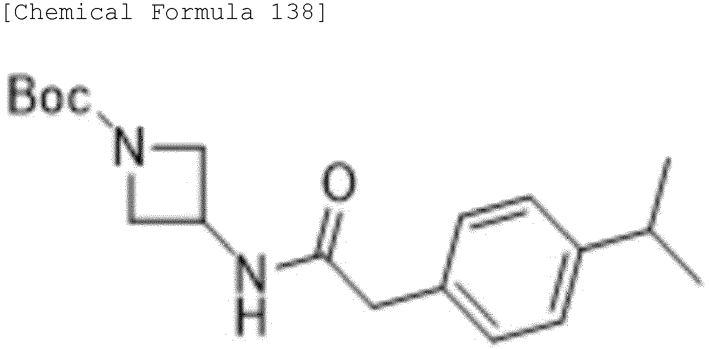

According to a technique similar to Reference Example 7-1, the title compound (pale yellow amorphous, 964 mg, 100%) was obtained using tert-butyl 3-aminoazetidine-1-carboxylate (500 mg, 2.90 mmol) and 2-(4-isopropylphenyl)-acetic acid (624 mg, 3.50 mmol).
¹H NMR (CDCl₃, 400MHz) :δ=1.26 (d, 6H, J=7Hz), 2.8-3.0(m,1H),3.54(s,2H),3.5-3.7(m,2H),4.2-4.4(m,2H),4.5-4.7(m,1H),6.6-6.8(m,1H),7.15(d,2H,J=8Hz),7.23(d,2H,J=8Hz).

### Reference Example 66

### Tert-butyl (R)-(1-(5-(trifluoromethyl)pyridin-3-yl)piperidin-3-yl) carbamate

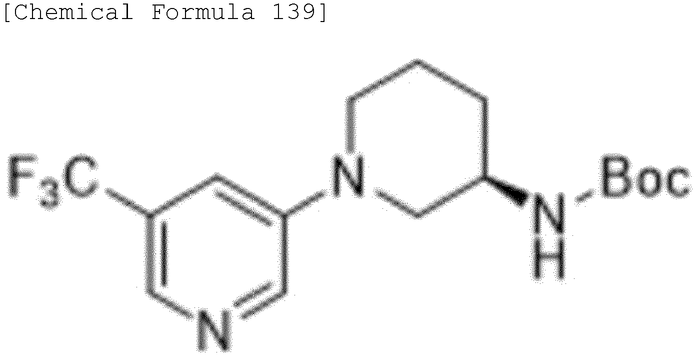

According to a technique similar to Reference Example 4, the title compound (yellow amorphous, 100 mg, 20%) was obtained using 3-bromo-5-(trifluoromethyl)pyridine (407 mg, 1.80 mmol) and (R)-3-(tert-butoxycarbonylamino)-piperidine (300 mg, 1.50 mmol).
¹H NMR (CDCl₃, 400MHz) :δ=1.46 (s,9H),1.6-1.8 (m,1H), 1.8-2.0(m,2H),2.9-3.2(m,2H),3.2-3.4(m,1H),3.5-3.6(m,1H), 3.8-3.9(m,1H),4.7-4.8(m,1H),7.33(s,1H),8.32(s,1H), 8.46(d,1H,J=2Hz). The 1H content is not observable.

### Reference Example 67

### Tert-butyl (1-(4-cyclopropylphenyl)-2-oxo-2-(((R)-1-(5-(trifluoromethyl)pyridin-3-yl)pyrrolidin-3-yl)amino)ethyl)carbamate

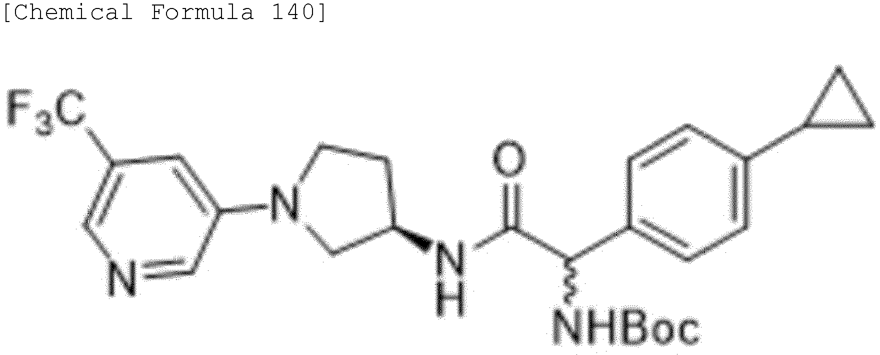

According to a technique similar to Reference Example 7-1, the title compounds (diastereomer A: Rf value in TLC (ethyl acetate : heptane = 1 : 1) = 0.2, yellow amorphous, 15 mg, 5%; diastereomer B: Rf value in TLC (ethyl acetate : heptane = 1 : 1) = 0.18, yellow amorphous, 15 mg, 5%, diastereomer mixture: 290 mg, 88%) were respectively obtained using (R)-1-(5-(trifluoromethyl)-pyridin-3-yl)pyrrolidin-3-amine (150 mg, 0.65 mmol) and 2-((tert-butoxycarbonyl)amino)-2-(4-cyclopropylphenyl)-acetic acid (189 mg, 0.65 mmol).

### Diastereomer A

¹H NMR (CDCl₃, 400MHz) :δ=0.6-0.7(m,2H),0.9-1.0(m,2H), 1.39(s,9H),1.8-1.9(m,1H),2.0-2.1(m,1H),2.3-2.4(m,1H), 3.0-3.1(m,1H),3.3-3.5(m,2H),3.5-3.6(m,1H),4.5-4.7(m,1H), 5.0-5.1(m,1H),5.5-5.7(m,1H),6.2-6.3(m,1H),6.83(s,1H), 7.00(d,2H,J=8Hz),7.19(d,2H,J=8Hz),8.00(d,1H,J=3Hz), 8.14(s,1H).

### Diastereomer B

¹H NMR (CDCl₃, 400MHz) :δ=0.6-0.8 (m, 2H),0.9-1.0 (m,2H), 1.40(s,9H)1.8-2.0(m,2H),2.2-2.3(m,1H),3.2-3.4(m,3H), 3.6-3.7(m,1H),4.6-4.7(m,1H),5.0-5.1(m,1H),5.5-5.7(m,1H), 6.2-6.3(m,1H),6.85(s,1H),7.04(d,2H,J=8Hz),7.22(d,2H,J=8Hz), 8.01(d,1H,J=3Hz),8.12(s,1H).

### Reference Example 68

### Tert-butyl (R)-(3-methyl-1-(5-(trifluoromethyl)-pyridin-3-yl)pyrrolidin-3-yl)carbamate

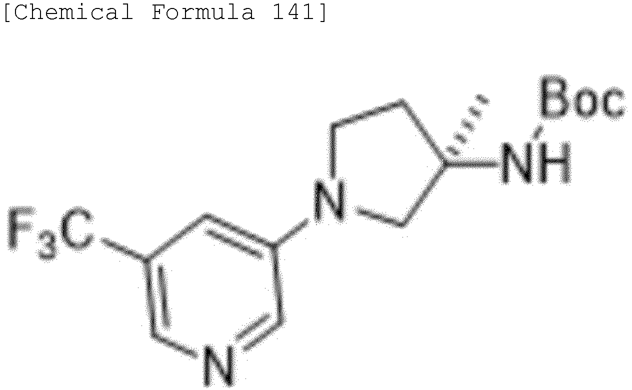

According to a technique similar to Reference Example 4, the title compound (yellow amorphous, 50 mg, 63%) was obtained using 3-bromo-5-(trifluoromethyl)pyridine (79 mg, 0.23 mmol) and tert-butyl (R)-(3-methylpyrrolidin-3-yl)carbamate (47 mg, 0.23 mmol).
¹H NMR (CDCl₃, 400MHz) :δ=1.45(s,9H),1.54(s,3H), 1.9-2.1(m,1H), 2.3-2.5(m,1H), 3.3-3.5(m,3H), 3.5-3.6(m,1H),4.66(brs,1H),6.92(s,1H),8.10(d,1H,J=3Hz), 8.19(s,1H).

### Reference Example 69

### Ethyl 2-(4-(3-oxa-8-azabicyclo[3.2.1]octan-8-yl)phenyl) acetate

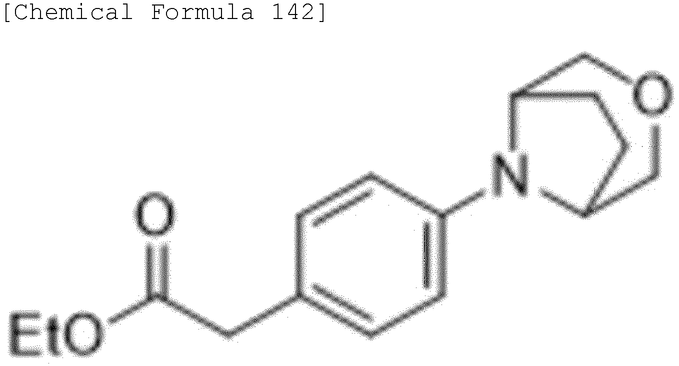

According to a technique similar to Reference Example 27, the title compound (pale yellow amorphous, 87 mg, 70%) was obtained using ethyl 4-bromophenylacetate (109 mg, 0.45 mmol) and 3-oxa-8-azabicyclo[3.2.1]octane hydrochloride (100 mg, 0.67 mmol).
¹H NMR (CDCl₃, 400MHz) :δ=1.25(t,3H,J=7Hz),1.9-2.1(m,4H),3.51(s,2H),3.4-3.6(m,2H),3.91(d,2H,J=10Hz), 4.0-4.1(m,2H),4.14(q,2H,J=7Hz),6.74(d,2H,J=8Hz), 7.15 (d,2H, J=8Hz) .

### Reference Example 70

### Ethyl 2-(4-((1S,4S)-2-oxa-5-azabicyclo[2.2.2]octan-5-yl)phenyl) acetate

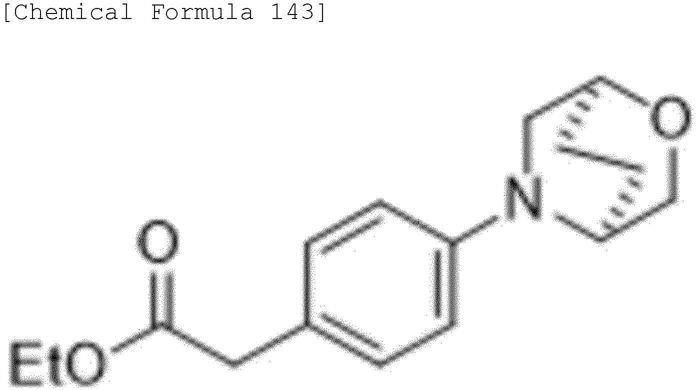

According to a technique similar to Reference Example 27, the title compound (pale yellow amorphous, 109 mg, 88%) was obtained using ethyl 4-bromophenylacetate (109 mg, 0.45 mmol) and (1S,4S)-2-oxa-5-azabicyclo[2.2.2]octane oxalate (106 mg, 0.67 mmol).
¹H NMR (CDCl₃, 400MHz) :δ=1.25(t,3H,J=7Hz), 1.6-1.8(m,1H),1.8-2.0(m,1H),2.0-2.3(m,2H),3.3-3.4(m,1H), 3.51(s,2H),3.7-3.9(m,2H),4.0-4.1(m,2H),4.1-4.2(m,1H), 4.13(q,2H,J=7Hz),6.59(d,2H,J=8Hz),7.16(d,2H,J=8Hz).

### Reference Example 71

### Tert-butyl 3-(trifluoromethyl)-1-(5-(trifluoromethyl)pyridin-3-yl)pyrrolidin-3-yl)carbamate

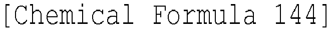

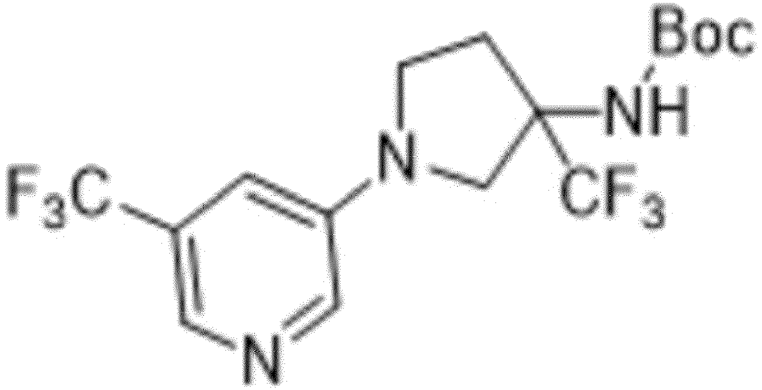

According to a technique similar to Reference Example 4, the title compound (yellow amorphous, 145 mg, 62%) was obtained using 3-bromo-5-(trifluoromethyl)pyridine (160 mg, 0.23 mmol) and tert-butyl 3-(trifluoromethyl)-pyrrolidin-3-ylcarbamate (150 mg, 0.59 mmol).
¹H NMR (CDCl₃, 400MHz) :δ=1 . 45 (s,9H),2 .5-2. 8 (m,2H), 3.5-3.7(m,2H),3.8-3.9(m,1H),3.9-4.0(m,1H),4.85(brs,1H), 6.98(s,1H),8.14(d,1H,J=3Hz),8.26(s,1H).

### Reference Example 72

### Tert-butyl (R)-(1-(6-(trifluoromethyl)pyridin-3-yl)piperidin-3-yl)carbamate

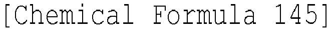

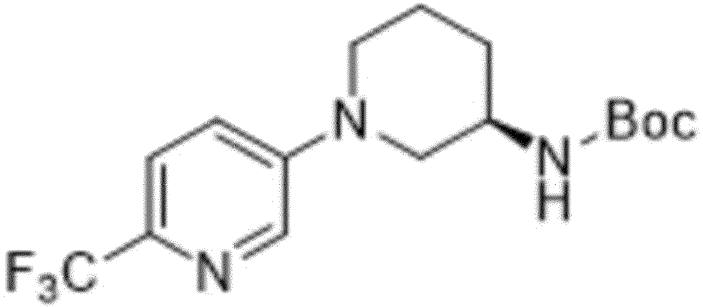

According to a technique similar to Reference Example 4, the title compound (yellow crystals, 400 mg, 77%) was obtained using 5-bromo-2-(trifluoromethyl)pyridine (407 mg, 1.80 mmol) and (R)-3-(tert-butoxycarbonylamino)piperidine (300 mg, 1.50 mmol).
¹H NMR (CDCl₃, 400MHz) : δ=1 . 45 (s, 9H),1.6-2.0(m,4H), 2.9-3.1(m,1H),3.1-3.2(m,1H),3.3-3.5(m,1H),3.6-3.7(m,1H), 3.7-3.8(m,1H),4.65(br s,1H),7.17(d,1H,J=7Hz), 7.49(d,1H,J=7Hz),8.33(s,1H).

### Reference Example 73-1

### Tert-butyl (R)-3-(2-(4-(trifluoromethyl)-phenyl)acetamido)pyrrolidine-1-carboxylate

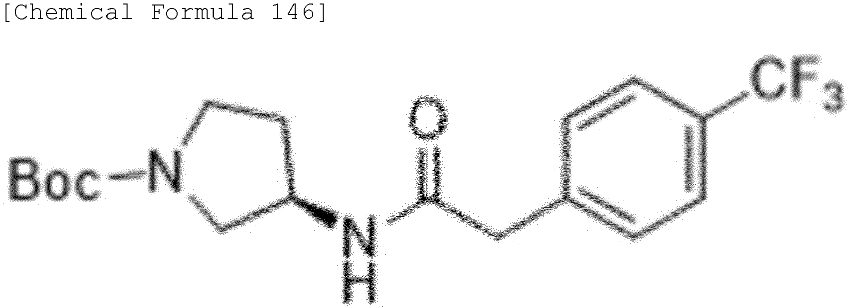

According to a technique similar to Reference Example 7-1, the title compound (white solid, 1.96 g, 88%) was obtained using tert-butyl (R)-aminopyrrolidine-1-carbamate (1.11 g, 6.0 mmol) and 2-(4-(trifluoromethyl)-phenyl)acetic acid (1.35 g, 6.6 mmol).
¹H NMR (CDCl₃, 400MHz) : δ=1 . 45(s,9H),1.6-1.9(m,1H), 2.0-2.2(m,1H),3.0-3.5(m,3H),3.5-3.7(m,1H),3.60(s,2H), 4.3-4.5(m,1H),5.53(br s,1H),7.39(d,2H,J=8Hz), 7.61(d,2H,J=8Hz).

### Reference Example 73-2

### (R)-N-(pyrrolidin-3-yl)-2-(4-(trifluoromethyl)-phenyl)acetamide

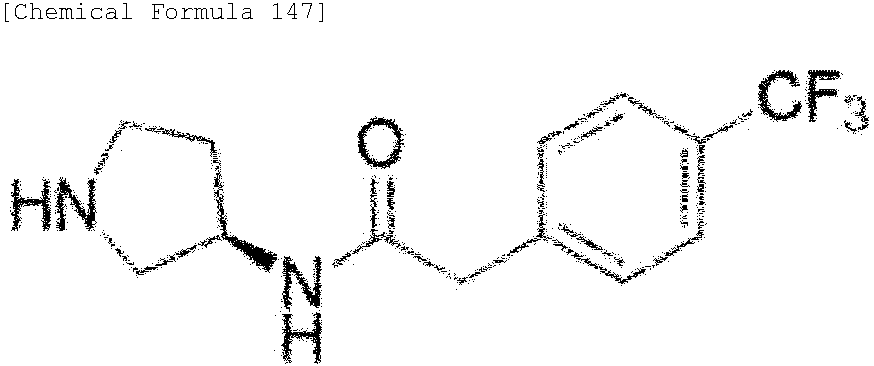

According to a technique similar to Reference Example 1-2, the title compound (white solid, 650 mg, 80%) was obtained using tert-butyl (R)-3-(2-(4-(trifluoromethyl)phenyl)acetamido)pyrrolidine-1-carboxylate (1.12 g, 3.0 mmol) synthesized in Reference Example 73-1.
¹H NMR(CDCl₃, 400MHz) :δ=1.4-1.6(m,1H),2.0-2.2(m,1H), 2.71(dd,1H,J=4,11Hz),2.8-3.1(m,2H),3.10(dd,1H,J=7,12Hz), 3.57(s,2H),4.3-4.4(m,1H),5.69(br s, 1H), 7.39(d,2H,J=8Hz),7.60(d,2H,J=8Hz). The 1H content is not observable.

### Reference Example 74-1

### Tert-butyl (R)-3-(2-(4-cyclopropylphenyl)-acetamido)pyrrolidine-1-carboxylate

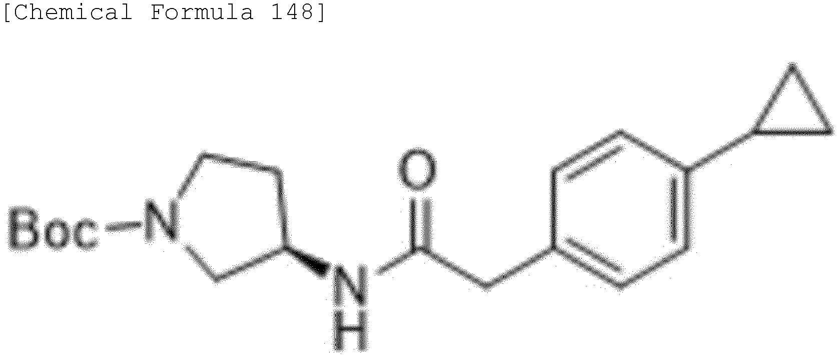

According to a technique similar to Reference Example 7-1, the title compound (yellow crystals, 2.0 g, 70%) was obtained using tert-butyl 3-aminopyrrolidine-1-carboxylate (1.5 mL, 8.29 mmol) and 2-(4-cyclopropylphenyl)acetic acid (1.8 g, 9.95 mmol) synthesized in Reference Example 33-2.
¹H NMR (CDCl₃, 400MHz) : δ=0.6-0.7 (m, 2H),0.9-1.0 (m, 2H), 1.43(s,9H),1.5-1.7(m,1H),1.8-1.9(m,1H),2.0-2.1(m,1H), 2.9-3.1(m,1H),3.2-3.5(m,2H),3.51(s,2H),3.5-3.6(m,1H),4.3-4.5(m,1H),5.3-5.5(m,1H),7.04(d,2H,J=8Hz),7.10(d,2H,J=8Hz).

### Reference Example 74-2

### (R)-2-(4-cyclopropylphenyl)-N-(pyrrolidin-3-yl)acetamide

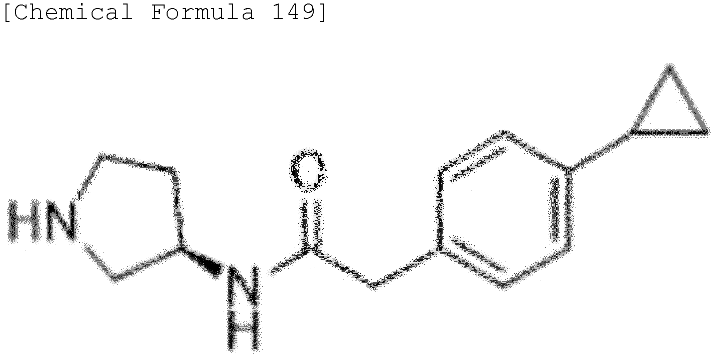

According to a technique similar to Reference Example 1-2, the title compound (pale yellow crystals, 1.1 g, 77%) was obtained using tert-butyl (R)-3-(2-(4-cyclopropylphenyl)acetamido)pyrrolidine-1-carboxylate (2.0 g, 5.81 mmol) synthesized in Reference Example 74-1.
¹H NMR (CDCl₃, 400MHz) : δ=0.6-0.7 (m, 2H),0.9-1.0 (m, 2H), 1.4-1.5(m,1H),1.8-1.9(m,2H),2.0-2.2(m,1H),2.6-2.7(m,1H), 2.8-3.0(m,2H),3.0-3.1(m,1H),3.48(s,2H),4.2-4.4(br s,1H), 5.62(br s,1H),7.03(d,2H,J=8Hz),7.11(d,2H,J=8Hz).

### Reference Example 75-1

### Tert-butyl 3-(2-(4-cyclopropylphenyl)-acetamido)azetidine-1-carbamate

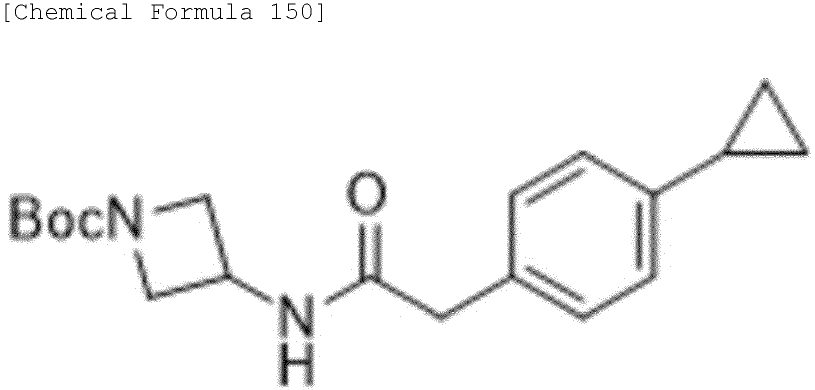

According to a technique similar to Reference Example 7-1, the title compound (white solid, 434 mg, 82%) was obtained using tert-butyl 3-aminoazetidine-1-carbamate (276 mg, 1.6 mmol) and 2-(4-cyclopropylphenyl)acetic acid (352 mg, 2.0 mmol) synthesized in Reference Example 33-2.
¹H NMR (CDCl₃, 400MHz) : δ=0.6-0.8 (m, 2H),0.9-1.1 (m, 2H), 1.41(s,9H),1.8-2.0(m,1H),3.53(s,2H),3.58(dd,2H,J=5,10Hz), 4.28(t,2H,J=9Hz),4.5-4.7(m,1H),5.71(d,1H,J=7Hz), 7.07(d,2H,J=8Hz),7.12(d,2H,J=8Hz).

### Reference Example 75-2

### N-(azetidin-3-yl)-2-(4-cyclopropylphenyl)acetamide

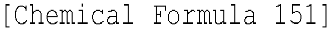

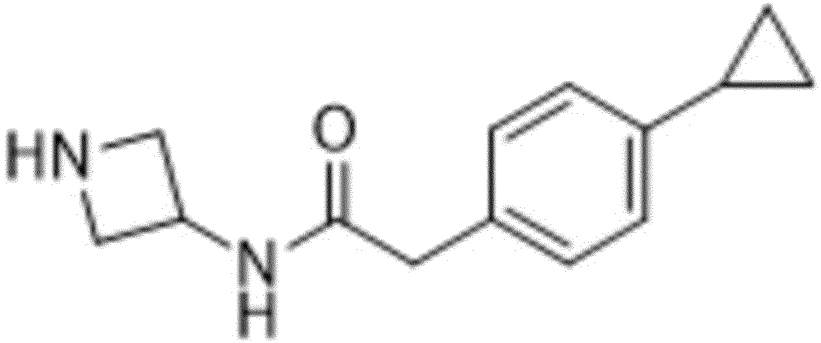

According to a technique similar to Reference Example 1-2, the title compound (white solid, 83 mg, 28%) was obtained using tert-butyl 3-(2-(4-cyclopropylphenyl)acetamido)azetidine-1-carbamate (431 mg, 1.3 mmol) synthesized in Reference Example 75-1.
¹H NMR (CDCl₃, 400MHz) : δ=0.6-0.8 (m, 2H),0.9-1.1 (m, 2H), 1.8-2.0(m,1H),3.3-3.6(m,2H),3.51(s,2H),3.8-3.9(m,2H), 4.6-4.8(m,1H),5.71(d,1H,J=7Hz),7.06(d,2H,J=8Hz), 7.13(d,2H,J=8Hz). The 1H content is not observable.

### Reference Example 76

### Ethyl 2-(3-bromophenoxy)-2-methylpropanoate

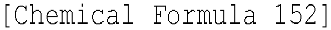

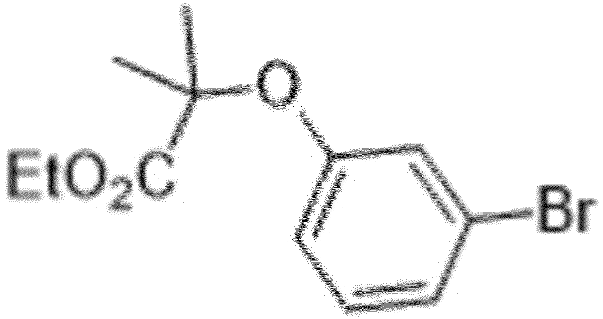

According to a technique similar to Reference Example 52, the title compound (white amorphous, 149 mg, 86%) was obtained using 3-bromophenol (173 mg, 1.0 mmol) and ethyl 2-bromo-2-methylpropanoate (195 mg, 1.0 mmol).
¹H NMR(CDCl₃, 400MHz) :δ=1.26(t,3H,J=7Hz),1.60(s,6H), 4.24(q,2H,J=7Hz),6.7-6.8(m,1H),7.0-7.2(m,3H).

### Reference Example 77

### Tert-butyl (R)-(1-(3-(trifluoromethyl)-phenyl)pyrrolidin-3-yl)carbamate

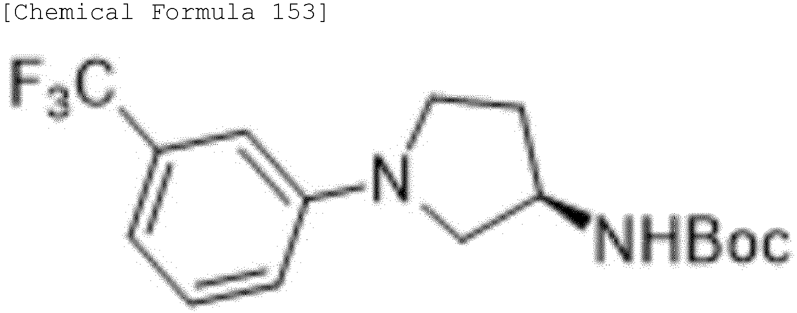

According to a technique similar to Reference Example 1-1, the title compound (yellow powder, 105 mg, 59%) was obtained using 3-bromobenzotrifluoride (89 µL, 0.644 mmol) and tert-butyl (R)-pyrrolidin-3-ylcarbamate (100 mg, 0.537 mmol).
¹H NMR (CDCl₃, 400MHz) : δ=1.46(s,9H),1.9-2.1(m,1H), 2.2-2.4(m,1H),3.19(dd,1H,J=4,10Hz),3.3-3.5(m,2H), 3.60(dd,1H,J=6,10Hz),4.38(br s,1H),4.69(br s, 1H), 6.68(dd,1H,J=2,8Hz),6.73(s,1H),6.93(d,1H,J=8Hz), 7.31(t,1H,J=8Hz).

### Example 1

### (R)-2-(4-cyclopropylphenyl)-N-(1-(5-(trifluoromethyl)pyridin-3-yl)pyrrolidin-3-yl)acetamide

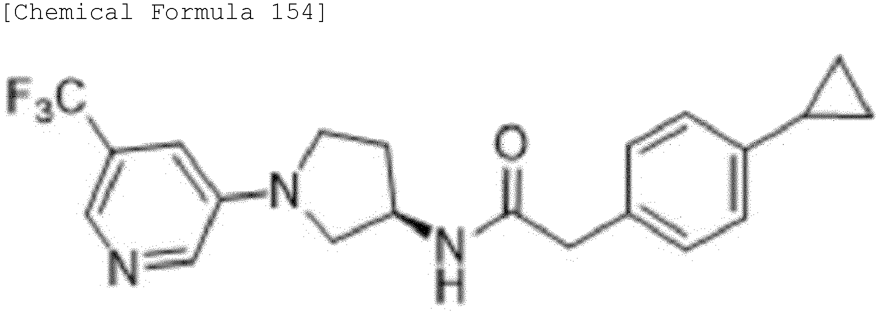

(R)-1-(5-(trifluoromethyl)pyridin-3-yl)pyrrolidin-3-amine (20 mg, 0.09 mmol) synthesized in Reference Example 1-2 and 2-(4-cyclopropylphenyl)acetic acid (30 mg, 0.09 mmol) synthesized in Reference Example 33-2 were dissolved in DMF (2.0 mL), subsequently DIPEA (29 µL, 0.17 mmol) and HATU (33 mg, 0.09 mmol) were added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (white powder, 18 mg, 53%) was obtained.
¹H NMR (CDCl₃, 400MHz) :δ=0.6-0.7(m,2H),0.9-1.0(m,2H), 1.8-2.0(m,2H),2.2-2.4(m,1H),3.11(dd,1H,J=4,10Hz), 3.3-3.4(m,2H),3.54(s,2H),3.64(dd,1H,J=6,10Hz), 4.6-4.7(m,1H),5.60(d,1H,J=6Hz),6.8-6.9(m,1H),7.04 (d,2H,J=8Hz),7.12(d,2H,J=8Hz),8.06(d,1H,J=2Hz),8.19(s,1H).
MS:390.20[M+H]⁺

### Example 2

### (R)-2-(5-(trifluoromethyl)pyridin-2-yl)-N-(1-(5-(trifluoromethyl)pyridin-3-yl)pyrrolidin-3-yl)acetamide

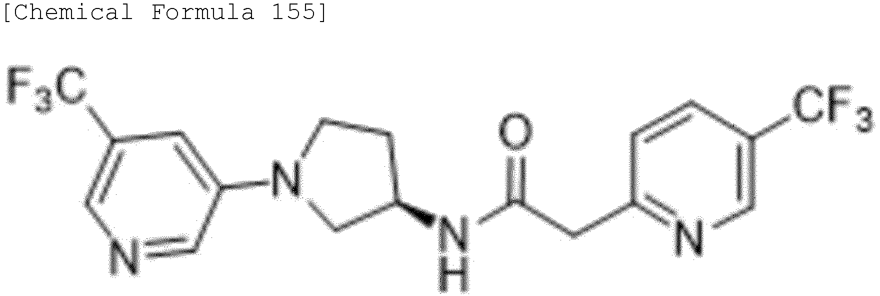

According to a technique similar to Example 1, the title compound (white crystals, 11 mg, 31%) was obtained using (R)-1-(5-(trifluoromethyl)pyridin-3-yl)pyrrolidin-3-amine (20 mg, 0.09 mmol) synthesized in Reference Example 1-2 and the crude form of 2-(5-(trifluoromethyl)pyridin-2-yl)acetic acid (26 mg) synthesized in Reference Example 31-2.
¹H NMR (CDCl₃, 400MHz) : δ=2.0-2.1(m,2H),2.3-2.4(m,1H), 3.25(dd,1H,J=4,10Hz),3.4-3.6(m,2H),3.68(dd,1H,J=6,10Hz), 3.81(s,2H),4.6-4.7(m,1H),6.9-7.0(m,1H),7.4-7.5(m,2H), 8.12(br s,1H),8.22(brs,1H),8.77(s,1H).
MS:419.15[M+H] ⁺

### Example 3

### (R)-2-(4-isopropylphenyl)-N-(1-(2-(trifluoromethyl)-pyrimidin-5-yl)pyrrolidin-3-yl)acetamide

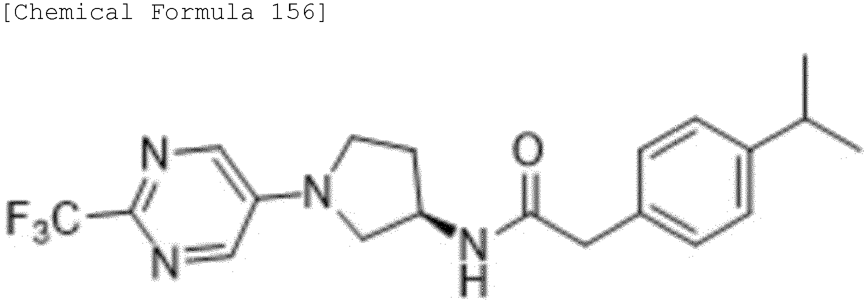

To tert-butyl (R)-(1-(2-(trifluoromethyl)pyrimidin-5-yl)pyrrolidin-3-yl)carbamate (41 mg, 0.12 mmol) synthesized in Reference Example 2, trifluoroacetic acid (2.0 mL) was added under ice cooling, and the mixture was stirred for 3 hours at room temperature. Under ice cooling, a saturated aqueous solution of sodium hydrogen carbonate and ethyl acetate were added to the reaction liquid, the mixture was stirred for a while, subsequently the organic layer was dried over anhydrous sodium sulfate, insoluble materials were filtered, subsequently the solvent was distilled off under reduced pressure, and thus the a crude form of (R)-1-(2-(trifluoromethyl)pyrimidin-5-yl)pyrrolidin-3-amine (pale yellow oily material, 18 mg) was obtained. According to a technique similar to Example 1, the title compound (ivory-colored powder, 14 mg, 29%) was obtained using the crude form of (R)-1-(2-(trifluoromethyl)pyrimidin-5-yl)pyrrolidin-3-amine (18 mg) thus obtained and 2-(4-isopropylphenyl)acetic acid (26 mg, 0.15 mmol).
¹H NMR (CDCl₃, 400MHz) :δ=1.23(d,6H,J=7Hz),1.9-2.0(m,1H),2.3-2.4(m,1H),2.8-2.9(m,1H),3.17(dd,1H,J=5,10Hz), 3.4-3.5(m,2H),3.56(s,2H),3.71(dd,1H,J=6,10Hz), 4.6-4.7(m,1H),5.50(d,1H,J=6Hz),7.15(d,2H,J=8Hz), 7.21(d,2H,J=8Hz),8.06(s,2H).
MS:391.26[M-H]⁻

### Example 4

### (R)-2-(4-isopropylphenyl)-N-(1-(5-(trifluoromethyl)-pyridin-3-yl)pyrrolidin-3-yl)acetamide

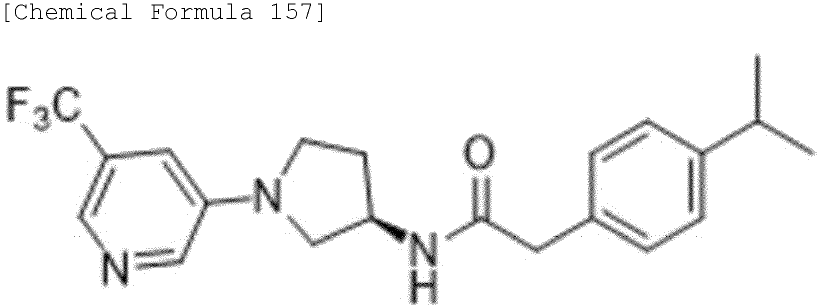

According to a technique similar to Example 3, a crude form of (R)-1-(5-(trifluoromethyl)pyridin-3-yl)pyrrolidin-3-amine (100 mg) was synthesized from tert-butyl (R)-1-(5-(trifluoromethyl)pyridin-3-yl)pyrrolidin-3-yl)carbamate (130 mg, 0.39 mmol) synthesized in Reference Example 1-1, and the title compound (white amorphous, 90 mg, 58%) was obtained using 2-(4-isopropylphenyl)acetic acid (85 mg, 0.48 mmol).
¹H NMR(CDCl3,400MHz):δ=1.22(d,6H,J=7Hz),1.9-2.0(m,1H),2.3-2.4(m,1H),2.8-2.9(m,1H),3.12(dd,1H,J=4,9Hz), 3.3-3.4(m,2H),3.55(s,2H),3.6-3.7(m,1H),4.6-4.7(m,1H),5.7-5.8(m,1H),6.87(s,1H),7.1-7.3(m,4H),8.04(s,1H),8.17(s,1H).
MS:391.19[M-H]⁻

### Example 5

### 2-(4-Cyclopropylphenyl)-N-((3S,4S)-4-hydroxy-1-(5-(trifluoromethyl)pyridin-3-yl)pyrrolidin-3-yl)acetamide

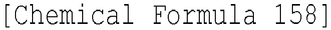

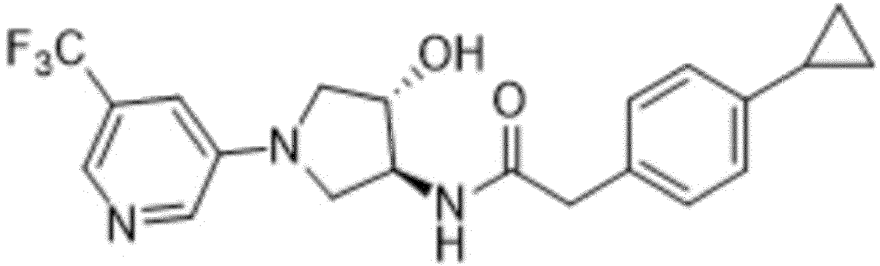

According to a technique similar to Example 3, (3S,4S)-4-amino-1-(5-(trifluoromethyl)pyridin-3-yl)pyrrolidin-3-ol (white crystals) was synthesized from tert-butyl ((3S,4S)-4-hydroxy-1-(5-(trifluoromethyl)-pyridin-3-yl)pyrrolidin-3-yl)carbamate (106 mg, 0.31 mmol) synthesized in Reference Example 3, and the title compound (white crystals, 15 mg, 12%) was obtained using 2-(4-cyclopropylphenyl)acetic acid (54 mg, 0.31 mmol) synthesized in Reference Example 33-2.
¹H NMR (CDCl₃, 400MHz) : δ=0.6-0.7 (m, 2H),0.9-1.0 (m, 2H), 1.8-1.9(m,1H),3.11(dd,1H,J=5,10Hz),3.27(dd,1H,J=4,10Hz), 3.56(s,2H),3.61(dd,1H,J=6,10Hz),3.76(dd,1H,J=6,10Hz), 4.09(brs,1H),4.3-4.4(m,2H),5.97(d,1H,J=6Hz), 6.8-6.9(m,1H),7.0-7.1(m,2H),7.1-7.2(m,2H),8.02(d,1H,J=2Hz), 8.17(s,1H).
MS:406.14[M+H]⁺

### Example 6

### (R)-2-(4-cyclopropylphenyl)-N-(5-(5-(trifluoromethyl)pyridin-3-yl)-5-azaspiro[2.4]heptan-7-yl)acetamide

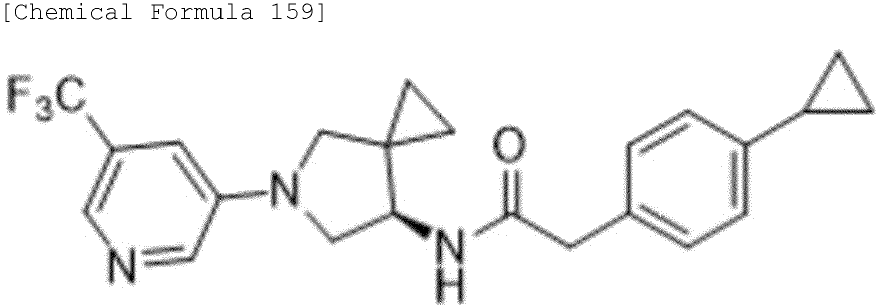

According to a technique similar to Example 3, (R)-5-(5-(trifluoromethyl)pyridin-3-yl)-5-azaspiro[2.4]heptan-7-amine (white crystals) was synthesized from tert-butyl (R)-(5-(5-(trifluoromethyl)pyridin-3-yl)-5-azaspiro[2.4]heptan-7-yl)carbamate (130 mg, 0.36 mmol) synthesized in Reference Example 4, and the title compound (white crystals, 30 mg, 20%) was obtained using 2-(4-cyclopropylphenyl)acetic acid (64 mg, 0.36 mmol) synthesized in Reference Example 33-2.
¹H NMR (CDCl₃, 400MHz) :δ=0.5-0.6(m,1H),0.6-0.7(m,4H), 0.8-0.9(m,1H),0.9-1.0(m,2H),1.8-1.9(m,1H),3.09(d,1H,J=9Hz), 3.39(dd,1H,J=2,10Hz),3.45(d,1H,J=10Hz),3.52(d,2H,J=3Hz), 3.74(dd,1H,J=6,10Hz),4.1-4.2(m,1H),5.62(d,1H,J=8Hz), 6.8-6.9(m,1H),7.0-7.1(m,2H),7.1-7.2(m,2H), 8.04(d,1H,J=3Hz),8.20(s,1H).
MS:416.18[M+H]⁺

### Example 7

### (R)-2-(4-cyclopropylphenyl)-N-(5-(6-(trifluoromethyl)pyridin-3-yl)-5-azaspiro[2.4]heptan-7-yl)acetamide

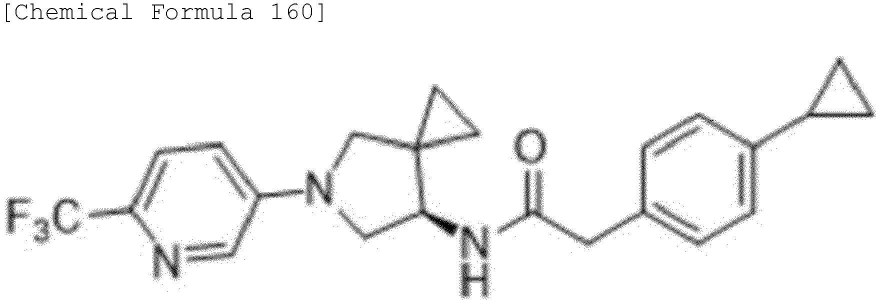

To tert-butyl (R)-(5-(6-(trifluoromethyl)pyridin-3-yl)-5-azaspiro[2.4]heptan-7-yl)carbamate (271 mg, 0.76 mmol) synthesized in Reference Example 5, a 2 N hydrochloric acid-methanol solution (15 mL) was added under ice cooling, the mixture was stirred for 2 hours at room temperature, subsequently the solvent was distilled off under reduced pressure, and thereby (R)-5-(6-(trifluoromethyl)pyridin-3-yl)-5-azaspiro[2.4]heptan-7-amine (187 mg) was obtained. According to a technique similar to Example 1, the title compound (white crystals, 280 mg, 89%) was obtained using (R)-5-(6-(trifluoromethyl)pyridin-3-yl)-5-azaspiro[2.4]heptan-7-amine (187 mg) thus obtained and 2-(4-cyclopropylphenyl)-acetic acid (154 mg, 0.87 mmol) synthesized in Reference Example 33-2.
¹H NMR (CDCl₃, 400MHz) :δ=0.5-0.7(m,5H),0.8-0.9(m,1H), 0.9-1.0(m,2H),1.8-1.9(m,1H),3.11(d,1H,J=10Hz), 3.40(dd,1H,J=3,10Hz),3.45(d,1H,J=10Hz),3.53(d,2H,J=3Hz), 3.75(dd,1H,J=6,10Hz),4.1-4.2(m,1H),5.56(d,1H,J=7Hz), 6.77(dd,1H,J=3,8Hz),7.0-7.1(m,2H),7.1-7.2(m,2H), 7.48(d,1H,J=9Hz),7.93(d,1H,J=3Hz).
MS:415.19[M+H]⁺

### Example 8

### (R)-2-(4-cyclopropylphenyl)-N-(5-(4-(trifluoromethyl)thiazol-2-yl)-5-azaspiro[2.4]heptan-7-yl)acetamide

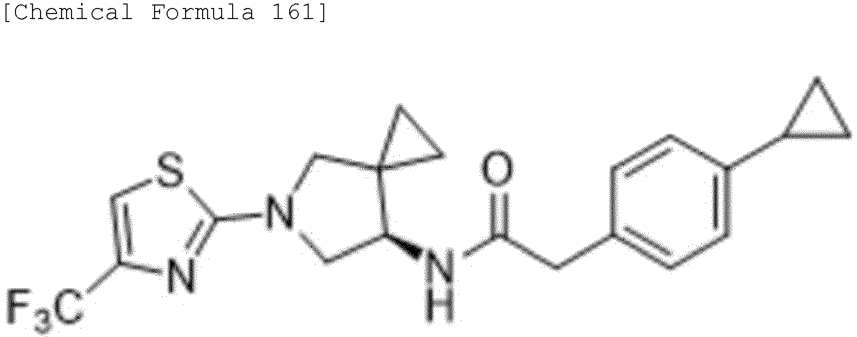

According to a technique similar to Example 7, (R)-5-(4-(trifluoromethyl)thiazol-2-yl)-5-azaspiro[2.4]heptan-7-amine (white crystals) was synthesized from tert-butyl (R)-(5-(4-(trifluoromethyl)thiazol-2-yl)-5-azaspiro[2.4]heptan-7-yl)carbamate (192 mg, 0.53 mmol) synthesized in Reference Example 6, and the title compound (white crystals, 177 mg, 79%) was obtained using 2-(4-cyclopropylphenyl)acetic acid (112 mg, 0.64 mmol) synthesized in Reference Example 33-2.
¹H NMR (CDCl₃, 400MHz) :δ=0.5-0.6(m,1H),0.6-0.7(m,4H), 0.7-0.8(m,1H),0.9-1.0(m,2H),1.8-1.9(m,1H), 3.32(d,1H,J=10Hz),3.44(dd,1H,J=2,10Hz),3.52(d,2H,J=2Hz), 3.5 6(d,1H,J=10Hz),3.83(dd,1H,J=6,10Hz),4.1-4.2(m,1H), 5.53(d,1H,J=7Hz),6.93(d,1H,J=1Hz),7.0-7.1(m,2H), 7.1-7.2(m,2H).
MS:421.14[M+H] ⁺

### Example 9

### 2-(4-Cyclopropylphenyl)-N-((3R,5S)-5-methyl-1-(5-(trifluoromethyl)pyridin-3-yl)pyrrolidin-3-yl)acetamide

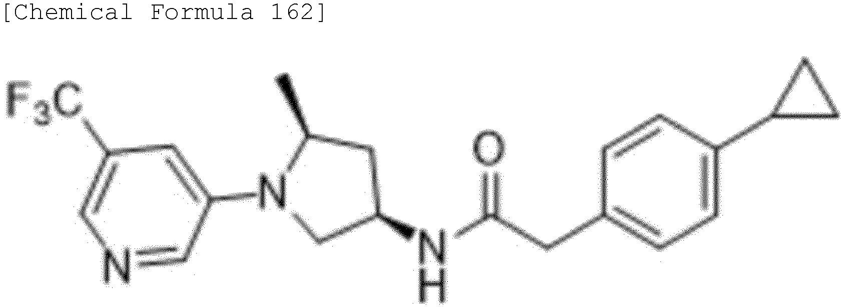

2-(4-Cyclopropylphenyl)-N-((3R,5S)-5-methylpyrrolidin-3-yl)acetamide (150 mg, 0.58 mmol) synthesized in Reference Example 7-2, 3-bromo-5-(trifluoromethyl)pyridine (157 mg, 0.70 mmol), tris(dibenzylidene acetone)dipalladium(0) (53 mg, 0.06 mmol), XantPhos (67 mg, 0.12 mmol), and sodium tert-butoxide (112 mg, 1.16 mmol) were suspended in toluene (5.8 mL), and the suspension was stirred for 16 hours at 85°C under a nitrogen gas stream. To the reaction liquid that had been left to cool to room temperature, water was added, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 0 to 100%), and the title compound (white crystals, 45 mg, 19%) was obtained.
¹H NMR (CDCl₃, 400MHz) : δ=0.6-0.7(m, 2H),0.9-1.0(m,2H), 1.22(d,3H,J=6Hz),1.8-2.0(m,2H),2.0-2.1(m,1H), 2.93(dd,1H,J=7,10Hz),3.54(s,2H),3.80(dd,1H,J=8,10Hz), 3.9-4.0(m,1H),4.7-4.8(m,1H),5.48(d,1H,J=8Hz), 6.88(dd,1H,J=2,2Hz),7.0-7.1(m,2H),7.1-7.2(m,2H), 8.07(d,1H,J=3Hz),8.17(s,1H).
MS:404.16[M+H]⁺

### Example 10

### 2-(4-Cyclopropylphenyl)-N-(3-(5-(trifluoromethyl)pyridin-3-yl)-3-azabicyclo[3.1.0]hexan-1-yl)acetamide

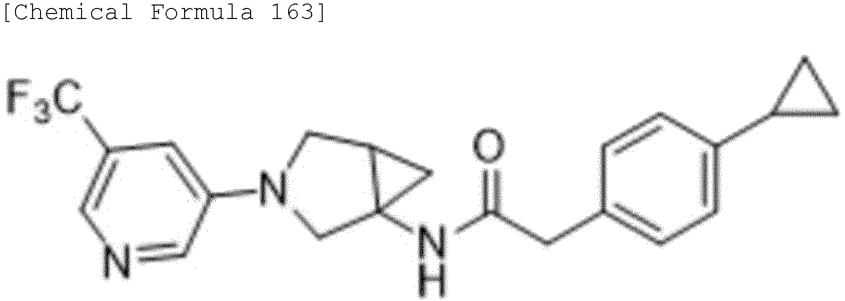

According to a technique similar to Example 7, 3-(5-(trifluoromethyl)pyridin-3-yl)-3-azabicyclo[3.1.0]hexan-1-amine (yellow oily material, 121 mg) was synthesized from tert-butyl (3-(5-(trifluoromethyl)pyridin-3-yl)-3-azabicyclo[3.1.0]hexan-1-yl)carbamate (103 mg, 0.30 mmol) synthesized in Reference Example 8, and the title compound (pale yellow amorphous, 73 mg, 61%) was obtained using 2-(4-cyclopropylphenyl)acetic acid (63 mg, 0.36 mmol) synthesized in Reference Example 33-2.
¹H NMR(CDCl₃,400MHz):δ=0.6-0.7(m,2H)0.8-0.9(m,1H) 0.9-1.1(m,3H),1.8-2.0(m,2H),3.3-3.4(m,1H),3.5-3.6(m,4H), 3.7-3.8(m,1H),5.89(s,1H),6.90(s,1H),7.07(d,2H,J=8Hz), 7.13(d,2H,J=8Hz),8.06(d,1H,J=2Hz),8.18(s,1H).
MS:402.16[M+H]⁺

### Example 11

### (R)-2-(4-(trifluoromethyl)phenyl)-N-(1-(6-(trifluoromethyl)pyrazin-2-yl)pyrrolidin-3-yl)acetamide

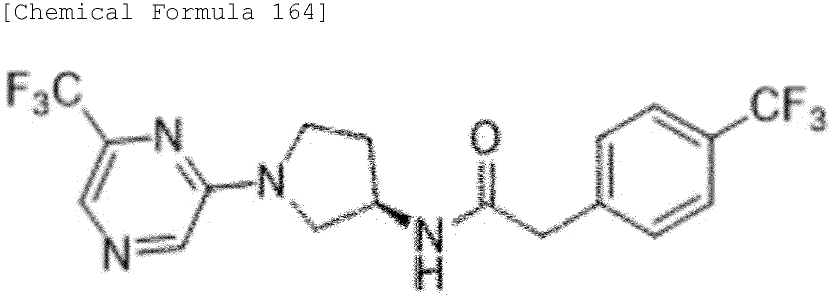

According to a technique similar to Example 7, (R)-1-(6-(trifluoromethyl)pyrazin-2-yl)pyrrolidin-3-amine was synthesized from tert-butyl (R)-(1-(6-(trifluoromethyl)-pyrazin-2-yl)pyrrolidin-3-yl)carbamate (159 mg, 0.48 mmol) synthesized in Reference Example 9, and the title compound (white powder, 187 mg, 94%) was obtained using 2-(4-(trifluoromethyl)phenyl)acetic acid (117 mg, 0.57 mmol).
¹H NMR (CDCl₃, 400MHz) :δ=1.9-2.0(m,1H),2.3-2.4(m,1H), 3.38(dd,1H,J=4,11Hz),3.6-3.7(m,4H),3.87(dd,1H,J=6,12Hz), 4.6-4.7(m,1H),5.53(d,1H,J=6Hz),7.4 0(d,2H,J=8Hz), 7.61(d,2H,J=8Hz),8.03(s,1H),8.17(s,1H).
MS:419.10[M+H] ⁺

### Example 12

### (R)-N-(1-(6-cyano-5-(trifluoromethyl)pyridin-3-yl)pyrrolidin-3-yl)-2-(4-cyclopropylphenyl)acetamide

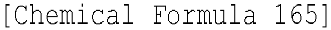

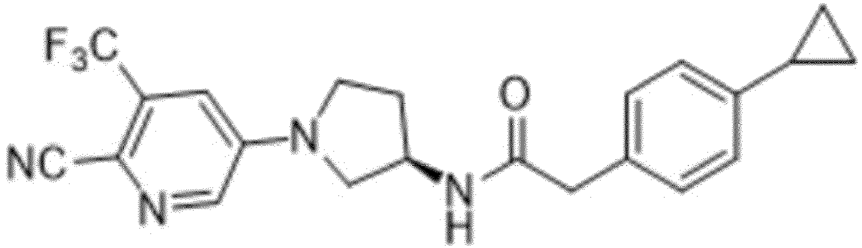

According to a technique similar to Example 3, (R)-1-(6-cyano-5-(trifluoromethyl)pyridin-3-yl)pyrrolidin-3-amine was synthesized from tert-butyl (R)-(1-(6-cyano-5-(trifluoromethyl)pyridin-3-yl)pyrrolidin-3-yl)carbamate (139 mg, 0.39 mmol) synthesized in Reference Example 10, and the title compound (white amorphous, 52 mg, 32%) was obtained using 2-(4-cyclopropylphenyl)acetic acid (82 mg, 0.47 mmol) synthesized in Reference Example 33-2.
¹H NMR (CDCl₃, 400MHz) : δ=0.6-0.7(m, 2H),0.9-1.0(m,2H), 1.8-1.9(m,1H),1.9-2.0(m,1H),2.3-2.4(m,1H), 3.23(dd,1H,J=8,10Hz),3.4-3.6(m,4H),3.7-3.8(m,1H), 4.5-4.7(m,1H),5.80(d,1H,J=6Hz),6.88(d,1H,J=3Hz), 7.02(d,2H,J=8Hz),7.11(d,2H,J=8Hz),8.00(d,1H,J=2Hz).
MS:413.15[M-H]⁻

### Example 13

### Methyl 3-(2-(4-cyclopropylphenyl)acetamido)-1-(5-(trifluoromethyl)pyridin-3-yl)pyrrolidine-3-carboxylate

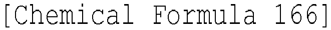

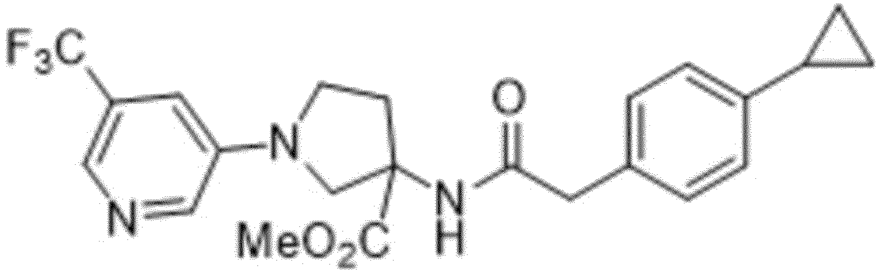

According to a technique similar to Example 9, the title compound (white amorphous, 53 mg, 38%) was obtained using methyl 3-(2-(4-cyclopropylphenyl)-acetamido)pyrrolidine-3-carboxylate (94 mg, 0.31 mmol) synthesized in Reference Example 11-2 and 3-bromo-5-(trifluoromethyl)pyridine (84 mg, 0.37 mmol).
¹H NMR (CDCl₃, 400MHz) : δ=0.6-0.7 (m, 2H),0.9-1.0 (m, 2H), 1.8-1.9(m,1H),2.3-2.4(m,1H),2.5-2.6(m,1H),3.4-3.5(m,2H), 3.55(s,2H),3.5-3. 6(m,1H),3.75(s,3H),3.98(d,1H,J=10Hz), 6.17(s,1H),6.8-6.9(m,1H),7.0-7.1(m,2H),7.1-7.2(m,2H), 8.05(d,1H,J=3Hz),8.20(s,1H).
MS:448.14[M+H]⁺

### Example 14

### 3-(2-(4-Cyclopropylphenyl)acetamido)-1-(5-(trifluoromethyl)pyridin-3-yl)pyrrolidine-3-carboxylic acid

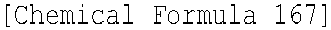

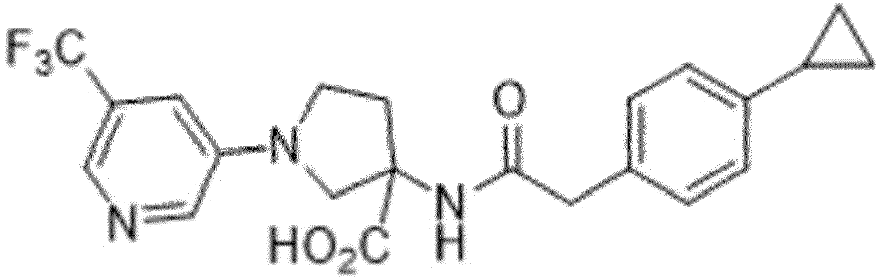

Methyl 3-(2-(4-cyclopropylphenyl)acetamido)-1-(5-(trifluoromethyl)pyridin-3-yl)pyrrolidine-3-carboxylate (50 mg, 0.11 mmol) synthesized in Example 13 was dissolved in THF (2.0 mL), a 2 N aqueous solution of sodium hydroxide (2.0 mL) was added thereto, and the mixture was stirred for 3 hours at room temperature. a 1 N aqueous solution of hydrochloric acid was added to the reaction liquid to neutralize the reaction liquid, and then the reaction liquid was extracted with a mixed liquid of chloroform and methanol. An organic layer thus separated was dried over anhydrous sodium sulfate, insoluble materials were filtered, and then the solvent was distilled off under reduced pressure. A residue thus obtained was purified by silica gel column chromatography (ethyl acetate : methanol) (concentration gradient: 0 to 90%), subsequently methanol and water were added to a powder thus obtained, and the mixture was stirred for a while. A precipitated powder was filtered and dried under reduced pressure for 3 hours at 100°C, and thereby the title compound (white powder, 38 mg, 79%) was obtained.
¹H NMR (CD₃OD, 400MHz) : δ=0.5-0.6(m,2H), 0.8-0.9(m,2H),1.8-1.9(m,1H),2.4-2.6(m,2H),3.4-3.6(m,4H), 3.6-3.7(m,1H),3.9-4.0(m,1H),4.60(br s,1H),6.9-7.0(m,2H), 7.0-7.2(m,3H),8.0-8.1(m,2H). The 1H content is not observable.
MS:434.16[M+H]⁺

### Example 15

### (R)-2-(1H-indazol-6-yl)-N-(1-(5-(trifluoromethyl)pyridin-3-yl)pyrrolidin-3-yl)acetamide

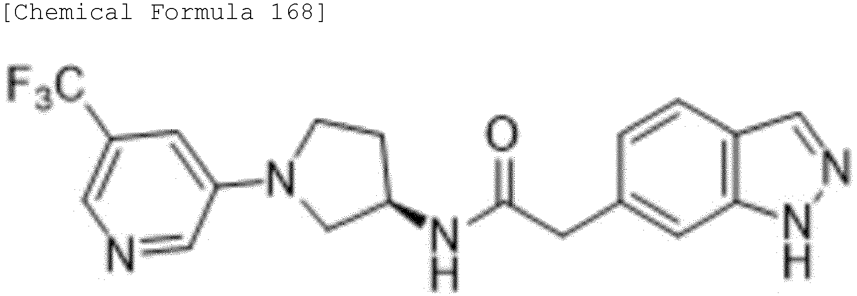

According to a technique similar to Example 1, the title compound (white powder, 82 mg, 74%) was obtained using (R)-1-(5-(trifluoromethyl)pyridin-3-yl)pyrrolidin-3-amine (66 mg, 0.28 mmol) synthesized in Reference Example 1-2 and 2-(1H-indazol-6-yl)acetic acid (50 mg, 0.28 mmol).
¹H NMR (CDCl₃, 400MHz) : δ=1.8-2.0 (m,1H),2.3-2.4(m,1H), 3.13(dd,1H,J=4,10Hz),3.3-3.4(m,2H),3.63(dd,1H,J=6,10Hz), 3.70(s,2H),4.6-4.7(m,1H),5.62(d,1H,J=7Hz),6.8-6.9(m,1H), 7.04(dd,1H,J=1,8Hz),7.39(s,1H),7.72(d,1H,J=8Hz), 8.0-8.1(m,2H),8.18(s,1H),10.1(br s,1H).
MS:388.15[M-H]⁻

### Example 16

### (R)-2-(4-cyclopropylphenyl)-N-(1-(4-(trifluoromethyl)thiazol-2-yl)pyrrolidin-3-yl)acetamide

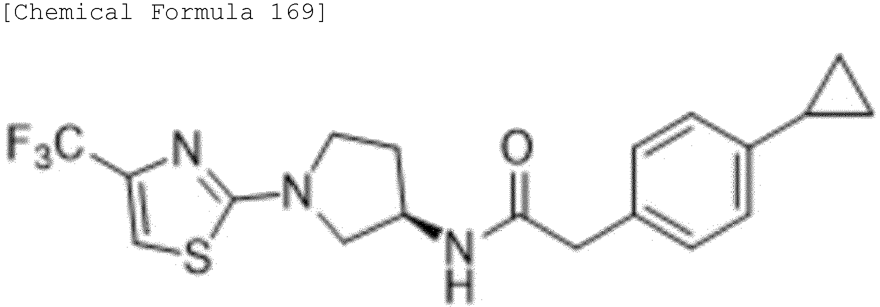

According to a technique similar to Example 1, the title compound (yellow-green powder, 30 mg, 55%) was obtained using (R)-1-(4-(trifluoromethyl)thiazol-2-yl)pyrrolidin-3-amine (33 mg, 0.14 mmol) synthesized in Reference Example 12-2 and 2-(4-cyclopropylphenyl)acetic acid (29 mg, 0.17 mmol) synthesized in Reference Example 33-2.
¹H NMR (CDCl₃, 400MHz) : δ=0.6-0.7 (m, 2H),0.9-1.0 (m, 2H), 1.8-2.0(m,2H),2.2-2.4(m,1H),3.23(dd,1H,J=4,10Hz), 3.4-3.6(m,4H),3.74(dd,1H,J=6,10Hz),4.5-4.7(m,1H), 5.47(d,1H,J=6Hz),6.92(s,1H),7.04(d,2H,J=8Hz), 7.10(d,2H,J=2Hz).
MS:394.14[M-H]⁻

### Example 17

### 2-(4-Isobutylphenyl)-N-((R)-1-(5-(trifluoromethyl)-pyridin-3-yl)pyrrolidin-3-yl)propanamide

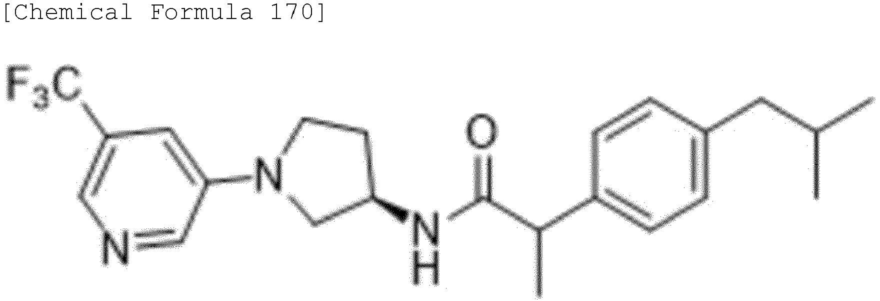

According to a technique similar to Example 1, the title compound (pale yellow amorphous, 50 mg, 79%) was obtained using (R)-1-(5-(trifluoromethyl)pyridin-3-yl)pyrrolidin-3-amine (35 mg, 0.15 mmol) synthesized in Reference Example 1-2 and 2-(4-isobutylphenyl)propanoic acid (37 mg, 0.18 mmol).
¹H NMR (CDCl₃, 400MHz) :δ=0.8-0.9(m,6H), 1.51(d,1.5H,J=8Hz),1.52(d,1.5H,J=8Hz),1.8-2.0(m,2H), 2.2-2.3(m,1H),2.4-2.5(m,2H),3.03(dd,0.5H,J=4,10Hz), 3.12(dd,0.5H,J=4,10Hz),3.2-3.4(m,2H),3.5-3.7(m,2H), 4.5-4.7(m,1H),5.67(d,0.5H,J=7Hz),5.70(d,0.5H,J=7Hz), 6.8-6.9(m,1H),7.0-7.1(m,2H),7.1-7.2(m,2H), 8.01(dd,1H,J=2,2Hz),8.15(s,1H).
MS:420.20[M+H] ⁺

### Example 18

### (R)-2-(4-cyclopropylphenyl)-N-(1-(6-methyl-5-(trifluoromethyl)pyridin-3-yl)pyrrolidin-3-yl)acetamide

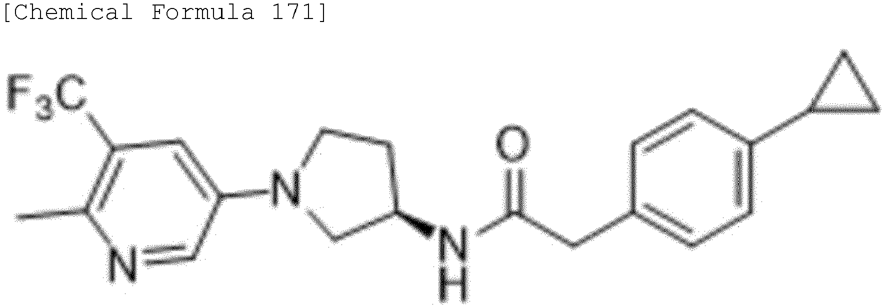

According to a technique similar to Example 7, (R)-1-(6-methyl-5-(trifluoromethyl)pyridin-3-yl)pyrrolidin-3-amine (white powder, 22 mg) was synthesized from tert-butyl (R)-(1-(6-methyl-5-(trifluoromethyl)pyridin-3-yl)pyrrolidin-3-yl)carbamate (22 mg, 0.06 mmol) synthesized in Reference Example 13, and the title compound (white amorphous, 5.9 mg, 23%) was obtained using 2-(4-cyclopropylphenyl)acetic acid (13 mg, 0.07 mmol) synthesized in Reference Example 33-2.
¹H NMR (CDCl₃, 400MHz) : δ=0.6-0.7 (m, 2H),0.9-1.0 (m,2H), 1.8-1.9(m,2H),2.2-2.4(m,1H),2.57(s,3H), 3.08(dd,1H,J=4,10Hz),3.3-3.4(m,2H),3.53(s,2H), 3.60(dd,1H,J=6,10Hz),4.5-4.7(m,1H),5.50(d,1H,J=8Hz), 6.95(d,1H,J=2Hz),7.04(d,2H,J=8Hz),7.11(d,2H,J=8Hz), 7.93 (d, 1H,J=2Hz) .
MS:404.16[M+H]⁺

### Example 19

### (R)-2-(4-cyclopropylphenyl)-N-(1-(5-(2-hydroxypropan-2-yl)pyridin-3-yl)pyrrolidin-3-yl)acetamide

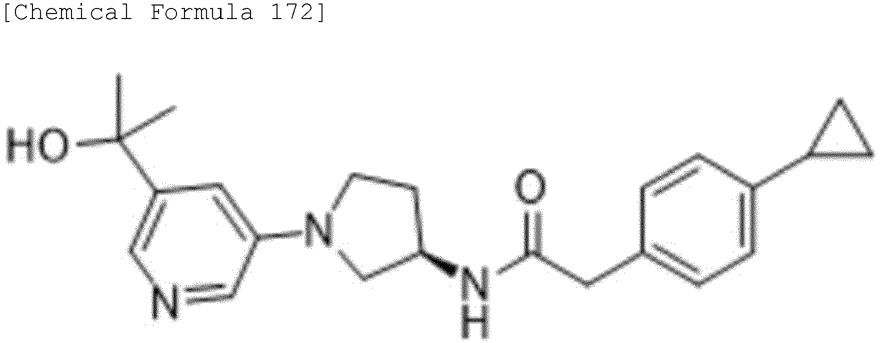

According to a technique similar to Example 1, the title compound (pale yellow amorphous, 81 mg, 86%) was obtained using (R)-2-(5-(3-aminopyrrolidin-1-yl)pyridin-3-yl)propan-2-ol (55 mg, 0.17 mmol) synthesized in Reference Example 14-2 and 2-(4-cyclopropylphenyl)acetic acid (36 mg, 0.20 mmol) synthesized in Reference Example 33-2.
¹H NMR (CDCl₃, 400MHz) : δ=0.6-0.7 (m, 2H),0.9-1.0 (m,2H), 1.59(s,6H),1.7-1.8(m,1H),1.8-1.9(m,2H),2.2-2.4(m,1H), 3.0-3.1(m,1H),3.3-3.4(m,2H),3.53(s,2H), 3.60(dd,1H,J=4,10Hz),4.6-4.7(m,1H),5.61(d,1H,J=7Hz), 6.9-7.0(m,1H),7.04(d,2H,J=8Hz),7.12(d,2H,J=8Hz), 7.82(br s,1H),8.04(br s,1H) .
MS:380.20[M+H]⁺

### Example 20

### (R)-2-(4-cyclopropylphenyl)-N-(1-(8-(trifluoromethyl)imidazo[1,2-a]pyridin-6-yl)pyrrolidin-3-yl)acetamide

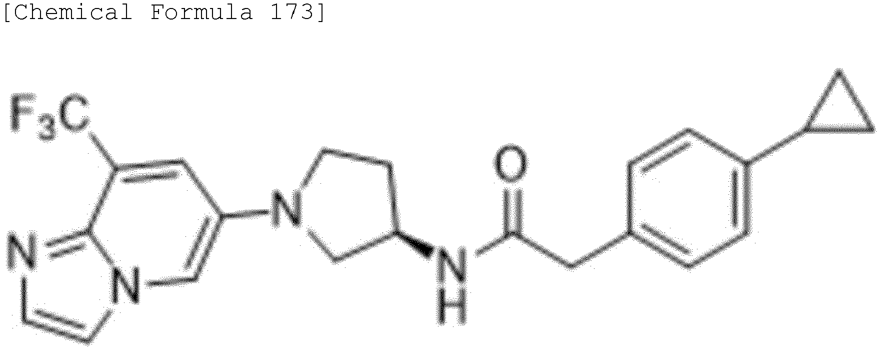

According to a technique similar to Example 7, (R)-1-(8-(trifluoromethyl)imidazo[1,2-a]pyridin-6-yl)pyrrolidin-3-amine (56 mg) was synthesized from tert-butyl (R)-(1-(8-(trifluoromethyl)imidazo[1,2-a]pyridin-6-yl)pyrrolidin-3-yl)-carbamate (90 mg, 0.24 mmol) synthesized in Reference Example 15, and the title compound (yellow amorphous, 58 mg, 56%) was obtained using 2-(4-cyclopropylphenyl)acetic acid (44 mg, 0.25 mmol) synthesized in Reference Example 33-2.
¹H NMR (CDCl₃, 400MHz) : δ=0.6-0.7 (m, 2H),0.9-1.0 (m,2H), 1.8-2.0(m,2H),2.3-2.4(m,1H),3.0-3.1(m,1H),3.2-3.3(m,1H), 3.3-3.4(m,1H),3.5-3. 6(m,1H),3.56(s,2H),4.6-4. 7(m,1H), 6.70(d,1H,J=6Hz),7.04(d,2H,J=8Hz),7.08(s,1H), 7.15(d,2H,J=8Hz),7.52(s,1H),7.60(s,1H). The 1H content is not observable.
MS:429.16[M+H]⁺

### Example 21

### 3-(2-(4-Cyclopropylphenyl)acetamido)-1-(5-(trifluoromethyl)pyridin-3-yl)pyrrolidine-3-carboxyamide

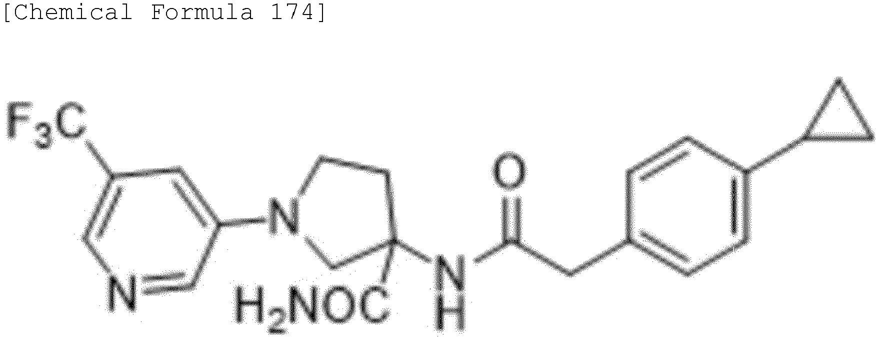

According to a technique similar to Example 1, the title compound (white powder, 11 mg, 73%) was obtained using 3-(2-(4-cyclopropylphenyl)acetamido)-1-(5-(trifluoromethyl)pyridin-3-yl)pyrrolidine-3-carboxylic acid (15 mg, 0.04 mmol) synthesized in Example 14 and ammonium acetate (5.3 mg, 0.07 mmol).
¹H NMR(DMSO-d₆,40OMHz):δ=0.5-0.6(m,2H)0.8-0.9(m,2H), 1.8-1.9(m,1H),2.3-2.4(m,2H),3.2-3.5(m,4H),3.5-3.6(m,1H), 3.82(d,1H,J=10Hz),6.85(d,2H,J=8Hz),6.9-7.1(m,3H), 7.11(s,1H),7.29(br s,1H),8.1-8.2(m,2H),8.54(s,1H).
MS: 431.20 [M-H]⁻

### Example 22

### (R)-2-(4-hydroxyphenyl)-N-(1-(5-(trifluoromethyl)pyridin-3-yl)pyrrolidin-3-yl)acetamide

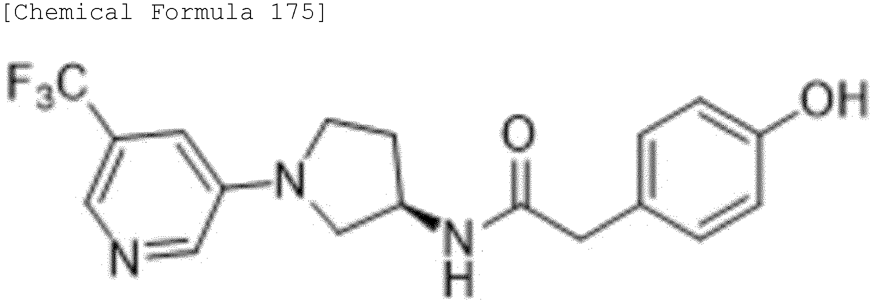

According to a technique similar to Example 1, the title compound (white amorphous, 292 mg, 92%) was obtained using (R)-1-(5-(trifluoromethyl)pyridin-3-yl)pyrrolidin-3-amine (200 mg, 0.86 mmol) synthesized in Example 1-2 and 2-(4-hydroxyphenyl)acetic acid (158 mg, 1.04 mmol).
¹H NMR (CDCl₃, 400MHz) : δ=1.8-2.0 (m, 1H),2.3-2.4(m, 1H), 3.12(dd,1H,J=5,10Hz),3.3-3.4(m,2H),3.51(s,2H), 3.65(dd,1H,J=6,10Hz),5.18(s,1H),5.50(d,1H,J=6Hz), 6.8-6.9(m,2H),6.9-7.0(m,1H),7.1-7.2(m,2H), 8.07(d,2H,J=3Hz),8.21(s,1H).
MS:366.11[M+H]⁺

### Example 23

### (R)-2-(4-(1,1-dioxide thiomorpholino)phenyl)-N-(1-(5-(trifluoromethyl)pyridin-3-yl)pyrrolidin-3-yl)acetamide

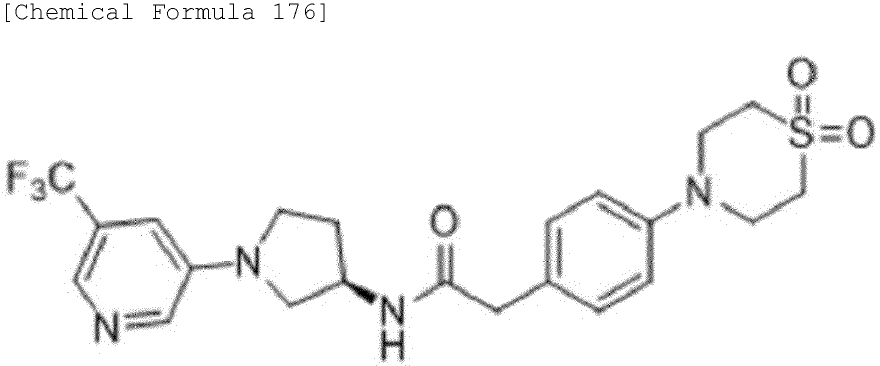

Ethyl 2-(4-(1,1-dioxide thiomorpholino)phenyl)acetate (30 mg, 0.10 mmol) synthesized in Reference Example 16 was dissolved in THF (2.0 mL), subsequently a 2 N aqueous solution of sodium hydroxide (2.0 mL) was added thereto, the mixture was stirred for 20 minutes at 50°C, and the solvent was distilled off under reduced pressure. Water and ethyl acetate were added to a residue thus obtained, the mixture was stirred for a while, subsequently a 1 N aqueous solution of hydrochloric acid was added to an aqueous layer thus separated to neutralize the aqueous layer, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby a crude form of 2-(4-(1,1-dioxide thiomorpholino)phenyl)acetic acid (pale yellow crystals) was obtained. According to a technique similar to Example 1, the title compound (white amorphous, 30 mg, 61%) was obtained using the crude form of 2-(4-(1,1-dioxide thiomorpholino)phenyl)acetic acid thus obtained and (R)-1-(5-(trifluoromethyl)pyridin-3-yl)pyrrolidin-3-amine (23 mg, 0.10 mmol) synthesized in Reference Example 1-2.
¹H NMR (CDCl₃, 400MHz) :δ=1.9-2.0(m,1H),2.3-2.4(m,1H), 3.0-3.1(m,4H),3.16(dd,1H,J=5,10Hz),3.3-3.5(m,2H), 3.51(s,2H),3.67(dd,1H,J=6,10Hz),3.8-3.9(m,4H), 4.6-4.7(m,1H),5.53(d,1H,J=7Hz),6.8-7.0(m,3H), 7.1-7.2(m,2H),8.09(d,1H,J=2Hz),8.21(s,1H).
MS:481.18[M-H]⁻

### Example 24

### (R)-1-(4-chlorophenyl)-N-(1-(5-(trifluoromethyl)pyridin-3-yl)pyrrolidin-3-yl)cyclopropane-1-carboxyamide

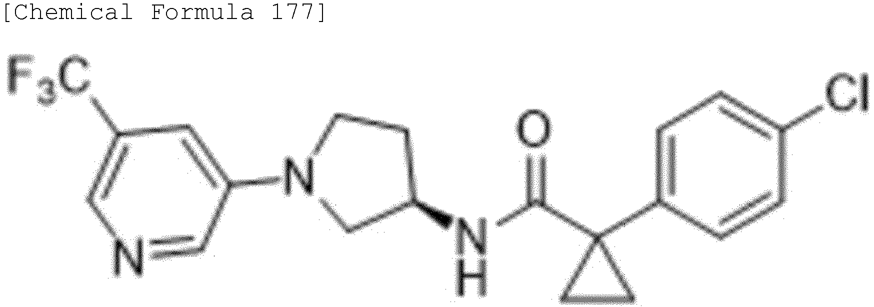

According to a technique similar to Example 1, the title compound (white amorphous, 70 mg, 79%) was obtained using (R)-1-(5-(trifluoromethyl)pyridin-3-yl)pyrrolidin-3-amine (50 mg, 0.22 mmol) synthesized in Reference Example 1-2 and 1-(4-chlorophenyl)cyclopropane-1-carboxylic acid (51 mg, 0.26 mmol).
¹H NMR (CDCl₃, 400MHz) :δ=1.0-1.1(m,2H),1.6-1.7(m,2H), 1.7-1.9(m,1H),2.2-2.4(m,1H),3.0-3.1(m,1H),3.2-3.4(m,2H), 3.6-3.7(m,1H),4.5-4.6(m,1H),5.36(d,1H,J=5Hz), 6.89(s,1H),7.2-7.4(m,4H),8.05(s,1H),8.19(s,1H).
MS:410.07[M+H]⁺

### Example 25

### 2-(4-Bromophenyl)-2-hydroxy-N-((R)-1-(5-(trifluoromethyl)pyridin-3-yl)pyrrolidin-3-yl)acetamide

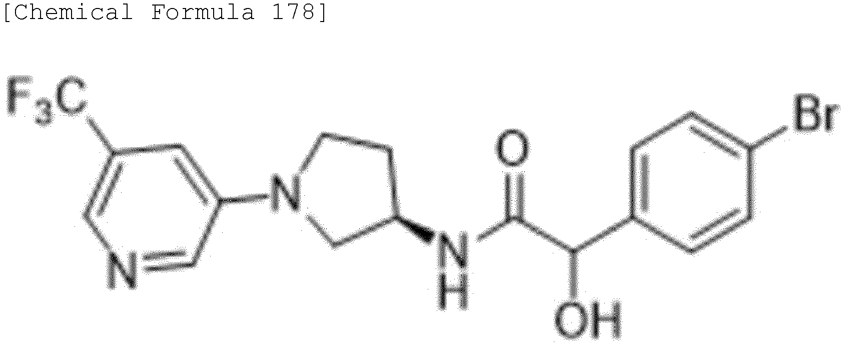

According to a technique similar to Example 1, the title compound (white powder, 68 mg, 71%) was obtained using (R)-1-(5-(trifluoromethyl)pyridin-3-yl)pyrrolidin-3-amine (50 mg, 0.22 mmol) synthesized in Reference Example 1-2 and 2-(4-bromophenyl)-2-hydroxyacetic acid (60 mg, 0.26 mmol) .
¹H NMR (CDCl₃, 400MHz) :δ=2.0-2.1(m,1H),2.3-2.4(m,1H), 3.1-3.2(m,1H),3.3-3.5(m,2H),3.6-3.7(m,1H),3.92(s,0.5H), 4.15(s,0.5H),4.6-4.7(m,1H),5.06(s,1H),6.8-7.0(m,2H), 7.31(d,2H,J=8Hz),7.49(d,2H,J=8Hz),7.94(d,1H,J=8Hz), 8.12(d,1H,J=4Hz).
MS:444.00[M+H]⁺

### Example 26

### (R)-N-(1-(5-(trifluoromethyl)pyridin-3-yl)pyrrolidin-3-yl)-2-(4-(trimethylsilyl)phenyl)acetamide

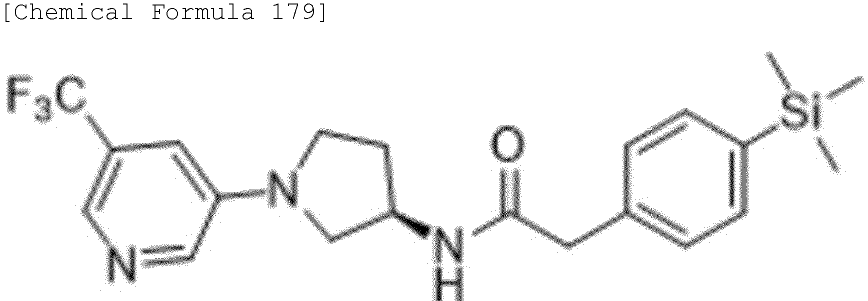

Ethyl 2-(4-(trimethylsilyl)phenyl)acetate (30 mg, 0.13 mmol) synthesized in Reference Example 17 was dissolved in methanol (2.0 mL), a 2 N aqueous solution of sodium hydroxide (2.0 mL) was added thereto, the mixture was stirred for 4 hours at room temperature, and the solvent was distilled off under reduced pressure. Water was added thereto to obtain an aqueous solution, subsequently the aqueous solution was washed with ethyl acetate, a 1 N aqueous solution of hydrochloric acid was added thereto to neutralize the aqueous solution, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby a crude form of 2-(4-(trimethylsilyl)phenyl)acetic acid was obtained. According to a technique similar to Example 1, the title compound (colorless oily material, 6.8 mg, 13%) was obtained using the crude form of 2-(4-(trimethylsilyl)phenyl)acetic acid thus obtained and (R)-1-(5-(trifluoromethyl)pyridin-3-yl)pyrrolidin-3-amine (29 mg, 0.13 mmol) synthesized in Reference Example 1-2.
¹H NMR (CDCl₃, 400MHz) :δ=0.26(s,9H),1.9-2.0(m,1H), 2.3-2.4(m,1H),3.13(dd,1H,J=4,10Hz),3.3-3.4(m,2H), 3.58(s,2H),3.66(dd,1H,J=6,10Hz),4.6-4.7(m,1H), 5.65(d,1H,J=7Hz),6.89(dd,1H,J=2,2Hz),7.2-7.3(m,2H), 7.4-7.5(m,2H),8.07(d,1H,J=3Hz),8.19(s,1H).
MS:422.16[M+H]⁺

### Example 27

### (R)-2-(4-(2-hydroxy-2-methylpropoxy)phenyl)-N-(1-(5-(trifluoromethyl)pyridin-3-yl)pyrrolidin-3-yl)acetamide

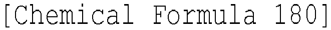

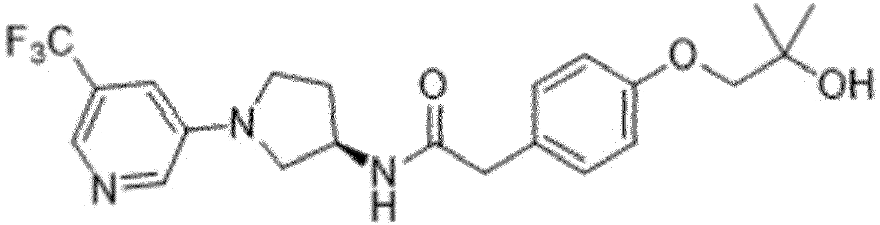

(R)-2-(4-hydroxyphenyl)-N-(1-(5-(trifluoromethyl)pyridin-3-yl)pyrrolidin-3-yl)acetamide (30 mg, 0.08 mmol) synthesized in Example 22 was dissolved in DMF (1.0 mL), subsequently 2,2-dimethyloxirane (22 µL, 0.25 mmol) and potassium carbonate (34 mg, 0.25 mmol) were added thereto, and the mixture was stirred for 16 hours at 110°C. To the reaction liquid that had been left to cool to room temperature, water was added, the mixture was stirred for a while, and then the mixture was extracted with a mixed liquid of chloroform and methanol. An organic layer thus separated was dried over anhydrous sodium sulfate, insoluble materials were filtered, and then the solvent was distilled off under reduced pressure. A residue thus obtained was purified by silica gel column chromatography (ethyl acetate : methanol) (concentration gradient: 0 to 40%), and the title compound (colorless oily material, 3.4 mg, 9.4%) was obtained.
¹H NMR (CDCl₃, 400MHz) :δ=1.35(s,6H),1.8-1.9(m,1H), 2.25(s,1H),2.3-2.4(m,1H),3.12(dd,1H,J=5,10Hz), 3.3-3.4(m,2H),3.53(s,2H),3.65(dd,1H,J=6,10Hz), 3.78(s,2H),4.6-4.7(m,1H),5.48(d,1H,J=8Hz),6.8-6.9(m,3H),7.1-7.2(m,2H),8.07(d,1H,J=2Hz),8.21(s,1H).
MS:438.17[M+H]⁺

### Example 28

### 2-(4-Cyclopropylphenyl)-N-((3S,4S)-4-fluoro-1-(5-(trifluoromethyl)pyridin-3-yl)pyrrolidin-3-yl)acetamide

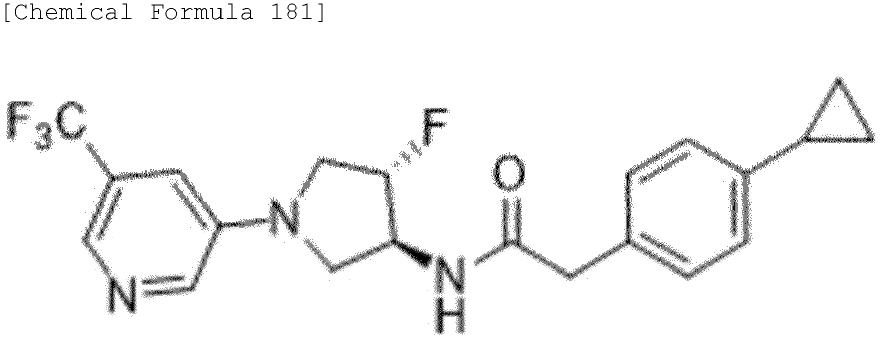

A 2 N hydrochloric acid-methanol solution (12 mL) was added to tert-butyl (3S,4S)-3-(2-(4-cyclopropylphenyl)-acetamido)-4-fluoropyrrolidine-1-carboxylate (369 mg, 1.02 mmol) synthesized in Reference Example 18 under ice cooling, the mixture was stirred for 2 hours at room temperature, subsequently the solvent was distilled off under reduced pressure, and thereby a crude form of (2-(4-cyclopropylphenyl)-N-((3S,4S)-4-fluoropyrrolidin-3-ylacetamide (230 mg) was obtained. The crude form of 2-(4-Cyclopropylphenyl)-N-((3S,4S)-4-fluoropyrrolidin-3-ylacetamide (230 mg) thus obtained, 3-bromo-5-(trifluoromethyl)pyridine (230 mg, 1.02 mmol), tris(dibenzylidene acetone)dipalladium(0) (92 mg, 0.10 mmol), Xantphos (118 mg, 0.20 mmol), and potassium carbonate (281 mg, 2.04 mmol) were suspended in toluene (5.8 mL), and the suspension was stirred for 16 hours at 85°C under a nitrogen gas stream. The reaction liquid that had been left to cool to room temperature was filtered through Celite, and the solvent was distilled off under reduced pressure. A residue thus obtained was purified by silica gel column chromatography (heptane : ethyl acetate) (concentration gradient: 0 to 100%), and the title compound (white amorphous, 28 mg, 6.7%) was obtained.
¹H NMR (CDCl₃, 400MHz) : δ=0.6-0.7 (m, 2H), 0.9-1.0 (m, 2H), 1.8-1.9(m,1H), 3.24(d, 1H, J=11Hz), 3.4-3.6(m,4H), 3.7-3.8(m,1H), 4.6-4.7(m,1H), 5.1-5.3(m,1H), 5.6-5.7(m,1H), 6.88(dd, 1H, J=2,2Hz), 7.0-7.1(m,4H), 8.04(d, 1H, J=3Hz), 8.21(s, 1H).
MS:408.16[M+H]⁺

### Example 29

### (R)-2-(4-(dimethylamino)phenyl)-N-(1-(5-(trifluoromethyl)pyridin-3-yl)pyrrolidin-3-yl)acetamide

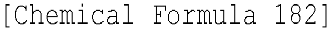

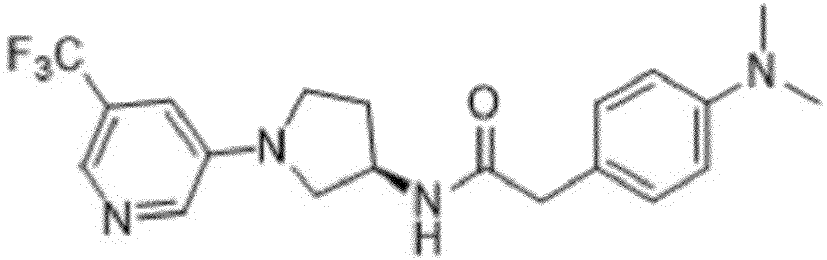

According to a technique similar to Example 1, the title compound (white amorphous, 40 mg, 51%) was obtained using (R)-1-(5-(trifluoromethyl)pyridin-3-yl)pyrrolidin-3-amine (46 mg, 0.20 mmol) synthesized in Reference Example 1-2 and 2-(4-(dimethylamino)phenyl)acetic acid (43 mg, 0.24 mmol) .
¹H NMR (CDCl₃, 400MHz) : δ=1.8-1.9 (m, 1H), 2.3-2.4 (m, 1H), 2.94(s,6H), 3.09(dd, 1H, J=5, 10Hz), 3.3-3.4(m, 2H), 3.49(s,2H), 3.64(dd, 1H, J=6,10Hz), 4.6-4. 7(m,1H), 5.63(d, 1H, J=7Hz), 6.6-6.7(m,2H), 6.88(dd, 1H, J=2,2Hz), 7.0-7.1(m,2H), 8.06(d, 1H, J=3Hz), 8.18(s,1H).
MS:393.16[M+H]⁺

### Example 30

### (R)-2-(4-nitrophenyl)-N-(1-(5-(trifluoromethyl)pyridin-3-yl)pyrrolidin-3-yl)acetamide

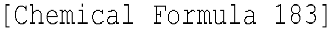

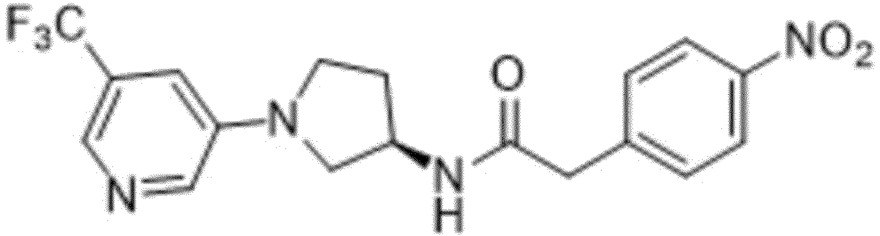

According to a technique similar to Example 1, the title compound (pale yellow amorphous, 219 mg, 86%) was obtained using (R)-1-(5-(trifluoromethyl)pyridin-3-yl)pyrrolidin-3-amine (150 mg, 0.65 mmol) synthesized in Reference Example 1-2 and 2-(4-nitrophenyl)acetic acid (141 mg, 0.78 mmol).
¹H NMR (CDCl₃, 400MHz) : δ=2.0-2.1 (m, 1H), 2.3-2.4 (m, 1H), 3.20(dd, 1H, J=3,10Hz), 3.3-3.4(m,2H), 3.63(dd, 1H, J=6,10Hz), 3.67(s,2H), 4.6-4.7(m, 1H), 6.59(d, 1H, J=7Hz), 6.8-6.9(m, 1H), 7.4-7.5(m, 2H),7.97(d, 1H, J=3Hz), 8.11(br s,1H), 8.1-8.2(m, 2H).
MS : 395.09[M+H]⁺

### Example 31

### (S)-2-(4-isobutylphenyl)-N-((R)-1-(5-(trifluoromethyl)pyridin-3-yl)pyrrolidin-3-yl)propanamide

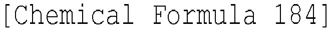

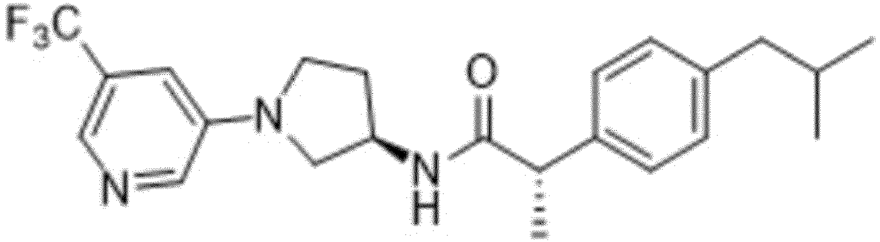

According to a technique similar to Example 1, the title compound (white crystals, 22 mg, 81%) was obtained using (R)-1-(5-(trifluoromethyl)pyridin-3-yl)pyrrolidin-3-amine (15 mg, 0.06 mmol) synthesized in Reference Example 1-2 and (S)-2-(4-isobutylphenyl)propanoic acid (16 mg, 0.08 mmol) .
¹H NMR (CDCl₃, 400MHz) : δ=0. 8-0. 9 (m, 6H), 1.51(d,3H, J=7Hz), 1.8-1.9(m,2H), 2.2-2.3(m,1H), 2.45(d,2H, J=7Hz), 3.13(dd,1H, J=4,10Hz), 3.2-3.4(m,2H), 3.5-3. 6(m,1H), 3. 63(dd,1H, J=6,10Hz), 4.5-4. 7(m,1H), 5.59(d, 1H, J=7Hz), 6.86(dd, 1H, J=2,2Hz), 7.0-7.1(m, 2H), 7.1-7.2(m, 2H), 8.03(d, 1H, J=3Hz), 8.16(s,1H).
MS : 418.25[M-H]⁻

### Example 32

### (R)-2-(3-fluoro-4-(trifluoromethyl)phenyl)-N-(1-(5-(trifluoromethyl)pyridin-3-yl)pyrrolidin-3-yl)acetamide

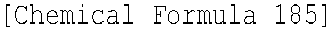

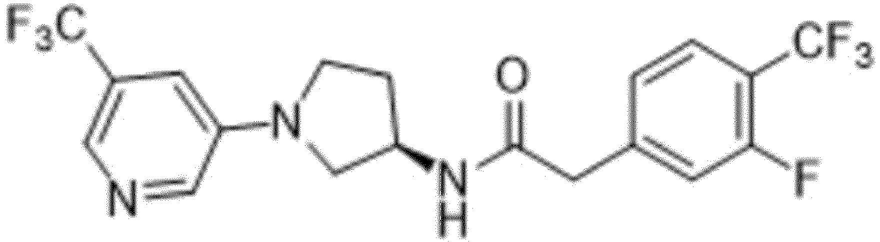

According to a technique similar to Example 1, the title compound (white amorphous, 25 mg, 66%) was obtained using (R)-1-(5-(trifluoromethyl)pyridin-3-yl)pyrrolidin-3-amine (20 mg, 0.09 mmol) synthesized in Reference Example 1-2 and 2-(3-fluoro-4-(trifluoromethyl)phenyl)acetic acid (23 mg, 0.10 mmol).
¹H NMR (CDCl₃, 400MHz) : δ=2.0-2.1 (m, 1H), 2.3-2.4 (m, 1H), 3.20(dd, 1H, J=4, 10Hz), 3.3-3.5(m, 2H), 3.60(s, 2H), 3.65(dd, 1H, J=6, 10Hz), 4.6-4.7(m, 1H), 6.27(d, 1H, J=7Hz), 6.86(dd, 1H, J=2,2Hz), 7.16(s,1H), 7.1-7.2(m, 1H), 7.57(t, 1H, J=7Hz), 8.02(d, 1H, J=3Hz), 8.14(s, 1H).
MS : 436.08[M+H]⁺

### Example 33

### (R)-2-(4-aminophenyl)-N-(1-(5-(trifluoromethyl)-pyridin-3-yl)pyrrolidin-3-yl)acetamide

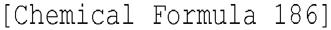

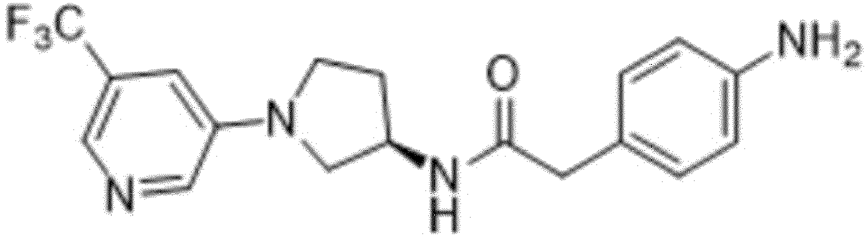

(R)-2-(4-nitrophenyl)-N-(1-(5-(trifluoromethyl)pyridin-3-yl)pyrrolidin-3-yl)acetamide (215 mg, 0.55 mmol) synthesized in Example 30 was dissolved in methanol (20 mL), palladium-carbon (20 mg) was added thereto, and the mixture was stirred for 6 hours at room temperature under a hydrogen gas stream. Insoluble materials were filtered, subsequently the solvent was distilled off under reduced pressure, a residue thus obtained was purified by silica gel column chromatography (ethyl acetate : methanol) (concentration gradient: 0 to 30%), and the title compound (white amorphous, 195 mg, 98%) was obtained.
¹H NMR (CDCl₃, 400MHz) : δ=1.8-1.9(m, 1H), 2.3-2.4 (m, 1H), 3.10(dd, 1H, J=5,10Hz), 3.3-3.4(m, 2H), 3.48(s, 2H), 3.64(dd, 1H, J=6,10Hz), 3.69(br s, 2H), 4.6-4.7(m, 1H), 5.59(d, 1H, J=7Hz), 6.6-6.7(m,2H), 6.8-6.9(m, 1H), 7.0-7.1(m,2 H), 8.06(d, 1H, J=3Hz).
MS:365.10[M+H]⁺

### Example 34

### (R)-1-(4-(trifluoromethyl)phenyl)-N-(1-(5-(trifluoromethyl)pyridin-3-yl)pyrrolidin-3-yl)cyclopropane-1-carboxyamide

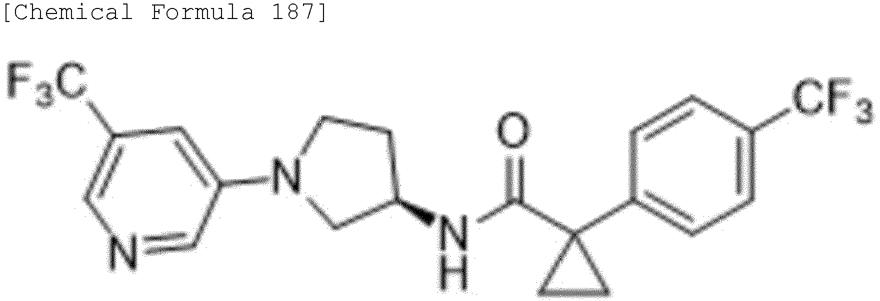

2-(4-(Trifluoromethyl)phenyl)acetonitrile (1.00 g, 5.40 mmol) was dissolved in THF (8.0 mL), subsequently the solution was stirred for a while at -50°C, a THF solution of lithium bis(trimethylsilyl)amide (1.3 M) (4.15 mL, 5.40 mmol) was added thereto, and then 1-bromo-2-chloroethane was added thereto. Subsequently, a THF solution of lithium bis(trimethylsilyl)amide (1.3 M) (4.15 mL, 5.40 mmol) was added thereto again, the mixture was stirred for 30 minutes, and then the mixture was stirred for 2 hours at room temperature. Water was added to the reaction liquid, the mixture was extracted with ethyl acetate, an organic layer thus separated was dried over anhydrous sodium sulfate, insoluble materials were filtered, subsequently the solvent was distilled off under reduced pressure, and thereby a crude form of 1-4-(trifluoromethyl)phenyl)cyclopropane-1-carbonitrile (pale yellow powder, 219 mg) was obtained. The crude form of 1-4-(trifluoromethyl)phenyl)cyclopropane-1-carbonitrile (219 mg) was dissolved in THF (3.0 mL), subsequently a 4 N aqueous solution of sodium hydroxide (3.0 mL) was added thereto, the mixture was stirred for 16 hours at 100°C, and the solvent was distilled off under reduced pressure. Water was added thereto to obtain an aqueous solution, subsequently the aqueous solution was washed with ethyl acetate, a 2 N aqueous solution of hydrochloric acid was added thereto to neutralize the aqueous solution, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and thereby a crude form of 1-(4-(trifluoromethyl)phenyl)cyclopropane-1-carboxylic acid (yellow crystals, 42 mg) was obtained. According to a technique similar to Example 1, the title compound (white amorphous, 43 mg, 1.8%) was obtained using the crude form of 1-(4-(trifluoromethyl)-phenyl)cyclopropane-1carboxylic acid (42 mg) and (R)-1-(5-(trifluoromethyl)pyridin-3-yl)pyrrolidin-3-amine (42 mg, 0.18 mmol) synthesized in Reference Example 1-2.
¹H NMR (CDCl₃, 400MHz) : δ=1.0-1.2(m, 2H), 1.6-1.7(m, 2H), 1.7-1.8(m, 1H), 2.3-2.4(m, 1H), 3.05(dd, 1H, J=5, 10Hz), 3.3-3.4(m,2H), 3.64(dd, 1H, J=6, 10Hz), 4.5-4.6(m, 1H), 5.26(d, 1H, J=6Hz), 6.8-6.9(m,1H), 7.53(d, 2H, J=8Hz), 7.64(d, 2H, J=8Hz), 8.0-8.1(m, 1H), 8.20(s,1H).
MS:444.07[M+H]⁺

### Example 35

### (R)-2-(4-acetamidophenyl)-N-(1-(5-(trifluoromethyl)pyridin-3-yl)pyrrolidin-3-yl)acetamide

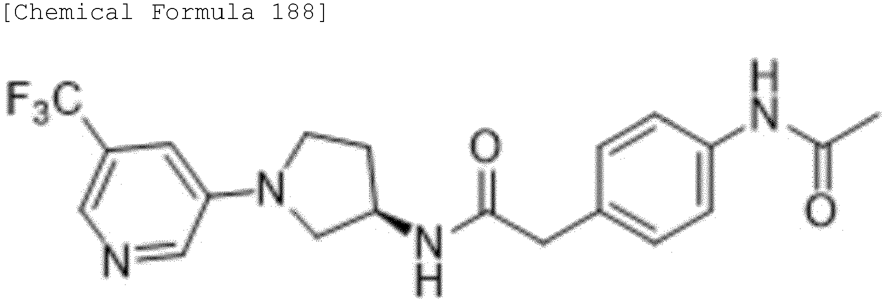

(R)-2-(4-aminophenyl)-N-(1-(5-(trifluoromethyl)pyridin-3-yl)pyrrolidin-3-yl)acetamide (30 mg, 0.08 mmol) synthesized in Example 33 was dissolved in chloroform (1.0 mL), subsequently acetyl chloride (5.9 µL, 0.08 mmol) and DIPEA (30 µL, 0.16 mmol) were added thereto, and the mixture was stirred for 60 hours at room temperature. Water was added to the reaction liquid, the mixture was stirred for a while, an organic layer thus separated was dried over anhydrous sodium sulfate, insoluble materials were filtered, and then the solvent was distilled off under reduced pressure. A residue thus obtained was purified by silica gel column chromatography (ethyl acetate : methanol) (concentration gradient: 0 to 40%), and the title compound (white amorphous, 6.0 mg, 18%) was obtained.
¹H NMR (CDCl₃, 400MHz) : δ=1.9-2.0(m, 1H), 2.18(s, 3H), 2.3-2.4(m, 1H), 3.12(dd, 1H, J=5,10Hz), 3.3-3.4(m, 2H), 3.54(s, 2H), 3.64(dd, 1H, J=6,10Hz), 4.6-4.7(m, 1H), 5.66(d, 1H, J=7Hz), 6.8-6.9(m, 1H), 7.2-7.3(m, 2H), 7.23(br s,1H), 7.4-7.5(m, 2H), 8.05(d, 1H, J=2Hz), 8.19(s, 1H)
MS:407.12[M+H]⁺

### Example 36

### N-(3-cyano-1-(5-(trifluoromethyl)pyridin-3-yl)pyrrolidin-3-yl)-2-(4-cyclopropylphenyl)acetamide

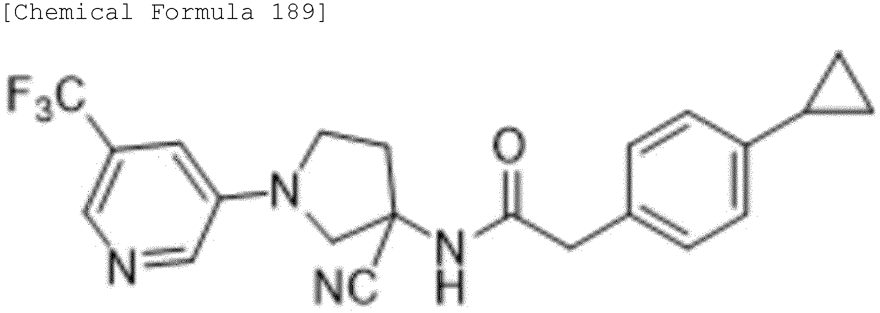

Trifluoroacetic acid (15 mL) was added to tert-butyl 3-cyano-3-(2-(4-cyclopropylphenyl)acetamido)pyrrolidine-1-carboxylate (454 mg, 1.23 mmol) synthesized in Reference Example 19 under ice cooling, the mixture was stirred for 4 hours at room temperature, subsequently a residue obtained by distilling off the solvent under reduced pressure was purified by silica gel column chromatography (ethyl acetate : methanol) (concentration gradient: 0 to 80%), and N-(3-cyanopyrrolidin-3-yl)-2-(4-cyclopropylphenyl)acetamide (brown oily material, 367 mg) was obtained. According to a technique similar to Example 9, the title compound (yellow powder, 30 mg, 5.8%) was obtained using N-(3-cyanopyrrolidin-3-yl)-2-(4-cyclopropylphenyl)acetamide (367 mg) thus obtained and 3-bromo-5-(trifluoromethyl)pyridine (278 mg, 1.23 mmol).
¹H NMR (CDCl₃, 400MHz) : δ=0.6-0.7 (m, 2H), 0.9-1.0 (m, 2H), 1.8-1.9(m, 1H), 2.1-2.2 (m, 1H), 2.4-2.5(m, 1H), 3.49(d, 1H, J=10Hz), 3.67(d, 1H, J=10Hz), 3.6-3.8(m, 4H), 6.9-7.0(m, 1H), 7.0-7.1(m, 2H), 7.1-7.2(m, 2H), 8.00(br s, 1H), 8.12(d, 1H, J=3Hz), 8.20(s, 1H).
MS : 415.13[M+H]⁺

### Example 37

### Methyl 3-(2-(1H-indol-6-yl)acetamido)-1-(5-(trifluoromethyl)pyridin-3-yl)pyrrolidine-3-carboxylate

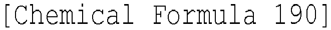

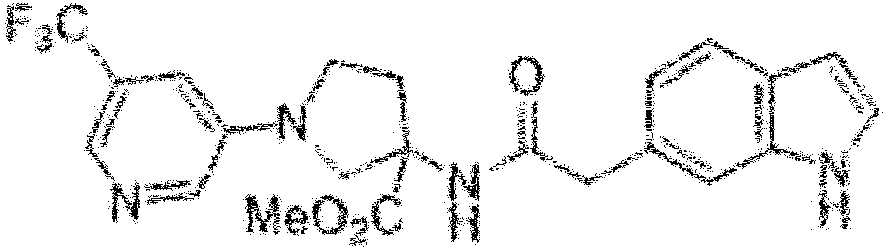

According to a technique similar to Example 7, methyl 3-amino-1-(5-(trifluoromethyl)pyridin-3-yl)pyrrolidine-3-carboxylate was synthesized from methyl 3-((tert-butoxycarbonyl)amino)-1-(5-(trifluoromethyl)pyridin-3-yl)pyrrolidine-3-carboxylate (34 mg, 0.09 mmol) synthesized in Reference Example 20-2, and the title compound (white powder, 26 mg, 67%) was obtained using 2-(1H-indol-6-yl)acetic acid (15 mg, 0.09 mmol).
¹H NMR (CDCl₃, 400MHz) : δ=2.2-2.3 (m, 1H), 2.4-2.5 (m, 1H), 3.3-3.5(m,2H),3.61(d, 1H, J=10Hz), 3.71(s,2H), 3.76(s,3H), 4.00(d, 1H, J=10Hz), 5.94(s, 1H), 6.5-6.6(m, 1H), 6.8-6.9(m,1H),6.98(dd, 1H, J=2,8Hz),7.2-7.3(m, 2H), 7.62(d, 1H, J=8Hz), 8.06(d, 1H, J=3Hz), 8.16(br s,1H), 8.22(s, 1H).
MS : 447.10[M+H]⁺

### Example 38

### Methyl 3-(2-(4-(trifluoromethyl)phenyl)acetamido)-1-(5-(trifluoromethyl)pyridin-3-yl)pyrrolidine-3-carboxylate

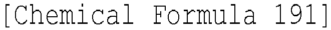

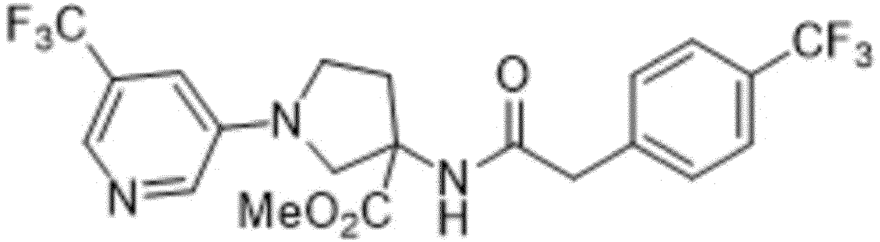

Methyl 3-(2-(4-(trifluoromethyl)phenyl)acetamido)pyrrolidine-3-carboxylate (294 mg, 0.89 mmol) synthesized in Reference Example 21-2, 3-bromo-5-(trifluoromethyl)pyridine (302 mg, 1.34 mmol), palladium acetate (20 mg, 0.09 mmol), XPhos (85 mg, 0.18 mmol), and cesium carbonate (628 mg, 1.78 mmol) were suspended in toluene (9.0 mL), and the suspension was stirred for 6 hours at 85°C under a nitrogen gas stream. The suspension was left to cool to room temperature, insoluble materials were filtered through Celite, and the solvent was distilled off under reduced pressure. A residue thus obtained was purified by silica gel column chromatography (heptane : ethyl acetate) (concentration gradient: 0 to 100%), and the title compound (white amorphous, 8.1 mg, 1.9%) was obtained.
¹H NMR (CDCl₃, 400MHz) : δ=2.4-2.5 (m, 1H), 2.5-2.6 (m, 1H), 3.4-3.5(m, 2H), 3.6-3.7(m, 3H), 3.76(s, 3H), 3.98(d, 1H, J=11Hz), 6.24(s, 1H), 6.9-7.0(m, 1H), 7.40(d, 2H, J=8Hz), 7.61(d, 2H, J=8Hz), 8.06(d, 1H, J=3Hz), 8.21(s, 1H).
MS : 476.09[M+H]⁺

### Example 39

### (1S,2S)-2-(benzo[d]oxazol-2-yl)-N-((R)-1-(5-(trifluoromethyl)pyridin-3-yl)pyrrolidin-3-yl)cyclopropane-1-carboxyamide

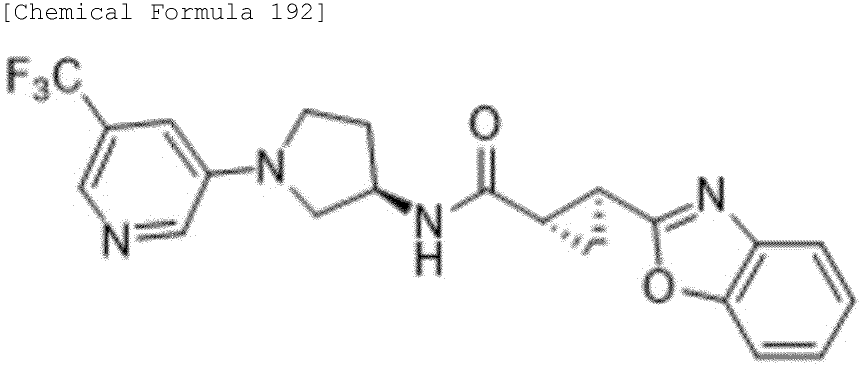

According to a technique similar to Example 26, (1S,2S)-2-(benzo[d]oxazol-2-yl)cyclopropane-1-carboxylic acid (white powder) was synthesized from methyl (1S,2S)-2-(benzo[d]oxazol-2-yl)cyclopropane-1-carboxylate (95 mg, 0.44 mmol) synthesized in Reference Example 25-2, and the title compound (white powder, 72 mg, 40%) was obtained using (R)-1-(5-(trifluoromethyl)pyridin-3-yl)pyrrolidin-3-amine (101 mg, 0.44 mmol) synthesized in Reference Example 1-2.
¹H NMR (CDCl₃, 400MHz) : δ=1.6-1.7 (m, 1H), 1.8-1.9 (m, 1H), 2.0-2.1(m, 1H), 2.1-2.2(m, 1H), 2.3-2.4(m, 1H), 2.7-2.8(m, 1H), 3.2-3.3(m, 1H), 3.4-3.5(m, 1H), 3.5-3.6(m, 1H), 3.6-3.7(m, 1H), 4.7-4.8(m, 1H), 6.18(d, 1H, J=6Hz), 6.92(s,1H), 7.2-7.3(m, 2H), 7.4-7.5(m, 1H), 7.6-7.7(m, 1H), 8.10(d, 1H, J=2Hz), 8.20(s, 1H).
MS : 417.11[M+H]⁺

### Example 40

### (R)-2-(4-morpholinophenyl)-N-(1-(4-(trifluoromethyl)thiazol-2-yl)pyrrolidin-3-yl)acetamide

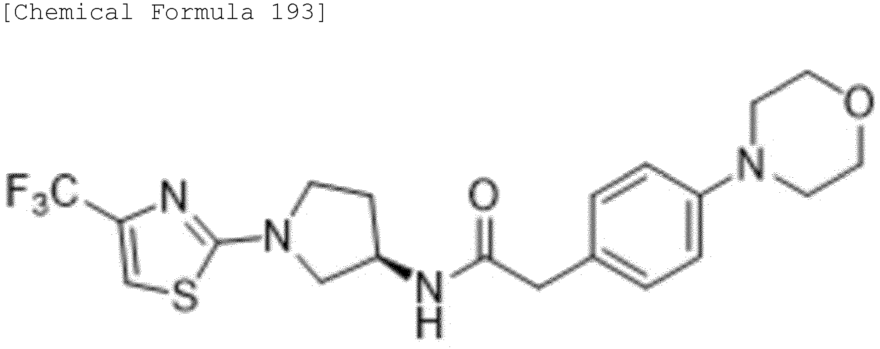

According to a technique similar to Example 1, the title compound (white powder, 60 mg, 46%) was obtained using (R)-1-(4-(trifluoromethyl)thiazol-2-yl)pyrrolidin-3-amine (70 mg, 0.30 mmol) synthesized in Reference Example 12-2 and 2-(4-morpholinophenyl)acetic acid (98 mg, 0.44 mmol) synthesized in Reference Example 23-2.
¹H NMR (CDCl₃, 400MHz) : δ=1.8-1.9(m, 1H), 2.3-2.4 (m, 1H), 3.1-3.2(m, 4H), 3.23(dd, 1H, J=4, 10Hz), 3.4-3.6(m, 4H), 3.75(dd, 1H, J=6, 10Hz), 3.8-3.9(m, 4H), 4.5-4.7(m, 1H), 5.48 (d, 1H, J=7Hz), 6.88(d, 2H, J=9Hz), 6.92(s, 1H), 7.12(d, 2H, J=8Hz).
MS : 441.10[M+H]⁺

### Example 41

### Methyl 3-(2-(4-morpholinophenyl)acetamido)-1-(5-(trifluoromethyl)pyridin-3-yl)pyrrolidine-3-carboxylate

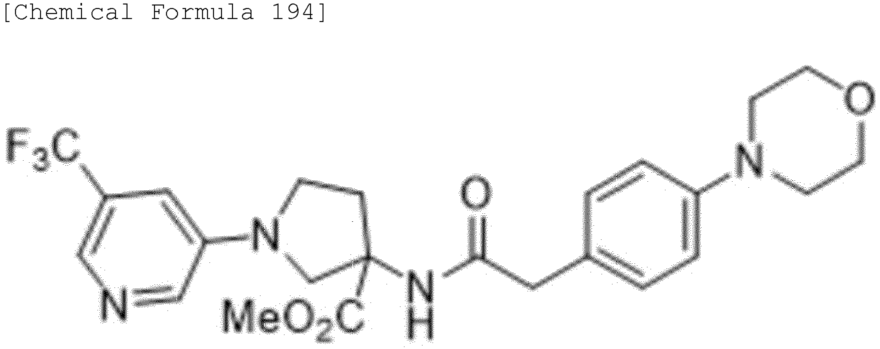

According to a technique similar to Example 7, methyl 3-amino-1-(5-(trifluoromethyl)pyridin-3-yl)pyrrolidine-3-carboxylate was synthesized from methyl 3-((tert-butoxycarbonyl)amino)-1-(5-(trifluoromethyl)pyridin-3-yl)pyrrolidine-3-carboxylate (39 mg, 0.10 mmol) synthesized in Reference Example 20-2, and the title compound (white powder, 21 mg, 43%) was obtained using 2-(4-morpholinophenyl)acetic acid (33 mg, 0.15 mmol) synthesized in Reference Example 23-2.
¹H NMR (CDCl₃, 400MHz) :δ=2.3-2.4(m, 1H), 2.5-2.6(m, 1H), 3.1-3.2(m, 4H), 3.4-3.5(m, 2H), 3.53(s, 2H), 3.62(d, 1H, J=10Hz), 3.76(s,3H), 3.8-3.9(m, 4H), 4.00(d, 1H, J=10Hz), 5.99(s, 1H), 6.8-6.9(m, 2H), 6.91(dd, 1H, J=2,2Hz), 7.1-7.2(m, 2H), 8.07(d, 1H, J=3Hz), 8.22(s, 1H).
MS : 493.16[M+H]⁺

### Example 42

### (R)-2-(4-morpholinophenyl)-N-(1-(6-(trifluoromethyl)pyridin-3-yl)pyrrolidin-3-yl)acetamide

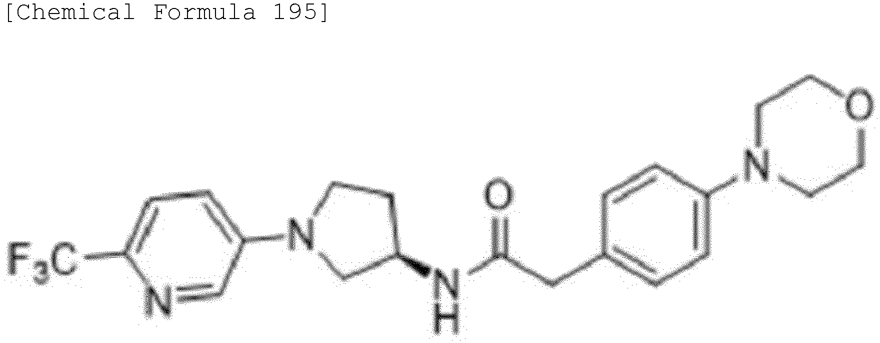

According to a technique similar to Example 1, the title compound (white powder, 59 mg, 45%) was obtained using (R)-1-(6-(trifluoromethyl)pyridin-3-yl)pyrrolidin-3-amine (70 mg, 0.30 mmol) synthesized in Reference Example 32-2 and 2-(4-morpholinophenyl)acetic acid (100 mg, 0.45 mmol) synthesized in Reference Example 23-2.
¹H NMR (CDCl₃, 400MHz) : δ=1.8-1.9 (m, 1H), 2.3-2.4 (m, 1H), 3.1-3.2(m,5H),3.3-3.4(m,2H),3.51(s,2H), 3.66(dd, 1H, J=6,10Hz), 3.8-3.9(m, 4H), 4.6-4.7(m, 1H), 5.50(d, 1H, J=7Hz), 6.79(dd, 1H, J=3,9Hz), 6.8-6.9(m, 2H), 7.1-7.2(m, 2H), 7.51(d, 1H, J=9Hz), 7.97(d, 1H, J=9Hz).
MS : 435.15[M+H]⁺

### Example 43

### 2-(4-Cyclopropylphenyl)-N-(3-ethyl-1-(5-(trifluoromethyl)pyridin-3-yl)pyrrolidin-3-yl)acetamide

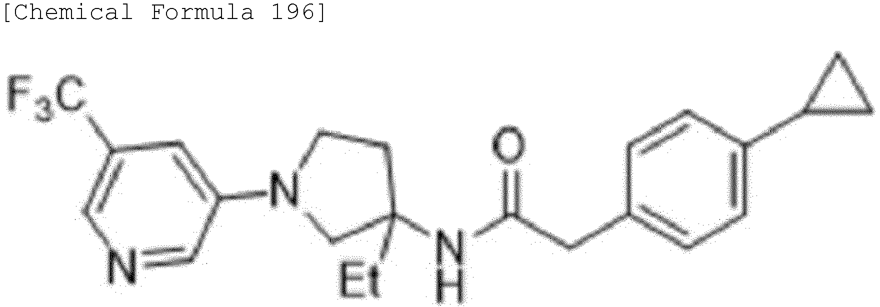

According to a technique similar to Example 38, 2-(4-cyclopropylphenyl)-N-(3-ethylpyrrolidin-3-yl)acetamide (yellow amorphous, 578 mg) was synthesized from tert-butyl 3-(2-(4-cyclopropylphenyl)acetamido)-3-ethylpyrrolidine-1-carboxylate (782 mg, 2.10 mmol) synthesized in Reference Example 24, and the title compound (orange-colored amorphous, 400 mg, 46%) was obtained using 3-bromo-5-(trifluoromethyl)pyridine (575 mg, 2.54 mmol).
¹H NMR (CDCl₃, 400MHz) : δ=0.6-0.7 (m, 2H), 0.84(t,3H,J=8Hz),0.9-1.0(m,2H),1.8-1.9(m,2H), 1.9-2.0(m, 1H), 2.0-2.1(m, 1H), 2.2-2.3(m, 1H), 3.2-3.4(m, 3H), 3.49(s,2H), 3.65(d, 1H, J=10Hz), 5.21(s, 1H), 6.88(dd, 1H, J=2,2Hz) , 6.9-7.0(m, 2H), 7.0-7.1(m, 2H), 8.05(d, 1H, J=3Hz), 8.19(s, 1H).
MS : 418.17[M+H]⁺

### Example 44

### (1S, 2S)-2-(6-fluorobenzo[d]oxazol-2-yl)-N-((R)-1-(5-(trifluoromethyl)pyridin-3-yl)pyrrolidin-3-yl)cyclopropane-1-carboxyamide

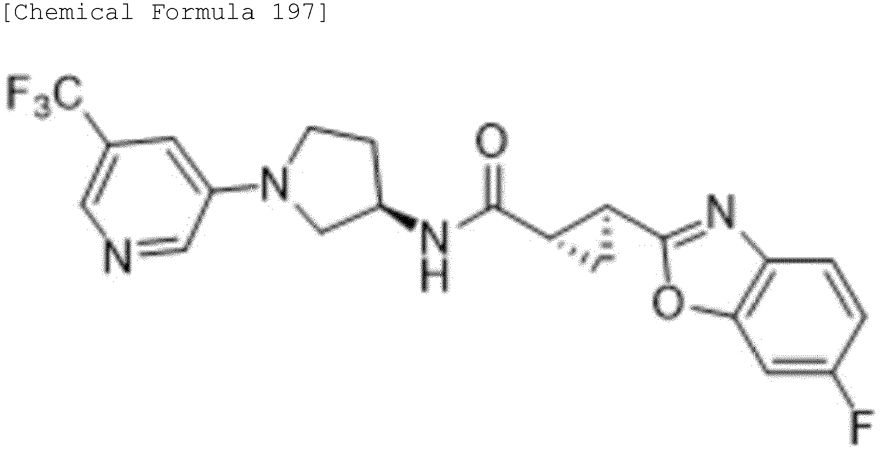

According to a technique similar to Example 26, (1S,2S)-2-(6-fluorobenzo[d]oxazol-2-yl)cyclopropane-1-carboxylic acid (white powder) was synthesized from methyl (1S,2S)-2-(6-fluorobenzo[d]oxazol-2-yl)cyclopropane-1-carboxylate (105 mg, 0.45 mmol) synthesized in Reference Example 22-4, and the title compound (white powder, 89 mg, 46%) was obtained using (R)-1-(5-(trifluoromethyl)pyridin-3-yl)pyrrolidin-3-amine (103 mg, 0.45 mmol) synthesized in Reference Example 1-2.
¹H NMR(CDCl₃, 400MHz) :δ=1.6-1.7 (m, 1H), 1.7-1.8 (m, 1H), 2.0-2.1(m, 1H), 2.1-2.2(m, 1H), 2.3-2.4(m, 1H), 2.7-2.8(m, 1H), 3.27(dd, 1H, J=3,10Hz), 3.3-3.5(m, 2H), 3.65(dd, 1H, J=6,10Hz), 4.7-4.8(m, 1H), 6.71(d, 1H, J=7Hz), 6.85(dd, 1H, J=2,2Hz), 7.0-7.1(m, 1H), 7.16(dd, 1H, J=2,8Hz), 7.51(dd, 1H, J=5,8Hz), 8.04(d, 1H, J=3Hz), 8.15(d, 1H, J=0.8Hz).
MS : 435.10 [M+H]⁺

### Example 45

### (1S,2S)-2-(5,6-difluorobenzo[d]oxazol-2-yl)-N-((R)-1-(5-(trifluoromethyl)pyridin-3-yl)pyrrolidin-3-yl)cyclopropane-1-carboxyamide

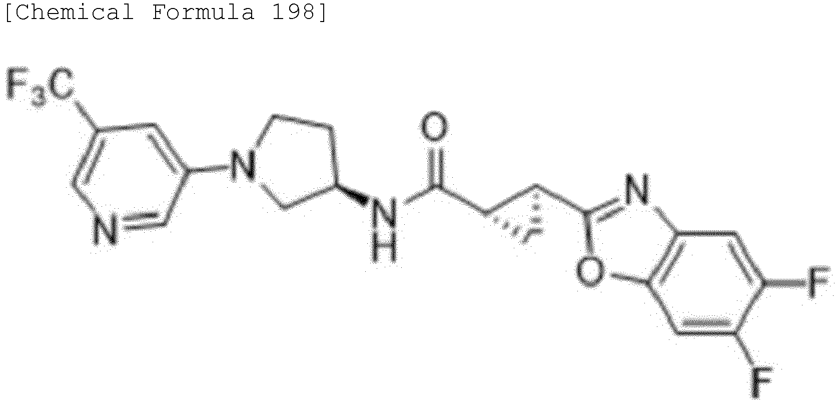

According to a technique similar to Example 26, (1S,2S)-2-(5,6-difluorobenzo[d]oxazol-2-yl)cyclopropane-1-carboxylic acid (white powder) was synthesized from methyl (1S,2S)-2-(5,6-difluorobenzo[d]oxazol-2-yl)cyclopropane-1-carboxylate (68 mg, 0.27 mmol) synthesized in Reference Example 26-2, and the title compound (white powder, 35 mg, 29%) was obtained using (R)-1-(5-(trifluoromethyl)pyridin-3-yl)pyrrolidin-3-amine (62 mg, 0.27 mmol) synthesized in Reference Example 1-2.
¹H NMR(CDCl₃, 400MHz) :δ=1.6-1.7(m, 1H), 1.7-1.8(m, 1H), 2.0-2.1(m, 1H), 2.1-2.2(m, 1H),2.3-2.4(m,1H), 2.7-2.8(m, 1H), 3.29(dd, 1H, J=4,10Hz), 3.4-3.5(m, 1H), 3.5-3.6(m, 1H), 3.67(dd, 1H, J=6,10Hz), 4.7-4.8(m, 1H), 6.25(d, 1H, J=7Hz), 6.91(dd, 1H, J=2,2Hz), 7.30(dd, 1H, J=7,9Hz), 7.39(dd, 1H, J=8,10Hz), 8.10(d, 1H, J=3Hz), 8.19(d, 1H, J=0.8Hz).
MS : 453.09 [M+H]⁺

### Example 46

### (R)-2-(4-(2-oxa-6-azaspiro[3.3]heptan-6-yl)phenyl)-N-(1-(5-(trifluoromethyl)pyridin-3-yl)pyrrolidin-3-yl)acetamide

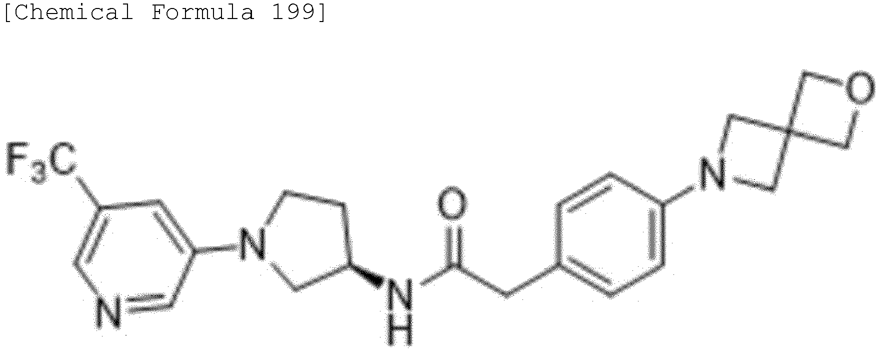

According to a technique similar to Example 26, 2-(4-(2-oxa-6-azaspiro[3.3]heptan-6-yl)phenyl)acetic acid (white powder, 1.02 g) was synthesized from ethyl 2-(4-(2-oxa-6-azaspiro[3.3]heptan-6-yl)phenyl)acetate (1.26 g, 4.82 mmol) synthesized in Reference Example 27, and the title compound (ivory-colored powder, 106 mg, 91%) was obtained using (R)-1-(5-(trifluoromethyl)pyridin-3-yl)pyrrolidin-3-amine (60 mg, 0.26 mmol) synthesized in Reference Example 1-2.
¹H NMR (CDCl₃, 400MHz) : δ=1.8-1.9 (m, 1H), 2.3-2.4 (m, 1H), 3.09(dd, 1H, J=5,10Hz), 3.3-3.4(m,2H), 3.47(s, 2H), 3.62(dd, 1H, J=6,10Hz), 4.00(s,4H), 4.6-4.7(m, 1H), 4.83(s, 4H), 5.73(d, 1H, J=7Hz), 6.4-6.5(m,2H), 6.86(dd, 1H, J=3,9Hz), 7.0-7.1(m, 2H), 8.04(d, 1H, J=3Hz), 8.17(d, 1H, J=0.8Hz).
MS : 447.19[M+H]⁺

### Example 47

### (1S,2S)-2-(benzo[d]oxazol-2-yl)-N-((R)-1-(4-(trifluoromethyl)thiazol-2-yl)pyrrolidin-3-yl)cyclopropane-1-carboxyamide

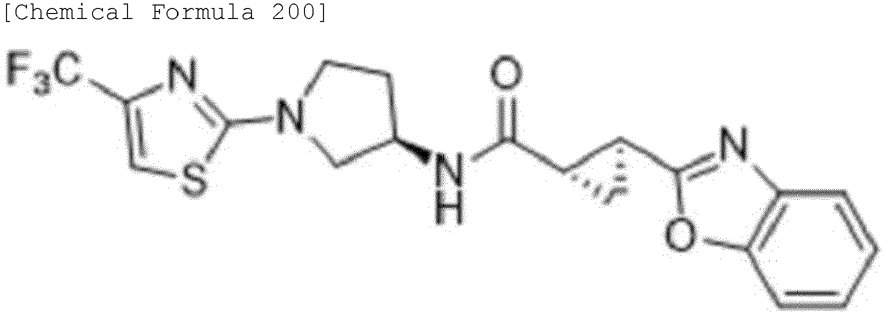

According to a technique similar to Example 26, (1S,2S)-2-(benzo[d]oxazol-2-yl)cyclopropane-1-carboxylic acid was synthesized from methyl (1S,2S)-2-(benzo[d]oxazol-2-yl)cyclopropane-1-carboxylate (110 mg, 0.51 mmol) synthesized in Reference Example 25-2, and the title compound (white powder, 90 mg, 42%) was obtained using (R)-1-(4-(trifluoromethyl)thiazol-2-yl)pyrrolidin-3-amine (120 mg, 0.51 mmol) synthesized in Reference Example 25-2.
¹H NMR (CDCl₃, 400MHz) : δ=1.6-1.7 (m, 1H), 1.7-1.8 (m, 1H), 2.0-2.1(m, 1H), 2.1-2.2(m, 1H), 2.3-2.4(m, 1H), 2.7-2.8(m, 1H), 3.44(dd, 1H, J=4,10Hz), 3.5-3.7(m, 2H), 3.80(dd, 1H, J=6,10Hz), 4.6-47(m, 1H), 5.95(d, 1H, J=7Hz), 6.95(d, 1H, J=0.8Hz), 7.2-7.3(m, 2H), 7.4-7.5(m, 1H), 7.6-7.7(m, 1H).
MS : 423.07[M+H]⁺

### Example 48

### (1S,2S)-2-(6-fluorobenzo[d]oxazol-2-yl)-N-((R)-1-(6-(trifluoromethyl)pyridin-3-yl)pyrrolidin-3-yl)cyclopropane-1-carboxyamide

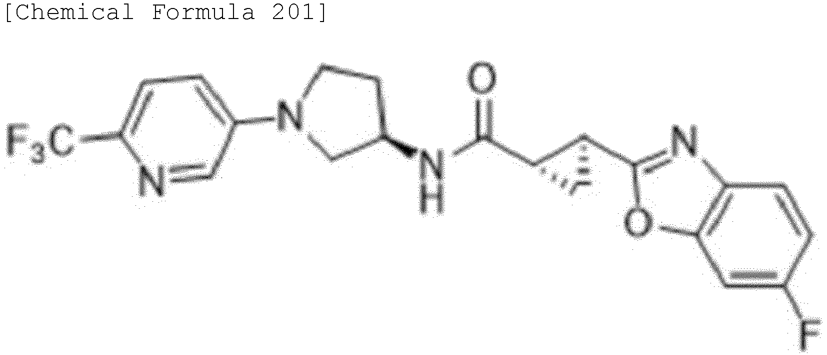

According to a technique similar to Example 26, (1S,2S)-2-(6-fluorobenzo[d]oxazol-2-yl)cyclopropane-1-carboxylic acid (ivory-colored powder) was synthesized from methyl (1S,2S)-2-(6-fluorobenzo[d]oxazol-2-yl)cyclopropane-1-carboxylate (121 mg, 0.51 mmol) synthesized in Reference Example 22-4, and the title compound (white powder, 118 mg, 53%) was obtained using (R)-1-(6-(trifluoromethyl)pyridin-3-yl)pyrrolidin-3-amine (119 mg, 0.51 mmol) synthesized in Reference Example 22-2.
¹H NMR (CDCl₃, 400MHz) : δ=1.6-1.7 (m, 1H), 1.7-1.8 (m, 1H), 2.1-2.2(m, 1H), 2.2-2.3(m, 1H), 2.3-2.4(m,1H), 2.7-2.8(m, 1H), 3.2-3.3(m, 1H), 3.3-3.4(m, 1H), 3.4-3.5(m,1H), 3.6-3.7(m, 1H), 4.7-4.8(m, 1H), 6.7-6.8(m, 2H), 7.0-7.1(m,1H), 7.1-7.2(m, 1H), 7.39(d, 1H, J=8Hz),7.50(dd, 1H, J=5,8Hz), 7.90(s, 1H).
MS : 435.10[M+H]⁺

### Example 49

### (1S,2S)-2-(5-fluorobenzo[d]oxazol-2-yl)-N-((R)-1-(5-(trifluoromethyl)pyridin-3-yl)pyrrolidin-3-yl)cyclopropane-1-carboxyamide

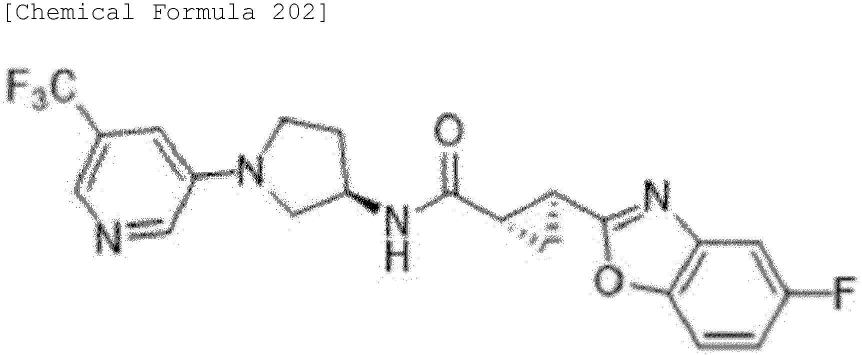

According to a technique similar to Example 26, (1S,2S)-2-(5-fluorobenzo[d]oxazol-2-yl)cyclopropane-1-carboxylic acid (ivory-colored powder) was synthesized from methyl (1S,2S)-2-(5-fluorobenzo[d]oxazol-2-yl)cyclopropane-1-carboxylate (47 mg, 0.20 mmol) synthesized in Reference Example 28-2, and the title compound (white powder, 51 mg, 59%) was obtained using (R)-1-(5-(trifluoromethyl)pyridin-3-yl)pyrrolidin-3-amine (66 mg, 0.29 mmol) synthesized in Reference Example 1-2.
¹H NMR (CDCl₃, 400MHz) : δ=1.6-1.7 (m, 1H), 1.7-1.8 (m, 1H), 2.0-2.1(m, 1H), 2.1-2.2(m, 1H), 2.3-2.4(m, 1H), 2.7-2.8(m, 1H), 3.29(dd, 1H, J=4,10Hz), 3.4-3.5(m, 1H), 3.5-3.6(m, 1H), 3.69(dd, 1H, J=6,10Hz), 4.7-4.8(m, 1H), 6.07(d, 1H, J=7Hz), 6.9-7.0(m, 1H),7.0-7.1(m, 1H), 7.29(dd, 1H, J=2,8Hz), 7.37(dd, 1H, J=4,9Hz), 8.12(d, 1H, J=3Hz), 8.22(s, 1H).
MS : 435.10[M+H]⁺

### Example 50

### (1R,2R)-2-(benzo[d]oxazol-2-yl)-N-((R)-1-(5-(trifluoromethyl)pyridin-3-yl)pyrrolidin-3-yl)cyclopropane-1-carboxyamide

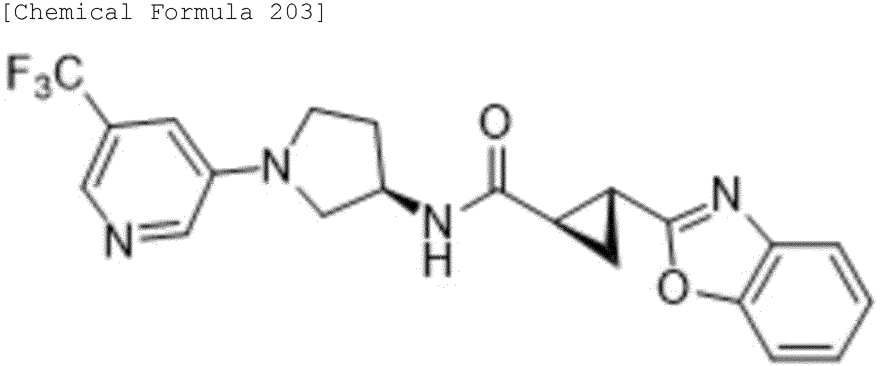

According to a technique similar to Example 26, trans-2-(benzo[d]oxazol-2-yl)cyclopropane-1-carboxylic acid (ivory-colored powder) was synthesized from ethyl trans-2-(benzo[d]oxazol-2-yl)cyclopropane-1-carboxylate (200 mg, 0.86 mmol) synthesized in Reference Example 29-3, and the title compound (white amorphous, 7.3 mg, 2.0%) was obtained using (R)-1-(5-(trifluoromethyl)pyridin-3-yl)pyrrolidin-3-amine (200 mg, 0.86 mmol) synthesized in Reference Example 1-2.
¹H NMR(CDCl₃, 400MHz) :δ=1.6-1.7 (m, 1H), 1.7-1.8 (m, 1H), 2.0-2.1(m, 1H), 2.1-2.2(m, 1H), 2.3-2.4(m, 1H), 2.7-2.8(m, 1H), 3.27(dd, 1H, J=4,10Hz), 3.4-3.5(m, 1H), 3.5-3.6(m, 1H), 3.67(dd, 1H, J=6,10Hz), 4.7-4.8(m, 1H), 6.07(d, 1H, J=8Hz), 6.9-7.0(m, 1H), 7.2-7.3(m, 2H), 7.4-7.5(m, 1H), 7.6-7.7(m, 1H), 8.10(d, 1H, J=2Hz), 8.20(s, 1H).
MS : 417.09[M+H]⁺

### Example 51

### (1S,2S)-2-(benzo[d]oxazol-2-yl)-N-((R)-1-(5-(trifluoromethyl)pyridin-3-yl)pyrrolidin-3-yl)cyclopropane-1-carboxyamide

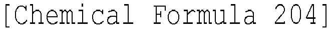

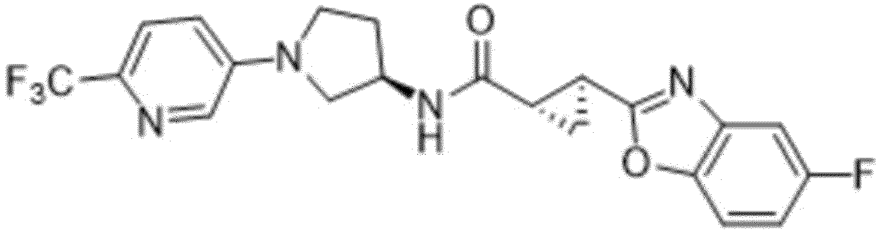

According to a technique similar to Example 26, (1S,2S)-2-(5-fluorobenzo[d]oxazol-2-yl)cyclopropane-1-carboxylic acid (ivory-colored powder) was synthesized from ethyl (1S,2S)-2-(5-fluorobenzo[d]oxazol-2-yl)cyclopropane-1-carboxylate (141 mg, 0.57 mmol) synthesized in Reference Example 30-3, and the title compound (white amorphous, 60 mg, 24%) was obtained using (R)-1-(6-(trifluoromethyl)-pyridin-3-yl)pyrrolidin-3-amine (131 mg, 0.57 mmol) synthesized in Reference Example 32-2.
¹H NMR (CDCl₃, 400MHz) : δ=1.6-1.7 (m, 1H), 1.7-1.8 (m, 1H), 2.0-2.1(m, 1H), 2.1-2.2(m, 1H), 2.3-2.4(m, 1H), 2.7-2.8(m, 1H), 3.30(dd, 1H, J=4,10Hz), 3.4-3.5(m, 1H), 3.5-3.6(m, 1H), 3.69(dd, 1H, J=6,10Hz), 4.7-4.8(m,1H), 6.20(d, 1H, J=7Hz), 6.80(dd,1H, J=2,8Hz), 7.0-7.1(m,1H), 7.2-7.3(m, 1H), 7.36(dd, 1H, J=4,8Hz), 7.46(d, 1H, J=8Hz), 7.98(d, 1H, J=2Hz).
MS : 435.10[M+H]⁺

### Example 52

### Trans-2-(1H-benzo[d]imidazol-2-yl)-N-((R)-1-(4-(trifluoromethyl)phenyl)pyrrolidin-3-yl)cyclopropane-1-carboxyamide

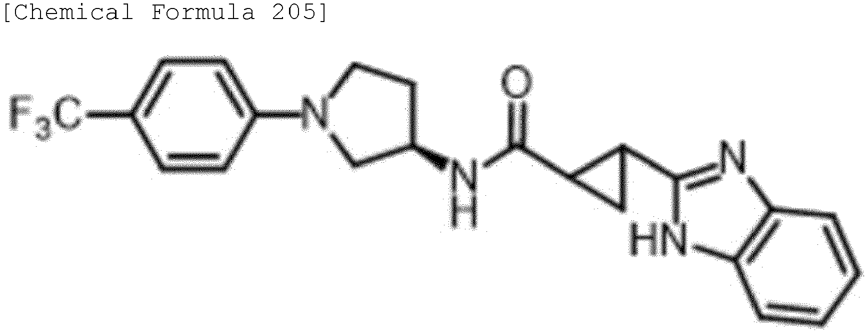

According to a technique similar to Example 3, a crude form of (R)-1-(4-(trifluoromethyl)phenyl)pyrrolidin-3-amine (317 mg) was synthesized from tert-butyl (R)-(1-(4-(trifluoromethyl)phenyl)pyrrolidin-3-yl)carbamate (273 mg, 0.83 mmol) synthesized in Reference Example 34, and diastereomer A of the title compound (Rf value in TLC (ethyl acetate) = 0.4, pale yellow amorphous, 16 mg, 19%) was obtained using trans-2-(1H-benzo[d]imidazol-2-yl)cyclopropane-1-carboxylic acid (40 mg, 0.20 mmol).

### Diastereomer A

¹H NMR(DMSO-d₆, 400MHz) : δ=1.4-1.5(m, 2H), 1.8-2.0 (m, 1H), 2.1-2.3(m, 2H), 2.4-2.5(m, 1H), 3.15(dd, 1H, J=4,10Hz), 3.3-3.5(m,2H), 3.55(dd, 1H, J=6,10Hz), 4.3-4.5(m, 1H), 6.63(d, 2H, J=9Hz), 7.0-7.2(m, 2H), 7.3-7.5(m, 2H), 7.46(d, 2H, J=9Hz), 8.64(d, 1H, J=6Hz), 12.38(s, 1H).
MS : 415.16[M+H]⁺

### Example 53

### Trans-2-(1H-benzo[d]imidazol-2-yl)-N-((S)-1-(4-(trifluoromethyl)phenyl)pyrrolidin-3-yl)cyclopropane-1-carboxyamide

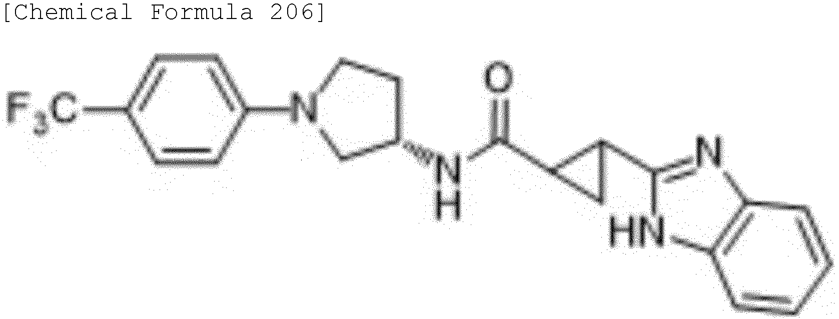

According to a technique similar to Example 3, a crude form of (S)-1-(4-(trifluoromethyl)phenyl)pyrrolidin-3-amine (170 mg) was synthesized from tert-butyl (S)-(1-(4-(trifluoromethyl)phenyl)pyrrolidin-3-yl)carbamate (236 mg, 0.72 mmol) synthesized in Reference Example 35, and diastereomer A of the title compound (Rf value in TLC (ethyl acetate) = 0.5, white powder, 14 mg, 17%) was obtained using trans-2-(1H-benzo[d]imidazol-2-yl)cyclopropane-1-carboxylic acid (40 mg, 0.20 mmol).

### Diastereomer A

¹H NMR(DMSO-d₆, 400MHz) : δ=1.4-1.5(m, 2H), 1.8-2.0 (m, 1H), 2.1-2.3(m, 2H), 2.4-2.5(m, 1H), 3.15(dd, 1H, J=4,10Hz), 3.3-3.6(m, 3H), 4.3-4.5(m, 1H), 6.63(d, 2H, J=9Hz), 7.0-7.2(m, 2H), 7.3-7.5(m, 2H), 7.46(d, 2H, J=9Hz), 8.64(d, 1H, J=7Hz), 12.39(s, 1H).
MS : 415.18 [M+H]⁺

### Example 54

### (1S,2S)-2-(1H-benzo[d]imidazol-2-yl)-N-((R)-1-(6-(trifluoromethyl)pyridin-3-yl)pyrrolidin-3-yl)cyclopropane-1-carboxyamide

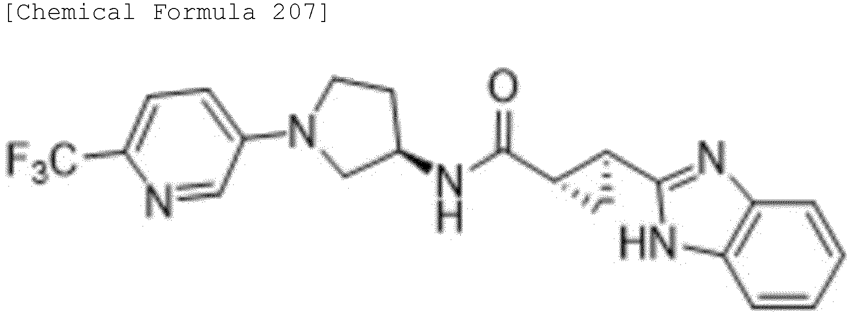

According to a technique similar to Example 3, a crude form of (R)-1-(6-(trifluoromethyl)pyridin-3-yl)pyrrolidin-3-amine (300 mg) was synthesized from tert-butyl (R)-(1-(6-(trifluoromethyl)pyridin-3-yl)pyrrolidin-3-yl)carbamate (429 mg, 1.30 mmol) synthesized in Reference Example 36, and the title compound (white powder, 32 mg, 48%) was obtained using (1S,2S)-2-(1H-benzo[d]imidazol-2-yl)cyclopropane-1-carboxylic acid (32 mg, 0.16 mmol).
¹H NMR(DMSO-d₆, 400MHz) : δ=1.4-1.5(m, 2H), 1.9-2.0 (m, 1H), 2.1-2.3(m, 2H), 2.4-2.5(m, 1H), 3.21(dd, 1H, J=4,10Hz), 3.3-3.5(m,2H), 3.60(dd, 1H,J=6,10Hz), 4.4-4.5(m, 1H), 7.01(d, 1H, J=3,9Hz), 7.0-7.2(m, 2H), 7.3-7.5(m, 2H), 7.59(d, 1H, J=9Hz), 8.04(d, 1H, J=3Hz), 8.65(d, 1H, J=7Hz), 12.41(s, 1H).
MS : 416.18[M+H]⁺

### Example 55

### (1S,2S)-2-(1H-benzo[d]imidazol-2-yl)-N-((S)-1-(6-(trifluoromethyl)pyridin-3-yl)pyrrolidin-3-yl)cyclopropane-1-carboxyamide

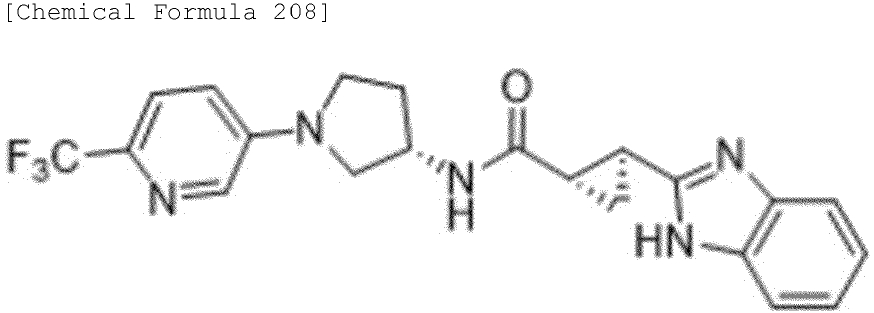

According to a technique similar to Example 3, a crude form of (S)-1-(6-(trifluoromethyl)pyridin-3-yl)pyrrolidin-3-amine (37 mg) was synthesized from tert-butyl (S)-(1-(6-(trifluoromethyl)pyridin-3-yl)pyrrolidin-3-yl)carbamate (59 mg, 0.18 mmol) synthesized in Reference Example 37, and the title compound (white powder, 48 mg, 73%) was obtained using (1S,2S)-2-(1H-benzo[d]imidazol-2-yl)cyclopropane-1-carboxylic acid (32 mg, 0.16 mmol).
¹H NMR(DMSO-d₆, 400MHz) : δ=1.4-1.5(m, 2H), 1.9-2.0 (m, 1H), 2.1-2.3(m,2H), 2.4-2.5(m, 1H), 3.18(dd, 1H, J=4,10Hz), 3.3-3.6(m,2H), 3.60(dd, 1H, J=6,10Hz), 4.4-4.6(m, 1H), 7.00(d, 1H, J=3,9Hz), 7.0-7.2(m, 2H), 7.3-7.5(m,2H), 7.58(d, 1H, J=9Hz), 8.02(d, 1H, J=3Hz), 8.64 (d, 1H, J=7Hz), 12.34(s, 1H).
MS : 416.18 [M+H]⁺

### Example 56

### (1S,2S)-2-(1H-benzo[d]imidazol-2-yl)-N-((R)-1-(5-(trifluoromethyl)pyrimidin-2-yl)pyrrolidin-3-yl)cyclopropane-1-carboxyamide

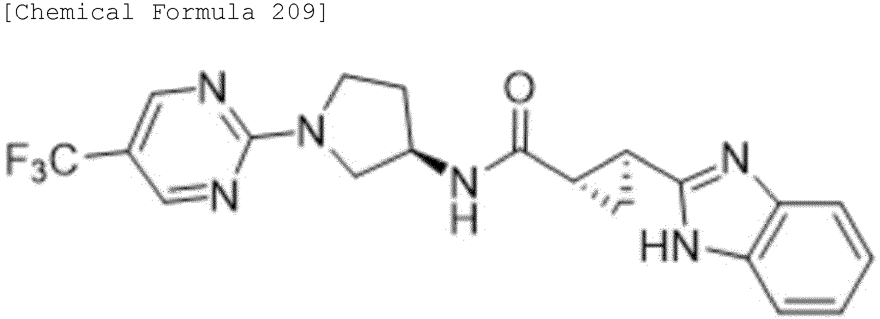

According to a technique similar to Example 1, the title compound (white powder, 28 mg, 68%) was obtained using (R)-1-(5-(trifluoromethyl)pyrimidin-2-yl)pyrrolidin-3-amine (45 mg, 0.20 mmol) synthesized in Reference Example 38-2 and (1S,2S)-2-(1H-benzo[d]imidazol-2-yl)cyclopropane-1-carboxylic acid (20 mg, 0.10 mmol).
¹H NMR(DMSO-d₆, 400MHz) : δ=1.4-1.5(m, 2H), 1.8-2.0 (m, 1H), 2.1-2.3(m, 2H), 2.4-2.5(m, 1H), 3.49(dd, 1H, J=4,8Hz), 3.6-3.7(m, 2H), 3.75(dd, 1H, J=6,12Hz), 4.3-4.5(m, 1H), 7.0-7.2(m, 2H), 7.3-7.5(m, 2H), 8.64(d, 1H, J=6Hz), 8.71(s, 2H), 12.39(s,1H).
MS:417.20[M+H]⁺

### Example 57

### (1S,2S)-2-(1H-benzo[d]imidazol-2-yl)-N-((R)-1-(4-fluoro-3-(trifluoromethyl)phenyl)pyrrolidin-3-yl)cyclopropane-1-carboxyamide

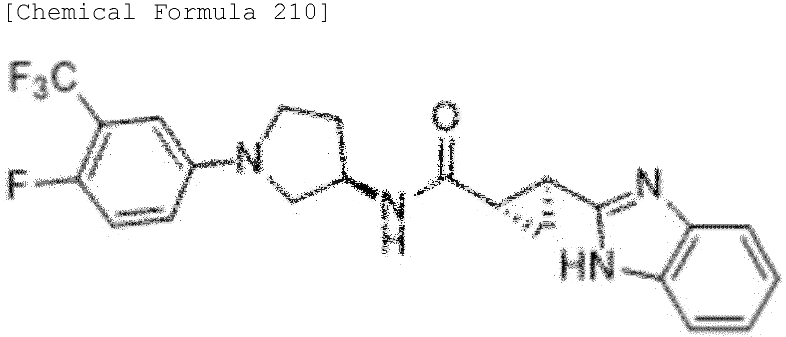

According to a technique similar to Example 3, a crude form of (R)-1-(4-fluoro-3-(trifluoromethyl)-phenyl)pyrrolidin-3-amine (80 mg) was synthesized from tert-butyl (R)-(1-(4-fluoro-3-(trifluoromethyl)-phenyl)pyrrolidin-3-yl)carbamate (112 mg, 0.32 mmol) synthesized in Reference Example 39, and the title compound (white powder, 35 mg, 77%) was obtained using (1S,2S)-2-(1H-benzo[d]imidazol-2-yl)cyclopropane-1-carboxylic acid (21 mg, 0.11 mmol).
¹H NMR(CDCl₃, 400MHz) :δ=1.6-1.8 (m, 2H), 1.9-2.1(m,1H), 2.2-2.4(m, 2H) ,2.6-2.7(m, 1H), 3.23(dd, 1H, J=4,10Hz), 3.30(dt, 1H, J=5,9Hz), 3.4-3.5(m, 1H), 3.59(dd, 1H, J=6,10Hz), 4.6-4.7(m, 1H), 6.51(d, 1H, J=7Hz), 6.6-6.7(m, 2H), 7.05(t, 1H, J=9Hz), 7.2-7.3(m, 2H), 7.4-7.5(m, 2H). The 1H content is not observable.
MS:433.18[M+H]⁺

### Example 58

### (1S,2S)-N-((R)-1-(4-fluoro-3-(trifluoromethyl)-phenyl)pyrrolidin-3-yl)-2-(quinazolin-2-yl)cyclopropane-1-carboxyamide

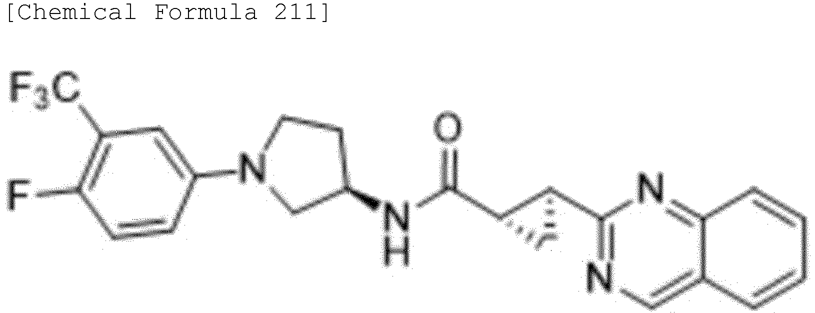

According to a technique similar to Example 3, a crude form of (R)-1-(4-fluoro-3-(trifluoromethyl)-phenyl)pyrrolidin-3-amine (80 mg) was synthesized from tert-butyl (R)-(1-(4-fluoro-3-(trifluoromethyl)-phenyl)pyrrolidin-3-yl)carbamate (112 mg, 0.32 mmol) synthesized in Reference Example 39, and the title compound (white powder, 23 mg, 56%) was obtained using (1S,2S)-2-(quinazolin-2-yl)cyclopropane-1-carboxylic acid (20 mg, 0.09 mmol) synthesized in Reference Example 40-3.
¹H NMR (CDCl₃, 400MHz) : δ=1.69 (ddd, 1H, J=4,6, 8Hz), 1.7-1.8(m, 1H), 1.9-2.1(m, 1H), 2.1-2.4(m, 2H), 2.9-3.0(m, 1H), 3.22(dd, 1H, J=4, 10Hz), 3.31(dt, 1H, J=5, 9Hz), 3.4-3.5(m,1H), 3.59(dd, 1H, J=6, 10Hz), 4.6-4.8(m, 1H), 5.88(d, 1H, J=7Hz), 6.6-6.7(m, 2H), 7.06(t, 1H, J=9Hz), 7.5-7.6(m, 1H), 7.8-8.0(m, 3H), 9.25(s, 1H).
MS : 445.19[M+H]⁺

### Example 59

### (1S,2S)-2-(1H-benzo[d]imidazol-2-yl)-N-((R)-1-(3-(methylsulfonyl)phenyl)pyrrolidin-3-yl)cyclopropane-1-carboxyamide

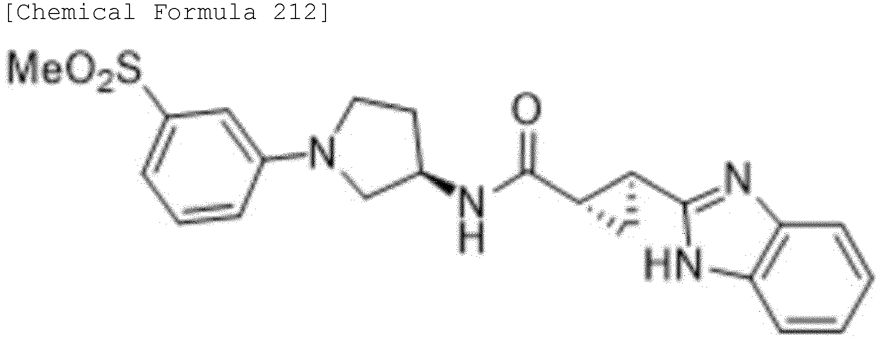

According to a technique similar to Example 1, the title compound (pale yellow powder, 7 mg, 65%) was obtained using (R)-1-(3-(methylsulfonyl)phenyl)pyrrolidin-3-amine (6 mg, 0.02 mmol) synthesized in Reference Example 41-2 and (lS,2S)-2-(1H-benzo[d]imidazol-2-yl)cyclopropane-1-carboxylic acid (5 mg, 0.02 mmol).
¹H NMR (CDCl₃, 400MHz) : δ=1.5-1.8 (m, 2H), 2.0-2.1 (m, 1H), 2.2-2.4(m, 2H), 2.6-2.7(m, 1H), 3.04(s, 3H), 3.27(dd, 1H, J=4,10Hz), 3.3-3.5(m, 2H), 3.62(dd, 1H, J=6,10Hz), 4.5-4.7(m, 1H), 6.53(d, 1H, J=7Hz), 6.72(dd, 1H, J=2, 8Hz), 7.00(t, 1H, J=2Hz), 7.1-7.3(m, 3H), 7.36(t, 1H, J=8Hz), 7.4-7.6(m,2H). The 1H content is not observable.
MS : 425.19[M+H]⁺

### Example 60

### (1S,2S)-2-(1H-benzo[d]imidazol-2-yl)-N-((R)-1-(3-cyanophenyl)pyrrolidin-3-yl)cyclopropane-1-carboxyamide

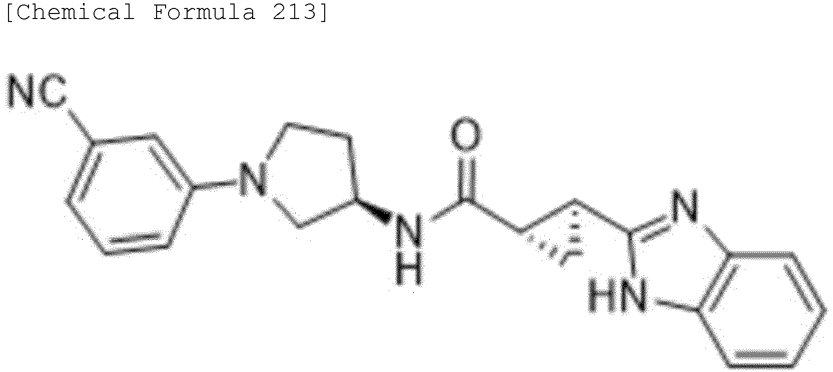

According to a technique similar to Example 1, the title compound (pale yellow powder, 9 mg, 96%) was obtained using (R)-3-(3-aminopyrrolidin-1-yl)benzonitrile (5 mg, 0.02 mmol) synthesized in Reference Example 42-2 and (1S,2S)-2-(1H-benzo[d]imidazol-2-yl)cyclopropane-1-carboxylic acid (5 mg, 0.02 mmol).
¹H NMR (CDCl₃, 400MHz) : δ=1.6-1.8 (m, 2H), 1.9-2.1(m, 1H), 2.2-2.4(m, 2H), 2.6-2.8(m, 1H), 3.2-3.5(m, 2H), 3.22(dd, 1H, J=4,10Hz), 3.60(dd, 1H, J=6,10Hz), 4.5-4.7(m, 1H), 6.61(d, 1H, J=7Hz), 6.7-6.8(m, 2H), 6.96(d, 1H, J=7Hz), 7.1-7.3(m, 3H), 7.4-7.6(m, 2H). The 1H content is not observable.
MS : 372.21[M+H]⁺

### Example 61

### Trans-2-(5-cyano-1H-benzo[d]imidazol-2-yl)-N-((R)-1-(6-(trifluoromethyl)pyridin-3-yl)pyrrolidin-3-yl)cyclopropane-1-carboxyamide

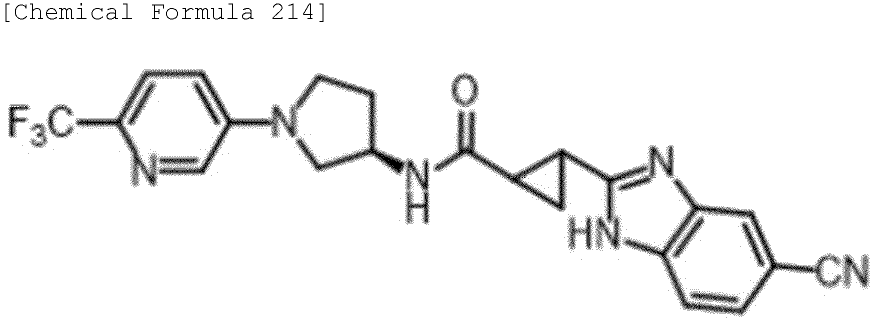

According to a technique similar to Example 1, diastereomer A of the title compound (upper spot in TLC (methanol/ethyl acetate = 1/9), white powder, 22 mg, 37%) and diastereomer B of the title compound (lower spot in TLC (methanol/ethyl acetate = 1/9), white powder, 19 mg, 32%) were obtained using (R)-1-(6-(trifluoromethyl)pyridin-3-yl)pyrrolidin-3-amine (31 mg, 0.14 mmol) synthesized in Reference Example 1-2 and trans-2-(5-cyano-1H-benzo[d]imidazol-2-yl)cyclopropane-1-carboxylic acid (31 mg, 0.14 mmol) synthesized in Reference Example 43-3.

### Diastereomer A

¹H NMR (CDCl₃, 400MHz) : δ=1.6-1.8 (m, 2H), 2.0-2.1 (m, 1H), 2.3-2.5(m,2H), 2.6-2.7(m, 1H), 3.31(dd, 1H, J=4,10Hz), 3.4-3.6(m, 2H),3.72(dd, 1H, J=6,10Hz), 4.6-4.8(m, 1H), 6.16(d, 1H, J=7Hz), 6.85(dd, 1H, J=3,9Hz), 7.4-7.6(m, 3H), 7.8-7.9(m, 1H), 8.03(d, 1H, J=3Hz), 9.78(br s, 1H).
MS : 439.28[M-H]⁻

### Diastereomer B

¹H NMR (CDCl₃, 400MHz) : δ=1.7-1.9(m, 2H), 2.0-2.5(m, 3H), 2.6-2.7(m, 1H), 3.23(dd, 1H, J=4,10Hz), 3.3-3.6(m, 2H), 3.58(dd, 1H, J=6,10Hz), 4.6-4. 7(m, 1H), 6.35(d, 1H, J=7Hz), 6.77(dd,1H, J=3,9Hz), 7.4-8.0(m, 4H), 7.94(d, 1H, J=3Hz), 10.05(br s, 1H).
MS:439.28[M-H]⁻

### Example 62

### (1S,2S)-2-(1H-benzo[d]imidazol-2-yl)-N-((R)-1-(2-(trifluoromethyl)pyridin-4-yl)pyrrolidin-3-yl)cyclopropane-1-carboxyamide

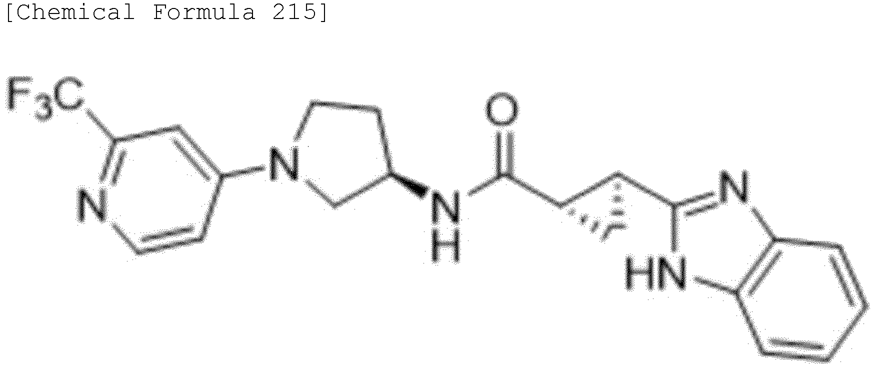

According to a technique similar to Example 3, a crude form of (R)-1-(2-(trifluoromethyl)pyridin-4-yl)pyrrolidin-3-amine (30 mg) was synthesized from tert-butyl (R)-(1-(2-(trifluoromethyl)pyridin-4-yl)pyrrolidin-3-yl)carbamate synthesized in Reference Example 44, and the title compound (white powder, 31 mg, 76%) was obtained using (1S,2S)-2-(1H-benzo[d]imidazol-2-yl)cyclopropane-1-carboxylic acid (20 mg, 0.10 mmol).
¹H NMR (CDCl₃, 400MHz) : δ=1.6-1.8 (m, 2H), 2.0-2.2 (m, 1H), 2.2-2.5(m, 2H), 2.5-2.7(m, 1H), 3.30(dd, 1H, J=4,10Hz), 3.4-3.6(m, 2H), 3.73(dd, 1H, J=6,11Hz), 4.6-4.7(m, 1H), 6.19(d, 1H, J=7Hz), 6.49(dd, 1H, J=2,9Hz), 6.74(d, 1H, J=2Hz), 7.2-7.3(m, 2H), 7.4-7.5(m, 2H), 8.31(d, 1H, J=6Hz). The 1H is not observable.
MS : 416.17[M+H]⁺

### Example 63

### Trans-2-(1H-benzo[d]imidazol-2-yl)-N-((R)-1-(6-(trifluoromethyl)pyridin-3-yl)piperidin-3-yl)cyclopropane-1-carboxyamide

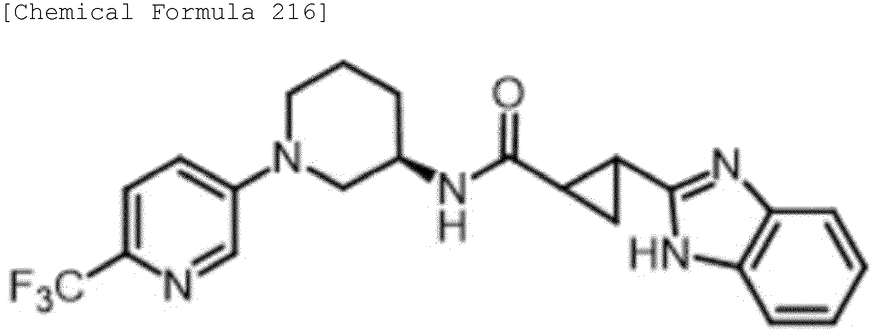

According to a technique similar to Example 1, diastereomer A of the title compound (upper spot in TLC (ethyl acetate), white powder, 14 mg, 16%) and diastereomer B of the title compound (lower spot in TLC (ethyl acetate), white powder, 5 mg, 6%) were obtained using (R)-1-(6-(trifluoromethyl)pyridin-3-yl)piperidin-3-amine (50 mg, 0.20 mmol) synthesized in Reference Example 45-2 and trans-2-(1H-benzo[d]imidazol-2-yl)cyclopropane-1-carboxylic acid (41 mg, 0.20 mmol).

### Diastereomer A

¹H NMR(CDCl₃, 400MHz) : δ=1.6-2.0 (m, 6H), 2.2-2.4 (m, 1H), 2.5-2.7(m, 1H), 3.08(dd, 1H, J=7, 12Hz), 3.1-3.4(m,2H), 3.55(dd, 1H, J=3,12Hz), 4.1-4.3(m, 1H), 6.14(d, 1H, J=8Hz), 7.2-7.3(m, 2H), 7.3-7.4(m, 1H), 7.4-7.6(m, 2H), 8.35(s, 1H), 8.49(d, 1H, J=3Hz). The 1H content is not observable.
MS : 430.17[M+H]⁺

### Diastereomer B

¹H NMR (CDCl₃, 400MHz) : δ=1.6-2.0 (m, 6H), 2.2-2.3 (m, 1H), 2.6-2.7(m, 1H), 3.1-3.3(m, 4H), 4.1-4.3(m, 1H), 6.46(d, 1H, J=7Hz), 7.1-7.3(m, 3H), 7.4-7.6(m, 2H), 8.23(s, 1H), 8.28(d, 1H, J=3Hz).

The 1H content is not observable.
MS:430.18[M+H]⁺

### Example 64

### (1S,2S)-2-(1H-benzo[d]imidazol-2-yl)-N-((R)-1-(4-(trifluoromethyl)thiazol-2-yl)pyrrolidin-3-yl)cyclopropane-1-carboxyamide

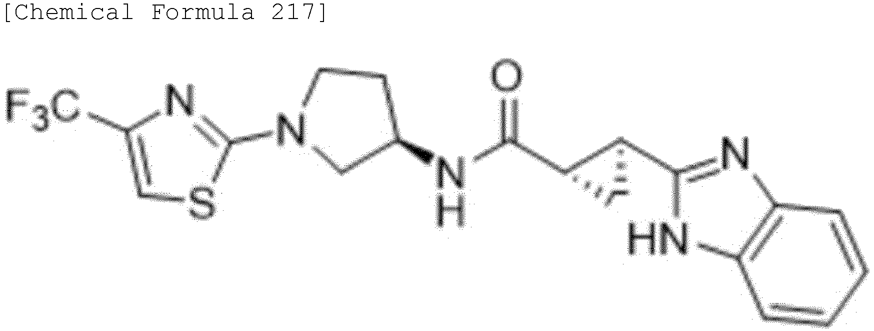

According to a technique similar to Example 1, the title compound (white powder, 20 mg, 47%) was obtained using (R)-1-(4-(trifluoromethyl)thiazol-2-yl)pyrrolidin-3-amine (24 mg, 0.10 mmol) synthesized in Reference Example 46-3 and (1S,2S)-2-(1H-benzo[d]imidazol-2-yl)cyclopropane-1-carboxylic acid (20 mg, 0.10 mmol).
¹H NMR (CDCl₃, 400MHz) : δ=1.5-1.8 (m, 2H), 2.0-2.1(m, 1H), 2.2-2.4(m, 2H), 2.5-2.7(m, 1H),3.43(dd, 1H, J=4,10Hz), 3.5-3.7(m, 2H), 3.8 0(dd, 1H, J=6,11Hz), 4.6-4.7(m, 1H), 6.29(d, 1H, J=7Hz), 6.94(s, 1H), 7.2-7.3(m, 2H), 7.4-7.6(m, 2H).

The 1H content is not observable.
MS : 422.13[M+H]⁺

### Example 65

### (1S,2S)-2-(1H-benzo[d]imidazol-2-yl)-N-((R)-1-(4-(trifluoromethyl)phenyl)piperidin-3-yl)cyclopropane-1-carboxyamide

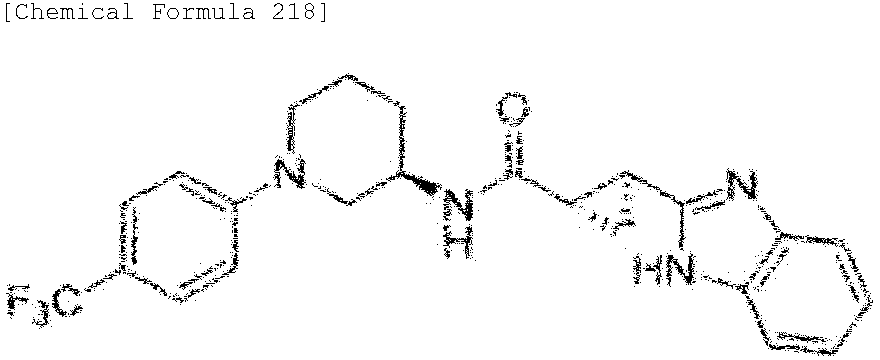

According to a technique similar to Example 1, the title compound (white powder, 30 mg, 70%) was obtained using (R)-1-(4-(trifluoromethyl)phenyl)piperidin-3-amine (22 mg, 0.10 mmol) synthesized in Reference Example 47-2 and (1S,2S)-2-(1H-benzo[d]imidazol-2-yl)cyclopropane-1-carboxylic acid (20 mg, 0.10 mmol).
¹H NMR(CDCl₃, 400MHz) : δ=1.6-1.9(m, 6H), 2.2-2.4(m, 1H), 2.6-2.7(m, 1H), 3.1-3.4(m, 2H), 3.10(dd, 1H, J=7,12Hz), 3.57(dd, 1H, J=3, 12Hz), 4.1-4.3(m, 1H), 6.16(d, 1H, J=8Hz), 6.97(d, 2H, J=9Hz), 7.1-7.3(m, 2H), 7.4-7.6(m,2H),7.49(d,2H,J=9Hz),9.60(br s, 1H).
MS : 429.19 [M+H]⁺

### Example 66

### Trans-2-(1H-indol-3-yl)-N-((R)-1-(6-(trifluoromethyl)pyridin-3-yl)pyrrolidin-3-yl)cyclopropane-1-carboxyamide

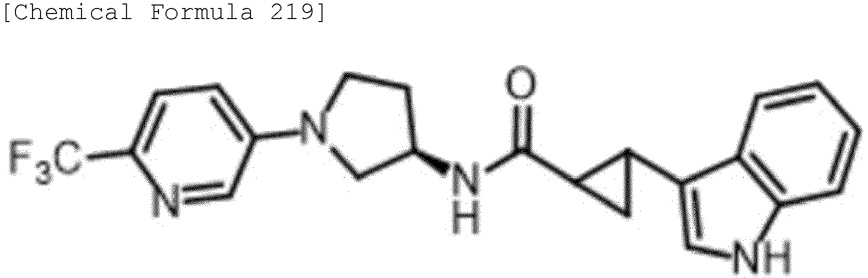

According to a technique similar to Example 3, diastereomer A of the title compound (Rf value in TLC (ethyl acetate) = 0.9, pale yellow powder, 6 mg, 19%) was obtained using (R)-1-(6-(trifluoromethyl)pyridin-3-yl)pyrrolidin-3-amine (17 mg, 0.07 mmol) synthesized in Reference Example 1-2 and trans-2-(1H-indol-3-yl)cyclopropane-1-carboxylic acid (15 mg, 0.07 mmol).

### Diastereomer A

¹H NMR(CDCl₃, 400MHz) : δ=1.3-1.4(m, 1H), 1.5-1.7(m, 2H), 2.0-2.2(m, 1H),2.3-2.5(m, 1H),2.6-2.7(m, 1H), 3.28 (dd, 1H, J=4, 10Hz), 3.4-3.6(m, 2H), 3.72(dd, 1H, J=6, 10Hz), 4.7-4.8(m, 1H), 5.85(d, 1H, J=7Hz), 6.84(dd, 1H, J=3,9Hz), 6.94(dd, 1H, J=1,2Hz), 7.14(dt, 1H, J=1,7Hz), 7.22 (dt, 1H, J=1, 7Hz), 7.36 (d, 1H, J=8Hz), 7.49(d, 1H, J=9Hz), 7.65(d, 1H, J=8Hz), 7.97(br s, 1H), 8.02(d, 1H, J=3Hz).
MS:415.18[M+H]⁺

### Example 67

### (1S,2S)-2-(1H-benzo[d]imidazol-2-yl)-N-((R)-1-(6-methoxy-5-(trifluoromethyl)pyridin-3-yl)pyrrolidin-3-yl)cyclopropane-1-carboxyamide

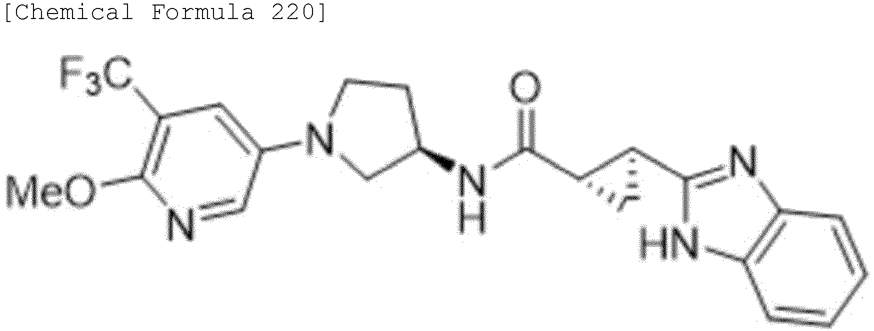

According to a technique similar to Example 1, the title compound (white powder, 21 mg, 65%) was obtained using (R)-1-(6-methoxy-5-(trifluoromethyl)pyridin-3-yl)pyrrolidin-3-amine (18 mg, 0.07 mmol) synthesized in Reference Example 48-2 and 1(1S,2S)-2-(1H-benzo[d]imidazol-2-yl)cyclopropane-1-carboxylic acid (14 mg, 0.07 mmol).
¹H NMR (CDCl₃, 400MHz) : δ=1.6-1.8 (m, 2H), 1.9-2.1(m, 1H), 2.2-2.4(m, 2H), 2.6-2.7(m, 1H), 3.2-3.5(m, 3H), 3.57(dd, 1H, J=6,10Hz), 3.96(s, 3H), 4.6-4.8(m, 1H), 6.1-6.3(m, 1H), 7.14(d,1H, J=3Hz),7.2-7.3(m,2H),7.3-7.7(m,2H), 7.65(d, 1H, J=3Hz),9.39(br s, 1H).
MS:444.17[M-H]⁻

### Example 68

### 3(1S,2S)-2-(1H-benzo[d]imidazol-2-yl)-N-((R)-1-(4-(tert-butyl)thiazol-2-yl)pyrrolidin-3-yl)cyclopropane-1-carboxyamide

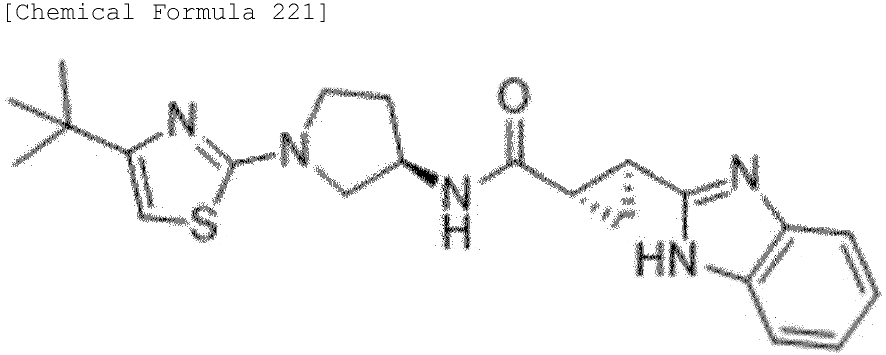

According to a technique similar to Example 1, the title compound (white powder, 23 mg, 57%) was obtained using (R)-1-(4-(tert-butyl)thiazol-2-yl)pyrrolidin-3-amine (22 mg, 0.10 mmol) synthesized in Reference Example 49-2 and (1S,2S)-2-(1H-benzo[d]imidazol-2-yl)cyclopropane-1-carboxylic acid (20 mg, 0.10 mmol).
¹H NMR (CDCl₃, 400MHz) : δ=1.27 (s, 9H), 1.6-1.8 (m, 2H), 2.0-2.2(m, 1H),2.2-2.5(m, 2H), 2.5-2.7(m, 1H),3.4-3.6(m, 2H), 3.41(dd, 1H, J=4,10Hz), 3. 77(dd, 1H, J=6,11Hz), 4.5-4.7(m,1H), 6.09(s, 1H), 6.1-6.3(m, 1H), 7.1-7.3(m, 2H), 7.3-7. 7(m, 2H), 9.68(br s, 1H).
MS : 410.21[M+H]⁺

### Example 69

### (1S,2S)-2-(1H-benzo[d]imidazol-2-yl)-N-((R)-1-(6-(trifluoromethyl)pyridazin-3-yl)pyrrolidin-3-yl)cyclopropane-1-carboxyamide

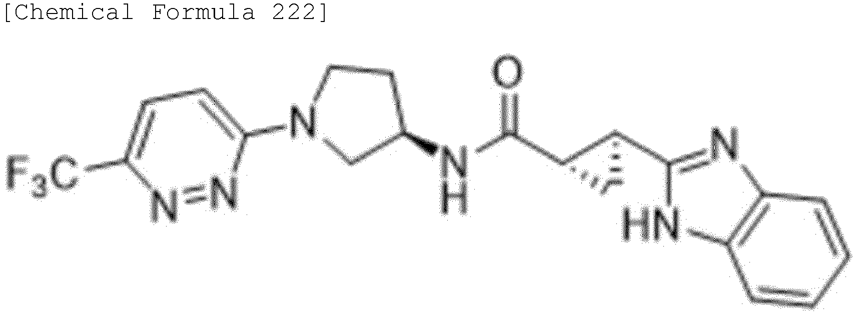

According to a technique similar to Example 3, a crude form of (R)-1-(6-(trifluoromethyl)pyridazin-3-yl)pyrrolidin-3-amine (91 mg) was synthesized from tert-butyl (R)-(1-(6-(trifluoromethyl)pyridazin-3-yl)pyrrolidin-3-yl)carbamate (90 mg, 0.271 mmol) synthesized in Reference Example 50, and the title compound (white powder, 67 mg, 60%) was obtained using (1S,2S)-2-(1H-benzo[d]imidazol-2-yl)cyclopropane-1-carboxylic acid (110 mg, 0.542 mmol).
¹H NMR(DMSO-d₆, 400MHz) : δ=1.4-1.5(m, 2H), 1.9-2.0 (m, 1H), 2.1-2.3(m, 2H), 2.4-2.5(m, 1H), 3.3-3.4(m, 2H), 3.5-3.8(m, 2H), 4.4-4.5(m, 1H), 7.04(d,1H, J=10Hz), 7.10(dd, 2H, J=2,6Hz), 7.44(br s, 2H), 7.77(d, 1H, J=10Hz), 8.66(d, 1H, J=7Hz), 12.4(br s, 1H).
MS : 417.20[M+H]⁺

### Example 70

### (1S,2S)-2-(1H-benzo[d]imidazol-2-yl)-N-((R)-1-(2-(trifluoromethyl)pyrimidin-5-yl)pyrrolidin-3-yl)cyclopropane-1-carboxyamide

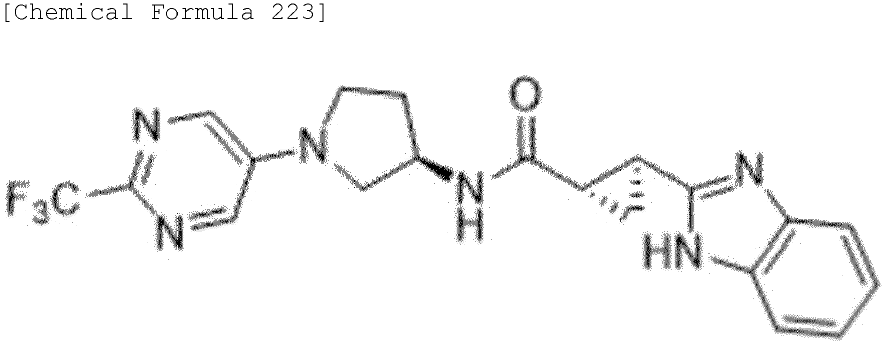

According to a technique similar to Example 3, a crude form of (R)-1-(2-(trifluoromethyl)pyrimidin-5-yl)pyrrolidin-3-amine (101 mg) was synthesized from tert-butyl (R)-(1-(2-(trifluoromethyl)pyrimidin-5-yl)pyrrolidin-3-yl)carbamate (129 mg, 0.388 mmol) synthesized in Reference Example 51, and the title compound (white powder, 145 mg, 90%) was obtained using (1S,2S)-2-(1H-benzo[d]imidazol-2-yl)cyclopropane-1-carboxylic acid (157 mg, 0.776 mmol).
¹H NMR(DMSO-d₆, 400MHz) : δ=1.4-1.5(m, 2H), 1.9-2.0 (m, 1H), 2.1-2.3(m, 2H), 2.4-2.5(m, 1H), 3.26(dd, 1H, J=4,11Hz), 3.3-3.5(m, 2H), 3.64(dd, 1H, J=6, 11Hz), 4.4-4.5(m, 1H), 7.0-7.2(m, 2H), 7.3-7.5(m, 2H), 8.25(s, 2H), 8.64(d, 1H, J=7Hz), 12.4(br s, 1H).
MS : 415.27[M-H]⁻

### Example 71

### (1S,2S)-2-(1H-benzo[d]imidazol-2-yl)-N-((R)-1-(5-(trifluoromethyl)pyridin-3-yl)pyrrolidin-3-yl)cyclopropane-1-carboxyamide

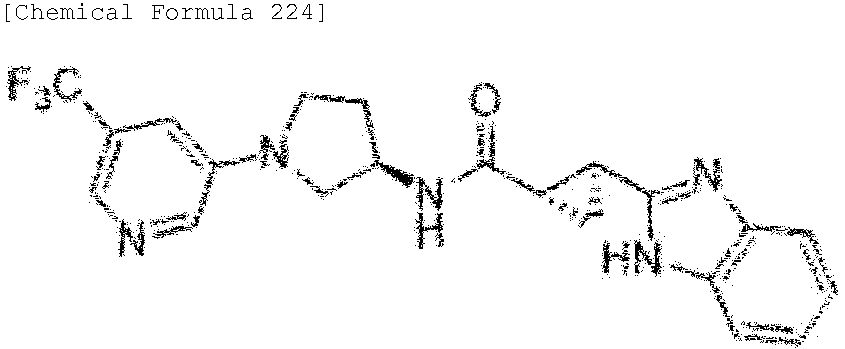

According to a technique similar to Example 3, a crude form of (R)-1-(5-(trifluoromethyl)pyridin-3-yl)pyrrolidin-3-amine (79 mg) was synthesized from tert-butyl (R)-(1-(5-(trifluoromethyl)pyridin-3-yl)pyrrolidin-3-yl)carbamate (102 mg, 0.308 mmol) synthesized in Reference Example 1-1, and the title compound (white powder, 73 mg, 57%) was obtained using (12S,2S)-2-(1H-benzo[d]imidazol-2-yl)cyclopropane-1-carboxylic acid (125 mg, 0.616 mmol).
¹H NMR(CDCl₃, 400MHz) : δ=1.5-1.8 (m, 3H), 2.0-2.1 (m, 1H), 2.3-2.4(m, 2H), 2.6-2.7(m, 1H), 3.28(dd, 1H, J=4,10Hz), 3.3-3.6(m, 2H), 3.67(dd, 1H, J=6,10Hz), 4.6-4.8(m, 1H), 6.66(d, 1H, J=7Hz), 6.92(s, 1H), 7.1-7.3(m, 2H), 7.49(br s, 2H), 8.09(d, 1H, J=3Hz), 8.20(s, 1H).
MS : 16.18[M+H]⁺

### Example 72

### (1S,2S)-2-(1H-benzo[d]imidazol-2-yl)-N-((R)-1-(6-fluorobenzo[d]thiazol-2-yl)pyrrolidin-3-yl)cyclopropane-1-carboxyamide

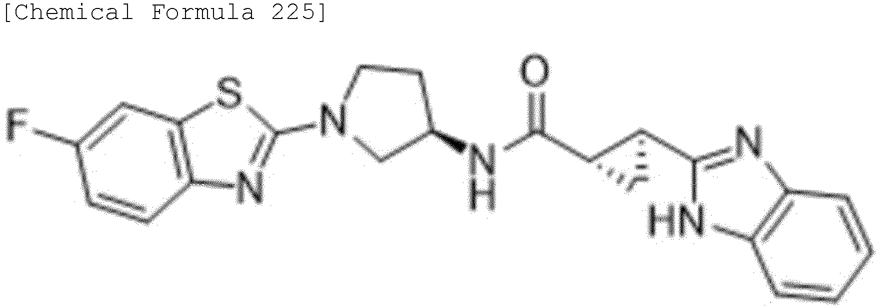

According to a technique similar to Example 3, a crude form of (R)-1-(6-fluorobenzo[d]thiazol-2-yl)pyrrolidin-3-amine (93 mg) was synthesized from tert-butyl (R)-(1-(6-fluorobenzo[d]thiazol-2-yl)pyrrolidin-3-yl)carbamate (85 mg, 0.253 mmol) synthesized in Reference Example 52, and the title compound (white powder, 50 mg, 47%) was obtained using (1S,2S)-2-(1H-benzo[d]imidazol-2-yl)cyclopropane-1-carboxylic acid (90 mg, 0.380 mmol).
¹H NMR(DMSO-d₆, 400MHz) : δ=1.4-1.5(m, 2H), 1.9-2.0 (m, 1H), 2.2-2.3(m, 2H), 2.4-2.5(m, 1H), 3.3-3.4(m, 1H), 3.5-3.7(m, 2H), 3.73(dd, 1H, J=6,11Hz), 4.4-4.5(m, 1H), 7.0-7.2(m, 3H), 7.3-7.5(m,3H), 7.71(dd, 1H, J=3,9Hz), 8.70(d, 1H, J=6Hz), 12.4(br s, 1H).
MS : 422.14[M+H]⁺

### Example 73

### (1S,2S)-2-(1-cyclopropyl-1H-benzo[d]imidazol-2-yl)-N-((R)-1-(6-(trifluoromethyl)pyridin-3-yl)pyrrolidin-3-yl)cyclopropane-1-carboxyamide

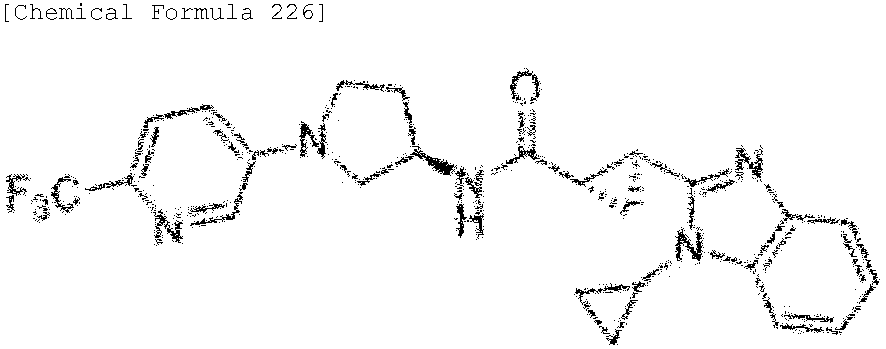

According to a technique similar to Example 1, the title compound (white powder, 15 mg, 35%) was obtained using (R)-1-(6-(trifluoromethyl)pyridin-3-yl)pyrrolidin-3-amine (21 mg, 0.090 mmol) synthesized in Reference Example 32-2 and (1S,2S)-2-(1-cyclopropyl-1H-benzo[d]imidazol-2-yl)cyclopropane-1-carboxylic acid (33 mg, 0.135 mmol) synthesized in Reference Example 54-2.
¹H NMR (CD₃OD, 400MHz) :δ=1.0-1.2 (m, 2H), 1.2-1.4(m, 2H), 1.6-1.7(m,2H), 2.0-2.3(m, 2H), 2.3-2.5(m, 1H), 2.8-3.0(m, 1H), 3.3-3.6(m, 4H), 3.71(dd, 1H, J=6,11Hz), 4.5-4.7(m, 1H), 7.07(dd, 1H, J=3,9Hz), 7.1-7.3(m, 2H), 7.50(d, 1H, J=7Hz), 7.5-7.6(m, 2H), 7.98(d, 1H, J=3Hz). The 1H content is not observable.
MS : 456.20[M+H]⁺

### Example 74

### (1S,2S)-2-(1H-benzo[d]imidazol-2-yl)-N-((R)-1-(6-fluoroquinolin-2-yl)pyrrolidin-3-yl)cyclopropane-1-carboxyamide

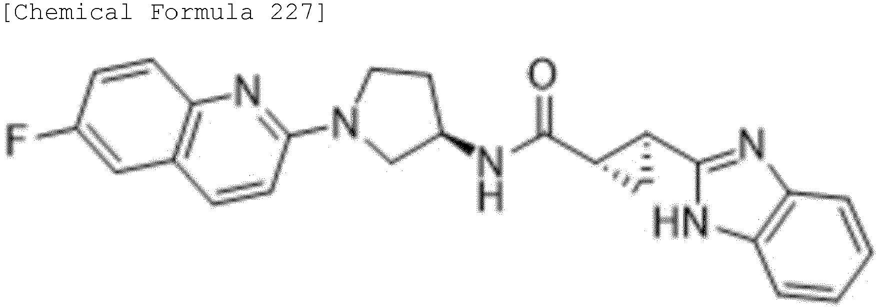

According to a technique similar to Example 3, a crude form of (R)-1-(6-fluoroquinolin-2-yl)pyrrolidin-3-amine (81 mg) was synthesized from tert-butyl (R)-(1-(6-fluoroquinolin-2-yl)pyrrolidin-3-yl)carbamate (39 mg, 0.118 mmol) synthesized in Reference Example 53, and the title compound (pale yellow powder, 10 mg, 21%) was obtained using (1S,2S)-2-(1H-benzo[d]imidazol-2-yl)cyclopropane-1-carboxylic acid (42 mg, 0.177 mmol).
¹H NMR (DMSO-d₆, 400MHz) : δ=1.4-1.5 (m, 2H) , 1.8-2.0 (m, 1H), 2.1-2.5 (m, 3H), 3.4-3.5 (m, 1H), 3.5-3.7 (m, 2H), 3. 76 (dd, 1H, J=6, 11Hz), 4.3-4.5 (m, 1H), 6. 95 (d, 1H, J=9Hz), 7.0-7.2 (m, 2H), 7.3-7.6 (m, 5H), 8.01 (d, 1H, J=9Hz), 8.64 (d, 1H, J=7Hz),12.4(br s, 1H).
MS:416.18[M+H]⁺

### Example 75

### (1S,2S)-2-(1-cyclopropyl-1H-benzo[d]imidazol-2-yl)-N-((R)-1-(3-(trifluoromethyl)phenyl)pyrrolidin-3-yl)cyclopropane-1-carboxyamide

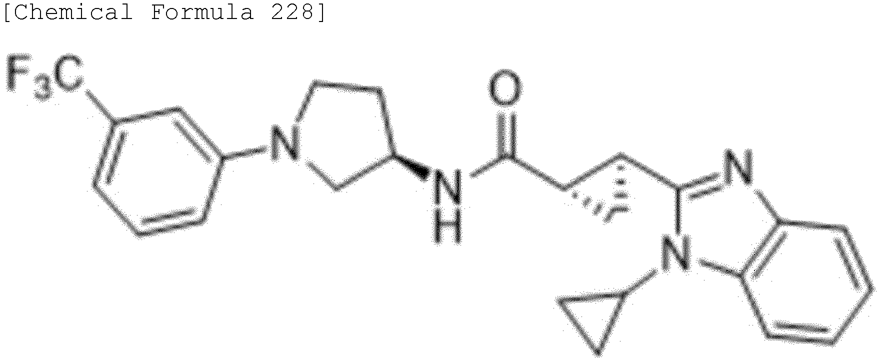

According to a technique similar to Example 3, a crude form of (R)-1-(3-(trifluoromethyl)phenyl)pyrrolidin-3-amine (99 mg) was synthesized from tert-butyl (R)-(1-(3-(trifluoromethyl)phenyl)pyrrolidin-3-yl)carbamate (105 mg, 0.319 mmol) synthesized in Reference Example 77, and the title compound (white powder, 27 mg, 28%) was obtained using (1S,2S)-2-(1-cyclopropyl-1H-benzo[d]imidazol-2-yl)cyclopropane-1-carboxylic acid (52 mg, 0.213 mmol) synthesized in Reference Example 54-2.
¹H NMR (DMSO-d₆, 400MHz) : δ=0.9-1.3 (m, 4H), 1.4-1.6 (m, 2H), 1.9-2.0 (m, 1H), 2.1-2.3 (m, 2H), 2.6-2.8 (m, 1H), 3.1-3.2 (m, 1H), 3.3-3.5 (m, 3H), 3.56 (dd, 1H, J=6, 10Hz), 4.4-4.5 (m, 1H), 6.72 (s, 1H), 6.80 (dd, 1H, J=2, 8Hz), 6.89 (d, 1H, J=8Hz), 7.1-7.3 (m, 2H), 7.36 (t, 1H, J=8Hz), 7.47 (d, 1H, J=7Hz), 7.52 (d, 1H, J=7Hz), 8. 62 (d, 1H, 7Hz).
MS:455.20 [M+H]⁺

### Example 76

### (1S,2S)-2-(1H-benzo[d]imidazol-2-yl)-N-((R)-1-(5-bromopyridin-3-yl)pyrrolidin-3-yl)cyclopropane-1-carboxyamide

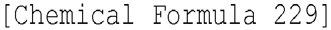

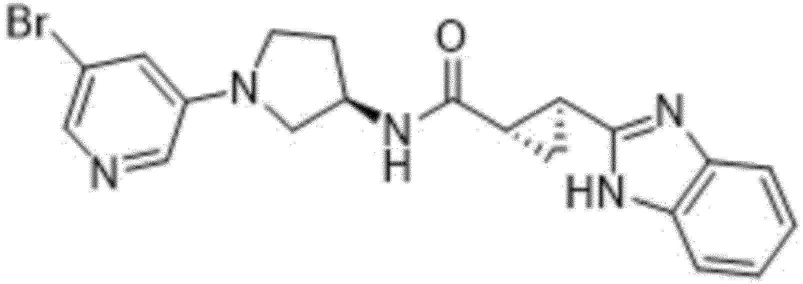

According to a technique similar to Example 3, a crude form of (R)-1-(5-bromopyridin-3-yl)pyrrolidin-3-amine (138 mg) was synthesized from tert-butyl (R)-(1-(5-bromopyridin-3-yl)pyrrolidin-3-yl)carbamate (71 mg, 0.209 mmol) synthesized in Reference Example 55, and the title compound (white powder, 56 mg, 62%) was obtained using (1S,2S)-2-(1H-benzo[d]imidazol-2-yl)cyclopropane-1-carboxylic acid (63 mg, 0.314 mmol).
¹H NMR(DMSO-d₆, 400MHz) : δ=1.4-1.5 (m, 2H), 1.8-2.0 (m, 1H), 2.1-2.5 (m, 3H), 3.14 (dd, 1H, J=4, 10Hz), 3.3-3.5 (m, 2H), 3.53 (dd, 1H, J=6, 10Hz), 4.3-4.5 (m, 1H), 7.0-7.2 (m, 3H), 7.3-7.5 (m, 2H), 7.89 (d, 1H, J=2Hz), 7.91 (d, 1H, J=2Hz), 8.60 (d, 1H, J=7Hz), 12.4 (br s, 1H).
MS:426.09 [M+H]⁺

### Example 77

### (R)-2-(quinolin-6-yl)-N-(1-(5-(trifluoromethyl)pyridin-3-yl)pyrrolidin-3-yl)acetamide

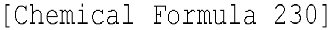

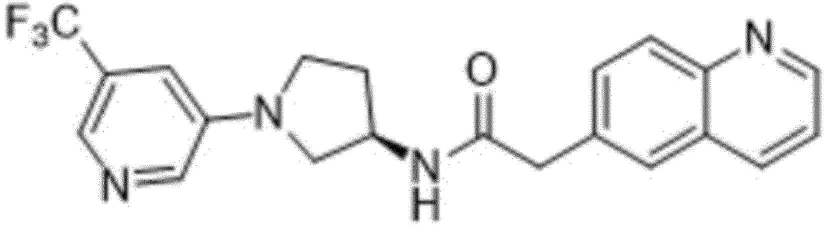

According to a technique similar to Example 1, the title compound (pale yellow amorphous, 88 mg, 100%) was obtained using (R)-1-(5-(trifluoromethyl)pyridin-3-yl)pyrrolidin-3-amine (50 mg, 0.216 mmol) synthesized in Reference Example 1-2 and 6-quinolineacetic acid (49 mg, 0.259 mmol).
¹H NMR (CDCl₃, 400MHz) : δ=1.9-2.1 (m, 1H), 2.2-2.4 (m, 1H), 3.14 (dd, 1H, J=4, 10Hz), 3.2-3.4 (m, 2H), 3.57 (dd, 1H, J=6, 10Hz), 3.78 (s, 2H), 4.6-4.7 (m, 1H), 6.73 (s, 1H), 6.81 (d, 1H, J=7Hz), 7.38 (dd, 1H, J=4, 8Hz), 7.60 (dd, 1H, J=2, 8Hz), 7.69 (s, 1H), 7.85 (br s, 1H), 8.0-8.1 (m, 3H), 8.86 (d, 1H, J=3Hz).
MS:401.19 [M+H]⁺

### Example 78

### (R)-2-(1H-indol-3-yl)-N-(1-(5-(trifluoromethyl)-pyridin-3-yl)pyrrolidin-3-yl)acetamide

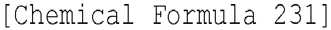

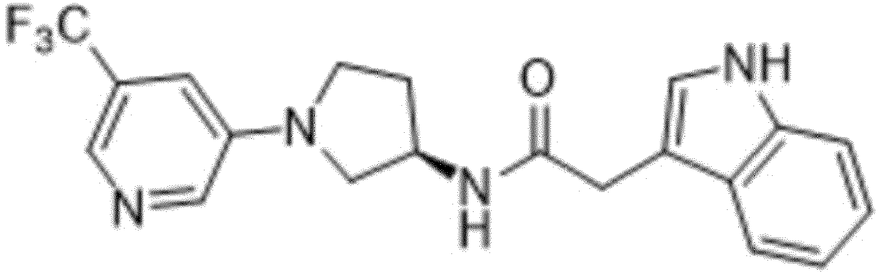

According to a technique similar to Example 1, the title compound (white amorphous, 87 mg, 100%) was obtained using (R)-1-(5-(trifluoromethyl)pyridin-3-yl)pyrrolidin-3-amine (50 mg, 0.216 mmol) synthesized in Reference Example 1-2 and 3-indoleacetic acid (45 mg, 0.259 mmol).
¹H NMR (CDCl₃, 400MHz) : δ=1.7-1.9 (m, 1H), 2.2-2.4 (m, 1H), 3.07 (dd, 1H, J=4, 10Hz), 3.1-3.4 (m,2H), 3.59 (dd, 1H, J=6, 10Hz), 3.76 (s, 2H), 4.5-4.7 (m, 1H), 5.79 (d, 1H, J=6Hz), 6.82 (s, 1H), 7.0-7.3 (m, 3H), 7.39 (d, 1H, J=8Hz), 7.49 (d, 1H, J=7Hz), 8.00 (d, 1H, J=3Hz), 8.18 (s, 2H).
MS:389.19 [M+H]⁺

### Example 79

### (R)-2-(quinolin-7-yl)-N-(1-(5-(trifluoromethyl)pyridin-3-yl)pyrrolidin-3-yl)acetamide hydrochloride

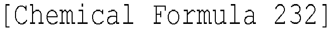

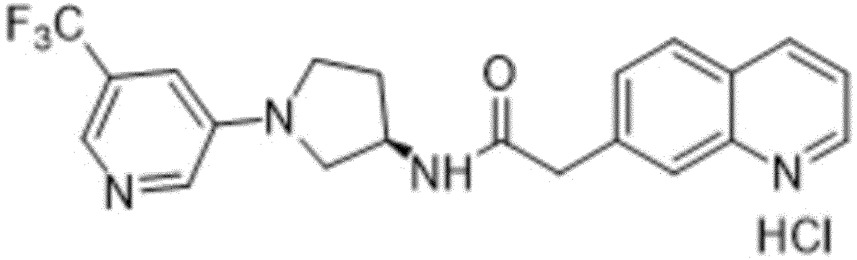

According to a technique similar to Example 1, (R)-2-(quinolin-7-yl)-N-(1-(5-(trifluoromethyl)pyridin-3-yl)pyrrolidin-3-yl)acetamide (colorless oily material, 56 mg) was obtained using (R)-1-(5-(trifluoromethyl)pyridin-3-yl)pyrrolidin-3-amine (99 mg, 0.427 mmol) synthesized in Reference Example 1-2 and 7-quinolineacetic acid (80 mg, 0.427 mmol). (R)-2-(quinolin-7-yl)-N-(1-(5-(trifluoromethyl)pyridin-3-yl)pyrrolidin-3-yl)acetamide (56 mg) thus obtained was dissolved in ethyl acetate (2 mL), a 2 M solution (2 mL) of hydrogen chloride in ethyl acetate was added thereto, the mixture was stirred for a while, subsequently the solvent was distilled off under reduced pressure, and the title compound (yellow powder, 52 mg, 28%) was obtained.
¹H NMR (DMSO-d₆, 400MHz) : δ=1.9-2.1 (m, 1H), 2.1-2.3 (m, 1H), 3.2-3.9 (m, 6H), 4.4-4.5 (m, 1H), 7.2-7.3 (m, 1H), 7.84 (d, 1H, J=8Hz), 7.9-8.1 (m, 1H), 8.1-8.3 (m, 4H), 8.7-8.8 (m, 1H), 9.0-9.1 (m, 1H), 9.24 (d, 1H, J=4Hz). The 1H content is not observable.
MS:401.16[M+H]⁺

### Example 80

### (R)-2-(4-cyclopropylphenyl)-N-(1-(4-(trifluoromethyl)oxazol-2-yl)pyrrolidin-3-yl)acetamide

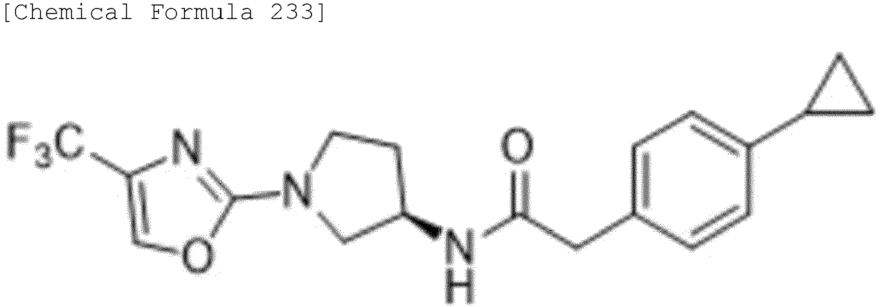

According to a technique similar to Example 27, the title compound (white powder, 61 mg, 92%) was obtained using 2-(4-cyclopropylphenyl)acetic acid (43 mg, 0.176 mmol) synthesized in Reference Example 33-2 and 2-bromo-4-(trifluoromethyl)-1,3-oxazole (38 mg, 0.176 mmol).
¹H NMR (CDCl₃, 400MHz) :δ=0.6-0.8 (m, 2H), 0.9-1.0 (m, 2H), 1.8-2.0 (m, 2H), 2.2-2.3 (m, 1H), 3.28 (dd, 1H, J=5, 11Hz), 3.4-3.6 (m, 4H), 3.77 (dd, 1H, J=6, 11Hz), 4.5-4.6 (m, 1H), 5.44 (d, 1H, J=7Hz), 7.0-7.2 (m, 4H), 7.49 (dd, 1H, J=1, 3Hz).
MS:402.12[M+Na]⁺

### Example 81

### (R)-2-(1-methyl-5-(trifluoromethyl)-1H-pyrazol-3-yl)-N-(1-(5-(trifluoromethyl)pyridin-3-yl)pyrrolidin-3-yl)acetamide

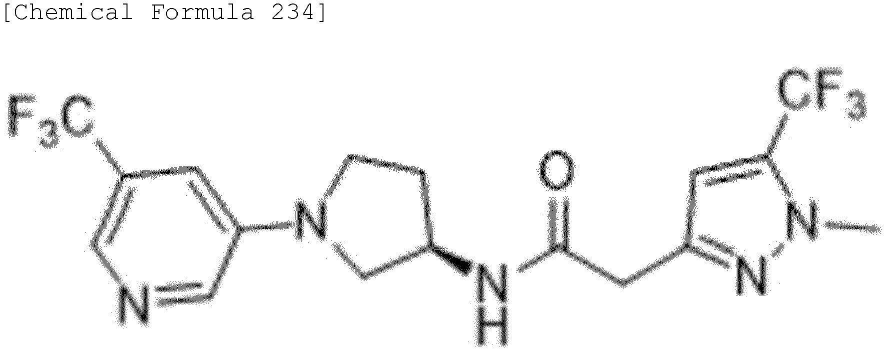

According to a technique similar to Example 1, the title compound (white powder, 62 mg, 98%) was obtained using (R)-1-(5-(trifluoromethyl)pyridin-3-yl)pyrrolidin-3-amine (35 mg, 0.151 mmol) synthesized in Reference Example 1-2 and 2-(1-methyl-5-(trifluoromethyl)-1H-pyrazol-3-yl)acetic acid (33 mg, 0.159 mmol).
¹H NMR (CDCl₃, 400MHz) : δ=2.0-2.1 (m, 1H), 2.3-2.5 (m, 1H), 3.23 (dd, 1H, J=4, 10Hz), 3.3-3.6 (m, 4H), 3.68 (dd, 1H, J=6, 10Hz), 3.91 (s, 3H), 4.6-4.7 (m, 1H), 6.54 (s, 1H), 6.68 (d, 1H, J=6Hz), 6.94 (t, 1H, J=2Hz), 8.12 (d, 1H, J=3Hz), 8.21 (d, 1H, J=1Hz).
MS:444.09[M+Na]⁺

### Example 82

### (R)-2-(6-(trifluoromethyl)pyridin-3-yl)-N-(1-(5-(trifluoromethyl)pyridin-3-yl)pyrrolidin-3-yl)acetamide

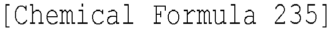

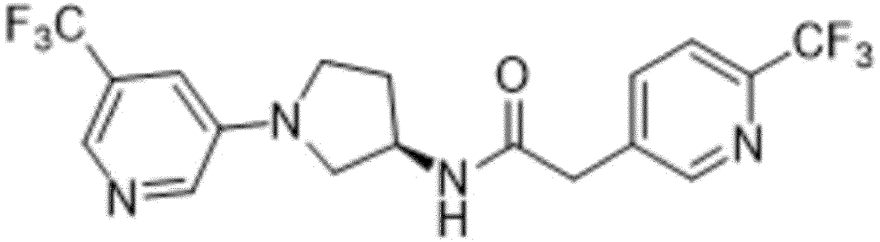

According to a technique similar to Example 1, the title compound (white powder, 27 mg, 74%) was obtained using (R)-1-(5-(trifluoromethyl)pyridin-3-yl)pyrrolidin-3-amine (20 mg, 0.0865 mmol) synthesized in Reference Example 1-2 and 2-(6-(trifluoromethyl)pyridin-3-yl)acetic acid (20 mg, 0.0952 mmol).
¹H NMR (CDCl₃, 400MHz) : δ=2.0-2.1 (m, 1H), 2.3-2.5 (m, 1H), 3.24 (dd, 1H, J=4, 10Hz), 3.3-3.6 (m, 2H), 3.62 (s, 2H), 3.67 (dd, 1H, J=6, 10 Hz), 4.6-4.7 (m, 1H), 6.02 (d, 1H, J=7Hz), 6.92 (s, 1H), 7.68 (d, 1H, J=8Hz), 7.90 (dd, 1H, J=1, 8Hz), 8.08 (d, 1H, 3Hz), 8.20 (s, 1H), 8.62 (d, 1H, J=2Hz).
MS:419.09 [M+H]⁺

### Example 83

### (R)-2-(4-(trifluoromethyl)phenyl)-N-(1-(5-(trifluoromethyl)pyridin-3-yl)pyrrolidin-3-yl)acetamide-2,2-d₂

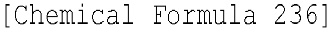

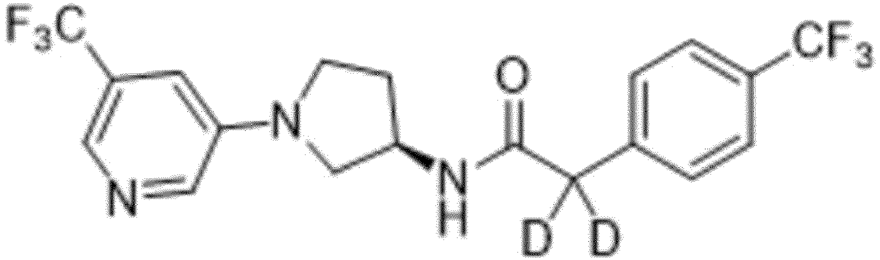

According to a technique similar to Example 1, the title compound (white crystals, 34 mg, 57%) was obtained using (R)-1-(5-(trifluoromethyl)pyridin-3-yl)pyrrolidin-3-amine (34 mg, 0.145 mmol) synthesized in Reference Example 1-2 and 2-(4-(trifluoromethyl)phenyl)acetic acid-2,2-d₂ (30 mg, 0.145 mmol) synthesized in Reference Example 56.
¹H NMR (CDCl₃, 400MHz) : δ=1.9-2.1 (m, 1H), 2.3-2.5 (m, 1H), 3.19 (dd, 1H, J=5, 10Hz), 3.3-3.5 (m, 2H), 3.67 (dd, 1H, J=6, 10Hz), 4.6-4.7 (m, 1H), 5.62 (d, 1H, J=6Hz), 6.9-7.0 (m, 1H), 7.40 (d, 2H, J=8Hz), 7.61 (d, 2H, J=8Hz), 8.10 (d, 1H, J=3Hz), 8.21 (s, 1H).
MS:420.10[M+H]⁺

### Example 84

### (R)-2-(4-(3,3-difluoropyrrolidin-1-yl)phenyl)-N-(1-(6-(trifluoromethyl)pyridin-3-yl)pyrrolidin-3-yl)acetamide

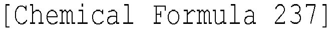

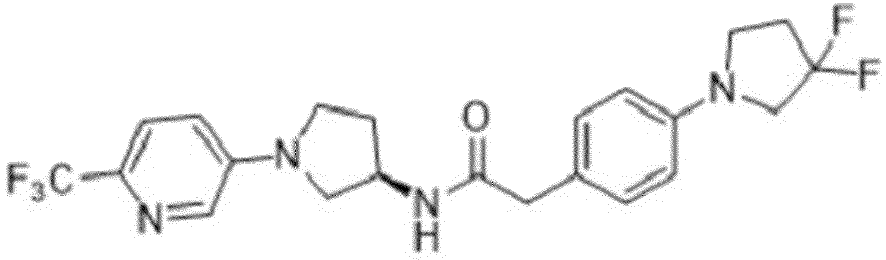

According to a technique similar to Example 9, the title compound (pale yellow powder, 22 mg, 24%) was obtained using (R)-2-(4-bromophenyl)-N-(1-(6-(trifluoromethyl)pyridin-3-yl)pyrrolidin-3-yl)acetamide (88 mg, 0.205 mmol) synthesized in Reference Example 57 and 3,3-difluoropyrrolidine hydrochloride (35 mg, 0.247 mmol).
¹H NMR (CDCl₃, 400MHz) : δ=1.8-2.0 (m, 1H), 2.2-2.6 (m, 3H), 3.11 (dd, 1H, J=5, 10Hz), 3.38 (t, 2H, J=7Hz), 3.4-3.6 (m, 4H), 3.6-3.7 (m, 3H), 4.5-4.7 (m, 1H), 5.49 (d, 1H, J=7Hz), 6.4-6.6 (m, 2H), 6.79 (dd, 1H, J=3, 8Hz), 7.0-7.2 (m, 2H), 7.47 (d, 1H, J=9Hz), 7.97 (d, 1H, J=3Hz).
MS:477.12[M+Na]⁺

### Example 85

### (R)-2-(1H-indol-6-yl)-N-(1-(6-(trifluoromethyl)-pyridin-3-yl)pyrrolidin-3-yl)acetamide

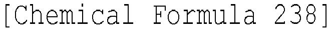

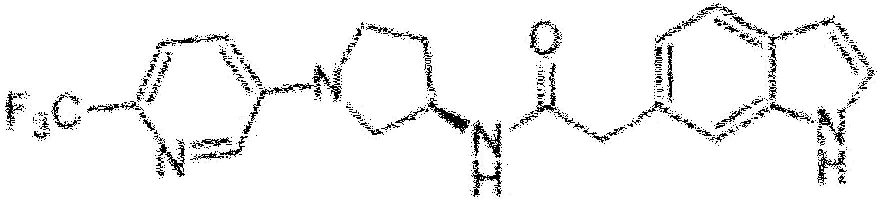

According to a technique similar to Example 1, the title compound (pale yellow amorphous, 35 mg, 84%) was obtained using (R)-1-(6-(trifluoromethyl)pyridin-3-yl)pyrrolidin-3-amine (25 mg, 0.108 mmol) synthesized in Reference Example 32-2 and 2-(1H-indol-6-yl)acetic acid (23 mg, 0.130 mmol).
¹H NMR (CDCl₃, 400MHz) : δ=1.8-1.9 (m, 1H), 2.2-2.4 (m, 1H), 3.10 (dd, 1H, J=5, 10Hz), 3.2-3.4 (m, 2H), 3.65 (dd, 1H, J=6, 10Hz), 3.70 (s, 2H), 4.5-4.7 (m, 1H), 5.45 (d, 1H, J=7Hz), 6.5-6.6 (m, 1H), 6.75 (dd, 1H, J=3, 8Hz), 6.96 (dd, 1H, J=1, 8Hz), 7.2-7.3 (m, 2H), 7.45 (d, 1H, J=9Hz), 1.62 (d, 1H, J=8Hz), 7.94 (d, 1H, J=3Hz), 8.17 (br s, 1H).
MS:389.12 [M+H]⁺

### Example 86

### 2-(4-((2R,6S)-2,6-dimethylmorpholino)phenyl)-N-((R)-1-(5-(trifluoromethyl)pyridin-3-yl)pyrrolidin-3-yl)acetamide

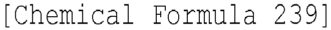

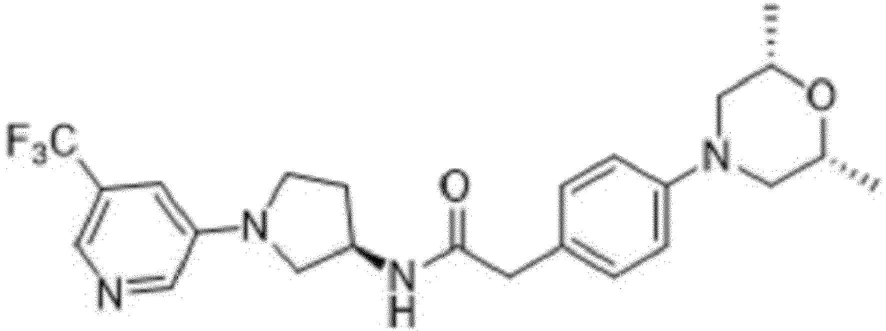

According to a technique similar to Example 9, the title compound (white powder, 12 mg, 22%) was obtained using (R)-2-(4-bromophenyl)-N-(1-(5-(trifluoromethyl)-pyridin-3-yl)pyrrolidin-3-yl)acetamide (50 mg, 0.117 mmol) synthesized in Example 171 and cis-2,6-dimethylmorpholine (16 mg, 0.140 mmol) .
¹H NMR (CDCl₃, 400MHz) : δ=1.26 (d, 6H, J=6Hz), 1.8-2.0 (m, 1H), 2.2-2.5 (m, 3H), 3.11 (dd, 1H, J=5, 10Hz), 3.37(t,2H,J=7Hz),3.4-3.5(m,2H),3.51(s,2H), 3.65 (dd, 1H, J=6, 10Hz), 3.7-3.9 (m, 2H), 4.5-4.7(m, 1H), 5.52 (d, 1H, J=7Hz), 6.8-7.0 (m, 3H), 7.12 (d, 2H, J=9Hz), 8.07 (d, 1H, J=3Hz), 8.20 (s, 1H).
MS:463.18[M+H]⁺

### Example 87

### (R)-2-(4-(pyrrolidin-1-yl)phenyl)-N-(1-(5-(trifluoromethyl)pyridin-3-yl)pyrrolidin-3-yl)acetamide

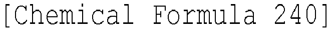

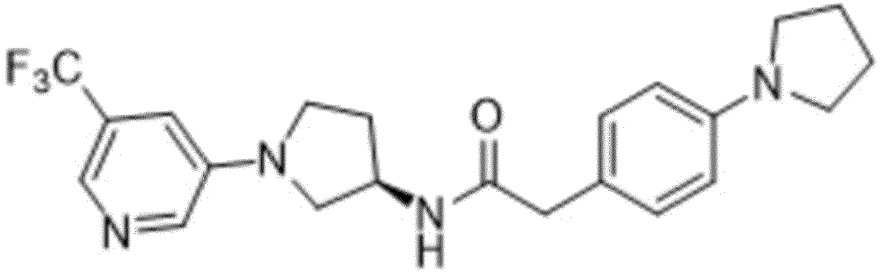

According to a technique similar to Example 9, the title compound (white crystals, 8 mg, 16%) was obtained using (R)-2-(4-bromophenyl)-N-(1-(5-(trifluoromethyl)pyridin-3-yl)pyrrolidin-3-yl)acetamide (50 mg, 0.117 mmol) synthesized in Example 171 and pyrrolidine (12 µL, 0.140 mmol) .
¹H NMR (CDCl₃, 400MHz) :δ=1.8-2.1 (m, 5H), 2.2-2.4 (m, 1H), 3.08 (dd, 1H, J=5, 10Hz), 3.2-3.3 (m, 4H), 3.35 (t, 2H, J=7Hz), 3.49 (s, 2H), 3.64 (dd, 1H, J=6, 10Hz), 4.5-4.7 (m, 1H), 5.55 (d, 1H, J=7Hz), 6.52 (d, 2H, J=9Hz), 6.8-6.9 (m, 1H), 7.05 (d, 2H, J=9Hz), 8.06 (d, 1H, J=3Hz), 8.19 (s, 1H).
MS:419.17[M+H]⁺

### Example 88

### (R)-2-(4-((2-methoxyethyl)(methyl)amino)phenyl)-N-(1-(5-(trifluoromethyl)pyridin-3-yl)pyrrolidin-3-yl)acetamide

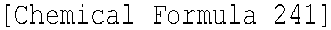

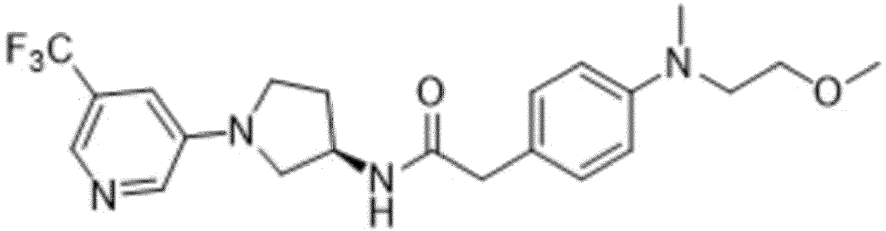

According to a technique similar to Example 9, the title compound (white amorphous, 2.5 mg, 5%) was obtained using (R)-2-(4-bromophenyl)-N-(1-(5-(trifluoromethyl)pyridin-3-yl)pyrrolidin-3-yl)acetamide (50 mg, 0.117 mmol) synthesized in Example 171 and N-(2-methoxyethyl)methylamine (15 µL, 0.140 mmol).
¹H NMR (CDCl₃, 400MHz) : δ=1.8-2.0 (m, 1H), 2.2-2.4 (m, 1H), 2.97 (s, 3H), 3.10 (dd, 1H, J=5, 10Hz), 3.3-3.4 (m, 5H), 3.4-3.6 (m, 6H), 3.65 (dd, 1H, J=6, 10Hz), 4.5-4.7 (m, 1H), 5.54 (d, 1H, J=7Hz), 6.69 (d, 2H, J=9Hz), 6.8-7.0 (m, 1H), 7.06 (d, 2H, J=9Hz), 8.07 (d, 1H, J=2Hz), 8.20 (s, 1H).
MS:437.17[M+H]⁺

### Example 89

### 2-(4-(Trifluoromethyl)phenyl)-N-(1-(5-(trifluoromethyl)pyridin-3-yl)azetidin-3-yl)acetamide

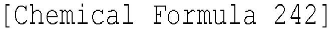

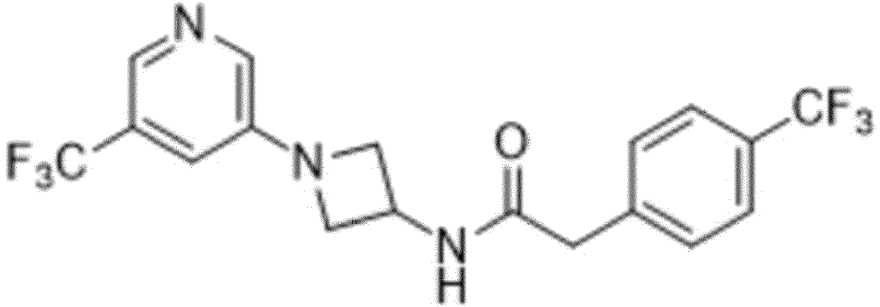

According to a technique similar to Example 1, the title compound (white crystals, 22 mg, 80%) was obtained using 1-(5-(trifluoromethyl)pyridin-3-yl)azetidin-3-amine (15 mg, 0.06691 mmol) synthesized in Reference Example 58-2 and 4-(trifluoromethyl)phenylacetic acid (17 mg, 0.0829 mmol) .
¹H NMR (DMSO-d₆, 400MHz) : δ=3.56 (s, 2H), 3.77 (dd, 2H, J=5, 8Hz), 4.26 (t, 2H, J=8Hz), 4.5-4.7 (m, 1H), 7.1-7.2 (m, 1H), 7.49 (d, 2H, J=8Hz), 7.67 (d, 2H, J=8Hz), 8.11 (t, 1H, J=2Hz), 8.23 (s, 1H), 8.89 (d, 1H, J=7Hz).
MS:404.06 [M+H]⁺

### Example 90

### 2-(1H-indol-6-yl)-N-(1-(5-(trifluoromethyl)pyridin-3-yl)azetidin-3-yl)acetamide

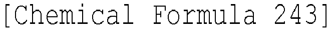

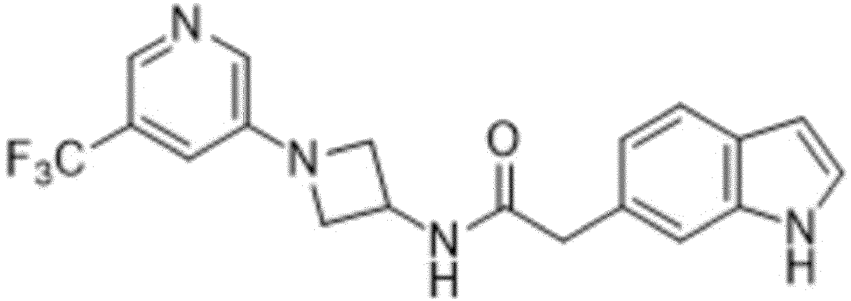

According to a technique similar to Example 1, the title compound (white crystals, 20 mg, 75%) was obtained using 1-(5-(trifluoromethyl)pyridin-3-yl)azetidin-3-amine (15 mg, 0.0691 mmol) synthesized in Reference Example 58-2 and 2-(1H-indol-6-yl)acetic acid (15 mg, 0.0829 mmol).
¹H NMR(DMSO-d₆, 400MHz) : δ=3.48 (s, 2H), 3.76 (dd, 2H, J=6, 8Hz), 4.25 (t, 2H, J=8Hz), 4.5-4.7 (m, 1H), 6.37 (t, 1H, J=2Hz), 6.90 (dd, 1H, J=1, 8Hz), 7.13 (t, 1H, J=2Hz), 7.2-7.3 (m, 2H), 7.44 (t, 1H, J=8Hz), 8.10 (d, 1H, J=3Hz), 8.23 (s, 1H), 8.72 (d, 1H, J=7Hz), 11.0 (s, 1H).
MS:375.09[M+H]⁺

### Example 91

### (R)-2-(1-methyl-1H-indol-6-yl)-N-(1-(5-(trifluoromethyl)pyridin-3-yl)pyrrolidin-3-yl)acetamide

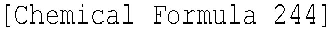

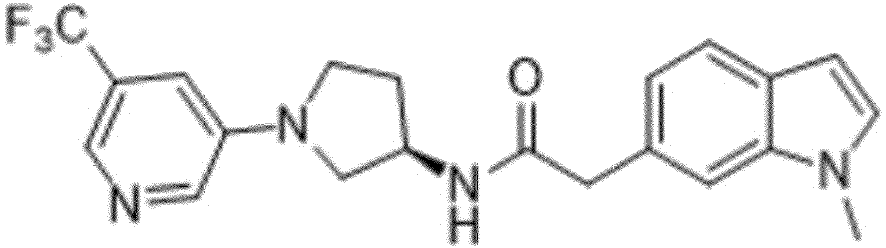

According to a technique similar to Example 1, the title compound (white crystals, 14 mg, 63%) was obtained using (R)-1-(5-(trifluoromethyl)pyridin-3-yl)pyrrolidin-3-amine (12 mg, 0.0529 mmol) synthesized in Reference Example 1-2 and 2-(1-methyl-1H-indol-6-yl)acetic acid (10 mg, 0.0529 mmol).
¹H NMR (CDCl₃, 400MHz) : δ=1.8-1.9 (m, 1H), 2.2-2.4 (m, 1H), 3.08 (dd, 1H, J=5, 10Hz), 3.2-3.4 (m, 2H), 3.64 (dd, 1H, J=6, 10Hz), 3.73 (s, 2H), 3.77 (s, 3H), 4.5-4.7 (m, 1H), 5.57 (d, 1H, J=7Hz), 6.48 (d, 1H, J=2Hz), 6.8-6.9 (m,1H), 6.95 (dd, 1H, J=1, 8Hz), 7.07 (d, 1H, J=3Hz), 7.19 (s, 1H), 7.60 (d, 1H, J=8Hz), 8.05 (d, 1H, J=3Hz).
MS:403.11[M+H]⁺

### Example 92

### (R)-2-(1-methyl-1H-indol-6-yl)-N-(1-(6-(trifluoromethyl)pyridin-3-yl)pyrrolidin-3-yl)acetamide

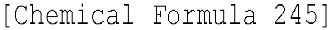

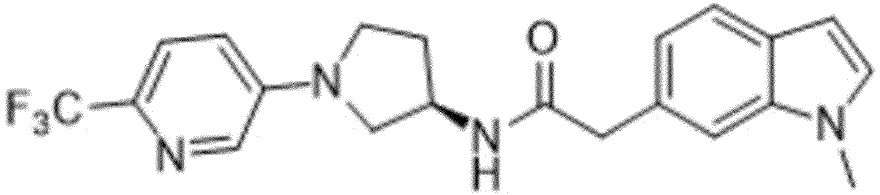

According to a technique similar to Example 1, the title compound (white crystals, 9 mg, 41%) was obtained using (R)-1-(6-(trifluoromethyl)pyridin-3-yl)pyrrolidin-3-amine (12 mg, 0.0529 mmol) synthesized in Reference Example 32-2 and 2-(1-methyl-1H-indol-6-yl)acetic acid (10 mg, 0.0529 mmol).
¹H NMR (CDCl₃, 400MHz) : δ=1.8-1.9 (m, 1H), 2.2-2.4 (m, 1H), 3.09 (dd, 1H, J=5, 10Hz), 3.2-3.4 (m, 2H), 3.65 (dd, 1H, J=6, 10Hz), 3.73 (s, 2H), 3.76 (s, 3H), 4.5-4.7 (m, 1H), 5.56 (d, 1H, J=7Hz), 6.48 (d, 1H, J=3Hz), 6.75 (dd, 1H, J=3, 9Hz), 6.94 (dd, 1H, J=1, 8Hz), 7.07 (d, 1H, J=3Hz), 7.19 (s, 1H), 7.45 (d, 1H, J=9Hz), 7.59 (d, 1H, J=8Hz), 7.93 (d, 1H, J=3Hz).
MS:403.11[M+H]⁺

### Example 93

### (R)-2-(4-(4-methylpiperazin-1-yl)phenyl)-N-(1-(5-(trifluoromethyl)pyridin-3-yl)pyrrolidin-3-yl)acetamide

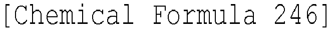

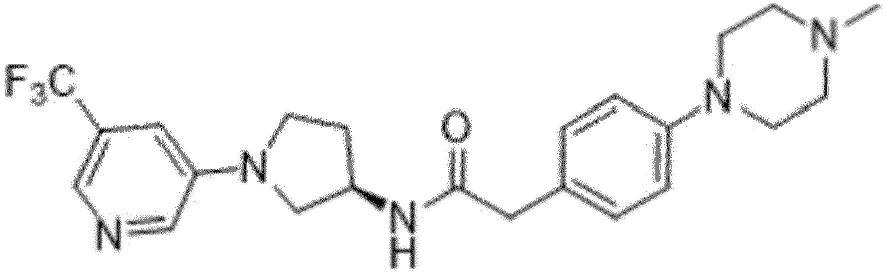

Ethyl 2-(4-(4-methylpiperazin-1-yl)phenyl)acetate (95 mg, 0.362 mmol) was dissolved in methanol (2 mL) and water (2 mL), lithium hydroxide monohydrate (46 mg, 1.09 mmol) was added thereto, and the mixture was stirred overnight at room temperature. Water was added to the reaction liquid, the mixture was washed with ethyl acetate, 6 M hydrochloric acid (190 µL) was added to an aqueous layer, and the aqueous layer was washed again with ethyl acetate. The aqueous layer was distilled off under reduced pressure, and a crude form of 2-(4-(4-methylpiperidin-1-yl)phenyl)acetic acid (colorless oily material, 192 mg) was obtained. The crude form of 2-(4-(4-methylpiperidin-1-yl)phenyl)acetic acid (96 mg) thus obtained and (R)-1-(5-(trifluoromethyl)pyridin-3-yl)pyrrolidin-3-amine (38 mg, 0.163 mmol) synthesized in Reference Example 1-2 were dissolved in methanol (1.5 mL), subsequently DMT-MM (90 mg, 0.326 mmol) and DIPEA (166 µL, 0.978 mmol) were added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by NH silica gel column chromatography (heptane : ethyl acetate) (concentration gradient: 50 to 100%), and the title compound (white powder, 51 mg, 69%) was obtained.
¹H NMR (CDCl₃, 400MHz) : δ=1.8-2.0 (m, 1H), 2.2-2.4 (m, 4H), 2.58 (t, 4H, J=5Hz), 3.11 (dd, 1H, J=5, 10Hz), 3.21 (t, 4H, J=5Hz), 3.37 (t, 2H, J=7Hz), 3.51 (s, 2H), 3.65 (dd, 1H, J=6, 10Hz), 4.5-4.7 (m, 1H), 5.52 (d, 1H, J=7Hz), 6.8-7.0 (m, 3H), 7.1-7.2 (m, 2H), 8.07 (d, 1H, J=3Hz), 8.20 (s, 1H).
MS:448.17[M+H]⁺

### Example 94

### (R)-2-(4-(piperazin-1-yl)phenyl)-N-(1-(5-(trifluoromethyl)pyridin-3-yl)pyrrolidin-3-yl)acetamide

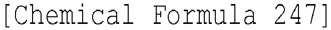

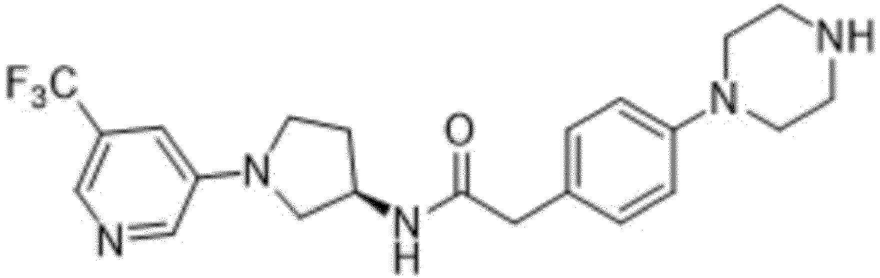

According to a technique similar to Example 33, the title compound (white powder, 122 mg, 91%) was obtained using benzyl (R)-4-(4-(2-oxo-2-((1-(5-(trifluoromethyl)-pyridin-3-yl)pyrrolidin-3-yl)amino)ethyl)phenyl)piperazine-1-carboxylate (176 mg, 0.310 mmol) synthesized in Reference Example 59.
¹H NMR (CDCl₃, 400MHz) : δ=1.8-2.0 (m, 1H), 2.2-2.4 (m, 1H), 3.0-3.2(m,9H),3.37(t,2H,J=7Hz),3.51(s,2H), 3.65 (dd, 1H, J=6, 10Hz), 4.5-4.7 (m, 1H), 5.51 (d, 1H, J=7Hz), 6.8-7.0 (m, 3H), 7.1-7.2 (m, 2H), 8.08 (d, 1H, J=3Hz), 8.20 (s, 1H).

The 1H content is not observable.
MS:434.15[M+Na]⁺

### Example 95

### (R)-2-(4-(4-acetylpiperazin-1-yl)phenyl)-N-(1-(5-(trifluoromethyl)pyridin-3-yl)pyrrolidin-3-yl)acetamide

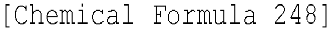

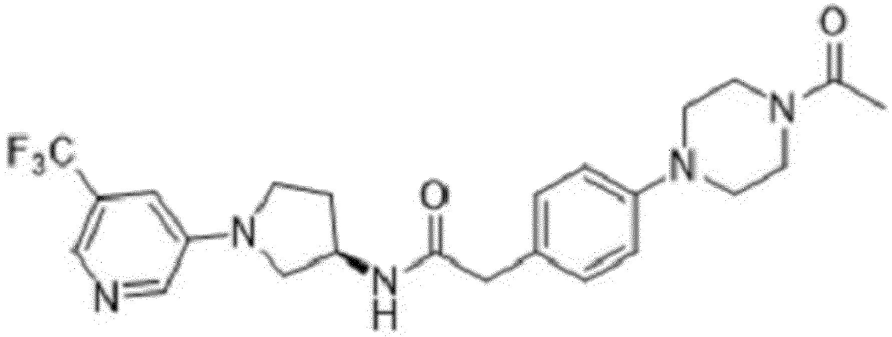

(R)-2-(4-(piperazin-1-yl)phenyl)-N-(1-(5-(trifluoromethyl)pyridin-3-yl)pyrrolidin-3-yl)acetamide (45 mg, 0.104 mmol) synthesized in Example 94 and triethylamine (36 µL, 0.104 mmol) were dissolved in chloroform (2 mL), subsequently acetic anhydride (12 µL, 0.125 mmol) was added thereto under ice cooling, and the mixture was stirred at room temperature. After 18 hours, the solvent was distilled off under reduced pressure, a residue thus obtained was purified by NH silica gel column chromatography (heptane : ethyl acetate) (concentration gradient: 90 to 100%), and the title compound (white powder, 43 mg, 86%) was obtained.
¹H NMR (CDCl₃, 400MHz) : δ=1.8-2.0 (m, 1H), 2.15 (s, 3H), 2.2-2.4 (m, 1H), 3.0-3.2 (m, 5H), 3.38 (t, 2H, J=7Hz), 3.51 (s, 2H), 3.6-3.7 (m, 3H), 3.77 (t, 2H, J=5Hz), 4.5-4.7 (m, 1H), 5.52 (d, 1H, J=7Hz), 6.8-7.0 (m, 3H), 7.1-7.2 (m, 2H), 8.08 (d, 1H, J=3Hz), 8.20 (s, 1H).
MS:498.14[M+Na]⁺

### Example 96

### (R)-2-(4-(2-oxopiperidin-1-yl)phenyl)-N-(1-(5-(trifluoromethyl)pyridin-3-yl)pyrrolidin-3-yl)acetamide

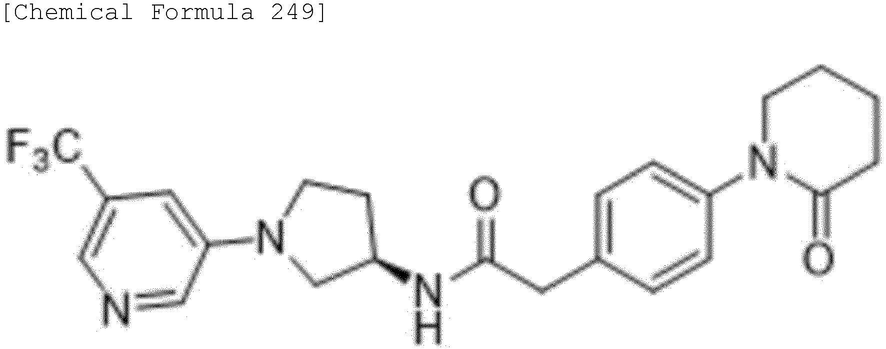

According to a technique similar to Example 1, the title compound (white powder, 45 mg, 47%) was obtained using (R)-1-(5-(trifluoromethyl)pyridin-3-yl)pyrrolidin-3-amine (50 mg, 0.215 mmol) synthesized in Reference Example 1-2 and 2-(4-(2-oxopiperidin-1-yl)phenyl)acetic acid (55 mg, 0.236 mmol).
¹H NMR (CDCl₃, 400MHz) :δ=1.8-2.1 (m, 5H), 2.2-2.4 (m, 1H), 2.53 (t, 2H, J=6Hz), 3.18 (dd, 1H, J=5, 10Hz), 3.3-3.7(m, 7H), 4.5-4.7 (m, 1H), 5.88 (d, 1H, J=7Hz), 6.8-7.0 (m, 1H), 7.2-7.3 (m, 4H), 8.08 (d, 1H, J=3Hz), 8.20 (s, 1H).
MS:469.11[M+H]⁺

### Example 97

### (R)-2-(6-chlorobenzo[d]oxazol-2-yl)-N-(1-(5-(trifluoromethyl)pyridin-3-yl)pyrrolidin-3-yl)acetamide

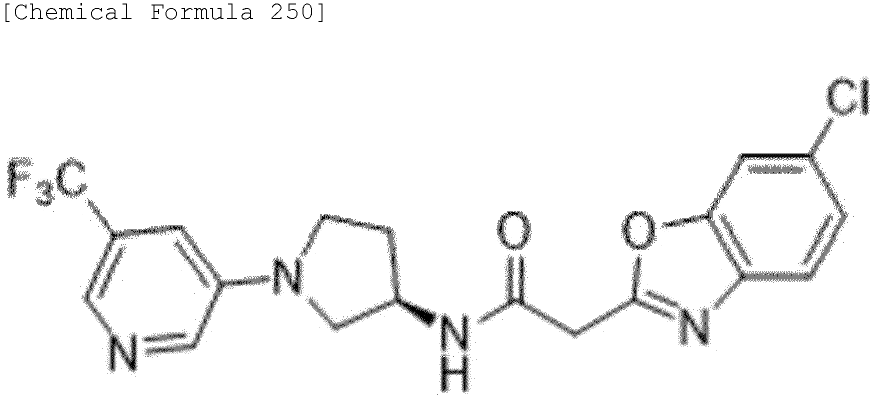

According to a technique similar to Example 1, the title compound (yellow powder, 57 mg, 71%) was obtained using (R)-1-(5-(trifluoromethyl)pyridin-3-yl)pyrrolidin-3-amine (44 mg, 0.189 mmol) synthesized in Reference Example 1-2 and 2-(6-chloro-1,3-benzoxazol-2-yl)acetic acid (40 mg, 0.189 mmol).
¹H NMR (CDCl₃, 400MHz) : δ=2.1-2.2 (m, 1H), 2.3-2.5 (m, 1H), 3.33 (dd, 1H, J=4, 10Hz), 3.4-3.6 (m, 2H), 3.73 (dd, 1H, J=6, 10Hz), 3.69 (s, 2H), 4.6-4.8 (m, 1H), 6.9-7.0 (m, 1H), 7.34 (dd, 1H, J=2, 9Hz), 7.45 (d, 1H, J=9Hz), 7.65 (d, 1H, J=2Hz), 7.99 (d, 1H, J=6Hz), 8.13 (d, 1H, J=3Hz), 8.22 (s, 1H).
MS:425.06 [M+H]⁺

### Example 98

### (R)-2-(benzo[d]oxazol-2-yl)-N-(1-(5-(trifluoromethyl)pyridin-3-yl)pyrrolidin-3-yl)acetamide

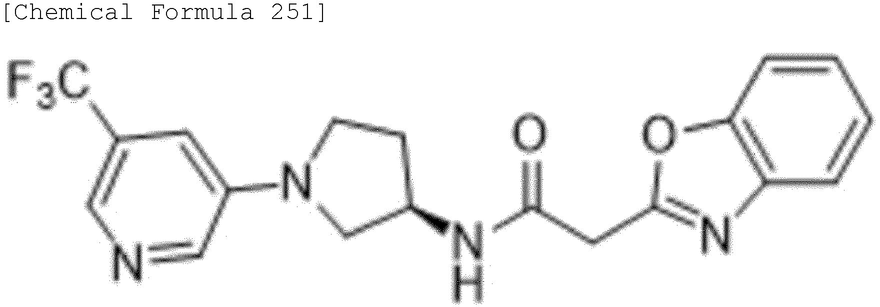

According to a technique similar to Example 1, the title compound (yellow powder, 51 mg, 76%) was obtained using (R)-1-(5-(trifluoromethyl)pyridin-3-yl)pyrrolidin-3-amine (40 mg, 0.173 mmol) synthesized in Reference Example 1-2 and sodium 2-(benzo[d]oxazol-2-yl)acetate monohydrate (41 mg, 0.190 mmol).
¹H NMR (CDCl₃, 400MHz) : δ=2.1-2.2 (m, 1H), 2.3-2.5 (m, 1H), 3.34 (dd, 1H, J=4, 10Hz), 3.4-3.6 (m, 2H), 3.73 (dd, 1H, J=6, 10Hz), 3.97 (s, 2H), 4.6-4.8 (m, 1H), 6.9-7.0 (m, 1H), 7.3-7.4 (m, 2H), 7.5-7.6 (m, 1H), 7.6-7.7 (m, 1H), 8.13 (d, 1H, J=3Hz), 8.21 (s, 2H).
MS:391.10 [M+H]⁺

### Example 99

### Trans-2-(3,5-dichlorophenyl)-N-((R)-1-(5-(trifluoromethyl)pyridin-3-yl)pyrrolidin-3-yl)cyclopropane-1-carboxyamide

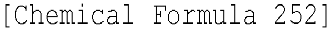

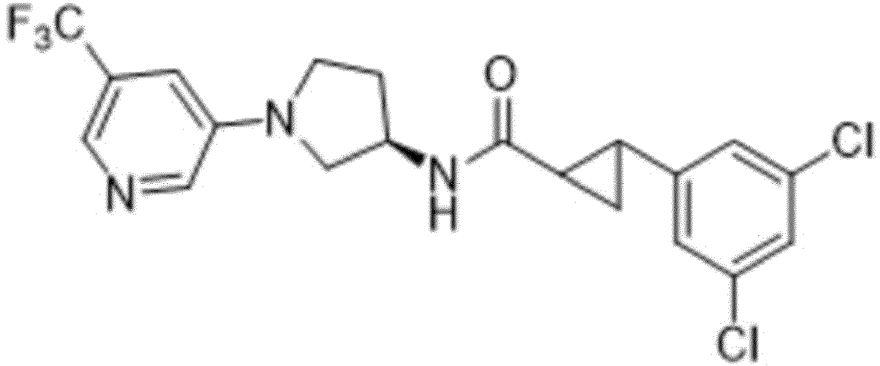

According to a technique similar to Example 1, diastereomer A (upper spot in TLC (ethyl acetate), white powder, 25 mg, 42%) and diastereomer B (lower spot in TLC (ethyl acetate), white powder, 19 mg, 32%) of the title compound were obtained using (R)-1-(5-(trifluoromethyl)-pyridin-3-yl)pyrrolidin-3-amine (30 mg, 0.130 mmol) synthesized in Reference Example 1-2 and trans-2-(3,5-dichlorophenyl)cyclopropane-1-carboxylic acid (33 mg, 0.143 mmol) .

### Diastereomer A

¹H NMR (CDCl₃, 400MHz) : δ=1.2-1.3 (m, 1H), 1.5-1.7 (m, 2H), 2.0-2.2 (m, 1H), 2.3-2.6 (m, 2H), 3.28 (dd, 1H, J=4, 10Hz), 3.4-3.6 (m, 2H), 3.69 (dd, 1H, J=6, 10Hz), 4.6-4.8 (m, 1H), 5.87 (d, 1H, J=7Hz), 6.9-7.0 (m, 3H), 7.19 (t, 1H, J=2Hz), 8.13 (d, 1H, J=3Hz), 8.22 (s, 1H).
MS:444.02 [M+H]⁺

### Diastereomer B

¹H NMR (CDCl₃, 400MHz) : δ=1.2-1.3 (m, 1H), 1.5-1.7 (m, 2H), 2.0-2.2 (m, 1H), 2.3-2.5 (m, 2H), 3.27 (dd, 1H, J=4, 10Hz), 3.4-3.6 (m, 2H), 3.68 (dd, 1H, J=6, 10Hz), 4.6-4.8 (m, 1H), 5.88 (d, 1H, J=7Hz), 6.9-7.0 (m, 3H), 7.18 (t, 1H, J=2Hz), 8.12 (d, 1H, J=3Hz), 8.21 (s, 1H).
MS:444.01 [M+H]⁺

### Example 100

### Trans-2-(4-bromophenyl)-N-((R)-1-(5-(trifluoromethyl)pyridin-3-yl)pyrrolidin-3-yl)cyclopropane-1-carboxyamide

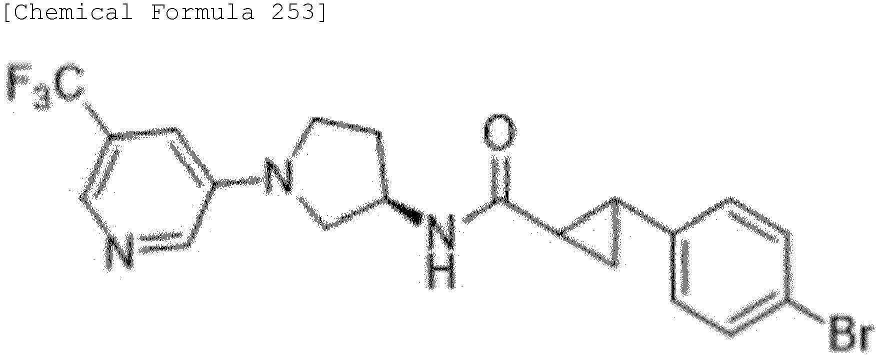

According to a technique similar to Example 1, diastereomer A (upper spot in TLC (ethyl acetate), white powder, 26 mg, 43%) and diastereomer B (lower spot in TLC (ethyl acetate), white powder, 23 mg, 39%) of the title compound were obtained using (R)-1-(5-(trifluoromethyl)-pyridin-3-yl)pyrrolidin-3-amine (30 mg, 0.130 mmol) synthesized in Reference Example 1-2 and trans-2-(4-bromophenyl)cyclopropane-1-carboxylic acid (34 mg, 0.143 mmol) .

### Diastereomer A

¹H NMR (CDCl₃, 400MHz) : δ=1.2-1.3 (m, 1H), 1.5-1.7 (m, 2H), 2.0-2.2 (m, 1H), 2.3-2.6 (m, 2H), 3.27 (dd, 1H, J=5, 10Hz), 3.4-3.6 (m, 2H), 3.69 (dd, 1H, J=6, 10Hz), 4.6-4.8 (m, 1H), 5.80 (d, 1H, J=7Hz), 6.9-7.0 (m, 3H), 7.3-7.5 (m, 2H), 8.13 (d, 1H, J=3Hz), 8.22 (s, 1H).
MS:454.00 [M+H]⁺

### Diastereomer B

¹H NMR (CDCl₃, 400MHz) : δ=1.2-1.3 (m, 1H), 1.5-1.7 (m, 2H), 2.0-2.2 (m, 1H), 2.3-2.6 (m, 2H), 3.26 (dd, 1H, J=4, 10Hz), 3.4-3.6 (m, 2H), 3.68 (dd, 1H, J=6, 10Hz), 4.6-4.8 (m, 1H), 5.84 (d, 1H, J=7Hz), 6.9-7.0 (m, 3H), 7.3-7.4 (m, 2H), 8.12 (d, 1H, J=3Hz), 8.21 (s, 1H).
MS:454.01 [M+H]⁺

### Example 101

### (R)-2-(4-(1,4-dioxa-8-azaspiro[4.5]decan-8-yl)phenyl)-N-(1-(5-(trifluoromethyl)pyridin-3-yl)pyrrolidin-3-yl)acetamide

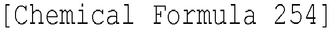

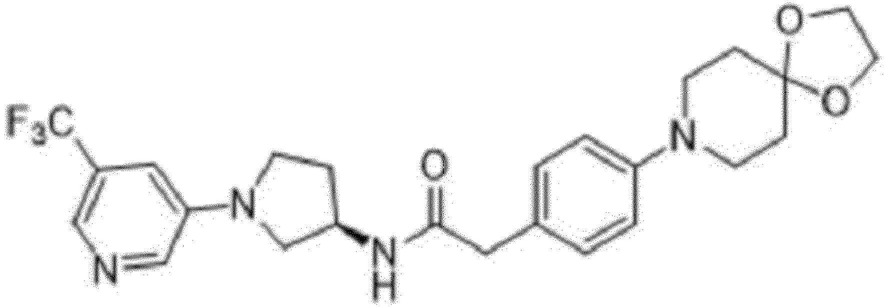

Ethyl 2-(4-(1,4-dioxa-8-azaspiro[4.5]decan-8-yl)phenyl)acetate (16 mg, 0.0524 mmol) synthesized in Reference Example 60 was dissolved in methanol (1 mL) and water (1 mL), lithium hydroxide monohydrate (7 mg, 0.176 mmol) was added thereto, and the mixture was stirred overnight at room temperature. Water was added to the reaction liquid, the mixture was washed with ethyl acetate, 2 M hydrochloric acid (88 µL) was added to an aqueous layer to neutralize the aqueous layer, and the aqueous layer was extracted with a mixed liquid of chloroform and methanol. An organic layer thus separated was dried over anhydrous sodium sulfate, insoluble materials were filtered, subsequently the solvent was distilled off under reduced pressure, and a crude form of 2-(4-(1,4-dioxa-8-azaspiro[4.5]decan-8-yl)phenyl)acetic acid (white powder, 28 mg) was obtained. According to a technique similar to Example 1, the title compound (white powder, 18 mg, 70%) was obtained using the crude form of 2-(4-(1,4-dioxa-8-azaspiro[4.5]decan-8-yl)phenyl)acetic acid (28 mg) thus obtained and (R)-1-(5-(trifluoromethyl)pyridin-3-yl)pyrrolidin-3-amine (28 mg, 0.0524 mmol) synthesized in Reference Example 1-2.
¹H NMR (CDCl₃, 400MHz) : δ=1.8-2.0 (m, 5H), 2.2-2.4 (m, 1H), 3.11 (dd, 1H, J=5, 10Hz), 3.3-3.4 (m, 6H), 3.50 (s, 2H), 3.65 (dd, 1H, J=6, 10Hz), 4.00 (s, 4H), 4.5-4.7 (m, 1H), 5.50 (d, 1H, J=7Hz), 6.8-7.0 (m, 3H), 7.0-7.2 (m, 2H), 8.08 (d, 1H, J=3Hz), 8.20 (s, 1H).
MS:491.18 [M+H]⁺

### Example 102

### (R)-2-(4-(4-oxopiperidin-1-yl)phenyl)-N-(1-(5-(trifluoromethyl)pyridin-3-yl)pyrrolidin-3-yl)acetamide

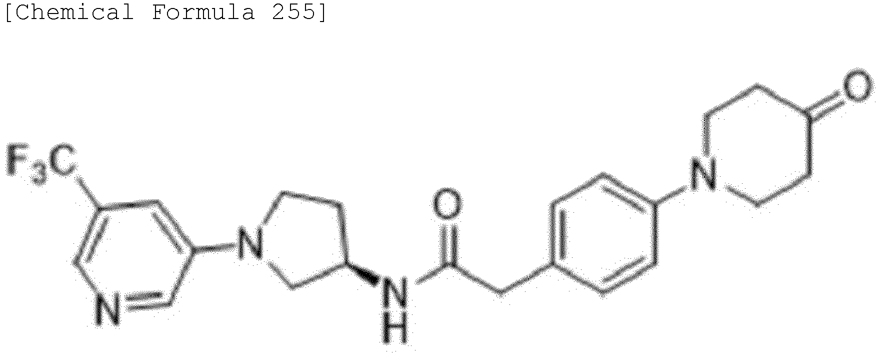

(R)-2-(4-(1,4-dioxa-8-azaspiro[4.5]decan-8-yl)phenyl)-N-(1-(5-(trifluoromethyl)pyridin-3-yl)pyrrolidin-3-yl)acetamide (17 mg, 0.032 mmol) synthesized in Example 101 was dissolved in ethanol (1.0 mL), 2 N hydrochloric acid (1.0 mL) was added thereto, and the mixture was heated to reflux overnight in a nitrogen atmosphere. To the reaction liquid that had been left to cool to room temperature, a saturated aqueous solution of sodium hydrogen carbonate was added to neutralize the reaction liquid, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (ethyl acetate : methanol) (concentration gradient: 0 to 5%), and the title compound (white powder, 2 mg, 14%) was obtained.
¹H NMR (CDCl₃, 400MHz) : δ=1.8-2.0 (m, 1H), 2.3-2.4 (m, 1H), 2.56 (t, 4H, 6Hz), 3.14 (dd, 1H, J=5, 10Hz), 3.3-3.5 (m, 2H), 3.52 (s, 2H), 3.60 (t, 4H, J=6Hz), 3.66 (dd, 1H, J=6, 10Hz), 4.5-4.7 (m, 1H), 5.52 (d, 1H, J=6Hz), 6.8-7.0 (m, 3H), 7.16 (d, 2H, J=9Hz), 8.09 (d, 1H, J=3Hz), 8.21 (s, 1H).
MS:469.13[M+Na]⁺

### Example 103

### 2-(4-cyclopropylphenyl)-N-(3-(hydroxymethyl)-1-(5-(trifluoromethyl)pyridin-3-yl)pyrrolidin-3-yl)acetamide

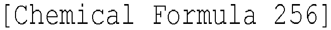

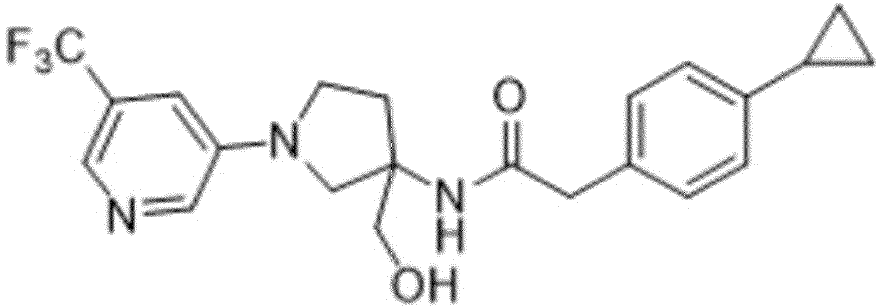

(3-Amino-1-(5-(trifluoromethyl)pyridin-3-yl)pyrrolidin-3-yl)methanol (51 mg, 0.195 mmol) synthesized in Reference Example 61-2 and 2-(4-cyclopropylphenyl)acetic acid (34 mg, 0.195 mmol) synthesized in Reference Example 33-2 were dissolved in ethanol (3 mL), subsequently DMT-MM (81 mg, 0.293 mmol) was added thereto, and the mixture was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (ethyl acetate : methanol) (concentration gradient: 0 to 5%), and the title compound (white powder, 50 mg, 61%) was obtained.
¹H NMR (CDCl₃, 400MHz) : δ=0.6-0.7 (m, 2H), 0.9-1.0 (m, 2H), 1.8-1.9 (m, 1H), 2.1-2.3 (m, 2H), 3.2-3.5 (m, 3H), 3.54 (s, 2H), 3.59 (d, 1H, J=11Hz), 3.82 (d, 2H, J=6Hz), 4.03 (t, 1H, J=6Hz), 5.62 (s, 1H), 6.8-7.1 (m, 5H), 8.06 (d, 1H, J=3Hz), 8.22 (s, 1H).
MS:420.14[M+H]⁺

### Example 104

### (3-(2-(4-Cyclopropylphenyl)acetamido)-1-(5-(trifluoromethyl)pyridin-3-yl)pyrrolidin-3-yl)methyl acetate

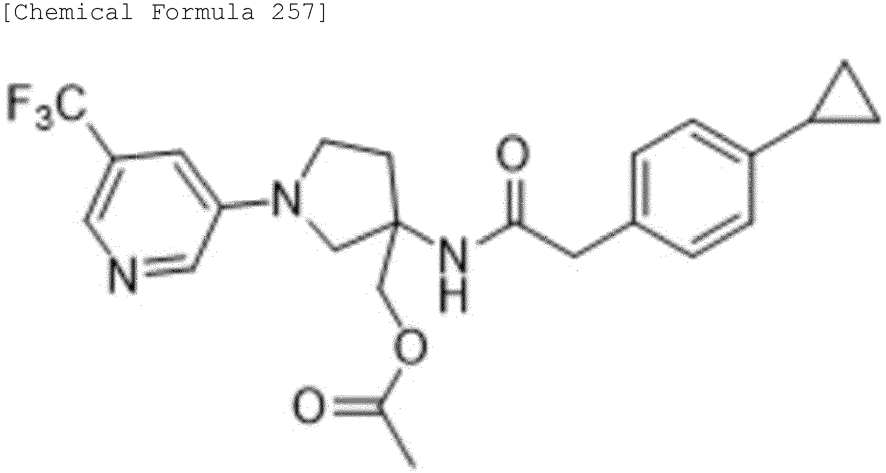

According to a technique similar to Example 95, the title compound (colorless oily material, 10 mg, 91%) was obtained using 2-(4-cyclopropylphenyl)-N-(3-(hydroxymethyl)-1-(5-(trifluoromethyl)pyridin-3-yl)pyrrolidin-3-yl)acetamide (10 mg, 0.0238 mmol) synthesized in Example 103.
¹H NMR (CDCl₃, 400MHz) : δ=0.6-0.7 (m, 2H), 0.9-1.0 (m, 2H), 1.8-1.9 (m, 1H), 2.02 (s, 3H), 2.1-2.4 (m, 2H), 3.3-3.5 (m, 2H), 3.51 (s, 2H), 3.55 (d, 1H, J=11Hz), 3.63 (d, 1H, J=11Hz), 4.35 (dd, 2H, J=12, 16Hz), 5.49 (s, 1H),6.8-7.2(m,5H), 8.08 (d, 1H, J=2Hz), 8.22 (s, 1H).
MS:484.14[M+Na]⁺

### Example 105

### 2-(4-cyclopropylphenyl)-N-(3-(methoxymethyl)-1-(5-(trifluoromethyl)pyridin-3-yl)pyrrolidin-3-yl)acetamide

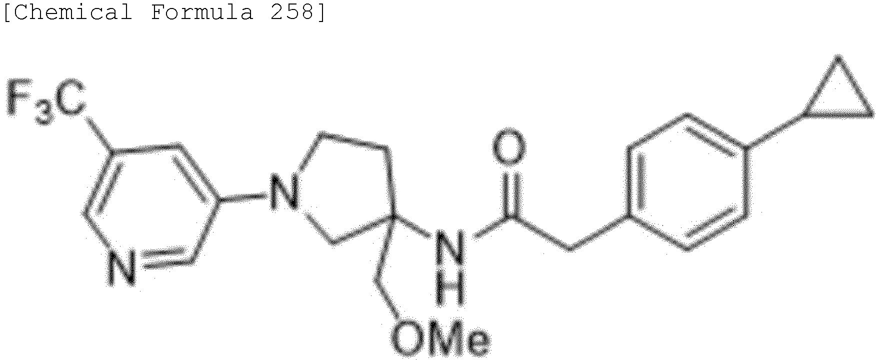

2-(4-Cyclopropylphenyl)-N-(3-(hydroxymethyl)-1-(5-(trifluoromethyl)pyridin-3-yl)pyrrolidin-3-yl)acetamide (37 mg, 0.0882 mmol) synthesized in Example 103 was dissolved in THF (1 mL), subsequently 60% sodium hydride (4 mg, 0.0926 mmol) and iodomethane (6 µL, 0.0926 mmol) were added thereto under ice cooling, and the mixture was stirred overnight at room temperature. A saturated aqueous solution of sodium hydrogen carbonate was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 60 to 80%), and the title compound (white powder, 16 mg, 42%) was obtained.
¹H NMR (CDCl₃, 400MHz) : δ=0.6-0.7 (m, 2H), 0.9-1.0 (m, 2H), 1.8-1.9 (m, 1H), 2.1-2.5 (m, 2H), 3.33 (s, 3H), 3.39 (t, 2H, J=7Hz), 3.4-3.7 (m, 6H), 5.58 (s, 1H), 6.8-7.0 (m, 1H), 7.03 (d, 2H, J=8Hz), 7.11 (d, 2H, J=8Hz), 8.08 (d, 1H, J=3Hz), 8.20 (s, 1H).
MS:434.15[M+Na]⁺

### Example 106

### Trans-2-(4-cyclopropylphenyl)-N-((R)-1-(5-(trifluoromethyl)pyridin-3-yl)pyrrolidin-3-yl)cyclopropane-1-carboxyamide

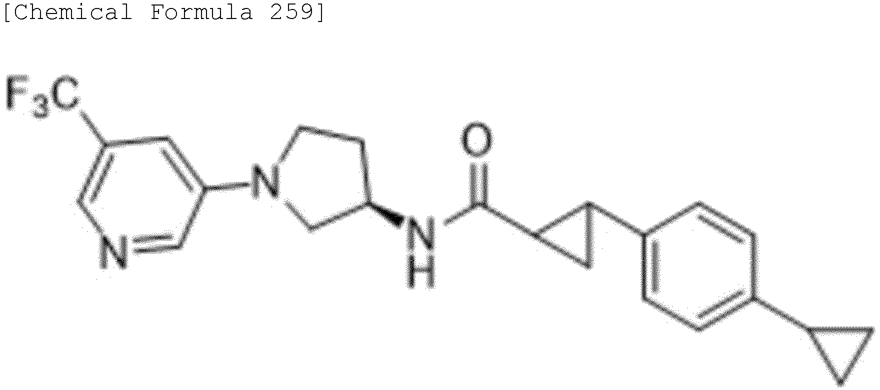

According to a technique similar to Example 1, diastereomer A (upper spot in TLC (ethyl acetate), white powder, 37 mg, 33%) and diastereomer B (lower spot in TLC (ethyl acetate), white powder, 35 mg, 31%) of the title compound were obtained using (R)-1-(5-(trifluoromethyl)-pyridin-3-yl)pyrrolidin-3-amine (62 mg, 0.218 mmol) synthesized in Reference Example 1-2 and trans-2-(4-cyclopropylphenyl)cyclopropane-1-carboxylic acid (59 mg, 0.294 mmol).

### Diastereomer A

¹H NMR (CDCl₃, 400MHz) : δ=0.6-0.7 (m, 2H), 0.9-1.0 (m, 2H), 1.2-1.3 (m, 1H), 1.5-1.7 (m, 2H), 1.8-1.9 (m, 1H), 2.0-2.1 (m, 1H), 2.3-2.5 (m, 2H), 3.26 (dd, 1H, J=4, 10Hz), 3.3-3.6 (m, 2H), 3.68 (dd, 1H, J=6, 10Hz), 4.6-4.8 (m, 1H), 5.78 (d, 1H, J=7Hz), 6.9-7.0 (m, 5H), 8.13 (d, 1H, J=3Hz), 8.21 (s, 1H).
MS:416.14[M+H]⁺

### Diastereomer B

¹H NMR (CDCl₃, 400MHz) : δ=0.6-0.7 (m, 2H), 0.9-1.0 (m, 2H), 1.2-1.3 (m, 1H), 1.5-1.7 (m, 2H), 1.8-1.9 (m, 1H), 2.0-2.1 (m, 1H), 2.3-2.5 (m, 2H), 3.24 (dd, 1H, J=4, 10Hz), 3.3-3.6 (m, 2H), 3.68 (dd, 1H, J=6, 10Hz), 4.6-4.8 (m, 1H), 5.78 (d, 1H, J=7Hz), 6.9-7.0 (m, 5H), 8.12 (d, 1H, J=2Hz), 8.21 (s, 1H).
MS:416.14[M+H]⁺

### Example 107

### Trans-2-(3,4-difluorophenyl)-N-((R)-1-(5-(trifluoromethyl)pyridin-3-yl)pyrrolidin-3-yl)cyclopropane-1-carboxyamide

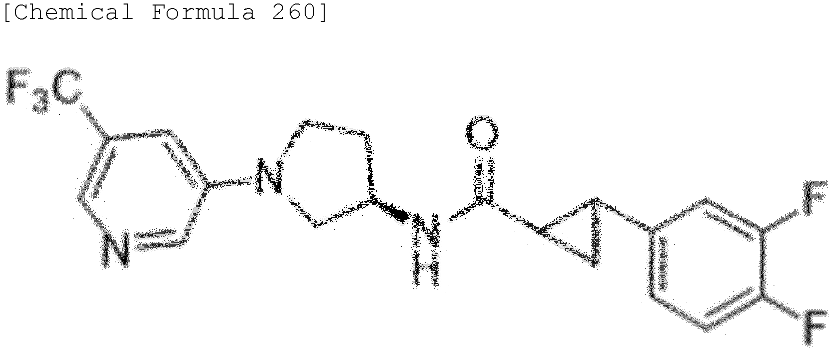

According to a technique similar to Example 1, diastereomer A (upper spot in TLC (ethyl acetate), white powder, 42 mg, 38%) and diastereomer B (lower spot in TLC (ethyl acetate), white powder, 36 mg, 33%) of the title compound were obtained using (R)-1-(5-(trifluoromethyl)-pyridin-3-yl)pyrrolidin-3-amine (62 mg, 0.218 mmol) synthesized in Reference Example 1-2 and trans-2-(3,4-difluorophenyl)cyclopropane-1-carboxylic acid (58 mg, 0.294 mmol) .

### Diastereomer A

¹H NMR (CDCl₃, 400MHz) : δ=1.1-1.3 (m, 1H), 1.5-1.7 (m, 2H), 2.0-2.2 (m, 1H), 2.3-2.6 (m, 2H), 3.27 (dd, 1H, J=4, 10Hz), 3.4-3.6 (m, 2H), 3.69 (dd, 1H, J=6, 10Hz), 4.6-4.8 (m, 1H), 5.90 (d, 1H, J=7Hz), 6.8-7.1 (m, 4H), 8.12 (d, 1H, J=3Hz), 8.21 (s, 1H).
MS:412.10[M+H]⁺

### Diastereomer B

¹H NMR (CDCl₃, 400MHz) : δ=1.1-1.3 (m, 1H), 1.5-1.7 (m, 2H), 2.0-2.2 (m, 1H), 2.3-2.6 (m, 2H), 3.27 (dd, 1H, J=4, 10Hz), 3.4-3.6 (m, 2H), 3.68 (dd, 1H, J=6, 10Hz), 4.6-4.8 (m, 1H), 5.87 (d, 1H, J=7Hz), 6.7-7.1 (m, 4H), 8.12 (d, 1H, J=2Hz), 8.21 (s, 1H).
MS:412.10 [M+H]⁺

### Example 108

### Trans-2-(4-chlorophenyl)-N-((R)-1-(5-(trifluoromethyl)pyridin-3-yl)pyrrolidin-3-yl)cyclopropane-1-carboxyamide

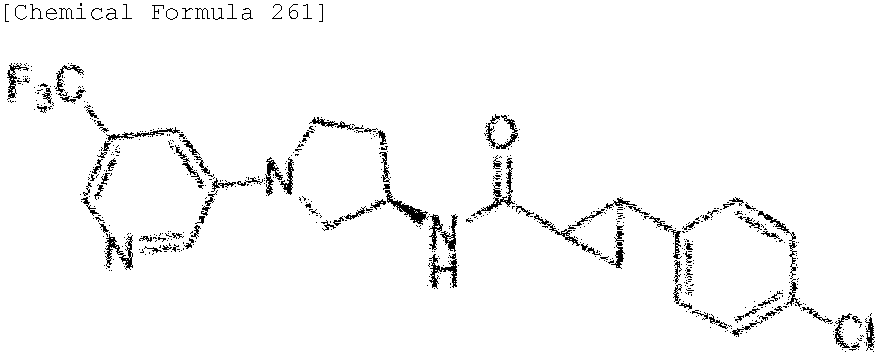

According to a technique similar to Example 1, diastereomer A (upper spot in TLC (ethyl acetate), white powder, 39 mg, 35%) and diastereomer B (lower spot in TLC (ethyl acetate), white powder, 33 mg, 30%) of the title compound were obtained using (R)-1-(5-(trifluoromethyl)-pyridin-3-yl)pyrrolidin-3-amine (62 mg, 0.218 mmol) synthesized in Reference Example 1-2 and trans-2-(4-chlorophenyl)cyclopropane-1-carboxylic acid (58 mg, 0.294 mmol) .

### Diastereomer A

¹H NMR (CDCl₃, 400MHz) : δ=1.2-1.3 (m, 1H), 1.5-1.7 (m, 2H), 2.0-2.1 (m, 1H), 2.3-2.6 (m, 2H), 3.27 (dd, 1H, J=4, 10Hz), 3.3-3.6 (m, 2H), 3.68 (dd, 1H, J=6, 10Hz), 4.6-4.8 (m, 1H), 5.90 (d, 1H, J=7Hz), 6.9-7.1 (m, 3H), 7.2-7.3 (m, 2H), 8.12 (d, 1H, J=3Hz), 8.21 (s, 1H).
MS:410.07 [M+H]⁺

### Diastereomer B

¹H NMR (CDCl₃, 400MHz) : δ=1.2-1.3 (m, 1H), 1.5-1.7 (m, 2H), 2.0-2.2 (m, 1H), 2.3-2.6 (m, 2H), 3.26 (dd, 1H, J=4, 10Hz), 3.3-3.6 (m, 2H), 3.68 (dd, 1H, J=6, 10Hz), 4.6-4.8 (m, 1H), 5.87 (d, 1H, J=7Hz), 6.9-7.1 (m, 3H), 7.2-7.3 (m, 2H), 8.11 (d, 1H, J=3Hz), 8.21 (s, 1H).
MS:410.07 [M+H]⁺

### Example 109

### 2-(4-Isopropylphenyl)-N-(1-(5-(trifluoromethyl)-pyridin-3-yl)piperidin-4-yl)acetamide

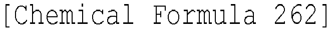

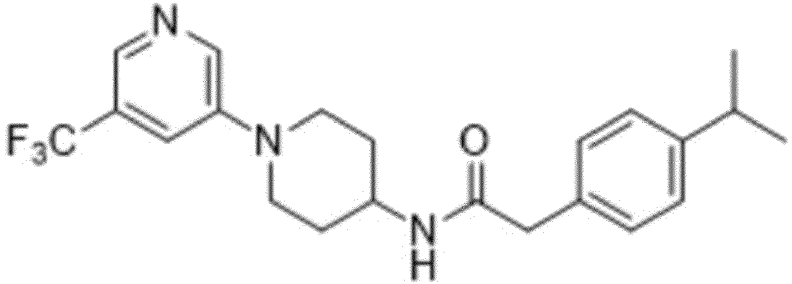

According to a technique similar to Example 9, the title compound (pale yellow crystals, 62 mg, 39%) was obtained using 2-(4-isopropylphenyl)-N-(piperidin-4-yl)acetamide (100 mg, 0.384 mmol) synthesized in Reference Example 62 and 3-bromo-5-(trifluoromethyl)pyridine (104 mg, 0.461 mmol).
¹H NMR (CDCl₃, 400MHz) : δ=1.1-1.5 (m, 8H), 2.02 (d, 2H, J=11Hz), 2.8-3.1 (m, 3H), 3.5-3.7 (m, 4H), 3.9-4.1 (m, 1H), 5.32 (d, 1H, J=6Hz), 7.1-7.3 (m, 5H), 8.29 (s, 1H), 8.41 (s,1H).
MS:404.25 [M-H]⁻

### Example 110

### (R)-2-(4-isopropylphenyl)-N-(1-(6-methoxy-5-(trifluoromethyl)pyridin-3-yl)pyrrolidin-3-yl)acetamide

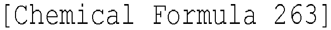

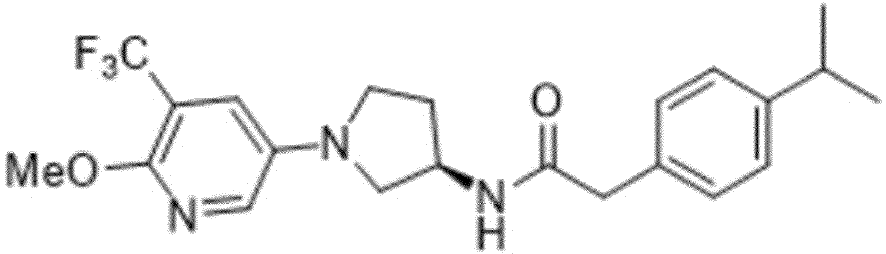

According to a technique similar to Example 9, the title compound (pale yellow crystals, 292 mg, 57%) was obtained using (R)-2-(4-isopropylphenyl)-N-(pyrrolidin-3-yl)acetamide (296 mg, 1.20 mmol) synthesized in Reference Example 63 and 5-bromo-2-methoxy-3-(trifluoromethyl)-pyridine (369 mg, 1.44 mmol).
¹H NMR (CDCl₃, 400MHz) : δ=1.23 (d, 6H, J=7Hz), 1.8-1.9 (m, 1H), 2.2-2.4 (m, 1H), 2.8-3.0 (m, 1H), 3.06 (dd, 1H, J=5, 10Hz), 3.2-3.4 (m, 2H), 3.5-3.6 (m, 3H), 3.95 (s, 3H), 4.5-4.7 (m, 1H), 5.53 (d, 1H, J=7Hz), 7.09 (d, 1H, J=3Hz), 7.16 (d, 2H, J=8Hz), 7.20 (d, 2H, J=8Hz), 7.59 (d, 1H, J=3Hz).
MS:420.26 [M-H]⁻

### Example 111

### (R)-2-(4-isopropylphenyl)-N-(1-(5-(trifluoromethyl)pyrimidin-2-yl)pyrrolidin-3-yl)acetamide

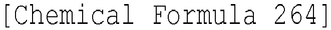

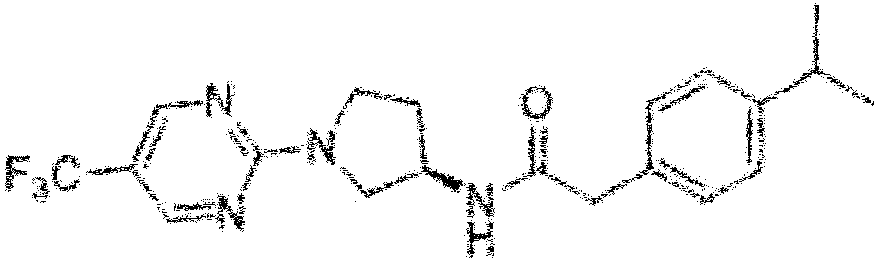

According to a technique similar to Example 27, the title compound (white crystals, 125 mg, 82%) was obtained using (R)-2-(4-isopropylphenyl)-N-(pyrrolidin-3-yl)acetamide (95 mg, 0.386 mmol) synthesized in Reference Example 63 and 2-chloro-5-(trifluoromethyl)pyrimidine (77 mg, 0.424 mmol).
¹H NMR (CDCl₃, 400MHz) : δ=1.23 (d, 6H, J=7Hz), 1.8-2.0 (m, 1H), 2.2-2.4 (m, 1H), 2.8-3.0 (m, 1H), 3.36 (dd, 1H, J=5, 12Hz), 3.55 (s, 2H), 3.63 (t, 2H, J=7Hz), 3.88 (dd, 1H, J=6, 12Hz), 4.5-4.7 (m, 1H), 5.50 (d, 1H, J=6Hz), 7.15 (d, 2H, J=8Hz), 7.19 (d, 2H, J=8Hz), 8.48 (s, 2H).
MS:391.23 [M-H]⁻

### Example 112

### (R)-2-(4-isopropylphenyl)-N-(1-(4-(trifluoromethyl)thiazol-2-yl)pyrrolidin-3-yl)acetamide

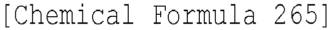

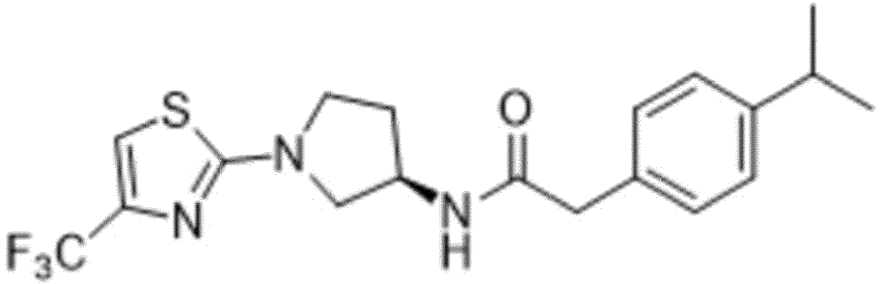

According to a technique similar to Example 9, the title compound (pale yellow crystals, 77 mg, 47%) was obtained using (R)-2-(4-isopropylphenyl)-N-(pyrrolidin-3-yl)acetamide (100 mg, 0.406 mmol) synthesized in Reference Example 63 and 2-bromo-4-(trifluoromethyl)thiazole (113 mg, 0.487 mmol).
¹H NMR (CDCl₃, 400MHz) : δ=1.24 (d, 6H, J=7Hz), 1.8-2.0 (m, 1H), 2.2-2.4 (m, 1H), 2.8-3.0 (m, 1H), 3.24 (dd, 1H, J=5, 11Hz), 3.4-3.6 (m, 4H), 3.75 (dd, 1H, J=6, 11Hz), 4.5-4.7 (m, 1H), 5.52 (d, 1H, J=6Hz), 6.92 (s, 1H), 7.14 (d, 2H, J=8Hz), 7.21 (d, 2H, J=8Hz).
MS:396.19 [M-H]⁻

### Example 113

### (R)-2-(4-isopropylphenyl)-N-(1-(6-(trifluoromethyl)-pyridin-3-yl)pyrrolidin-3-yl)acetamide

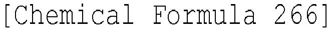

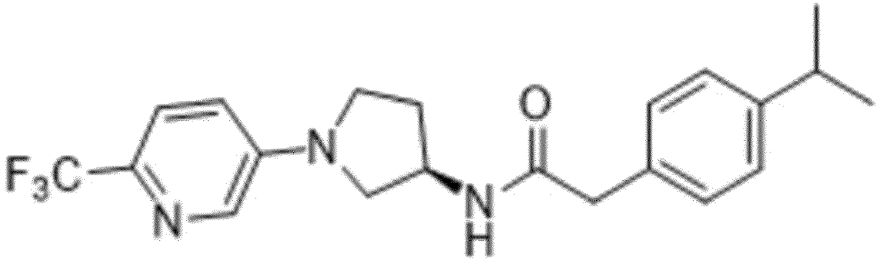

According to a technique similar to Example 9, the title compound (white crystals, 93 mg, 58%) was obtained using (R)-2-(4-isopropylphenyl)-N-(pyrrolidin-3-yl)acetamide (100 mg, 0.406 mmol) synthesized in Reference Example 63 and 5-bromo-2-(trifluoromethyl)pyridine (110 mg, 0.487 mmol).
¹H NMR (CDCl₃, 400MHz) : δ=1.23 (d, 6H, J=7Hz), 1.8-2.0 (m, 1H), 2.2-2.4 (m, 1H), 2.8-3.0 (m, 1H), 3.13 (dd, 1H, J=5, 10Hz), 3.38 (t, 2H, J=7Hz), 3.55 (s, 2H), 3.66 (dd, 1H, J=6, 10Hz), 4.5-4.7 (m, 1H), 5.57 (d, 1H, J=6Hz), 6.78 (dd, 1H, J=2, 9Hz), 7.16 (d, 2H, J=8Hz), 7.20 (d, 2H, J=8Hz), 7.46 (d, 1H, J=9Hz), 7.95 (d, 1H, J=2Hz).
MS:390.23 [M-H]⁻

### Example 114

### (S)-2-(4-isopropylphenyl)-N-(1-(5-(trifluoromethyl)-pyridin-3-yl)pyrrolidin-3-yl)acetamide

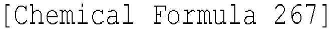

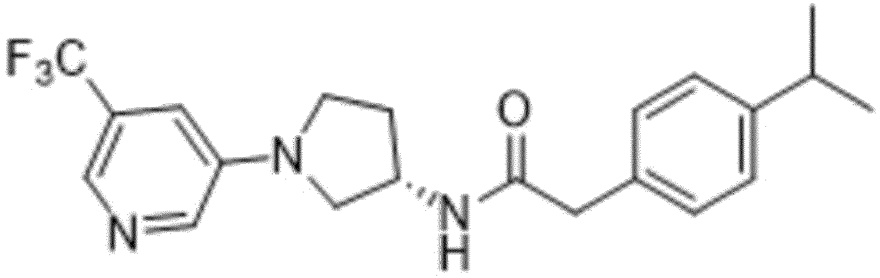

According to a technique similar to Example 1, the title compound (white crystals, 144 mg, 86%) was obtained using (S)-1-(5-(trifluoromethyl)pyridin-3-yl)pyrrolidin-3-amine (99 mg, 0.428 mmol) synthesized in Reference Example 64-2 and 2-(4-isopropylphenyl)acetic acid (92 mg, 0.514 mmol) .
¹H NMR (CDCl₃, 400MHz) : δ=1.23 (d, 6H, J=7Hz), 1.8-2.0 (m, 1H), 2.2-2.4 (m, 1H), 2.8-3.0 (m, 1H), 3.12 (dd, 1H, J=5, 10Hz), 3.36 (t, 2H, J=7Hz), 3.55 (s, 2H), 3.65 (dd, 1H, J=6, 10Hz), 4.5-4.7 (m, 1H), 5.61 (d, 1H, J=6Hz), 6.89 (s, 1H), 7.16 (d, 2H, J=8Hz), 7.20 (d, 2H, J=8Hz), 8.06 (d, 1H, J=3Hz), 8.19 (s, 1H).
MS:390.19 [M-H]⁻

### Example 115

### (R)-2-(4-(trifluoromethoxy)phenyl)-N-(1-(5-(trifluoromethyl)pyridin-3-yl)pyrrolidin-3-yl)acetamide

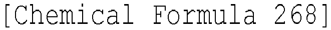

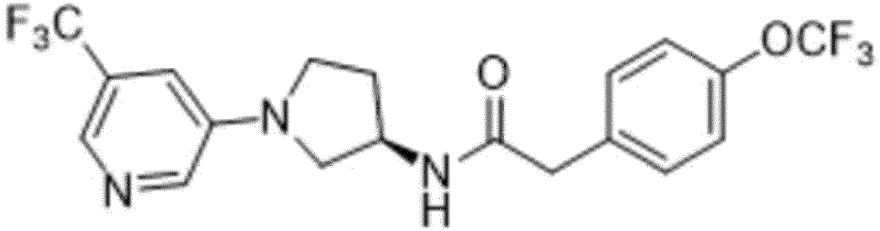

According to a technique similar to Example 1, the title compound (white crystals, 9 mg, 46%) was obtained using (R)-1-(5-(trifluoromethyl)pyridin-3-yl)pyrrolidin-3-amine (10 mg, 0.0432 mmol) synthesized in Reference Example 1-2 and 2-(4-(trifluoromethoxy)phenyl)acetic acid (11 mg, 0.0519 mmol).
¹H NMR (CDCl₃, 400MHz) :δ=1.9-2.1 (m, 1H), 2.3-2.4 (m, 1H), 3.18 (dd, 1H, J=4, 10Hz), 3.3-3.5 (m, 2H), 3.57 (s, 2H), 3.67 (dd, 1H, J=7, 10Hz), 4.6-4.7 (m, 1H), 5.65 (d, 1H, J=5Hz), 6.91 (s, 1H), 7.1-7.4 (m, 4H), 8.09 (s, 1H), 8.21 (s, 1H).
MS:432.14 [M-H]⁻

### Example 116

### (R)-2-(4-(2,2,2-trifluoroethoxy)phenyl)-N-(1-(5-(trifluoromethyl)pyridin-3-yl)pyrrolidin-3-yl)acetamide

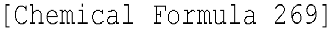

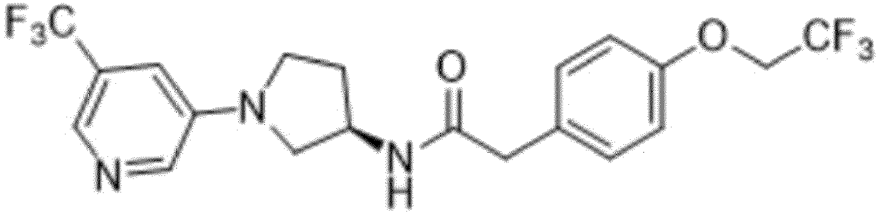

According to a technique similar to Example 1, the title compound (white crystals, 21 mg, 99%) was obtained using (R)-1-(5-(trifluoromethyl)pyridin-3-yl)pyrrolidin-3-amine (10 mg, 0.0432 mmol) synthesized in Reference Example 1-2 and 2-(4-(2,2,2-trifluoroethoxy)phenyl)acetic acid (12 mg, 0.0519 mmol).
¹H NMR (CDCl₃, 400MHz) : δ=1.8-2.0 (m, 1H), 2.2-2.4 (m, 1H), 3.14 (dd, 1H, J=5, 10Hz), 3.3-3.5 (m, 2H), 3.54 (s, 2H), 3.65 (dd, 1H, J=6, 10Hz), 4.34 (q, 2H, J=8Hz), 4.5-4.7 (m, 1H), 5.59 (d, 1H, J=6Hz), 6.8-7.0 (m, 3H), 7.21 (d, 2H, J=9Hz), 8.07 (d, 1H, J=2Hz), 8.20 (s, 1H).
MS:446.14 [M-H]⁻

### Example 117

### 2-(4-Isopropylphenyl)-N-(1-(5-(trifluoromethyl)pyridin-3-yl)azetidin-3-yl)acetamide

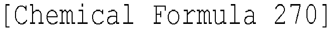

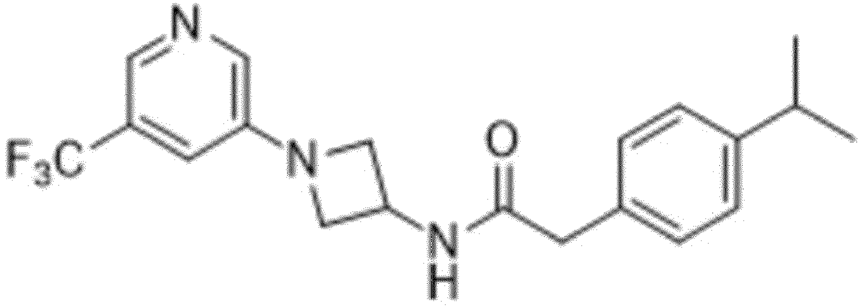

According to a technique similar to Example 36, a crude form of N-(azetidin-3-yl)-2-(4-isopropylphenyl)-acetamide (600 mg) was synthesized from tert-butyl 3-(2-(4-isopropylphenyl)acetamido)azetidine-1-carboxylate (964 mg, 2.90 mmol) synthesized in Reference Example 65, and the title compound (white crystals, 57 mg, 43%) was obtained using a portion of the crude form thus obtained (100 mg) and 3-bromo-5-(trifluoromethyl)pyridine (118 mg, 0.52 mmol) .
¹H NMR (DMSO-d₆, 400MHz) : δ=1.18 (d, 6H, J=7Hz), 2.7-2.9 (m, 1H), 3.38 (s, 2H), 3.7-3.8 (m, 2H), 4.2-4.3 (m, 2H), 4.5-4.7 (m, 1H), 7.1-7.2 (m, 5H), 8.10 (d, 1H, J=2Hz), 8.23 (s, 1H), 8.7-8.8 (m, 1H).
MS:378.21 [M+H]⁺

### Example 118

### 2-(4-Isopropylphenyl)-N-(1-(6-(trifluoromethyl)-pyridin-3-yl)azetidin-3-yl)acetamide

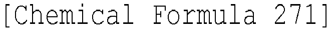

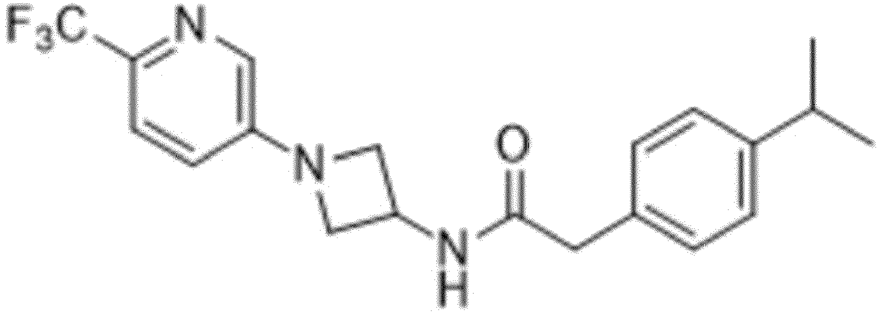

According to a technique similar to Example 36, the title compound (white crystals, 55 mg, 34%) was obtained using the crude form of N-(azetidin-3-yl)-2-(4-isopropylphenyl)acetamide (100 mg) synthesized from tert-butyl 3-(2-(4-isopropylphenyl)acetamido)azetidine-1-carboxylate in Example 117, and 5-bromo-2-(trifluoromethyl)pyridine (118 mg, 0.52 mmol).
¹H NMR (DMSO-d₆, 400MHz) : δ=1.17 (d, 6H, J=7Hz), 2.7-2.9 (m, 1H), 3.38 (s, 2H), 3.7-3.9 (m, 2H), 4.2-4.3 (m, 2H), 4.5-4.7 (m, 1H), 6.95 (dd, 1H, J=3, 8Hz), 7.17 (s, 4H), 7.61 (d, 1H, J=8Hz), 7.93 (d, 1H, J=3Hz), 8.7-8.8 (m, 1H).
MS:378.21 [M+H]⁺

### Example 119

### (R)-2-(3,5-dichlorophenyl)-N-(1-(5-(trifluoromethyl)pyridin-3-yl)pyrrolidin-3-yl)acetamide

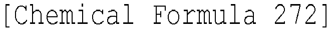

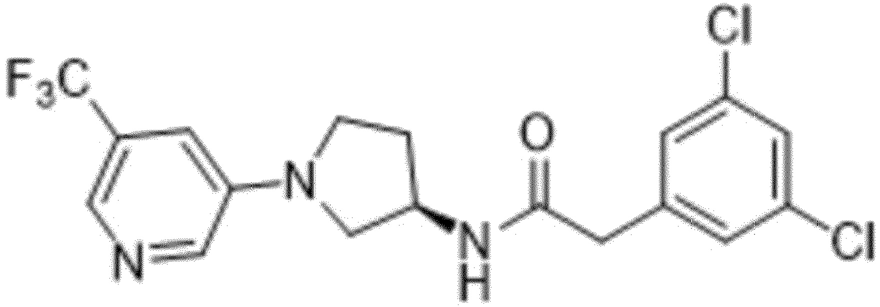

According to a technique similar to Example 1, the title compound (white crystals, 83 mg, 76%) was obtained using (R)-1-(5-(trifluoromethyl)pyridin-3-yl)pyrrolidin-3-amine (60 mg, 0.26 mmol) synthesized in Reference Example 1-2 and 2-(3,5-dichlorophenyl)acetic acid (64 mg, 0.31 mmol) .
¹H NMR (DMSO-d₆, 400MHz) :δ=1.8-2.0 (m, 1H), 2.1-2.3 (m, 1H), 3.1-3.7 (m, 4H), 3.46 (s, 2H), 4.3-4.5 (m, 1H), 7.11 (s, 1H), 7.30 (d, 2H, J=2Hz), 7.47 (t, 1H, J=2Hz), 8.14 (s, 1H), 8.20 (d, 1H, J=2Hz), 8.4-8.5 (m, 1H).
MS:416.13 [M-H]⁻

### Example 120

### (R)-2-(3-chloro-5-fluorophenyl)-N-(1-(5-(trifluoromethyl)pyridin-3-yl)pyrrolidin-3-yl)acetamide

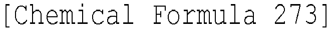

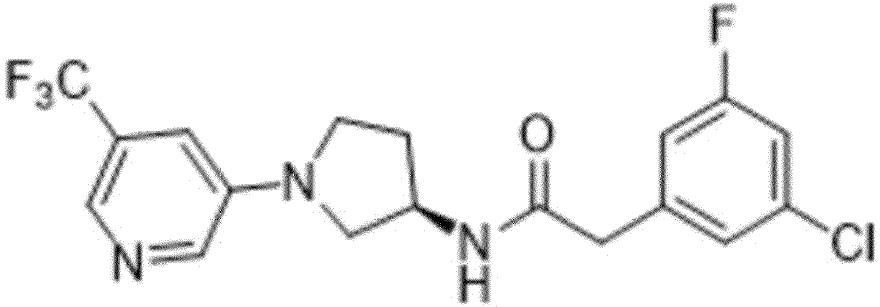

According to a technique similar to Example 1, the title compound (white crystals, 70 mg, 67%) was obtained using (R)-1-(5-(trifluoromethyl)pyridin-3-yl)pyrrolidin-3-amine (60 mg, 0.26 mmol) synthesized in Reference Example 1-2 and 2-(3-chloro-5-fluorophenyl)acetic acid (58 mg, 0.31 mmol) .
¹H NMR (DMSO-d₆, 400MHz) : δ=1.8-2.0 (m, 1H), 2.1-2.3 (m, 1H), 3.1-3.2 (m, 1H), 3.2-3.6 (m, 3H), 3.45 (s, 2H), 4.3-4.5 (m, 1H), 7.0-7.1 (m, 2H), 7.17 (s, 1H), 7.28 (dt, 1H, J=3,8Hz), 8.13 (s, 1H), 8.19 (d, 1H, J=3Hz), 8.4-8.5 (m, 1H).
MS:400.16[M-H]⁻

### Example 121

### (R)-2-(4-cyclopropylphenyl)-N-(1-(6-(trifluoromethyl)pyridin-3-yl)pyrrolidin-3-yl)acetamide

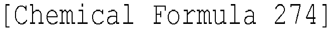

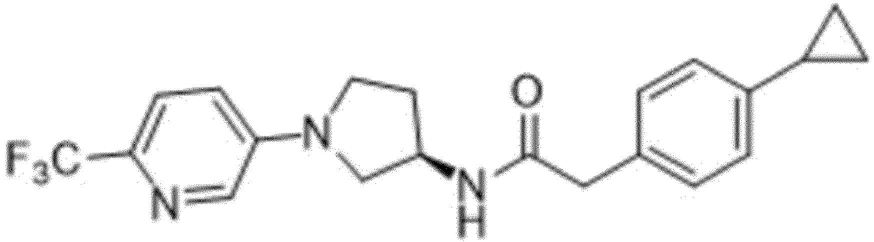

According to a technique similar to Example 1, the title compound (white crystals, 89 mg, 100%) was obtained using (R)-1-(6-(trifluoromethyl)pyridin-3-yl)pyrrolidin-3-amine (60 mg, 0.18 mmol) synthesized in Reference Example 32-2 and 2-(4-cyclopropylphenyl)acetic acid (51 mg, 0.29 mmol) synthesized in Reference Example 33-2.
¹H NMR (CDCl₃, 400MHz) : δ=0.6-0.7 (m, 2H), 0.9-1.0 (m, 2H), 1.8-2.0 (m, 2H), 2.2-2.4 (m, 1H), 3.0-3.2 (m, 1H), 3.3-3.4 (m, 2H), 3.53 (s, 2H),3.6-3.7 (m, 1H),4.5-4.7 (m, 1H),5.5-5.7 (m, 1H), 6.77 (dd, 1H, J=3,8Hz), 7.03 (d, 2H, J=8Hz), 7.11 (d, 2H, J=8Hz), 7.46 (d, 1H, J=8Hz), 7.94 (d, 1H, J=3Hz).
MS:388.22[M-H]⁻

### Example 122

### (R)-2-(4-isopropylphenyl)-N-(1-(5-(trifluoromethyl)-thiophen-2-yl)pyrrolidin-3-yl)acetamide

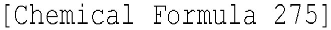

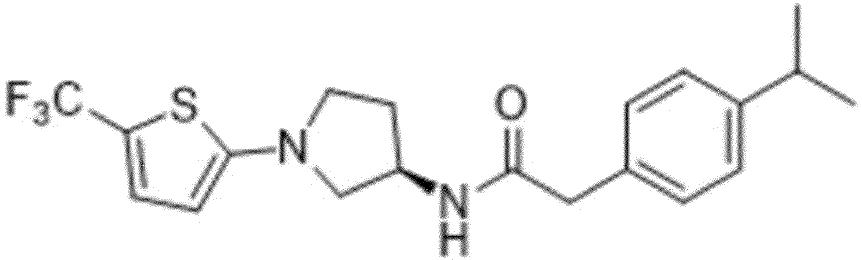

According to a technique similar to Example 9, the title compound (white crystals, 49 mg, 30%) was obtained using (R)-2-(4-isopropylphenyl)-N-(pyrrolidin-3-yl)acetamide (100 mg, 0.41 mmol) synthesized in Reference Example 63 and 2-bromo-5-(trifluoromethyl)thiophene (114 mg, 0.49 mmol).
¹H NMR (CDCl₃, 400MHz) : δ=1.23 (d, 6H, J=7Hz), 1.8-2.0 (m, 1H), 2.2-2.4 (m, 1H), 2.8-3.0 (m, 1H), 3.0-3.1 (m, 1H), 3.2-3.4 (m, 2H), 3.54 (s, 2H), 3.5-3.6 (m, 1H), 4.5-4.7 (m, 1H), 5.4-5.6 (m, 1H), 5.63 (d, 1H, J=4Hz), 7.0-7.2 (m, 1H), 7.14 (d, 2H, J=8Hz), 7.20 (d, 2H, J=8Hz).
MS:395.21[M-H]⁻

### Example 123

### (R)-2-(4-(trifluoromethyl)phenyl)-N-(1-(5-(trifluoromethyl)pyridin-3-yl)pyrrolidin-3-yl)acetamide

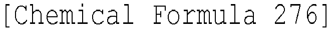

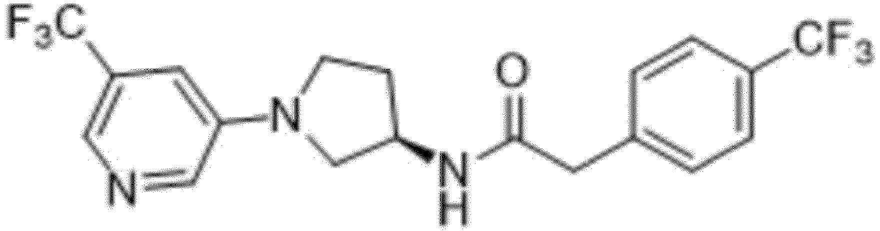

According to a technique similar to Example 1, the title compound (white crystals, 85 mg, 92%) was obtained using (R)-1-(5-(trifluoromethyl)pyridin-3-yl)pyrrolidin-3-amine (50 mg, 0.22 mmol) synthesized in Reference Example 1-2 and 2-(4-(trifluoromethyl)phenyl)acetic acid (53 mg, 0.26 mmol).
¹H NMR (CDCl₃, 400MHz) : δ=1.9-2.1 (m, 1H), 2.2-2.4 (m, 1H), 3.1-3.3 (m, 1H), 3.3-3.4 (m, 2H), 3.5-3.7 (m, 1H), 3.63 (s, 2H), 4.6-4.7 (m, 1H), 6.4-6.6 (m, 1H), 6.81 (s, 1H), 7.40 (d, 2H, J=8Hz), 7.59 (d, 2H, J=8Hz), 7.95 (d, 1H, J=2Hz), 8.09 (s, 1H).
MS:416.19[M-H]⁻

### Example 124

### (R)-2-(1H-indol-6-yl)-N-(1-(5-(trifluoromethyl)pyridin-3-yl)pyrrolidin-3-yl)acetamide

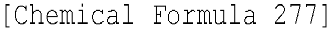

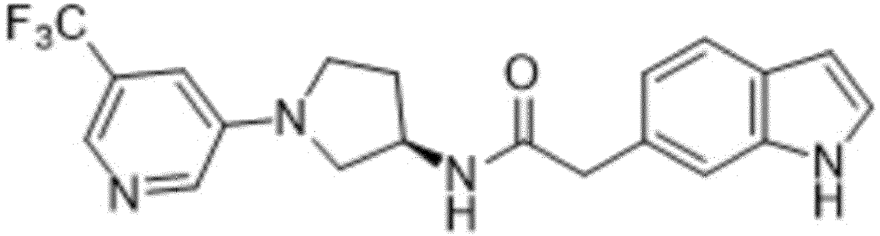

According to a technique similar to Example 1, the title compound (white crystals, 89 mg, 100%) was obtained using (R)-1-(5-(trifluoromethyl)pyridin-3-yl)pyrrolidin-3-amine (50 mg, 0.22 mmol) synthesized in Reference Example 1-2 and 2-(1H-indol-6-yl)acetic acid (53 mg, 0.26 mmol).
¹H NMR (CDCl₃, 400MHz) : δ=0.6-0.7 (m, 2H), 0.9-1.0 (m, 2H), 1.7-2.0 (m, 2H), 2.2-2.4 (m, 1H), 2.9-3.1 (m, 1H), 3.1-3.4 (m, 2H), 3.4-3.6 (m,1H), 3.52 (s, 2H), 4.5-4.7 (m, 1H), 4.67 (q, 2H, J=9Hz), 5.5-5.6 (m, 1H), 6.75 (d, 1H, J=9Hz), 6.92 (dd. 1H, J=3,9Hz), 7.03 (d, 2H, J=8Hz), 7.11 (d, 2H, J=8Hz), 7.39 (d, 1H, J=3Hz).
MS:387.22[M-H]⁻

### Example 125

### (R)-2-(4-cyclopropylphenyl)-N-(1-(6-(2,2,2-trifluoroethoxy)pyridin-3-yl)pyrrolidin-3-yl)acetamide

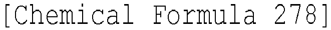

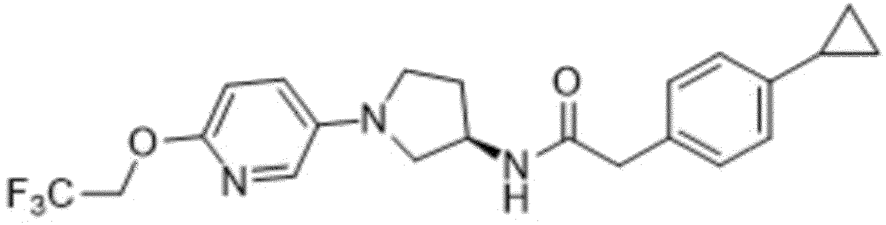

According to a technique similar to Example 9, the title compound (yellow amorphous, 40 mg, 45%) was obtained using (R)-2-(4-cyclopropylphenyl)-N-(pyrrolidin-3-yl)acetamide (50 mg, 0.20 mmol) synthesized in Reference Example 74-2 and 5-bromo-2-(2,2,2-trifluoroethoxy)pyridine (61 mg, 0.24 mmol).
¹H NMR (CDCl₃, 400MHz) : δ=δ=0.6-0.7 (m, 2H), 0.9-1.0 (m, 2H), 1.7-2.0 (m, 2H), 2.2-2.4 (m, 1H), 2.9-3.1 (m, 1H), 3.1-3.4 (m, 2H), 3.4-3.6 (m, 1H), 3.52 (s,2H), 4.5-4.7 (m, 1H), 4.67 (q, 2H, J=9Hz), 5.5-5.6 (m, 1H), 6.75 (d, 1H, J=9Hz), 6.92 (dd, 1H, J=3,9Hz), 7.03 (d, 2H, J=8Hz), 7.11 (d, 2H, J=8Hz), 7.39 (d, 1H, J=3Hz).
MS:420.16[M+H]⁺

### Example 126

### (R)-2-(4-cyclopropylphenyl)-N-(1-(5-(2,2,2-trifluoroethoxy)pyridin-3-yl)pyrrolidin-3-yl)acetamide

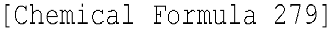

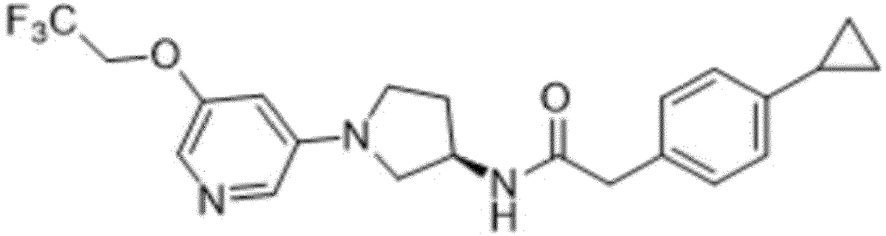

According to a technique similar to Example 9, the title compound (yellow crystals, 36 mg, 40%) was obtained using (R)-2-(4-cyclopropylphenyl)-N-(pyrrolidin-3-yl)acetamide (50 mg, 0.20 mmol) synthesized in Reference Example 74-2 and 3-bromo-5-(2,2,2-trifluoroethoxy)pyridine (61 mg, 0.24 mmol).
¹H NMRR (CDCl₃, 400MHz) : δ=0.6-0.7 (m, 2H), 0.9-1.0 (m, 2H), 1.8-2.0 (m, 2H), 2.2-2.4 (m, 1H), 3.0-3.1 (m, 1H), 3.31 (t, 2H, J=7Hz), 3.53 (s, 2H), 3.5-3.7 (m, 1H), 4.37 (q, 2H, J=8Hz), 4.5-4.7 (m, 1H), 5.5-5.7 (m, 1H), 6.33 (t, 1H, J=2Hz), 7.03 (d, 2H, J=8Hz), 7.11 (d, 2H, J=8Hz), 7.6-7.7 (m, 2H).
MS:420.16[M+H]⁺

### Example 127

### (R)-2-(4-cyclopropylphenyl)-N-(1-(4-(2,2,2-trifluoroethoxy)pyridin-2-yl)pyrrolidin-3-yl)acetamide

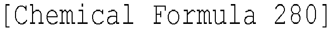

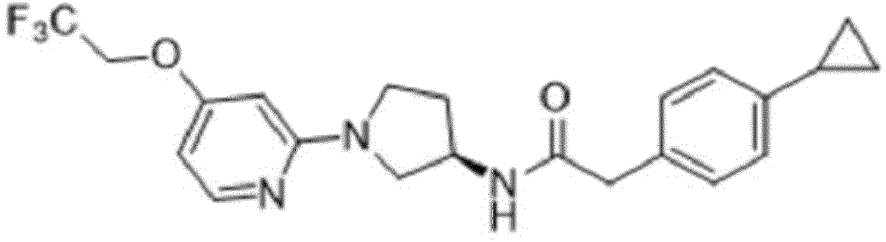

According to a technique similar to Example 27, the title compound (yellow amorphous, 7 mg, 6%) was obtained using (R)-2-(4-cyclopropylphenyl)-N-(pyrrolidin-3-yl)acetamide (50 mg, 0.20 mmol) synthesized in Reference Example 74-2 and 2-chloro-4-(2,2,2-trifluoroethoxy)pyridine (51 mg, 0.24 mmol).
¹H NMR (CDCl₃, 400MHz) : δ=0.6-0.7 (m, 2H), 0.9-1.0 (m, 2H), 1.8-1.9 (m, 2H), 2.2-2.3 (m, 1H), 3.1-3.3 (m, 1H), 3.4-3.5 (m, 2H), 3.52 (s, 2H), 3.6-3.8 (m, 1H), 4.35 (q, 2H, J=8Hz), 4.5-4.6 (m, 1H), 5.4-5.6 (m, 1H), 5.79 (d, 1H, J=2Hz), 6.20 (dd, 1H, J=2,6Hz), 7.03 (d, 2H, J=8Hz), 7.10 (d, 2H, J=8Hz), 8.02 (d, 1H, J=6Hz).
MS:420.16[M+H]⁺

### Example 128

### (R)-2-(4-cyclopropylphenyl)-N-(1-(2-methoxy-5-(trifluoromethyl)pyridin-3-yl)pyrrolidin-3-yl)acetamide

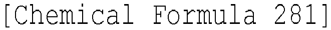

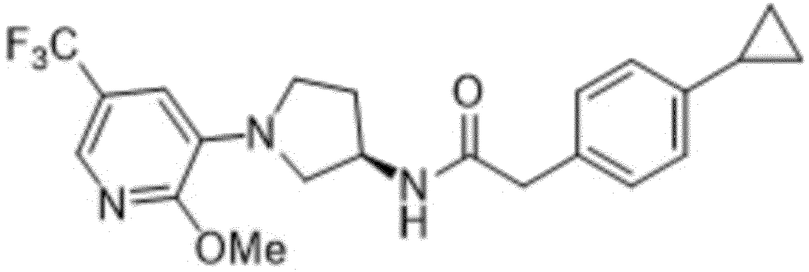

According to a technique similar to Example 9, the title compound (white crystals, 46 mg, 27%) was obtained using (R)-2-(4-cyclopropylphenyl)-N-(pyrrolidin-3-yl)acetamide (100 mg, 0.40 mmol) synthesized in Reference Example 74-2 and 3-bromo-2-methoxy-5-(trifluoromethyl)pyridine (123 mg, 0.48 mmol).
¹H NMR (CDCl₃, 400MHz) : δ=0.6-0.7 (m, 2H), 0.9-1.0 (m, 2H), 1.7-2.0 (m, 2H), 2.2-2.3 (m, 1H), 3.2-3.3 (m, 2H), 3.4-3.5 (m, 1H), 3.52 (s, 2H), 3.5-3.6 (m, 1H), 3.96 (s, 3H), 4.4-4.6 (m, 1H), 5.5-5.6 (m, 1H), 6.81 (d, 1H, J=2Hz), 7.03 (d, 2H, J=8Hz), 7.11 (d, 2H, J=8Hz), 7.86(d, 1H, J=2Hz).
MS:420.16[M+H]⁺

### Example 129

### (R)-2-(4-cyclopropylphenyl)-N-(1-(5-(trifluoromethyl)pyridin-3-yl)piperidin-3-yl)acetamide

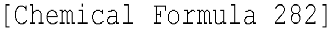

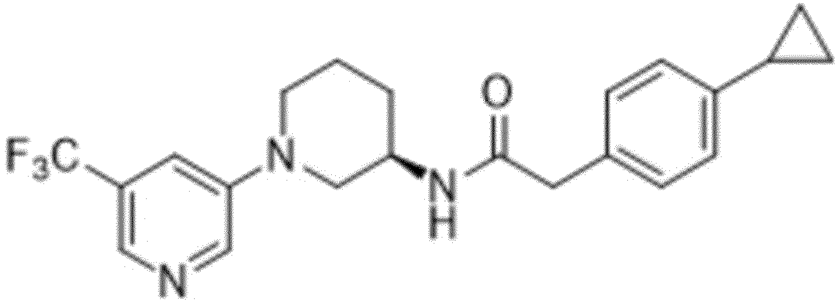

According to a technique similar to Example 3, a crude form of (R)-1-(5-(trifluoromethyl)pyridin-3-yl)piperidin-3-amine (70 mg) was synthesized from tert-butyl (R)-(1-(5-(trifluoromethyl)pyridin-3-yl)piperidin-3-yl)carbamate (100 mg, 0.29 mmol) synthesized in Reference Example 66, and the title compound (white crystals, 80 mg, 58%) was obtained using the crude form of (R)-1-(5-(trifluoromethyl)pyridin-3-yl)piperidin-3-amine thus obtained (70 mg) and 2-(4-cyclopropylphenyl)acetic acid (78 mg, 0.44 mmol) synthesized in Reference Example 33-2.
¹H NMR(DMSO-d₆, 400MHz): δ=0.5-0.7(m, 2H), 0.8-1.0(m, 2H), 1.4-1.6(m, 2H), 1.7-2.0(m, 3H), 2.7-2.9(m, 1H), 2.9-3.1(m, 1H), 3.2-3.5(m, 2H), 3.6-3.8(m, 3H), 6.97(d, 2H, J=8Hz), 7.12(d, 2H, J=8Hz), 7.52(s, 1H), 8.0-8.2(m, 1H), 8.24(s, 1H), 8.54 (d, 1H, J=3Hz) .
MS:404.17[M+H]⁺

### Example 130

### (R)-2-(4-cyclopropylphenyl)-N-(1-(6-(trifluoromethyl)pyridin-3-yl)piperidin-3-yl)acetamide

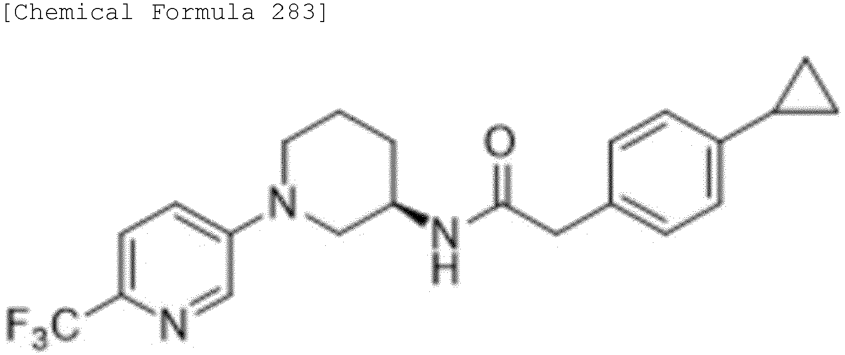

According to a technique similar to Example 3, a crude form of (R)-1-(6-(trifluoromethyl)pyridin-3-yl)piperidin-3-amine (280 mg) was synthesized from tert-butyl (R)-(1-(6-(trifluoromethyl)pyridin-3-yl)piperidin-3-yl)carbamate (400 mg, 1.16 mmol) synthesized in Reference Example 72, and the title compound (pale yellow crystals, 65 mg, 54%) was obtained using a portion of the crude form of (R)-1-(6-(trifluoromethyl)pyridin-3-yl)piperidin-3-amine (70 mg) thus obtained and 2-(4-cyclopropylphenyl)acetic acid (78 mg, 0.44 mmol) synthesized in Reference Example 33-2.
¹H NMR(DMSO-d₆, 400MHz):δ=0.5-0.7(m, 2H), 0.8-1.0(m, 2H), 1.4-1.6(m, 2H), 1.7-1.9(m, 3H), 2.8-3.0(m, 1H), 3.0-3.1(m, 1H), 3.2-3.5(m, 2H), 3.6-3.8(m, 3H), 6.97(d, 2H, J=8Hz), 7.11(d, 2H, J=8Hz), 7.37(dd, 1H, J=3,7Hz), 7.58(d, 1H, J=8Hz), 8.0-8.2(m, 1H), 8.37(d, 1H, J=3Hz).
MS:404.17[M+H]⁺

### Example 131

### (R)-2-(4-(2-hydroxypropan-2-yl)phenyl)-N-(1-(5-(trifluoromethyl)pyridin-3-yl)pyrrolidin-3-yl)acetamide

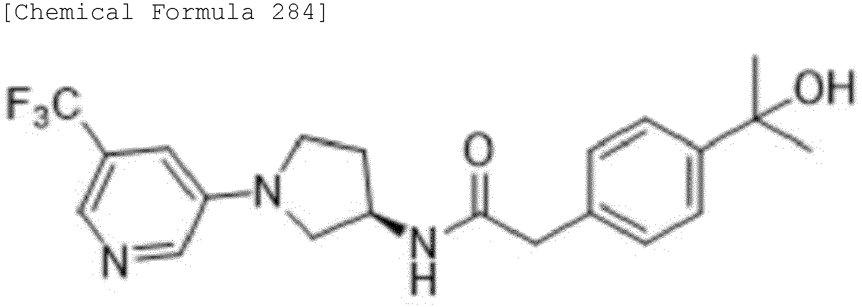

(R)-1-(5-(trifluoromethyl)pyridin-3-yl)pyrrolidin-3-amine (39 mg, 0.20 mmol) synthesized in Reference Example 76-2, 2-(4-(2-hydroxypropan-2-yl)phenyl)acetic acid (27 mg, 0.24 mmol), and DMT-MM (46 mg, 0.19 mmol) were dissolved in isopropanol (0.80 mL), and then the solution was stirred overnight at room temperature. Water was added to the reaction liquid, the mixture was stirred for a while, and then the mixture was extracted with ethyl acetate. An organic layer thus separated was dried over anhydrous sodium sulfate, insoluble materials were filtered, and then the solvent was distilled off under reduced pressure. A residue thus obtained was purified by silica gel column chromatography (chloroform : methanol) (concentration gradient: 0 to 40%), and the title compound (white amorphous, 57 mg, 99%) was obtained.
¹H NMR (CDCl₃, 400MHz) : δ=1. 58 (s, 3H), 1.59 (s, 3H), 1.7-1.9(br s, 1H), 1.9-2.0(m, 1H), 2.3-2.4(m, 1H), 3.1-3.2(m, 2H), 3.3-3.5(m, 2H), 3.58(s, 2H), 3.6-3.7(m, 1H), 4.6-4.7(m, 1H), 5.5-5.6(m, 1H), 6.92(s, 1H), 7.26(d, 1H, J=8Hz), 7.47(d, 1H, J=8Hz), 8.0-8.1(m, 1H), 8.21(s, 1H).
MS:430.14[M+Na]⁺

### Example 132

### (R)-2-(4-(3-methylpyrazin-2-yl)phenyl)-N-(1-(5-(trifluoromethyl)pyridin-3-yl)pyrrolidin-3-yl)acetamide

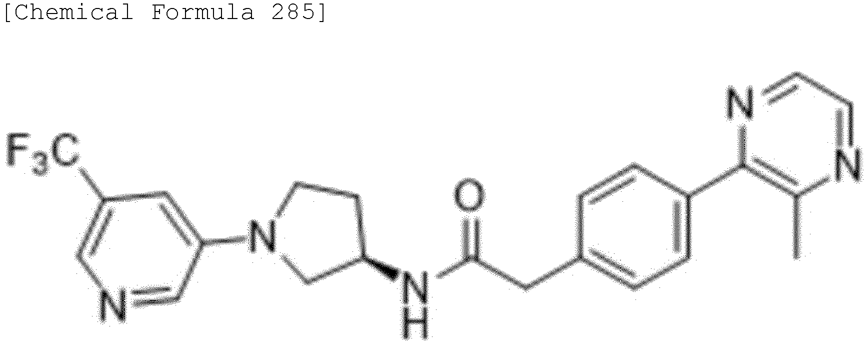

According to a technique similar to Example 1, the title compound (white crystals, 14 mg, 62%) was obtained using (R)-1-(5-(trifluoromethyl)pyridin-3-yl)pyrrolidin-3-amine (10 mg, 0.048 mmol) synthesized in Reference Example 1-2 and 2-(4-(3-methylpyrazin-2-yl)phenyl)acetic acid (10 mg, 0.044 mmol).
¹H NMR (CDCl₃, 400MHz) : δ=1.9-2.1 (m, 1H), 2.3-2.4 (m, 1H), 2.63(s, 3H), 3.1-3.2(m, 1H), 3.3-3.5(m, 2H), 3.6-3.7(m, 1H), 3.66(s, 2H), 4.6-4.7(m, 1H), 5.7-5.9(m, 1H), 6.90(s, 1H), 7.39(d, 2H, J=8H), 7.59(d, 2H, J=8Hz), 8.07(d, 1H, J=3Hz), 8.19(s, 1H), 8.46(d, 1H, J=2Hz), 8.48(d, 1H, J=2Hz).
MS:442.15[M+H]⁺

### Example 133

### (R)-2-(4-(4-methyloxazol-5-yl)phenyl)-N-(1-(5-(trifluoromethyl)pyridin-3-yl)pyrrolidin-3-yl)acetamide

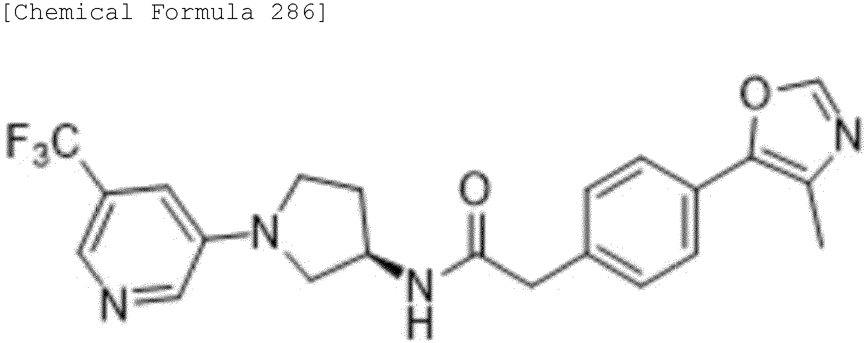

According to a technique similar to Example 1, the title compound (white crystals, 8.6 mg, 100%) was obtained using (R)-1-(5-(trifluoromethyl)pyridin-3-yl)pyrrolidin-3-amine (6 mg, 0.024 mmol) synthesized in Reference Example 1-2 and 2-(4-(4-methyloxazol-5-yl)phenyl)acetic acid (4 mg, 0.020 mmol).
¹H NMR (CDCl₃, 400MHz) : δ=1.9-2.0 (m, 1H), 2.3-2.4 (m, 1H), 2.44(s, 3H), 3.1-3.2(m, 1H), 3.3-3.5(m, 2H), 3.61(s, 2H), 3.6-3.7(m, 1H), 4.6-4.7(m, 1H), 5.4-5.6(m, 1H), 6.91(s, 1H), 7.34(d, 2H, J=8Hz)7.59(d, 2H, J=8Hz), 7.83(s, 1H), 8.08(s, 1H), 8.20(s, 1H).
MS:429.17[M-H]⁻

### Example 134

### (R)-3-(3-(2-(4-cyclopropylphenyl)acetamido)pyrrolidin-1-yl)-5-(trifluoromethyl)pyridine 1-oxide

### (3R)-3-(2-(4-cyclopropylphenyl)acetamido)-1-(5-(trifluoromethyl)pyridin-3-yl)pyrrolidine 1-oxide

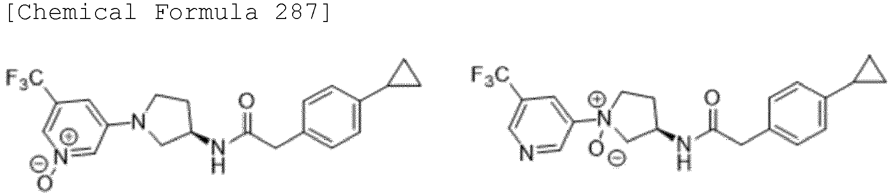

(R)-2-(4-cyclopropylphenyl)-N-(1-(5-(trifluoromethyl)pyridin-3-yl)pyrrolidin-3-yl)acetamide (85 mg, 0.22 mmol) synthesized in Example 1 was dissolved in dichloromethane (3.0 mL), m-CPBA (purity 65%, 58 mg, 0.22 mmol) was added thereto, and the mixture was stirred overnight at room temperature. The reaction liquid was washed with a saturated aqueous solution of sodium hydrogen carbonate, an organic layer thus separated was dried over anhydrous sodium sulfate, insoluble materials were filtered, and then the solvent was distilled off under reduced pressure. A residue thus obtained was purified by silica gel column chromatography (chloroform : methanol) (concentration gradient: 0 to 20%), and (R)-3-(3-(2-(4-cyclopropylphenyl)acetamido)pyrrolidin-1-yl)-5-(trifluoromethyl)pyridine 1-oxide (Rf value in TLC (dichloromethane : methanol = 10 : 1) = 0.4, pale yellow amorphous, 12 mg, 13%) and (3R)-3-(2-(4-cyclopropylphenyl)acetamido)-1-(5-(trifluoromethyl)pyridin-3-yl)pyrrolidine 1-oxide (Rf value in TLC (dichloromethane : methanol = 10 : 1) = 0.2, white amorphous, 42 mg, 47%) were obtained.

### (R)-3-(3-(2-(4-cyclopropylphenyl)-acetamido)pyrrolidin-1-yl)-5-(trifluoromethyl)pyridine 1-oxide

¹H NMR (CDCl₃, 400MHz) : δ=0.6-0.7 (m, 2H), 0.9-1.0 (m, 2H), 1.8-2.0(m, 2H), 2.2-2.4(m, 1H), 3.0-3.1(m, 1H), 3.31(t, 2H, J=7Hz), 3.54(s, 2H), 3.5-3.7(m, 1H), 4.5-4.7(m, 1H), 5.7-5.9(m, 1H), 6.54(s, 1H), 7.04(d ,2H, J=8Hz), 7.12(d, 2H, J=8Hz), 7.63(s, 1H), 7.81(s, 1H).
MS:406.14[M+H] ⁺

### (3R)-3-(2-(4-cyclopropylphenyl)acetamido)-1-(5-(trifluoromethyl)pyridin-3-yl)pyrrolidine 1-oxide

¹H NMR (CDCl₃, 400MHz) : δ=0.6-0.7 (m, 2H), 0.9-1.0 (m, 2H), 1.8-1.9(m, 1H), 2.4-2.6(m, 1H), 2.7-2.9(m, 1H), 3.50(s, 2H), 3.5-3.7(m, 1H), 3.8-4.0(m, 2H), 4.0-4.1(m, 1H), 5.1-5.3(m, 1H), 7.03(d, 2H, J=8Hz), 7.18(d, 2H, J=8Hz), 7.7-7.8(m, 1H), 8.82(s, 1H), 8.95(s, 1H), 9.15(s, 1H).
MS: 406.14 [M+H] ⁺

### Example 135

### (R)-2-(4-cyclopropylphenyl)-2-methyl-N-(1-(5-(trifluoromethyl)pyridin-3-yl)pyrrolidin-3-yl)propanamide

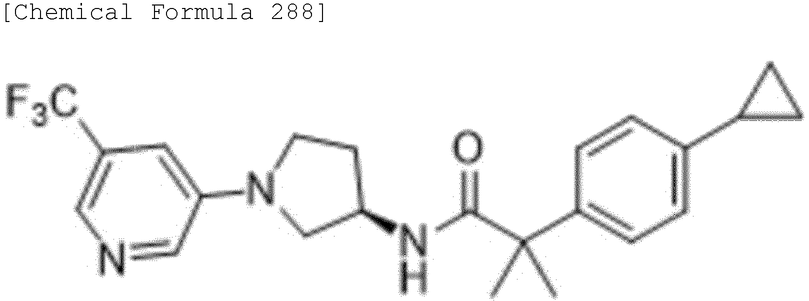

According to a technique similar to Example 1, the title compound (white amorphous, 45 mg, 94%) was obtained using (R)-1-(5-(trifluoromethyl)pyridin-3-yl)pyrrolidin-3-amine (25 mg, 0.11 mmol) synthesized in Reference Example 1-2 and 2-(4-cyclopropylphenyl)-2-methylpropanoic acid (23 mg, 0.11 mmol) .
¹H NMR (CDCl₃, 400MHz) : δ=0.6-0.7 (m, 2H), 0.9-1.0 (m, 2H), 1.54(s, 3H), 1.55(s, 3H), 1.7-1.9(m, 2H), 2.2-2.4(m, 1H), 3.0-3.1(m, 1H), 3.2-3.4(m, 2H), 3.6-3.7(m, 1H), 4.5-4.6(m,1H), 5.2-5.3(m, 1H), 6.88(s, 1H), 7.02(d, 2H, J=8Hz), 7.21(d, 2H, J=8Hz), 8.05(s, 1H), 8.19(s, 1H).
MS:416.21[M-H]⁻

### Example 136

### (R)-2-(4-cyclopropylphenyl)-N-(1-(3-fluoro-5-(trifluoromethyl)phenyl)pyrrolidin-3-yl) acetamide

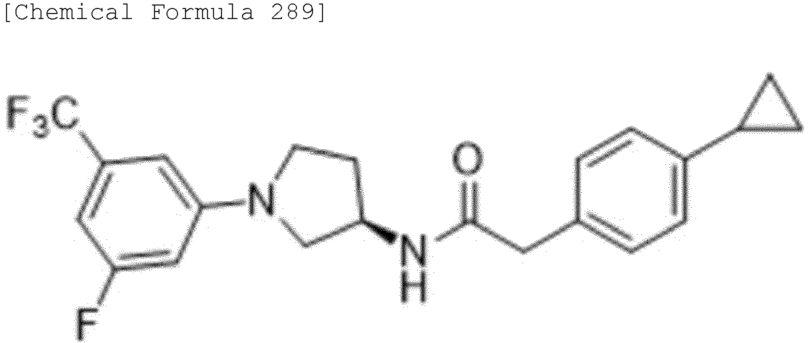

According to a technique similar to Example 9, the title compound (pale yellow amorphous, 34 mg, 49%) was obtained using (R)-2-(4-cyclopropylphenyl)-N-(pyrrolidin-3-yl)acetamide (42 mg, 0.17 mmol) synthesized in Reference Example 74-2 and 1-fluoro-3-iodo-5-(trifluoromethyl)benzene (50 mg, 0.17 mmol).
¹H NMR (CDCl₃, 400MHz) : δ=0.6-0.7 (m, 2H), 0.9-1.0 (m, 2H), 1.8-2.0(m, 2H), 2.2-2.4(m, 1H), 3.0-3.1(m, 1H), 3.2-3.4(m, 2H), 3.53(s, 2H), 3.5-3.7(m, 1H), 4.5-4.7(m, 1H), 5.4-5.6(m, 1H), 6.2-6.4(m, 1H), 6.47(s, 1H), 6.5-6.7(m, 1H), 7.04(d, 2H, J=8Hz), 7.11(d, 2H, J=8Hz).
MS:407.15[M+H]⁺

### Example 137

### 2-Amino-2-(4-cyclopropylphenyl)-N-((R)-1-(5-(trifluoromethyl)pyridin-3-yl)pyrrolidin-3-yl)acetamide

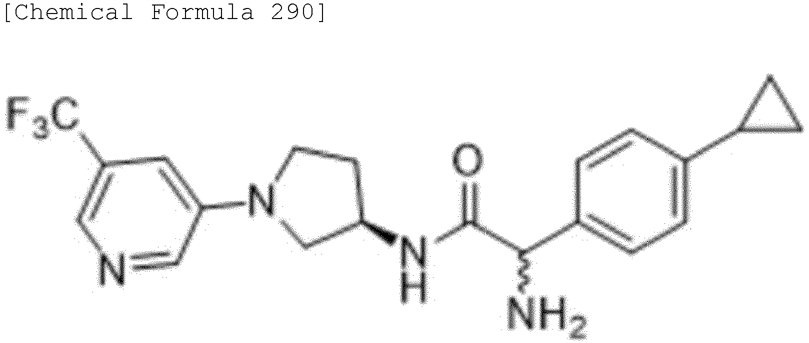

To diastereomer A of tert-butyl (1-(4-cyclopropylphenyl)-2-oxo-2-(((R)-1-(5-(trifluoromethyl)-pyridin-3-yl)pyrrolidin-3-yl)amino)ethyl)carbamate (15 mg, 0.030 mmol) synthesized in Reference Example 67, trifluoroacetic acid (0.50 mL) was added under ice cooling, and the mixture was stirred for 1 hour at room temperature. The solvent of the reaction liquid was distilled off under reduced pressure, a residue thus obtained was purified by silica gel column chromatography (chloroform : methanol) (concentration gradient: 0 to 20%), and diastereomer A of 2-amino-2-(4-cyclopropylphenyl)-N-((R)-1-(5-(trifluoromethyl)pyridin-3-yl)pyrrolidin-3-yl)acetamide (pale yellow amorphous, 3.8 mg, 9%) was obtained. According to a technique similar to that described above, diastereomer B of 2-amino-2-(4-cyclopropylphenyl)-N-((R)-1-(5-(trifluoromethyl)pyridin-3-yl)pyrrolidin-3-yl)acetamide (pale yellow amorphous, 3.4 mg, 8%) was obtained using diastereomer B of tert-butyl (1-(4-cyclopropylphenyl)-2-oxo-2-(((R)-1-(5-(trifluoromethyl)pyridin-3-yl)pyrrolidin-3-yl)amino)ethyl)carbamate (15 mg, 0.030 mmol) synthesized in Reference Example 67.

### Diastereomer A

¹H NMR (CDCl₃, 400MHz) : δ=0.6-0.8 (m, 2H), 0.9-1.0 (m, 2H), 1.2-1.3(br s, 2H), 1.8-2.0(m, 1H), 2.0-2.1(m, 1H), 2.3-2.5(m, 1H), 3.1-3.3(m, 1H), 3.3-3.6(m, 2H), 3.6-3.7(m, 1H), 4.49(s, 1H), 4.5-4.7(m, 1H), 6.94(s, 1H)7.01(d, 2H, J=8Hz), 7.21(d, 2H, J=8Hz), 7.4-7.5(m, 1H), 8.11(d, 1H, J=3Hz), 8.22(s, 1H).
MS:405.14[M+H] ⁺

### Diastereomer B

¹H NMR (CDCl₃, 400MHz) : δ=0.6-0.7 (m, 2H), 0.9-1.0 (m, 2H), 1.2-1.3(m, 2H), 1.8-1.9(m, 1H), 2.0-2.1(m, 1H), 2.3-2.4(m, 1H), 3.2-3.3(m, 1H), 3.3-3.6(m, 2H), 3.6-3.7(m, 1H), 4.50(s, 1H), 4.6-4.7(m, 1H), 6.95(s, 1H), 7.05(d, 2H, J=8Hz), 7.25(d, 2H, J=8Hz), 7.4-7.5(m, 1H), 8.13(d, 1H, J=3Hz), 8.22(s, 1H).
MS:405.14[M+H] ⁺

### Example 138

### 2-(4-Cyclopropylphenyl)-2-(dimethylamino)-N-((R)-1-(5-(trifluoromethyl)pyridin-3-yl)pyrrolidin-3-yl)acetamide hydrochloride

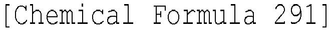

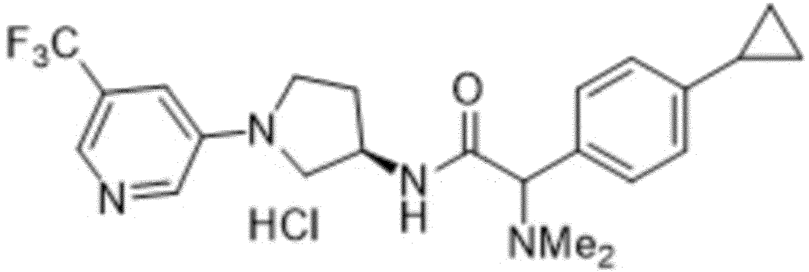

According to a technique similar to Example 137, a crude form of 2-amino-2-(4-cyclopropylphenyl)-N-((R)-1-(5-(trifluoromethyl)pyridin-3-yl)pyrrolidin-3-yl)acetamide (200 mg) was obtained using tert-butyl (1-(4-cyclopropylphenyl)-2-oxo-2-(((R)-1-(5-(trifluoromethyl)-pyridin-3-yl)amino)ethyl)carbamate (290 mg, 0.57 mmol) synthesized in Reference Example 67. A portion of the crude form of 2-amino-2-(4-cyclopropylphenyl)-N-((R)-1-(5-(trifluoromethyl)pyridin-3-yl)pyrrolidin-3-yl)acetamide (50 mg) thus obtained was dissolved in dichloromethane (1.0 mL), paraformaldehyde (11 mg, 0.36 mmol), acetic acid (14 µL, 0.24 mmol), and triacetoxyborohydride (127 mg, 0.6 mmol) were added thereto, and the mixture was stirred overnight at room temperature. The reaction liquid was diluted with ethyl acetate, an organic layer was washed with a saturated aqueous solution of sodium hydrogen carbonate, water, and saturated brine, subsequently the separated organic layer was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 10 to 70%), and 2-(4-cyclopropylphenyl)-2-(dimethylamino)-N-((R)-1-(5-(trifluoromethyl)pyridin-3-yl)pyrrolidin-3-yl)acetamide was obtained. 2-(4-Cyclopropylphenyl)-2-(dimethylamino)-N-((R)-1-(5-(trifluoromethyl)pyridin-3-yl)pyrrolidin-3-yl)acetamide thus obtained was dissolved in methanol (0.5 mL), subsequently a 1 N aqueous solution of hydrochloric acid (0.5 mL) was added thereto, and the solvent was distilled off under reduced pressure. Distilled water (0.1 mL) was added to a residue thus obtained, the residue was freeze-dried, and the title compound (yellow crystals, 10 mg, 19%) was obtained.
¹H NMR (DMSO-d₆, 400MHz) : δ=0.6-0.8 (m, 2H), 0.9-1.1 (m, 2H), 1.7-2.3(m,3H),2.50(s,3H),2.87(s,3H),3.0-3.8(m,4H), 4.3-4.5(m, 1H), 4.8-5.0(m, 1H), 7.12(d, 1H, J=8Hz), 7.18(d, 1H, J=8Hz), 7.2-7.4(m, 1H), 7.4-7.5(m, 2H), 8.1-8.3(m, 2H), 9.3-9.6(m, 1H), 10.3-10.5(m, 1H).
MS:433.18[M+H]⁺

### Example 139

### (R)-2-(1-methyl-1H-indol-5-yl)-N-(1-(5-(trifluoromethyl)pyridin-3-yl)pyrrolidin-3-yl)acetamide

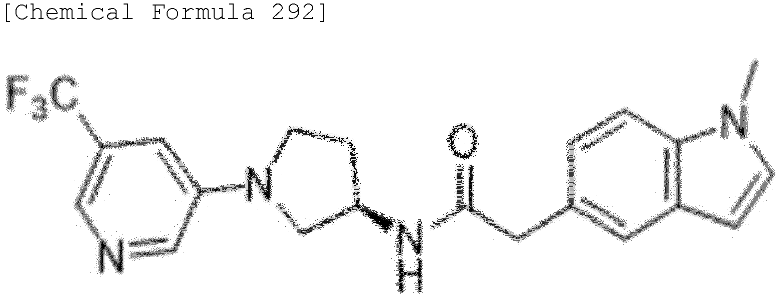

According to a technique similar to Example 1, the title compound (white crystals, 6 mg, 26%) was obtained using (R)-1-(5-(trifluoromethyl)pyridin-3-yl)pyrrolidin-3-amine (13 mg, 0.058 mmol) synthesized in Reference Example 1-2 and 2-(1-methyl-1H-indol-5-yl)acetic acid (10 mg, 0.053 mmol) .
¹H NMR (CDCl₃, 400MHz) :δ=1.7-1. 9 (m, 1H), 2.2-2.4 (m, 1H), 3.0-3.1(m, 1H), 3.2-3.4(m, 2H), 3.6-3.7(m, 1H), 3,70(s, 2H), 3.80(s, 3H), 4.5-4.7(m, 1H), 5.5-5.6(m, 1H), 6.44(d, 1H, J=6Hz), 6.86(s, 1H), 7.0-7.1(m, 2H), 7.29(d, 1H, J-9Hz), 7.46(d, 1H, J=1Hz), 8.03(d, 1H, J=3Hz), 8.17(s, 1H).
MS:403.13[M+H]⁺

### Example 140

### (R)-2-(2,4-bis(trifluoromethyl)phenyl)-N-(1-(5-(trifluoromethyl)pyridin-3-yl)pyrrolidin-3-yl)acetamide

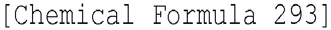

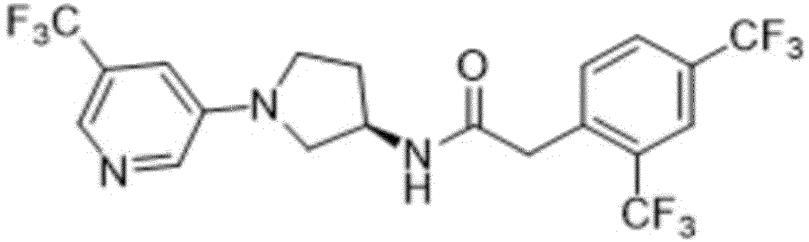

According to a technique similar to Example 1, the title compound (white crystals, 56 mg, 89%) was obtained using (R)-1-(5-(trifluoromethyl)pyridin-3-yl)pyrrolidin-3-amine (30 mg, 0.13 mmol) synthesized in Reference Example 1-2 and 2-(2,4-bis(trifluoromethyl)phenyl)acetic acid (44 mg, 0.16 mmol).
¹H NMR (CDCl₃, 400MHz) : δ=2.0-2.2 (m, 1H), 2.2-2.4 (m, 1H), 3.1-3.3(m, 1H), 3.3-3.5(m, 2H), 3.6-3.7(m, 1H), 3.79(s, 2H), 4.6-4.8(m, 1H), 6.5-6.6(m, 1H), 6.83(s, 1H), 7.68(d, 1H, J=8Hz), 7.82(d, 1H, J=8Hz), 7.91(s, 1H), 8.00(d, 1H, J=2Hz), 8.12(s, 1H).
MS:484.11[M-H]⁻

### Example 141

### (R)-2-(2-fluoro-4-(trifluoromethyl)phenyl)-N-(1-(5-(trifluoromethyl)pyridin-3-yl)pyrrolidin-3-yl)acetamide

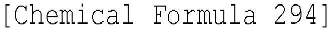

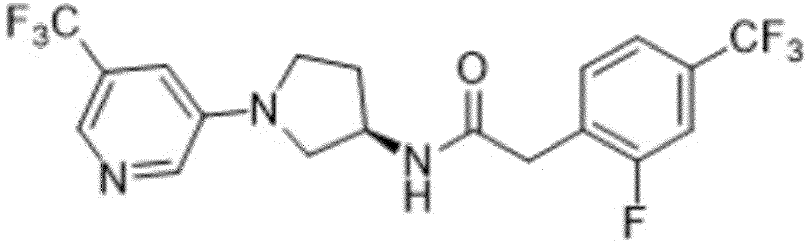

According to a technique similar to Example 1, the title compound (white crystals, 59 mg, 100%) was obtained using (R)-1-(5-(trifluoromethyl)pyridin-3-yl)pyrrolidin-3-amine (30 mg, 0.13 mmol) synthesized in Reference Example 1-2 and 2-(2-fluoro-4-(trifluoromethyl)phenyl)acetic acid (36 mg, 0.16 mmol).
¹H NMR (CDCl₃, 400MHz) : δ=2.0-2.1 (m, 1H), 2.2-2.4 (m, 1H), 3.1-3.3(m, 1H), 3.3-3.5(m, 2H), 3.6-3.7(m, 1H), 3.64(s, 2H), 4.6-4.8(m, 1H), 6.6-6.8(m, 1H), 6.82(s, 1H), 7.33(d, 1H, J=10Hz), 7.41(d, 1H, J=8Hz), 7.49(t, 1H, J=8Hz), 7.99(d, 1H, J=8Hz), 8.12(s, 1H).
MS:434.12[M-H]⁻

### Example 142

### (R)-2-(1-methyl-1H-indazol-5-yl)-N-(1-(5-(trifluoromethyl)pyridin-3-yl)pyrrolidin-3-yl)acetamide

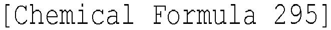

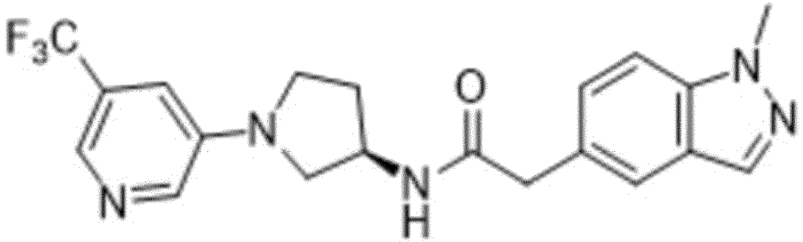

According to a technique similar to Example 1, the title compound (white crystals, 36 mg, 56%) was obtained using (R)-1-(5-(trifluoromethyl)pyridin-3-yl)pyrrolidin-3-amine (30 mg, 0.13 mmol) synthesized in Reference Example 1-2 and 2-(1-methyl-1H-indazol-5-yl)acetic acid (32 mg, 0.16 mmol).
¹H NMR (CDCl₃, 400MHz) : δ=1.8-2.0 (m, 1H), 2.2-2.4 (m, 1H), 3.0-3.2(m, 1H), 3.35(t, 2H, J=7Hz), 3.6-3.7(m, 1H), 3.69(s, 2H), 4.08(s, 3H), 4.6-4.7(m, 1H), 5.5-5.6(m, 1H), 6.87(s, 1H), 7.2-7.3(m, 1H), 7.38(d, 1H, J=9Hz), 7.59(s, 1H), 7.94(s,1H), 8.04(d, 1H, J=2Hz), 8.18(s, 1H).
MS:404.10[M+H]⁺

### Example 143

### (R)-2-(1-methyl-1H-indazol-5-yl)-N-(1-(4-(trifluoromethyl)thiazol-2-yl)pyrrolidin-3-yl)acetamide

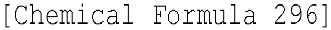

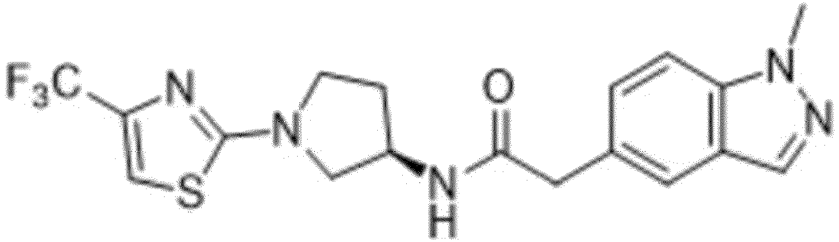

According to a technique similar to Example 1, the title compound (white crystals, 35 mg, 66%) was obtained using (R)-1-(4-(trifluoromethyl)thiazol-2-yl)pyrrolidin-3-amine (31 mg, 0.13 mmol) synthesized in Reference Example 12-2 and 2-(1-methyl-1H-indazol-5-yl)acetic acid (32 mg, 0.16 mmol).
¹H NMR (CDCl₃, 400MHz) : δ=1.8-2.0 (m, 1H), 2.2-2.4 (m, 1H), 3.1-3.3(m, 1H), 3.4-3.6(m, 2H), 3.68(s, 2H), 3.7-3.8(m, 1H), 4.09(s, 3H), 4.5-4.7(m, 1H), 5.4-5.6(m, 1H), 6.91(s, 1H), 7.2-7.3(m, 1H), 7.38(d, 1H, J=8Hz), 7.58(s, 1H), 7.95(s, 1H).
MS:410.06[M+H] ⁺

### Example 144

### (R)-2-(1-methyl-1H-indazol-5-yl)-N-(1-(6-(trifluoromethyl)pyridin-3-yl)pyrrolidin-3-yl)acetamide

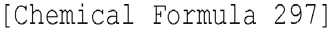

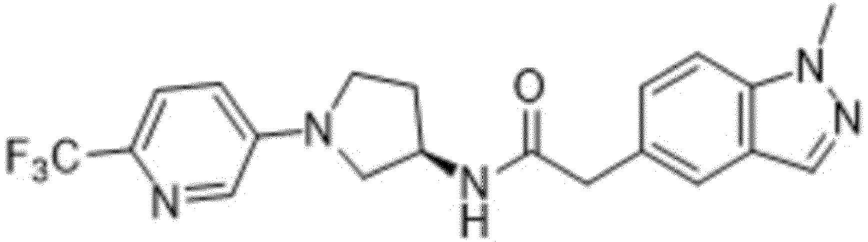

According to a technique similar to Example 1, the title compound (white crystals, 35 mg, 67%) was obtained using (R)-1-(6-(trifluoromethyl)pyridin-3-yl)pyrrolidin-3-amine (30 mg, 0.13 mmol) synthesized in Reference Example 32-2 and 2-(1-methyl-1H-indazol-5-yl)acetic acid (32 mg, 0.16 mmol).
¹H NMR (CDCl₃, 400MHz) : δ=1.8-2.0 (m, 1H), 2.2-2.4 (m, 1H), 3.0-3.2(m, 1H), 3.36(t, 2H, J=7Hz), 3.6-3.7(m, 1H), 3.69(s, 2H), 4.08(s, 3H), 4.5-4.7(m, 1H), 5.4-5.6(m, 1H), 6.76(dd, 1H, J=3,9Hz), 7.2-7.3(m, 1H), 7.37(d, 1H, J=9Hz), 7.45(d, 1H, J=8Hz), 7.59(s, 1H), 7.9-8.0 (m, 2H).
MS:404.10[M+H]⁺

### Example 145

### (R)-2-(4-(2,2-dimethylmorpholino)phenyl)-N-(1-(5-(trifluoromethyl)pyridin-3-yl)pyrrolidin-3-yl)acetamide

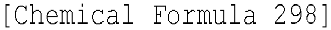

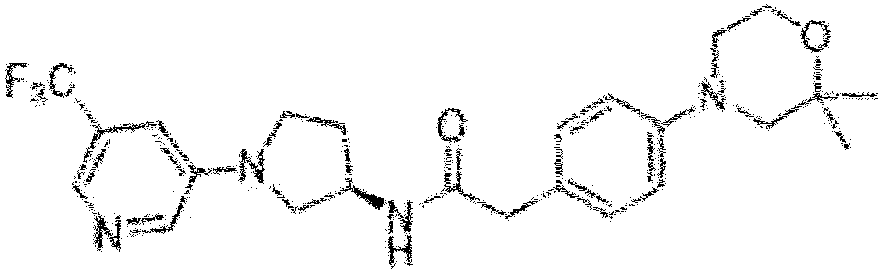

According to a technique similar to Example 9, the title compound (yellow amorphous, 2.1 mg, 5%) was obtained using (R)-2-(4-bromophenyl)-N-(1-(5-(trifluoromethyl)pyridin-3-yl)pyrrolidin-3-yl)acetamide (40 mg, 0.093 mmol) synthesized in Example 171 and 2,2-dimethylmorpholine (21 mg, 0.19 mmol).
¹H NMR (CDCl₃, 400MHz) : δ=1.32 (s, 6H), 1.8-2.0 (m, 1H), 2.3-2.4(m, 1H), 2.94(s, 2H), 3.0-3.2(m, 3H), 3.3-3.4(m, 2H), 3.51(s, 2H), 3.6-3.7(m, 1H)3.8-3.9(m, 2H), 4.5-4.7(m, 1H), 5.4-5.6(m, 1H), 6.85(d, 2H, J=8Hz), 6.94(s, 1H), 7.12(d, 2H, J=8Hz), 8.0-8.4(m, 2H).
MS:463.15[M+H]⁺

### Example 146

### (R)-2-(4-(1H-pyrazol-1-yl)phenyl)-N-(1-(5-(trifluoromethyl)pyridin-3-yl)pyrrolidin-3-yl)acetamide

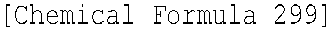

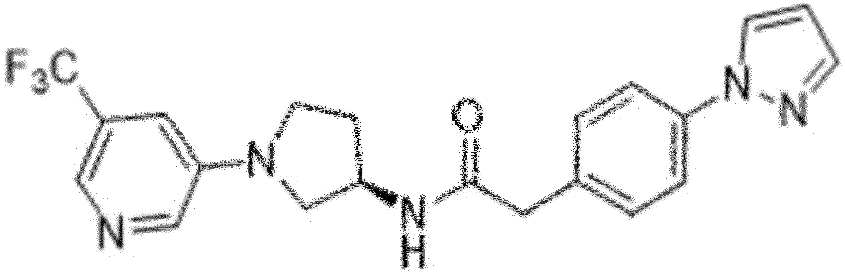

(R)-2-(4-bromophenyl)-N-(1-(5-(trifluoromethyl)-pyridin-3-yl)pyrrolidin-3-yl)acetamide (40 mg, 0.093 mmol) synthesized in Example 171, pyrazole (13 mg, 0.19 mmol), copper(I) iodide (2 mg, 9.3 µmmol), N,N'-dimethylethylenediamine (2 µL, 18.6 µmmol), and cesium carbonate (91 mg, 0.30 mmol) were suspended in DMF (1.0 mL), and the suspension was stirred for 2 hours at 200°C (microwaved). Ethyl acetate was added to the reaction liquid that had been left to cool to room temperature, and then the organic layer was washed with water and saturated brine. An organic layer thus separated was dried over anhydrous sodium sulfate, insoluble materials were filtered, and then the solvent was distilled off under reduced pressure. A residue thus obtained was purified by silica gel column chromatography (hexane : ethyl acetate) (concentration gradient: 0 to 100%), and the title compound (white crystals, 22 mg, 57%) was obtained.
¹H NMR (CDCl₃, 400MHz) : δ=1.9-2.0 (m, 1H), 2.3-2.4 (m, 1H), 3.1-3.2(m, 1H), 3.3-3.5(m, 2H), 3.62(s, 2H), 3.6-3.7(m, 1H), 4.6-4.7(m, 1H), 5.5-5.6(m, 1H), 6.4-6.5(m, 1H), 6.9-7.0(m, 1H), 7.35(d, 2H, J=9Hz), 7.69(d, 2H, J=9Hz), 7.73(d, 1H, J=1Hz), 7.92(d, 1H, J=1Hz), 8.0-8.1(m, 1H), 8.20(s, 1H).
MS:416.11 [M+H]⁺

### Example 147

### (R)-N-(3-methyl-1-(5-(trifluoromethyl)pyridin-3-yl)pyrrolidin-3-yl)-2-(4-morpholinophenyl)acetamide

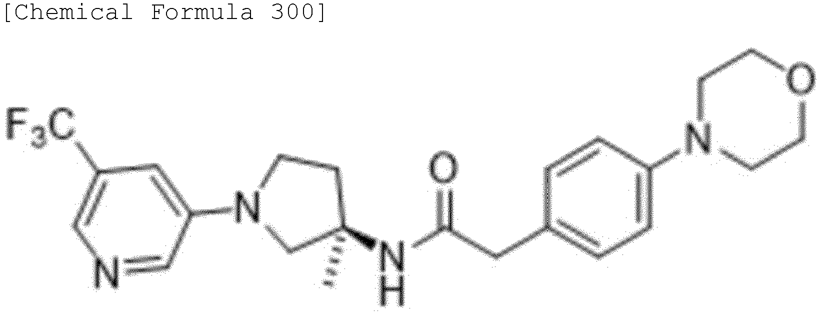

According to a technique similar to Example 3, a crude form of (R)-3-methyl-1-(5-(trifluoromethyl)pyridin-3-yl)pyrrolidin-3-amine (50 mg) was synthesized from tert-butyl (R)-(3-methyl-1-(5-(trifluoromethyl)pyridin-3-yl)pyrrolidin-3-yl)carbamate (47 mg, 0.23 mmol) synthesized in Reference Example 68, and the title compound (white crystals, 22 mg, 61%) was obtained using a portion of the crude form of (R)-3-methyl-1-(5-(trifluoromethyl)pyridin-3-yl)pyrrolidin-3-amine (20 mg) thus obtained and 2-(4-morpholinophenyl)acetic acid (30 mg, 0.12 mmol) synthesized in Reference Example 23-2.
¹H NMR (CDCl₃, 400MHz) : δ=1. 53(s, 3H), 1.9-2.1(m, 1H), 2.2-2.4(m, 1H), 3.13(t, 4H, J=4Hz), 3.2-3.4(m, 3H), 3.46(s, 2H), 3.6-3.7(m, 1H), 3.86(t, 4H, J=4Hz), 5.37(br s, 1H), 6.84(d, 2H, J=8Hz), 6.89(s, 1H), 7.09(d, 2H, J=8Hz), 8.06(s, 1H), 8.19(s, 1H).
MS:449.15[M+H]⁺

### Example 148

### (R)-2-(4-cyclopropylphenyl)-N-(3-methyl-1-(5-(trifluoromethyl)pyridin-3-yl)pyrrolidin-3-yl)acetamide

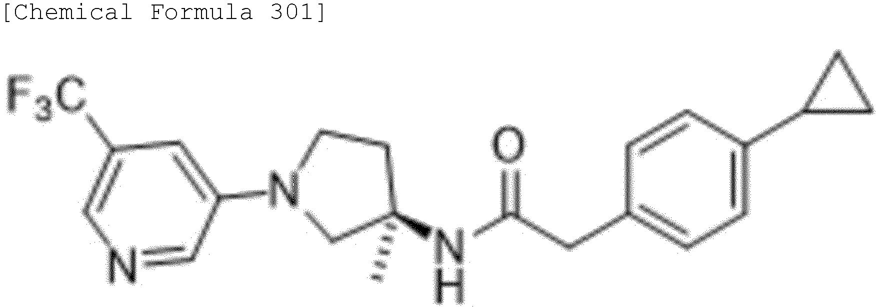

According to a technique similar to Example 3, a crude form of (R)-3-methyl-1-(5-(trifluoromethyl)pyridin-3-yl)pyrrolidin-3-amine (20 mg) was synthesized from tert-butyl (R)-(3-methyl-1-(5-(trifluoromethyl)pyridin-3-yl)pyrrolidin-3-yl)carbamate (47 mg, 0.23 mmol) synthesized in Reference Example 68, and the title compound (white amorphous, 12 mg, 100%) was obtained using a portion of the crude form of (R)-3-methyl-1-(5-(trifluoromethyl)pyridin-3-yl)pyrrolidin-3-amine (7 mg) thus obtained and 2-(4-cyclopropylphenyl)acetic acid (8 mg, 0.044 mmol) synthesized in Reference Example 33-2.
¹H NMR (CDCl₃, 400MHz) : δ=0.6-0.7 (m, 2H), 0.9-1.0 (m, 2H), 1.53(s, 3H), 1.8-1.9(m, 1H), 1.9-2.1(m, 1H), 2.2-2.4(m, 1H), 3.2-3.4(m, 3H), 3.48(s, 2H), 3.62(d, 1H, J=10Hz), 5.36(br s, 1H), 6.88(s, 1H), 7.00(d, 2H, J=8Hz), 7.07(d, 2H, J=8Hz), 8.05(d, 1H, J=2Hz), 8.19(s, 1H).
MS:404.12[M+H]⁺

### Example 149

### 2-(4-(3-Oxa-8-azabicyclo[3.2.1]octan-8-yl)phenyl)-N-((R)-1-(5-(trifluoromethyl)pyridin-3-yl)pyrrolidin-3-yl)acetamide

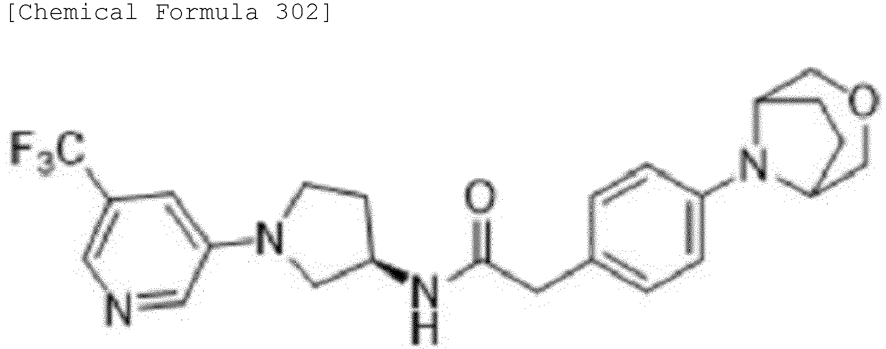

Ethyl 2-(4-(3-oxa-8-azabicyclo[3.2.1]octan-8-yl)phenyl)acetate (87 mg, 0.32 mmol) synthesized in Reference Example 69 was dissolved in ethanol (1.6 mL), an 8 N aqueous solution of sodium hydroxide (0.20 mL, 1.6 mmol) was added thereto, and the mixture was stirred for 2 hours at room temperature. A 1 N aqueous solution of hydrochloric acid (1.6 mL, 1.6 mmol) was added to the reaction liquid in an ice bath, and then the mixture was distilled off under reduced pressure. A mixed liquid (5 mL) of chloroform : methanol = 10 : 1 was added to a residue thus obtained, insoluble materials were filtered, subsequently the solvent was distilled off under reduced pressure, and a crude form of 2-(4-(3-oxa-8-azabicyclo[3.2.1]octan-8-yl)phenyl)acetic acid (white crystals) was obtained. Subsequently, according to a technique similar to Example 1, the title compound (pale yellow crystals, 50 mg, 71%) was obtained using (R)-1-(5-(trifluoromethyl)pyridin-3-yl)pyrrolidin-3-amine (35 mg, 0.15 mmol) synthesized in Reference Example 1-2 and a half of the crude form of 2-(4-(3-oxa-8-azabicyclo[3.2.1]octan-8-yl)phenyl)acetic acid synthesized as described above.
¹H NMR (CDCl₃, 400MHz) : δ=1.8-2.2 (m, 5H), 2.2-2.4 (m, 1H), 3.0-3.2(m, 1H), 3.37(t, 2H, J=7Hz), 3.4-3.6(m, 2H), 3.49(s, 2H), 3.6-3.7(m, 1H), 3.8-3.9(m, 2H), 4.0-4.1(m, 2H), 4.5-4.7(m, 1H), 5.5-5.6(m, 1H), 6.76(d, 2H, J=9Hz), 6.90(s, 1H), 7.10(d, 2H, J=9Hz)8.08(d, 1H, J=3Hz), 8.20(s, 1H).
MS:461.17[M+H]⁺

### Example 150

### 2-(4-(3-Oxa-8-azabicyclo[3.2.1]octan-8-yl)phenyl)-N-((R)-1-(6-(trifluoromethyl)pyridin-3-yl)pyrrolidin-3-yl)acetamide

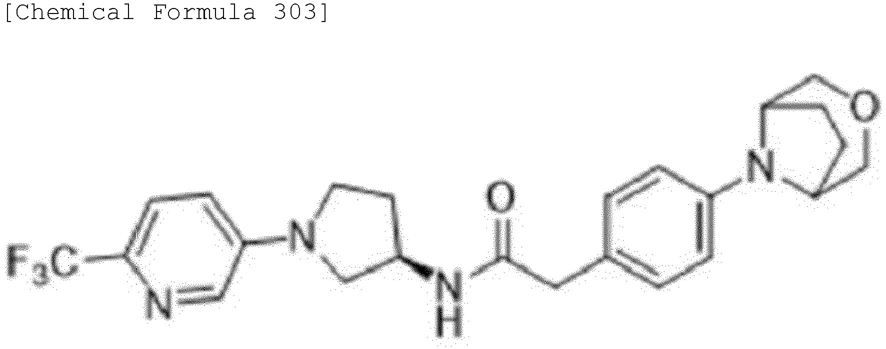

According to a technique similar to Example 149, a crude form of 2-(4-(3-oxa-8-azabicyclo[3.2.1]octan-8-yl)phenyl)acetic acid (white crystals) was obtained from ethyl 2-(4-(3-oxa-8-azabicyclo[3.2.1]octan-8-yl)phenyl)acetate (87 mg, 0.32 mmol) synthesized in Reference Example 69. The title compound (pale yellow crystals, 51 mg, 73%) was obtained using (R)-1-(6-(trifluoromethyl)pyridin-3-yl)pyrrolidin-3-amine (35 mg, 0.15 mmol) synthesized in Reference Example 32-2 and a half of the crude form of 2-(4-(3-oxa-8-azabicyclo[3.2.1]octan-8-yl)phenyl)acetic acid synthesized as described above.
¹H NMR (CDCl₃, 400MHz) : δ=1.8-2.2 (m, 5H), 2.2-2.4 (m, 1H), 3.0-3.2(m, 1H), 3.3-3.4(m, 2H), 3.4-3.6(m, 2H), 3.49(s, 2H), 3.6-3.8(m, 1H), 3.8-3.9(m, 2H), 4.0-4.1(m, 2H), 4.5-4.7(m, 1H), 5.5-5.6(m, 1H), 6.7-6.8(m, 3H), 7.08(d, 2H, J=8Hz), 7.46(d, 1H, J=8Hz), 7.96(d, 1H, J=3Hz).
MS:461.17[M+H]⁺

### Example 151

### (R)-2-(benzo[d]oxazol-5-yl)-N-(1-(6-(trifluoromethyl)pyridin-3-yl)pyrrolidin-3-yl)acetamide

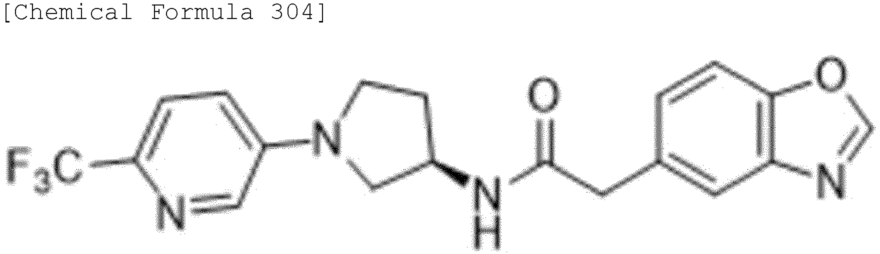

According to a technique similar to Example 1, the title compound (white crystals, 26 mg, 52%) was obtained using (R)-1-(6-(trifluoromethyl)pyridin-3-yl)pyrrolidin-3-amine (30 mg, 0.13 mmol) synthesized in Reference Example 32-2 and 2-(benzo[d]oxazol-5-yl)acetic acid (35 mg, 0.16 mmol) .
¹H NMR (CDCl₃, 400MHz) : δ=1.8-2.0 (m, 1H), 2.2-2.4 (m, 1H), 3.1-3.3(m, 1H), 3.3-3.5(m, 2H), 3.6-3.7(m, 1H), 3.70(s, 2H), 3.6-3.7(m, 1H), 5.5-5.7(m, 1H), 6.76(dd, 1H, J=3,9Hz), 7.30(dd, 1H, J=1,8Hz), 7.45(d, 1H, J=9Hz), 7.56(d, 1H, J=8Hz), 7.67(d, 1H, J=1Hz), 7.94(d, 1H, J=3Hz), 8.11(s, 1H).
MS:391.10[M+H] ⁺

### Example 152

### (R)-2-(benzo[d]oxazol-5-yl)-N-(1-(5-(trifluoromethyl)pyridin-3-yl)pyrrolidin-3-yl)acetamide

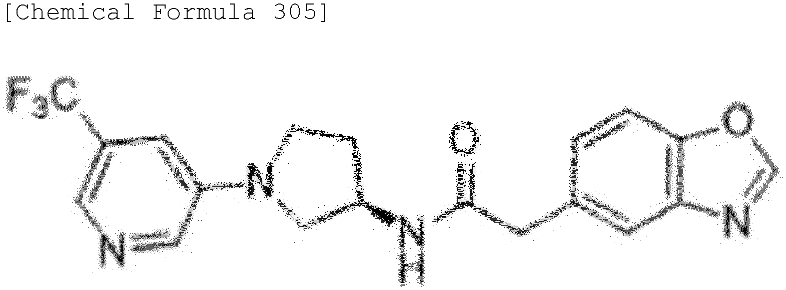

According to a technique similar to Example 1, the title compound (pale yellow crystals, 33 mg, 65%) was obtained using (R)-1-(5-(trifluoromethyl)pyridin-3-yl)pyrrolidin-3-amine (30 mg, 0.13 mmol) synthesized in Reference Example 1-2 and 2-(benzo[d]oxazol-5-yl)acetic acid (35 mg, 0.16 mmol).
¹H NMR (CDCl₃, 400MHz) : δ=1.8-2.0 (m, 1H), 2.2-2.4 (m, 1H), 3.0-3.2(m, 1H), 3.3-3.5(m, 2H), 3.6-3.7(m, 1H), 3.71(s, 2H), 3.6-3.7(m, 1H), 5.6-5.8(m, 1H)6.88(s, 1H), 7.31(dd, 1H, J=2,8Hz), 7.57(d, 1H, J=8Hz), 7.68(d, 1H, J=2Hz), 8.0-8.3(m ,2H), 8.12(s, 1H).
MS:391.10[M+H] ⁺

### Example 153

### (R)-2-(2-methylbenzo[d]oxazol-5-yl)-N-(1-(5-(trifluoromethyl)pyridin-3-yl)pyrrolidin-3-yl)acetamide

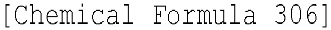

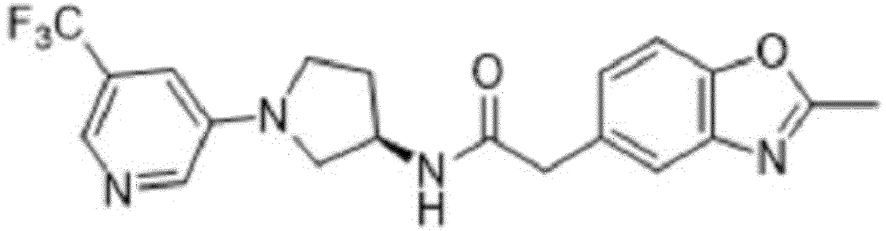

According to a technique similar to Example 1, the title compound (pale yellow crystals, 48 mg, 89%) was obtained using (R)-1-(5-(trifluoromethyl)pyridin-3-yl)pyrrolidin-3-amine (30 mg, 0.13 mmol) synthesized in Reference Example 1-2 and 2-(2-methylbenzo[d]oxazol-5-yl)acetic acid (35 mg, 0.16 mmol) synthesized as described above.
¹H NMR (CDCl₃, 400MHz) : δ=1.8-2.0 (m, 1H), 2.2-2.4 (m, 1H), 2.65(s, 3H), 3.0-3.2(m, 1H), 3.36(t, 2H, J=7Hz), 3.6-3.7(m, 1H), 3.89(s, 2H), 4.6-4.7(m, 1H), 5.5-5.7(m, 1H), 6.88(s, 1H), 7.19(dd, 1H, J=2,8Hz), 7.44(d, 1H, J=8Hz), 7.52(d, 1H, J=2Hz), 8.05(d, 1H, J=2Hz), 8.19(s, 1H).
MS:405.12[M+H] ⁺

### Example 154

### (R)-2-(2-methylbenzo[d]oxazol-5-yl)-N-(1-(6-(trifluoromethyl)pyridin-3-yl)pyrrolidin-3-yl)acetamide

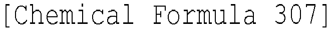

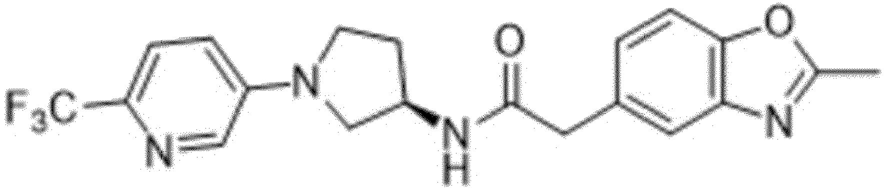

According to a technique similar to Example 1, the title compound (white crystals, 53 mg, 100%) was obtained using (R)-1-(6-(trifluoromethyl)pyridin-3-yl)pyrrolidin-3-amine (30 mg, 0.13 mmol) synthesized in Reference Example 32-2 and 2-(2-methylbenzo[d]oxazol-5-yl)acetic acid (31 mg, 0.16 mmol).
¹H NMR (CDCl₃, 400MHz) : δ=1.8-2.0 (m, 1H), 2.2-2.4 (m, 1H), 2.64(s, 3H), 3.0-3.2(m, 1H), 3.37(t, 2H, J=7Hz), 3.6-3.7(m, 1H), 3.68(s, 2H), 4.6-4.7(m, 1H), 5.6-5.8(m, 1H), 6.75(dd, 1H, J=3,9Hz), 7.19(dd, 1H, J=2,8Hz), 7.43(d, 1H, J=8Hz), 7.45(d, 1H, J=9Hz), 7.51(d, 1H, J=2Hz), 7.91(d, 1H, J=3Hz).
MS:405.11[M+H] ⁺

### Example 155

### 2-(4-((1S,4S)-2-oxa-5-azabicyclo[2.2.2]octan-5-yl)phenyl)-N-((R)-1-(5-(trifluoromethyl)pyridin-3-yl)pyrrolidin-3-yl)acetamide

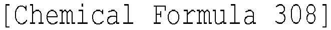

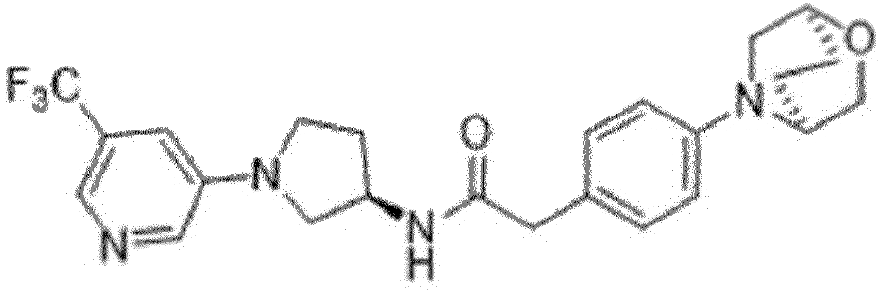

Ethyl 2-(4-((1S,4S)-2-oxa-5-azabicyclo[2.2.2]octan-5-yl)phenyl)acetate (112 mg, 0.41 mmol) synthesized in Reference Example 70 was dissolved in ethanol (2.0 mL), an 8 N aqueous solution of sodium hydroxide (0.30 mL, 2.4 mmol) was added thereto, and the mixture was stirred for 2 hours at room temperature. A 1 N aqueous solution of hydrochloric acid (2.4 mL, 2.4 mmol) was added to the reaction liquid in an ice bath, and then the mixture was distilled off under reduced pressure. A mixed liquid (5 mL) of chloroform : methanol = 10 : 1 was added to a residue thus obtained, insoluble materials were filtered, subsequently the solvent was distilled off under reduced pressure, and a crude form of 2-(4-((1S,4S)-2-oxa-5-azabicyclo[2.2.2]octan-5-yl)phenyl)acetic acid (white crystals, 84 mg) was obtained. Subsequently, according to a technique similar to Example 131, the title compound (pale yellow crystals, 40 mg, 47%) was obtained using (R)-1-(5-(trifluoromethyl)pyridin-3-yl)pyrrolidin-3-amine (42 mg, 0.18 mmol) synthesized in Reference Example 1-2 and a portion of the crude form of 2-(4-((1S,4S)-2-oxa-5-azabicyclo[2.2.2]octan-5-yl)phenyl)acetic acid (41 mg) synthesized as described above.
¹H NMR (CDCl₃, 400MHz) : δ=1.6-1.8 (m, 1H), 1.8-2.0 (m, 2H), 2.0-2.3(m, 2H), 2.3-2.4(m, 1H), 3.0-3.2(m, 1H), 3.2-3.4(m, 3H), 3.49(s, 2H), 3.6-3.7(m, 1H), 3.7-3.9(m, 2H), 4.0-4.1(m, 2H), 4.1-4.2(m, 1H), 4.5-4.7(m, 1H), 5.5-5.6(m, 1H), 6.60(d, 2H, J=8Hz), 6.91(s, 1H), 7.09(d, 2H, J=8Hz), 8.08(s, 1H), 8.20(s, 1H).
MS:461.17[M+H]⁺

### Example 156

### 2-(4-((1S,4S)-2-oxa-5-azabicyclo[2.2.2]octan-5-yl)phenyl)-N-((R)-1-(6-(trifluoromethyl)pyridin-3-yl)pyrrolidin-3-yl)acetamide

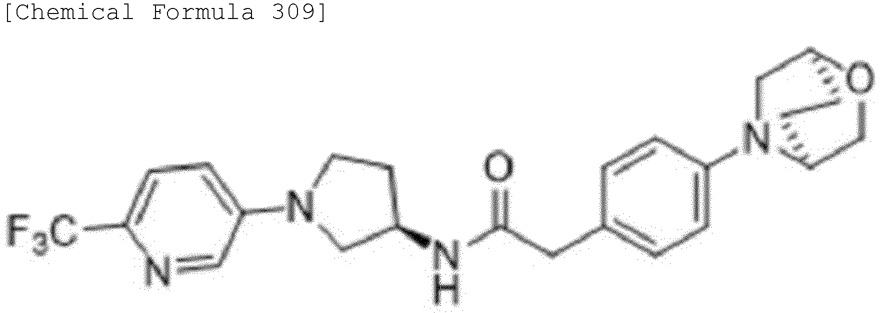

According to a technique similar to Example 155, a crude form of 2-(4-((1S,4S)-2-oxa-5-azabicyclo[2.2.2]octan-5-yl)phenyl)acetic acid (white crystals, 84 mg) was obtained from the crude form of ethyl 2-(4-((1S,4S)-2-oxa-5-azabicyclo[2.2.2]octan-5-yl)phenyl)acetate (112 mg, 0.41 mmol) synthesized in Reference Example 70. Subsequently, the title compound (white crystals, 16 mg, 18%) was obtained using (R)-1-(6-(trifluoromethyl)pyridin-3-yl)pyrrolidin-3-amine (42 mg, 0.18 mmol) synthesized in Reference Example 32-2 and a portion of the crude form of 2-(4-((1S,4S)-2-oxa-5-azabicyclo[2.2.2]octan-5-yl)phenyl)acetic acid (41 mg) synthesized as described above.
¹H NMR (CDCl₃, 400MHz) :δ=1.6-1.8 (m, 1H), 1.8-2.0 (m, 2H), 2.0-2.3(m, 2H), 2.3-2.4(m, 1H), 3.0-3.2(m, 1H), 3.3-3.5(m, 3H), 3.49(s, 2H), 3.6-3.7(m, 1H), 3.7-3.9(m, 2H), 4.0-4.1(m, 2H), 4.1-4.2(m, 1H), 4.5-4.7(m, 1H), 5.5-5.6(m, 1H), 6.59(d, 2H, J=8Hz), 6.78(dd, 1H, J=3,9Hz), 7.08(d, 2H, J=8Hz), 7.47(d, 1H, J=9Hz), 7.99(s, 1H).
MS:461.17[M+H]⁺

### Example 157

### (R)-2-(benzo[d]thiazol-6-yl)-N-(1-(5-(trifluoromethyl)pyridin-3-yl)pyrrolidin-3-yl)acetamide

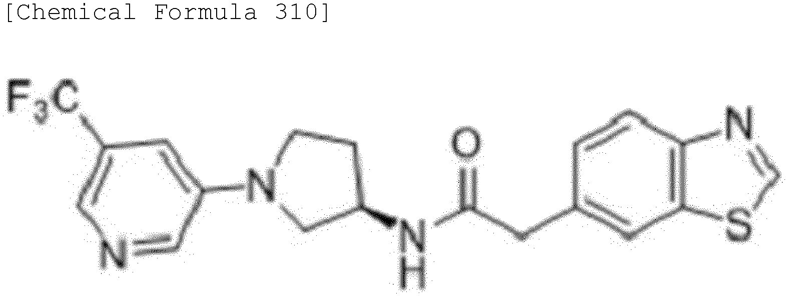

According to a technique similar to Example 1, the title compound (pale yellow amorphous, 33 mg, 61%) was obtained using (R)-1-(5-(trifluoromethyl)pyridin-3-yl)pyrrolidin-3-amine (30 mg, 0.13 mmol) synthesized in Reference Example 1-2 and 2-(benzo[d]thiazol-6-yl)acetic acid (31 mg, 0.16 mmol).
¹H NMR (CDCl₃, 400MHz) : δ=1.9-2.0 (m, 1H), 2.3-2.4 (m, 1H), 3.1-3.2(m, 1H), 3.3-3.5(m, 2H), 3.6-3.7(m, 1H), 3.74(s, 2H), 4.6-4.7(m, 1H), 5.6-5.8(m, 1H), 6.90(s, 1H), 7.41(dd, 1H, J=2,8Hz), 7.89(d, 1H, J=2Hz), 8.0-8.1(m, 1H), 8.11(d, 1H, J=8Hz), 8.19(s, 1H), 9.00(s, 1H).
MS:407.08[M+H]⁺

### Example 158

### (R)-2-(benzo[d]thiazol-6-yl)-N-(1-(6-(trifluoromethyl)pyridin-3-yl)pyrrolidin-3-yl)acetamide

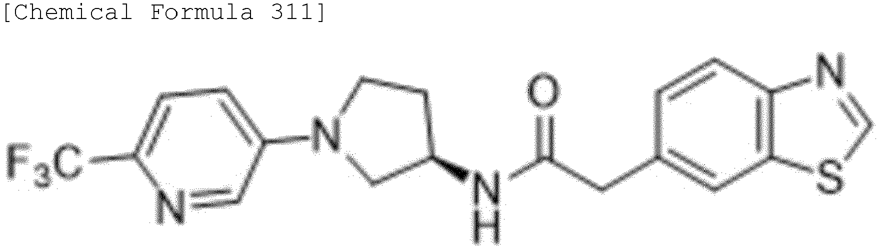

According to a technique similar to Example 1, the title compound (white crystals, 20 mg, 36%) was obtained using (R)-1-(6-(trifluoromethyl)pyridin-3-yl)pyrrolidin-3-amine (30 mg, 0.13 mmol) synthesized in Reference Example 32-2 and 2-(benzo[d]thiazol-6-yl)acetic acid (31 mg, 0.16 mmol) .
¹H NMR (CDCl₃, 400MHz) : δ=1.9-2.0 (m, 1H), 2.3-2.4 (m, 1H), 3.1-3.2(m, 1H), 3.3-3.5(m, 2H), 3.6-3.7(m, 1H), 3.73(s, 2H), 4.6-4.7(m, 1H), 5.7-5.8(m, 1H), 6.76(dd, 1H, J=2,8Hz), 7.40(d, 1H, J=2Hz), 7.45(d, 1H, J=8Hz), 7.88(s, 1H), 7.91(d, 1H, J=3Hz), 8.10(d, 1H, J=8Hz), 9.00(s, 1H).
MS:429.05[M+Na]⁺

### Example 159

### (R)-2-(1H-indazol-5-yl)-N-(1-(5-(trifluoromethyl)-pyridin-3-yl)pyrrolidin-3-yl)acetamide

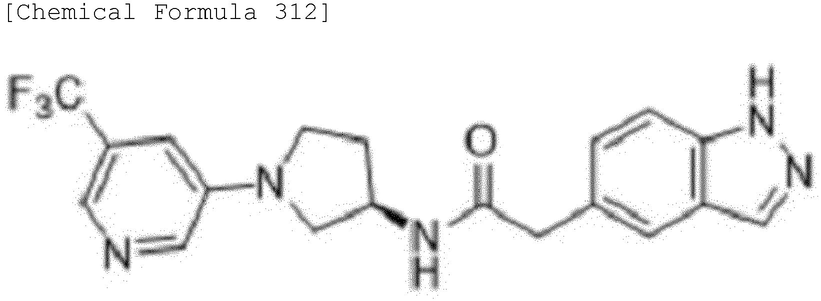

According to a technique similar to Example 1, the title compound (white crystals, 59 mg, 57%) was obtained using (R)-1-(5-(trifluoromethyl)pyridin-3-yl)pyrrolidin-3-amine (60 mg, 0.26 mmol) synthesized in Reference Example 1-2 and 2-(1H-indazol-5-yl)acetic acid (56 mg, 0.32 mmol).
¹H NMR (DMSO-d₆, 400MHz) : δ=1.8-2.0 (m, 1H), 2.1-2.3(m, 1H), 3.1-3.3(m, 1H), 3.3-3.7(m, 4H), 3.49(s, 2H), 4.39(br s, 1H), 7.11(s, 1H), 7.25(d, 1H, J=8Hz), 7.45(d, 1H, J=8Hz), 7.59(s, 1H), 8.00(s, 1H), 8.15(s, 1H), 8.21(s, 1H), 8.3-8.5(m, 1H).
MS:390.12[M+H]⁺

### Example 160

### (R)-2-(1H-indazol-5-yl)-N-(1-(6-(trifluoromethyl)-pyridin-3-yl)pyrrolidin-3-yl)acetamide

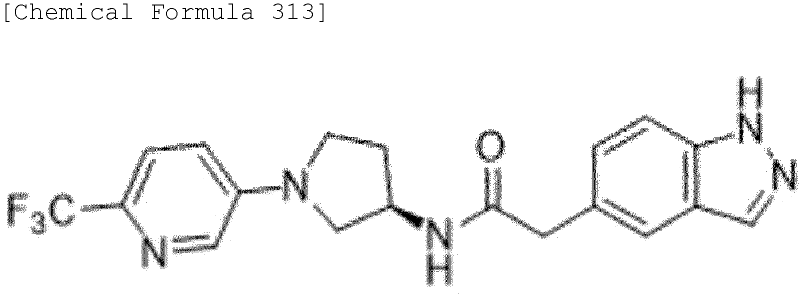

According to a technique similar to Example 1, the title compound (white crystals, 55 mg, 53%) was obtained using (R)-1-(6-(trifluoromethyl)pyridin-3-yl)pyrrolidin-3-amine (60 mg, 0.26 mmol) synthesized in Reference Example 32-2 and 2-(1H-indazol-5-yl)acetic acid (56 mg, 0.32 mmol).
¹H NMR (DMSO-d₆, 400MHz) : δ=1.8-2.0 (m, 1H), 2.1-2.3(m, 1H), 3.1-3.5(m, 3H), 3.49(s, 2H), 3.5-3.7(m, 1H), 4.3-4.5(m, 1H), 7.01(dd, 1H, J=3,8Hz), 7.24(d, 1H, J=8Hz), 7.45(d, 1H, J=8Hz), 7.5-7.7(m, 2H), 7.99(s, 1H), 8.04(d, 2H, J=3Hz), 8.3-8.5(m, 1H).
MS:390.12[M+H]⁺

### Example 161

### 2-(4-Cyclopropylphenyl)-N-(3-(trifluoromethyl)-1-(5-(trifluoromethyl)pyridin-3-yl)pyrrolidin-3-yl)acetamide

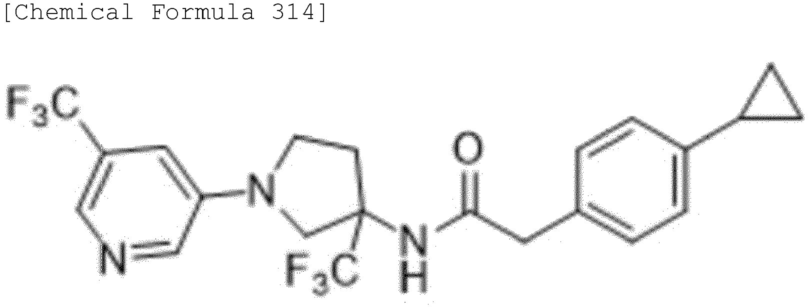

According to a technique similar to Example 3, a crude form of 3-(trifluoromethyl)-1-(5-(trifluoromethyl)-pyridin-3-yl)pyrrolidin-3-amine (58 mg) was synthesized from tert-butyl (3-(trifluoromethyl)-1-(5-(trifluoromethyl)pyridin-3-yl)pyrrolidin-3-yl)carbamate (145 mg, 0.36 mmol) synthesized in Reference Example 71, and the title compound (white amorphous, 11 mg, 25%) was obtained using a portion of the crude form of 3-(trifluoromethyl)-1-(5-(trifluoromethyl)pyridin-3-yl)pyrrolidin-3-amine (29 mg) thus obtained and 2-(4-cyclopropylphenyl)acetic acid (26 mg, 0.15 mmol) synthesized in Reference Example 33-2.
¹H NMR (CDCl₃, 400MHz) : δ=0.6-0.7 (m, 2H), 0.9-1.0 (m, 2H), 1.8-1.9(m, 1H), 2.5-2.6(m, 2H), 3.4-3.6(m, 2H), 3.56(s, 2H), 3.86(d, 1H, J=11Hz), 3.96(d, 1H, J=11Hz), 5.45(br s, 1H), 6.94(s, 1H), 7.40(d, 2H, J=8Hz), 7.90(d, 2H, J=8Hz), 8.0-8.2(m, 1H), 8.2-8.3(m, 1H).
MS:458.13[M+H]⁺

### Example 162

### N-(3-(trifluoromethyl)-1-(5-(trifluoromethyl)-pyridin-3-yl)pyrrolidin-3-yl)-2-(4-(trifluoromethyl)-phenyl)acetamide

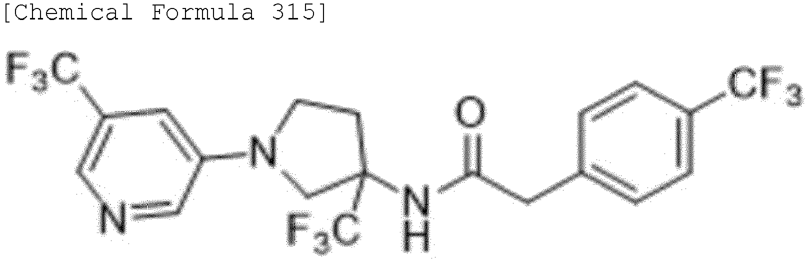

According to a technique similar to Example 3, a crude form of 3-(trifluoromethyl)-1-(5-(trifluoromethyl)-pyridin-3-yl)pyrrolidin-3-amine (58 mg) was synthesized from tert-butyl (3-(trifluoromethyl)-1-(5-(trifluoromethyl)pyridin-3-yl)pyrrolidin-3-yl)carbamate (145 mg, 0.36 mmol) synthesized in Reference Example 71, and the title compound (white amorphous, 25 mg, 51%) was obtained using a portion of the crude form of 3-(trifluoromethyl)-1-(5-(trifluoromethyl)pyridin-3-yl)pyrrolidin-3-amine (29 mg) thus obtained and 2-(4-(trifluoromethyl)phenyl)acetic acid (30 mg, 0.15 mmol).
¹H NMR (CDCl₃, 400MHz) : δ=2.5-2.7(m, 2H), 3.4-3.6(t, 2H, J=7Hz), 3.66(s, 2H), 3.91(d, 1H, J=11Hz), 3.95(d, 1H, J=11Hz), 5.52(br s, 1H), 6.96(s, 1H), 7.37(d, 2H, J=8Hz), 7.62(d, 2H, J=8Hz), 8.1-8.2(m, 1H), 8.2-8.3(m, 1H).
MS:486.09[M+H]⁺

### Example 163

### (R)-2-(1H-benzo[d][1,2,3]triazol-5-yl)-N-(1-(5-(trifluoromethyl)pyridin-3-yl)pyrrolidin-3-yl)acetamide

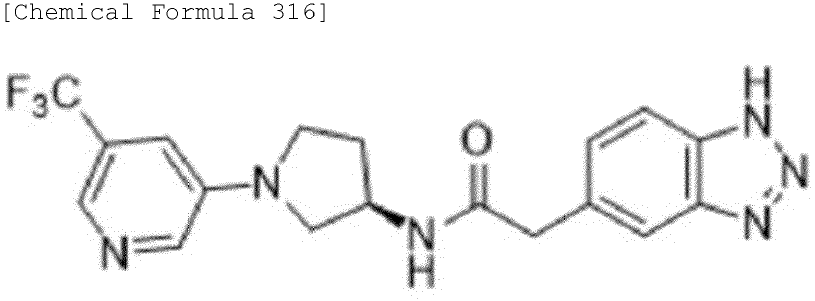

According to a technique similar to Example 1, the title compound (white crystals, 30 mg, 57%) was obtained using (R)-1-(5-(trifluoromethyl)pyridin-3-yl)pyrrolidin-3-amine (30 mg, 0.13 mmol) synthesized in Reference Example 1-2 and 2-(1H-benzo[d][1,2,3]triazol-5-yl)acetic acid (28 mg, 0.16 mmol).
¹H NMR (CD₃OD, 400MHz) : δ=2.0-2.1 (m, 1H), 2.2-2.4 (m, 1H), 3.2-3.3(m, 1H), 3.3-3.5(m, 2H), 3.6-3.7(m, 1H), 3.66(s, 2H), 4.4-4.6(m, 1H), 7.11(s, 1H), 7.38(d, 1H, J=9Hz), 7.7-7.8(m, 2H), 8.04(s, 1H), 8.07(d, 1H, J=3Hz). The 2H content is not observable.
MS:391.11[M+H] ⁺

### Example 164

### (R)-2-(1H-benzo[d][1,2,3]triazol-5-yl)-N-(1-(6-(trifluoromethyl)pyridin-3-yl)pyrrolidin-3-yl)acetamide

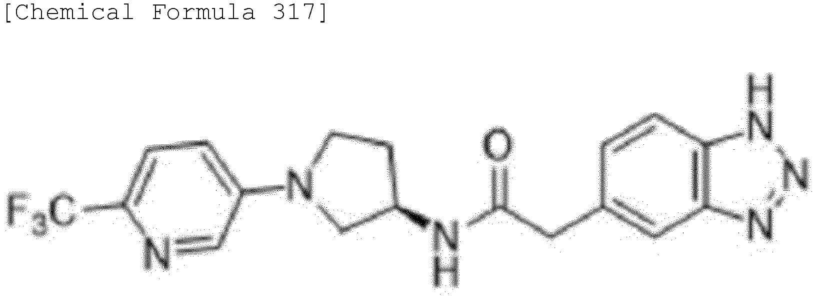

According to a technique similar to Example 1, the title compound (white crystals, 20 mg, 37%) was obtained using (R)-1-(6-(trifluoromethyl)pyridin-3-yl)pyrrolidin-3-amine (30 mg, 0.13 mmol) synthesized in Reference Example 32-2 and 2-(1H-benzo[d][1,2,3]triazol-5-yl)acetic acid (28 mg, 0.16 mmol).
¹H NMR (CD₃OD, 400MHz) : δ=2.0-2.1 (m, 1H), 2.2-2.4 (m, 1H), 3.2-3.3(m, 1H), 3.4-3.6(m, 2H), 3.6-3.7(m, 1H), 3.66(s, 2H), 4.4-4.6(m, 1H), 7.01(dd, 1H, J=3,9Hz), 7.38(d, 1H, J=8Hz), 7.52(d, 1H, J=9Hz), 7.7-7.8(m, 2H), 7.93(d, 1H, J=3Hz). The 2H content is not observable.
MS:391.11[M+H] ⁺

### Example 165

### 2-(4-(2-Oxa-5-azabicyclo[2.2.1]heptan-5-yl)phenyl)-N-((R)-1-(5-(trifluoromethyl)pyridin-3-yl)pyrrolidin-3-yl)acetamide

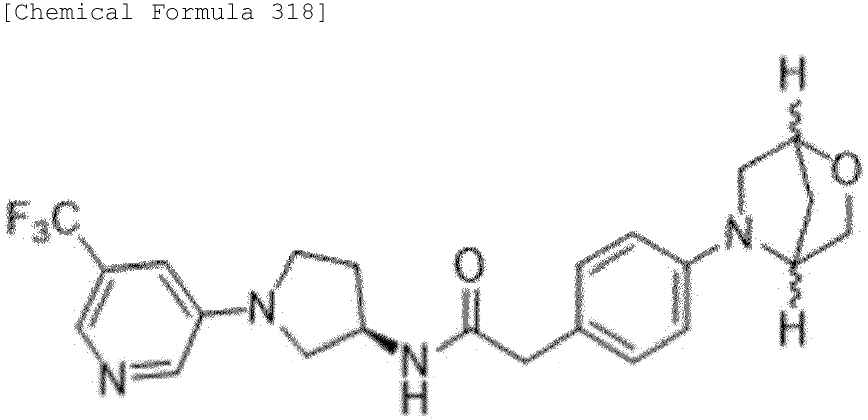

According to a technique similar to Example 38, a crude form of ethyl 2-(4-(2-oxa-5-azabicyclo[2.2.1]heptan-5-yl)phenyl)acetate (106 mg) was synthesized using ethyl 4-bromophenylacetate (109 mg, 0.45 mmol) and 2-oxa-5-azabicyclo[2.2.1]heptane hydrochloride (91 mg, 0.67 mmol), and subsequently, according to a technique similar to Example 155, a crude form of 2-(4-(2-oxa-5-azabicyclo[2.2.1]heptan-5-yl)phenyl)acetic acid (white crystals, 88 mg) was obtained from the crude form of ethyl 2-(4-(2-oxa-5-azabicyclo[2.2.1]heptan-5-yl)phenyl)acetate (106 mg) obtained as described above. Subsequently, diastereomer A of the title compound (pale yellow amorphous, 41 mg, 47%) was obtained using (R)-1-(5-(trifluoromethyl)pyridin-3-yl)pyrrolidin-3-amine (44 mg, 0.19 mmol) synthesized in Reference Example 1-2 and a portion of the crude form of 2-(4-(2-oxa-5-azabicyclo[2.2.1]heptan-5-yl)phenyl)acetic acid (44 mg) synthesized as described above.
¹H NMR (CDCl₃, 400MHz) : δ=1.8-2.1 (m, 3H), 2.2-2.4 (m, 1H), 3.0-3.2(m,2H),3.36(t,2H,J=7Hz),3.49(s,2H), 3.54(d, 1H, J=9Hz), 3.6-3.7(m, 1H), 3.8-3.9(m, 2H), 4.38(s, 1H), 4.5-4.7 (m, 1H), 4.65(s, 1H), 5.5-5.7(m, 1H), 6.54(d, 2H, J=8Hz), 6.90(s, 1H), 7.07(d, 2H, J=8Hz), 8.07(s, 1H), 8.19(s, 1H).
MS:447.17[M+H]⁺

### Example 166

### 2-(4-(2-Oxa-5-azabicyclo[2.2.1]heptan-5-yl)phenyl)-N-((R)-1-(6-(trifluoromethyl)pyridin-3-yl)pyrrolidin-3-yl)acetamide

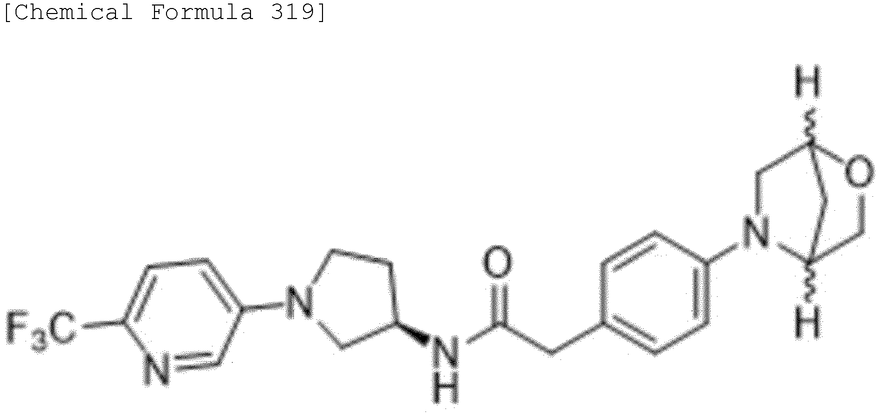

According to a technique similar to Example 165, diastereomer A of the title compound (pale yellow crystals, 41 mg, 47%) was obtained using (R)-1-(6-(trifluoromethyl)pyridin-3-yl)pyrrolidin-3-amine (44 mg, 0.19 mmol) synthesized in Reference Example 32-2 and a portion of the crude form of 2-(4-(2-oxa-5-azabicyclo[2.2.1]heptan-5-yl)phenyl)acetic acid (44 mg) synthesized in Example 165.
¹H NMR (CDCl₃, 400MHz) : δ=1.8-2.1 (m, 3H), 2.2-2.4 (m, 1H), 3.1-3.2(m,2H),3.38(t,2H,J=7Hz),3.49(s,2H), 3.55 (d, 1H, J=9Hz), 3.6-3.7 (m, 1H), 3.8-3.9 (m, 2H), 4.38 (s, 1H), 4.5-4.7 (m, 1H), 4.65 (s, 1H), 5.5-5.7 (m, 1H) 6.54 (d, 2H, J=8Hz), 6.7-6.9 (m, 1H), 7.07 (d, 2H, J=8Hz), 7.47(d, 1H, J=8Hz), 7.9-8.0 (m, 1H).
MS : 447.15[M+H]⁺

### Example 167

### (R)-2-(1-methyl-1H-benzo[d][1,2,3]triazol-5-yl)-N-(1-(5-(trifluoromethyl)pyridin-3-yl)pyrrolidin-3-yl)acetamide

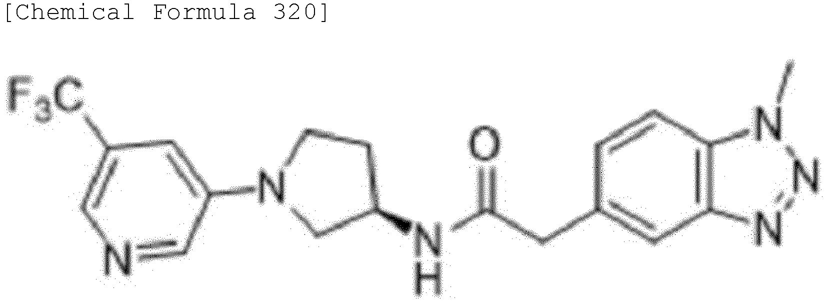

According to a technique similar to Example 1, the title compound (yellow amorphous, 39 mg, 64%) was obtained using (R)-1-(5-(trifluoromethyl)pyridin-3-yl)pyrrolidin-3-amine (35 mg, 0.15 mmol) synthesized in Reference Example 1-2 and 2-(1-methyl-1H-benzo[d][1,2,3]triazol-5-yl)acetic acid (30 mg, 0.16 mmol).
¹H NMR (CDCl₃, 400MHz) : δ=1.9-2.1 (m, 1H), 2.2-2.4 (m, 1H), 3.1-3.2 (m, 1H), 3.2-3.4 (m, 2H), 3.5-3.7 (m, 1H), 3.37 (s, 2H), 4.27 (s, 3H), 4.6-4.7 (m, 1H), 6.48 (br s, 1H), 6.78 (s, 1H), 7.47 (d, 1H, J=9Hz), 7.50 (d, 1H, J=9Hz), 7.88 (s, 1H), 7.96 (d, 1H, J=3Hz), 8.10 (s, 1H).
MS : 405.12 [M+H]⁺

### Example 168

### (R)-2-(1-methyl-1H-benzo[d][1,2,3]triazol-5-yl)-N-(1-(6-(trifluoromethyl)pyridin-3-yl)pyrrolidin-3-yl)acetamide

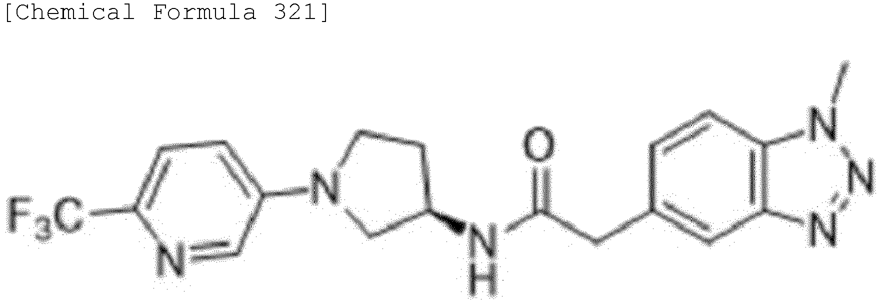

According to a technique similar to Example 1, the title compound (white crystals, 8 mg, 13%) was obtained using (R)-1-(6-(trifluoromethyl)pyridin-3-yl)pyrrolidin-3-amine (35 mg, 0.15 mmol) synthesized in Reference Example 32-2 and 2-(1-methyl-1H-benzo[d][1,2,3]triazol-5-yl)acetic acid (30 mg, 0.16 mmol).
¹H NMR (CD₃OD, 400MHz) : δ=2.0-2.2 (m, 1H), 2.2-2.4 (m, 1H), 3.2-3.4 (m, 1H), 3.4-3.6 (m, 2H), 3.6-3.7 (m, 1H), 3.70 (s, 2H) 4.32 (s, 3H), 4.4-4.6 (m, 1H), 7.04 (dd, 1H, J=3,9Hz) 7.52 (dd, 1H, J=1,8Hz), 7.56 (d, 1H, J=8Hz), 7.69 (d, 1H, J=8Hz), 7.88 (s, 1H), 7.96 (d, 1H, J=3Hz). The 1H content is not observable.
MS : 405.12 [M+H] ⁺

### Example 169

### 2-(4-Cyclopropylphenyl)-N-(1-(4-(trifluoromethyl)phenyl)azetidin-3-yl)acetamide

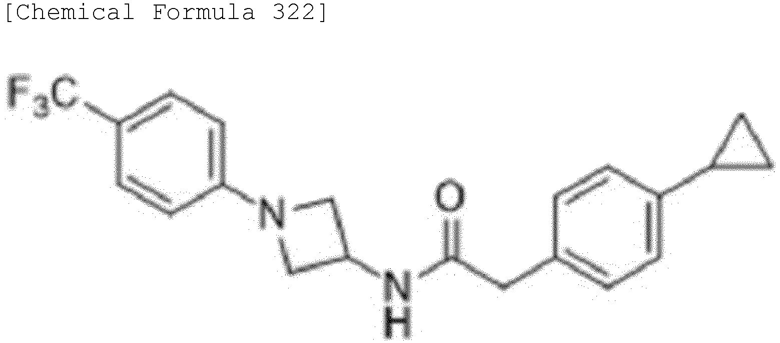

According to a technique similar to Example 9, the title compound (pale yellow solid, 33 mg, 50%) was obtained using N-(azetidin-3-yl)-2-(4-cyclopropylphenyl)acetamide (40 mg, 0.17 mmol) synthesized in Reference Example 75-2 and 1-bromo-4-(trifluoromethyl)benzene (29 µL, 0.21 mmol).
¹H NMR (CDCl₃, 400MHz) : δ=0.6-0.8 (m, 2H), 0.9-1.1 (m, 2H), 1.8-2.0 (m, 1H), 3.5-3.7 (m, 4H), 4.23 (t, 2H, J=8Hz), 4.7-4.9 (m, 1H), 5.78 (d, 1H, J=7Hz), 6.39 (d, 2H, J=8Hz), 7.06 (d, 2H, J=8Hz), 7.13 (d, 2H, J=8Hz), 7.42 (d, 2H, J=8Hz).
MS : 397.12 [M+Na]⁺

### Example 170

### 2-(4-Cyclopropylphenyl)-N-(1-(4-fluoro-3-(trifluoromethyl)phenyl)azetidin-3-yl)acetamide

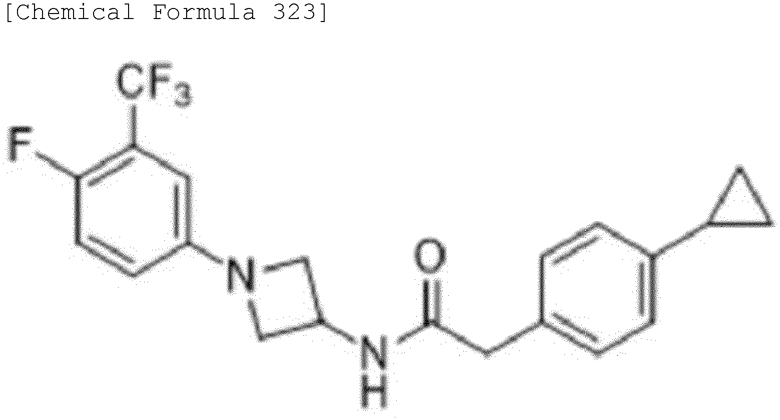

According to a technique similar to Example 9, the title compound (pale yellow solid, 27 mg, 38%) was obtained using N-(azetidin-3-yl)-2-(4-cyclopropylphenyl)acetamide (40 mg, 0.17 mmol) synthesized in Reference Example 75-2 and 4-bromo-1-fluoro-2-(trifluoromethyl)benzene (29 µL, 0.21 mmol).
¹H NMR (CDCl₃, 400MHz) : δ=0.6-0.8 (m, 2H), 0.9-1.1 (m, 2H), 1.8-2.0 (m, 1H), 3.4-3.7(m, 4H), 4.16(t, 2H, J=8Hz), 4.7-4.9 (m, 1H), 5.78 (d, 1H, J=6Hz), 6.3-6.6 (m, 2H), 6.9-7.2(m, 5H).
MS : 415.11 [M+Na]⁺

### Example 171

### (R)-2-(4-bromophenyl)-N-(1-(5-(trifluoromethyl)pyridin-3-yl)pyrrolidin-3-yl)acetamide

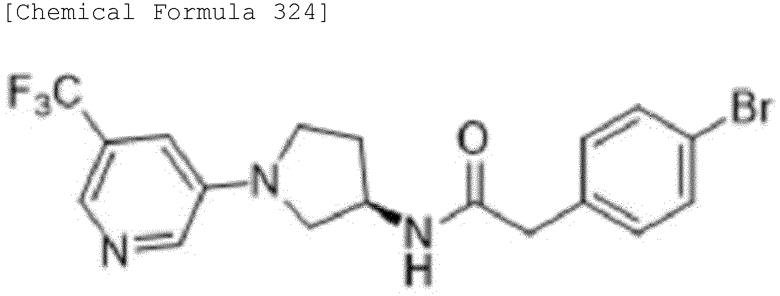

According to a technique similar to Example 1, the title compound (white solid, 100 mg, 100%) was obtained using (R)-1-(5-(trifluoromethyl)pyridin-3-yl)pyrrolidin-3-amine (52 mg, 0.22 mmol) synthesized in Reference Example 1-2 and 2-(4-bromophenyl)acetic acid (60 mg, 0.28 mmol).
¹H NMR (CDCl₃, 400MHz) : δ=1.9-2.1 (m, 1H), 2.2-2.4 (m, 1H), 3.14 (dd, 1H, J=4, 10Hz), 3.36 (t, 2H, J=7Hz), 3.52 (s, 2H), 3.62 (dd, 1H, J=6, 10Hz), 4.6-4. 7 (m, 1H), 6.22 (d, 1H, J=7Hz), 6.8-6.9 (m, 1H), 7.14 (d, 2H, J=8Hz), 7.46 (d, 2H, J=8Hz), 7.98 (d, 1H, J=3Hz), 8.13 (s, 1H).
MS : 428.02 [M+H] ⁺

### Example 172

### (R)-2-(4-cyclopropylphenyl)-N-(1-(6-fluorobenzo[d]thiazol-2-yl)pyrrolidin-3-yl) acetamide

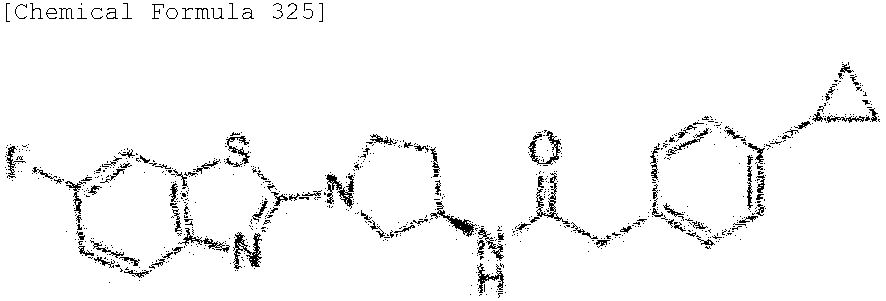

(R)-2-(4-cyclopropylphenyl)-N-(pyrrolidin-3-yl)acetamide (49 mg, 0.20 mmol) synthesized in Reference Example 74-2 was dissolved in DMF (1.0 mL), subsequently 2-chloro-6-fluorobenzo[d]thiazole (45 mg, 0.24 mmol) and cesium carbonate (78 mg, 0.24 mmol) were added thereto, and the mixture was stirred for 2 hours at 100°C. Saturated sodium hydrogen carbonate was added to the reaction liquid that had been left to cool to room temperature, and then the mixture was extracted with chloroform. An organic layer thus separated was dried over anhydrous sodium sulfate, insoluble materials were filtered, and then the solvent was distilled off under reduced pressure. A residue thus obtained was purified by silica gel column chromatography (heptane : ethyl acetate) (concentration gradient: 50 to 100%), and the title compound (white solid, 60 mg, 71%) was obtained.
¹H NMR (CDCl₃, 400MHz) : δ=0.6-0.7 (m, 2H), 0.9-1.0 (m, 2H), 1.8-2.0 (m, 2H), 2.2-2.4 (m, 1H), 3.31 (dd, 1H, J=5, 11Hz), 3.5-3.7 (m, 2H), 3.53 (s, 2H), 3.82 (dd, 1H, J=7, 11Hz), 4.5-4.7 (m, 1H), 5.48 (d, 1H, J=7Hz), 6.9-7.1 (m, 3H), 7.11 (d, 2H, J=8Hz), 7.31 (dd, 1H, J=3, 8Hz), 7.48 (dd, 1H, J=5, 9Hz).
MS : 396.13 [M+H]⁺

### Example 173

### (R)-N-(1-(5-bromothiazol-2-yl)pyrrolidin-3-yl)-2-(4-cyclopropylphenyl) acetamide

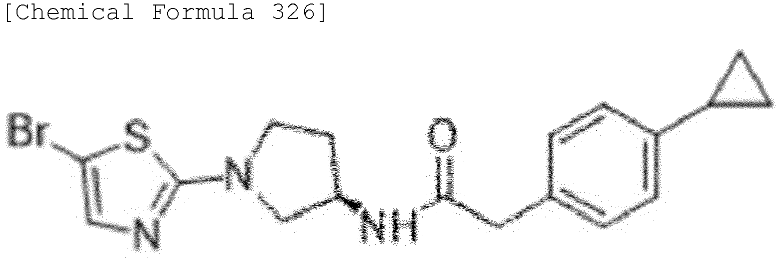

According to a technique similar to Example 172, the title compound (pale yellow solid, 26 mg, 32%) was obtained using (R)-2-(4-isopropylphenyl)-N-(pyrrolidin-3-yl)acetamide (49 mg, 0.20 mmol) synthesized in Reference Example 74-2 and 2,5-dibromothiazole (58 mg, 0.24 mmol).
¹H NMR (CDCl₃, 400MHz) : δ=0.6-0.8 (m, 2H), 0.9-1.0 (m, 2H), 1.8-2.0 (m, 2H), 2.2-2.4 (m, 1H), 3.16 (dd, 1H, J=5, 11Hz), 3.3-3.5 (m, 2H), 3.52 (s, 2H), 3.67 (dd, 1H, J=6, 10Hz), 4.5-4.7 (m, 1H), 5.46 (d, 1H, J=7Hz), 7.0-7.1 (m, 3H), 7.11 (d, 2H, J=8Hz).
MS : 428.02 [M+Na]⁺

### Example 174

### (R)-2-(4-cyclopropylphenyl)-N-(1-(4-fluoro-3-(trifluoromethyl)phenyl)pyrrolidin-3-yl) acetamide

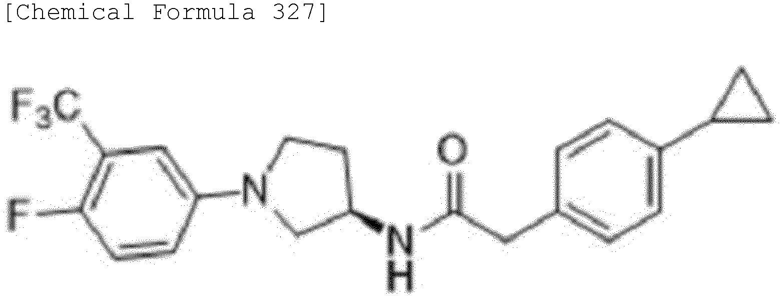

According to a technique similar to Example 9, the title compound (white solid, 102 mg, 84%) was obtained using (R)-2-(4-cyclopropylphenyl)-N-(pyrrolidin-3-yl)acetamide (73 mg, 0.30 mmol) synthesized in Reference Example 74-2 and 4-bromo-1-fluoro-2-(trifluoromethyl)-benzene (51 µL, 0.36 mmol).
¹H NMR (CDCl₃, 400MHz) : δ=0.6-0.8 (m, 2H), 0.9-1.1 (m, 2H), 1.8-2.0 (m, 2H), 2.2-2.4 (m, 1H), 3.04 (dd, 1H, J=4, 9Hz), 3.2-3.4 (m, 2H), 3.4-3.6 (m, 1H), 3.52 (s, 2H), 4.5-4.7 (m, 1H), 5.50 (d, 1H, J=7Hz), 6.5-6.7 (m, 2H), 6.9-7.2 (m, 5H).
MS : 407.16 [M+H]⁺

### Example 175

### (R)-2-(4-cyclopropylphenyl)-N-(1-(3-methoxyphenyl)-pyrrolidin-3-yl)acetamide

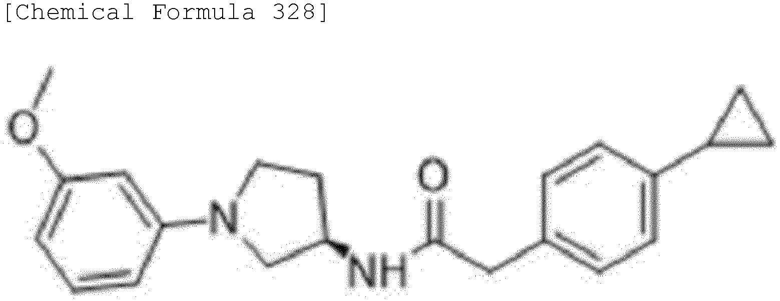

According to a technique similar to Example 9, the title compound (white solid, 203 mg, 58%) was obtained using (R)-2-(4-cyclopropylphenyl)-N-(pyrrolidin-3-yl)acetamide (244 mg, 1.0 mmol) synthesized in Reference Example 74-2 and 1-bromo-3-methoxybenzene (152 µL, 1.2 mmol) .
¹H NMR (CDCl₃, 400MHz) : δ=0.6-0.7 (m, 2H), 0.9-1.0 (m, 2H), 1.7-1.9 (m, 2H), 2.1-2.3 (m, 1H), 3.05 (dd, 1H, J=4, 10Hz), 3.2-3.4(m,2H),3.5-3.6(m,3H),3.51(s,3H),4.5-4.7(m,1H), 5.53 (d, 1H, J=7Hz), 6.07 (t, 1H, J=2Hz), 6.15 (dd, 1H, J=1, 8Hz), 6.28 (dd, 1H, J=2, 8Hz), 7.03 (d, 2H, J=8Hz), 7.0-7.2 (m, 3H).
MS : 351.19[M+H] ⁺

### Example 176

### (R)-2-(4-(3,3-difluoropyrrolidin-1-yl)phenyl)-N-(1-(5-(trifluoromethyl)pyridin-3-yl)pyrrolidin-3-yl)acetamide

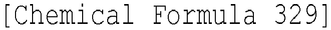

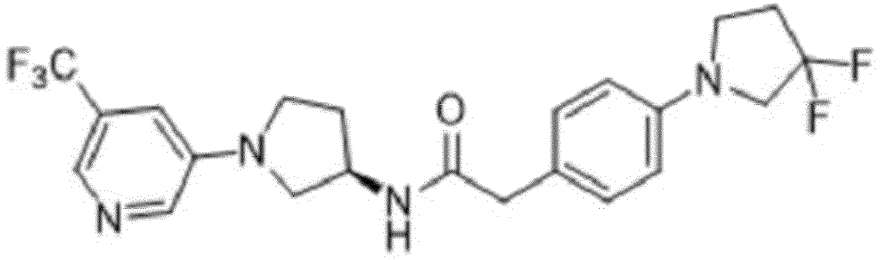

According to a technique similar to Example 9, the title compound (white solid, 11 mg, 24%) was obtained using (R)-2-(4-bromophenyl)-N-(1-(5-(trifluoromethyl)pyridin-3-yl)pyrrolidin-3-yl)acetamide (43 mg, 0.10 mmol) synthesized in Example 171 and 3,3-difluoropyrrolidine hydrochloride (17 mg, 0.12 mmol).
¹H NMR (CDCl₃, 400MHz) : δ=1.8-2.0 (m, 1H), 2.2-2.4 (m, 1H), 2.4-2.6 (m, 2H), 3.10 (dd, 1H, J=5, 10Hz), 3.36 (t, 2H, J=7Hz), 3.4-3.6 (m, 4H), 3.6-3.8 (m, 3H), 4.5-4.7 (m, 1H), 5.52 (d, 1H, J=7Hz), 6.52 (d, 2H, J=9Hz), 6.90 (s, 1H), 7.11 (d, 2H, J=8Hz), 8.07 (s, 1H), 8.20 (s, 1H).
MS : 455.15[M+H] ⁺

### Example 177

### Ethyl (R)-2-(3-(3-(2-(4-cyclopropylphenyl)acetamido)pyrrolidin-1-yl)phenoxy)-2-methylpropanoate

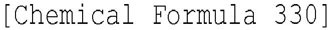

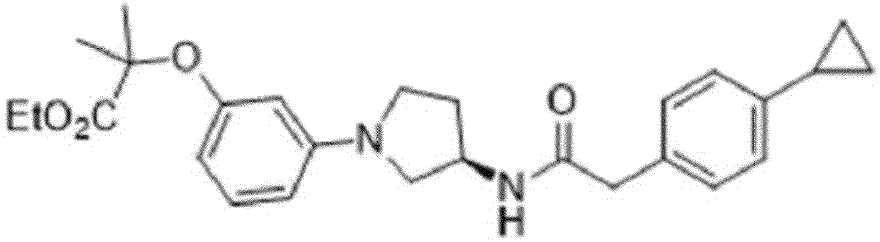

According to a technique similar to Example 9, the title compound (white solid, 43 mg, 24%) was obtained using (R)-2-(4-cyclopropylphenyl)-N-(pyrrolidin-3-yl)acetamide (98 mg, 0.40 mmol) synthesized in Reference Example 74-2 and ethyl 2-(3-bromophenoxy)-2-methylpropanoate (138 mg, 0.48 mmol) synthesized in Reference Example 76.
¹H NMR (CDCl₃, 400MHz) : δ=0.6-0.8 (m, 2H), 0.9-1.0 (m, 2H), 1.25 (t, 3H, J=7Hz), 1.59 (s, 6H), 1.7-2.0 (m, 2H), 2.1-2.3 (m, 1H), 3.01 (dd, 1H, J=4, 10Hz), 3.1-3.4 (m, 2H), 3.4-3.6 (m, 3H), 4.22 (dd, 2H, J=7, 15Hz), 4.5-4.7 (m, 1H), 5.55 (d, 1H, J=7Hz), 6.0-6.1 (m, 1H), 6.1-6.2 (m, 2H), 7.0-7.1 (m, 3H), 7.11 (d, 2H, J=8Hz).
MS : 451.22 [M+H]⁺

### Example 178

### (R)-2-(4-cyclopropylphenyl)-N-(1-(3-hydroxyphenyl)-pyrrolidin-3-yl)acetamide

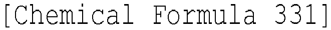

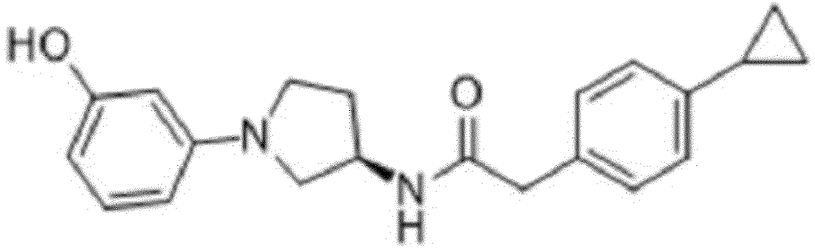

Pyridine hydrochloride (500 mg) was added to (R)-2-(4-cyclopropylphenyl)-N-(1-(3-methoxyphenyl)pyrrolidin-3-yl)acetamide (35 mg, 0.10 mmol) synthesized in Example 175, and the mixture was heated for 3 hours at 150°C. Saturated sodium hydrogen carbonate was added to the reaction liquid that had been left to cool to room temperature, and then the mixture was extracted with ethyl acetate. An organic layer thus separated was washed with saturated brine and dried over anhydrous sodium sulfate, insoluble materials were filtered, and then the solvent was distilled off under reduced pressure. A residue thus obtained was purified by NH silica gel column chromatography (heptane : ethyl acetate) (concentration gradient: 50 to 100%), and the title compound (white solid, 26 mg, 77%) was obtained.
¹H NMR (CDCl₃, 400MHz) : δ=1.5-2.0 (m, 5H), 2.1-2.3 (m, 1H), 3.04 (dd, 1H, J=4, 10Hz), 3.1-3.4 (m, 2H), 3.4-3.6 (m, 3H), 4.5-4.7 (m, 1H), 4.8-5.0 (m, 1H), 5.54 (d, 1H, J=7Hz), 6.0-6.4 (m, 4H), 7.0-7.2 (m, 3H), 7.29 (d, 2H, J=8Hz).
MS : 337.17 [M+H]⁺

### Example 179

### (R)-2-(4-cyclopropylphenyl)-N-(1-(6-(trifluoromethyl)pyridin-2-yl)pyrrolidin-3-yl)acetamide

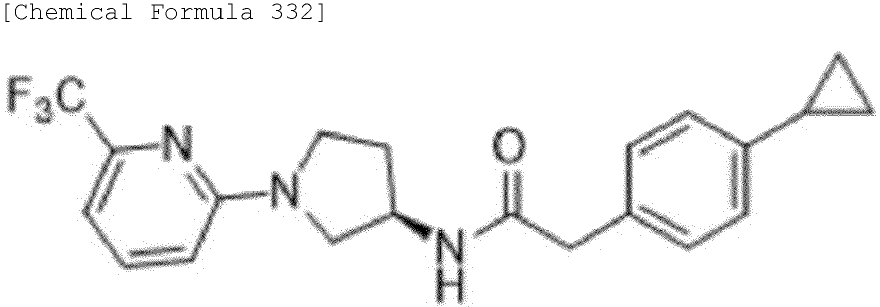

According to a technique similar to Example 172, the title compound (pale yellow solid, 21 mg, 26%) was obtained using (R)-2-(4-cyclopropylphenyl)-N-(pyrrolidin-3-yl)acetamide (49 mg, 0.20 mmol) synthesized in Reference Example 74-2 and 2-chloro-6-(trifluoromethyl)pyridine (44 mg, 0.24 mmol).
¹H NMR (CDCl₃, 400MHz) : δ=0.6-0.7 (m, 2H), 0.9-1.0 (m, 2H), 1.8-2.0 (m, 2H), 2.2-2.4 (m, 1H), 3.24 (dd, 1H, J=5, 11Hz), 3.4-3.6 (m, 4H), 3.76 (dd, 1H, J=6, 11Hz), 4.5-4.7 (m, 1H), 5.60 (d, 1H, J=7Hz), 6.45 (d, 1H, J=8Hz), 6.90 (d, 1H, J=7Hz), 7.02 (d, 2H, J=8Hz), 7.10 (d, 2H, J=8Hz), 7.53 (t, 1H, J=8Hz).
MS : 412.13 [M+Na]⁺

### Example 180

### (R)-2-(4-cyclopropylphenyl)-N-(1-(4,6-dimethoxypyrimidin-2-yl)pyrrolidin-3-yl)acetamide

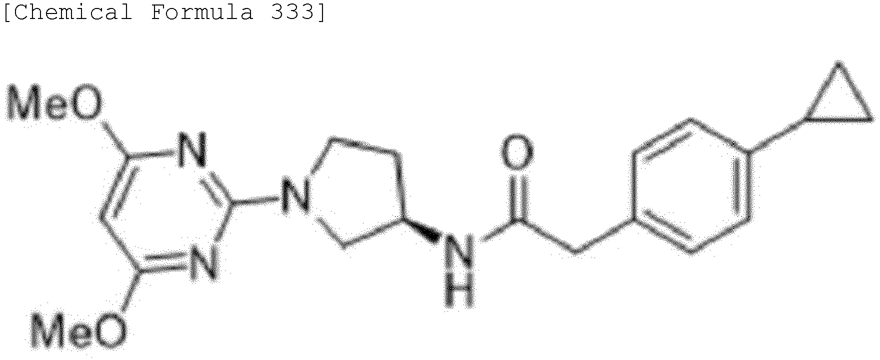

According to a technique similar to Example 172, the title compound (white solid, 56 mg, 73%) was obtained using (R)-2-(4-cyclopropylphenyl)-N-(pyrrolidin-3-yl)acetamide (49 mg, 0.20 mmol) synthesized in Reference Example 74-2 and 2-chloro-4,6-dimethoxypyridine (44 mg, 0.24 mmol).
¹H NMR (CDCl₃, 400MHz) : δ=0.6-0.8 (m, 2H), 0.9-1.1 (m, 2H), 1.7-2.0 (m, 2H), 2.1-2.3 (m, 1H), 3.2-3.4 (m, 1H), 3.5-3.7 (m, 4H), 3.7-3.9 (m, 1H), 3.84 (s, 6H), 4.4-4.6 (m, 1H), 5.37 (s, 1H), 5.51 (d, 1H, J=5Hz), 7.03 (d, 2H, J=8Hz), 7.11 (d, 2H, J=7Hz).
MS : 405.16 [M+Na]⁺

### Example 181

### (R)-2-(4-morpholinophenyl)-N-(1-(5-(trifluoromethyl)pyridin-3-yl)pyrrolidin-3-yl)acetamide

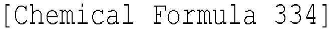

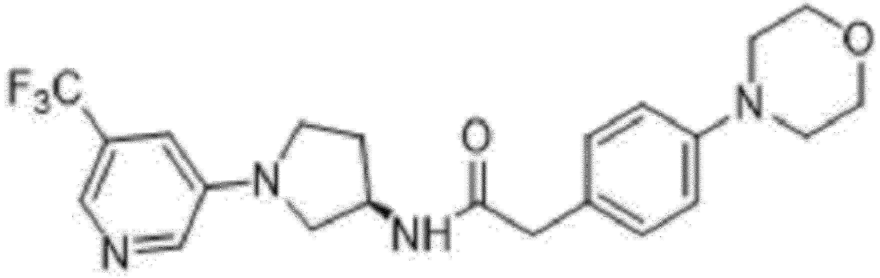

According to a technique similar to Example 9, the title compound (white solid, 12 mg, 13%) was obtained using (R)-2-(4-bromophenyl)-N-(1-(5-(trifluoromethyl)pyridin-3-yl)pyrrolidin-3-yl)acetamide (86 mg, 0.20 mmol) synthesized in Example 171 and morpholine (35 µL, 0.40 mmol).
¹H NMR (CDCl₃, 400MHz) : δ=1.8-2.0 (m, 1H), 2.2-2.4 (m, 1H), 3.0-3.2 (m, 5H), 3.37 (t, 2H, J=7Hz) .3.51 (s, 2H), 3.65 (dd, 1H, J=7, 10Hz), 3.8-3.9 (m, 4H), 4.5-4.7 (m, 1H), 5.4-5.6 (m, 1H), 6.8-7.0 (m, 3H), 7.14 (d, 2H, J=8Hz), 8.07 (d, 1H, J=2Hz), 8.20 (s, 1H).
MS : 435.17 [M+H]⁺

### Example 182

### (R)-2-(4-cyclopropylphenyl)-N-(1-(4-(trifluoromethyl)pyridin-2-yl)pyrrolidin-3-yl)acetamide

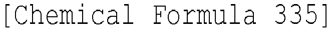

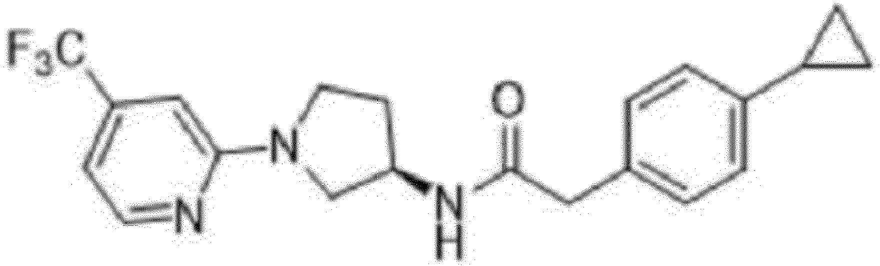

According to a technique similar to Example 172, the title compound (white solid, 40 mg, 51%) was obtained using (R)-2-(4-cyclopropylphenyl)-N-(pyrrolidin-3-yl)acetamide (49 mg, 0.20 mmol) synthesized in Reference Example 74-2 and 2-chloro-4-(trifluoromethyl)pyridine (44 mg, 0.24 mmol) .
¹H NMR (CDCl₃, 400MHz) : δ=0.6-0.7 (m, 2H), 0.9-1.0 (m, 2H), 1.8-2.0 (m, 2H), 2.2-2.4 (m, 1H), 3.23 (dd, 1H, J=5,11Hz), 3.4-3.6 (m, 4H), 3.76 (dd, 1H, J=6, 10Hz), 4.5-4.7 (m, 1H), 5.49 (d, 1H, J=6Hz), 6.48 (s, 1H), 6.73 (d, 1H, J=5Hz), 7.03 (d, 2H, J=8Hz), 7.11 (d, 2H, J=8Hz), 8.25 (d, 1H, J=5Hz).
MS : 390.14 [M+H]⁺

### Example 183

### (R)-2-(4-(trifluoromethyl)phenyl)-N-(1-(6-(trifluoromethyl)pyridin-3-yl)pyrrolidin-3-yl)acetamide

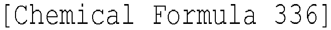

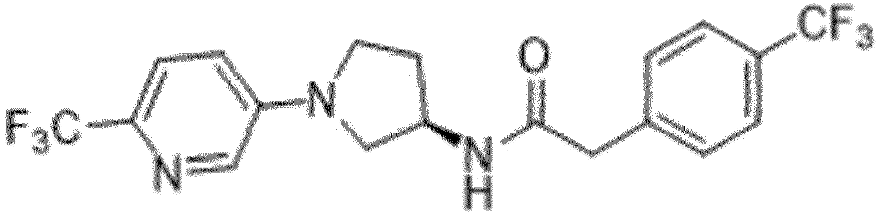

According to a technique similar to Example 9, the title compound (pale yellow solid, 24 mg, 29%) was obtained using (R)-N-(pyrrolidin-3-yl)-2-(4-(trifluoromethyl)-phenyl)acetamide (55 mg, 0.20 mmol) synthesized in Reference Example 73-2 and 5-bromo-2-(trifluoromethyl)-pyridine (54 mg, 0.24 mmol).
¹H NMR (CDCl₃, 400MHz) : δ=1.9-2.1 (m, 1H), 2.3-2.5 (m, 1H), 3.19 (dd, 1H, J=4, 11Hz), 3.3-3.5 (m, 2H), 3.62 (s, 2H), 3. 67 (dd, 1H, J=6, 8Hz), 4. 6-4.7 (m, 1H) , 5.70 (d, 1H, J=6Hz), 6.79 (dd, 1H, J=3, 9Hz), 7.40 (d, 2H, J=8Hz), 7.47 (d, 1H, J=9Hz), 7.61 (d, 2H, J=8Hz), 7.95 (d, 1H, J=2Hz).
MS : 418.11 [M+H]⁺

### Example 184

### (R)-N-(1-(4-fluoro-3-(trifluoromethyl)phenyl)pyrrolidin-3-yl)-2-(4-(trifluoromethyl)phenyl)acetamide

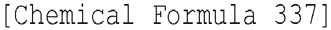

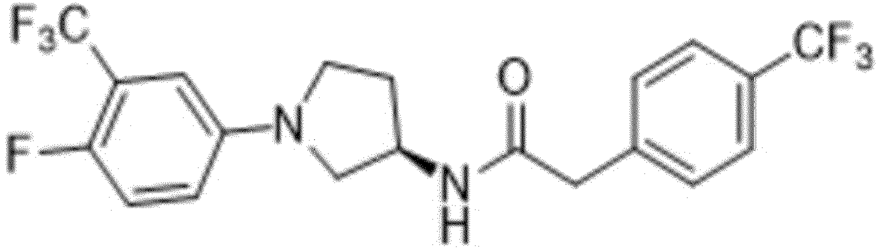

According to a technique similar to Example 9, the title compound (pale yellow solid, 22 mg, 25%) was obtained using (R)-N-(pyrrolidin-3-yl)-2-(4-(trifluoromethyl)-phenyl)acetamide (55 mg, 0.20 mmol) synthesized in Reference Example 73-2 and 4-bromo-1-fluoro-2-(trifluoromethyl)benzene (58 mg, 0.24 mmol).
¹H NMR (CDCl₃, 400MHz) : δ=1.8-2.0 (m, 1H), 2.2-2.4 (m, 1H), 3.11 (dd, 1H, J=4, 10Hz), 3.2-3.5 (m, 2H), 3.57 (dd, 1H, J=6, 10Hz), 3.61 (s, 2H), 4.5-4.7 (m, 1H), 5.56 (d, 1H, J=6Hz), 6.5-6.7 (m, 2H), 7.05 (t, 1H, J=9Hz), 7.39 (d, 2H, J=8Hz), 7.61 (d, 2H, J=8Hz).
MS : 435.11[M+H] ⁺

### Example 185

### 2-(4-(Trifluoromethyl)phenyl)-N-((R)-1-(6-(trifluoromethyl)pyridin-3-yl)pyrrolidin-3-yl)propanamide

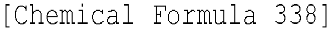

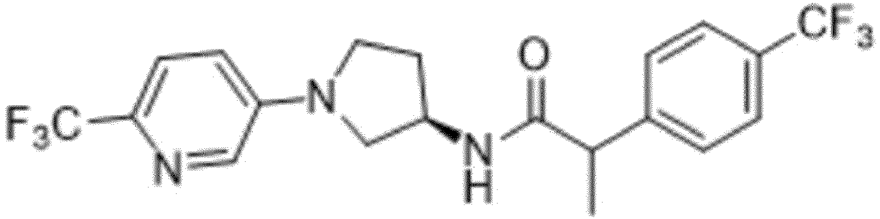

Tert-butyl (R)-3-(2-(4-(trifluoromethyl)-phenyl)acetamido)pyrrolidine-1-carbamate (149 mg, 0.40 mmol) synthesized in Reference Example 73-1 was dissolved in DMF (2 mL), sodium hydride (60% oil, 24 mg, 0.60 mmol) and methyl iodide (37 µL, 0.60 mmol) were added thereto, and the mixture was stirred for one hour at room temperature. Saturated sodium hydrogen carbonate was added to a residue thus obtained under ice cooling, and then the mixture was extracted with chloroform. An organic layer thus separated was dried over anhydrous sodium sulfate, insoluble materials were filtered, subsequently the solvent was distilled off under reduced pressure, and thereby a crude form of tert-butyl (3R)-3-(2-(4-trifluoromethyl)-phenyl)propanamido)pyrrolidine-1-carbamate was obtained. Subsequently, according to a technique similar to Example 36, the title compound (white solid, 25 mg, 15% in three stages) was obtained using the crude form of tert-butyl (3R)3-(2-(4-trifluoromethyl)phenyl)propanamido)pyrrolidine-1-carbamate thus obtained and 5-bromo-2-(trifluoromethyl)-pyridine (92 mg, 0.41 mmol).
¹H NMR (CDCl₃, 400MHz) : δ=1. 53 (d, 3H, J=7Hz), 1.9-2.1 (m, 1H), 2.3-2.5 (m, 1H), 3.07 (dd, 1H, J=4, 10Hz), 3.3-3.5 (m, 2H), 3.5-3.7 (m, 2H), 4.5-4.7 (m, 1H), 5.69 (d, 1H, J=6Hz), 6.75 (dd, 1H, J=3,7Hz), 7.3-7.5 (m, 3H), 7.57 (d, 2H, J=8Hz), 7.89 (d, 1H, J=3Hz).
MS : 454.09 [M+Na]⁺

### Example 186

### (1S,2S)-2-(1H-benzo[d]imidazol-2-yl)-N-((R)-1-(5-(trifluoromethyl)pyridin-3-yl)piperidin-3-yl)cyclopropane-1-carboxyamide

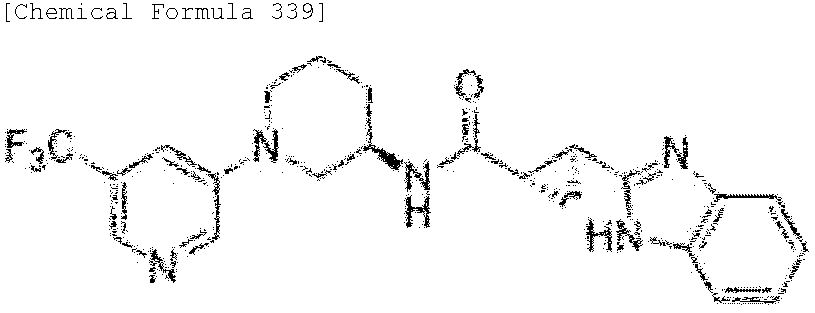

According to a technique similar to Example 3, a crude form of (R)-1-(5-(trifluoromethyl)pyridin-3-yl)piperidin-3-amine (166 mg) was synthesized from tert-butyl (R)-(1-(5-(trifluoromethyl)pyridin-3-yl)piperidin-3-yl)carbamate (76 mg, 0.219 mmol) synthesized in Reference Example 66, and the title compound (white powder, 70 mg, 74%) was obtained using (1S,2S)-2-(1H-benzo[d]imidazol-2-yl)cyclopropane-1-carboxylic acid (66 mg, 0.329 mmol).
¹H NMR (DMSO-d₆, 400MHz) : δ=1.4-1.6 (m, 4H), 1.7-1.9 (m, 2H), 2.2-2.5 (m, 2H), 2.88 (dd, 1H, J=9, 12Hz), 2.9-3.1 (m, 1H), 3.6-3.9 (m, 3H), 7.0-7.2 (m, 2H), 7.3-7.6 (m, 3H), 8.24 (s, 1H), 8.37 (d, 1H, J=7Hz), 8.56 (d, 1H, J=3Hz), 12.4 (br s, 1H).
MS : 430.18 [M+H]⁺

### Example 187

### (R)-2-(1H-indol-6-yl)-N-(1-(4-(trifluoromethyl)thiazol-2-yl)pyrrolidin-3-yl) acetamide

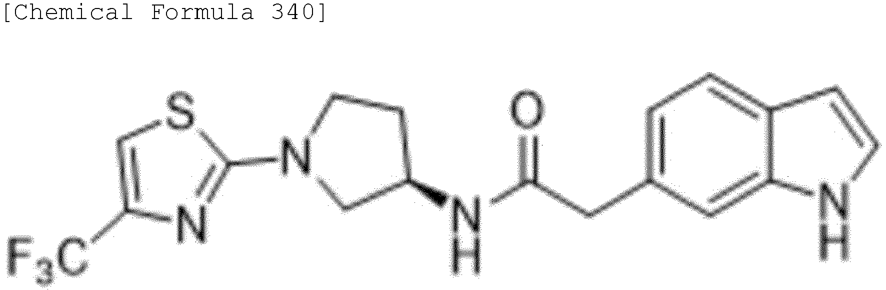

According to a technique similar to Example 1, the title compound (pale yellow powder, 36 mg, 58%) was obtained using (R)-1-(4-(trifluoromethyl)thiazol-2-yl)pyrrolidin-3-amine (38 mg, 0.160 mmol) synthesized in Reference Example 12-2 and 2-(1H-indol-6-yl)acetic acid (34 mg, 0.192 mmol).
¹H NMR (CDCl₃, 400MHz) : δ=1.7-1.9 (m, 1H), 2.2-2.4 (m, 1H), 3.19 (dd, 1H, J=5, 11Hz), 3.3-3.6 (m, 2H), 3.69 (s, 2H), 3.72 (dd, 1H, J=6, 11Hz), 4.5-4.7 (m, 1H), 5.59 (d, 1H, J=6Hz), 6.5-6.6 (m, 1H), 6.89 (d, 1H, J=1Hz), 6.95 (dd, 1H, J=1,8Hz), 7.2-7.3 (m, 2H), 7.61 (d, 1H, J=8Hz), 8.25 (br s, 1H).
MS : 417.07 [M+Na]⁺

### Example 188

### Pharmacological test 1

### T-type calcium blocking action

### (Testing method)

### (1) Construction of cells steadily expressing human Cav3.2 channels

A sequence obtained by adding HindIII site and a kozak sequence (GCCACC) to the 5'-side of human Cav3.2 channel ORF (Open Reading Frame) gene and adding a KpnI site to the 3'-side of the gene, was incorporated into pcDNA3.1(+) (Thermo Fisher Scientific, #V790-20), and the plasmid was introduced into HEK293 cells (ATCC No. CRL-1573) according to the protocol of FuGENE (registered trademark) HD Transfection Reagent (Promega, #E2311).

### (2) Method for measuring intracellular calcium concentration

The cell strain steadily expressing human Cav3.2 channels as produced by the testing method (1) was seeded on a 96-well plate using DMEM including 10% FBS, and the cells were cultured for 48 hours under the conditions of 37°C and 5% CO₂. Subsequently, the medium was removed, and each of the wells were washed with buffer A and treated with 80 µL of a fluorescent dye solution including a mixed liquid of 22 µg of a fluorescent calcium indicator, Fluo-4 AM (Dojindo Molecular Technologies, Inc., #F311), 20 µL of dimethyl sulfoxide (DMSO), 1 µL of 10% Pluronic F-127 (Thermo Fisher Scientific, #P6866), and 4 mL of buffer A. After the cells were left to stand for 45 minutes at room temperature under light-shielded conditions, and then the cells were washed with buffer B. DMSO as a negative control and various test compound solutions each obtained by dissolving a test compound dissolved in DMSO, in buffer B so as to obtain a 10-fold concentration of the evaluation concentration, were introduced into respective wells. The final concentration of DMSO was adjusted to 0.1%. The cells were left to stand for another 15 minutes at room temperature under light-shielded conditions, and measurement was made using a microplate reader, EnVision (PerkinElmer Inc.) using the following method. First, the background fluorescence of each well was measured, subsequently an ionophore was introduced into each well, and the baseline fluorescence was measured. Subsequently, buffer B having 83.8 mM KCl added thereto was introduced, and an increase in fluorescence caused by calcium influx generated by the stimulus was measured for 10 seconds. The percentage inhibition was calculated from the maximum fluorescence increase value from the baseline, the logarithmic value of the test compound concentration and the inhibitory activity are plotted, and thereby the IC₅₀ value was calculated. The measurement was carried out by observing the fluorescence at 510 nm that was emitted when the cells were irradiated with excitation light at 485 nm.

### (Buffer composition)

Buffer A: 140 mM NaCl, 5 mM KCl, 1 mM MgCl₂, 0.5 mM CaCl₂, 10 mM Glucose, 10 mM HEPES, pH 7.3
Buffer B: 137.9 mM Choline-Cl, 4.1 mM KCl, 1 mM MgCl₂, 0.5 mM CaCl₂, 10 mM Glucose, 10 mM HEPES, pH 7.3, equilibrium potential of potassium: -92 mV

### (Test results)

The test results are presented in Tables 1 to 4.

**[Table 1]**

| Test compound | IC₅₀ (µM) | Test compound | IC₅₀ (µM) | Test compound | IC₅₀ (µM) |
|---|---|---|---|---|---|
| Example 1 | 0.013 | Example 65 | 8.9 | Example 126 | 0.83 |
| Example 2 | 1.3 | Example 66 | 0.069 | Example 127 | 1.1 |
| Example 3 | 0.85 | Example 67 | 0.029 | Example 128 | >10 |
| Example 4 | 0.014 | Example 68 | 0.075 | Example 129 | 0.55 |
| Example 5 | 0.29 | Example 69 | 1.2 | Example 130 | >10 |
| Example 6 | 0.13 | Example 70 | 0.21 | Example 131 | 0.20 |
| Example 7 | 0.097 | Example 71 | 0.0079 | Example 132 | 0.19 |
| Example 8 | 0.20 | Example 72 | 0.21 | Example 133 | 0.055 |
| Example 9 | 0.48 | Example 73 | 1.0 | Example 134A | 0.93 |
| Example 10 | 0.41 | Example 74 | 0.13 | Example 134B | 1.0 |
| Example 11 | 0.18 | Example 75 | 0.31 | Example 135 | 0.23 |
| Example 12 | 0.28 | Example 76 | 0.067 | Example 136 | 1.5 |
| Example 13 | 0.018 | Example 77 | 0.20 | Example 137A | 0.10 |
| Example 14 | 5.2 | Example 78 | 0.27 | Example 137B | 0.82 |
| Example 15 | 0.37 | Example 79 | 0.15 | Example 138 | 0.69 |
| Example 16 | 0.32 | Example 80 | 0.72 | Example 139 | 0.0054 |

**[Table 2]**

| | | | | | |
|---|---|---|---|---|---|
| Example 17 | 0.38 | Example 81 | 0.55 | Example 140 | 1.1 |
| Example 18 | 0.090 | Example 82 | 0.75 | Example 141 | 0.18 |
| Example 19 | 3.5 | Example 83 | 0.082 | Example 142 | 0.0094 |
| Example 20 | >10 | Example 84 | 0.18 | Example 143 | 0.12 |
| Example 21 | 1.2 | Example 85 | 0.11 | Example 144 | 0.022 |
| Example 22 | 1.6 | Example 86 | 0.37 | Example 145 | 0.13 |
| Example 23 | 0.15 | Example 87 | 0.0054 | Example 146 | 0.023 |
| Example 24 | 0.72 | Example 88 | 0.019 | Example 147 | 0.17 |
| Example 25 | 2.0 | Example 89 | 0.43 | Example 148 | 0.20 |
| Example 26 | 0.15 | Example 90 | 0.13 | Example 149 | 0.069 |
| Example 27 | 0.70 | Example 91 | 0.032 | Example 150 | 0.40 |
| Example 28 | 0.20 | Example 92 | 0.072 | Example 151 | 2.7 |
| Example 29 | 0.040 | Example 93 | 0.34 | Example 152 | 1.3 |
| Example 30 | 0.65 | Example 94 | 0.91 | Example 153 | 0.57 |
| Example 31 | 0.21 | Example 95 | 0.23 | Example 154 | 1.0 |
| Example 32 | 0.15 | Example 96 | 0.41 | Example 155 | 0.075 |

**[Table 3]**

| | | | | | |
|---|---|---|---|---|---|
| Example 33 | 1.0 | Example 97 | 0.25 | Example 156 | 1.3 |
| Example 34 | 0.24 | Example 98 | 1.1 | Example 157 | 0.28 |
| Example 35 | 0.63 | Example 99A | 0.090 | Example 158 | 1.1 |
| Example 36 | 0.98 | Example 99B | 0.48 | Example 159 | 0.51 |
| Example 37 | 0.027 | Example 100A | 0.0093 | Example 160 | 6.4 |
| Example 38 | 0.035 | Example 100B | 0.46 | Example 161 | 0.26 |
| Example 39 | 0.022 | Example 101 | 0.10 | Example 162 | 0.56 |
| Example 40 | 0.30 | Example 102 | 0.15 | Example 163 | 1.4 |
| Example 41 | 0.025 | Example 103 | 0.14 | Example 164 | 2.9 |
| Example 42 | 0.25 | Example 104 | 0.46 | Example 165 | 0.030 |
| Example 43 | 0.19 | Example 105 | 0.24 | Example 166 | 0.22 |
| Example 44 | 0.014 | Example 106A | 0.011 | Example 167 | 0.16 |
| Example 45 | 0.011 | Example 106B | 0.4 | Example 168 | 0.17 |
| Example 46 | 0.26 | Example 107A | 0.0047 | Example 169 | >10 |
| Example 47 | 0.35 | Example 107B | 0.56 | Example 170 | 0.88 |
| Example 48 | 0.24 | Example 108A | 0.0057 | Example 171 | 0.11 |
| Example 49 | 0.0071 | Example 108B | 0.2 | Example 172 | 0.55 |

**[Table 4]**

| | | | | | |
|---|---|---|---|---|---|
| Example 50 | 0.68 | Example 109 | 0.24 | Example 173 | 1.1 |
| Example 51 | 0.12 | Example 110 | 0.29 | Example 174 | 0.20 |
| Example 52 | 0.11 | Example 111 | 9. 9 | Example 175 | 0.49 |
| Example 53 | 1.0 | Example 112 | 0.11 | Example 176 | 0.0073 |
| Example 54 | 0.087 | Example 113 | 0.10 | Example 177 | 0.30 |
| Example 55 | 0.47 | Example 114 | 1.2 | Example 178 | 0. 59 |
| Example 56 | 0.29 | Example 115 | 0.091 | Example 179 | 0.24 |
| Example 57 | 0.020 | Example 116 | 0.040 | Example 180 | 2.1 |
| Example 58 | 4.4 | Example 117 | 0.071 | Example 181 | 0.025 |
| Example 59 | 1.8 | Example 118 | 2.1 | Example 182 | 0.72 |
| Example 60 | 0.47 | Example 119 | 2.3 | Example 183 | 0.20 |
| Example 61A | 1.1 | Example 120 | 0.82 | Example 184 | 3.0 |
| Example 61B | 2.2 | Example 121 | 0.12 | Example 185 | 1.4 |
| Example 62 | 0.41 | Example 122 | 1.5 | Example 186 | 0.19 |
| Example 63A | 0.19 | Example 123 | 0.044 | Example 187 | 0.51 |
| Example 63B | >10 | Example 124 | 0.062 | | |
| Example 64 | 0.037 | Example 125 | >10 | | |

### Example 189

### Pharmacological test 2

### Rat formalin test

### (Testing method)

30 mg/kg of a vehicle or a test substance was orally administrated to SD rats once, and according to the method described in McNamara C.R., Mandel-Brehm J., Bautista D.M., Siemens J., Deranian K.L., Zhao M., Hayward N.J., Chong J.A., Julius D., Moran M.M., Fanger C.M.: TRPA1 mediates formalin-induced pain. Proc. Natl. Acad. Sci. U.S.A. 104, 2007, 13525-13530, 50 µL of a 2.0% formalin solution as a pain-inducing substance was subcutaneously administered under the skin of the plantar plate of the right hind limb of each rat.

Regarding the analysis, pain behavior of the rats such as licking or biting of the site of formalin administration exhibited for 45 minutes after the formalin administration was cumulatively measured in seconds at an interval of 5 minutes, and the pain behavior time was calculated. Regarding the evaluation, the pain behavior inhibition ratio was determined from the pain behavior time of each test substance-administered group with respect to the pain behavior time of the vehicle-administered group for 15 minutes measured from 15 minutes to 30 minutes after the administration.

### (Test results)

The test results are presented in Table 5.

**[Table 5]**

| Test compound | Inhibition ratio | Test compound | Inhibition ratio | Test compound | Inhibition ratio |
|---|---|---|---|---|---|
| Example 1 | +++ | Example 57 | + | Example 113 | ++ |
| Example 4 | +++ | Example 70 | + | Example 117 | +++ |
| Example 5 | +++ | Example 71 | + | Example 121 | + |
| Example 8 | + | Example 77 | + | Example 123 | +++ |
| Example 11 | +++ | Example 79 | + | Example 124 | +++ |
| Example 12 | ++ | Example 84 | + | Example 131 | + |
| Example 23 | + | Example 85 | + | Example 135 | + |
| Example 36 | + | Example 99A | + | Example 137 | +++ |
| Example 37 | + | Example 100A | +++ | Example 141 | + |
| Example 39 | +++ | Example 109 | +++ | Example 144 | +++ |
| Example 49 | +++ | Example 110 | +++ | Example 181 | +++ |
| Example 54 | + | Example 112 | +++ | | |

| | | | | | |
|---|---|---|---|---|---|
| * Blocking ratio ≥ 20%; +++, 20% > blocking ratio ≥ 10%; ++, 10% > blocking ratio; + | | | | | |

### Example 190

### Pharmacological test 3

### Mechanical allodynia in mouse partial sciatic nerve ligation (PSNL) model

### (Testing method)

The production of a mouse PSNL model was achieved according to the method of Seltzer et al. A mouse was subjected to inhalation anesthesia with isoflurane. The femoral region of the mouse was shaved and disinfected with isodine. The skin on a thighbone of the mouse was incised, the fascia directly under the skin was cut, and the muscle heads of the musculus biceps femoris were split. The sciatic nerve directly under the fascia was checked, and then the sciatic nerve was detached so as not to damage the nerve. The needle of a needle with 8-0 silk thread was passed through the sciatic nerve to penetrate therethrough, and the sciatic nerve was ligated for about 1/2 to 1/3 of the whole nerve. The skin was sutured, and the mouse was raised for about 7 days. For the surgical operation of a Sham control moue, only the checking of the sciatic nerve was carried out, and the skin was sutured.

The measurement of the pain threshold was carried out after transferring the mice into a cage for measurement and waiting for the exploratory behavior to disappear. Regarding the pain threshold (paw withdrawal filament ((g)), the plantar plates of the mice were stimulated with von Frey filaments (Sttoelting Co.: TOUCH-TEST SENSORY EVLUATOR) having different stimulus intensities, and the presence or absence of an escape response was observed. The 50% threshold was calculated by an up-down method with reference to the method of Chaplan et al. (Quantitative assessment of tactile allodynia in the rat paw. J. Neurosci. Methods, 53, 1994, 55-63).

Regarding the efficacy evaluation, the compound described in Example 4 or Example 144 was orally administered to PSNL, and the effect on pain threshold was observed through a von Frey filament test.

### (Test results)

The results are presented in Fig. 1 and Fig. 2.

As is obvious from Fig. 1, the compound described in Example 4 inhibited mechanical allodynia statistically significantly compared with the vehicle group, when 30 mg/kg of the compound was orally administered. Furthermore, as is obvious from Fig. 2, the compound described in Example 144 inhibited mechanical allodynia statistically significantly compared to the vehicle group, when 30 mg/kg of the compound was orally administered.

### Industrial Applicability

The compounds of the present invention can be used as pain treatment drugs.

### Reference Signs List

In Fig. 1, ○ represents Sham + Vehicle.
In Fig. 1, ▲ represents PSNL + Vehicle.
In Fig. 1, ■ represents PSNL + 10 mg/kg of Example 4.
In Fig. 1, ▼ represents PSNL + 30 mg/kg of Example 4.
In Fig. 2, ○ represents Sham + Vehicle.
In Fig. 2, ▲ represents PSNL + Vehicle.
In Fig. 2, ■ represents PSNL + 10 mg/kg of Example 144.
In Fig. 2, ▼ represents PSNL + 30 mg/kg of Example 144.

## Claims

1. A compound represented by the following General Formula (I), a tautomer or a stereoisomer of the compound, a pharmaceutically acceptable salt of the compound, or a solvate of the compound, the tautomer, the stereoisomer, or the salt: 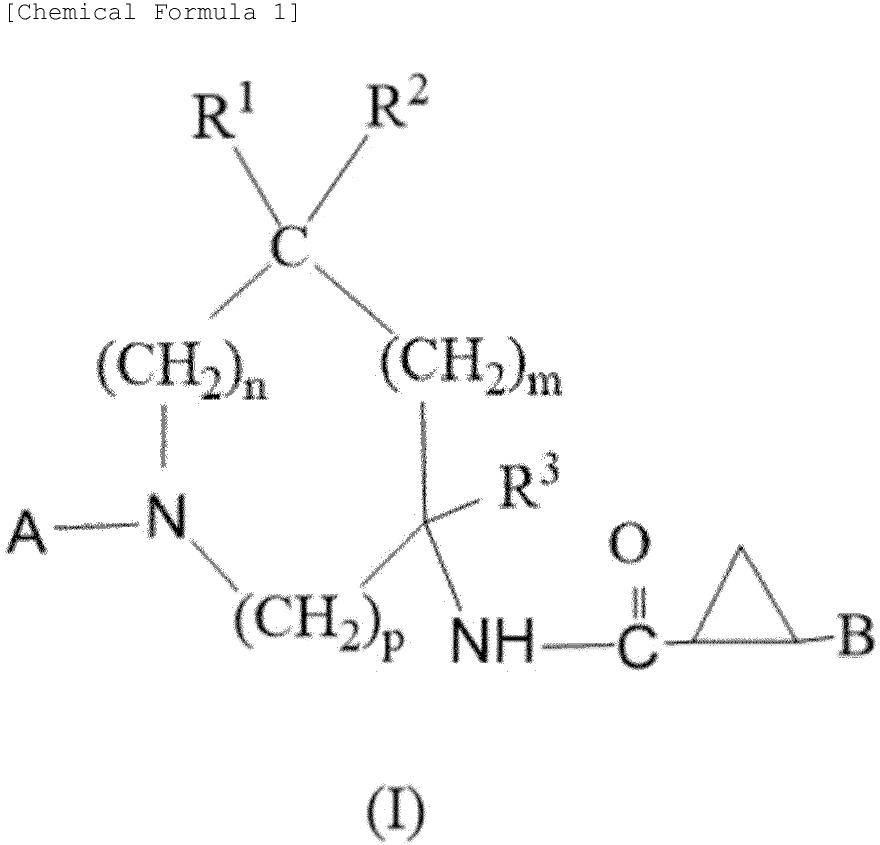
wherein A represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent cyclic amine by means of a carbon atom constituting these rings;
B represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to the adjacent cyclopropane ring by means of a carbon atom constituting these rings;
R¹ and R², which may be identical or different, each represent a hydrogen atom, a halogen atom, a hydroxy group, a C₁₋₈ alkyl group, or a C₁₋₈ alkyl group substituted with one to three halogen atoms;
or R¹, R², and the carbon atom to which R¹ and R² are bonded may be joined together and form a 3- to 5-membered cycloalkyl group;
R³ represents a hydrogen atom, a halogen atom, a carboxyl group, a cyano group, a carbamoyl group, a C₁₋₈ alkyl group, a C₁₋₈ alkoxycarbonyl group, a C₁₋₈ alkyl group substituted with a C₁₋₈ alkoxy group, a C₁₋₈ alkyl group substituted with a hydroxy group, a C₁₋₈ alkyl group substituted with one to three halogen atoms, or a C₁₋₈ alkyl group substituted with an acyloxy group;
or R² and R³ may be joined together and form methylene or ethylene;
n and m, which may be identical or different, each represent 0 or 1; and p represents 1 or 2;
further, the substituent that may be carried by the phenyl of A, the heteroaryl ring of A, and the heterocondensed ring of A is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B, the heteroaryl ring of B, and the heterocondensed ring of B is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B, the heteroaryl ring of B, and the heterocondensed ring of B is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

2. The compound according to claim 1, a tautomer or a stereoisomer of the compound, a pharmaceutically acceptable salt of the compound, or a solvate of the compound, the tautomer, the stereoisomer, or the salt, wherein A represents a phenyl which may have a substituent.

3. The compound according to claim 1, a tautomer or a stereoisomer of the compound, a pharmaceutically acceptable salt of the compound, or a solvate of the compound, the tautomer, the stereoisomer, or the salt, wherein A represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent.

4. The compound according to claim 3, a tautomer or a stereoisomer of the compound, a pharmaceutically acceptable salt of the compound, or a solvate of the compound, the tautomer, the stereoisomer, or the salt, wherein the heteroaryl ring moiety is thiophene, oxazole, thiazole, pyridine, pyrazine, pyrimidine, or pyridazine.

5. The compound according to claim 1, a tautomer or a stereoisomer of the compound, a pharmaceutically acceptable salt of the compound, or a solvate of the compound, the tautomer, the stereoisomer, or the salt, wherein A represents a heterocondensed ring which may have a substituent, and the heterocondensed ring moiety is quinoline or benzo[d]thiazole.

6. A compound represented by the following General Formula (II), a tautomer or a stereoisomer of the compound, a pharmaceutically acceptable salt of the compound, or a solvate of the compound, the tautomer, the stereoisomer, or the salt: 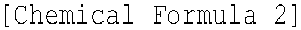 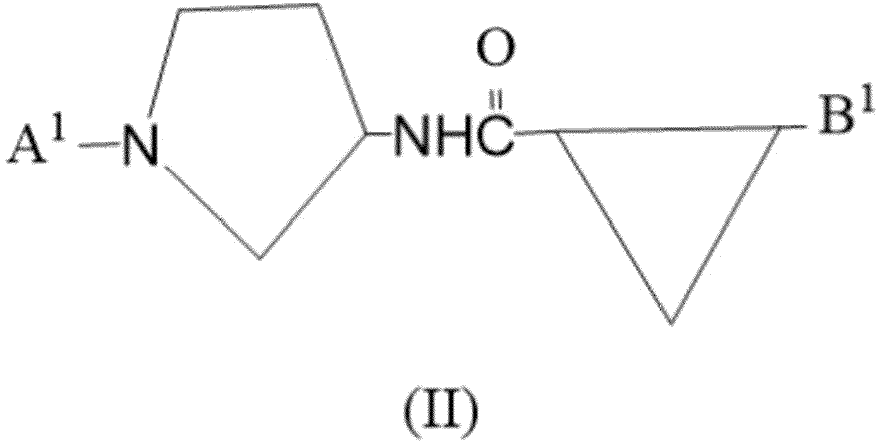
wherein A¹ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent pyrrolidine by means of a carbon atom constituting these rings; and
B¹ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to the adjacent cyclopropane ring by means of a carbon atom constituting these rings;
further, the substituent that may be carried by the phenyl of A¹, the heteroaryl ring of A¹, and the heterocondensed ring of A¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B¹, the heteroaryl ring of B¹, and the heterocondensed ring of B¹ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B¹, the heteroaryl ring of B¹, and the heterocondensed ring of B¹ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group.

7. The compound according to claim 6, a tautomer or a stereoisomer of the compound, a pharmaceutically acceptable salt of the compound, or a solvate of the compound, the tautomer, the stereoisomer, or the salt, wherein B¹ represents a phenyl which may have a substituent.

8. The compound according to claim 6, a tautomer or a stereoisomer of the compound, a pharmaceutically acceptable salt of the compound, or a solvate of the compound, the tautomer, the stereoisomer, or the salt, wherein B¹ represents an optionally substituted heterocondensed ring of a 5-membered or 6-membered heteroaryl ring composed of one to three identical or different heteroatoms selected from an oxygen atom, a sulfur atom, and a nitrogen atom and carbon atoms as ring-constituting atoms, and a benzene ring.

9. A compound represented by the following General Formula (III), a tautomer or a stereoisomer of the compound, a pharmaceutically acceptable salt of the compound, or a solvate of the compound, the tautomer, the stereoisomer, or the salt: 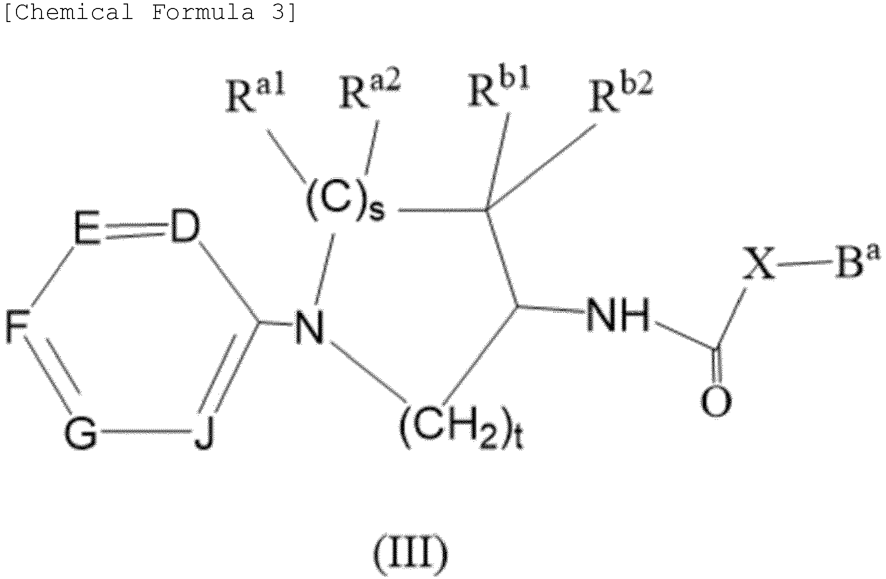
wherein D, E, F, G, and J are such that any two of them each represent N while the others represent CRs which may be identical or different, or any one of them represents N while the others represent CRs which may be identical or different, or all of them are CRs which may be identical or different;
wherein R represents a hydrogen atom, a C₁₋₈ alkyl group, a C₁₋₈ alkoxy group, a C₁₋₈ alkyl group substituted with one to three halogen atoms, a C₁₋₈ alkoxy group which may be substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, or a C₁₋₈ alkoxy group substituted with a C₁₋₈ alkoxycarbonyl group;
R^{a1}, R^{a2}, R^{b1}, and R^{b2}, which may be identical or different, each represent a hydrogen atom, a halogen atom, a hydroxy group, a C₁₋₈ alkyl group, or a C₁₋₈ alkyl group substituted with one to three halogen atoms;
or R^{a1}, R^{a2}, and the carbon atom to which R^{a1} and R^{a2} are bonded may be joined together and form a 3- to 5-membered cycloalkyl group, or R^{b1}, R^{b2}, and the carbon atom to which R^{b1} and R^{b2} are bonded may be joined together and form a 3- to 5-membered cycloalkyl group; s represents 0, 1, or 2; t represents 1 or 2;
B^{a} represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to adjacent X by means of a carbon atom constituting these rings;
X represents: 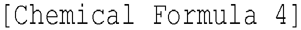 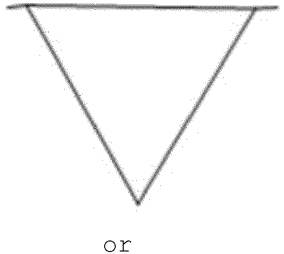 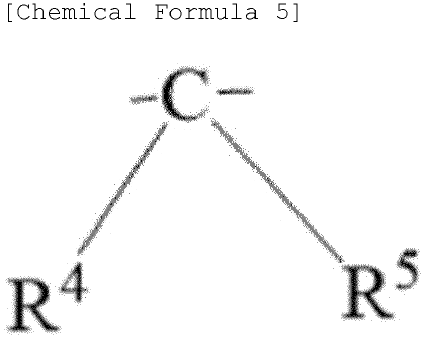 wherein - represents a linking bond;
R⁴ and R⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group;
or R⁴, R⁵, and the carbon atom to which R⁴ and R⁵ are bonded may be joined together and form a 3- to 5-membered cycloalkyl group;
further, the substituent that may be carried by the phenyl of B^{a}, the heteroaryl ring of B^{a}, and the heterocondensed ring of B^{a} is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri(C₁₋₈ alkyl)silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B^{a}, the heteroaryl ring of B^{a}, and the heterocondensed ring of B^{a} is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that, when a 6-membered ring composed of D to J may be a phenyl which may have a substituent, or a pyridazine which may have a substituent, and X represents the following: 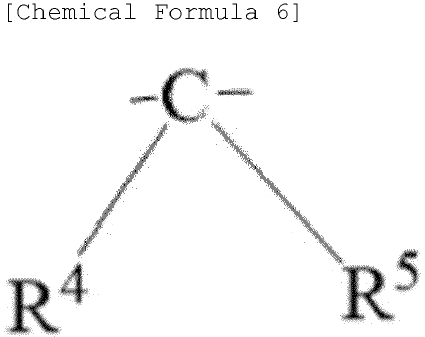
B^{a} represents a heterocondensed ring which may have the above-mentioned substituent.

10. The compound according to claim 9, a tautomer or a stereoisomer of the compound, a pharmaceutically acceptable salt of the compound, or a solvate of the compound, the tautomer, the stereoisomer, or the salt, wherein B^{a} represents a phenyl which may have a substituent.

11. The compound according to claim 9, a tautomer or a stereoisomer of the compound, a pharmaceutically acceptable salt of the compound, or a solvate of the compound, the tautomer, the stereoisomer, or the salt, wherein B^{a} represents a 5-membered or 6-membered heteroaryl ring composed of one to three identical or different heteroatoms selected from an oxygen atom, a sulfur atom, and a nitrogen atom and carbon atoms as ring-constituting atoms, which may have a substituent, and the heteroaryl ring moiety is pyridine.

12. The compound according to claim 9, a tautomer or a stereoisomer of the compound, a pharmaceutically acceptable salt of the compound, or a solvate of the compound, the tautomer, the stereoisomer, or the salt, wherein B^{a} represents a heterocondensed ring which may have a substituent, and the heterocondensed ring moiety is indole, benzimidazole, indazole, benzoxazole, benzothiazole, benzo[d][1,2,3]triazole, or quinoline.

13. A compound represented by the following General Formula (IV), a tautomer or a stereoisomer of the compound, a pharmaceutically acceptable salt of the compound, or a solvate of the compound, the tautomer, the stereoisomer, or the salt: 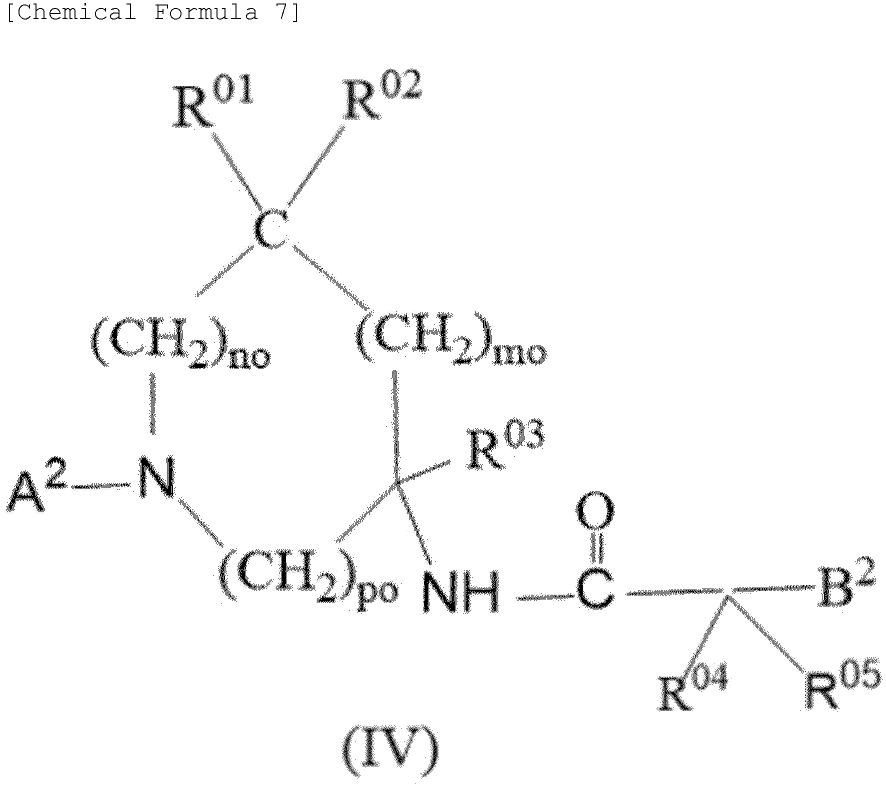
wherein A² represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent cyclic amine by means of a carbon atom constituting these rings;
B² represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to adjacent C(R⁰⁴) (R⁰⁵) by means of a carbon atom constituting these rings;
R⁰¹ and R⁰², which may be identical or different, each represent a hydrogen atom, a halogen atom, a hydroxy group, a C₁₋₈ alkyl group, or a C₁₋₈ alkyl group substituted with one to three halogen atoms;
or R⁰¹, R⁰², and the carbon atom to which R⁰¹ and R⁰² are bonded may be joined together and form a 3- to 5-membered cycloalkyl group;
R⁰³ represents a hydrogen atom, a halogen atom, a carboxyl group, a cyano group, a carbamoyl group, a C₁₋₈ alkyl group, a C₁₋₈ alkoxycarbonyl group, a C₁₋₈ alkyl group substituted with a C₁₋₈ alkoxy group, a C₁₋₈ alkyl group substituted with a hydroxy group, a C₁₋₈ alkyl group substituted with one to three halogen atoms, or a C₁₋₈ alkyl group substituted with an acyloxy group;
or R⁰² and R⁰³ may be joined together and form methylene or ethylene;
R⁰⁴ and R⁰⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group;
or R⁰⁴, R⁰⁵, and the carbon atom to which R⁰⁴ and R⁰⁵ are bonded may be joined together and form a 3- to 5-membered cycloalkyl group;
no and mo, which may be identical or different, each represent 0 or 1; and po represents 1 or 2;
further, the substituent that may be carried by the phenyl of A², the heteroaryl ring of A², and the heterocondensed ring of A² is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B², the heteroaryl ring of B², and the heterocondensed ring of B² is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B², the heteroaryl ring of B², and the heterocondensed ring of B² is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that, when A² represents a phenyl which may have a substituent, a pyridazine which may have a substituent, and a quinazoline which may have a substituent, B² represents a heterocondensed ring which may have the above-mentioned substituent.

14. The compound according to claim 13, a tautomer or a stereoisomer of the compound, a pharmaceutically acceptable salt of the compound, or a solvate of the compound, the tautomer, the stereoisomer, or the salt, wherein A² represents a phenyl which may have a substituent.

15. The compound according to claim 13, a tautomer or a stereoisomer of the compound, a pharmaceutically acceptable salt of the compound, or a solvate of the compound, the tautomer, the stereoisomer, or the salt, wherein A² represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent, and the heteroaryl ring moiety is thiophene, oxazole, thiazole, pyridine, pyrazine, pyrimidine, or pyridazine.

16. The compound according to claim 13, a tautomer or a stereoisomer of the compound, a pharmaceutically acceptable salt of the compound, or a solvate of the compound, the tautomer, the stereoisomer, or the salt, wherein A² represents a heterocondensed ring which may have a substituent, and the heterocondensed ring moiety is quinoline or benzo[d]thiazole.

17. A compound represented by the following General Formula (V), a tautomer or a stereoisomer of the compound, a pharmaceutically acceptable salt of the compound, or a solvate of the compound, the tautomer, the stereoisomer, or the salt: 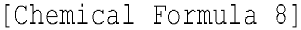 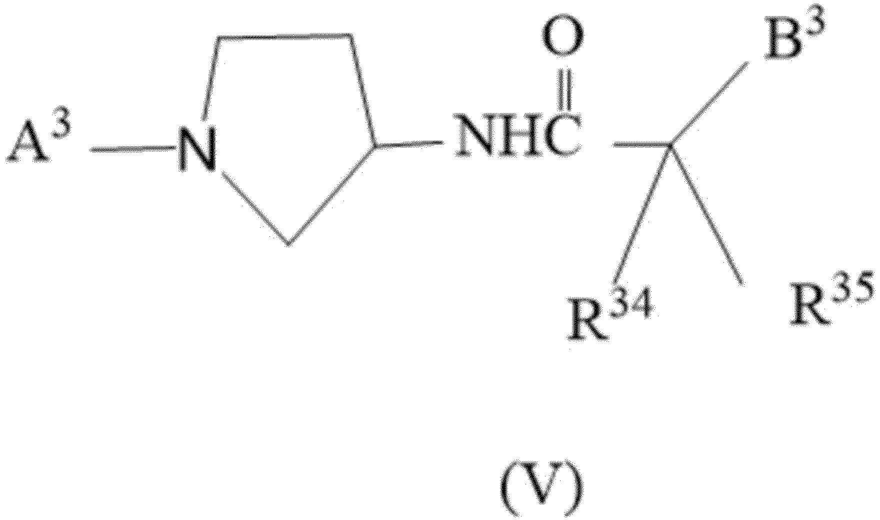
wherein A³ represents a phenyl which may have a substituent, a 4- to 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to a nitrogen atom of the adjacent pyrrolidine by means of a carbon atom constituting these rings; and
B³ represents a phenyl which may have a substituent, a 5- or 6-membered heteroaryl ring composed of one to three identical or different heteroatoms selected from an oxygen atom, a sulfur atom, and a nitrogen atom and carbon atoms as ring-constituting atoms, or a heterocondensed ring composed of the heteroaryl ring and either a benzene ring or a 6-membered heteroaryl ring composed of one to two nitrogen atoms and carbon atoms, wherein the heteroaryl ring or the heterocondensed ring may have a substituent and is bonded to adjacent C(R³⁴)(R³⁵) by means of a carbon atom constituting these rings;
R³⁴ and R³⁵, which may be identical or different, each represent a hydrogen atom, deuterium, a hydroxy group, a C₁₋₈ alkyl group, a C₁₋₈ alkyl group substituted with one to three halogen atoms, an amino group, a C₁₋₈ alkylamino group, or a C₂₋₁₂ dialkylamino group;
or R³⁴, R³⁵, and the carbon atom to which R³⁴ and R³⁵ are bonded may be joined together and form a 3- to 5-membered cycloalkyl group;
further, the substituent that may be carried by the phenyl of A³, the heteroaryl ring of A³, and the heterocondensed ring of A³ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a C₁₋₈ alkylsulfonyl group, and a C₁₋₈ alkoxy group substituted with a C₁₋₈ alkoxycarbonyl group;
the substituent that may be carried by the phenyl of B³, the heteroaryl ring of B³, and the heterocondensed ring of B³ is selected from a C₁₋₈ alkyl group, a C₁₋₈ alkoxy group, a C₁₋₈ alkyl group substituted with one to three halogen atoms, a C₁₋₈ alkoxy group substituted with one to three halogen atoms, a C₁₋₈ alkyl group substituted with a hydroxy group, a C₁₋₈ alkoxy group substituted with a hydroxy group, a hydroxy group, a halogen atom, a cyano group, a nitro group, an amino group, a C₁₋₈ alkylamino group, a C₂₋₁₂ dialkylamino group, a (C₁₋₈ alkyl) (C₁₋₈ alkoxy-substituted C₁₋₈ alkyl) amino group, a tri (C₁₋₈ alkyl) silyl group, an acylamino group, an (N-acyl)(N-C₁₋₈ alkyl)amino group, a 3- to 6-membered cyclic ether, a cyclic amino group, or a 4- to 6-membered heteroaryl ring composed of one to three identical or different heteroatoms selected from an oxygen atom, a sulfur atom, and a nitrogen atom and carbon atoms as ring-constituting atoms, which may be substituted with a substituent selected from a halogen atom, a C₁₋₈ alkyl group, a C₁₋₈ alkoxy group, a C₁₋₈ alkyl group substituted with one to three halogen atoms, and a C₁₋₈ alkoxy group substituted with one to three halogen atoms, as a substituent; and
wherein the cyclic amino group of the substituent that may be carried by the phenyl of B³, the heteroaryl ring of B³, and the heterocondensed ring of B³ is selected from pyrrolidino, piperidino, piperazino, 2- or 3-oxopyrrolidino, 2-, 3-, or 4-oxopiperidino, morpholino, 1,1-dioxide thiomorpholino, 1,4-dioxa-8-azaspiro[4.5]decan-8-yl, 2-oxa-6-azaspiro[3.3]heptan-6-yl, 3-oxa-8-azabicyclo[3.2.1]octan-8-yl, 2-oxa-5-azabicyclo[2.2.2]octan-5-yl, and 2-oxa-5-azabicyclo[2.2.1]heptan-5-yl, while such a cyclic amino group may be further substituted with a C₁₋₈ alkyl group, a halogen atom, or an acyl group;
provided that, when A³ represents a phenyl which may have a substituent, a pyridazine which may have a substituent, and a quinazoline which may have a substituent, B³ represents a heterocondensed ring which may have the above-mentioned substituent.

18. The compound according to claim 17, a tautomer or a stereoisomer of the compound, a pharmaceutically acceptable salt of the compound, or a solvate of the compound, the tautomer, the stereoisomer, or the salt, wherein A³ represents a 4-membered to 6-membered heteroaryl ring composed of one to three identical or different heteroatoms selected from an oxygen atom, a sulfur atom, and a nitrogen atom and carbon atoms as ring-constituting atoms, which may have a substituent, and the heteroaryl ring moiety is thiophene, oxazole, thiazole, pyridine, pyrazine, pyrimidine, or pyridazine.

19. The compound according to claim 17, a tautomer or a stereoisomer of the compound, a pharmaceutically acceptable salt of the compound, or a solvate of the compound, the tautomer, the stereoisomer, or the salt, wherein A³ represents a heterocondensed ring which may have a substituent, and the heterocondensed ring moiety is quinoline or benzo[d]thiazole.

20. A compound represented by the following General Formula (VI), a tautomer or a stereoisomer of the compound, a pharmaceutically acceptable salt of the compound, or a solvate of the compound, the tautomer, the stereoisomer, or the salt: [Chemical Formula 9] 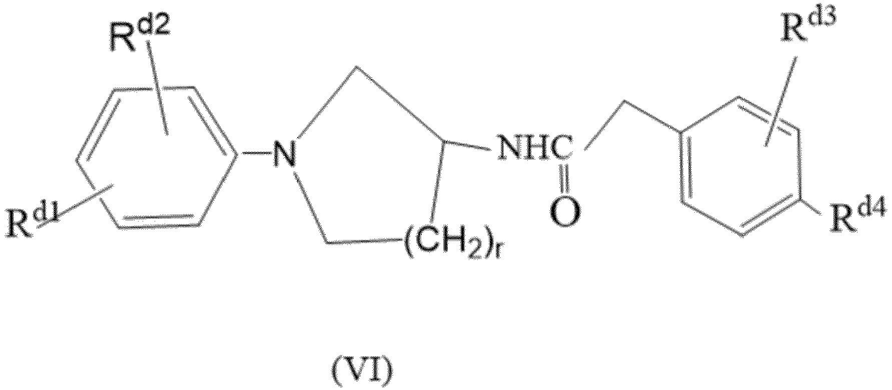
wherein R^{d1}, R^{d2}, and R^{d3}, which are identical or different, each represent a hydrogen atom, a halogen atom, a C₁₋₈ alkyl group, a C₁₋₈ alkyl group substituted with one to three halogen atoms, a C₁₋₈ alkoxy group, a C₁₋₈ alkoxy group substituted with one to three halogen atoms, a hydroxy group, or a C₁₋₈ alkoxy group substituted with a C₁₋₈ alkoxycarbonyl group;
R^{d4} represents a C₁₋₈ alkyl substituted with one to three halogen atoms, a tert-butyl, or a cyclopropyl;
and r represents 0, 1, or 2.

21. A compound selected from the following, a tautomer or a stereoisomer of the compound, a pharmaceutically acceptable salt of the compound, or a solvate of the compound, the tautomer, the stereoisomer, or the salt:
(R)-2-(4-cyclopropylphenyl)-N-(1-(5-(trifluoromethyl)pyridin-3-yl)pyrrolidin-3-yl)acetamide,
(R)-2-(5-(trifluoromethyl)pyridin-2-yl)-N-(1-(5-(trifluoromethyl)pyridin-3-yl)pyrrolidin-3-yl)acetamide,
(R)-2-(4-isopropylphenyl)-N-(1-(2-(trifluoromethyl)pyrimidin-5-yl)pyrrolidin-3-yl)acetamide,
(R)-2-(4-isopropylphenyl)-N-(1-(5-(trifluoromethyl)pyridin-3-yl)pyrrolidin-3-yl)acetamide,
2-(4-cyclopropylphenyl)-N-((3S,4S)-4-hydroxy-1-(5-(trifluoromethyl)pyridin-3-yl)pyrrolidin-3-yl)acetamide,
(R)-2-(4-cyclopropylphenyl)-N-(5-(5-(trifluoromethyl)pyridin-3-yl)-5-azaspiro[2.4]heptan-7-yl)acetamide,
(R)-2-(4-cyclopropylphenyl)-N-(5-(6-(trifluoromethyl)pyridin-3-yl)-5-azaspiro[2.4]heptan-7-yl)acetamide,
(R)-2-(4-cyclopropylphenyl)-N-(5-(4-(trifluoromethyl)thiazol-2-yl)-5-azaspiro[2.4]heptan-7-yl)acetamide,
2-(4-cyclopropylphenyl)-N-((3R,5S)-5-methyl-l-(5-(trifluoromethyl)pyridin-3-yl)pyrrolidin-3-yl)acetamide,
2-(4-cyclopropylphenyl)-N-((1S,5R)-3-(5-(trifluoromethyl)pyridin-3-yl)-3-azabicyclo[3.1.0]hexan-1-yl)acetamide,
2-(4-cyclopropylphenyl)-N-((1R,5S)-3-(5-(trifluoromethyl)pyridin-3-yl)-3-azabicyclo[3.1.0]hexan-1-yl)acetamide,
(R)-2-(4-(trifluoromethyl)phenyl)-N-(1-(6-(trifluoromethyl)pyrazin-2-yl)pyrrolidin-3-yl)acetamide,
(R)-N-(1-(6-cyano-5-(trifluoromethyl)pyridin-3-yl)pyrrolidin-3-yl)-2-(4-cyclopropylphenyl)acetamide,
methyl (R)-3-(2-(4-cyclopropylphenyl)acetamido)-1-(5-(trifluoromethyl)pyridin-3-yl)pyrrolidine-3-carboxylate,
methyl (S)-3-(2-(4-cyclopropylphenyl)acetamido)-1-(5-(trifluoromethyl)pyridin-3-yl)pyrrolidine-3-carboxylate,
(R)-3-(2-(4-cyclopropylphenyl)acetamido)-1-(5-(trifluoromethyl)pyridin-3-yl)pyrrolidine-3-carboxylic acid,
(S)-3-(2-(4-cyclopropylphenyl)acetamido)-1-(5-(trifluoromethyl)pyridin-3-yl)pyrrolidine-3-carboxylic acid,
(R)-2-(1H-indazol-6-yl)-N-(1-(5-(trifluoromethyl)pyridin-3-yl)pyrrolidin-3-yl)acetamide,
(R)-2-(4-cyclopropylphenyl)-N-(1-(4-(trifluoromethyl)thiazol-2-yl)pyrrolidin-3-yl)acetamide,
(R)-2-(4-isobutylphenyl)-N-((R)-1-(5-(trifluoromethyl)pyridin-3-yl)pyrrolidin-3-yl)propanamide,
(S)-2-(4-isobutylphenyl)-N-((R)-1-(5-(trifluoromethyl)pyridin-3-yl)pyrrolidin-3-yl)propanamide,
(R)-2-(4-cyclopropylphenyl)-N-(1-(6-methyl-5-(trifluoromethyl)pyridin-3-yl)pyrrolidin-3-yl)acetamide,
(R)-2-(4-cyclopropylphenyl)-N-(1-(5-(2-hydroxypropan-2-yl)pyridin-3-yl)pyrrolidin-3-yl)acetamide,
(R)-2-(4-cyclopropylphenyl)-N-(1-(8-(trifluoromethyl)imidazo[1,2-a]pyridin-6-yl)pyrrolidin-3-yl)acetamide,
(R)-3-(2-(4-cyclopropylphenyl)acetamido)-1-(5-(trifluoromethyl)pyridin-3-yl)pyrrolidine-3-carboxyamide,
(S)-3-(2-(4-cyclopropylphenyl)acetamido)-1-(5-(trifluoromethyl)pyridin-3-yl)pyrrolidine-3-carboxyamide,
(R)-2-(4-hydroxyphenyl)-N-(1-(5-(trifluoromethyl)pyridin-3-yl)pyrrolidin-3-yl)acetamide,
(R)-2-(4-(1,1-dioxide thiomorpholino)phenyl)-N-(1-(5-(trifluoromethyl)pyridin-3-yl)pyrrolidin-3-yl)acetamide,
(R)-1-(4-chlorophenyl)-N-(1-(5-(trifluoromethyl)pyridin-3-yl)pyrrolidin-3-yl)cyclopropane-1-carboxyamide,
(R)-2-(4-bromophenyl)-2-hydroxy-N-((R)-1-(5-(trifluoromethyl)pyridin-3-yl)pyrrolidin-3-yl)acetamide,
(S)-2-(4-bromophenyl)-2-hydroxy-N-((R)-1-(5-(trifluoromethyl)pyridin-3-yl)pyrrolidin-3-yl)acetamide,
(R)-N-(1-(5-(trifluoromethyl)pyridin-3-yl)pyrrolidin-3-yl)-2-(4-(trimethylsilyl)phenyl)acetamide,
(R)-2-(4-(2-hydroxy-2-methylpropoxy)phenyl)-N-(1-(5-(trifluoromethyl)pyridin-3-yl)pyrrolidin-3-yl)acetamide,
2-(4-cyclopropylphenyl)-N-((3S,4S)-4-fluoro-1-(5-(trifluoromethyl)pyridin-3-yl)pyrrolidin-3-yl)acetamide,
(R)-2-(4-(dimethylamino)phenyl)-N-(1-(5-(trifluoromethyl)pyridin-3-yl)pyrrolidin-3-yl)acetamide,
(R)-2-(4-nitrophenyl)-N-(1-(5-(trifluoromethyl)pyridin-3-yl)pyrrolidin-3-yl)acetamide,
(S)-2-(4-isobutylphenyl)-N-((R)-1-(5-(trifluoromethyl)pyridin-3-yl)pyrrolidin-3-yl)propanamide,
(R)-2-(3-fluoro-4-(trifluoromethyl)phenyl)-N-(1-(5-(trifluoromethyl)pyridin-3-yl)pyrrolidin-3-yl)acetamide,
(R)-2-(4-aminophenyl)-N-(1-(5-(trifluoromethyl)pyridin-3-yl)pyrrolidin-3-yl)acetamide,
(R)-1-(4-(trifluoromethyl)phenyl)-N-(1-(5-(trifluoromethyl)pyridin-3-yl)pyrrolidin-3-yl)cyclopropane-1-carboxyamide,
(R)-2-(4-acetamidophenyl)-N-(1-(5-(trifluoromethyl)pyridin-3-yl)pyrrolidin-3-yl)acetamide,
(R)-N-(3-cyano-1-(5-(trifluoromethyl)pyridin-3-yl)pyrrolidin-3-yl)-2-(4-cyclopropylphenyl)acetamide,
(S)-N-(3-cyano-1-(5-(trifluoromethyl)pyridin-3-yl)pyrrolidin-3-yl)-2-(4-cyclopropylphenyl)acetamide,
methyl (R)-3-(2-(1H-indol-6-yl)acetamido)-1-(5-(trifluoromethyl)pyridin-3-yl) pyrrolidine-3-carboxylat e,
methyl (S)-3-(2-(1H-indol-6-yl)acetamido)-1-(5-(trifluoromethyl)pyridin-3-yl) pyrrolidine-3-carboxylat e,
methyl (R)-3-(2-(4-(trifluoromethyl)phenyl)acetamido)-1-(5-(trifluoromethyl)pyridin-3-yl) pyrrolidine-3-carboxylat e,
methyl (S)-3-(2-(4-(trifluoromethyl)phenyl)acetamido)-1-(5-(trifluoromethyl)pyridin-3-yl) pyrrolidine-3-carboxylat e,
(1S,2S)-2-(benzo[d]oxazol-2-yl)-N-((R)-1-(5-(trifluoromethyl)pyridin-3-yl)pyrrolidin-3-yl)cyclopropane-1-carboxyamide,
(R)-2-(4-morpholinophenyl)-N-(1-(4-(trifluoromethyl)thiazol-2-yl)pyrrolidin-3-yl) acetamide,
methyl 3-(2-(4-morpholinophenyl)acetamido)-1-(5-(trifluoromethyl)pyridin-3-yl) pyrrolidine-3-carboxylat e,
(R)-2-(4-morpholinophenyl)-N-(1-(6-(trifluoromethyl)pyridin-3-yl)pyrrolidin-3-yl)acetamide,
(R)-2-(4-cyclopropylphenyl)-N-(3-ethyl-1-(5-(trifluoromethyl)pyridin-3-yl)pyrrolidin-3-yl)acetamide,
(S)-2-(4-cyclopropylphenyl)-N-(3-ethyl-1-(5-(trifluoromethyl)pyridin-3-yl)pyrrolidin-3-yl)acetamide,
(1S,2S)-2-(6-fluorobenzo[d]oxazol-2-yl)-N-((R)-1-(5-(trifluoromethyl)pyridin-3-yl)pyrrolidin-3-yl)cyclopropane-1-carboxyamide,
(lS,2S)-2-(5,6-difluorobenzo[d]oxazol-2-yl)-N-((R)-1-(5-(trifluoromethyl)pyridin-3-yl)pyrrolidin-3-yl)cyclopropane-1-carboxyamide,
(R)-2-(4-(2-oxa-6-azaspiro[3.3]heptan-6-yl)phenyl)-N-(1-(5-(trifluoromethyl)pyridin-3-yl)pyrrolidin-3-yl)acetamide,
(1S,2S)-2-(benzo[d]oxazol-2-yl)-N-((R)-1-(4-(trifluoromethyl)thiazol-2-yl)pyrrolidin-3-yl) cyclopropane-1-carboxyamide,
(1S,2S)-2-(6-fluorobenzo[d]oxazol-2-yl)-N-((R)-1-(6-(trifluoromethyl)pyridin-3-yl)pyrrolidin-3-yl)cyclopropane-1-carboxyamide,
(1S,2S)-2-(5-fluorobenzo[d]oxazol-2-yl)-N-((R)-1-(5-(trifluoromethyl)pyridin-3-yl)pyrrolidin-3-yl)cyclopropane-1-carboxyamide,
(1R,2R)-2-(benzo[d]oxazol-2-yl)-N-((R)-1-(5-(trifluoromethyl)pyridin-3-yl)pyrrolidin-3-yl)cyclopropane-1-carboxyamide,
(1S,2S)-2-(benzo[d]oxazol-2-yl)-N-((R)-1-(5-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(4-(trifluoromethyl)phenyl)pyrrolidin-3-yl)cyclopropane-1-carboxyamide,
(1R,2R)-2-(1H-benzo[d]imidazol-2-yl)-N-((R)-1-(4-(trifluoromethyl)phenyl)pyrrolidin-3-yl)cyclopropane-1-carboxyamide,
(1S,2S)-2-(1H-benzo[d]imidazol-2-yl)-N-((S)-1-(4-(trifluoromethyl)phenyl)pyrrolidin-3-yl)cyclopropane-1-carboxyamide,
(1R,2R)-2-(1H-benzo[d]imidazol-2-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((S)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(5-(trifluoromethyl)pyrimidin-2-yl)pyrrolidin-3-yl)cyclopropane-1-carboxyamide,
(1S,2S)-2-(1H-benzo[d]imidazol-2-yl)-N-((R)-1-(4-fluoro-3-(trifluoromethyl)phenyl)pyrrolidin-3-yl)cyclopropane-1-carboxyamide,
(1S,2S)-N-((R)-1-(4-fluoro-3-(trifluoromethyl)phenyl)pyrrolidin-3-yl)-2-(quinazolin-2-yl)cyclopropane-1-carboxyamide,
(1S,2S)-2-(1H-benzo[d]imidazol-2-yl)-N-((R)-1-(3-(methylsulfonyl)phenyl)pyrrolidin-3-yl)cyclopropane-1-carboxyamide,
(1S,2S)-2-(1H-benzo[d]imidazol-2-yl)-N-((R)-1-(3-cyanophenyl)pyrrolidin-3-yl)cyclopropane-1-carboxyamide,
(1S,2S)-2-(5-cyano-1H-benzo[d]imidazol-2-yl)-N-((R)-1-(6-(trifluoromethyl)pyridin-3-yl)pyrrolidin-3-yl)cyclopropane-1-carboxyamide,
(1R,2R)-2-(5-cyano-1H-benzo[d]imidazol-2-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(2-(trifluoromethyl)pyridin-4-yl)pyrrolidin-3-yl)cyclopropane-1-carboxyamide,
(1S,2S)-2-(1H-benzo[d]imidazol-2-yl)-N-((R)-1-(6-(trifluoromethyl)pyridin-3-yl)piperidin-3-yl)cyclopropane-1-carboxyamide,
(1R,2R)-2-(1H-benzo[d]imidazol-2-yl)-N-((R)-1-(6-(trifluoromethyl)pyridin-3-yl)piperidin-3-yl)cyclopropane-1-carboxyamide,
(1S,2S)-2-(1H-benzo[d]imidazol-2-yl)-N-((R)-1-(4-(trifluoromethyl)thiazol-2-yl)pyrrolidin-3-yl)cyclopropane-1-carboxyamide,
(1S,2S)-2-(1H-benzo[d]imidazol-2-yl)-N-((R)-1-(4-(trifluoromethyl)phenyl)piperidin-3-yl)cyclopropane-1-carboxyamide,
(1S,2S)-2-(1H-indol-3-yl)-N-((R)-1-(6-(trifluoromethyl)pyridin-3-yl)pyrrolidin-3-yl)cyclopropane-1-carboxyamide,
(1R,2R)-2-(1H-indol-3-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(6-methoxy-5-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(4-(tert-butyl)thiazol-2-yl)pyrrolidin-3-yl)cyclopropane-1-carboxyamide,
(1S,2S)-2-(1H-benzo[d]imidazol-2-yl)-N-((R)-1-(6-(trifluoromethyl)pyridazin-3-yl)pyrrolidin-3-yl)cyclopropane-1-carboxyamide,
(1S,2S)-2-(1H-benzo[d]imidazol-2-yl)-N-((R)-1-(2-(trifluoromethyl)pyrimidin-5-yl)pyrrolidin-3-yl)cyclopropane-1-carboxyamide,
(1S,2S)-2-(1H-benzo[d]imidazol-2-yl)-N-((R)-1-(5-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(6-fluorobenzo[d]thiazol-2-yl)pyrrolidin-3-yl)cyclopropane-1-carboxyamide,
(1S,2S)-2-(1-cyclopropyl-1H-benzo[d]imidazol-2-yl)-N-((R)-1-(6-(trifluoromethyl)pyridin-3-yl)pyrrolidin-3-yl)cyclopropane-1-carboxyamide,
(1S,2S)-2-(1H-benzo[d]imidazol-2-yl)-N-((R)-1-(6-fluoroquinolin-2-yl)pyrrolidin-3-yl)cyclopropane-1-carboxyamide,
(1S,2S)-2-(1-cyclopropyl-1H-benzo[d]imidazol-2-yl)-N-((R)-1-(3-(trifluoromethyl)phenyl)pyrrolidin-3-yl)cyclopropane-1-carboxyamide,
(1S,2S)-2-(1H-benzo[d]imidazol-2-yl)-N-((R)-1-(5-bromopyridin-3-yl)pyrrolidin-3-yl)cyclopropane-1-carboxyamide,
(R)-2-(quinolin-6-yl)-N-(1-(5-(trifluoromethyl)pyridin-3-yl)pyrrolidin-3-yl)acetamide,
(R)-2-(1H-indol-3-yl)-N-(1-(5-(trifluoromethyl)pyridin-3-yl)pyrrolidin-3-yl)acetamide,
(R)-2-(quinolin-7-yl)-N-(1-(5-(trifluoromethyl)pyridin-3-yl)pyrrolidin-3-yl)acetamide,
(R)-2-(4-cyclopropylphenyl)-N-(1-(4-(trifluoromethyl)oxazol-2-yl)pyrrolidin-3-yl)acetamide,
(R)-2-(1-methyl-5-(trifluoromethyl)-1H-pyrazol-3-yl)-N-(1-(5-(trifluoromethyl)pyridin-3-yl)pyrrolidin-3-yl)acetamide,
(R)-2-(6-(trifluoromethyl)pyridin-3-yl)-N-(1-(5-(trifluoromethyl)pyridin-3-yl)pyrrolidin-3-yl)acetamide,
(R)-2-(4-(trifluoromethyl) phenyl)-N-(1-(5-(trifluoromethyl)pyridin-3-yl)pyrrolidin-3-yl)acetamide-2, 2-d₂,
(R)-2-(4-(3,3-difluoropyrrolidin-1-yl)phenyl)-N-(1-(6-(trifluoromethyl)pyridin-3-yl)pyrrolidin-3-yl)acetamide,
(R)-2-(1H-indol-6-yl)-N-(1-(6-(trifluoromethyl)pyridin-3-yl)pyrrolidin-3-yl)acetamide,
2-(4-((2R,6S)-2,6-dimethylmorpholino)phenyl)-N-((R)-1-(5-(trifluoromethyl)pyridin-3-yl)pyrrolidin-3-yl)acetamide,
(R)-2-(4-(pyrrolidin-1-yl)phenyl)-N-(1-(5-(trifluoromethyl)pyridin-3-yl)pyrrolidin-3-yl)acetamide,
(R)-2-(4-((2-methoxyethyl)(methyl)amino)phenyl)-N-(1-(5-(trifluoromethyl)pyridin-3-yl)pyrrolidin-3-yl)acetamide,
2-(4-(trifluoromethyl)phenyl)-N-(1-(5-(trifluoromethyl)pyridin-3-yl)azetidin-3-yl)acetamide,
2-(1H-indol-6-yl)-N-(1-(5-(trifluoromethyl)pyridin-3-yl)azetidin-3-yl)acetamide,
(R)-2-(1-methyl-1H-indol-6-yl)-N-(1-(5-(trifluoromethyl)pyridin-3-yl)pyrrolidin-3-yl)acetamide,
(R)-2-(1-methyl-1H-indol-6-yl)-N-(1-(6-(trifluoromethyl)pyridin-3-yl)pyrrolidin-3-yl)acetamide,
(R)-2-(4-(4-methylpiperazin-1-yl)phenyl)-N-(1-(5-(trifluoromethyl)pyridin-3-yl)pyrrolidin-3-yl)acetamide,
(R)-2-(4-(piperazin-1-yl)phenyl)-N-(1-(5-(trifluoromethyl)pyridin-3-yl)pyrrolidin-3-yl)acetamide,
(R)-2-(4-(4-acetylpiperazin-1-yl)phenyl)-N-(1-(5-(trifluoromethyl)pyridin-3-yl)pyrrolidin-3-yl)acetamide,
(R)-2-(4-(2-oxopiperidin-1-yl)phenyl)-N-(1-(5-(trifluoromethyl)pyridin-3-yl)pyrrolidin-3-yl)acetamide,
(R)-2-(6-chlorobenzo[d]oxazol-2-yl)-N-(1-(5-(trifluoromethyl)pyridin-3-yl)pyrrolidin-3-yl)acetamide,
(R)-2-(benzo[d]oxazol-2-yl)-N-(1-(5-(trifluoromethyl)pyridin-3-yl)pyrrolidin-3-yl)acetamide,
(1S,2S)-2-(3,5-dichlorophenyl)-N-((R)-1-(5-(trifluoromethyl)pyridin-3-yl)pyrrolidin-3-yl)cyclopropane-1-carboxyamide,
(1R,2R)-2-(3,5-dichlorophenyl)-N-((R)-1-(5-(trifluoromethyl)pyridin-3-yl)pyrrolidin-3-yl)cyclopropane-1-carboxyamide,
(1S,2S)-2-(4-bromophenyl)-N-((R)-1-(5-(trifluoromethyl)pyridin-3-yl)pyrrolidin-3-yl)cyclopropane-1-carboxyamide,
(1R,2R)-2-(4-bromophenyl)-N-((R)-1-(5-(trifluoromethyl)pyridin-3-yl)pyrrolidin-3-yl)cyclopropane-1-carboxyamide,
(R)-2-(4-(1,4-dioxa-8-azaspiro[4.5]decan-8-yl)phenyl)-N-(1-(5-(trifluoromethyl)pyridin-3-yl)pyrrolidin-3-yl) acetamide,
(R)-2-(4-(4-oxopiperidin-1-yl)phenyl)-N-(1-(5-(trifluoromethyl)pyridin-3-yl)pyrrolidin-3-yl)acetamide,
(R)-2-(4-cyclopropylphenyl)-N-(3-(hydroxymethyl)-1-(5-(trifluoromethyl)pyridin-3-yl)pyrrolidin-3-yl)acetamide,
(S)-2-(4-cyclopropylphenyl)-N-(3-(hydroxymethyl)-1-(5-(trifluoromethyl)pyridin-3-yl)pyrrolidin-3-yl)acetamide,
(3-(2-(4-cyclopropylphenyl)acetamido)-1-(5-(trifluoromethyl)pyridin-3-yl)pyrrolidin-3-yl)methyl (R)-acetate,
(3-(2-(4-cyclopropylphenyl)acetamido)-1-(5-(trifluoromethyl)pyridin-3-yl)pyrrolidin-3-yl)methyl (S)-acetate,
(R)-2-(4-cyclopropylphenyl)-N-(3-(methoxymethyl)-1-(5-(trifluoromethyl)pyridin-3-yl)pyrrolidin-3-yl)acetamide,
(S)-2-(4-cyclopropylphenyl)-N-(3-(methoxymethyl)-1-(5-(trifluoromethyl)pyridin-3-yl)pyrrolidin-3-yl)acetamide,
(1S,2S)-2-(4-cyclopropylphenyl)-N-((R)-1-(5-(trifluoromethyl)pyridin-3-yl)pyrrolidin-3-yl)cyclopropane-1-carboxyamide,
(1R,2R)-2-(4-cyclopropylphenyl)-N-((R)-1-(5-(trifluoromethyl)pyridin-3-yl)pyrrolidin-3-yl)cyclopropane-1-carboxyamide,
(1S,2S)-2-(3,4-difluorophenyl)-N-((R)-1-(5-(trifluoromethyl)pyridin-3-yl)pyrrolidin-3-yl)cyclopropane-1-carboxyamide,
(1R,2R)-2-(3,4-difluorophenyl)-N-((R)-1-(5-(trifluoromethyl)pyridin-3-yl)pyrrolidin-3-yl)cyclopropane-1-carboxyamide,
(1S,2S)-2-(4-chlorophenyl)-N-((R)-1-(5-(trifluoromethyl)pyridin-3-yl)pyrrolidin-3-yl)cyclopropane-1-carboxyamide,
(1R,2R)-2-(4-chlorophenyl)-N-((R)-1-(5-(trifluoromethyl)pyridin-3-yl)pyrrolidin-3-yl)cyclopropane-1-carboxyamide,
2-(4-isopropylphenyl)-N-(1-(5-(trifluoromethyl)pyridin-3-yl)piperidin-4-yl)acetamide,
(R)-2-(4-isopropylphenyl)-N-(1-(6-methoxy-5-(trifluoromethyl)pyridin-3-yl)pyrrolidin-3-yl)acetamide,
(R)-2-(4-isopropylphenyl)-N-(1-(5-(trifluoromethyl)pyrimidin-2-yl)pyrrolidin-3-yl)acetamide,
(R)-2-(4-isopropylphenyl)-N-(1-(4-(trifluoromethyl)thiazol-2-yl)pyrrolidin-3-yl)acetamide,
(R)-2-(4-isopropylphenyl)-N-(1-(6-(trifluoromethyl)pyridin-3-yl)pyrrolidin-3-yl)acetamide,
(S)-2-(4-isopropylphenyl)-N-(1-(5-(trifluoromethyl)pyridin-3-yl)pyrrolidin-3-yl)acetamide,
(R)-2-(4-(trifluoromethoxy)phenyl)-N-(1-(5-(trifluoromethyl)pyridin-3-yl)pyrrolidin-3-yl)acetamide,
(R)-2-(4-(2,2,2-trifluoroethoxy)phenyl)-N-(1-(5-(trifluoromethyl)pyridin-3-yl)pyrrolidin-3-yl)acetamide,
2-(4-isopropylphenyl)-N-(1-(5-(trifluoromethyl)pyridin-3-yl)azetidin-3-yl)acetamide,
2-(4-isopropylphenyl)-N-(1-(6-(trifluoromethyl)pyridin-3-yl)azetidin-3-yl)acetamide,
(R)-2-(3,5-dichlorophenyl)-N-(1-(5-(trifluoromethyl)pyridin-3-yl)pyrrolidin-3-yl)acetamide,
(R)-2-(3-chloro-5-fluorophenyl)-N-(1-(5-(trifluoromethyl)pyridin-3-yl)pyrrolidin-3-yl)acetamide,
(R)-2-(4-cyclopropylphenyl)-N-(1-(6-(trifluoromethyl)pyridin-3-yl)pyrrolidin-3-yl)acetamide,
(R)-2-(4-isopropylphenyl)-N-(1-(5-(trifluoromethyl)thiophen-2-yl)pyrrolidin-3-yl)acetamide,
(R)-2-(4-(trifluoromethyl)phenyl)-N-(1-(5-(trifluoromethyl)pyridin-3-yl)pyrrolidin-3-yl)acetamide,
(R)-2-(1H-indol-6-yl)-N-(1-(5-(trifluoromethyl)pyridin-3-yl)pyrrolidin-3-yl)acetamide,
(R)-2-(4-cyclopropylphenyl)-N-(1-(6-(2,2,2-trifluoroethoxy)pyridin-3-yl)pyrrolidin-3-yl)acetamide,
(R)-2-(4-cyclopropylphenyl)-N-(1-(5-(2,2,2-trifluoroethoxy)pyridin-3-yl)pyrrolidin-3-yl) acetamide,
(R)-2-(4-cyclopropylphenyl)-N-(1-(4-(2,2,2-trifluoroethoxy)pyridin-2-yl)pyrrolidin-3-yl) acetamide,
(R)-2-(4-cyclopropylphenyl)-N-(1-(2-methoxy-5-(trifluoromethyl)pyridin-3-yl)pyrrolidin-3-yl)acetamide,
(R)-2-(4-cyclopropylphenyl)-N-(1-(5-(trifluoromethyl)pyridin-3-yl)piperidin-3-yl) acetamide,
(R)-2-(4-cyclopropylphenyl)-N-(1-(6-(trifluoromethyl)pyridin-3-yl)piperidin-3-yl) acetamide,
(R)-2-(4-(2-hydroxypropan-2-yl)phenyl)-N-(1-(5-(trifluoromethyl)pyridin-3-yl)pyrrolidin-3-yl)acetamide,
(R)-2-(4-(3-methylpyrazin-2-yl)phenyl)-N-(1-(5-(trifluoromethyl)pyridin-3-yl)pyrrolidin-3-yl)acetamide,
(R)-2-(4-(4-methyloxazol-5-yl) phenyl)-N-(1-(5-(trifluoromethyl)pyridin-3-yl)pyrrolidin-3-yl)acetamide,
(R)-3-(3-(2-(4-cyclopropylphenyl)acetamido)pyrrolidin-1-yl)-5-(trifluoromethyl)pyridine 1-oxide,
(3R)-3-(2-(4-cyclopropylphenyl)acetamido)-1-(5-(trifluoromethyl)pyridin-3-yl)pyrrolidine 1-oxide,
(R)-2-(4-cyclopropylphenyl)-2-methyl-N-(1-(5-(trifluoromethyl)pyridin-3-yl)pyrrolidin-3-yl)propanamide,
(R)-2-(4-cyclopropylphenyl)-N-(1-(3-fluoro-5-(trifluoromethyl)phenyl)pyrrolidin-3-yl) acetamide,
(R)-2-amino-2-(4-cyclopropylphenyl)-N-((R)-1-(5-(trifluoromethyl)pyridin-3-yl)pyrrolidin-3-yl)acetamide,
(S)-2-amino-2-(4-cyclopropylphenyl)-N-((R)-1-(5-(trifluoromethyl)pyridin-3-yl)pyrrolidin-3-yl)acetamide,
(R)-2-(4-cyclopropylphenyl)-2-(dimethylamino)-N-((R)-1-(5-(trifluoromethyl)pyridin-3-yl)pyrrolidin-3-yl)acetamide,
(S)-2-(4-cyclopropylphenyl)-2-(dimethylamino)-N-((R)-1-(5-(trifluoromethyl)pyridin-3-yl)pyrrolidin-3-yl)acetamide,
(R)-2-(1-methyl-1H-indol-5-yl)-N-(1-(5-(trifluoromethyl)pyridin-3-yl)pyrrolidin-3-yl)acetamide,
(R)-2-(2,4-bis(trifluoromethyl)phenyl)-N-(1-(5-(trifluoromethyl)pyridin-3-yl)pyrrolidin-3-yl)acetamide,
(R)-2-(2-fluoro-4-(trifluoromethyl)phenyl)-N-(1-(5-(trifluoromethyl)pyridin-3-yl)pyrrolidin-3-yl)acetamide,
(R)-2-(1-methyl-1H-indazol-5-yl)-N-(1-(5-(trifluoromethyl)pyridin-3-yl)pyrrolidin-3-yl)acetamide,
(R)-2-(1-methyl-1H-indazol-5-yl)-N-(1-(4-(trifluoromethyl)thiazol-2-yl)pyrrolidin-3-yl)acetamide,
(R)-2-(1-methyl-1H-indazol-5-yl)-N-(1-(6-(trifluoromethyl)pyridin-3-yl)pyrrolidin-3-yl)acetamide,
(R)-2-(4-(2,2-dimethylmorpholino)phenyl)-N-(1-(5-(trifluoromethyl)pyridin-3-yl)pyrrolidin-3-yl)acetamide,
(R)-2-(4-(1H-pyrazol-1-yl)phenyl)-N-(1-(5-(trifluoromethyl)pyridin-3-yl)pyrrolidin-3-yl)acetamide,
(R)-N-(3-methyl-1-(5-(trifluoromethyl)pyridin-3-yl)pyrrolidin-3-yl)-2-(4-morpholinophenyl)acetamide,
(R)-2-(4-cyclopropylphenyl)-N-(3-methyl-1-(5-(trifluoromethyl)pyridin-3-yl)pyrrolidin-3-yl)acetamide,
2-(4-(3-oxa-8-azabicyclo[3.2.1]octan-8-yl)phenyl)-N-((R)-1-(5-(trifluoromethyl)pyridin-3-yl)pyrrolidin-3-yl)acetamide,
2-(4-(3-oxa-8-azabicyclo[3.2.1]octan-8-yl)phenyl)-N-((R)-1-(6-(trifluoromethyl)pyridin-3-yl)pyrrolidin-3-yl)acetamide,
(R)-2-(benzo[d]oxazol-5-yl)-N-(1-(6-(trifluoromethyl)pyridin-3-yl)pyrrolidin-3-yl)acetamide,
(R)-2-(benzo[d]oxazol-5-yl)-N-(1-(5-(trifluoromethyl)pyridin-3-yl)pyrrolidin-3-yl)acetamide,
(R)-2-(2-methylbenzo[d]oxazol-5-yl)-N-(1-(5-(trifluoromethyl)pyridin-3-yl)pyrrolidin-3-yl)acetamide,
(R)-2-(2-methylbenzo[d]oxazol-5-yl)-N-(1-(6-(trifluoromethyl)pyridin-3-yl)pyrrolidin-3-yl)acetamide,
2-(4-((1S,4S)-2-oxa-5-azabicyclo[2.2.2]octan-5-yl)phenyl)-N-((R)-1-(5-(trifluoromethyl)pyridin-3-yl)pyrrolidin-3-yl)acetamide,
2-(4-((1S,4S)-2-oxa-5-azabicyclo[2.2.2]octan-5-yl)phenyl)-N-((R)-1-(6-(trifluoromethyl)pyridin-3-yl)pyrrolidin-3-yl)acetamide,
(R)-2-(benzo[d]thiazol-6-yl)-N-(1-(5-(trifluoromethyl)pyridin-3-yl)pyrrolidin-3-yl)acetamide,
(R)-2-(benzo[d]thiazol-6-yl)-N-(1-(6-(trifluoromethyl)pyridin-3-yl)pyrrolidin-3-yl)acetamide,
(R)-2-(1H-indazol-5-yl)-N-(1-(5-(trifluoromethyl)pyridin-3-yl)pyrrolidin-3-yl)acetamide,
(R)-2-(1H-indazol-5-yl)-N-(1-(6-(trifluoromethyl)pyridin-3-yl)pyrrolidin-3-yl)acetamide,
2-(4-cyclopropylphenyl)-N-(3-(trifluoromethyl)-1-(5-(trifluoromethyl)pyridin-3-yl)pyrrolidin-3-yl)acetamide,
(R)-N-(3-(trifluoromethyl)-1-(5-(trifluoromethyl)pyridin-3-yl)pyrrolidin-3-yl)-2-(4-(trifluoromethyl)phenyl)acetamide,
(S)-N-(3-(trifluoromethyl)-1-(5-(trifluoromethyl)pyridin-3-yl)pyrrolidin-3-yl)-2-(4-(trifluoromethyl)phenyl)acetamide,
(R)-2-(1H-benzo[d][1,2,3]triazol-5-yl)-N-(1-(5-(trifluoromethyl)pyridin-3-yl)pyrrolidin-3-yl)acetamide,
(R)-2-(1H-benzo[d][1,2,3]triazol-5-yl)-N-(1-(6-(trifluoromethyl)pyridin-3-yl)pyrrolidin-3-yl)acetamide,
2-(4-((1R,4R)-2-oxa-5-azabicyclo[2.2.1]heptan-5-yl)phenyl)-N-((R)-1-(5-(trifluoromethyl)pyridin-3-yl)pyrrolidin-3-yl)acetamide,
2-(4-((1S,4S)-2-oxa-5-azabicyclo[2.2.1]heptan-5-yl)phenyl)-N-((R)-1-(5-(trifluoromethyl)pyridin-3-yl)pyrrolidin-3-yl)acetamide,
2-(4-((1R,4R)-2-oxa-5-azabicyclo[2.2.1]heptan-5-yl)phenyl)-N-((R)-1-(6-(trifluoromethyl)pyridin-3-yl)pyrrolidin-3-yl)acetamide,
2-(4-((1S,4S)-2-oxa-5-azabicyclo[2.2.1]heptan-5-yl)phenyl)-N-((R)-1-(6-(trifluoromethyl)pyridin-3-yl)pyrrolidin-3-yl)acetamide,
(R)-2-(1-methyl-1H-benzo[d][1,2,3]triazol-5-yl)-N-(1-(5-(trifluoromethyl)pyridin-3-yl)pyrrolidin-3-yl)acetamide,
(R)-2-(1-methyl-1H-benzo[d][1,2,3]triazol-5-yl)-N-(1-(6-(trifluoromethyl)pyridin-3-yl)pyrrolidin-3-yl)acetamide,
2-(4-cyclopropylphenyl)-N-(1-(4-(trifluoromethyl)phenyl)azetidin-3-yl)acetamide,
2-(4-cyclopropylphenyl)-N-(1-(4-fluoro-3-(trifluoromethyl)phenyl)azetidin-3-yl)acetamide,
(R)-2-(4-bromophenyl)-N-(1-(5-(trifluoromethyl)pyridin-3-yl)pyrrolidin-3-yl)acetamide,
(R)-2-(4-cyclopropylphenyl)-N-(1-(6-fluorobenzo[d]thiazol-2-yl)pyrrolidin-3-yl)acetamide,
(R)-N-(1-(5-bromothiazol-2-yl)pyrrolidin-3-yl)-2-(4-cyclopropylphenyl)acetamide,
(R)-2-(4-cyclopropylphenyl)-N-(1-(4-fluoro-3-(trifluoromethyl)phenyl)pyrrolidin-3-yl)acetamide,
(R)-2-(4-cyclopropylphenyl)-N-(1-(3-methoxyphenyl)pyrrolidin-3-yl) acetamide,
(R)-2-(4-(3,3-difluoropyrrolidin-1-yl)phenyl)-N-(1-(5-(trifluoromethyl)pyridin-3-yl)pyrrolidin-3-yl)acetamide,
ethyl (R)-2-(3-(3-(2-(4-cyclopropylphenyl)acetamido)pyrrolidin-1-yl)phenoxy)-2-methylpropanoate,
(R)-2-(4-cyclopropylphenyl)-N-(1-(3-hydroxyphenyl)pyrrolidin-3-yl) acetamide,
(R)-2-(4-cyclopropylphenyl)-N-(1-(6-(trifluoromethyl)pyridin-2-yl)pyrrolidin-3-yl)acetamide,
(R)-2-(4-cyclopropylphenyl)-N-(1-(4, 6-dimethoxypyrimidin-2-yl)pyrrolidin-3-yl) acetamide,
(R)-2-(4-morpholinophenyl)-N-(1-(5-(trifluoromethyl)pyridin-3-yl)pyrrolidin-3-yl)acetamide,
(R)-2-(4-cyclopropylphenyl)-N-(1-(4-(trifluoromethyl)pyridin-2-yl)pyrrolidin-3-yl)acetamide,
(R)-2-(4-(trifluoromethyl)phenyl)-N-(1-(6-(trifluoromethyl)pyridin-3-yl)pyrrolidin-3-yl)acetamide,
(R)-N-(1-(4-fluoro-3-(trifluoromethyl)phenyl)pyrrolidin-3-yl)-2-(4-(trifluoromethyl)phenyl)acetamide,
(R)-2-(4-(trifluoromethyl)phenyl)-N-((R)-1-(6-(trifluoromethyl)pyridin-3-yl)pyrrolidin-3-yl)propanamide,
(S)-2-(4-(trifluoromethyl)phenyl)-N-((R)-1-(6-(trifluoromethyl)pyridin-3-yl)pyrrolidin-3-yl)propanamide,
(1S,2S)-2-(1H-benzo[d]imidazol-2-yl)-N-((R)-1-(5-(trifluoromethyl)pyridin-3-yl)piperidin-3-yl)cyclopropane-1-carboxyamide,
(R)-2-(1H-indol-6-yl)-N-(1-(4-(trifluoromethyl)thiazol-2-yl)pyrrolidin-3-yl) acetamide, and
(R)-2-(1-methyl-1H-indol-5-yl)-N-(1-(6-(trifluoromethyl)pyridin-3-yl)pyrrolidin-3-yl)acetamide.

22. A pharmaceutical composition comprising a compound according to any one of claims 1 to 21, a tautomer or a stereoisomer of the compound, a pharmaceutically acceptable salt of the compound, or a solvate of the compound, the tautomer, the stereoisomer, or the salt, as an active ingredient.

23. A T-type calcium channel blocker comprising a compound according to any one of claims 1 to 21, a tautomer or a stereoisomer of the compound, a pharmaceutically acceptable salt of the compound, or a solvate of the compound, the tautomer, the stereoisomer, or the salt, as an active ingredient.

24. A therapeutic or prophylactic agent for stroke, atopic dermatitis, hypertension, hyperaldosteronemia, edema, ischemic heart disease, age-related macular degeneration, cancer, diabetes mellitus, infertility, sexual dysfunction, arrhythmia, kidney diseases, epilepsy, essential tremor, schizophrenia, Parkinson's disease, manic depression, bipolar disorder, depression, anxiety, cognitive impairment, drug dependence, Huntington's disease, sleep disorders, or overactive bladder, the therapeutic or prophylactic agent comprising a compound according to any one of claims 1 to 21, a tautomer or a stereoisomer of the compound, a pharmaceutically acceptable salt of the compound, or a solvate of the compound, the tautomer, the stereoisomer, or the salt, as an active ingredient.

25. A therapeutic or prophylactic agent for acute pain or chronic pain, and preferably neuropathic pain, the therapeutic or prophylactic agent comprising a compound according to any one of claims 1 to 21, a tautomer or a stereoisomer of the compound, a pharmaceutically acceptable salt of the compound, or a solvate of the compound, the tautomer, the stereoisomer, or the salt, as an active ingredient.

26. Use of a compound according to any one of claims 1 to 21, a tautomer or a stereoisomer of the compound, a pharmaceutically acceptable salt of the compound, or a solvate of the compound, the tautomer, the stereoisomer, or the salt, for the prevention or treatment of stroke, atopic dermatitis, hypertension, hyperaldosteronemia, edema, ischemic heart disease, age-related macular degeneration, cancer, diabetes mellitus, infertility, sexual dysfunction, arrhythmia, kidney diseases, epilepsy, essential tremor, schizophrenia, Parkinson's disease, manic depression, bipolar disorder, depression, anxiety, cognitive impairment, drug dependence, Huntington's disease, sleep disorders, or overactive bladder.

27. Use of a compound according to any one of claims 1 to 21, a tautomer or a stereoisomer of the compound, a pharmaceutically acceptable salt of the compound, or a solvate of the compound, the tautomer, the stereoisomer, or the salt, for the prevention or treatment of acute pain or chronic pain, and preferably neuropathic pain.

28. A method for treating acute pain or chronic pain, and, preferably, neuropathic pain in a human being, the method comprising a step of administering an effective amount of a compound according to any one of claims 1 to 21, a tautomer or a stereoisomer of the compound, a pharmaceutically acceptable salt of the compound, or a solvate of the compound, the tautomer, the stereoisomer, or the salt, to the human being.

29. Use of a compound according to any one of claims 1 to 21, a tautomer or a stereoisomer of the compound, a pharmaceutically acceptable salt of the compound, or a solvate of the compound, the tautomer, the stereoisomer, or the salt, for the production of a T-type calcium channel blocker.

30. Use of a compound according to any one of claims 1 to 21, a tautomer or a stereoisomer of the compound, a pharmaceutically acceptable salt of the compound, or a solvate of the compound, the tautomer, the stereoisomer, or the salt, for the production of a prophylactic agent or a therapeutic agent for stroke, atopic dermatitis, hypertension, hyperaldosteronemia, edema, ischemic heart disease, age-related macular degeneration, cancer, diabetes mellitus, infertility, sexual dysfunction, arrhythmia, kidney diseases, epilepsy, essential tremor, schizophrenia, Parkinson's disease, manic depression, bipolar disorder, depression, anxiety, cognitive impairment, drug dependence, Huntington's disease, sleep disorders, or overactive bladder.

31. Use of a compound according to any one of claims 1 to 21, a tautomer or a stereoisomer of the compound, a pharmaceutically acceptable salt of the compound, or a solvate of the compound, the tautomer, the stereoisomer, or the salt, for the production of a prophylactic agent or a therapeutic agent for acute pain or chronic pain, and preferably neuropathic pain.
